(19) Europäisches Patentamt
European Patent Office
Office européen des brevets

(11) EP 2 222 165 B1

(12) **EUROPEAN PATENT SPECIFICATION**

(45) Date of publication and mention of the grant of the patent:
**31.07.2013 Bulletin 2013/31**

(21) Application number: **08852528.2**

(22) Date of filing: **20.11.2008**

(51) Int Cl.:
***C07D 277/46*** *(2006.01)*

(86) International application number:
**PCT/US2008/084216**

(87) International publication number:
**WO 2009/067613 (28.05.2009 Gazette 2009/22)**

# (54) NOVEL COMPOUNDS AS CANNABINOID RECEPTOR LIGANDS AND USES THEREOF

NEUE VERBINDUNGEN ALS CANNABINOIDREZEPTORLIGANDEN UND IHRE VERWENDUNGEN

NOUVEAUX COMPOSÉS EN TANT QUE LIGANDS DE RÉCEPTEUR DE CANNABINOÏDE ET LEURS UTILISATIONS

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **21.11.2007 US 989492 P**

(43) Date of publication of application:
**01.09.2010 Bulletin 2010/35**

(73) Proprietor: **AbbVie Inc.**
**North Chicago, IL 60064 (US)**

(72) Inventors:
- **FLORJANCIC, Alan, S.** **Kenosha** **Wisconsin 53144 (US)**
- **DART, Michael, J.** **Highland Park** **Illinois 60035 (US)**
- **RYTHER, Keith, B.** **Round Lake Park** **Illinois 60073 (US)**
- **PEREZ-MEDRANO, Arturo** **Grayslake** **Illinois 60030 (US)**
- **CARROLL, William, A.** **Evanston** **Illinois 60201 (US)**
- **PATEL, Meena, V.** **Green Oaks** **Illinois 60048 (US)**
- **TIETJE, Karin, Rosemarie** **Mundelein** **Illinois 60060 (US)**
- **LI, Tongmei** **Lake Bluff** **Illinois 60044 (US)**
- **KOLASA, Teodozyj** **Lake Villa** **Illinois 60046 (US)**
- **GALLAGHER, Megan, E.** **Chicago** **Illinois 60625 (US)**
- **PEDDI, Sridhar** **Grayslake** **Illinois 60030 (US)**
- **FROST,Jennifer, M.** **Grayslake** **Illinois 60030 (US)**
- **NELSON, Derek, W.** **Highland Park** **Illinois 60035 (US)**

(74) Representative: **Modiano, Micaela Nadia et al**
**Modiano & Partners**
**Thierschstrasse 11**
**80538 München (DE)**

(56) References cited:
**EP-A1- 1 820 504** **WO-A2-2007/140385**
**WO-A2-2007/140439**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- OHTA ET AL: "N-Alkylidenearylcarboxamides as new potent and selective CB2 cannabinoid receptor agonists with good oral bioavailability" BIOORGANIC & MEDICINAL CHEMISTRY LETTERS, PERGAMON, ELSEVIER SCIENCE, GB LNKD- DOI:10.1016/J.BMCL.2007.09.004, vol. 17, no. 22, 12 October 2007 (2007-10-12), pages 6299-6304, XP022297147 ISSN: 0960-894X
- GOULDSON ET AL.: 'Mutational analysis and molecular modeling of the antagonist SR144528 binding site on the human cannabinoid CB2 receptor.' EUROPEAN JOURNAL OF PHARMACOLOGY vol. 401, 2000, pages 17 - 25

## Description

### Technical Field

The present invention relates to compounds that are cannabinoid receptor ligands, compositions comprising such compounds, and methods of treating conditions and disorders using such compounds and compositions.

### Background

(-)-$\Delta^9$-Tetrahydrocannabinol ($\Delta^9$-THC), the major psychoactive constituent of marijuana, exerts a broad range of therapeutic effects through its interactions with two cannabinoid (CB) receptor subtypes, $CB_1$ and $CB_2$. $CB_1$ receptors are highly expressed in the central nervous system and to a lesser degree in the periphery in a variety of tissues of the cardiovascular and gastrointestinal systems. By contrast, $CB_2$ receptors are most abundantly expressed in multiple lymphoid organs and cells of the immune system, including spleen, thymus, tonsils, bone marrow, pancreas and mast cells.

The psychotropic effects caused by $\Delta^9$-THC and other nonselective CB agonists are mediated by $CB_1$ receptors. These $CB_1$ receptor-mediated effects, such as euphoria, sedation, hypothermia, catalepsy, and anxiety, have limited the development and clinical utility of nonselective CB agonists. Recent studies have demonstrated that $CB_2$ modulators are analgesic in preclinical models of nociceptive and neuropathic pain without causing the adverse side effects associated with $CB_1$ receptor activation. Therefore, compounds that selectively target $CB_2$ receptors are an attractive approach for the development of novel analgesics.

Pain is the most common symptom of disease and the most frequent complaint with which patients present to physicians. Pain is commonly segmented by duration (acute vs. chronic), intensity (mild, moderate, and severe), and type (nociceptive vs. neuropathic).

Nociceptive pain is the most well known type of pain, and is caused by tissue injury detected by nociceptors at the site of injury. After the injury, the site becomes a source of ongoing pain and tenderness. This pain and tenderness are considered "acute" nociceptive pain. This pain and tenderness gradually diminish as healing progresses and disappear when healing is complete. Examples of acute nociceptive pain include surgical procedures (post-op pain) and bone fractures. Even though there may be no permanent nerve damage, "chronic" nociceptive pain results from some conditions when pain extends beyond six months. Examples of chronic nociceptive pain include osteoarthritis, rheumatoid arthritis, and musculoskeletal conditions (e.g., back pain), cancer pain, etc.

Neuropathic pain is defined as "pain initiated or caused by a primary lesion or dysfunction in the nervous system" by the International Association for the Study of Pain. Neuropathic pain is not associated with nociceptive stimulation, although the passage of nerve impulses that is ultimately perceived as pain by the brain is the same in both nociceptive and neuropathic pain. The term neuropathic pain encompasses a wide range of pain syndromes of diverse etiologies. The three most commonly diagnosed pain types of neuropathic nature are diabetic neuropathy, cancer neuropathy, and HIV pain. In addition, neuropathic pain is diagnosed in patients with a wide range of other disorders, including trigeminal neuralgia, post-herpetic neuralgia, traumatic neuralgia, phantom limb, as well as a number of other disorders of ill-defined or unknown origin.

Managing the spectrum of pain etiologies remains a major public health problem and both patients and clinicians are seeking improved strategies to effectively manage pain. No currently available therapies or drugs effectively treat all types of nociceptive and neuropathic pain states. The compounds of the present invention are novel $CB_2$ receptor modulators that have utility in treating pain, including nociceptive and neuropathic pain.

The location of $CB_2$ receptors on the surface of immune cells suggests a role for these receptors in immunomodulation and inflammation. Recent studies have demonstrated that $CB_2$ receptor ligands have immunomodulatory and anti-inflammatory properties. OHTA et al "N-Alkylidenearylcarboxamides as new potent and selective CB2 cannabinoid receptor agonists with good oral bioavailability "Bioorganic and Medicinal Chemistry Letters, Pergamon, Elsevier Science, GB LNKD-DO1, vol.17 Issue 22, 12 Oct. 2007, pages 6299-6304, disclose a novel series of N-alkylidenearyl carboxamides as CB2 receptor agonists.

Accordingly, the need exists to further explore and develop $CB_2$ receptor ligands that exhibit immunomodulatory and anti-inflammatory properties. These $CB_2$ receptors ligands will offer a unique pharmacotherapy for the treatment of immune and inflammatory disorders.

### Summary

The present invention generally provides compounds that are $CB_2$ receptor ligands and pharmaceutical compositions and methods for the treatment of disorders using these compounds and pharmaceutical compositions.

One aspect of the invention is directed towards compounds of formula (I), or pharmaceutical salts,

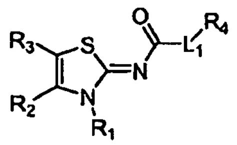

(I),

$R_1$ is A, or A-alkylene-;

$R_1$ is hydrogen, alkoxy, alkoxyalkyl, alkoxycarbonyl, alkoxycarbonylalkyl, alkyl, alkyl-$S(O)_2$-, aryl, arylalkyl, arylalkenyl, azidoalkyl, cyano, cycloalkyl, halo, haloalkyl, heteroaryl, heterocycle, $-(CR_{21}R_{22})_m$-OH, $R_aR_bN$-, $R_aR_bN$-alkyl-, $R_cR_dNC(O)$-, or $R_8$-$R_7$-;

$R_3$ is hydrogen, alkoxy, alkoxyalkyl, alkyl, alkylcarbonyl, alkyl-$S(O)_2$-, aryl, arylalkyl, arylalkenyl, cyano, cycloalkyl, halo, haloalkyl, heteroaryl, heterocycle, $-(CR_{31}R_{32})_m$-OH, $R_aR_bN$-, $R_aR_bN$-alkyl-, or $R_8$-$R_7$-; or

$R_2$ and $R_3$, together with the carbon atoms to which they are attached, form a 4-, 5-, 6, or 7-membered monocyclic ring, optionally fused to benzo or oxadiazole, said monocyclic ring contains zero or one additional double bond, zero or one oxygen atom, and zero or one nitrogen atom as ring atoms; two non-adjacent atoms of said monocyclic ring are optionally linked by an alkenylene bridge of 2, 3, or 4 carbon atoms, or optionally linked by an alkylene bridge of 1, 2, 3, or 4 carbon atoms, said monocyclic ring is independently unsubstituted or substituted with 1, 2, 3, 4, or 5 substituents independently selected from the group consisting of oxo, alkyl, halo, -OH, -O(alkyl), and haloalkyl; two substituents on the same carbon atom of said monocyclic ring, together with the carbon atom to which they are attached, optionally form a 3-, 4-, 5-, or 6-membered monocyclic cycloalkyl ring, wherein the monocyclic cycloalkyl ring is optionally substituted with 1, 2, 3, 4, 5, or 6 substituents independently selected from the group consisting of alkyl and haloalkyl;

$R_4$ is alkyl, alkynyl, cycloalkyl, cycloalkenyl, aryl, heteroaryl, heterocycle, cycloalkylalkyl, cycloalkenylalkyl, arylalkyl, heteroarylalkyl, heterocycloalkyl, or $R_{10}$-$L_2$-$R_9$-; wherein the alkyl group is optionally substituted with one substituent selected from the group consisting of alkoxy, alkoxycarbonyl, carboxy, halo, -OH, and $R_eR_fN$-;

$R_7$, $R_8$, and $R_9$, are each independently aryl, cycloalkyl, cycloalkenyl, heteroaryl, or heterocycle;

$R_{10}$ is aryl, cycloalkyl, cycloalkenyl, heteroaryl, heterocycle, or cycloalkylalkyl;

$R_a$ and $R_b$, at each occurrence, are each independently hydrogen, alkoxycarbonyl, alkyl, alkylcarbonyl, alkyl-$S(O)_2$-, or arylalkyl;

$R_c$ and $R_d$, are each independently hydrogen or alkyl;

$R_e$ and $R_f$, are each independently hydrogen, alkyl, or alkylcarbonyl; A is furanyl, oxazolyl, isoxazolyl, or oxadiazolyl; each ring A is optionally fused with a monocyclic ring selected from the group consisting of benzo, cycloalkyl, cycloalkenyl, heterocycle and heteroaryl; and each A is independently unsubstituted or substituted with 1, 2, 3,4, 5, or 6 substituents independently selected from the group consisting ofoxo, alkyl, halo, -OH, -O(alkyl), and haloalkyl;

$L_1$ is a single bond or $NR_8$-;

$L_2$ is a single bond, alkylene, or -O-;

$R_8$ is hydrogen or alkyl;

the aryl, cycloalkyl, cycloalkenyl, heterocycle, or heteroaryl moieties, as a substituent, or as part of a substituent, as represented by $R_1$, $R_3$, $R_4$, $R_a$, $R_b$, $R_7$, $R_8$, $R_9$, and $R_{10}$, are each independently unsubstituted or substituted with 1, 2, 3, 4, 5, or 6 substituents independently selected from the group consisting of alkenyl, alkoxy, alkoxyalkoxy, alkoxyalkoxyalkyl, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfinyl, alkylsulfinylalkyl, alkyl-$S(O)_2$-, alkyl-$S(O)_2$-$(CR_{41}R_{42})_p$=$C(R_{41})$-, alkyl-$S(O)_2$-$(CR_{41}R_{42})_p$-, alkyl-S-, alkyl-S-$(CR_{41}R_{42})_p$-, alkynyl, carboxy, carboxyalkyl, cyano, cyanoalkyl, formyl, formylalkyl, halogen, haloalkyl, haloalkoxy, hydroxy, hydroxyalkyl, oxo, -SH, $N(O)_2$, $-C(R_{41})$=N-$O(R_{42})$, $-(CR_{41}R_{42})_p$-$C(R_{41})$=N-$O(R_{42})$, =N-O(alkyl), =N-OH, $NZ_1Z_2$-$(CR_{41}R_{42})_p$-O-, -O-$(CR_{41}R_{42})_p$-$G_1$, $G_1$, -$NZ_1Z_2$, $-(CR_{41}R_{42})_p$-$NZ_1Z_2$, and $(NZ_3Z_4)$carbonyl;

$G_1$ is a 4-, 5-, 6-, or 7-membered monocyclic heterocycle containing one nitrogen atom and optionally 1 or 2 additional heteroatom in the ring, wherein said ring is attached to the parent moiety through the nitrogen atom, and said ring is optionally substituted with 1, 2, 3, 4, or 5 substituents selected from the group consisting of alkyl, alkenyl, alkynyl, halo, haloalkyl, =N-CN, =N-$OR_{51}$, -CN, oxo, -$OR_{51}$, -OC(O)$R_{51}$, -OC(O)$N(R_{51})_2$, -$S(O)_2R_{52}$, -$S(O)_2N(R_{51})_2$, -C(O)$R_{51}$, -C(O)$OR_{51}$, -C(O)$N(R_{51})_2$, -$N(R_{51})_2$, -$N(R_{51})C(O)R_{51}$, -$N(R_{51})S(O)_2R_{52}$, -$N(R_{51})C(O)O(R_{52})$, -$N(R_{51})C(O)N(R_{51})_2$, $-(CR_{1c}R_{1d})_q$-$OR_{51}$, $-(CR_{1c}R_{1d})_q$-OC(O)$R_{51}$, $-(CR_{1c}R_{1d})_q$-OC(O)$N(R_{51})_2$, $-(CR_{1c}R_{1d})_q$-$S(O)_2R_{52}$, $-(CR_{1c}R_{1d})_q$-$S(O)_2N(R_{51})_2$, $-(CR_{1c}R_{1d})_q$-C(O)$R_{51}$, $-(CR_{1c}R_{1d})_q$-C(O)$OR_{51}$, $-(CR_{1c}R_{1d})_q$-C(O)$N(R_{51})_2$, $-(CR_{1c}R_{1d})_q$-$N(R_{51})_2$, $-(CR_{1c}R_{1d})_q$-$N(R_{51})C(O)R_{51}$, $-(CR_{1c}R_{1d})_q$-$N(R_{51})S(O)_2R_{52}$, $-(CR_{1c}R_{1d})_q$-$N(R_{51})C(O)O(R_{52})$, $-(CR_{1c}R_{1d})_q$-$N(R_{51})C(O)N(R_{51})_2$, and $-(CR_{1c}R_{1d})_q$-CN;

$R_{51}$, at each occurrence, is independently hydrogen, $C_1$-$C_4$ alkyl, $C_1$-$C_4$ haloalkyl, -$\{CR_{2c}R_{2d})_u$-$OR^{53}$, monocyclic cycloalkyl, or $-(CR_{2c}R_{2d})_u$-(monocyclic cycloalkyl); wherein $R_{53}$ is .hydrogen, $C_1$-$C_4$ alkyl, $C_1$-$C_4$ haloalkyl, monocyclic cycloalkyl, or $-(CR_{2c}R_{2d})_u$-(monocyclic cycloalkyl);

$R_{52}$, at each occurrence, is independently $C_1$-$C_4$ alkyl, $C_1$-$C_4$ haloalkyl, monocyclic cycloalkyl, or -($CR_{2c}R_{2d}$)$_u$-(monocyclic cycloalkyl);

the monocyclic cycloalkyl moiety, as a substituent, or as part of a substituent, as represented by $R_{51}$, $R_{52}$, and $R_{53}$ are each independently unsubstituted or substituted with 1, 2, 3, or 4 substituents selected from the group consisting of $C_1$-$C_4$ alkyl, halo, hydroxy, $C_1$-$C_4$ alkoxy, $C_1$-$C_4$ haloalkoxy, and $C_1$-$C_4$ haloalkyl;

$R_{21}$, $R_{22}$, $R_{31}$, $R_{32}$, $R_{41}$, $R_{42}$, $R_{1c}$, $R_{1d}$, $R_{2c}$, and $R_{2d}$, at each occurrence, are each independently hydrogen, alkyl, haloalkyl, or halo;

m, p, q, and u, at each occurrence, are each independently 1, 2, 3, or 4;

$Z_1$ and $Z_2$ are each independently hydrogen, alkyl, alkoxyalkyl, alkylcarbonyl, cyanoalkyl, haloalkyl, or **formyl;** and

$Z_3$ and $Z_4$ are each independently hydrogen, alkyl, haloalkyl, phenyl or benzyl wherein the phenyl moiety is optionally substituted with 1, 2, 3, or 4 substituents selected from the group consisting of alkyl, hydroxyl, and haloalkyl.

In another embodiment, the present invention provides a pharmaceutical composition comprising a therapeutically effective amount of a compound of the invention or a pharmaceutically acceptable salt thereof in combination with one or more pharmaceutically acceptable carriers. Such compositions can be administered in accordance with a method of the invention, typically as part of a therapeutic regimen for treatment or prevention of conditions and disorders related to cannabinoid (CB) receptor subtype, $CB_2$. More particularly, the method is useful for treating conditions related to neuropathic pain, nociceptive pain, inflammatory pain, inflammatory disorders, immune disorders, neurological disorders, cancers of the immune system, respiratory disorders, obesity, diabetes, cardiovascular disorders, or for providing ncuroprotection.

Further, the present invention provides the use of compounds of the present invention or a pharmaceutically acceptable salt thereof, in the manufacture of a medicament for the treatment of the disease conditions described above, alone or in combination with one or more pharmaceutically acceptable carrier(s), particularly for the treatment of neuropathic pain, nociceptive pain, inflammatory pain, or combination thereof.

The compounds, compositions comprising the compounds, and methods for treating or preventing conditions and disorders by administering the compounds are further described herein.

These and other objects of the invention are described in the following paragraphs. These objects should not be deemed to narrow the scope of the invention.

## DETAILED DESCRIPTION

Compounds of formulae (I) and (II) are disclosed in this invention,

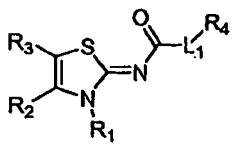

(I)

wherein $R_1$, $R_2$, $R_3$, $R_4$, $L_1$, $R_{1a}$, $R_{2a}$, $R_x$, and n are as defined above in the Summary of the Invention and below in the Detailed Description. Compositions comprising such compounds and methods for treating conditions and disorders using such compounds and compositions are also disclosed.

In various embodiments, the present invention provides at least one variable that occurs more than one time in any substituent or in the compound of the invention or any other formulae herein. Definition of a variable on each occurrence is independent of its definition at another occurrence. Further, combinations of substituents are permissible only if such combinations result in stable compounds. Stable compounds are compounds, which can be isolated from a reaction mixture.

### a. Definitions

As used in the specification and the appended claims, unless specified to the contrary, the following terms have the meaning indicated:

The term "alkenyl" as used herein, means a straight or branched chain hydrocarbon containing from 2 to 10 carbons and containing at least one carbon-carbon double bond. Representative examples of alkenyl include, but are not limited to, ethenyl (vinyl), 2-propenyl, 2-methyl-2-propenyl, 3-butenyl, 4-pentenyl, 5-hexenyl, 2-heptenyl, 2-methyl-1-heptenyl, and 3-decenyl.

The term "alkenylene" denotes a divalent group derived from a straight or branched chain hydrocarbon of 2, 3, or 4 carbon atoms and contains at least one carbon-carbon double bond. Representative examples of alkenylene include, but are not limited to, -CH=CH- and $-CH_2CH=CH-$.

The term "alkoxy" as used herein, means an alkyl group, as defined herein, appended to the parent molecular moiety through an oxygen atom. The term "$C_1$-$C_4$ alkoxy" a $C_1$-$C_4$ alkyl group, as defined herein, appended to the parent molecular moiety through an oxygen atom. Representative examples of alkoxy include, but arc not limited to, methoxy, ethoxy, propoxy, 2-propoxy, butoxy, tert-butoxy, pentyloxy, and hexyloxy.

The term "alkoxyalkoxy" as used herein, means an alkoxy group, as defined herein, appended to the parent molecular moiety through another alkoxy group, as defined herein. Representative examples of alkoxyalkoxy include, but are not limited to, tert-butoxymethoxy, 2-ethoxyethoxy, 3-methoxy-3-methylbutoxy, 2-methoxyethoxy, and methoxymethoxy.

The term "alkoxyalkoxyalkyl" as used herein, means an alkoxyalkoxy group, as defined herein, appended to the parent molecular moiety through an alkylene group, as defined herein. Representative examples of alkoxyalkoxyalkyl include, but are not limited to, tert-butoxymethoxymethyl, ethoxymethoxymethyl, (2-methoxyethoxy)methyl, and 2-(2-methoxyethoxy)ethyl.

The tenn "alkoxyalkyl" as used herein, means an alkoxy group, as defined herein, appended to the parent molecular moiety through an alkylene group, as defined herein. Representative examples of alkoxyalkyl include, but are not limited to, tert-butoxymethyl, 2-ethoxyethyl, 2-methoxyethyl, methoxymethyl, 2-methoxy-2-methylpropyl, and 3-methoxypropyl.

The term "alkoxycarbonyl" as used herein, means an alkoxy group, as defined herein, appended to the parent molecular moiety through a carbonyl group, as defined herein. Representative examples of alkoxycarbonyl include, but are not limited to, methoxycarbonyl, ethoxycarbonyl, and tert-butoxycarbonyl.

The term "alkoxycarbonylalkyl" as used herein, means an alkoxycarbonyl group, as defined herein, appended to the parent molecular moiety through an alkylene group, as defined herein. Representative examples of alkoxycarbonylalkyl include, but are not limited to, ethoxycarbonylmethyl, 3-methoxycarbonylpropyl, 4-ethoxycarbonylbutyl, and 2-tert-butoxycarbonylethyl.

The term "alkyl" as used herein, means a straight or branched saturated hydrocarbon chain containing from 1 to 10 carbon atoms. The term "$C_{1-6}$ alkyl" or "$C_1$-$C_6$ alkyl" means a straight or branched saturated hydrocarbon chain containing from I to 6 carbon atoms. The term "$C_1$-$C_4$ alkyl" means a straight or branched saturated hydrocarbon chain containing from I to 4 carbon atoms. Representative examples ofalkyl include, but are not limited to, methyl, ethyl, n-propyl, iso-propyl, n-butyl, sec-butyl, iso-butyl, tert-butyl, n-pentyl, isopentyl, neopentyl, n-hexyl, 1-methyl butyl, 1-methylethyl, 2-methylbutyl, 3-methylbutyl, 1-methylpropyl, I-ethylpropyl, 2-methylpropyl, 1,1-dimethylethyl, 1,1-dimethylpropyl, 1,2-dimethylpropyl, 2,2-dimethylpropyl, 1,2,2-trimethylpropyl, 3-methylhexyl, 2,2-dimethylpentyl, 2,3-dimethylpentyl, n-heptyl, n-octyl, n-nonyl, and n-decyl.

The term "alkylcarbonyl" as used herein, means an alkyl group, as defined herein, appended to the parent molecular moiety through a carbonyl group, as defined herein. Representative examples of alkyl carbonyl include, but are not limited to, acetyl, 1-oxopropyl, 2,2-dimethyl-1-oxopropyl, 1-oxobutyl, and 1-oxopentyl.

The term "alkylcarbonylalkyl" as used herein, means an alkylcarbonyl group, as defined herein, appended to the parent molecular moiety through an alkylene group, as defined herein. Representative examples of alkylcarbonylalkyl include, but are not limited to, 2-oxopropyl, 3,3-dimethyl-2-oxopropyl, 3-oxobutyl, and 3-oxopentyl.

The tenn "alkylcarbonyloxy" as used herein, means an alkylcarbonyl group, as defined herein, appended to the parent molecular moiety through an oxygen atom. Representative examples of alkylcarbonyloxy include, but are not limited to, acetyloxy, ethylcarbonyloxy, and tert-butylcarbonyloxy.

The term "alkylene" means a divalent group derived from a straight or branched saturated hydrocarbon chain of I to 10 carbon atoms. The term "$C_1$-$C_6$ alkylene" means a divalent group derived from a straight or branched saturated hydrocarbon chain of I to 6 carbon atoms. The term "$C_1$-$C_3$ alkylene" means a divalent group derived from a straight or branched saturated hydrocarbon chain of I to 3 carbon atoms. The term "$C_1$-$C_2$ alkylene" means a divalent group derived from a straight or branched saturated hydrocarbon chain of 1 to 2 carbon atoms. Representative examples of alkylene include, but are not limited to, $-CH_2-$, $-CH(CH_3)-$, $-CH(C_2H_5)$, $-CH(CH(CH_3)(C_2H_5))-$, $-C(H)(CH_3)CH_2CH_2-$, $-C(CH_3)_2-$, $-CH_2CH_2-$, $-CH_2CH_2CH_2-$, $-CH_2CH_2CH_2CH_2-$, and $-CH_2CH(CH_3)CH_2-$.

The tenn "alkylsulfinyl" as used herein, means an alkyl group, as defined herein, appended to the parent molecular moiety through a sulfinyl group, as defined herein. Representative examples of alkylsulfinyl include, but are not limited to, methylsulfinyl and ethylsulfinyl.

The tenn "alkylsulfinylalkyl" as used herein, means an alkylsulfinyl group, as defined herein, appended to the parent molecular moiety through an alkylene group, as defined herein. Representative examples of alkylsulfinylalkyl include, but are not limited to, methylsulfinylmethyl and ethylsulfinylmethyl.

The term "alkynyl" as used herein, means a straight or branched chain hydrocarbon group containing from 2 to 10 carbon atoms and containing at least one carbon-carbon triple bond. Representative examples of alkynyl include,

but are not limited, to acetylenyl, 1-propynyl, 2-propynyl, 1-propyl-pent-3-ynyl, 3-butynyl, 2-pentynyl, and 1-butynyl.

The tenn "aryl," as used herein, means phenyl, a bicyclic aryl, or a tricyclic aryl. The bicyclic aryl is naphthyl, or a phenyl fused to a monocyclic cycloalkyl, or a phenyl fused to a monocyclic cycloalkenyl. Representative examples of the bicyclic aryl include, but are not limited to, dihydroindenyl, indenyl, naphthyl, dihydronaphthalenyl, and tetrahydronaphthalenyl. The tricyclic aryl is exemplified by a bicyclic aryl fused to a monocyclic cycloalkyl, or a bicyclic aryl fused to a monocyclic cycloalkenyl, or a bicyclic aryl fused to a phenyl. Representative examples of tricyclic aryl ring include, but are not limited to, anthracene, phenanthrene, dihydroanthracenyl, fluorenyl, 1,2-dihydroacenaphthylenyl (including 1,2-dihydroacenaphthylen-5-yl), and tetrahydrophenanthrenyl. The phenyl, bicyclic and tricyclic aryls are attached to the parent molecular moiety through any carbon atom contained within the phenyl, bicyclic, and tricyclic aryls respectively.

The term "arylalkyl" as used herein, means an aryl group, as defined herein, appended to the parent molecular moiety through an alkylene group, as defined herein. Representative examples of arylalkyl include, but are not limited to, benzyl, 2-phenylethyl, 1-methyl-3-phenylpropyl, 2-methyl-1-phenylbutyl, 1-phenylpropyl, 3-phenylpropyl, and 2-naphth-2-ylethyl.

The tenn "azido" as used herein, means a $-N_3$ group.

The tenn "azidoalkyl" as used herein, means an azido group, as defined herein, appended to the parent molecular moiety through an alkylene group, as defined herein.

The term "carbonyl" as used herein, means a -C(O)- group.

The tenn "carboxy" as used herein, means a $-CO_2H$ group.

The tenn "carboxyalkyl" as used herein, means a carboxy group, as defined herein, appended to the parent molecular moiety through an alkylene group, as defined herein. Representative examples of carboxyalkyl include, but are not limited to, carboxymethyl, 2-carboxyethyl, and 3-carboxypropyl.

The tenn "cyano" as used herein, means a -CN group.

The term "cyanoalkyl" as used herein, means a cyano group, as defined herein, appended to the parent molecular moiety through an alkylene group, as defined herein. Representative examples of cyanoalkyl include, but are not limited to, cyanomethyl, 2-cyanoethyl, and 3-cyanopropyl.

The tenn "cycloalkenyl" as used herein, means a monocyclic or bicyclic ring system containing zero heteroatoms in the ring. The monocyclic cycloalkenyl has three, four, five, six, seven or eight carbon atoms and zero heteroatoms. The three or four-membered ring systems have one double bond, the five-or six-membered ring systems have one or two double bonds, and the seven- or eight-membered ring systems have one, two or three double bonds. Representative examples of monocyclic ring systems include, but are not limited to, 2-cyclohexen-1-yl, 3-cyclohexen-1-yl, 2,4-cyclohexadien-1-yl and 3-cyclopenten-1-yl. Bicyclic ring systems are exemplified by a monocyclic cycloalkenyl ring fused to a monocyclic cycloalkyl ring, or a monocyclic cycloalkenyl ring fused to a monocyclic cycloalkenyl ring. Representative examples of bicyclic ring systems include, but are not limited to 3a, 4, 5, 6, 7, 7a-hexahydro-1H-indenyl, 4,5,6,7-tetrahydro-3aH-indene, and octahydronaphthalenyl. The monocyclic or the bicyclic cycloalkenyl ring can be appended to the parent molecular moiety through any substitutable carbon atom within the monocyclic or the bicyclic cycloalkenyl.

The term "cycloalkenylalkyl" as used herein, means a cycloalkenyl group as defined herein, appended to the parent molecular moiety through an alkylene group, as defined herein.

The term "cycloalkyl" as used herein, means a monocyclic, or a bicyclic ring system, or a spirocyclic cycloalkyl. The monocyclic cycloalkyl is a carbocyclic ring system containing 3, 4, 5, 6, 7, or 8 carbon atoms, zero heteroatoms and zero double bonds. Examples of monocyclic ring systems include cyclopropyl, cyclobutyl, cyclopentyl, cyclohexyl, cycloheptyl, and cyclooctyl. Bicyclic ring systems are exemplified by a monocyclic cycloalkyl ring fused to a monocyclic cycloalkyl ring. Representative examples of bicyclic ring systems include, but are not limited to, bicyclo[4.1.0]heptane, bicyclo[6.1.0]nonane, octahydroindene, and decahydronaphthalene. Spirocyclic cycloalkyl is exemplified by a monocyclic cycloalkyl ring wherein two of the substituents on the same carbon atom of the ring, together with said carbon atom, fonn a 4-, 5-, or 6-membered monocyclic cycloalkyl. An example of a spirocyclic cycloalkyl is spiro[2.5]octane. The monocyclic, bicyclic and spirocyclic cycloalkyl groups of the present invention can be appended to the parent molecular moiety through any substitutable carbon atom of the groups. The monocyclic and bicyclic cycloalkyl groups of the present invention, with the exception of $R^4$, may contain one or two alkylene bridges of 1, 2, 3, or 4 carbon atoms, each of which linking two non adjacent carbon atoms of the group. Examples of such a bridged system include, but are not limited to, adamantane (tricyclo[3.3.1.1$^{3,7}$]decane) and bicyclo[2.2.1]heptane.

The term "cycloalkylalkyl" as used herein, means a cycloalkyl group, as defined herein, appended to the parent molecular moiety through an alkylene group, as defined herein. Representative examples of cycloalkylalkyl include, but are not limited to, cyclopentylmethyl, cyclohexylmethyl, cyclopropylmethyl, and 1-cyclopropylethyl.

The tenn "formyl" as used herein, means a -C(O)H group.

The term "formylalkyl" as used herein, means a formyl group, as defined herein, appended to the parent molecular moiety through an alkylene group, as defined herein. Representative examples of formylalkyl include, but are not

limited to, formylmethy) and 2-formylethyl.

The tenn "halo" or "halogen" as used herein, means -Cl, -Br, -I or -F.

The tenn "haloalkoxy" as used herein, means an alkoxy group, as defined herein, in which one, two, three, four, five or six hydrogen atoms are replaced by halogen. The term "$C_1$-$C_4$ haloalkoxy" as used herein, means a $C_1$-$C_4$ alkoxy group, as defined herein, in which one, two, three, four, five or six hydrogen atoms are replaced by halogen. Representative examples of haloalkoxy include, but are not limited to, trifluoromethoxy, difluoromethoxy, 2,2,2-trifluoroethoxy, and 2,2-difluoroethoxy.

The tenn "haloalkyl" as used herein, means an alkyl group, as defined herein, in which one, two, three, four, five, six, or seven hydrogen atoms are replaced by halogen. The tenn "$C_1$-$C_4$ haloalkyl" as used herein, means a $C_1$-$C_4$ alkyl group, as defined herein, in which one, two, three, four, five, or six hydrogen atoms are replaced by halogen. Representative examples of haloalkyl include, but are not limited to, chloromethyl, 2-fluoroethyl, 2,2-difluoroethyl, trifluoromethyl, 2,2,2-trifluoroethyl, difluoromethyl, pentafluoroethyl, 2-chloro-3-fluoropentyl, and 2-iodoethyl.

The tenn "heteroaryl," as used herein, means a monocyclic heteroaryl or a bicyclic heteroaryl. The monocyclic heteroaryl is a 5 or 6 membered ring containing at least one heteroatom independently selected from the group consisting of O, N, and S. The 5-membered ring contains two double bonds, and one, two, three, or four heteroatoms as ring atoms. The 6-membered ring contains three double bonds, and one, two, three or four heteroatoms as ring atoms. Representative examples of monocyclic heteroaryl include, but are not limited to, furanyl (including furan-3-yl, furan-2-yl), imidazolyl, isoxazolyl (including isoxazol-5-yl), isothiazolyl, oxadiazolyl (including 1,2,4-oxadiazol-3-yl), oxazolyl (including 1,3-oxazol-2-yl), pyridinyl (including pyridin-2-yl, pyridin-3-yl, pyridine-4-yl), pyridazinyl, pyrimidinyl, pyrazinyl, pyrazolyl (including pyrazol-5-yl), pyrrolyl, tetrazolyl, thiadiazolyl, thiazolyl, thienyl (including thien-2-yl), triazolyl, and triazinyl. The bicyclic heteroaryl is exemplified by a monocyclic heteroaryl fused to phenyl, or a monocyclic heteroaryl fused to a monocyclic cycloalkyl, or a monocyclic heteroaryl fused to a monocyclic cycloalkenyl, or a monocyclic heteroaryl fused to a monocyclic heteroaryl, or a monocyclic heteroaryl fused to a monocyclic heterocycle. Representative examples of bicyclic heteroaryl include, but are not limited to, benzofuranyl (including benzofuran-5-yl), benzoxadiazolyl, 1,3-benzothiazolyl, benzimidazolyl, benzodioxolyl, benzothienyl, chromenyl, cinnolinyl, furopyridinyl, indolyl, indazolyl, isoindolyl, isoquinolinyl (including isoquinolin-5-yl), naphthyridinyl, oxazolopyridine, quinolinyl (including quinolin-4-yl, quinolin-5-yl, quinolin-8-yl), thienopyridinyl and thienopyridinyl. The monocyclic and the bicyclic heteroaryl groups are connected to the parent molecular moiety through any substitutable carbon atom or any substitutable nitrogen atom contained within the groups. The nitrogen and sulfur heteroatoms of the heteroaryl rings may optionally be oxidized, and are contemplated within the scope of the invention.

The term "heteroarylalkyl" as used herein, means a heteroaryl group as defined herein, appended to the parent molecular moiety through an alkylene group, as defined herein. An example of heteroarylalkyl is 3-thienylpropyl.

The term "heterocycle" or "heterocyclic" as used herein, refers to a monocyclic, bicyclic, tricyclic, or a spirocyclic ring system, containing at least one heteroatom. The monocyclic heterocycle is a 3, 4, 5, 6, 7, or 8- membered ring containing at least one heteroatom independently selected from the group consisting of O, N, and S. The 3 or 4 membered ring contains 1 heteroatom selected from the group consisting of O, N and S, and optionally one double bond. The 5-membered ring contains zero or one double bond, and one, two or three heteroatoms in the ring selected from the group consisting of O, N and S. The 6, 7, or 8-membered ring contains zero, one, or two double bonds, and one, two, or three heteroatoms in the ring selected from the group consisting of O, N and S. Representative examples of monocyclic heterocycle include, but are not limited to, azetidinyl (including azetidin-3-yl), azepanyl, aziridinyl, diazepanyl, 1,3-dioxanyl, 1,4-dioxanyl, 1,3-dioxolanyl, 4,5-dihydroisoxazol-S-yl, dihydropyranyl (including 3,4-dihydro-2H-pyran-6-yl), 1,3-dithiolanyl, 1,3-dithianyl, imidazolinyl, imidazolidinyl, isothiazolinyl, isothiazolidinyl, isoxazolinyl, isoxazolidinyl, morpholinyl, oxadiazolinyl, oxadiazolidinyl, oxazolinyl, oxazolidinyl, oxetanyl, piperazinyl, piperidinyl, pyranyl, pyrazolinyl, pyrazolidinyl, pyrrolinyl, pyrrolidinyl (pyrrolidin-3-yl), tetrahydrofuranyl (including tetrahydrofuran-2-yl tetrahydrofuran-3-yl), tetrahydropyranyl (including tetrahydro-2H-pyran-4-yl), tetrahydropyridinyl (including 1,2,3,6-tetrahydropyridin-4-yl), tetrahydrothienyl, thiadiazolinyl, thiadiazolidinyl, thiazolinyl, thiazolidinyl, thiomorpholinyl, 1,1-dioxidothiomorpholinyl (thiomorpholine sulfone), thiopyranyl, and trithianyl. The bicyclic heterocycle of the present invention is exemplified by a monocyclic heterocycle fused to a phenyl group, or a monocyclic heterocycle fused to a monocyclic cycloalkyl group, or a monocyclic heterocycle fused to a monocyclic cycloalkenyl group, or a monocyclic heterocycle fused to a monocyclic heterocycle group. Representative examples of bicyclic heterocycle include, but are not limited to, 1,3-benzodioxolyl (including 1,3-benzodioxol-4-yl), 1,3-benzodithiolyl, 2,3-dihydro-1,4-benzodioxinyl, dihydrobenzofuranyl (including 2,3-dihydro-1-benzofuran-7-yl), 2,3-dihydro-1-benzothienyl, 2,3-dihydro-1H-indolyl, and 1,2,3,4-tetrahydroquinolinyl. Spirocyclic heterocycle means a 4, 5-, 6-, 7-, or 8-membered monocyclic heterocycle ring wherein two of the substituents on the same carbon atom form a 4-, 5-, or 6-membered monocyclic cycloalkyl, wherein the cycloalkyl is optionally substituted with 1, 2, 3, 4, or 5 alkyl groups. One example of a spiroheterocycle is 5-oxaspiro[3,4]octane. The tricyclic heterocycle is a bicyclic heterocycle fused to a phenyl, or a bicyclic heterocycle fused to a monocyclic cycloalkyl, or a bicyclic heterocycle

fused to a monocyclic cycloalkenyl, or a bicyclic heterocycle fused to a monocyclic heterocycle. Representative examples of tricyclic heterocycle include, but are not limited to, 2,3,4,4a,9,9a-hexahydro-1H-carbazolyl, 5a,6,7,8,9,9a-hexahydrodibenzo[b,d]furanyl, and 5a,6,7,8,9,9a-hexahydrodibenzo[b,d]thienyl. The monocyclic, bicyclic, tricyclic, and spirocyclic heterocycle groups, unless otherwise noted, are connected to the parent molecular moiety through any substitutable carbon atom or any substitutable nitrogen atom contained within the group. The nitrogen and sulfur heteroatoms in the heterocycle rings may optionally be oxidized and the nitrogen atoms may optionally be quarternized.

The term "heterocyclealkyl" as used herein, means a heterocycle group as defined herein, appended to the parent molecular moiety through an alkylene group, as defined herein. An example includes, but is not limited to, tetrahydropyranmethyl (including tetrahydro-2H-pyran-4-ylmethyl).

The term "hydroxy" as used herein, means an -OH group.

The term "hydroxyalkyl" as used herein, means at least one hydroxy group, as defined herein, appended to the parent molecular moiety through an alkylene group, as defined herein. Representative examples of hydroxyalkyl include, but are not limited to, hydroxymethyl, 2-hydroxyethyl, 3-hydroxypropyl, 3-hydroxy-3-methylbutyl, 2,3-dihydroxypentyl, and 2-ethyl-4-hydroxyheptyl.

The term "hydroxy-protecting group" or "O-protecting group" means a substituent which protects hydroxy groups against undesirable reactions during synthetic procedures. Examples of hydroxy-protecting groups include, but are not limited to, substituted methyl ethers, for example, methoxymethyl, benzyloxymethyl, 2-methoxyethoxymethyl, 2-(trimethylsilyl)-ethoxymethyl, benzyl, and triphenylmethyl; tetrahydropyranyl ethers; substituted ethyl ethers, for example, 2,2,2-trichloroethyl and t-butyl; silyl ethers, for example, trimethylsilyl, t-butyldimethylsilyl and t-butyldiphenylsilyl; cyclic acetals and ketals, for example, methylene acetal, acetonide and benzylidene acetal; cyclic ortho esters, for example, methoxymethylene; cyclic carbonates; and cyclic boronates. Commonly used hydroxy-protecting groups are disclosed in T.W. Greene and P.G.M. Wuts, Protective Groups in Organic Synthesis, 3rd edition, John Wiley & Sons, New York (1999).

The term "nitrogen protecting group" as used herein, means those groups intended to protect an amino group against undesirable reactions during synthetic procedures. Preferred nitrogen protecting groups are acetyl, benzoyl, benzyl, benzyloxycarbonyl (Cbz), formyl, phenylsulfonyl, tert-butoxycarbonyl (Boc), tert-butylacetyl, trifluoroacetyl, and triphenylmethyl (trityl).

The term "oxo" as used herein, means a =O moiety.

The term "sulfinyl" as used herein, means a -S(O)- group.

The term "tautomer" as used herein means a proton shift from one atom of a compound to another atom of the same compound wherein two or more structurally distinct compounds are in equilibrium with each other.

b. Compounds

Compounds of the invention have the formula (I) as described above.

Particular values of variable groups in compounds of formula (I) are as follows. Such values may be used where appropriate with any of the other values, definitions, claims or embodiments defined hereinbefore or hereinafter. In compounds of formula (I), $R_1$ is A, or A-alkylene- wherein A is as disclosed in the Summary. Embodiments of the present invention include compounds wherein $R_1$ is A or A-alkylene- , each ring A is optionally fused with a monocyclic ring selected from the group consisting of benzo, cycloalkyl, cycloalkenyl, heterocycle, and heteroaryl, and each Ring A is optionally substituted as described in the Summary. Some examples of Ring A are those that are represented by formula (i), (ii), (iii), (iv), (v), (vi), (vii), and (viia) wherein each ring is independently unsubstituted or substituted as described in the Summary. Certain examples of the optional substituents of Ring A include, but are not limited to, alkyl such as $C_{1-6}$ alkyl (for example, methyl, ethyl), haloalkyl (e.g. trifluoromethyl), and oxo.

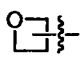
(i)

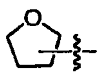
(ii)

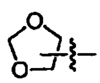
(iii)

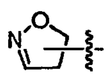
(iv)

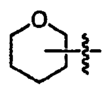
(v)

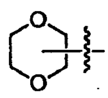
(vi)

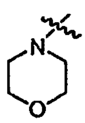
(vii)

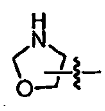
(viia)

Examples of compounds of the invention include, but are not limited to, those wherein $R_1$ is oxetan-2-ylmethyl, oxetan-3-ylmethyl, tetrahydrofuran-2-ylmethyl, tetrahydrofuran-3-ylmethyl tetrahydro-2H-pyran-2-ylmethyl, tetrahydro-2H-pyran-3-ylmethyl, tetrahydro-2H-pyran-4-ylmethyl, 2-tetrahydro-2H-pyran-4-ylethyl, 1,3-dioxolan-2-ylmethyl, 2-1,3-dioxolan-2-ylethyl, 1,3-dioxolan-4-ylmethyl, 4,5-dihydroisoxazol-5-ylmethyl, 1,4-dioxan-2-ylmethyl, 2-morpholin-4-ylethyl, tetrahydro-2H-pyran-4-yl, and 1,3-oxazolidin-4-ylmethyl, wherein each of the oxetan-2-yl, oxetan-3-yl, tetrahydrofuran-2-yl, tetrahydrofuran-3-yl, tetrahydro-2H-pyran-2-yl, tetrahydro-2H-pyran-3-yl, tetrahydro-2H-pyran-4-yl, 1,3-dioxolan-2-yl, 1,3-dioxolan-4-yl, 4,5-dihydroisoxazol-5-yl, 1,4-dioxan-2-yl, morpholin-4-yl, and 1,3-oxazolidin-4-yl, is each independently unsubstituted or substituted with 1, 2, 3, or 4 substituents selected from the group consisting of alkyl (for example, methyl, ethyl), haloalkyl (e.g. trifluoromethyl), and oxo.

Yet other examples of compounds of formula (I) include those wherein $R_1$ is A-alkylene- and A is furanyl, oxazolyl, isoxazolyl, or oxadiazolyl, each of which is optionally substituted as described in the Summary. For example $R_1$ is furanylmethyl, oxazolylmethyl, isoxazolylmethyl, or oxadiazolylmethyl, wherein each of the furanyl, oxazolyl, isoxazolyl and oxadiazolyl is optionally substituted with the group consisting of alkyl (e.g.methyl, ethyl), halo, and haloalkyl (e.g. trifluoromethyl).

The alkylene moiety of A-alkylene-, for example, is $C_1$-$C_6$ alkylene. Further examples of the alkylene moiety of A-alkylene- is $C_1$-$C_3$ alkylene. Yet further examples of the alkylene moiety of A-alkylene- is $C_1$-$C_2$ alkylene.

$R_2$ is hydrogen, alkoxy, alkoxyalkyl, alkoxycarbonyl, alkoxycarbonylalkyl, alkyl, alkyl-$S(O)_2$-, aryl, arylalkyl, arylalkenyl, azidoalkyl, cyano, cycloalkyl, halo, haloalkyl, heteroaryl, heterocycle, $-(CR_{21}R_{22})_m$-OH, $R_aR_bN$-, $R_aR_bN$-alkyl-, $R_cR_dNC(O)$-, or $R_8$-$R_7$-; wherein $R_{21}$, $R_{22}$, m, $R_a$, $R_b$, $R_c$, $R_d$, $R_7$, and $R_8$, and the optional substituents of the aryl, cycloalkyl, heteroaryl and heterocycle moieties are as described in the Summary. Certain examples of compounds of formula (I) include those wherein $R_2$ is hydrogen, alkoxycarbonyl, alkoxycarbonylalkyl, alkyl (for example, methyl, ethyl, propyl, tert-butyl), aryl (for example, optionally substituted phenyl), halo, haloalkyl (e.g. trifluoromethyl), or $-(CR_{21}R_{22})_m$-OH; wherein $R_{21}$, $R_{22}$, and m, and the optional substituents of the aryl moiety are as disclosed in the Summary. For example, the optional substituents of the aryl moiety are selected from the group consisting of alkyl and halo. Included, but not limited to, are compounds of formula (I) in which $R_{21}$ and $R_{22}$ are the same or different, and are each independently hydrogen, methyl, or ethyl, and m is 1. Embodiments of the present invention include compounds in which $R_2$ is hydrogen or alkyl (for example, methyl, ethyl, propyl, tert-butyl).

$R_3$ is hydrogen, alkoxy, alkoxyalkyl, alkyl, alkylcarbonyl, alkyl-$S(O)_2$-, aryl, arylalkyl, arylalkenyl, cyano, cycloalkyl, halo, haloalkyl, heteroaryl, heterocycle, $-(CR_{31}R_{32})_m$-OH, $R_aR_bN$-, $R_aR_bN$-alkyl-, or $R_8$-$R_7$-; wherein $R_{31}$, $R_{32}$, m, $R_a$, $R_b$, $R_7$, and $R_8$, and the optional substituents of the aryl, cycloalkyl, heteroaryl and heterocycle moieties are as disclosed in the Summary. Examples of compounds of formula (I) include, but are not limited to, those wherein $R_3$ is hydrogen, alkyl (for example, methyl, ethyl, n-propyl, tert-butyl), alkylcarbonyl (e.g. acetyl), aryl (for example, optionally substituted phenyl), cycloalkyl (for example, cyclopropyl, cyclohexyl, each of which is optionally substituted), halo, haloalkyl (e.g. trifluoromethyl), heterocycle (for example, morpholinyl), or $-(CR_{31}R_{32})_m$-OH, wherein $R_{31}$, $R_{32}$, and m are as disclosed in the Summary. The optional substituents ofaryl, cycloalkyl, and heterocycle moieties are as disclosed in the Summary, for example, the optional substituents are selected from the group consisting of alkyl (e.g. methyl), haloalkyl (e.g. trifluoromethyl), and halo. Non limiting examples of $R_{31}$, and $R_{32}$ ($R_{31}$ and $R_{32}$ can be the same or different) are alkyl (for example, methyl) or haloalkyl (for example, 2-iodoethyl, trifluoromethyl). m, for example, is 1. Embodiments of the present invention include compounds in which $R_3$ is alkyl (for example, methyl, ethyl, n-propyl, or tert-butyl) or $-(CR_{31}R_{32})_m$-OH. Other examples include those wherein $R_3$ is $-(CR_{31}R_{32})_m$-OH, wherein m is 1, and $R_{31}$ and $R_{32}$ are alkyl (such as, but not limited to, methyl) or haloalkyl (such as, but not limited to, trifluoromethyl).

In another embodiment, $R_2$ and $R_3$, together with the carbon atoms to which they are attached, form a 4-, 5-, 6-, or 7-membered monocyclic ring as described in the Summary. Embodiments of the present invention include compounds of formula (I) wherein $R_2$ and $R_3$, together with the carbon atoms to which they are attached, form a monocyclic ring as described in the Summary, containing zero heteroatoms in said monocyclic ring. Formulae (viii), (ix), (ixa), (xi), (xii), (xiii), and (xiv), each of which is optionally substituted as described in the Summary, represent some of these rings formed by $R_2$, $R_3$, and the carbon atoms to which they are attached,

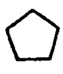 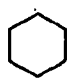 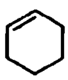 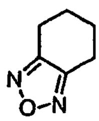 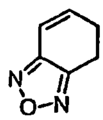 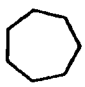

(viii) (ix) (ixa) (x) (xa) (xi)

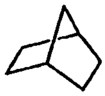 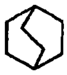 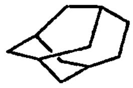

(xii) (xiii) (xiv)

Yet other compounds of the present invention include those wherein $R_2$ and $R_3$, together with the carbon atoms to which they are attached, form a 4-, 5-, 6-, or 7-membered monocyclic ring optionally fused to benzo or oxadiazole, said monocyclic ring contains zero or one additional double bond, zero oxygen atom and zero nitrogen atom as ring atoms; and two non-adjacent atoms of said monocyclic ring are linked by an alkenylene bridge of 2, 3, or 4 carbon atoms, or linked by an alkylene bridge of 1, 2, 3, or 4 carbon atoms. Examples include, but are not limited to, formulae (xii), (xiii) and (xiv), each of which is optionally substituted.

Further examples of compounds of formula (I) include, but are not limited to, those wherein $R_2$ and $R_3$, together with the carbon atoms to which they are attached, form a monocyclic ring containing zero additional double bond, zero oxygen and zero nitrogen atom, such as those represented by (viii), (ix) or (xi).

Yet other compounds of the present invention include those wherein $R_2$ and $R_3$, together with the carbon atoms to which they are attached, form a 4-, 5-, 6-, or 7-membered monocyclic ring optionally fused to benzo or oxadiazole, said monocyclic ring contains zero or one additional double bond, and one oxygen atom and zero or one nitrogen atom as ring atoms; and two non-adjacent atoms of said monocyclic ring are optionally linked by an alkenylene bridge of 2, 3, or 4 carbon atoms, or optionally linked by an alkylene bridge of 1, 2, 3, or 4 carbon atoms. Examples include, but are not limited to, formula (xv) - (xxix), particularly, (xv), (xviii) and ((xxix).

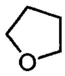 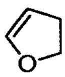 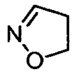 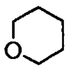 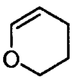 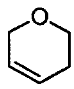 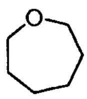 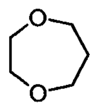

(xv) (xvi) (xvii) (xviii) (xix) (xx) (xxi) (xxii)

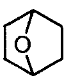 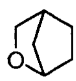 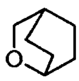 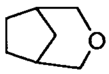 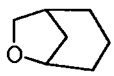 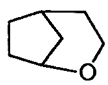 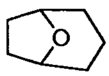

(xxiii) (xxiv) (xxv) (xxvi) (xxvii) (xxviii) (xxix)

Each ring formed by $R_1$, $R_3$, and the carbon atoms to which they are attached is independently unsubstituted or substituted as described in the Summary, for example, these rings can be independently unsubstituted or substituted with 1, 2, 3, 4, 5 or 6 substituents independently selected from alkyl such as $C_{1-6}$ alkyl (for example, methyl), hydroxy, and oxo. Such rings are optionally fused with benzo or oxadiazole. Examples of such an optionally substituted fused ring are represented by formula (x) and (xa).

$R_4$ is alkyl, alkynyl, cycloalkyl, cycloalkenyl, aryl, heteroaryl, heterocycle, cycloalkylalkyl, cycloalkenylalkyl, arylalkyl, heteroarylalkyl, heterocyclealkyl, or $R_{10}$-$L_2$-$R_9$-; wherein the alkyl group is optionally substituted with one substituent selected from the group consisting of alkoxy, alkoxycarbonyl, carboxy, halo, -OH, and $R_eR_fN$-; $R_9$, $R_{10}$, $R_e$, and $R_f$ and the aryl moiety, cycloalkyl moiety, cycloalkenyl moiety, heteroaryl moiety and the heterocycle moiety are independently unsubstituted or substituted as described in the Summary. In one embodiment, $R_4$ is optionally substituted aryl. In another embodiment, $R_4$ is phenyl or naphthyl, each of which is optionally substituted. Yet other examples of compounds of formula (I) are those wherein $R_4$ is arylalkyl wherein the aryl moiety is optionally substituted. Other examples of compounds of formula (I) are those wherein $R_4$ is heteroaryl (for example, quinolinyl, isoquinolinyl, benzofuranyl, thienyl, pyrazolyl, pyridinyl), heterocycle (tetrahydropyranyl, dihydropyranyl, 2,3-dihydrobenzofuranyl, 1,3-benzodioxolyl), heteroarylalkyl (e.g. 3-thien-2-ylpropyl), or heterocyclealkyl (for example, tetrahydro-2H-pyranylmethyl), each of the heterocycl and heteroaryl moieties is optionally substituted as described in the Summary. Yet other examples are those wherein $R_4$ is alkynyl. Other examples are those wherein $R_4$ is alkyl optionally substituted with $R_eR_fN$-, -OH, or alkoxycarbonyl, wherein $R_e$ and $R_f$ are as disclosed in the Summary. Further examples are those wherein $R_4$ is cycloalkyl (e.g. cyclopropyl, cyclobutyl, cyclopentyl, cyclohexyl, cycloheptyl, spiro[2.5]octane) or cycloalkylalkyl (e.g. cyclopentylmethyl, cyclohexylmethyl) wherein the cycloalkyl moiety is optionally substituted.

Yet other examples of compounds of formula (I) are those wherein $R_4$ is $R_{10}$-$L_2$-$R_9$- wherein $R_9$ is aryl (for example, phenyl, naphthyl) or heteroaryl (e.g. pyrazolyl), $L_2$ is alkylene (e.g. $CH_2$, $CH_2CH_2$), and $R_{10}$ is heterocycle (e.g. tetrahydrofuranyl, azetidinyl, pyrrolidinyl, morpholinyl, thiomorpholinyl, piperazinyl) or aryl (e.g. phenyl); in other embod-

iments, $R_9$ is aryl (for example, phenyl, naphthyl) or heteroaryl (e.g. pyrazolyl), $L_2$ is O, and $R_{10}$ is cycloalkyl (cyclopropyl), cycloalkylalkyl (e.g. cyclopropylmethyl, cyclopentylmethyl), or heterocycle (e.g. tetrahydrofuranyl, azetidinyl, pyrrolidinyl, morpholinyl, thiomorpholinyl, piperazinyl). Each $R_9$ and $R_{10}$ is optionally substituted as described in the Summary and embodiments herein.

Examples of the optional substituents of $R_4$ and $R_{10}$ include, but are not limited to, alkyl (for example, methyl, ethyl), alkylcarbonyl (for example, acetyl), alkylcarbonylalkyl (e.g. acetylmethyl), alkoxy (for example, methoxy, ethoxy, isopropoxy, tert-butyoxy), alkoxyalkoxy (for example, 2-methoxyethoxy, 3-methyl-3-methoxybutoxy), alkoxycarbonyl (e.g. methoxycarbonyl), cyano, formyl, halogen (for example, Cl, Br, I, F), haloalkoxy (for example, trifluoromethoxy, difluoromethoxy, 2,2,2-trifluoroethoxy), hydroxy, haloalkyl (e.g. trifluoromethyl), alkyl-$S(O)_2$-$(CR_{41}R_{42})_p$=$C(R_{41})$-, alkyl-$S(O)_2$-$(CR_{41}R_{42})_p$-, alkyl-S- (e.g. $CH_3$-S-), alkenyl (e.g. vinyl), oxo, -$C(R_{41})$=N-$O(R_{42})$, -$(CR_{41}R_{42})_p$-$C(R_{41})$=N-$O(R_{42})$, =N-O(alkyl), $NZ_1Z_2$-$(CR_{41}R_{42})_p$-O-, -O-$(CR_{41}R_{42})_p$-$G_1$, $G_1$, -$NZ_1Z_2$, and -$(CR_{41}R_{42})_p$-$NZ_1Z_2$, wherein $Z_1$, $Z_2$, $R_{41}$, $R_{42}$, p, and $G_1$ are as described in the Summary. For example, $R_{41}$ and $R_{42}$ are the same or different, and at each occurrence, are each independently hydrogen or alkyl (e.g. methyl, ethyl). p, for example, is 1, 2, or 3. $Z_1$ and $Z_2$, are the same or different, and at each occurrence, are each, for example, independently hydrogen, alkyl (e.g. methyl, ethyl, tert-butyl), cyanoalkyl (e.g. cyanomethyl), or alkoxyalkyl (e.g. 2-methoxyethyl). $G_1$, for example, is morpholinyl, 1,2,3,6-tetrahydropyridinyl, piperidinyl, or thiomorpholinyl, each of which is optionally substituted as described in the Summary, for example, optionally substituted with 1, 2, or 3 substituents selected from alkyl (e.g. methyl), oxo, alkoxycarbonyl (e.g. tert-butoxycarbonyl). Examples of the optional substituents of $R_9$ include, but are not limited to, alkyl (e.g. methyl, tert-butyl, ethyl), haloalkyl (e.g. trifluoromethyl), and halogen.

$L_1$ is a single bond or -NRg- wherein $R_g$ is hydrogen or alkyl. Certain compounds of the present invention include those wherein $L_1$ is a single bond. Yet others are those wherein $L_1$ is -$NR_g$- wherein $R_g$ is hydrogen or alkyl. Other examples include those wherein $L_1$ is -NH-.

It is appreciated that the present invention contemplates compounds of formula (I) with combinations of the above embodiments, including particular, more particular and preferred embodiments.

Accordingly, one aspect of the invention relates to a group of compounds of formula (I), or pharmaceutically acceptable salts thereof, wherein $R_1$ is A or A-alkylene-, $R_2$ is hydrogen, alkoxycarbonyl, alkoxycarbonylalkyl, alkyl (for example, methyl, ethyl, propyl, tert-butyl), aryl (for example, optionally substituted phenyl), halo, haloalkyl (e.g. trifluoromethyl), or -$(CR_{21}R_{22})_m$-OH; $R_3$ is hydrogen, alkyl (for example, methyl, ethyl, n-propyl, tert-butyl), alkylcarbonyl (e.g. acetyl), aryl (for example, optionally substituted phenyl), cycloalkyl (for example, cyclopropyl, cyclohexyl, each of which is optionally substituted), halo, haloalkyl (e.g. trifluoromethyl), heterocycle (for example, morpholinyl), or -$(CR_{31}R_{31})_m$-OH; A is a 4-, 5-, 6-, 7-, 8-, or 9-membered monocyclic heterocycle containing zero or one double bond, one or two oxygen, and zero or one nitrogen as ring atoms; two non-adjacent atoms of each A can be optionally linked by an alkenylene bridge of 2, 3, or 4 carbon atoms, or optionally linked by an alkylene bridge of 1, 2, 3, or 4 carbon atoms; each ring A is optionally fused with a monocyclic ring selected from the group consisting of benzo, cycloalkyl, cycloalkenyl, heterocycle, and heteroaryl; and each A is independently unsubstituted or substituted with 1, 2, 3, 4, 5, or 6 substituents independently selected from the group consisting of oxo, alkyl, halo, -OH, -O(alkyl), and haloalkyl; and $L_1$, $R_4$, $R_{21}$, $R_{22}$, $R_{31}$, $R_{32}$, m; and the optional substituents of the aryl, cycloalkyl, and heterocycle moieties are as disclosed in the Summary and the Detailed Description. Ring A, for example, is formula (i), (ii), (iii), (iv), (v), (vi), (vii), or (viia), wherein each ring is independently unsubstituted or substituted as described in the Summary and Detailed Description. The alkylene moiety of A-alkylene-, for example, is $C_1$-$C_6$ alkylene. Further examples of the alkylene moiety of A-alkylene- is $C_1$-$C_3$ alkylene. Yet fiuther examples of the alkylene moiety of A-alkylene- is $C_1$-$C_2$ alkylene. Further examples of $R_1$ as A or A-alkylene include, but are not limited to, oxetan-2-ylmethyl, oxetan-3-ylmethyl, tetrahydrofuran-2-ylmethyl, tetrahydrofuran-3-ylmethyl, tetrahydro-2H-pyran-2-ylmethyl, tetrahydro-2H-pyran-3-ylmethyl, tetrahydro-2H-pyran-4-ylmethyl, 2-tetrahydro-2H-pyran-4-ylethyl, 1,3-dioxolan-2-ylmethyl, 2-1,3-dioxolan-2-ylethyl, 1,3-dioxolan-4-ylmethyl, 4,5-dihydroisoxazol-5-ylmethyl, 1,4-dioxan-2-ylmethyl, 2 -morpholin-4-ylethyl, tetrahydro-2H-pyran-4-yl, and 1,3-oxazolidin-4-ylmethyl, wherein each of the oxetan-2-yl, oxetan-3-yl, tetrahydrofuran-2-yl, tetrahydrofuran-3-yl, tetrahydro-2H-pyran-2-yl, tetrahydro-2H-pyran-3-yl, tetrahydro-2H-pyran-4-yl, 1,3-dioxolan-2-yl, 1,3-dioxolan-4-yl, 4,5-dihydroisoxazol-5-yl, 1,4-dioxan-2-yl, morpholin-4-yl, and 1,3-oxazolidin-4-yl, is each independently unsubstituted or substituted with 1, 2, 3, or 4 substituents selected from the group consisting of alkyl (for example, methyl, ethyl), haloalkyl (e.g. trifluoromethyl), and oxo.

Another aspect of the invention relates to a group of compounds of formula (I), or pharmaceutically acceptable salts thereof, wherein $R_1$ is A-alkylene- and ring A is furanyl, oxazolyl, isoxazolyl, or oxadiazolyl, each of which is optionally substituted as described in the Summary and in Detailed Description; $R_2$ is hydrogen, alkoxycarbonyl, alkoxycarbonylalkyl, alkyl (for example, methyl, ethyl, propyl, tert-butyl), aryl (for example, optionally substituted phenyl), halo, haloalkyl (e.g. trifluoromethyl), or -$(CR_{21}R_{22})_m$-OH; $R_3$ is hydrogen, alkyl (for example, methyl, ethyl, n-propyl, tert-butyl), alkylcarbonyl (e.g. acetyl), aryl (for example, optionally substituted phenyl), cycloalkyl (for example, cyclopropyl, cyclohexyl, each of which is optionally substituted), halo, haloalkyl (e.g. trifluoromethyl), heterocycle (for example, morpholinyl), or -$(CR_{31}R_{32})_m$-OH and $L_1$, $R_4$, $R_{21}$, $R_{22}$, $R_{31}$, $R_{32}$, m, and the optional substituents of the aryl, cycloalkyl, and heterocycle moieties are as disclosed in the Summary and Detailed Description. For example $R_1$ is furanylmethyl, oxazolylmethyl,

isoxazolylmethyl, or oxadiazolylmethyl, wherein each of the furanyl, oxazolyl, isoxazolyl and oxadiazolyl is optionally substituted with the group consisting of alkyl (e.g.methyl, ethyl), halo, and haloalkyl (e.g. trifluoromethyl).

For the preceding two groups of compounds of formula (I), $R_{21}$, and $R_{22}$ are the same or different, and are each independently hydrogen, methyl, or ethyl. m, for example, is 1. $R_{31}$ and $R_{32}$ ($R_{31}$ and $R_{32}$ can be the same or different) are, for example, alkyl (for example, methyl) or haloalkyl (for example, 2-iodoethyl, trifluoromethyl).

In one embodiment, $R_3$ is alkyl (for example, methyl, ethyl, n-propyl, or tert-butyl) or $-(CR_{31}R_{32})_m$-OH, and $R_2$ is hydrogen or alkyl (for example, methyl, ethyl, propyl, tert-butyl); wherein $R_{31}$, $R_{31}$ and m are as described in the Summary and in embodiments described hereina bove. Examples include those wherein $R_2$ is hydrogen or alkyl (for example, methyl), and $R_3$ is $-(CR_{31}R_{32})_m$-OH wherein m is 1, and $R_{31}$ and $R_{32}$ are alkyl (such as, but not limited to, methyl) or haloalkyl (such as, but not limited to, trifluoromethyl). Other examples include those wherein $R_2$ is hydrogen or alkyl (for example, methyl), and $R_3$ is alkyl (for example, tert-butyl). Yet other examples include those wherein $R_2$ is hydrogen or alkyl (for example, methyl), and $R_3$ is $-(CR_{31}R_{22})_m$OH, wherein $R_{31}$ and $R_{32}$ are alkyl (for example, methyl), and m is 1.

Another aspect of the invention provides a group of compounds of formula (I), or pharmaceutically acceptable salts thereof, wherein $R_1$ is A or A-alkylene-, $R_2$ and $R_3$, together with the carbon atoms to which they are attached form a monocyclic ring, and A, $R_4$, $L_1$, and said monocyclic ring are as described in the Summary. Some examples of ring A are as described herein above. Certain examples of the monocyclic ring formed by $R_2$, $R_3$, and the carbon atoms to which they are attached are represented by formulae (viii), (ix), (ixa), and (xi)-(xxix), each of which is optionally substituted with substituents as described in the Summary and in the Detailed Description, and each of which is optionally fused with benzo or oxadiazole. One example of such fused ring is represented by formula (x) or (xa). Examples of the optional substituents on the rings formed by $R_2$, $R_3$, and the carbon atoms to which they are attached include, but are not limited to, alkyl such as $C_{1-6}$ alkyl, hydroxy, and oxo.

Within each group of compounds of formula (I) as described in the preceding paragraphs, $L_1$ and $R_4$ have values as described in the Summary and the Detailed Description.

Thus, within each group of the compounds as described in the preceding paragraphs, examples of a subgroup include those wherein $R_4$ is aryl (e.g. phenyl, naphthyl, 1,2-dihydroacenaphthylenyl), heteroaryl (for example, quinolinyl, isoquinolinyl, benzofuranyl, thienyl, pyrazolyl, pyridinyl), cycloalkyl (e.g. cyclopropyl, cyclobutyl, cyclopentyl, cyclohexyl, cycloheptyl, spiro[2.5]octane), heterocycle (tetrahydropyranyl, dihydropyranyl, 2,3-dihydrobenzofuranyl, 1,3-benzodioxolyl), arylalkyl, heteroarylalkyl (e.g. 3-thien-2-ylpropyl), heterocyclealkyl (for example, tetrahydro-2H-pyranylmethyl), cycloalkylalkyl (e.g. cyclopentylmethyl, cyclohexylmethyl), $R_9$-$L_2$-$R_{10}$, alkynyl, or alkyl, wherein the alkyl group is optionally substituted with $R_eR_fN$-, -OH, or alkoxycarbonyl, wherein $R_9$, $L_2$, $R_{10}$, $R_e$ and $R_f$, and the optional substituents of the aryl, heteroaryl, cycloalkyl, and heterocycle moieties are as described in the Summary and in the Detailed Description.

Examples of another subgroup of compounds of formula (I) include those wherein $L_1$ is a bond, and $R_4$ is aryl (e.g. phenyl, naphthyl, 1,2-dihydroacenaphthylenyl), heteroaryl (for example, quinolinyl, isoquinolinyl, benzofuranyl, thienyl, pyrazolyl, pyridinyl), cycloalkyl (e.g. cyclopropyl, cyclobutyl, cyclopentyl, cyclohexyl, cycloheptyl, spiro[2.5]octane), heterocycle (tetrahydropyranyl, dihydropyranyl, 2,3-dihydrobenzofuranyl, 1,3-benzodioxolyl), arylalkyl, heteroarylalkyl (e.g. 3-thien-2-ylpropyl), heterocyclealkyl (for example, tetrahydro-2H-pyranylmethyl), cycloalkylalkyl (e.g. cyclopentylmethyl, cyclohexylmethyl), $R_9$-$L_2$-$R_{10}$, alkynyl, or alkyl, wherein the alkyl group is optionally substituted with $R_eR_fN$-, -OH, or alkoxycarbonyl, wherein $R_9$, $L_2$, $R_{10}$, $R_e$ and $R_f$, and the optional substituents of the aryl, heteroaryl, cycloalkyl, and heterocycle moieties are as described in the Summary and in the Detailed Description.

Other examples of a subgroup include those wherein $L_1$ is a bond, and $R_4$ is optionally substituted phenyl or $R_{10}$-$L_2$-$R_9$-, wherein $R_9$ is aryl (for example, phenyl, naphthyl) or heteroaryl (e.g. pyrazolyl), $L_2$ is alkylene (e.g. $CH_1$, $CH_1CH_2$), and $R_{10}$ is as described in the Summary. For example, $R_{10}$ is heterocycle (e.g. tetrahydrofuranyl, azetidinyl, pyrrolidinyl, morpholinyl, thiomorpholinyl, piperazinyl) or aryl (e.g. phenyl); wherein the phenyl, aryl, heteroaryl, and heterocycle moieties of $R_4$, $R_9$, and $R_{10}$ are each optionally substituted as described in the Summary and the Detailed Description.

Other examples of a subgroup include those wherein $L_1$ is a bond, and $R_4$ is optionally substituted phenyl or $R_{10}$-$L_2$-$R_9$-, wherein $R_9$ is aryl (for example, phenyl, naphthyl) or heteroaryl (e.g. pyrazolyl), $L_2$ is O, and $R_{10}$ is as described in the Summary. for example, $R_{10}$ is cycloalkyl (cyclopropyl), cycloalkylalkyl (e.g. cyclopropylmethyl, cyclopentylmethyl), or heterocycle (e.g. tetrahydrofuranyl, azetidinyl, pyrrolidinyl, morpholinyl, thiomorpholinyl, piperazinyl); wherein the phenyl, aryl, heteroaryl, cycloalkyl, and heterocycle moieties of $R_4$, $R_9$, and $R_{10}$ are each optionally substituted as described in the Summary and the Detailed Description.

In certain embodiments of the subgroups described above, $R_9$ is optionally aryl (for example, phenyl), optionally further substituted as described in the Summary and in the Detailed Description.

Examples of another subgroup of compounds of formula (I) include those wherein $L_1$ is $NR_g$, and $R_4$ is arylalkyl, cycloalkyl, cycloalkylalkyl, or alkyl, wherein the alkyl group is optionally substituted with $R_eR_fN$-, -OH, or alkoxycarbonyl, and $R_e$, $R_g$, and, $R_f$, and the optional substituents of the aryl and cycloalkyl moieties are as described in the Summary and in the Detailed Description.

Compounds of the present invention may exist as stereoisomers wherein, asymmetric or chiral centers are present. These stereoisomers are "R" or "S" depending on the configuration of substituents around the chiral carbon atom. The terms "R" and "S" used herein are configurations as defined in IUPAC 1974 Recommendations for Section E, Fundamental Stereochemistry, Pure Appl. Chem., 1976, 45: 13-30. The present invention contemplates various stereoisomers (including enantiomers and diastereomers) and mixtures of various ratio thereof and are included within the scope of this invention. Individual stereoisomers of compounds of the present invention may be prepared synthetically from commercially available starting materials containing asymmetric or chiral centers or by preparation of racemic mixtures followed by resolution well known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary or (2) direct separation of the mixture of optical enantiomers on chiral chromatographic columns.

Geometric isomers can exist in the present compounds. The invention contemplates the various geometric isomers and mixtures thereofresulting from the disposition of substituents around a carbon-carbon double bond, a carbon-nitrogen double bond, a cycloalkyl group, or a heterocycle group. Substituents around a carbon-carbon double bond or a carbon-nitrogen double bond are designated as being of Z or E configuration; and substituents around a cycloalkyl or heterocycle are designated as being of cis or trans configuration.

Within the present invention it is to be understood that compounds disclosed herein may exhibit the phenomenon of tautomerism.

The formulae drawings within this specification can represent only one of the possible tautomeric or stereoisomeric forms. It is to be understood that the invention encompasses any tautomeric or stereoisomeric form, and mixtures thereof, and is not to be limited merely to any one tautomeric or stereoisomeric form utilized within the naming of the compounds or formulae drawings.

c. Biological Data

(i) *In Vitro* Methods-- Human $CB_2$ and $CB_1$ Radioligand Binding Assays:

The $CB_1$ and $CB_2$ radioligand binding assays described herein are utilized to determine the selectivity of compounds of the present invention for binding to $CB_2$ relative to $CB_1$ receptors.

HEK293 cells stably expressing human $CB_2$ receptors were grown until a confluent monolayer was formed. Briefly, the cells were harvested and homogenized in TE buffer (50 mM Tris-HCl, 1 mM $MgCl_2$, and 1 mM EDTA) using a polytron for 2 X 10 second bursts in the presence of protease inhibitors, followed by centrifugation at 45,000Xg for 20 minutes. The final membrane pellet was re-homogenized in storage buffer (50 mM Tris-HCl, 1 mM $MgCl_2$, and 1 mM EDTA and 10% sucrose) and frozen at -78 °C until used. Saturation binding reactions were initiated by the addition of membrane preparation (protein concentration of 5 μg/ well for human $CB_2$) into wells of a deep well plate containing ([$^3$H]CP-55,940 (120 Ci/mmol, a nonselective CB agonist commercially available from Tocris) in assay buffer (50 mM Tris, 2.5 mM EDTA, 5 mM $MgCl_2$, and 0.5 mg/mL fatty acid free BSA, pH 7.4). After 90 min incubation at 30 °C, binding reaction was terminated by the addition of 300 μl/well of cold assay buffer followed by rapid vacuum filtration through a UniFilter-96 GF/C filter plates (pre-soaked in I mg/mL BSA for 2 hours). The bound activity was counted in a TopCount using Microseint-20. Saturation experiments were conducted with twelve concentrations of [$^3$H]CP-55,940 ranging from 0.01 to 8 nM. Competition experiments were conducted with 0.5 nM [$^3$H]CP-55,940 and five concentrations of displacing ligands selected from the range of 0.01 nM to 10 μM. The addition of 10 μM unlabeled CP-55,940 (Tocris, Ellisville, MO) was used to assess nonspecific binding.

HEK293 cells stably expressing rat $CB_2$ receptors were grown until a confluent monolayer was formed. Briefly, the cells were harvested and homogenized in TE buffer (50 mM Tris-HCl, 1 mM $MgCl_1$, and 1 mM EDTA) using a polytron for 2 X 10 second bursts in the presence of protease inhibitors, followed by centrifugation at 45,000Xg for 20 minutes. The final membrane pellet was re-homogenized in storage buffer (50 mM Tris-HCl, 1 mM $MgCl_2$, and 1 mM EDTA and 10% sucrose) and frozen at -78 °C until used Saturation binding reactions were initiated by the addition of membrane preparation (protein concentration of 20 μg/ well for rat $CB_2$) into wells of a deep well plate containing [$^3$H]CP-55,940 (120 Ci/mmol, a nonselective CB agonist commercially available from Tocris) in assay buffer (50 mM Tris, 2.5 mM EDTA, 5 mM $MgCl_2$, and 0.5 mg/mL fatty acid free BSA, pH 7.4). After 45 min incubation at 30°C., binding reaction was terminated by the addition of 300 μl/well of cold assay buffer followed by rapid vacuum filtration through a UniFilter-96 GF/C filter plates (pre-soaked in 1 mg/mL BSA for 2 hours). The bound activity was counted in a TopCount using Microseint-20. Saturation experiments were conducted with twelve concentrations of [$^3$H]CP-55,940 ranging from 0.01 to 8 nM. Competition experiments were conducted with 0.5 nM [$^3$H]CP-55,940 and five concentrations of displacing ligands selected from the range of 0.01 nM to 10 μM. The addition of 10 μM unlabeled CP-55,940 (Tocris, Ellisville, MO) was used to assess nonspecific binding.

Representative compounds of the present invention bound to $CB_2$ receptors with a $K_i$ of less than about 1,000

nM, preferably less than 400 nM, more preferably less than 200 nM and, most preferably lower than 100 nM.

HEK293 human $CB_1$ membranes were purchased from Perkin Elmer. Binding was initiated by the addition of membranes (8-12 μg per well) into wells (Scienceware 96-well DeepWell plate, VWR, West Chester, PA) containing [$^3$H]CP-55,940 (120 Ci/mmol, Perkin Elmer, Boston, MA) and a sufficient volume of assay buffer (50 mM Tris, 2.5 mM EDTA, 5 mM $MgCl_2$, and 0.5 mg/mL fatty acid free BSA, pH 7.4) to bring the total volume to 250 μL. After incubation (30 °C for 90 minutes), binding was terminated by the addition of 300 uL per well of cold assay buffer and rapid vacuum filtration (FilterMate Cell Harvester, Perkin Elmer, Boston, MA) through a UniFilter-96 GF/C filter plate (Perkin Elmer, Boston, MA) (pre-soaked in 0.3% PEI at least 3 hours), followed by five washes with cold assay buffer. The bound activity was counted in the TopCount using Microseint-20 (both from Perkin Elmer, Boston, MA). Competition experiments were conducted with I nM [$^3$H]CP-55,940 and five concentrations (1 nM to 10 μM) of displacing ligands. The addition of 10 μM unlabeled CP-55,940 (Tocris, Ellisville, MO) was used to assess nonspecific binding. Representative compounds of the present invention bound to $CB_1$ receptors with $K_i$ of about 10 fold to about 1000 fold or more higher than that for $CB_2$ receptors. These results show that the compounds of the present invention preferably bind to $CB_2$ receptors, therefore are selective ligands for the $CB_2$ receptor. (ii) *In Vivo* Data:

Animals

Adult male Sprague-Dawley rats (250-300 g body weight, Charles River Laboratories, Portage, MI) are used. Animal handling and experimental protocols are approved by the Institutional Animal Care and Use Committee (IACUC) at Abbott Laboratories. For all surgical procedures, animals are maintained under isoflurane anesthesia (4-5% to induce, 1-3% to maintain), and the incision sites are sterilized using a 10% povidone-iodine solution prior to and after surgeries.

Incisional Model of Postoperative Pain

A skin incision model of postoperative pain can be produced using the procedures described in Brennan et al., 1996, Pain, 64, 493. All rats are anesthetized with isoflurane delivered via a nose cone. Right hind paw incision is performed following sterilization procedures. The plantar aspect of the left hind paw is placed through a hole in a sterile plastic drape. A 1-cm longitudinal incision is made through the skin and fascia of the plantar aspect of the hind paw, starting 0.5 cm from the proximal edge of the heel and extending towards the toes, the plantar muscle is elevated and incised longitudinally leaving the muscle origin and insertion points intact. The skin is then closed with two mattress sutures (5-0 nylon). After surgery, animals are then allowed to recover for 2 hours, at which time tactile allodynia is assessed as described below. To evaluate the anti-nociceptive effects, animals are i.p. administered vehicle or test compound 90 minutes following skin incision and tactile allodynia is assessed 30 minutes after compound administration.

Tactile allodynia can be measured using calibrated von Frey filaments (Stoelting, Wood Dale, IL) as described in Chaplan, S.R., F.W. Bach, J.W. Pogrel, J.M. Chung and T.L. Yaksh, 1994, Quantitative Assessment of Tactile Allodynia in the Rat Paw, J. Neurosci. Methods, 53, 55. Rats are placed into inverted individual plastic cage (20 x 12.5 x 20 cm) on top of a suspended wire mesh grid, and are acclimated to the test chambers for 20 minutes. The von Frey filaments are applied perpendicularly from underneath the cage through openings in the wire mesh floor directly to an area within 1-3 mm (immediately adjacent) of the incision, and then held in this position for approximately 8 seconds with enough force to cause a slight bend in the filament. Positive responses include an abrupt withdrawal of the hind paw from the stimulus, or flinching behavior immediately following removal of the stimulus. A 50% withdrawal threshold can be determined using an up-down procedure (Dixon, W.J., 1980, Efficient Analysis of Experimental Observations, Ann. Rev. Pharmacol. Toxicol., 20, 441).

Representative compounds of the present invention showed efficacy at less than about 300 micromoles/kg in the skin incision model of postoperative pain. In a more preferred embodiment, compounds described herein showed efficacy at less than about 100 micromoles/kg in the skin incision model of postoperative pain.

Complete Freund's Adjuvant (CFA) Model of Inflammatory Pain

Chronic inflammatory thermal hyperalgesia was induced by injection of 150 μL of a 50% solution of CFA in phosphate buffered saline (PBS) into the plantar surface of the right hind paw in rats; control animals received only PBS treatment. Thermal hyperalgesia was assessed 48 hours post CFA injection. Thermal hyperalgesia was determined using a commercially available thermal paw stimulator (University Anesthesiology Research and Development Group (UARDG), University of California, San Diego, CA) described by Hargreaves et al. (Hargreaves, et. al., 1988, Pain 32, 77). Rats were placed into individual plastic cubicles mounted on a glass surface maintained at 30°C, and allowed a 20 min habituation period. A thermal stimulus, in the form of radiant heat emitted from a focused projection bulb, was then applied to the plantar surface of each hind paw. The stimulus current was maintained at 4.50 ± 0.05 amp, and the maximum time of exposure was set at 20.48 sec to limit possible tissue damage. The elapsed time until a brisk withdrawal

of the hind paw from the thermal stimulus was recorded automatically using photodiode motion sensors. The right and left hind paw of each rat was tested in three sequential trials at approximately 5-minute intervals. Paw withdrawal latency (PWL) was calculated as the mean of the two shortest latencies.

Representative compounds of the present invention showed a statistically significant change in paw withdrawal latency versus a saline vehicle at less than about 300 micromoles/kg in the Complete Freund's Adjuvant (CFA) model of inflammatory pain.

<u>Final Nerve Ligation Model of Neuropathic Pain</u>

A model of spinal nerve ligation-induced (SNL model) neuropathic pain was produced using the procedure originally described in Kim, S.H. and J.M. Chung, 1992, An experimental model for peripheral neuropathy produced by segmental spinal nerve ligation in the rat, Pain, 50, 355. The left L5 and L6 spinal nerves of the rat were isolated adjacent to the vertebral column and tightly ligated with a 5-0 silk suture distal to the DRG, and care was taken to avoid injury of the L4 spinal nerve. Sham rats underwent the same procedure, but without nerve ligation. All animals were allowed to recover for at least one week and not more than three weeks prior to assessment of tactile allodynia.

Tactile allodynia was measured using calibrated von Frey filaments (Stoelting, Wood Dale, IL) as described in Chaplan, S.R., F.W. Bach, J.W. Porgrel, J.M. Chung and T.L. Yaksh, 1994, Quantitative assessment of tactile allodynia in the rat paw, J. Neurosci. Methods, 53, 55. Rats were placed into inverted individual plastic containers (20 x 12.5 x 20 cm) on top of a suspended wire mesh grid, and acclimated to the test chambers for 20 minutes. The von Frey filaments were presented perpendicularly to the plantar surface of the selected hind paw, and then held in this position for approximately 8 sec with enough force to cause a slight bend in the filament. Positive responses included an abrupt withdrawal of the hind paw from the stimulus, or flinching behavior immediately following removal of the stimulus. A 50% withdrawal threshold was determined using an up-down procedure as described in Dixon, W.J., 1980, Efficient analysis of experimental observations, Ann. Rev. Pharmacol. Toxicol., 20, 441). Only rats with a baseline threshold score of less that 4.25 g were used in this study, and animals demonstrating motor deficit were excluded. Tactile allodynia thresholds were also assessed in several control groups, including naive, sham-operated, and saline infused animals a well as in the contralateral paws of nerve-injured rats.

Representative compounds of the present invention showed efficacy at less than about 300 micromoles/kg in the spinal nerve ligation model of neuropathic pain. In a more preferred embodiment, representative compounds of the present invention showed efficacy at less than about 100 micromoles/kg in the spinal nerve ligation model of neuropathic pain.

<u>Capsaicin-induced secondary mechanical hypersensitivity</u>:

Rats were allowed to acclimate to the study room for I hour. They were then briefly restrained, and capsaicin was administered at 10 μg in 10 μL of vehicle (10% ethanol and 2-hydroxypropyl cyclodextrin) by intraplantar injection into the center of the right hind paw. Secondary mechanical hyperalgesia was measured at the heel away from the site of injection at 180 min following capsaicin (Joshi et al 2006, Neuroscience 143, 587-596). Compounds are injected (i.p.) 30 min before testing (150 min post-capsaicin).

Tactile allodynia was measured as described above.

Representative compounds of the present invention showed a statistically significant change in paw withdrawal latency versus a saline vehicle at less than about 300 micromoles/kg. In a more preferred embodiment, representative compounds of the present invention showed efficacy at less than about 50 micromoles/kg.

<u>MIA-induced Knee Joint Osteoarthritic Pain Model</u>

Unilateral knee joint osteoarthritis was induced in the rats by a single intra-articular (i.a.) injection of sodium monoiodoacetate (MIA, 3 mg in 0.05 mL sterile isotonic saline) into the right knee joint cavity under light isoflurane anesthesia using a 26G needle. The dose of the MIA (3mg/i.a.injection) was selected based on results obtained from preliminary studies wherein an optimal pain behavior was observed at this dose. Pain behavioral assessment of hind limb grip force were conducted by recording the maximum compressive force exerted on the hind limb strain gauge setup, in a commercially available grip force measurement system (Columbus Instruments, Columbus, OH). The grip force data was converted to a maximum hindlimb cumulative compressive force (CFmax) (gram force) / kg body weight for each animal. The analgesic effects of test compounds were determined 20 days following the i.a. injection of MIA. The vehicle control group for each compound being tested was assigned 0% whereas the age matched naïve group was assigned as being 100% (normal). The % effects for each dose group was then expressed as % return to normalcy compared to the naïve group. Compounds were administered either orally (p.o.) or intraperitoneally (i.p.). The assessment of the analgesic effects of test compounds is typically made anytime between about 1 hour and about 5 hours following

oral administration. The assessment of the analgesic effects of test compounds is typically made anytime between about 0.5 hour and about 2 hours following i.p. administration. Selection of the preferred time points for measuring the analgesic effects of test compounds was based upon consideration of the individual phannacokinetic characteristics of test compounds in the rat. Time points that were known or expected to provide higher plasma concentrations of test compounds were preferred over those that were known or expected to provide lower concentrations. The assessment of the analgesic effects of test compounds can be made following a single dose or following repeated dosing of test compounds wherein the frequency of dosing is I to 2 times daily. The duration of such repeated daily dosing may last for any time greater than or equal to one day. A typical duration of repeated daily dosing is about 5 days to about 12 days.

Representative compounds of the present invention showed a statistically significant change in hind limb grip force strength versus a saline vehicle at less than about 300 micromoles/kg in the MIA model of osteoarthritic pain following a single dose. In a more preferred embodiment, representative compounds of the present invention showed a statistically significant change in hind liinb grip force strength versus a saline vehicle at less than about 50 micromoles/kg in the MIA model - of osteoarthritic pain following a single dose.

d. Methods of Using the Compounds

The data contained herein above demonstrates that compounds of the present invention bind to the $CB_2$ receptor. Certain compounds of the present invention were shown to have an analgesic effect in two types of animal pain models relating to neuropathic and nociceptive pain.

One embodiment of the present invention provides compounds for use in a method for treating pain (for example, neuropathic pain or nociceptive pain) in a mammal (including human) in need of such treatment. The method comprises administering to the mammal a therapeutically effective amount of any of the compounds described herein, or a phannaceutically acceptable salt thereof, alone or in combination with one or more phannaceutically acceptable carriers. The method further comprises administration of compounds of the invention as a single dose. The method also comprises repeated or chronic administration of compounds of the invention over a period of days, weeks, months, or longer. In certain embodiments, the method comprises administering to the mammal a therapeutically effective amount of any of the compounds as described herein, or a pharmaceutically acceptable salt thereof, in combination with one or more nonsteroidal anti-inflammatory drug (NSAID), or with other analgesic agent (e.g. acetaminophen), or a combination thereof.

Another embodiment of the present invention provides compounds for use in a method for treating a disorder selected from the group consisting of inflammatory disorders, immune disorders, neurological disorders, cancers of the immune system, respiratory disorders, and cardiovascular disorders in a mammal (including human) in need of such treatment. The method comprises administering to the mammal a therapeutically effective amount of any of the compounds described herein or a phannaceutically acceptable salt thereof, alone or in combination with one or more pharmaceutically acceptable carriers.

Yet another embodiment of the present invention relates to compounds for use in a method for providing neuroprotection in a mammal (including human) in need of such treatment. The method comprises administering to the mammal a therapeutically effective amount of any of the compounds described herein or a pharmaceutically acceptable salt thereof, alone or in combination with one or more pharmaceutically acceptable carriers.

Another embodiment of the present invention provides compounds for use in a method of increasing the therapeutic effectiveness or potency of compounds of the invention by repeated or chronic administration over a period of days, weeks, or months.

In addition to the data contained herein, several lines of evidence support the assertion that $CB_2$ receptors play a role in analgesia. HU-308 is one of the first highly selective $CB_2$ agonists identified that elicits an antinociceptive response in the rat formalin model of persistent pain (Hanus, L., et al., Proc. Nat. Acad. Sci., 1999, 96, 14228-14233). The $CB_2$-selective cannabiniod ligand AM-1241 exhibits robust analgesic efficacy in animal models of acute thermal pain (Malan, T. P., et al., Pain, 2001, 93, 239-245; Ibrahim, M. M., et al., Proc. Nat. Acad. Sci., 2005, 102(8), 3093-3098), persistent pain (Hohmann, A. G., et al., J. Pharmacol. Exp. Ther., 2004, 308, 446-453), inflammatory pain (Nackley, A. G., et al., Neuroscience, 2003, 119, 747-757; Quartilho, A. et al., Anesthesiology, 2003, 99, 955-60), and neuropathic pain (Ibrahim, M. M., et al., Proc. Nat. Acad. Sci., 2003, 100, 10529-10533). The $CB_2$-selective partial agonist GW405833, also known as L768242, is efficacious in rodent models of neuropathic, incisional, and both chronic and acute inflammatory pain (Valenzano, K. J., et al., Neuropharmacology, 2005, 48, 658-672 and Clayton, N., et al., Pain, 2002, 96, 253-260).

The potential exists for $CB_2$ modulators to have opioid sparing effects. A synergy between the analgesic effects of morphine and the nonselective CB agonist $\Delta^9$-THG has been documented (Cichewicz, D. L., Life Sci. 2004, 74, 1317-1324). Therefore, $CB_2$ ligands have additive or synergistic analgesic effects when used in combination with lower doses of morphine or other opioids, providing a strategy for reducing adverse opioid events, such as tolerance, constipation, and respiratory depression, without sacrificing analgesic efficacy.

$CB_2$ receptors are present in tissues and cell types associated with immune functions and $CB_2$ receptor mRNA

is expressed by human B cells, natural killer cells, monocytes, neutrophils, and T cells (Galiegue et al., Eur. J. Biochem., 1995, 232, 54-61). Studies with $CB_2$ knockout mice have suggested a role for $CB_2$ receptors in modulating the immune system (Buckley, N. E., et al., Eur. J. Pharmacol. 2000, 396, 141-149). Although immune cell development and differentiation are similar in knockout and wild type animals, the immunosuppressive effects of $\Delta^9$-THC are absent in the $CB_2$ receptor knockout mice, providing evidence for the involvement of $CB_2$ receptors in immunomodulation. As such, selective $CB_2$ modulators may be useful for the treatment of autoimmune diseases including but not limited to multiple sclerosis, rheumatoid arthritis, systemic lupus, myasthenia gravis, type I diabetes, irritable bowel syndrome, psoriasis, psoriatic arthritis, and hepatitis; and immune related disorders including but not limited to tissue rejection in organ transplants, gluten-sensitive enteropathy (Celiac disease), asthma, chronic obstructive pulmonary disease, emphysema, bronchitis, acute respiratory distress syndrome, allergies, allergic rhinitis, dennatitis, and Sjogren's syndrome.

Microglial cells are considered to be the immune cells of the central nervous system (CNS) where they regulate the initiation and progression of immune responses. $CB_2$ receptor expression on microglia is dependent upon inflammatory state with higher levels of $CB_2$ found in primed, proliferating, and migrating microglia relative to resting or fully activated microglial (Carlisle, S. J., et al. Int. Immunopharmacol., 2002, 2, 69). Neuroinflammation induces many changes in microglia cell morphology and there is an upregulation of $CB_2$ receptors and other components of the endocannabinoid system.-Neuroinflammation occurs in several neurodegenerative diseases, and induction of microglial $CB_2$ receptors has been observed (Carrier, E. J., et al., Current Drug Targets - CNS & Neurological Disorders, 2005, 4, 657-665). Thus, $CB_2$ ligands may be clinically useful for the treatment of neuroinflammation.

Multiple sclerosis is common immune-mediated disease of the CNS in which the ability of neurons to conduct impulses becomes impaired through demyelination and axonal damage. The demyelination occurs as a consequence of chronic inflammation and ultimately leads to a broad range of clinical symptoms that fluctuate unpredictably and generally worsen with age. These include painful muscle spasms, tremor, ataxia, motor weakness, sphincter dysfunction, and difficulty speaking (Pertwee, R. G., Pharmacol. Ther. 2002, 95, 165-174). The $CB_2$ receptor is up-regulated on activated microglial cells during experimental autoimmune encephalomyelitis (EAE) (Maresz, K., et al., J. Neurochem. 2005, 95, 437-445). $CB_2$ receptor activation prevents the recruitment of inflammatory cells such as leukocytes into the CNS (Ni, X., et al., Multiple Sclerosis, 2004, 10, 158-164) and plays a protective role in experimental, progressive demyelination (Arevalo-Martin, A.; et al., J. Neurosci., 2003, 23(7), 2511-2516), which are critical features in the development of multiple sclerosis. Thus, $CB_2$ receptor modulators may provide a unique treatment for demyelinating pathologies.

Alzheimer's disease is a chronic neurodegenerative disorder accounting for the most common form of elderly dementia. Recent studies have revealed that $CB_2$ receptor expression is upregulated in neuritic plaque-associated microglia from brains of Alzheimer's disease patients (Benito, C., et al., J. Neurosci., 2003, 23(35), 11136-11141). *In vitro,* treatment with the $CB_2$ agonist JWH-133 abrogated □-amyloid-induced microglial activation and neurotoxicity, effects that can be blocked by the $CB_2$ antagonist SR 144528 (Ramirez, B. G., et al., J. Neurosci. 2005, 25(8), 1904-1913). $CB_2$ modulators may possess both anti-inflammatory and neuroprotective actions and thus have clinical utility in treating neuroinflammation and in providing neuroprotection associated with the development of Alzheimer's disease.

Increased levels of epithelial $CB_2$ receptor expression are observed in human inflammatory bowel disease tissue (Wright, K., et al., Gastroenterology, 2005, 129, 437-453). Activation of $CB_2$ receptors re-established normal gastrointestinal transit after endotoxic inflammation was induced in rats (Mathison, R., et al., Br. J. Pharmacol. 2004, 142, 1247-1254). $CB_2$ receptor activation in a human colonic epithelial cell line inhibited TNF-□-induced interleukin-8 (IL-8) release (Ihenetu, K. et al., Eur. J. Pharmacol. 2003, 458, 207-215). Chemokines released from the epithelium, such as the neutrophil chemoattractant IL-8, are upregulated in inflammatory bowel disease (Warhurst, A. C., et al., Gut, 1998, 42, 208-213). Thus, administration of $CB_2$ receptor modulators may represent a novel approach for the treatment of inflammation and disorders of the gastrointestinal tract including but not limited to inflammatory bowel disease, irritable bowel syndrome, secretory diarrhea, ulcerative colitis, Crohn's disease and gastroesophageal reflux disease (GERD).

Hepatic fibrosis occurs as a response to chronic liver injury and ultimately leads to cirrhosis, which is a major worldwide health issue due to the severe accompanying complications of portal hypertension, liver failure, and hepatocellular carcinoma (Lotersztajn, S., et al., Annu. Rev. Pharmacol. Toxicol., 2005, 45, 605-628). Although $CB_2$ receptors were not detectable in normal human liver, $CB_2$ receptors were expressed liver biopsy specimens from patients with cirrhosis. Activation of $CB_2$ receptors in cultured hepatic myofibroblasts produced potent antifibrogenic effects (Julien, B., et al., Gastroenterology, 2005, 128, 742-755). In addition, $CB_2$ knockout mice developed enhanced liver fibrosis after chronic administration of carbon tetrachloride relative to wild-type mice. Administration of $CB_2$ receptor modulators may represent a unique approach for the treatment of liver fibrosis.

Cough is a dominant and persistent symptom of many inflammatory lung diseases, including asthma, chronic obstructive pulmonary disease, viral infections, and pulmonary fibrosis (Patel, H. J., et al., Brit. J. Pharmacol., 2003, 140, 261-268). Recent studies have provided evidence for the existence of neuronal $CB_2$ receptors in the airways, and have demonstrated a role for $CB_2$ receptor activation in cough suppression (Patel, H. J., et al., Brit. J. Pharmacol., 2003, 140, 261-268 and Yoshihara, S., et al., Am. J. Respir. Crit. Care Med., 2004, 170, 941-946). Both exogenous and

endogenous cannabinoid ligands inhibit the activation of C-fibers via $CB_2$ receptors and reduce neurogenic inflammatory reactions in airway tissues (Yoshihara, S., et al., J. Phannacol. Sci. 2005, 98(1), 77-82; Yoshihara, S., et al., Allergy and Immunology, 2005, 138, 80-87). Thus, $CB_2$-selective modulators may have utility as antitussive agents for the treatment of pulmonary inflammation, chronic cough, and a variety of airway inflammatory diseases including but not limited to asthma, chronic obstructive pulmonary disease, and pulmonary fibrosis.

There is a substantial genetic contribution to bone mass density and the $CB_2$ receptor gene is associated with human osteoporosis (Karsak, M., et al., Human Molecular Genetics, 2005, 14(22), 3389-3396). Osteoclasts and osteoblasts are largely responsible for maintaining bone structure and function through a process called remodeling, which involves resorption and synthesis of bone (Boyle, W. J., et al., Nature, 2003, 423, 337-342). $CB_2$ receptor expression has been detected on osteoclasts and osteoblastic precursor cells, and administration of a $CB_2$ agonist in mice caused a dose-dependent increase in bone formation (Grotenhennen, F. and Muller-Vahl, K., Expert Opin. Phannacother., 2003, 4(12), 2367-2371). Cannabinoid inverse agonists, including the $CB_2$-selective inverse agonist SR 144528, have been shown to inhibit osteoclast activity and reverse ovariectomy-induced bone loss in mice, which is a model for post-menopausal osteoporosis (Ralston, S. H., et al., Nature Medicine, 2005, 11, 774-779). Thus, $CB_2$ modulators may be useful for the treatment and prevention of osteoporosis, osteoarthritis, and bone disorders.

Artherosclerosis is a chronic inflammatory disease and is a leading cause of heart disease and stroke. $CB_2$ receptors have been detected in both human and mouse atherosclerotic plaques. Administration of low doses of THC in apolipoprotein E knockout mice slowed the progression of atherosclerotic lesions, and these effects were inhibited by the $CB_2$-selective antagonist SR144528 (Steffens, S., et al., Nature, 2005, 434, 782-786). Thus, compounds with activity at the $CB_2$ receptor may be clinically useful for the treatment of atheroscelorsis.

$CB_2$ receptors are expressed on malignant cells of the immune system and targeting $CB_2$ receptors to induce apoptosis may constitute a novel approach to treating malignancies of the immune system. Selective $CB_2$ agonists induce regression of malignant gliomas (Sanchez, C., et al., Cancer Res., 2001, 61, 5784-5789), skin carcinomas (Casanova, M. L., et al., J. Clin. Invest., 2003, 111, 43-50), and lymphomas (McKallip, R. J., et al., Blood, 2002, 15(2), 637-634). Thus, $CB_2$ modulators may have utility as anticancer agents against tumors of immune origin.

Activation of $CB_2$ receptors has been demonstrated to protect the heart against the deleterious effects of ischemia and reperfusion (Lepicier, P., et al., Brit. J. Phann. 2003, 139, 805-815; Bouchard, J.-F., et al., Life Sci. 2003, 72, 1859-1870; Filippo, C. D., et al., J. Leukoc. Biol. 2004, 75, 453-459). Thus, $CB_2$ modulators may have utility for the treatment or prophylaxis of cardiovascular disease and the development of myocardial infarction.

Actual dosage levels of active ingredients in the pharmaceutical compositions of this invention can be varied so as to obtain an amount of the active compound(s) that is effective to achieve the desired therapeutic response for a particular patient, compositions and mode of administration. The selected dosage level will depend upon the activity of the particular compound, the route of administration, the duration of treatment, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved. In the treatment of certain medical conditions, repeated or chronic administration of compounds of the invention may be required to achieve the desired therapeutic response. "Repeated or chronic administration" refers to the administration of compounds of the invention daily (i.e., every day) or intermittently (i.e., not every day) over a period of days, weeks, months, or longer. In particular, the treatment of chronic painful conditions is anticipated to require such repeated or chronic administration of compounds of the invention. Compounds of the invention may become more effective upon repeated or chronic administration such that the therapeutically effective doses on repeated or chronic administration may be lower than the therapeutically effective dose from a single administration.

Compounds of the invention can also be administered as a pharmaceutical composition comprising the compounds of interest in combination with one or more pharmaceutically acceptable carriers. The phrase "therapeutically effective amount" of the compound of the invention means a sufficient amount of the compound to treat disorders, at a reasonable benefit/risk ratio applicable to any medical treatment. It will be understood, however, that the total daily usage of the compounds and compositions of the invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well-known in the medical arts. For example, it is well within the skill of the art to start doses of the compound at levels lower than required to achieve the desired therapeutic effect and to gradually increase the dosage until the desired effect is achieved.

Compounds of the invention may be administered alone, or in combination with one or more other compounds of the invention, or in combination (i.e. co-administered) with one or more additional pharmaceutical agents. For example, a compound the invention, or a pharmaceutically acceptable salt or solvate thereof, may be administered in combination

with acetaminophen, or with one or more nonsteroidal anti-inflammatory drug (NSAID) such as, but not limited to, aspirin, diclofenac, diflusinal, etodolac, fenbufen, fenoprofen, flufenisal, flurbiprofen, ibuprofen, indomethacin, ketoprofen, ketorolac, meclofenamic acid, mefenamic acid, meloxicam, nabumetone, naproxen, nimesulide, nitroflurbiprofen, olsalazine, oxaprozin, phenylbutazone, piroxicam, sulfasalazine, sulindac, tolmetin and zomepirac. In certain embodiments of the invention, the nonsteroidal anti-inflammatory drug (NSAID) is ibuprofen. Combination therapy includes administration of a single pharmaceutical dosage formulation containing one or more of the compounds of invention and one or more additional pharmaceutical agents, as well as administration of the compounds of the invention and each additional pharmaceutical agent, in its own separate pharmaceutical dosage formulation. For example, a compound of the invention and one or more additional pharmaceutical agents, may be administered to the patient together, in a single oral dosage composition having a fixed ratio of each active ingredient, such as a tablet or capsule; or each agent may be administered in separate oral dosage formulations.

Where separate dosage formulations are used, compounds of the invention and one or more additional pharmaceutical agents may be administered at essentially the same time (e.g., concurrently) or at separately staggered times (e.g., sequentially).

The total daily dose of the compounds of this invention administered to a human or other animal range from about 0.01 mg/kg body weight to about 100 mg/kg body weight. More preferable doses can be in the range of from about 0.03 mg/kg body weight to about 30 mg/kg body weight. If desired, the effective daily dose can be divided into multiple doses for purposes of administration. Consequently, single dose compositions may contain such amounts or submultiples thereof to make up the daily dose. It is understood that the effective daily dose may vary with the duration of the treatment.

## e. Pharmaceutical Compositions

The present invention further provides pharmaceutical compositions that comprise compounds of the present invention or a pharmaceutically acceptable salt or solvate thereof. The pharmaceutical compositions comprise compounds of the present invention that may be formulated together with one or more non-toxic pharmaceutically acceptable carriers.

Another aspect of the present invention is a pharmaceutical composition comprising a compound of formula (I), or a pharmaceuticallly acceptable salt thereof, and one or more pharmaceutically acceptable carriers, alone or in combination with one or more nonsteroidal anti-inflammatory drug (NSAID).

The pharmaceutical compositions of this invention can be administered to humans and other mammals orally, rectally, parenterally, intracisternally, intravaginally, intraperitoneally, topically (as by powders, ointments or drops), bucally or as an oral or nasal spray. The tenn "parenterally" as used herein, refers to modes of administration which include intravenous, intramuscular, intraperitoneal, intrasternal, subcutaneous and intraarticular injection and infusion.

The term "pharmaceutically acceptable carrier" as used herein, means a non-toxic, inert solid, semi-solid or liquid filler, diluent, encapsulating material or formulation auxiliary of any type. Some examples of materials which can serve as pharmaceutically acceptable carriers are sugars such as, but not limited to, lactose, glucose and sucrose; starches such as, but not limited to, corn starch and potato starch; cellulose and its derivatives such as, but not limited to, sodium carboxymethyl cellulose, ethyl cellulose and cellulose acetate; powdered tragacanth; malt; gelatin; talc; excipients such as, but not limited to, cocoa butter and suppository waxes; oils such as, but not limited to, peanut oil, cottonseed oil, safflower oil, sesame oil, olive oil, corn oil and soybean oil; glycols; such a propylene glycol; esters such as, but not limited to, ethyl oleate and ethyl laurate; agar; buffering agents such as, but not limited to, magnesium hydroxide and aluminum hydroxide; alginic acid; pyrogen-free water; isotonic saline; Ringer's solution; ethyl alcohol, and phosphate buffer solutions, as well as other non-toxic compatible lubricants such as, but not limited to, sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Pharmaceutical compositions of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), vegetable oils (such as olive oil), injectable organic esters (such as ethyl oleate) and suitable mixtures thereof. Proper fluidity can be maintained, for example, by the use of coating materials such as lecithin, by the maintenance of the required particle size in the case of dispersions and by the use of surfactants.

These compositions may also contain adjuvants such as preservatives, wetting agents, emulsifying agents and dispersing agents. Prevention of the action of microorganisms can be ensured by the inclusion of various antibacterial and antifungal agents, for example, paraben, chlorobutanol, phenol sorbic acid and the like. It may also be desirable to include isotonic agents such as sugars, sodium chloride and the like. Prolonged absorption of the injectable pharmaceutical form can be brought about by the inclusion of agents which delay absorption such as aluminum monostearate

and gelatin.

In some cases, in order to prolong the effect of the drug, it is desirable to slow the absorption of the drug from subcutaneous or intramuscular injection. This can be accomplished by the use of a liquid suspension of crystalline or amorphous material with poor water solubility. The rate of absorption of the drug then depends upon its rate of dissolution which, in turn, may depend upon crystal size and crystalline fonn. Alternatively, delayed absorption of a parenterally administered drug form is accomplished by dissolving or suspending the drug in an oil vehicle.

Injectable depot forms are made by fonning microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter or by incorporating sterilizing agents in the fonn of sterile solid compositions which can be dissolved or dispersed in sterile water or other sterile injectable medium just prior to use.

Solid dosage fonns for oral administration include capsules, tablets, pills, powders and granules. In such solid dosage forms, the active compound may be mixed with at least one inert, phannaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage fonn may also comprise buffering agents.

Solid compositions of a similar type may also be employed as fillers in soft and hard-filled gelatin capsules using such carriers as lactose or milk sugar as well as high molecular weight polyethylene glycols and the like.

The solid dosage forms of tablets, dragees, capsules, pills and granules can be prepared with coatings and shells such as enteric coatings and other coatings well-known in the pharmaceutical fonnulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned carriers.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, genn, olive, castor and sesame oils), glycerol, tetrahydrofurfuryl alcohol, polyethylene glycols and fatty acid esters of sorbitan and mixtures thereof.

Besides inert diluents, the oral compositions may also include adjuvants such as wetting agents, emulsifying and suspending agents, sweetening, flavoring and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, tragacanth and mixtures thereof.

Compositions for rectal or vaginal administration are preferably suppositories which can be prepared by mixing the compounds of this invention with suitable non-irritating carriers or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients and the like. The preferred lipids are natural and synthetic phospholipids and phosphatidyl cholines (lecithins) used separately or together.

Methods to form liposomes are known in the art. See, for example, Prescott, Ed., Methods in Cell Biology, Volume XIV, Academic Press, New York, N.Y. (1976), p. 33 et seq.

Dosage forms for topical administration of a compound of this invention include powders, sprays, ointments and inhalants. The active compound may be mixed under sterile conditions with a pharmaceutically acceptable carrier

and any needed preservatives, buffers or propellants which may be required. Opthalmic formulations, eye ointments, powders and solutions are also contemplated as being within the scope of this invention.

The compounds of the present invention can be used in the form of pharmaceutically acceptable salts derived from inorganic or organic acids. The phrase "pharmaceutically acceptable salt" means those salts which are, within the scope of sound medical judgment, suitable for use in contact with the tissues of humans and lower animals without undue toxicity, irritation, allergic response and the like and are commensurate with a reasonable benefit/risk ratio.

Pharmaceutically acceptable salts are well known in the art. For example, S. M. Berge et al. describe pharmaceutically acceptable salts in detail in (J. Pharmaceutical Sciences, 1977, 66: 1 et seq). The salts can be prepared in situ during the final isolation and purification of the compounds of the invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate (isothionate), lactate, malate, maleate, methanesulfonate, nicotinate, 2-naphthalenesulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as, but not limited to, methyl, ethyl, propyl, and butyl chlorides, bromides and iodides; dialkyl sulfates like dimethyl, diethyl, dibutyl and diamyl sulfates; long chain halides such as, but not limited to, decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulfuric acid, and phosphoric acid and such organic acids as acetic acid, fumaric acid, maleic acid, 4-methylbenzenesulfonic acid, succinic acid and citric acid.

Basic addition salts can be prepared in situ during the final isolation and purification of compounds of this invention by reacting a carboxylic acid-containing moiety with a suitable base such as, but not limited to, the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as, but not limited to, lithium, sodium, potassium, calcium, magnesium and aluminum salts and the like and nontoxic quaternary ammonia and amine cations including ammonium, tetramethylammonium, tetraethylammonium, methylamine, dimethylamine, trimethylamine, triethylamine, diethylamine, ethylamine and the like. Other representative organic amines useful for the formation of base addition salts include ethylenediamine, ethanolamine, diethanolamine, piperidine, piperazine and the like.

The compounds of the invention can exist in unsolvated as well as solvated forms, including hydrated forms, such as hemi-hydrates. In general, the solvated forms, with pharmaceutically acceptable solvents such as water and ethanol among others are equivalent to the unsolvated forms for the purposes of the invention.

f. General Synthesis

This invention is intended to encompass compounds of the invention when prepared by synthetic processes .

The compounds of the invention may be prepared by a variety of processes well known for the preparation of compounds of this class. For example, the compounds of the invention wherein the groups $R_8$, $R_1$, $R_2$, $R_3$, $R_4$, $R_9$, $R_{10}$, $R_{31}$, $R_{32}$, $R_{41}$, $R_{42}$, $Z_1$, $Z_2$, $G_1$, and $L_1$ have the meanings as set forth in the summary section unless otherwise noted, can be synthesized as shown in Schemes 1-15.

As used in the descriptions of the schemes and the examples, certain abbreviations are intended to have the following meanings: aq. for aqueous; DME for dimethoxyethane, DMF for N,N-dimethylformamide; dppf for ,1'-bis(diphenylphosphino)ferrocene, EtOAc for ethyl acetate, EtOH for ethanol, $Et_3N$ for triethylamine, HATU for O-(7-azabenzotriazol-1-yl)-N,N,N',N'-tetramethyluronium hexafluorophosphate, $Et_2O$ for diethyl ether, $Et_3N$ for triethylamine, HPLC for high performance liquid chromatography, Ph for phenyl; mesyl for methanesulfonate; MeOH for methanol, min for minute or minutes; n-Bu for n-butyl; DMSO for dimethylsulfoxide; dppf for 1,1'-bis(diphenylphosphino)ferrocene; TFA for trifluoroacetic acid; THF for tetrahydrofuran; Ts or tosyl for $p\text{-}CH_3PhS(O)_2O\text{-}$; and Tf or triflate for $CF_3S(O)_2O\text{-}$.

Scheme 1

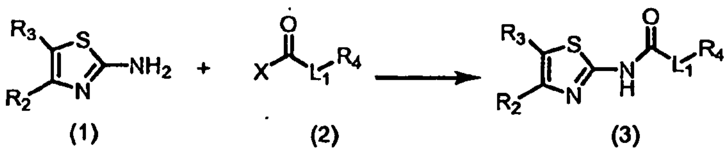

(1) (2) (3)

As shown in Scheme 1, compounds of formula (1) containing an amine group when treated with compounds of formula (2), wherein X is chloro or -OH under coupling conditions known to one skilled in the art, will provide compounds of formula (3). Typical conditions for the reaction of compounds of formula (2) wherein X is chloro and compounds of formula (1) include but are not limited to stirring an equimolar mixture of the compounds in solvents such as chloroform, dichloromethane or THF in the presence of a base such as but not limited to diisopropylethylamine at 0-30 °C for 8-24 hours. Acid coupling conditions of compounds of formula (2), wherein X is -OH and compounds of formula (1), include stirring an equimolar mixture of the compounds with a coupling reagent such as but not limited to bis(2-oxo-3-oxazolidinyl) phosphinic chloride (BOPCl), 1,3-dicyclohexylcarboiimide (DCC), polymer supported 1,3-dicyclohexylcarbodiimide (PS-DCC), O-(7-azabenzotriazol-1-yl)-N,N,N',N'-tetramethyluronium hexafluorophosphate (HATU), O-benzotriazol-1-yl-N, N,N',N'-tetramethyluronium tetrafluoroborate (TBTU) along with a coupling auxiliary such as but not limited to 1-hydroxy-7-azabenzotriazole (HOAT) or 1-hydroxybenzotriazole hydrate (HOBT) in the presence or absence of a base such as but not limited to N-methyl morpholine, diisopropylethylamine in a solvent such as, but not limited to, THF, N,N-dimethylacetamide, N,N-dimethylformamide, pyridine, chloroform, or mixtures thereof. Typical reactions can be carried out between 0-65 °C or may be carried out in a microwave reactor to facilitate the coupling.

Scheme 2

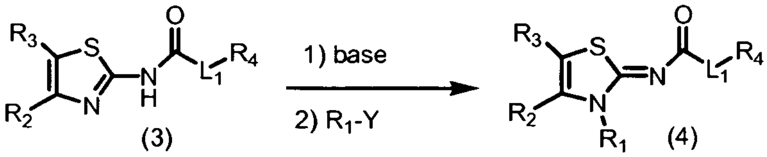


(3) (4)

As shown in Scheme 2, compounds of formula (3) may be converted into compounds of fonnula (4) which are representative compounds of the present invention. Typical conditions include, but are not limited to, the treatment of compounds of formula (3) with sodium hydride in DMF at 0 °C, followed by the addition of reagents such as $R_1$-Y, wherein $R_1$ is as defined in fonnula (I) and Y is chloro, bromo, iodo, tosyl, mesyl or triflate. Alternatively, other bases such as potassium hydroxide or potassium tert-butoxide in a mixture of THF and DMF, followed by treatment with $R_1$-Y will also provide compounds of formula (4).

Scheme 3

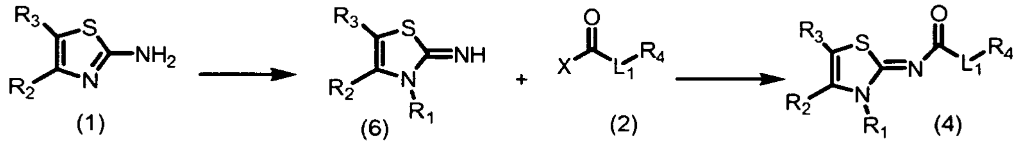

(1) (6) + (2) (4)

Alternatively, compounds of formula (4) may also be prepared according to the methods outlined in Scheme 3. Compounds of formula (1) when treated with sodium hydride in DMF at 0 °C, followed by the addition of reagents such as $R_1$-Y, wherein Y is chloro, bromo, iodo, tosyl, mesyl or triflate provide compounds of formula (6). Alternatively, compounds of formula (1) may be heated neat or in the presence of a minimal amount of solvent to facilitate mixing with compounds of formula $R_1$-Y to obtain compounds of formula (6). Compounds of formula (6) may be isolated as a salt or a free base. The treatment of compounds of formula (6) with compounds of formula (2), wherein X is chloro or -OH, under coupling conditions as outlined in Scheme 1 can generate compounds of formula (4).

Scheme 4

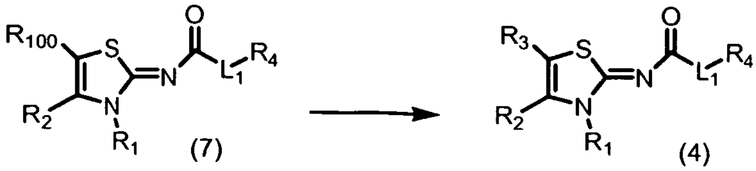

(7) (4)

As outlined in Scheme 4, compounds of formula (7), wherein $R_{100}$ is halide or triflate and which can be prepared according to the methods outlined in Schemes 1-3, when treated with a boronic acid of formula $R_3B(OH)_2$, wherein $R_3$

is aryl, arylalkenyl, cycloalkyl, heterocycle or heteroaryl, a palladium catalyst such as dichlorobis(triphenyl)phosphine) palladium (II) and sodium carbonate in a mixture of solvents which include but are not limited to various mixtures of DME, ethanol and water under heated conditions provide compounds of formula (4) wherein $R_3$ is alkenyl, aryl, arylalkenyl, cycloalkyl, heterocycle or heteroaryl.

Scheme 5

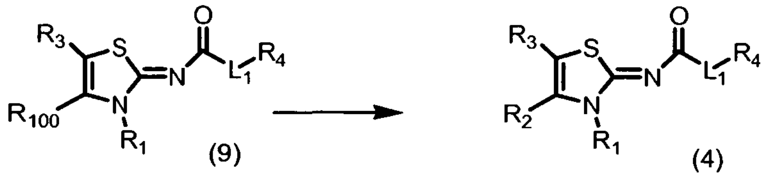

(9) (4)

As outlined in Scheme 5, compounds of formula (9) wherein $R_{100}$ is halide or triflate and which can be prepared according to the methods outlined in Schemes 1-3, when treated with a boronic acid of formula $R_1$B(OHh wherein $R_2$ is aryl, arylalkenyl, cycloalkyl, heterocycle or heteroaryl and a palladium catalyst according to the methods outlined is Scheme 4 can provide compounds of formula (4) wherein $R_2$ is aryl, arylalkenyl, cycloalkyl, heterocycle or heteroaryl.

Scheme 6

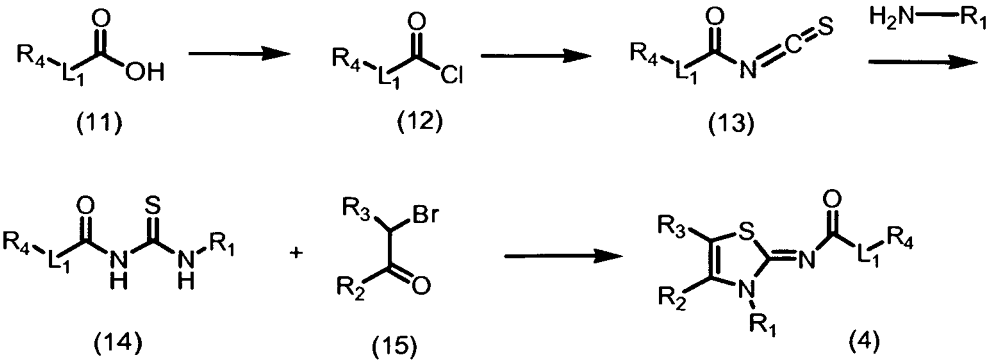


(11) (12) (13) (14) (15) (4)

Scheme 6 describes another alternative method for the preparation of compounds of formula (4). Compounds of formula (11) when treated with oxalyl chloride in dichloromethane containing a catalytic amount of DMF will provide the acid chloride of formula (12). The acid chloride of formula (12) when treated with potassium thiocyanate in acetone can provide compounds of formula (13). Compounds of formula (13) when treated with an amine of formula $R_1$-$NH_2$ in solvents such as but not limited to THF can provide compounds of formula (14). Compounds of formula (14) when treated with substituted alpha-bromo-ketones of formula (15) in ethanol or mixtures of ethanol and toluene under heated conditions will provide compounds of formula (4).

Scheme 7

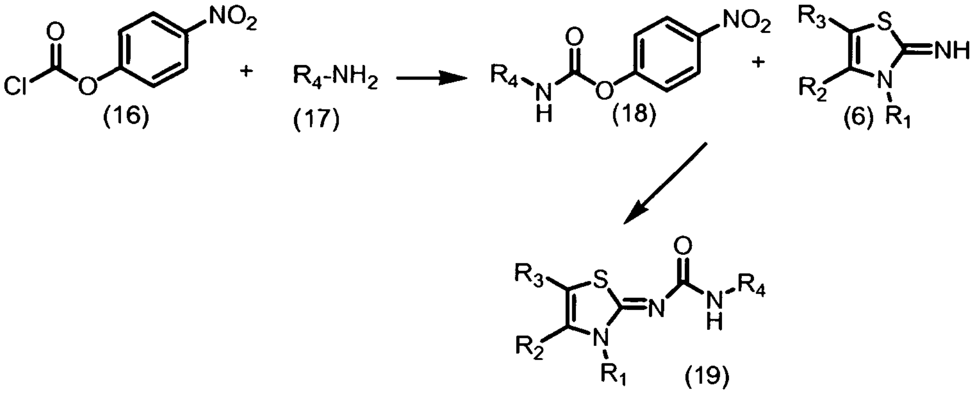

(16) + $R_4$-$NH_2$ (17) (18) + (6) (19)

Compounds of formula (I) wherein $L_1$ is -NH-, may be prepared as outlined in Scheme 7. Compounds of fonnula (16) when treated with an amine of fonnula (17), wherein $R_4$ is defined in formula (I), will provide compounds of formula (18). Compounds of formula (18) when treated with compounds of fonnula (6) can provide compounds of formula (19).

Scheme 8

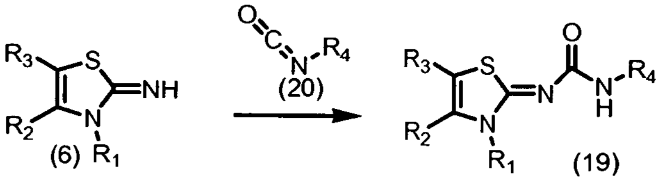

(6) (19)

Alternatively, compounds of formula (6) when treated with an isocyanate of formula (20) provide compounds of fonnula (19).

Scheme 9

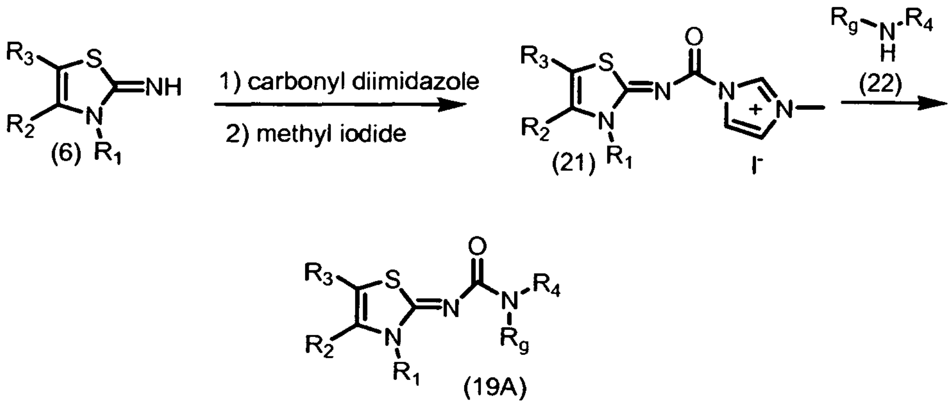

(6) (21) (19A)

Similarly, compounds of formula (6) when treated with carbonyl diimidazole, followed by treatment with methyl iodide, can provide the imidazolide compounds of fonnula (21). Compounds of fonnula (21) when treated with an amine of fonnula (22) can provide compounds of formla (19A).

Scheme 10

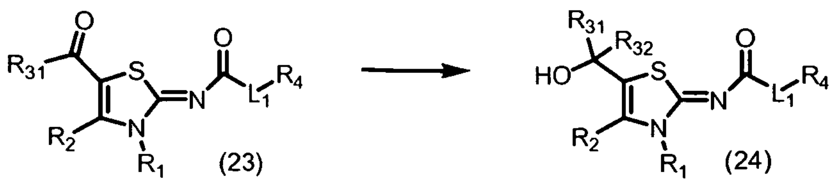
(23) (24)

As shown in Scheme 10, compounds of formula (23) when treated at low temperatures with an organolithium reagent such as but not limited to $R_{31}Li$ or a Grignard reagent such as but not limited to $R_{32}MgBr$ can be converted to compounds of formula (24). The reaction is typically conducted in a solvent such as but not limited to diethyl ether.

Scheme 11

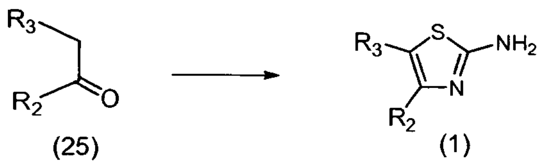
(25) (1)

As shown in Scheme 11, compounds of formula (25) when treated with pyrrolidine and p-toluenesulfonic acid monohydrate in a solvent such as but not limited to cyclohexane at reflux followed by treatment with sulfur and cyanamide in a solvent such as but not limited to methanol at temperatures between 0-70 °C, can provide compounds of formula (1).

Scheme 12

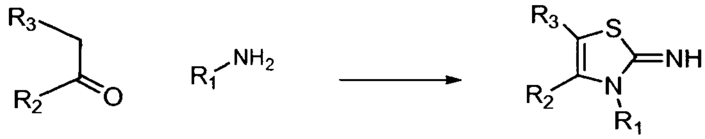

(25) (6)

As shown in Scheme 12, compounds of formula (25) when treated with amines of formula $R_1NH_2$, in the presence of molecular sieves in a solvent such as, but not limited to, acetonitrile, at a temperatures ranging from about 25 °C to about 80 °C, followed by treatment with potassium thiocyanate and iodine at temperatures between 40-80 °C, provide compounds of formula (6).

Certain compounds of formula (I) where $R_4$ or $R_9$ is phenyl and said phenyl is substituted with the group $-OR_{102}$ can be prepared using the methods described in Scheme 13.

Scheme 13

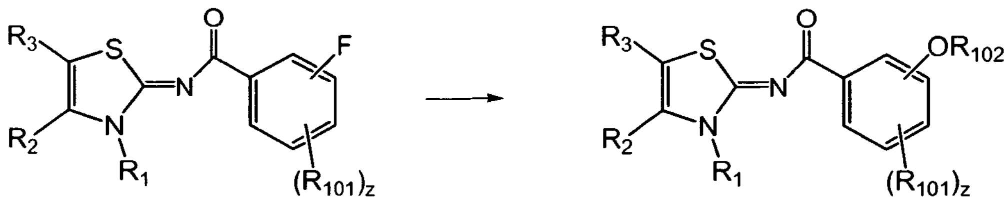

(26) (27)

Compounds of formula (27), wherein $R_{101}$ represents the optional substituents of $R_4$ and $R_9$ of formula (I), z is 0, 1, 2, 3, or 4, and $R_{102}$ is $R_{10}$, alkyl, alkoxyalkyl, haloalkyl, $NZ_1Z_2-(CR_4)R_{42})_p-$, or $G_1-(CR_{41}R_{42})_p-$can be prepared from compounds of formula (26) by reaction with an alcohol $HOR_{102}$ in the presence of a base such as, but not limited to, potassium tert-butoxide or sodium tert-butoxide in a solvent such as, but not limited to, tetrahydrofuran or dimethylformamide at temperatures between about 0 °C and 50 °C. In certain instances, a protecting group may be attached to a functional group present in $R_{102}$. Such protecting groups can be removed using methods well-known to those skilled in the art. The group $R_{102}$ can also be further transformed to provide other compounds of the invention using standard chemical techniques well-known to those skilled in the art such as alkylation, acylation, and reductive amination.

Certain compounds of formula (I) wherein $R_4$ or $R_9$ is phenyl and said phenyl is substituted with a group $R_{103}$, can be prepared according to the carbon-carbon bond fonning reactions described in Scheme 14.

Scheme 14

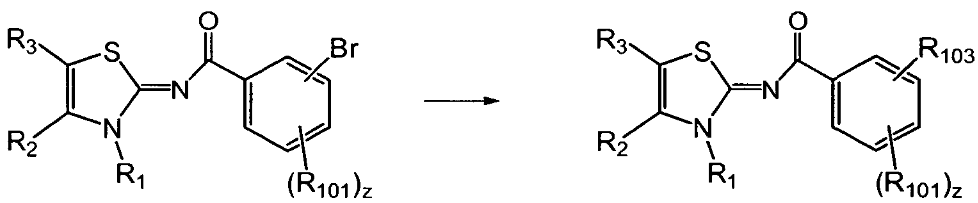

(28) (29)

Compounds of formula (29), wherein $R_{10}$, represents the optional substituents of $R_4$ and $R_9$ of formula (I), z is 0, 1, 2, 3, or 4, and $R_{103}$ is selected from the group consisting of alkenyl, alkoxyalkoxyalkyl, alkoxyalkyl, alkoxycarbonylalkyl, alkyl, alkylcarbonylalkyl, alkylsulfinylalkyl, alkyl-$S(O)_2-(CR_{41}R_{42})_p=C(R_{41})-$, alkyl-$S(O)_2-(CR_{41}R_{42})_p-$, alkyl-

S-$(CR_{41}R_{42})_p$-,alkynyl, carboxyalkyl, cyano, cyanoalkyl, formylalkyl, haloalkyl, hydroxyalkyl, -$C(R_{41})$=N-$O(R_{42})$, and -$(CR_4)R_{42})_p$-$C(R_4)$)=N-$O(R_{42})$, can be prepared from compounds of formula (28). Reactions well-known in the chemical literature for effecting these transformations include the Suzuki, Heck, Stille, Sonogashira, and Negishi reactions. Typical reaction conditions for can be found in the following references: Negishi, E. A. Handbook of Organopalladium Chemistry for Organic Synthesis; Wiley-Interscience: New York, 2002; Miyaura, N. Cross-Coupling Reactions: A Practical Guide; Springer: New York, 2002. More specifically, where $R_{103}$ is alkyl-$S(O)_2$-$(CR_{41}R_{42})_p$=$(R_{41})$-, or alkenyl, compounds can be prepared using palladium acetate, tri(o-tolyl)phosphine as the ligand, triethylamine as base with the corresponding vinyl sulfone, or alkene under microwave conditions at temperatures from 140-180 °C. In the conversion of (28) to (29), the -Br of (28) may also be a triflate, -I, -Cl, a boronic acid (or derivative), stannyl or the like.

Certain compounds of formula (I) wherein $R_4$ or $R_9$ is phenyl, can be prepared according to the method shown in Scheme 15.

Scheme 15

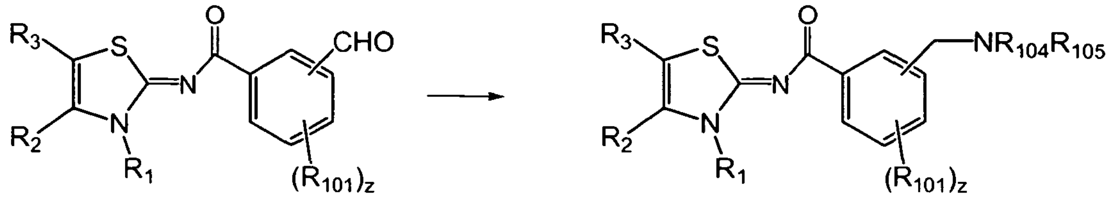

(30) (31)

Compounds of the invention of formula (31), wherein $R_{101}$ represents the optional substituents of $R_4$ and $R_9$ of formula (I), z is 0, 1, 2, 3, or 4, and $NR_{104}R_{105}$ is $NZ_1Z_2$ or $G_1$, can be prepared from compounds of formula (30) by a reductive amination reaction. The reductive amination reaction is well known to those skilled in the art. For example, reaction of compounds (30) with amines $HNR_{104}R_{105}$ in solvents such as, but not limited to, acetonitrile, tetrahydrofuran, dichloromethane or dichloroethane, in the presence of a reducing agent such as, but not limited to, sodium cyanoborohydride or sodium triacetoxyborohydride, can provide compounds (31). The reaction may be conducted in the presence of an acid (e.g., acetic acid).

It will be appreciated that the synthetic schemes and specific examples as illustrated in the Examples section are illustrative and are not to be read as limiting the scope of the invention as it is defined in the appended claims. All alternatives, modifications, and equivalents of the synthetic methods and specific examples are included within the scope of the claims.

Optimum reaction conditions and reaction times for each individual step may vary depending on the particular reactants employed and substituents present in the reactants used. Unless otherwise specified, solvents, temperatures and other reaction conditions may be readily selected by one of ordinary skill in the art. Specific procedures are provided in the Examples section. Reactions may be worked up in the conventional manner, e.g. by eliminating the solvent from the residue and further purified according to methodologies generally known in the art such as, but not limited to, crystallization, distillation, extraction, trituration and chromatography. Unless otherwise described, the starting materials and reagents are either commercially available or may be prepared by one skilled in the art from commercially available materials using methods described in the chemical literature.

Routine experimentations, including appropriate manipulation of the reaction conditions, reagents and sequence of the synthetic route, protection of any chemical functionality that may not be compatible with the reaction conditions, and deprotection at a suitable point in the reaction sequence of the method are included in the scope of the invention. Suitable protecting groups and the methods for protecting and deprotecting different substituents using such suitable protecting groups are well known to those skilled in the art; examples of which may be found in T. Greene and P. Wuts, Protecting Groups in Chemical Synthesis (3rd ed.), John Wiley & Sons, NY (1999), which is -incorporated herein by reference in its entirety. Synthesis of the compounds of the invention may be accomplished by methods analogous to those described in the synthetic schemes described hereinabove and in specific examples.

Starting materials, if not commercially available, may be prepared by procedures selected from standard organic chemical techniques, techniques that are analogous to the synthesis of known, structurally similar compounds, or techniques that are analogous to the above described schemes or the procedures described in the synthetic examples section.

When an optically active form of a compound of the invention is required, it may be obtained by carrying out one of the procedures described herein using an optically active starting material (prepared, for example, by asymmetric induction of a suitable reaction step), or by resolution of a mixture of the stereoisomers of the compound or intermediates

using a standard procedure (such as chromatographic separation, recrystallization or enzymatic resolution).

Similarly, when a pure geometric isomer of a compound of the invention is required. it may be obtained by carrying out one of the above procedures using a pure geometric isomer as a starting material, or by resolution of a mixture of the geometric isomers of the compound or intermediates using a standard procedure such as chromatographic separation.

Following Examples may be used for illustrative purposes and should not be deemed to narrow the scope of the invention.

Examples

Examples 336 - 342 and 367 form part of the invention. The remaining Examples are comparative Examples.

Example 1

N-[(2Z)-3-(2-methoxyethyl]-1,3-thiazol-2(3H)-ylidene]-2,2,3,3-tetramethylcyclopropanecarboxamide

Example 1

2,2,3,3-tetramethylcyclopropanecarbonyl chloride.

To a solution of 2,2,3,3-tetramethylcyclopropanecarboxylic acid (0.50 g, 3.5 mmol) in 18 mL of methylene chloride at 0 °C was added oxalyl chloride (0.61 mL, 7.0 mmol) and a catalytic amount of dimethylformamide (2 drops). The solution was stirred at ambient temperature for 1 hour, and then concentrated under reduced pressure to provide 0.56 g of the title compound.

Example 1B

2,2,3,3-tetramethyl-N-1,3-thiazol-2-ylcyclopropanecarboxamide

To a solution of 2-aminothiazole (0.39 g, 3.9 mmol) in 10 mL of methylene chloride at 0 °C was added a solution of the product from Example 1A in 8 mL of chloroform, followed by triethylamine (1.0 mL, 7.7 mmol). The mixture was stirred for 7 hours at 35 °C, cooled to ambient temperature and diluted with water. The phases were separated and the aqueous phase was extracted with methylene chloride. The combined organic extracts were washed twice with water and then brine, dried over magnesium sulfate, filtered, and concentrated under reduced pressure. Purification by column chromatography ($SiO_2$, 30-60% ethyl acetate/hexanes gradient) afforded 0.11 g (14%) of the title compound. $^1H$ NMR ($CDCl_3$, 300 MHz) $\delta$ ppm 1.18 (s, I H), 1.25 (s, 6 H), 1.35 (s, 6 H), 6.92 (d, J = 3.4 Hz, 1 H), 7.39 (d, J = 3.4 Hz, 1 H), 10.7 (s, 1 H); MS ($DCI/NH_3$) m/z 225 $(M+H)^+$. Anal. Calculated for $C_{11}H_{16}N_2OS$: C, 58.90; H, 7.19; N, 12.49. Found: C, 59.03; H, 7.34; N, 12.34.

Example 1C

*N*-[(2*Z*)-3-(2-methoxyethyl)-1,3-thiazol-2(3*H*)-ylidene]-2,2,3,3-tetramethylcyclopropanecarboxamide

To a solution of Example 1B (0.16 g, 0.71 mmol) in 3.5 mL of 4:1 tetrahydrofuran:dimethylformamide at 0 °C was added potassium hydroxide (90 mg, 1.7 mmol). After stirring for 1 hour at room temperature, 2-bromoethyl methyl ether (73 $\mu$L, 7.1 mmol) was added and the solution was heated to 65 °C for 14 hours. The solution was allowed to cool to ambient temperature and then diluted with ethyl acetate and washed twice with water and then brine. The organic extract was dried over magnesium sulfate, filtered, and concentrated under reduced pressure. Purification by column chromatography ($SiO_2$, 30-50% ethyl acetate/hexanes gradient) afforded 22 mg (11%) of the title compound. $^1H$ NMR ($CDCl_3$, 300 MHz) $\delta$ 1.25 (s, 12H), 1.75 (s, 1H), 3.32 (s, 3H), 3.74 (t, J = 5.6 Hz, 2H), 4.44 (t, J = 5.4 Hz, 2H), 6.95 (d, J = 3.4 Hz, 1H), 7.48 (t, J = 3.7 Hz, 1H); MS ($DCI/NH_3$) m/z 283 $(M+H)^+$. Anal. Calculated for $C_{14}H_{22}N_2O_2S$: C, 59.54; H, 7.85; N, 9.92. Found: C, 59.76; H, 7.97; N, 9.91.

Example 2

5-chloro-2-methoxy-N-[(2Z)-3-(2-methoxyethyl)-1,3-thiazol-2(3H)-ylidene]benzamide

Example 2A

3-(2-methoxyethyl)-1,3-thiazol-2(3*H*)-imine hydrobromide

A mixture of 2-aminothiazole (15 g, 0.15 mol) and 2-bromoethyl methyl ether (17 mL, 0.18 mol) were heated at 85 °C for 16 hours. After cooling to ambient temperature the resulting solid was triturated twice with isopropyl alcohol to afford 26 g (72%) of the title compound. $^1H$ NMR (DMSO-$d_6$, 300 MHz) δ ppm 3.27 (s, 3 H), 3.63 (t, J = 5.1 Hz, 2 H), 4.23 (t, J = 4.9 Hz, 2 H), 7.02 (d, J = 4.7 Hz, 1 H), 7.38 (d, J = 4.4 Hz, 1 H), 9.52 (s, 1 H); MS (DCI/$NH_3$) m/z 159 $(M+H)^+$.

Example 2B

5-Chloro-2-methoxy-*N*-[(2*Z*)-3-(2-methoxyethyl)-1,3-thiazol-2(3*H*)-ylidene]benzamide

To a solution of the product from Example 2A (0.77 g, 3.2 mmol) and 5-chloro-2-methoxybenzoic acid (0.50 g, 2.7 mmol) in 14 mL of THF at 0 °C was added *O*-(7-azabenzotriazol-1-yl)-*N,N,N',N'*-tetramethylronium hexafluorophosphate (HATU, 1.24 g, 3.2 mmol) and *N,N*-diisopropylethylamine (1.1 mL), 6.2 mmol). The mixture was heated to 65 °C for 2.5 hours, cooled to ambient temperature and then diluted with ethyl acetate. The mixture was washed twice with water, then saturated aqueous sodium bicarbonate, and brine. The organic extract was dried over magnesium sulfate, and concentrated under reduced pressure. Purification by column chromatography ($SiO_2$, 20-35% ethyl acetate/hexanes gradient) afforded 0.38 g (43%) of the title compound. $^1H$ NMR ($CDCl_3$, 300 MHz) δ ppm 3.35 (s, 3 H), 3.72-3.81 (m, 2 H), 3.91 (s, 3 H), 4.41-4.48 (m, 2 H), 6.65 (d, *J*=4.7 Hz, 1 H), 6.92 (d, *J*=8.8 Hz, 1 H), 7.16 (d, *J*=4.7 Hz, I H), 7.34 (dd, *J*=8.8, 3.1 Hz, 1 H), 7.99 (d, *J*=2.7 Hz, 1 H); MS (DCI/$NH_3$) m/z 349 $(M+H)^+$. Anal. Calculated for $C_{18}H_{14}N_2O_3S$: C, 62.04; H, 6.94; N, 8.04. Found: C, 62.24; H, 7.08; N, 8.04.

Example 3

N-[(2Z)-3-(2-methoxyethyl)-1,3-thiazol-2(3H)-ylidene]cycloheptanecarboxamide

Cycloheptanecarboxylic acid (29 mg, 0.20 mmol), 3 equiv of polymer bound dicyclohexylcarbodiimide (PS-DCC), 1-hydroxybenzotriazole hydrate (HOBT, 22 mg, 0.16 mmol), *N,N*-diisopropylethylamine (62 mg, 0.50 mmol), and the product of Example 2A (39 mg, 0.16 mmol) were combined in dimethylacetamide (DMA, 2.8 mL) and heated in a microwave to 100 °C for 420 seconds. The mixture was filtered through Si-Carbonate (6 mL-1g cartridge from Silicycle Chemical Division) and then concentrated to dryness. The residue was dissolved in 1: I DMSO/MeOH and purified by reverse phase HPLC to afford the title compound. $^1H$ NMR (500 MHz, DMSO-$d_6$) δ ppm 1.39 - 1.72 (m, 11 H) 1.83 - 1.95 (m, 2 H) 2.46 - 2.52 (m, 1 H) 3.23 - 3.26 (m, 3 H) 3.67 (t, 2 H) 4.29 (t, 2 H) 6.76 - 6.97 (d, I H) 7.30 - 7.43 (d, 1 H); MS (ESI) m/z 283 $(M+H)^+$.

Example 4

N-[(2Z)-3-(3-methoxypropyl)-1,3-thiazol-2(3H)-ylidene]-2,2,3,3-tetramethylcyclopropanecarboxamide

Example 4A

3-(3-methoxypropyl)-1,3-thiazol-2(3*H*)-imine hydrobromide

A mixture of 2-aminothiazole (1.0 g, 10 mmol) and 1-bromo-3-methoxypropane (1.8 g, 12 mmol) were heated at 85 °C for 16 hours. The solid was cooled to ambient temperature, triturated with ethanol, and then collected by filtration to provide 1.2 g (48%) of the title compound. MS (DCI/$NH_3$) m/z 173 $(M+H)^+$.

Example 4B

N-[(2Z)-3-(3-methoxypropyl)-1,3-thiazol-2(3H)-ylidene]-2,2,3,3-tetramethylcyclopronanecarboxamide

The product of Example 4A (0.60 g, 2.4 mmol) and 2,2,3,3-tetramethylcyclopropanecarboxylic acid (0.34 g,

2.4 mmol) were processed using the method described in Example 2B. Purification by column chromatography ($SiO_2$, 20-40% ethyl acetate/hexanes gradient) afforded 0.33 g (47%) of the title compound. $^1H$ NMR ($CDCl_3$, 300 NMz) δ ppm 1.21 (s, 6 H), 1.34 (s, 6 H), 1.56 (d, *J*=5.4 Hz, 1 H), 2.00-2.13 (m, 2 H), 3.31-3.39 (m, 5 H), 4.23 (t, *J*=6.8 Hz, 2 H), 6.50 (d, *J*=4.7 Hz, 1 H), 6.88 (d, *J*=4.7 Hz, 1 H); MS ($DCI/NH_3$) m/z 297 $(M+H)^+$. Anal. Calculated for $C_{15}H_{24}N_2O_2S$: C, 60.78; H, 8.27; N 9.45. Found: C, 60.78; H, 8.27; N, 9.34.

Example 5

N-[(2Z)-3-(2-methoxyethyl)-4-methyl-1,3-thiazol-2(3H)-ylidene]-2,2,3,3-tetramethylcyclopronanecarboxamide

Example 5A

3-(2-methoxyethyl)-4-methyl-1,3-thiazol-2(3*H*)-imine hydrobromide

A mixture of 4-methylthiazol-2-ylamine (0.75 g, 6.5 mmol) and 2-bromoethyl methyl ether (730 μL, 7.8 mmol) was heated at 85 °C for 15 hours. The mixture was cooled to ambient temperature and the resulting solid was triturated with isopropanol. Recrystallization from hot ethanol afforded 0.56 g (34%) of the title compound. $^1H$ NMR (DMSO-$d_6$, 300 MHz) δ ppm 2.25 (d, *J*=1.4 Hz, 3 H) 3.25 (s, 3 H) 3.57 (t, *J*=5.1 Hz, 2 H) 4.15 (t, *J*=5.1 Hz, 2 H) 6.68 (d, *J*=1.4 Hz, I H) 9.40 (s, I H); MS ($DCI/NH_3$) m/z 173 $(M+H)^+$.

Example 5B

N-[(2Z)-3-(2-methoxyethyl)-4-methyl-1,3-thiazol-2(3H)-ylidene]-2,2,3,3-tetramethylcyclopropanecarboxamide

The product of Example 5A (0.30 g, 1.2 mmol) and 2,2,3,3-tetramethylcyclopropanecarboxylic acid (0.19 g, 1.3 mmol) were processed using the method described in Example 2B. Purification by column chromatography ($SiO_2$, 20-40% ethyl acetate/hexanes gradient) afforded 0.14 g (41%) of the title compound. $^1H$ NMR ($CDCl_3$, 300 MHz) δ ppm 1.21 (s, 6 H), 1.34 (s, 6 H), 1.59 (s, I H), 2.30 (s, 3 H), 3.30 (s, 3 H), 3.70 (t, *J*=5.09 Hz, 2 H), 4.25 (t, *J*=5.26 Hz, 2 H), 6.09 (s, I H); MS ($DCI/NH_3$) m/z 297 $(M+H)^+$. Anal. Calculated for $C_{15}H_{24}N_2O_2S$: C, 60.78; H, 8.16; N, 9.45. Found: C, 60.79; H, 7.82; N, 9.36.

Example 6

ethyl ((2Z)-3-(2-methoxyethyl)-2-{[(2,2,3,3-tetramethylcyclopropyl)carbonyl]imino}-2,3-dihydro-1,3-thiazol-4-yl)acetate

Example 6A

ethyl (2-imino-3-(2-methoxyethyl)-2,3-dihydro-1,3-thiazol-4-yl]acetate hydrobromide

A mixture of (2-aminothiazol-4-yl)acetic acid ethyl ester (18.6 g, 100 mmol) and 2-bromoethyl methyl ether (15.3 g, 110 mmol) were processed using the method described in Example 2A to afford 14.1 g (83%) of the title compound.. $^1H$ NMR (300 MHz, DMSO-$d_6$) δ ppm 1.14 - 1.28 (m, 3 H) 3.24 (s, 3 H) 3.54 (t, *J*=5 Hz, 2 H) 3.91 (s, 2 H) 4.04 - 4.25 (m, 4 H) 6.92 (s, 1 H) 9.50 (s, I H); MS ($DCI/NH_3$) m/z 231$(M+H)^+$.

Example 6B

ethyl ((2Z)-3-(2-methoxyethyl)-2-{[(2,2,3,3-tetramethylcyclopropll)carbonyl]imino}-2,3-dihydro-1,3-thiazol-4-yl)acetate

The product of Example 6A (2.3 g, 10 mmol) and 2,2,3,3-tetramethylcyclopropane carboxylic acid (1.6 g, 11 mmol) were processed as described using the method described in Example 2B to afford 2.1g (54%) of the title compound. $^1H$ NMR (300 MHz), DMSO-$d_6$) δ ppm 1.06 - 1.33 (m, 15 H) 1.48(s, I H) 3.22 (s, 3 H) 3.59 (t, *J*=5 Hz, 2 H) 3.91 (s, 2 H) 4.12 (t, .*J*=7 Hz, 2 H) 4.14 - 4.24 (m, 2 H) 6.69 (s, 1 H); MS ($DCI/NH_3$) m/z 369 $(M+H)^+$. Anal. Calculated for $C_{18}H_{28}N_2O_4S$ C, 68.67; H, 7.66; N, 7.62. Found: C, 68.67; H, 7.66; N, 7.60.

Examnle 7

ethyl (2Z)-3-(2-methoxyethyl)-2-{(2,2,3,3-tetramethylcyclopropyl)carbonyl]imino}-2,3-dihydro-1,3-thiazole-4-carboxylate

Example 7A

ethyl 2-imino-3-(2-methoxyethyl)-2,3-dihydro-1,3-thiazole-4-carboxylate hydrobromide

A mixture of 2-aminothiazole-4-carboxylic acid ethyl ester (17.2 g, 100 mmol) and 2-bromoethyl methyl ether (15.3 g, 110 mmol) were processed using the method described in Example 2A to afford 17.1g (83%) of the title compound. $^1$H NMR (300 MHz, DMSO-$d_6$) δ ppm 1.30 (t, *J*=7 Hz, 3 H) 3.22 (s, 3 H) 3.60 (t, *J*=5 Hz, 2 H) 4.32 (t, *J*=7 Hz, 2 H) 4.35 - 4.61 (m, 2 H) 7.84 (s, I H) 9.76 (s, 1 H); MS (DCI/$NH_3$) m/z 231 $(M+H)^+$.

Example 7B

ethyl (2Z)-3-(2-methoxyethyl)-2-{[(2,2,3,3-tetramethylcylopropyl)carbonyl]imino}-2,3-dihydro-1,3-thiazole-4-carboxylate

The product of Example 7A (2.3 g, 10 mmol) and 2,2,3,3-tetramethylcyclopropane carboxylic acid (1.6 g, 11 mmol) were processed using the method described in Example 2B to afford 1.9 g (53 %) of the title compound. $^1$H NMR (300 MHz, DMSO-$d_6$) δ ppm 1.11 - 1.36 (m, 12 H) 1.53 (s, 1 H) 3.21 (s, 3 H) 3.31 (s, 3 H) 3.53 - 3.62 (m, 2 H) 4.30 (q, *J*=7 Hz, 2 H) 4.62 - 4.75 (m, 2 H) 7.77 (s, 1 H); MS (DCI/$NH_3$) m/z 355 (M+H); Anal. Calculated for $C_{17}H_{16}N_2O_4S$: C, 57.61; H, 7.39; N, 7.86. Found: C, 57.86; H, 7.67 N, 7.85.

Example 8

N-[(2Z)-4-(hydroxmethyl)-3-(2-methoxyethyl-1,3-thiazol-2(3H)-ylidene]-2,2,3,3-tetramethyl cyclopropanecarboxamide

To a solution of the product of Example 7B (0.355 g, 1.00 mmol) in 100 mL of THF at 0 °C was added lithium borohydride (10 mL of a 2.0 M solution in THF) and the resulting solution was allowed to warm to ambient temperature and stirred overnight. The mixture was quenched with water and then diluted with saturated aqueous $Na_2CO_3$ and extracted twice with ethyl acetate. The combined organic extracts were dried over anhydrous $Na_2SO_4$, filtered, and concentrated. Purification by column chromatography ($SiO_2$, 0-50% ethyl acetate/hexanes gradient) afforded 0.278 g (89%) of the title compound. $^1$H NMR (300 MHz, DMSO-$d_6$) δ ppm 1.20 (d, 12 H) 1.47 (s, 1 H) 3.24 (s, 3 H) 3.64 (t, *J*=6 Hz, 2 H) 4.30 (t, *J*=6 Hz, 2 H) 4.50 (d, *J*=5 Hz, 2 H) 5.75 (s, 1 H) 6.68 (s, 1 H); MS (DCI/NH3) m/z 313$(M+H)^+$; Anal. Calculated for $C_{15}H_{24}N_2O_3S$•$0.2H_2O$: C, 57.01; H, 7.78; N, 8.86. Found: C, 56.90; H, 7.61; N, 8.86.

Example 9

2-ethoxy-N-[(2Z)-3-(2-methoxyethyl)-4-(trifluoromethyl)-1,3-thiazol-2(3H)-ylidene]benzamide

Example 9A

2-ethoxy-*N*-(4-trifluoromethyl-thiazol-2-yl)-benzamide

A solution of 2-ethoxybenzoic acid (0.75 g, 4.5 mmol) in 23 mL of methylene chloride at 0 °C was treated with oxalyl chloride (0.44 mL, 4.9 mmol) followed by 2 drops of dimethylformamide. The solution was stirred at ambient temperature for 1 hour and then concentrated under reduced pressure to provide 0.83 g of 2-ethoxybenzoyl chloride. To a solution of 4-trifluromethylthiazol-2-ylamine (0.50 g, 3.0 mmol) in 10 mL THF at 0 °C was added a solution of the freshly prepared acid chloride in 5 mL of THF and 2 mL of methylene chloride, followed by triethylamine (1.0 mL, 6.6 mmol). The reaction mixture was wanned to 65 °C and stirred 8 hours. The mixture was diluted with ethyl acetate and washed twice with water, then brine. The organic extract was dried over magnesium sulfate, filtered, and concentrated under reduced pressure. Purification by column chromatography ($SiO_2$, 30-50% ethyl acetate/hexanes gradient) afforded 0.47 g (50%) of the title compound. $^1$H NMR ($CDCl_3$, 300 MHz) δ ppm 1.66 (t, *J*=6.95 Hz, 3 H), 4.38 (q, *J*=6.89 Hz, 2 H), 7.03 - 7.10 (m, 2 H), 7.17 (d, *J*=7.80 Hz, I H), 7.42 (s, 1 H), 8.29 (dd, *J*=7.97, 1.86 Hz, 1 H). MS (DCI/$NH_3$) m/z 317 $(M+H)^+$.

Example 9B

2-ethoxy-N-[(2Z)-3-(2-methoxyethyl)-4-(trifluoromethyl)-1,3-thiazol-2(3H)-ylidene]benzamide

The product of Example 9A (0.47 g, 1.5 mmol) and 1-bromo-2-methoxy-ethane (0.16 ml, 1.6 mmol) were processed using the method described in Example 1B. Purification by column chromatography ($SiO_2$, 30-40% ethyl acetate/hexanes gradient) afforded 0.06 g (11%) of the title compound. $^1H$ NMR (DMSO-$d_6$, 300 MHz,) $\delta$ ppm 1.24 (t, *J*=6.95 Hz, 3 H), 3.02 (s, 3 H), 3.36 - 3.63 (m, 2 H), 3.86 - 4.02 (m, I H), 4.13 (q, *J*=7.12 Hz, 2 H), 4.21 - 4.33 (m, I H), 7.08 (t, *J*=7.46 Hz, I H), 7.17 (d, *J*=8.14 Hz, 1 H), 7.40 (dd, *J*=7.46, 1.70 Hz, 1 H), 7.46 - 7.56 (m, 1 H), 8.09 (s, 1 H); MS ($DCI/NH_3$) m/z 375 $(M+H)^+$. Anal. Calculated for $C_{16}H_{17}F_3N_2O_3S$•0.2 $H_2O$: C, 50.84; H, 4.64; N, 7.41. Found: C, 50.62; H, 4.35; N, 7.61.

Example 10

N-[(2Z)-3-(2-methoxyethyl)-5-methyl-1,3-thiazol-2(3H)-ylidene]-2,2,3,3-tetramethylcyclopronanecarboxamide

Example 10A

3-(2-Methoxyethyl)-5-methyl-3H-thiazol-2-ylideneamine hydrobromide

A mixture of 5-methyl-thiazol-2-ylamine (1.0 g, 8.8 mmol) and 2-bromoethyl methyl ether (1.0 mL, 11 mmol) were heated at 85 °C for 16 hours. The mixture was cooled to ambient temperature, triturated with ethanol and the solid was collected by filtration to afford 0.90 g (40%) of the title compound. $^1H$ NMR ($CDCl_3$, 300 MHz) $\delta$ ppm 2.25 (d, *J*=1.4 Hz, 3 H), 3.36 (s, 3 H), 3.72-3.81 (m, 2 H), 4.36-4.43 (m, 2 H), 6.61 (d, *J*=1.7 Hz, I H), 9.54 (s, 1 H); MS ($DCI/NH_3$) m/z 173 $(M+H)^+$.

Example 10B

N-[(2Z)-3-(2-methoxyethyl)-5-methyl-1,3-thiazol-2(3H)-ylidene]-2,2,3,3-tetramethylcyclopropanecarboxamide

The product of Example 10A (0.40 g, 1.6 mmol) and 2,2,3,3-tetramethylcyclopropanecarboxylic acid (0.25 g, 1.8 mmol) were processed using the method described in Example 2B. Purification by column chromatography ($SiO_2$, 20-40% ethyl acetate/hexanes gradient) afforded 0.30 g (63%) of the title compound. $^1H$ NMR ($CDCl_3$, 300 MHz) $\delta$ ppm 1.21 (s, 6 H), 1.33 (s, 6 H), 1.52 (s, 1 H), 2.22 (s, 3 H), 3.35 (s, 3 H), 3.68 (t, *J*=5.09 Hz, 2 H), 4.24 (t, *J*=4.92 Hz, 2 H), 6.67 (s, I H); MS ($DCI/NH_3$) m/z 297 $(M+H)^+$. Anal. Calculated for $C_{15}H_{14}N_2O_2S$: C, 60.78; H, 8.16; N, 9.45. Found: C, 60.69; H, 8.31; N, 9.19.

Example 11

2-ethoxy-N-[(2Z)-3-(2-methoxyethyl)-5-methyl-1,3-thiazol-2(3H)-ylidene]benzamide

To the product of Example 10A (0.55 g, 2.2 mmnol) and 2-ethoxybenzoyl chloride (0.33 g, 1.8 mmol) in 10 mL of THF at 0 °C was added triethylamine (0.55 mL, 4.0 mmol). The solution was stirred at 65 °C for 4 hours then allowed to cool to ambient temperature and diluted with ethyl acetate. The solution was washed twice with water and then brine. The combined aqueous washings were extracted with ethyl acetate. The combined organic extracts were dried over magnesium sulfate, filtered, and concentrated under reduced pressure. Purification by column chromatography ($Sio_2$, 20-30% ethyl acetate/hexanes gradient) afforded 0.28 g (42%) of the title compound. $^1H$ NMR (DMSO-$d_6$, 300 MHz) $\delta$ ppm 1.31 (t, *J*=7.0 Hz, 3 H), 2.26 (d, *J*=1.4 Hz, 3 H), 3.25 (s, 3 H), 3.69 (t, *J*=5.3 Hz, 2 H), 4.05 (q, *J*=7.1 Hz, 2 H), 4.30 (t, *J*=5.3 Hz, 2 H), 6.95 (t, *J*=7.5 Hz, I H), 7.04 (d, *J*=7.8 Hz, 1 H), 7.20 (d, *J*=1.4 Hz, I H), 7.32-7.41 (m, I H), 7.68 (dd, *J*=7.6, 1.9 Hz, I H); MS ($DCI/NH_3$) m/z 321 $(M+H)^+$. Anal. Calculated for $C_{16}H_{20}N_2O_3S$•0.2 $H_2O$: C, 59.31; H, 6.35; N, 8.65. Found: C, 59.18; H, 6.02; N, 8.29.

Example 12

3-fluoro-N-[(2Z)-3-(2-methoxyethyl)-4,5-dimethyl-1,3-thiazol-2(3H)-ylidene]-2-methylbenzamide

Example 12A

3-(2-Methoxyethyl)-4,5-dimethyl-3H-thiazol-2-ylideneamine hydrobromide

A mixture of 4,5-dimethylthiazol-2-ylamine (9.0 g, 70 mmol) and 2-bromoethyl methyl ether (7.9 mL, 84 mmol) were heated at 85 °C for 12 hours. The mixture was cooled to ambient temperature and then triturated with isopropanol. The solid was collected by filtration and dried under vacuum to provide 10 g (56%) of the title compound. $^1$H NMR (DMSO-$d_6$, 300 MHz) δ ppm 2.17 (s, 3 H), 2.19 (s, 3 H), 3.25 (s, 3 H) 3.56 (t, *J*=5.1 Hz, 2 H) 4.16 (t, *J*=5.1 Hz, 2 H) 9.41 (s, I H); MS (DCI/$NH_3$) m/z 129 $(M+H)^+$.

Example 12B

3-fluoro-N-[(2Z)-3-(2-methoxyethyl)-4,5-dimethyl-1,3-thiazol-2(3H)-ylidene)-2-methylbenzamide

The product of Example 12A (39 mg, 0.15 mmol) and 3-fluoro-2-methylbenzoic acid (31 mg, 0.22 mmol) were processed using the method described in Example 3 to afford the title compound. $^1$H NMR (500 MHz, DMSO-$d_6$) δ ppm 2.21 - 2.24 (m, 3 H), 2.26 - 2.28 (m, 3 H), 2.44 - 2.47 (m, 3 H), 3.24 (s, 3 H), 3.66 - 3.71 (m, 2 H), 4.35 (t, 2 H), 7.21 - 7.31 (m, 2 H), 7.76 (d, I H); MS (ESI)m/z 324 $(M+H)^+$.

Example 13

5-fluor-N-[(2Z)-3-(2-methoxyethyl)-4,5-dimethyl-1,3-thiazol-2(3H)-ylidene]-2-methylbenzamide

Example 13A

5-Fluoro-2-methyl-benzoyl chloride

A solution of 5-fluoro-2-methylbenzoic acid (380 mg, 2.47 mmol) in thionyl chloride (5 mL) was heated to reflux for 3 hours. The solution was cooled to ambient temperature and the volatile components were removed under reduced pressure. The residue was dissolved in fresh toluene (10 mL) and concentrated under reduced pressure twice and then placed under high vacuum to afford the title compound (420 mg).

Example 13B

5-fluoro-N-[(2Z)-3-(2-methoxyethyl)-4,5-dimethyl-1,3-thiazol-2(3H)-ylidene]-2-methylbenzamide

To a suspension of the product of Example 12A (549 mg, 2.05 mmol) and triethylamine (0.859 mL, 6.16 mmol) in THF (6 mL) were added a solution of the product from Example 13A in THF (2 mL). The mixture was heated at reflux for 14 hours, then cooled to ambient temperature and diluted with water and $CH_2Cl_2$. The phases were separated and the organic extract was washed with water and brine, dried ($Na_2SO_4$) and concentrated. The residue was dissolved in warm EtOAc (10 mL) and allowed to stand at room temperature for 14 hours. The crystals were isolated by filtration (EtOAc wash) to afford the title compound (450 mg, 68%). MS (ESI) m/z 324 $(M+H)^+$.

Example 14

3-methoxy-N-[(2Z)-3-(2-methoxyethyl)-4,5-dimethyl-1,3-thiazol-2(3H)-ylidene]-4-methylbenzamide

The product of Example 12A (39 mg, 0.15 mmol) and 3-methoxy-4-methylbenzoic acid (37 mg, 0.22 mmol) were processed using the method described in Example 3 to afford the title compound. $^1$H NMR (500 MHz, DMSO-$d_6$) δ ppm 2.21 (d, 6 H), 2.26 (s, 3 H), 3.26 (s, 3 H), 3.74 (t, 2 H), 3.85 (s, 3 H), 4.40 (t, 2 H), 7.22 (d, 1 H), 7.68 - 7.72 (m, 2 H); MS (ESI) m/z 335 $(M+H)^+$.

Example 15

2-ethoxy-N-[(2Z)-3-(2-methoxyethyl)-4,5-dimethyl-1,3-thiazol-2(3H)-ylidene]benzamide

The product of Example 12A (0.35 g, 1.3 mmol) and 2-ethoxybenzoic acid (0.43 g, 2.6 mmol) were processed using the methods described in Example 13. Purification by column chromatography ($SiO_2$, 30-50% ethyl acetate/ hexanes gradient) afforded 0.078 g (18%) of the title compound $^1H$ NMR ($CDCl_3$, 300 MHz) $\delta$ ppm 1.46 (t, *J*=7.0 Hz, 3 H), 2.18-2.31 (n, 6 H), 3.30 (s, 3 H), 3.78 (t, *J*=5.3 Hz, 2 H), 4.17 (d, *J*=7.1 Hz, 2 H), 4.37 (s, 2 H), 6.89-7.04 (m, 2 H), 7.36 (t, *J*=7.6 Hz, 1 H), 7.96 (dd, *J*=7.8, 1.7 Hz, 1 H); MS ($DCI/NH_3$) m/z 335 $(M+H)^+$. Anal. Calculated for $C_{17}H_{22}N_2O_3S \cdot 0.1 H_2O$: C, 60.73; H, 6.65; N, 8.33. Found: C, 60.37; H, 6.42; N, 8.31.

Example 16

N-[(2Z)-3-(2-methoxyethyl)-4,5-dimethyl-1,3-thiazol-2(3H)-ylidene]-1-naphthamide

The product of Example 12A (39 mg, 0.15 mmol) and 1-naphthoic acid (39 mg, 0.22 mmol) were processed using the method described in Example 3 to afford the title compound. $^1H$ NMR (500 MHz, DMSO-$d_6$) $\delta$ ppm 2.25 (s, 3 H), 2.28 (s, 3 H), 3.26 (s, 3 H), 3.72 - 3.77 (m, 2 H), 4.37 - 4.43 (m, 2 H), 7.52 - 7.60 (m, 3 H), 7.95 - 7.99 (m, 1 H), 8.02 - 8.06 (m, I H), 8.28 - 8.31 (m, 1 H), 9.03 - 9.07 (m, 1H); MS (ESI) m/z 341 $(M+H)^+$.

Example 17

N-[(2Z)-3-(2-methoxyethyl)-4,5-dimethyl-1,3-thiazol-2(3H)-ylidene]-2-naphthamide

The product of Example 12A (39 mg, 0.15 mmol) and 2-napthoic acid (39 mg, 0.22 mmol) were processed using the method described in Example 3 to afford the title compound. $^1H$ NMR (500 MHz, DMSO-$d_6$) $\delta$ ppm 2.24 (s, 3 H) 2.29 (s, 3 H) 3.28 (s, 3 H) 3.80 (t, 2 H) 4.49 (t, 2 H) 7.55 - 7.62 (m, 2 H) 7.95 - 7.99 (m, 2 H) 8.08 (d, I H) 8.26 - 8.29 (m, 1H) 8.76 (s, I H); MS (ESI) m/z 341 $(M+H)^+$.

Example 18

5-chloro-2-methoxy-N-[(2Z)-3-(2-methoxyethyl)-4,5-dimethyl-1,3-thiazol-2(3H)-ylidenelbenzamide

The product of Example 12A (39 mg, 0.15 mmol) and 5-chloro-2-methoxybenzoic acid (41 mg, 0.22 mmol) were processed using the methods described in Example 13 to afford the title compound. MS (ESI) m/z 355 $(M+H)^+$.

Example 19

1-hydroxy-N-[(2Z)-3-(2-methoxyethyl)-4,5-dimethyl-1,3-thiazol-2(3H)-ylidene]-2-naphthamide

The product of Example 12A (39 mg, 0.15 mmol) and 1-hydroxy-2-naphthoic acid (41 mg, 0.22 mmol) were processed using the methods described in Example 13 to afford the title compound. $^1H$ NMR (500 MHz, DMSO-$d_6$) $\delta$ ppm 2.27 (s, 3 H), 2.31 (s, 3H), 3.28 (s, 3 H), 3.80 (t, 2 H), 4.44 (t, 2 H), 7.36 (d, 1 H), 7.53 (t, 1H), 7.61 (t, 1H), 7.86 (d, 1 H), 8.06 (d, 1H), 8.28 (d, 1 H), 14.38 (s, 1 H); MS (ESI) m/z 357 $(M+H)^+$.

Example 20

4-fluoro-N-[(2Z)-3-(2-methoxyethyl)-4,5-dimethyl-1,3-thiazol-2(3H)-ylidene]-1-naphthamide

The product of Example 12A (39 mg, 0.15 mmol) and 4-fluoro-1-naphthoic acid (42 mg, 0.22 mmol) were processed using the method described in Example 3 to afford the title compound. $^1H$ NMR (500 MHz, DMSO-$d_6$) $\delta$ ppm 2.25 (s, 3 H) 2.28 (s, 3 H) 3.26 (s, 3 H) 3.74 (t, 2 H) 4.41 (t, 2 H) 7.38 - 7.44 (m, 1 H) 7.65 - 7.72 (m, 2 H) 8.12 (d, I H) 8.37 - 8.41 (m, 1 H) 9.23 (d, 1 H) MS (ESI) m/z 359 $(M+H)^+$.

Example 21

2-methoxy-N-[(2Z)-3-(2-methoxyethyl)-4,5-dimethyl-1,3-thiazol-2(3H)-ylidene]-4-(methylthio)benzamide

The product of Example 12A (39 mg, 0.15 mmol) and 2-methoxy-4-methylsulfanylbenzoic acid (44 mg, 0.22 mmol) were processed using the method described in Example 3 to afford the title compound. $^1$H NMR (500 MHz, DMSO-$d_6$) δ ppm 2.20 (s, 3 H) 2.23 (s, 3 H) 2.53 (s, 3 H) 3.24 (s, 3 H) 3.68 (t, 2 H) 3.81 (s, 3 H) 4.29 (t, 2 H) 6.83 - 6.87 (m, 1 H) 6.87 - 6.90 (m, I H) 7.75 (d, I H) MS (ESI) m/z 367 (M+H)$^+$.

Example 22

2-chloro-N-[(2Z)-3-(2-methoxyethyl)-4,5-dimethyl-1,3-thiazol-2(3H)-ylidene]-5-(methylthio)benzamide

The product of Example 12A (39 mg, 0.15 mmol) and 2-chloro-5-methylsulfanylbenzoic acid (44 mg, 0.22 mmol) were processed using the method described in Example 3 to afford the title compound. $^1$H NMR (500 MHz, DMSO-$d_6$) δ ppm 2.23 (s, 3 H) 2.27 (s, 3 H) 3.24 (s, 3 H) 3.29 (s, 3 H) 3.69 (t, 2 H) 4.33 (t, 2 H) 7.30 - 7.33 (m, I H) 7.39 - 7.42 (m, 1 H) 7.67 (d, I H) MS (ESI) m/z 371 (M+H)$^+$.

Example 23

N-[(2Z)-3-(2-methoxyethyl)-4,5-dimethyl-1,3-thiazol-2(3H)-ylidene]-4-(trifluoromethyl)nicotinamide

The product of Example 12A (0.30 g, 1.1 mmol) and 4-trifluoromethylnicotinic acid (0.43 g, 2.2 mmol) were processed as in the methods of Example 13. Purification by column chromatography ($SiO_2$, 0-20% methanol/methylene chloride gradient) afforded 0.23 g (28%) of the title compound. $^1$H NMR ($CDCl_3$, 300 MHz) δ ppm 2.26 (s, 3 H), 2.29 (s, 3 H), 3.30 (s, 3 H), 3.72 (t, *J*=5.1 Hz, 2 H), 4.33 (t, *J*=5.1 Hz, 2 H), 7.59 (d, *J*=5.1 Hz, 1 H), 8.79 (d, *J*=5.1 Hz, 1 H), 9.23 (s, 1 H); MS (DCI/$NH_3$) m/z 360 (M+H)$^+$. Anal. Calculated for $C_{15}H_{16}F_3N_3O_2S$: C, 50.13; H, 4.49; N, 11.69. Found: C, 50.12; H, 4.33; N, 11.75.

Example 24

2-hydroxy-N-[(2Z)-3-(2-methoxyethyl)-4,5-dimethyl-1,3-thiazol-2(3H)-ylidene]benzamide

The product of Example 12A (1.7 g, 9.4 mmol) and 2-hydroxybenzoic acid ( 1.6 g, 11 mmol) were processed using the method described in Example 2B. Purification by column chromatography ($SiO_2$, 20-40% ethyl acetate/hexanes gradient) afforded 0.91 g (32%) of the title compound. $^1$H NMR ($CDCl_3$, 300 MHz) δ ppm 2.26 (d, *J*=1.0 Hz, 3 H), 2.29 (d, *J*=0.7 Hz, 3 H), 3.31 (s, 3 H), 3.78-3.86 (m, 2 H), 4.34 (t, *J*=5.1 Hz, 2 H), 6.89 (dt, *J*=7.9, 7.0, 1.0 Hz, 1 H), 6.95 (dd, *J*=8.1, 1.0 Hz, 1 H), 7.37 (dt, *J*=7.7, 1.9 Hz, 1 H), 8.15 (dd, *J*=8.0, 1.9 Hz, 1 H); MS (DCI/$NH_3$) m/z 307 (M+H)$^+$. Anal. Calculated for $C_{15}H_{18}N_2O_3S$: C, 58.80; H, 5.92; N, 9.14. Found: C, 58.60; H, 5.86; N, 9.01.

Example 25

2-(2-methoxyethoxy)-N-[(2Z)-3-(2-methoxyethyl)-4,5-dimethyl-1,3-thiazol-2(3H)-ylidene]benzamide

Example 25A

Methyl-2-(2-Methoxy-ethoxy)-benzoate

To a solution of triphenylphosphine (0.36 g, 1.40 mmol) in 10 mL of THF at 0 °C was added diisopropyl azodicarboxylate (275 μL, 1.40 mmol). The mixture was stirred for 0.5 hours and then methyl-2-hydroxybenzoate (400 mg, 1.3 mmol) and 2-methoxyethanol (110 μL, 1.40 mmol) were added. The mixture was allowed to wann to ambient temperature and stirred for 16 hours. The mixture was concentrated under reduced pressure and the residue was used without purification. MS (DCI/$NH_3$) m/z 211 (M+H)$^+$.

Example 25B

2-(2-Methoxy-ethoxy)-benzoic acid

A mixture of the product of Example 25A (0.27 g, 1.3 mmol) in 40% aqueous potassium hydroxide was stirred for 6 hours. The mixture was then diluted with water, made slightly acidic by the addition of 2 N aqueous HCl, and then extracted three times with ethyl acetate. The combined organic extracts were dried over magnesium sulfate, filtered, and concentrated under reduced pressure to afford 0.25 g of the title compound.

Example 25C

2-(2-Methoxy-ethoxy)-*N*-[3-(2-methoxyethyl)-4,5-dimethyl-3*H*-thiazol-2-ylidene]-benzamide

The product of Example 25B (0.25 g, 1.3 mmol) and the product of Example 12A (0.28 g, 1.5 mmol) were processed using the methods described in Example 13. Purification by column chromatography ($SiO_2$, 30-60% ethyl acetate/hexanes gradient) afforded 35 mg (7%) of the title compound. MS ($DCI/NH_3$) m/z 365 $(M+H)^+$. Anal. Calculated for $C_{18}H_{14}N_1O_4S{\cdot}0.5\ H_2O$: C, 57.89; H, 6.75; N, 7.50. Found: C, 57.77; H, 6.59; N, 7.44.

Example 26

5-chloro-2-ethoxy-N-[(2Z)-3-(2-methoxyethyl)-4,5-dimethyl-1,3-thiazol-2(3H)-ylidene]benzamide

The product of Example 12A (97 mg, 0.52 mmol) and 5-chloro-2-ethoxybenzoic acid (95 mg, 0.47 mmol) were processed using the methods described in Example 13 to afford the title compound. $^1H$ NMR ($CDCl_3$, 400 MHz) $\delta$ ppm 1.45 (t, *J*=6.9 Hz, 3 H), 2.24 (s, 3 H), 2.28 (s, 3 H), 3.31 (s, 3H), 3.78 (t, *J*=4.8 Hz, 2 H), 4.15 (q, *J*=7.1 Hz, 2 H), 4.45 (s, 2 H), 6.90 (d, *J*=8.6 Hz, 1 H), 7.30 (dd, *J*=8.9, 2.8 Hz, 1 H), 7.89 (d, *J*=2.8 Hz, 1 H); MS ($DCI/NH_3$) m/z 369 $(M+H)^+$. Anal. Calculated for $C_{17}H_{21}ClN_2O_3S$: C, 55.35; H, 5.74; N, 7.59. Found: C, 55.13; H, 5.59; N, 7.54.

Example 27

2-ethoxy-N-[(2Z)-3-(2-methoxyethyl)-4,5-dimethyl-1,3-thiazol-2(3H)-ylidene]nicotinamide

The product of Example 12A (0.40 g, 2.2 mmol) and 2-ethoxynicotinic acid (0.40 g, 2.4 mmol) were processed using the method described in Example 2B. Purification by column chromatography ($SiO_2$, 0-30% methano)/methylene chloride gradient) afforded 0.34 g (45%) of the title compound. $^1H$ NMR ($CDCl_3$, 300 MHz) $\delta$ ppm 1.45 (t, *J*=7.1 Hz, 3 H), 2.23 (s, 3 H), 2.26 (s, 3 H), 3.31 (s, 3 H), 3.78 (t, *J*=5.3 Hz, 2 H), 4.37 (d, *J*=4.7 Hz, 2 H), 4.52 (q, *J*=7.0 Hz, 2 H), 6.91 (dd, *J*=7.5, 4.7 Hz, 1 H), 8.21 (dd, *J*=4.7, 2.0 Hz, I H), 8.32 (dd, *J*=7.5, 2.0 Hz, 1 H); MS ($DCI/NH_3$) m/z 336 $(M+H)^+$. Anal. Calculated for $C_{16}H_{21}N_3O_3S{\cdot}0.2H_2O$: C, 56.68; H, 6.36; N, 12.39. Found: C, 56.65; H, 6.32; N, 12.38.

Example 28

2-chloro-N-[(2Z)-3-(2-methoxyethyl)-4,5-dimethyl-1,3-thiazol-2(3H)-ylidene]nicotinamide

A mixture of 2-chloronicotinic acid (0.42 g, 2.7 mmol) and 1,1'-carbonyldiimidazole (0.43 g, 2.7 mmol) in 3 mL of ethyl acetate was stirred at ambient temperature for 4 hours. The mixture was treated with water (3 mL) and the product of Example 12A (0.45 g, 2.4 mmol) and then heated at 65 °C for 13 hours. The mixture was cooled to ambient temperature, diluted with ethyl acetate and the layers separated. The organic phase was washed with twice with water and then brine, dried over magnesium sulfate, filtered and concentrated under reduced pressure. The residue was recrystallized from methylene chloride and afforded 0.14 g (18%) of the title compound. $^1H$ NMR ($CDCl_3$, 300 MHz) $\delta$ ppm 2.26 (s, 3 H), 2.28 (s, 3 H), 3.30 (s, 3 H), 3.76 (t, *J*=5.1 Hz, 2 H), 4.36 (t, *J*=5.1 Hz, 2 H), 7.28-7.32 (m, 1 H), 8.28 (dd, *J*=7.5, 2.0 Hz, 1 H), 8.42 (dd, *J*=4.7, 2.0 Hz, 1H); MS ($DCI/NH_3$) m/z 326 $(M+H)^+$. Anal. Calculated for $C_{14}H_{16}ClN_3O_2S$: C, 51.61; H, 4.95; N, 12.90. Found: C, 51.57; H, 4.76; N, 12.74.

Example 29

N-[(2Z)-3-(2-methoxyethyl)-4,5-dimethyl-1,3-thiazol-2(3H)-ylidene]-2-(trifluoromethoxy)benzamide

A mixture of 2-trifluoromethoxybenzoyl chloride (0.59 g, 2.6 mmol) and the product of Example 12A were

processed using the method described in Example 11 to afford the title compound. $^1$H NMR ($CDCl_3$, 300 MHz) δ ppm 2.24 (s, 3 H), 2.28 (s, 3 H), 3.29 (s, 3 H), 3.76 (t, *J*=4.9 Hz, 2 H), 4.45 (t, *J*=4.7 Hz, 2 H), 7.27-7.39 (m, 2 H), 7.46 (td, *J*=7.7, 1.9 Hz, 1 H), 8.06 (dd, *J*=7.6, 1.9 Hz, 1 H); Anal. Calculated for $C_{16}H_{17}F_3N_2O_3S$: C, 51.33; H, 4.58; N, 7.48. Found: C, 51.29; H, 4.40; N, 7.37.

Example 30

5-bromo-2-ethoxy-N-[(2Z)-3-(2-methoxyethyl)-4,5-dimethyl-1,3-thiazol-2(3H)-ylidene]benzamide

The product of Example 12A (0.20 g, 1.1 mmol) and 5-bromo-2-ethoxybenzoyl chloride (0.28 g, 1.1 mmol) were processed using the method described in Example 11. Purification by column chromatography ($SiO_2$, 30-60% ethyl acetate/hexanes gradient) afforded 149 mg (38%) of the title compound. $^1$H NMR ($CDCl_3$, 300 MHz) δ ppm 1.46 (t, *J*=7.0 Hz, 3 H), 2.25 (s, 3 H), 2.29 (s, 3 H), 3.31 (s, 3 H), 3.79 (t, *J*=4.6 Hz, 2 H), 4.09-4.23 (m, 2 H), 4.44-4.61 (m, 2H), 6.85 (d, *J*=8.8 Hz, 1 H), 7.42-7.48 (m, 1H), 8.02 (d, *J*=2.7 Hz, I H); MS (DCI/$NH_3$) m/z 415 $(M+H)^+$. Anal. Calculated for $C_{17}H_{21}BrN_2O_3S$: C, 49.40; H, 5.12; N, 6.78. Found: C, 49.68; H, 5.03; N, 6.71.

Example 31

N-[(2Z)-3-(2-methoxyethyl)-4,5-dimethyl-1,3-thiazol-2(3H)-ylidene]-2-(trifluoromethyl)benzamide

The product of Example 12A (0.50 g, 2.7 mmol) and 2-trifluoromethylbenzoyl chloride (0.62 g, 3.0 mmol) were processed using the method described in Example 11. Purification by column chromatography ($SiO_2$, 20-40% ethyl acetate/hexanes gradient) afforded 0.43 g (44%) of the title compound. $^1$H NMR ($CDCl_3$, 300 MHz) δ ppm 2.26 (s, 3 H), 2.28 (s, 3 H), 3.29 (s, 3 H), 3.72 (t, *J*=4.7 Hz, 2 H), 4.41 (t, *J*=4.6 Hz, 2 H), 7.45-7.62 (m, 2H), 7.71 (d, *J*=7.1 Hz, 1 H), 7.86 (d, *J*=7.1 Hz, I H); MS (DCI/$NH_3$) m/z 359 $(M+H)^+$. Anal. Calculated for $C_{16}H_{17}F_3N_2O_2S$: C, 53.62; H, 4.78; N, 7.82. Found: C, 53.58; H, 4.51; N, 7.70.

Example 32

2-iodo-N-[(2Z)-3-(2-methoxyethyl)-4,5-dimethyl-1,3-thiazol-2(3H)-ylidene]benzamide

The product of Example 12A (0.25 g, 1.3 mmol) and 2-iodobenzoyl chloride (0.37 g, 1.4 mmol) were processed using the method described in Example 11. Purification by preparative HPLC on a Waters Symmetry C8 column (40 mm X 100 mm, 7 μm particle size) using a gradient of 20% to 95% acetonitrile:0.1% aqueous TFA over 12 minutes (15 minutes run time) at a flow rate of 70 mL/min afforded 0.12 g (23%) of the title compound. $^1$H NMR ($CDCl_3$, 300 MHz) δ ppm 2.30 (s, 3 H), 2.31 (s, 3 H), 3.29 (s, 3 H), 3.72 (t, *J*=4.7 Hz, 2 H), 4.41 (t, *J*=4.6 Hz, 2 H), 7.45-7.62 (m, 2 H), 7.71 (d, *J*=7.1 Hz, 1 H), 7.86 (d, *J*=7.1 Hz, 1 H); MS (DCI/$NH_3$) m/z 417 $(M+H)^+$.

Example 33

2-fluoro-N-[(2Z)-3-(2-methoxyethyl)-4,5-dimethyl-1,3-thiazol-2(3H)-ylidene]-5-(trifluoromethyl)benzamide

The product of Example 12A (0.25 g, 1.3 mmol) and 2-fluoro-5-trifluoromethylbenzoyl chloride (0.32 g, 1.4 mmol) were processed using the method described in Example 11. Purification by preparative HPLC on a Waters Symmetry C8 column (40 mm X 100 mm, 7 μm particle size) using a gradient of 20% to 95% acetonitrile:0.1% aqueous TFA over 12 minutes (15 minutes run time) at a flow rate of 70 mL/min afforded 70 mg (14%) of the title compound. $^1$H NMR ($CDCl_3$, 300 MHz) δ ppm 2.29 (s, 3 H), 2.31 (s, 3 H), 3.33 (s, 3 H), 3.73-3.89 (m, 2 H), 4.44-4.57 (m, 2 H), 7.18-7.24 (m, I H), 7.68 (d, *J*=9.2 Hz, 1 H), 8.38 (d, *J*=6.8 Hz, 1H); MS (DCI/$NH_3$) m/z 377 $(M+H)^+$. Anal. Calculated for $C_{16}H_{16}F_4N_2O_2S\cdot0.1H_2O$: C, 51.06; H, 4.28; N, 7.44. Found: C, 50.54; H, 4.05; N, 7.27.

Example 34

2-bromo-5-methoxy-N-[(2Z)-3-(2-methoxyethyl)-4,5-dimethyl-1,3-thiazol-2(3H)-ylidene]benzamide

The product of Example 12A (0.20 g, 1.1 mmol) and 2-bromo-5-methoxybenzoic acid (0.25 g, 1.1 mmol) were processed using the methods described in Example 13. Purification by preparative HPLC on a Waters Symmetry C8 column (40 mm X 100 mm, 7 μm particle size) using a gradient of 20% to 95% acetonitrile:0.1% aqueous TFA over 12 minutes (15 minutes run time) at a flow rate of 70 mL/min afforded 0.13 g (29%) of the title compound. $^1$H NMR ($CDCl_3$,

300 MHz) δ ppm 2.27 (s, 3 H), 2.29 (s, 3 H), 3.30 (s, 3 H), 3.80 (t, *J*=4.7 Hz, 2 H), 3.83 (s, 3 H), 4.50-4.59 (m, 2 H), 6.82 (dd, *J*=8.8, 3.1 Hz, 1 H), 7.44 (d, *J*=3.1 Hz, I H), 7.50 (d, *J*=8.8 Hz, 1 H); MS (DCI/$NH_3$) m/z 401 $(M+H)^+$. Anal. Calculated for $C_{16}H_{19}BrN_2O_3S$: C, 48.13; H, 4.80; N, 7.02. Found: C, 47.88; H, 4.55; N, 6.89.

Example 35

5-fluoro-N-[(2Z)-3-(2-methoxyethyl)-4,5-dimethyl-1,3-thiazol-2(3H)-ylidene]-2-(trifluoromethyl)benzamide

The product of Example 12A (0.20 g, 1.1 mmol) and 5-fluoro-2-trifluoromethylbenzoyl chloride (0.18 mL, 1.2 mmol) were processed using the method described in Example 11. Purification by preparative HPLC on a Waters Symmetry C8 column (40 mm X 100 mm, 7 μm particle size) using a gradient of 20% to 95% acetonitrile:0.1% aqueous TFA over 12 minutes (15 minutes run time) at a flow rate of 70 mL/min afforded 43 mg (11%) of the title compound. $^1H$ NMR ($CDCl_3$, 300 MHz) δ ppm 2.27 (s, 3 H), 2.29 (s, 3 H), 3.30 (s, 3 H), 3.65-3.76 (m, 2 H), 4.37-4.48 (m, 2 H), 7.13-7.20 (m, 1 H), 7.56 (d, *J*=7.5 Hz, I H), 7.71 (dd, *J*=9.2, 5.4 Hz, 1 H); MS (DCI/$NH_3$) m/z 377 $(M+H)^+$. Anal. Calculated for $C_{16}H_{16}F_4N_2O_2S\cdot 0.3H_2O$: C, 50.34; H, 4.38; N, 7.34. Found: C, 49.95; H, 4.02; N, 7.09.

Example 36

N-[(2Z)-3-(2-methoxyethyl)-4,5-dimethyl-1,3-thiazol-2(3H)-ylidene]-2,5-bis(trifluoromethyl)benzamide

The product of Example 12A (0.20 g, 1.1 mmol) and 2,5-bis-trifluoromethylbenzoyl chloride (0.33 g, 1.2 mmol) were processed using the method described in Example 11. Purification by preparative HPLC on a Waters Symmetry C8 column (40 mm X 100 mm, 7 μm particle size) using a gradient of 20% to 95% acetonitrile:0.1% aqueous TFA over 12 minutes (15 minutes run time) at a flow rate of 70 mL/min afforded 0.14 g (31%) of the title compound. $^1H$ NMR ($CDCl_3$, 300 MHz) δ ppm 2.27 (s, 3 H), 2.29 (s, 3 H), 3.30 (s, 3 H) 3.71 (t, *J*=4.9 Hz, 2 H) 4.36 (t, *J*=5.1 Hz, 2 H) 7.72-7.77 (m, 1 H) 7.82-7.87 (m, I H) 8.15 (s, 1 H); MS (DCI/$NH_3$) m/z 427 $(M+H)^+$. Anal. Calculated for $C_{17}H_{16}F_6N_2O_2S$: C, 47.89; H, 3.78; N, 6.57. Found: C, 47.49; H, 3.42; N, 6.38.

Example 37

2-fluoro-N-[(2Z)-3-(2-methoxyethyl)-4,5-dimethyl-1,3-thiazol-2(3H)-ylidene]-6-(trifluoromethyl)benzamide

The product of Example 12A (0.20 g, 1.1 mmol) and 2-fluoro-6-trifluoromethylbenzoyl chloride (0.17 mL, 1.2 mmol) were processed using the method described in Example 11. Purification by preparative HPLC on a Waters Symmetry C8 column (40 mm X 100 mm, 7 μm particle size) using a gradient of 20% to 95% acetonitrile:0.1% aqueous TFA over 12 minutes (15 minutes run time) at a flow rate of 70 mL/min afforded 0.13 g (32%) of the title compound. $^1H$ NMR ($CDCl_3$, 300 MHz) δ ppm 2.27 (s, 3 H), 2.28 (s, 3 H), 3.27 (s, 3 H), 3.69 (t, *J*=4.9 Hz, 2 H), 4.37 (t, *J*=4.6 Hz, 2 H), 7.28-7.33 (m, I H), 7.38-7.50 (m, 2 H); MS (DCI/$NH_3$) m/z 377 $(M+H)^+$. Anal. Calculated for $C_{16}H_{16}F_4N_2O_2S$: C, 51.06; H, 4.28; N, 7.44. Found: C, 50.98; H, 4.07; N, 7.36.

Example 38

2-chloro-6-fluoro-N-[(2Z)-3-(2-methoxyethyl)-4,5-dimethyl-1,3-thiazol-2(3H)-ylidene]benzamide

The product of Example 12A (0.20 g, 1.1 mmol) and 2-chloro-6-fluorobenzoyl chloride (0.23 g, 1.2 mmol) were processed using the method described in Example 11. Purification by preparative HPLC on a Waters Symmetry C8 column (40 mm X 100 mm, 7 μm particle size) using a gradient of 20% to 95% acetonitrile:0.1% aqueous TFA over 12 minutes (15 minutes run time) at a flow rate of 70 mL/min afforded 66 mg (18%) of the title compound. $^1H$ NMR ($CDCl_3$, 300 MHz) δ ppm 2.26 (s, 3 H), 2.27 (s, 3 H), 3.28 (s, 3 H), 3.73 (t, *J*=4.9 Hz, 2 H), 4.35 (t, *J*=4.7 Hz, 2 H), 6.97-7.05 (m, I H), 7.19-7.24 (m, 2 H); MS (DCI/$NH_3$) m/z 343 $(M+H)^+$. Anal. Calculated for $C_{15}H_{16}ClFN_2O_1S\cdot 0.2C_2HF_3O_2$: C, 50.59; H, 4.47; N, 7.66. Found: C, 50.70; H, 4.34; N, 7.55.

Example 39

3-fluoro-N-[(2Z)-3-(2-methoxyethyl)-4,5-dimethyl-1,3-thiazol-2(3H)-ylidene]-2-(trifluoromethyl)benzamide

The product of Example 12A (0.335 g, 1.9 mmol) and 3-fluoro-2-trifluoromethylbenzoyl chloride (0.47 g, 2.1 mmol) were processed using the method described in Example 11. Purification by preparative HPLC on a Waters

Symmetry C8 column (40 mm X 100 mm, 7 μm particle size) using a gradient of 20% to 95% acetonitrile:0.1% aqueous TFA over 12 minutes (15 minutes run time) at a flow rate of 70 mL/min afforded 0.14 g (20%) of the title compound. $^1H$ NMR ($CDCl_3$, 300 MHz) δ ppm 2.25 (d, *J*=0.7 Hz, 3 H), 2.27 (s, 3 H), 3.28 (s, 3 H), 3.68 (t, *J*=5.1 Hz, 2 H), 4.31 (t, *J*=5.1 Hz, 2 H), 7.18 (dd, *J*=11.0, 8.3 Hz, 1 H), 7.38-7.42 (m, 1 H), 7.52 (td, *J*=8.0, 5.1 Hz, 1 H); MS (DCI/$NH_3$) m/z 377 $(M+H)^+$. Anal. Calculated for $C_{16}H_{16}F_4N_2O_2S$: C, 51.06; H, 4.28; N, 7.44. Found: C, 51.15; H, 3.96; N, 7.38.

Example 40

2-chloro-5-fluoro-N-[(2Z)-3-(2-methoxyethyl)-4,5-dimethyl-1,3-thiazol-2(3H)-ylidenelbenzamide

The product of Example 12A (0.20 g, 1.1 mmol) and 2-chloro-5-fluorobenzoyl chloride (0.23 g, 1.2 mmol) were processed using the method described in Example 11. Purification by preparative HPLC on a Waters Symmetry C8 column (40 mm X 100 mm, 7 μm particle size) using a gradient of 20% to 95% acetonitrile:0.1% aqueous TFA over 12 minutes (15 minutes run time) at a flow rate of 70 mL/min afforded 17 mg (4%) of the title compound. $^1H$ NMR (DMSO-$d_6$, 500 MHz) δ ppm 2.23 (s, 3 H), 2.26 (s, 3 H), 3.22 (s, 3 H), 3.68 (t, *J*=5.3 Hz, 2 H), 4.33 (t, *J*=5.3 Hz, 2 H), 7.30 (td, *J*=8.4, 3.1 Hz, 1 H), 7.52 (dd, *J*=8.9, 5.2 Hz, 1 H), 7.64 (dd, *J*=9.1, 3.3 Hz, 1 H); MS (DCI/$NH_3$) m/z 343 $(M+H)^+$. Anal. Calculated for $C_{15}H_{16}ClFN_2O_2S \cdot 0.1C_2HF_3O_2$: C, 51.54; H, 4.58; N, 7.91. Found: C, 51.68; H, 4.35; N, 7.95.

Example 41

N-[(2Z)-3-(2-methoxyethyl)-4,5-dimethyl-1,3-thiazol-2(3H)-ylidene]-2,2,3,3-tetramethylcyclopropanecarboxamide

The product of Example 12A (1.5 g, 8.0 mmol) and 2,2,3,3-tetramethylcyclopropanecarboxylic acid (0.77 g, 5.4 mmol) were processed using the methods described in Example 13. Recrystallization from ethyl acetate afforded 0.99 g (60%) of the title compound. $^1H$ NMR (DMSO-$d_6$, 300 MHz) δ ppm 1.16 (s, 6 H), 1.23 (s, 6H), 1.44 (s, 1H), 2.13 (s, 3 H), 2.19 (s, 3 H), 3.24 (s, 3 H), 3.61 (t, *J*=5.4 Hz, 2 H), 4.21 (t, *J*=5.4 Hz, 2 H); MS (DCI/$NH_3$) m/z 311 $(M+H)^+$. Anal. Calculated for $C_{16}H_{26}N_1O_2S$: C, 61.92; H, 8.44; N, 9.02. Found: C, 61.89; H, 8.38; N, 8.81.

Examnle 42

N-[(2Z)-3-(2-methoxyethyl)-4,5-dimethyl-1,3-thiazol-2(3H)-ylidene]-2-methylpentanamide

The product of Example 12A (39 mg, 0.15 mmol) and 2-methylvaleric acid (26 mg, 0.22 mmol) were processed using the method described in Example 3 to afford the title compound. $^1H$ NMR (DMSO-$d_6$, 300 MHz) δ ppm 0.85 (t, 3 H) 1.09 (d, 3 H) 1.20 - 1.28 (m, 2 H) 1.32 - 1.40 (m, I H) 1.59-1.67 (m, I H) 2.19 (s, 3 H) 2.22 (s, 3 H) 2.52 - 2.57 (m, I H) 3.24 (s, 3 H) 3.63 (t, 2 H) 4.26 - 4.33 (m, 2 H) MS (ESI) m/z 285 $(M+H)^+$.

Example 43

N-[(2Z)-3-(2-methoxythyl)-4,5-dimethyl-1,3-thiazol-2(3H)-ylidene]-2,2-dimethylbutanamide

The product of Example 12A (39 mg, 0.15 mmol) and 2,2-dimethylbutyric acid (26 mg, 0.22 mmol) were processed using the method described in Example 3 to afford the title compound. $^1H$ NMR (DMSO-$d_6$, 300 MHz) δ ppm 0.72 (t, 3H) 1.11 (s,6H) 1.53-1.59(m,2H)2.15(s,3H)2.19 (s, 3H) 3.24 (s, 3 H) 3.63 (t, 2 H) 4.22 (t, 2 H) MS (ESI) m/z 285 $(M+H)^+$.

Example 44

2-ethyl-N-[(2Z)-3-(2-methoxyethyl)-4,5-dimethyl-1,3-thiazol-2(3H)-ylidene]butanamide

The product of Example 12A (39 mg, 0.15 mmol) and 2-ethylbutyric acid (26 mg, 0.22 mmol) were processed using the method described in Example 3 to afford the title compound. $^1H$ NMR (DMSO-$d_6$, 300 MHz) δ ppm 0.80 (t, 6 H) 1.48 (s, 2 H) 1.56 - 1.65 (m, 2 H) 2.19 (s, 3 H) 2.23 (s, 3H) 2.27 - 2.33 (m, 1 H) 3.23 (s, 3 H) 3.63 (t, 2 H) 4.24 - 4.33 (m, 2 H) MS (ESI) m/z 285 $(M+H)^+$.

Example 45

N-[(2Z)-3-(2-methoxyethyl)-4,5-dimethyl-1,3-thiazol-2(3H)-ylidene]cyclohexanecarboxamide

The product of Example 12A (39 mg, 0.15 mmol) and cyclohexanecarboxylic acid (28 mg, 0.22 mmol) were processed using the method described in Example 3 to afford the title compound. $^1$H NMR (DMSO-$d_6$, 300 MHz) δ ppm 1.23 - 1.32 (m, 2 H) 1.34 - 1.43 (m, 2 H) 1.58 - 1.65 (m, 1 H) 1.67- 1.74(m, 2H) 1.83- 1.89 (m, 2 H) 2.19 (s, 3 H) 2.23 (s, 3 H) 2.35 - 2.42 (m, I H) 3.24 (s, 3 H) 3.64 (t, 2 H) 4.31 (t, 3 H) MS (ESI) m/z 297 $(M+H)^+$.

Example 46

N-[(2Z)-3-(2-methoxyethyl)-4,5-dimethyl-1,3-thiazol-2(3H)-ylidene]-1-methylcyclohexanecarboxamide

The product of Example 12A (0.30 g, 1.1 mmol) and 1-methylcyclohexane-carboxylic acid (0.32 g, 2.2 mmol) were processed using the methods described in Example 13. Purification by column chromatography ($SiO_2$, 30-50% ethyl acetate/hexanes gradient) afforded 80 mg (23%) of the title compound. $^1$H NMR ($CDCl_3$, 300 MHz) δ ppm 1.16 (s, 3 H), 1.19-1.58 (m, 10 H), 2.18 (s, 3 H), 2.20 (s, 3 H), 3.30 (s, 3 H), 3.69 (t, *J*=5.3 Hz, 2 H), 4.19-4.31 (m, 2 H); MS ($DCI/NH_3$) m/z 311 $(M+H)^+$. Anal. Calculated for $C_{16}H_{26}N_2O_2S$: C, 61.90; H, 8.44; N, 9.02. Found: C, 61.86; H, 8.80; N, 9.02.

Example 47

*cis-N*-[(2Z)-3-(2-Methoxyethyl)-4,5-dimethyl-1,3-thiazol-2(3*H*)-ylidene]-2-methylcyclohexanecarboxamide

The product of Example 12A ( 0.30 g, 1.1 mmol) and (cis)-2-methyl-cyclohexanecarboxyli acid (0.32 g, 2.2 mmol) were processed using the methods described in Example 13. Purification by column chromatography ($SiO_2$, 30-50% ethyl acetate/hexanes gradient) afforded 0.24 g (68%) of the title compound. $^1$H NMR ($CDCl_3$, 300 MHz) δ ppm 0.85 (d, *J*=7.1 Hz, 3 H), 1.36-1.84 (m, 8 H), 2.17 (s, 3 H), 2.20 (s, 3 H), 2.31-2.42 (m, 1 H), 2.53-2.65 (m, I H), 3.29 (s, 3 H), 3.69 (t,*J*=4.2 Hz, 2 H), 4.17-4.29 (m, 2 H); MS ($DCI/NH_3$) m/z 311 $(M+H)^+$. Anal. Calculated for $C_{16}H_{26}N_2O_2S$: C, 61.90; H, 8.44; N, 9.02. Found: C, 62.15; H, 8.70; N, 8.73.

Example 48

N-[(2Z)-3-(2-methoxyethyl)-4,5-dimethyl-1,3-thiazol-2(3H)-ylidene]-4-methylcyclohexanecarboxamide

The product of Example 12A (39 mg, 0.15 mmol) and 4-methylcyclohexanecarboxylic acid (31 mg, 0.22 mmol) were processed using the methods described in Example 13 to afford the title compound. $^1$H NMR (DMSO-$d_6$, 300 MHz) δ ppm 0.84 - 0.89 (m, 3 H) 1.15 - 1.23 (m, 2 H) 1.47 - 1.56 (m, 4 H) 1.95 - 2.03 (m, 2 H) 2.19 (s, 3 H) 2.23 (s, 3 H) 3.24 (s, 3 H) 3.60-3.67 (m, 2 H) 4.26 - 4.36 (m, 4 H) MS (ESI) m/z 311 $(M+H)^+$.

Example 49

N-[(2Z)-3-(2-methoxyethyl]4,5-ditmethyl-1,3-thiazol-2(3H)-ylidene]cycloheptanecarboxamide

The product of Example 12A (39 mg, 0.15 mmol) and cycloheptanecarboxylic acid (31 mg, 0.22 mmol) were processed using the methods described in Example 13 to afford the title compound. $^1$H NMR (DMSO-$d_6$, 300 MHz) δ ppm 1.45 - 1.52 (m, 4 H) 1.54 - 1.56 (m, 2 H) 1.60 - 1.70 (m, 4 H) 1.85-1.92 (m, 2H)2.19(s,3H) 2.23 (s, 3 H) 2.55-2.61 (m, I H)3.24(s,3H)3.61 -3.66(m,2H) 4.27 - 4.34 (m, 2 H) MS (ESI) m/z 311 $(M+H)^+$.

Example 50

(1S)-N-[(2Z)-3-(2-methoxyethyl)-4,5-dimethyl-1,3-thiazol-2(3H)-ylidene]spiro[2.5]octane-1-carboxamide

The product of Example 12A and (1S)-spiro[2.5]octane-1-carboxylic acid (Bennani, Y.L., et al. US 20042043961) were processed using the methods described in Example 13 to provide the title compound. MS ($DCI/NH_3$) m/z 323 $(M+H)^+$.

Example 51

(2R)-N-[(2Z)-3-(2-methoxyethyl)-4,5-dimethyl-1,3-thiazol-2(3H)-ylidene]-2-propylhex-4-ynamide

The product of Example 12A (0.30 g, 1.1 mmol) and (2*R*)-propyl-hex-4-ynoic acid (0.35 g, 2.2 mmol) were processed using the methods described in Example 13. Purification by column chromatography ($SiO_2$, 20-30% ethyl acetate/hexanes gradient) afforded 0.30 g (82%) of the title compound. $^1H$ NMR ($CDCl_3$, 300 MHz) $\delta$ ppm 0.90 (t, *J*=7.5 Hz, 3 H), 1.27-1.39 (m, 2 H), 1.62-1.72 (m, 2H), 1.75 (t,*J*=2.4 Hz, 3 H), 2.19 (s, 3 H), 2.22 (s, 3 H), 2.31 -2.74 (m, 3 H), 3.29 (s, 3 H), 3.65-3.75 (m, 2 H), 4.16-4.33 (m, 2 H); MS (DCI/$NH_3$) m/z 323 $(M+H)^+$. Anal. Calculated for $C_{l7}H_{26}N_2O_2S$: C, 63.32; H, 8.13; N, 8.69. Found: C, 63.12; H, 8.35; N, 8.51.

Example 52

(1S,3R,5S)-N-[(2Z)-3-(2-methoxyethyl)-4,5-dimethyl-1,3-thiazol-2(3H)-ylidene]-3,5-dimethylcyclohexanecarboxamide

The product of Example 12A (0.30 g, 1.1 mmol) and (1S,3R,5S)-dimethylcyclohexanecarboxylic acid (0.35 g, 2.2 mmol) were processed using the methods described in Example 13. Purification by column chromatography ($SiO_2$, 20-40% ethyl acetate/hexanes gradient) afforded 0.12 g (33%) of the title compound. $^1H$ NMR ($CDCl_3$, 300 MHz) $\delta$ ppm 0.91 (s, 3 H), 0.92-0.94 (m, 3 H), 1.01-1.13 (m, 2 H), 1.61-1.69 (m, 3 H), 1.90-2.00 (m, 3 H), 2.19 (s, 3 H), 2.21-2.25 (m, 3 H), 2.26-2.30 (m, 1 H), 3.30 (s, 3 H), 3.71 (t,*J*=5.3 Hz, 2 H), 4.19-4.44 (m, 2 H); MS (DCI/$NH_3$) m/z 325 $(M+H)^+$. Anal. Calculated for $C_{17}H_{28}N_2O_2S$: C, 62.93; H, 8.70; N, 8.63. Found: C, 63.29; H, 8.91; N, 8.71.

Example 53

(9R,1R,8S)-N-[(2Z)-3-(2-methoxyethyl)-4,5-dimethyl-1,3-thiazol-2(3H)-ylidene]bicyclo[6.1.0]nonane-9-carboxamide

(endo)-Bicyclo[6.1.0]nonane-9-carboxylic acid (0.38 g, 2.2 mmnol, Bennani, Y.L., et al., US2004077617) and the product of Example 12A (0.30 g, 1.1 mmol) were processed using the methods described in Example 13. Purification by column chromatography ($SiO_2$, 20-35% ethyl acetate/hexanes gradient) afforded 0.27 g (72%) of the title compound. $^1H$ NMR ($CDCl_3$, 300 MHz) $\delta$ ppm 1.03-1.20 (m, l H), 1.29-1.51 (m, 6 H), 1.52-1.77 (m, 6 H), 2.07 (dd, *J*=14.1, 2.9 Hz, 2 H), 2.17 (s, 3H), 2.20 (s, 3 H), 3.31 (s, 3H), 3.70 (t, *J*=4.9 Hz, 2 H), 4.20-4.30 (m, 2 H); MS (DCI/$NH_3$) m/z 337 $(M+H)^+$. Anal. Calculated for $C_{18}H_{28}N_2O_2S$: C, 64.25; H, 8.39; N, 8.32. Found: C, 64.06; H, 8.54; N, 8.22.

Example 54

(9S,1R,8S)-N-[(2Z)-3-(2-methoxyethyl)-4,5-dimethyl-1,3-thiazol-2(3H) ylidene]bicyclo[6.1.0]nonane-9-carboxamide

(exo)-Bicyclo[6.1.0]nonane-9-carboxylic acid (0.38 g, 2.2 mmol, Bennani, Y.L., et al., US2004077617) and the product of Example 12A (0.30 g, 1.1 mmol) were processed using the methods described in Example 13. Purification by column chromatography ($SiO_2$, 20-30% ethyl acetate/hexanes gradient) afforded 70 mg (19%) of the title compound. $^1H$ NMR ($CDCl_3$, 300 MHz) $\delta$ ppm 1.17-1.29 (m, 2 H), 1.34-1.49 (m, 6 H), 1.57-1.76 (m, 6 H), 1.95-2.04 (m, 1 H), 2.15 (s, 3 H), 2.20 (s, 3 H), 3.30 (s, 3 H), 3.70 (t, *J*=5.3 Hz, 2 H), 4.24 (t,*J*=5.3 Hz, 2 H); MS (DCI/$NH_3$) m/z 337 $(M+H)^+$. Anal. Calculated for $C_{18}H_{28}N_2O_2S$: C, 64.25; H, 8.39; N, 8.32. Found: C, 64.33; H, 8.52; N, 8.23.

Example 55

( 1R,6R)-N-[(2Z)-3-(2-methoxyethyl)-4,5-dimethyl-1,3-thiazol-2(3H)-ylidene]-1-methylbicyclo[4.1.0]heptane-7-carboxamide

The product of Example 12A (0.30 g, 1.1 mmol) and 1-methylbicyclo[4.1.0]heptane-7-carboxylic acid (0.35 g, 2.2 mmol, Bennani, Y.L., et al., US2004077617) were processed using the methods described in Example 13. Purification by column chromatography ($SiO_2$, 20-35% ethyl acetate/hexanes gradient) afforded 40 mg (11 %) of the title compound. $^1H$ NMR ($CDCl_3$, 300 MHz) $\delta$ ppm 1.15-1.42 (m, 7 H), 1.58-1.72 (m,3H), 1.77 (d, *J*=5.4Hz, 1 H), 1.84-2.04 (m, 2 H), 2.15 (s, 3 H), 2.19 (s, 3 H), 3.28-3.33 (m, 3 H), 3.69 (t, *J*=5.3 Hz, 2 H), 4.12-4.39 (m, 2 H); MS (DCI/$NH_3$) m/z 323 $(M+H)^+$. Anal. Calculated for $C_{17}H_{26}N_2O_2S$: C, 63.32; H, 8.13; N, 8.69. Found: C, 63.35; H, 8.3; N, 8.56.

Example 57

2.2,3.3-Tetramethylcyclopropanecarboxylic acid [4,5-dimethyl-3-(2-phenoxy-ethyl)-3*H*-thiazol-2- -2-ylidene]-amide

Example 57A

4.5-Dimethyl-3-(2-phenoxy-ethyl)-3H-thiazol-2-ylideneamine hydrobromide

A mixture of 4,5-dimethylthiazol-2-ylamine (1.0 g, 7.8 mmol) and (2-bromo-ethoxy)benzene (1.9 g, 9.4 mmol) were heated neat to 85 °C for 19 hours. The mixture was cooled to ambient temperature and the residue was crystallized from isopropanol. The solid was collected by filtration and dried under vacuum to afford 1.3 g (50%) of the title compound. MS ($DCI/NH_3$) m/z 249 $(M+H)^+$.

Example 57B

2,2,3,3-Tetramethylcyclopropanecarboxylic acid [4,5-dimethyl-3-(2_phenoxy-ethyl)-3*H*-thiazol-2-ylidene]-amide

The product of Example 57A (0.40 g, 1.2 mmol) and 2,2,3,3-tetramethylcyclopropanecarboxylic acid (0.19 g, 1.3 mmol) were processed using the methods described in Example 13. Purification by column chromatography ($SiO_2$, 20-40% ethyl acetate/hexanes gradient) afforded 0.14 g (34%) of the title compound. $^1H$ NMR ($CDCl_3$, 500 MHz) $\delta$ ppm 1.22 (s, 6 H), 1.32-1.35 (m, 6 H), 1.56 (s, I H), 2.15 (s, 3 H), 2.27 (s, 3 H), 4.32 (t, *J*=5.5 Hz, 2 H), 4.44 (t,*J*=5.3 Hz, 2 H), 6.90 (d,*J*=8.1 Hz, 2 H), 6.95 (t,*J*=7.3 Hz, I H), 7.25-7.29 (m, 2 H); MS ($DCI/NH_3$) m/z 373 $(M+H)^+$. Anal. Calculated for $C_{21}H_{28}N_2O_2S$: C, 67.71; H, 7.58; N, 7.52. Found: C, 67.31; H, 7.70; N, 7.30.

Example 58

N-[(2Z)-3-(2_methoxyethyl)-4,5-dimethyl-1,3-thiazol-2(3H)-ylidene]-2,2-dimethyltetrahydro-2H-pyran-4-carboxamide

A mixture of the product of Example 12A (150 mg, 0.56 mmol), 2,2-dimethyl-tetrahydropyran-4-carboxylic acid (127 mg, 0.56 mmol), N-(3-dimethylaminopropyl)-N-ethylcarbodiimide hydrochloride (133 mg, 0.70 mmol), 1-hydroxybenzotriazole (94.5 mg, 0.70 mmol) and triethylamine (312 $\mu$L, 2.24 mmol) in 5 mL of THF were stirred overnight at room temperature. The reaction mixture was diluted with ethyl acetate and washed with 1 M aqueous $NaHCO_3$ and brine. The organic extract was dried ($Na_2SO_4$), filtered and concentrated. Purification of the residue by chromatography afforded the title compound: $^1H$ NMR (400 MHz, DMSO-$d_6$) $\delta$1.13 (s, 3 H) 1.16 (s, 3 H) 1.33 - 1.53 (m, 2 H) 1.67 - 1.77 (m, 2 H) 2.16 (s, 3 H) 2.20 (s, 3 H) 2.59 - 2.72 (m, I H) 3.24 (s, 3 H) 3.53 - 3.61 (m, 2 H) 3.63 (t, *J*=5.1 Hz, 2 H) 4.24 (t, *J*=5.4 Hz, 2 H); MS (ESI+) m/z 327 $(M+H)^+$.

Example 59

2,2,3,3-tetrafluoro-N-[(2Z)-3-(2-methoxyethyl)-4,5-dimethyl-1,3-thiazol-2(3H)-ylidene]-1-methylcyclobutanecarboxamide

The product of 12A (0.30 g, 1.6 mmol) and 2,2,3,3-tetrafluoro-1-methylcyclobutanecarborboxylic acid (0.37 g, 1.8 mmol) were processed using the methods described in Example 13. Purification by column chromatography ($SiO_2$, 30-45% ethyl acetate/hexanes gradient) afforded 0.15 g (27%) of the title compound. $^1H$ NMR ($CDCl_3$, 300 MHz) $\delta$ ppm 1.59 (s, 3 H), 2.23 (s, 3 H), 2.25 (s, 3 H), 2.29-2.43 (m, I H), 3.29 (s, 3 H), 3.31-3.46 (m, 1 H), 3.70 (t,*J*=5.1 Hz, 2 H), 4.26-4.48 (m, 2 H); MS ($DCI/NH_3$) m/z 355 $(M+H)^+$. Anal. Calculated for $C_{14}H_{18}F_4N_2O_2S$: C, 47.45; H, 5.12; N, 7.91. Found: C, 47.41; H, 5.04; N, 7.81.

Example 60

1 -hydroxy-N-[(2Z)-3-(2-methoxyethyl)-4,5-dimethyl-1,3-thiazol-2(3H)-ylidene]cyclohexanecarboxamide

Commercially available 1-hydroxy-cyclohexanecarboxylic acid and the product of Example 12A were processed using the method described in Example 58 to afford the title compound. $^1H$ NMR (300 MHz, DMSO-$d_6$) $\delta$ ppm 1.09 - 1.32 (m, 1 H), 1.36 - 1.68 (m, 7 H), 1.72 - 1.88 (m, 2 H), 2.18 (s, 3 H), 2.22 (s, 3 H), 3.23 (s, 3 H), 3.64 (t, *J*=5.3 Hz, 2 H), 4.29 (t, *J*=5.4 Hz, 2 H), 4.34 (s, 1 H); MS ($ESI^+$) m/z 335 $(M+Na)^+$; Anal. Calculated for $C_{15}H_{24}N_2O_3S$: C, 57.66; H, 7.74; N, 8.97. Found: C, 57.76; H, 7.80; N, 8.88.

Example 61

1-({[(2Z)-4,5-dimethyl-1,3-thiazol-2(3H)-ylidenelamino}carbonyl)cyclohexyl propionate

Propionyloxy-cyclohexanecarboxylic acid (Hartmann, Willy et al., Synthesis (1989), 4, 272-4) and the product from Example 12A were processed using the method described in Example 58 to afford the title compound. $^1H$ NMR (300 MHz, DMSO-$d_6$) δ ppm 1.03 (t, *J*=7.5 Hz, 3 H), 1.15 - 1.34 (m, 1 H), 1.36-1.66 (m, 5 H), 1.75 (td, *J*=13.1,4.1 Hz, 2 H), 2.01 - 2.13 (m, 2 H), 2.16 (s, 3 H), 2.20 (s, 3H), 2.32(q, *J*=7.5 Hz, 2H), 3.22(s, 3H), 3.58 (t, *J*=5.4Hz, 2H), 4.19 (t, *J*=5.4Hz, 2H); MS ($ESI^+$) m/z 369 $(M+H)^+$; Anal. Calculated for $C_{18}H_{28}N_2O_4S$: C, 58.67; H, 7.66; N, 7.60. Found: C, 58.46; H, 7.64; N, 7.75.

Example 62

N-[(22)-3-(2-methoxyethyl)-1,3-benzothiazol-2(3H)-ylidene]-2,2,3,3 - tetramethylcyclopropanecarboxamide

Example 62A

2,2,3,3-Tetramethyl cyclopropanecarboxylic acid benxothiazol-2-ylamide

A mixture of 2,2,3,3-tetramethylcyclopropanecarboxylic acid (0.50 g, 3.5 mmol) and benzothiazol-2-ylamine (0.58 g, 3.9 mmol) were processed as in Example 9A. Purification by column chromatography ($SiO_2$, 20-40% ethyl acetate/hexanes gradient) afforded 0.26 g (27%) of the title compound. $^1H$ NMR ($CDCl_3$, 300 MHz) δ ppm 1.22 - 1.27 (m, 6 H), 1.36 (s, 6 H), 1.67 (s, I H), 2.07 - 2.18 (m, 2 H), 3.34 (s, 3 H), 3.41 (t, *J*=5.8 Hz, 2 H), 4.39 - 4.53 (m, 2 H), 7.20 - 7.26 (m, I H), 7.37 - 7.42 (m, 2 H), 7.60 (d, *J*=7.5 Hz, I H); MS ($DCI/NH_3$) m/z 275 $(M+H)^+$.

Example 62B

2,2,3,3-Tetramethylcyclopropanecarboxylic acid [3-(2-methoxyethyl)-3*H*-benzothiazol-2-ylidene]-amide

The product of Example 62A (0.12 g, 0.43 mmol), 2-bromoethyl methyl ether (0.44 mL, 4.7 mmol) and potassium hydroxide (56 mg, 1.0 mmol) were processed using the method described in Example I B. Purification by column chromatography ($SiO_2$, 20-50% ethyl acetate/hexanes gradient) afforded 12 mg (8%) of the title compound. $^1H$ NMR ($CDCl_3$, 300 MHz) δ ppm 1.25 (s, 6 H), 1.36 (s, 6 H), 1.66 (s, 1H), 3.34 (s, 3 H), 3.79 (t, *J*=5.6 Hz, 2 H), 4.53 (t,*J*=5.6 Hz, 2 H), 7.21-7.25 (m, 1 H), 7.37-7.42 (m, 2 H), 7.58 (d,*J*=7.8 Hz, 1 H); MS ($DCI/NH_3$) m/z 371 $(M+H)^+$. Anal. Calculated for $C_{18}H_{24}N_2O_2S$: C, 65.03; H, 7.28; N, 8.43. Found: C, 64.94; H, 7.10; N, 8.40.

Example 63

N-r(2Z)-3-(3-methoxypropyl)-1,3-benzothiazol-2(3H)-ylidene]-2.2.3,3- tetramethylcyclopropanecarboxamide

Example 63A

3-(3-Methoxy-propyl)-3H-benzothiazol-2-ylideneamine hydrobromide

Benzothiazol-2-ylamine (1.0 g, 6.6 mmol) and 1-bromo-3-methoxy-propane (1.2 g, 7.9 mmol) were processed using the method described in Example 12A. Recrystallization from ethyl acetate provided 1.7 g (89%) of the title compound. $^1H$ NMR (DMSO-$d_6$, 300 MHz) δ ppm 1.90-2.02 (m, 2H), 3.18 (s, 3H), 3.39 (t, *J*=5.9 Hz, 2 H), 4.31 (t,*J*=7.1 Hz, 2 H), 7.37-7.48 (m, 1 H), 7.53-7.69 (m, 2 H), 8.00 (dd, *J*=8.0,0.8 Hz, 1 H), 10.08 (s, I H); MS ($DCI/NH_3$) m/z 233 $(M+H)^+$.

Example 63B

2,2,3,3-Tetramethylcyclopronanecarboxylic acid [3-(3-methoxy-propyl)-3*H*-benzothiazol-2-ylidene)-amide

The product of Example 63A (0.40 g, 1.3 mmol) and 2,2,3,3-Tetramethylcyclopropanecarboxylic acid (U.19 g, 1.3 mmol) were processed using the methods described in Example 13. Purification by column chromatography ($SiO_2$, 20-30% ethyl acetate/hexanes gradient) afforded 0.32 g (70%) of the title compound. $^1H$ NMR ($CDCl_3$, 300 MHz) δ ppm 1.26 (s, 6 H), 1.36 (s, 6 H), 1.64 (br s, 1 H), 1.71 (s, 1 H), 3.87 (s, 3 H), 7.27-7.32 (m, 2 H), 7.40-7.47 (m, 1 H), 7.62 (d, *J*=7.5 Hz, 1 H); MS ($DCI/NH_3$) m/z 289 $(M+H)^+$. Anal. Calculated for $C_{16}H_{20}N_2O_2S$: C, 65.86; H, 7.56; N, 8.08. Found:

C, 65.54; H, 7.65; N, 7.81.

Example 64

N-[(2Z)-3-(2-methoxyethyl)-1,3-benzothiazol-2(3H)-ylidene]-3-phenylpropanamide

Example 64A

3-(2-Methoxyethyl)-3*H*-benzothiazol-2-ylideneamine hydrobromide

Benzothiazol-2-ylamine (10.0 g, 66.6 mmol) and 2-bromoethyl methyl ether (9.39 mL, 99.9 mmol) were combined and heated at 85 °C for 6 hours. The dark solid was triturated with EtOH then filtered and dried under vacuum to afford the title compound (15.8 g, 82%). $^1H$ NMR (DMSO-$d_6$, 300 MHz) δ ppm 3.23 (s, 3 H), 3.69 (t, *J*=5.1 Hz, 2 H), 4.51 (t, *J*=5.1 Hz, 2 H), 7.42 (dt, J=1.0, 8.0 Hz, I H), 7.56 (m, I H), 7.72 (d, *J*=8.0 Hz, I H), 8.00 (dd, *J*=1.1, 8.0 Hz, 1H), 10.16 (br s, 2 H); MS (DCI/$NH_3$) m/z 209 $(M+H)^+$.

Example 64B

N-r3-(2-Methoxyethyl)-3H-benzothiazol-2-ylidenel-3-phenylpropionainid

The product of Example 64A (39 mg, 0.14 mmol) and hydrocinnamic acid (26 mg, 0.17 mmol) were processed using the method described in Example 3 to afford the title compound. $^1H$ NMR (500 MHz, DMSO-$d_6$) δ ppm 2.77 - 2.85 (m, 2 H) 2.92 - 3.05 (m, 2 H) 3.19 - 3.25 (m, 3 H) 3.76 (t, 2 H) 4.52 (t, 2 H) 7.08 - 7.19 (m, 1 H) 7.22 - 7.37 (m, 5 H) 7.41 - 7.53 (m, I H) 7.59 - 7.74 (m, I H) 7.75 - 8.03 (m, 1 H); MS(ESI) m/z 341$(M+H)^+$.

Example 65

(2S)-N-[(2Z)-3-(2-methoxyethyl)-1,3-benzothiazol-2(3H)-ylidene]-2-phenylbutanamide

The product of Example 64A (39 mg, 0.14 mmol) and (S)-(+)-2-phenylbutyric acid (26 mg, 0.17 mmol) were processed using the method described in Example 3 to afford the title compound. $^1H$ NMR (500 MHz, DMSO-$d_6$) δ ppm 0.89 (t, 3 H) 1.69 - 1.89 (m, 1 H) 2.04 - 2.23 (m, 1 H) 3.11 - 3.20 (m, 3 H) 3.57 - 3.76 (m, 3 H) 4.54 (t, 2 H) 7.17 - 7.23 (m, I H) 7.27 - 7.41 (m, 5 H) 7.43 - 7.53 (m, I H) 7.61 - 7.71 (m, I H) 7.74 - 7.88 (m, I H); MS(ESI) m/z 355 $(M+H)^+$.

Example 66

N-[(2Z)-3-(2-methoxyethyl)-1,3-benzothiazol-2(3H)-ylidene]-4-thien-2-ylbutanamide

The product of Example 64A (39 mg, 0.14 mmol) and 4-(2-thienyl)butyric acid (29 mg, 0.17 mmol) were processed using the method described in Example 3 to afford the title compound. $^1H$ NMR (500 MHz, DMSO-$d_6$) δ ppm 1.86 - 2.08 (m, 2 H) 2.55 (t, 2 H) 2.87 (t, 2 H) 3.17 - 3.24 (m, 3 H) 3.72 (t, 2 H) 4.54 (t, 2 H) 6.83 - 6.89 (m, I H) 6.90 - 7.01 (m, 1 H) 7.22 - 7.37 (m, 2 H) 7.42 - 7.55 (m, 1 H) 7.64 - 7.72 (m, 1 H) 7.75 - 7.88 (m, I H); MS(ESI) m/z 361$(M+H)^+$.

Example 67

$N^2$-acetyl-$N^1$-[3-(2-methoxyethyl)-1,3-benzothiazol-2(3 H)-ylidene]-L-leucinamide

The product of Example 64A (39 mg, 0.14 mmol) and *N*-acetyl-L-leucine (29 mg, 0.17 mmol) were processed using the method described in Example 3 to afford the title compound. $^1H$ NMR (500 MHz, DMSO-$d_6$) δ ppm 0.78 - 1.07 (m, 6 H) 1.43 - 1.57 (m, I H) 1.57 - 1.75 (m, 2 H) 1.81 - 1.92 (m, 3 H) 3.22 - 3.26 (m, 3 H) 3.72 (t, 2 H) 4.44 - 4.55 (m, I H) 4.55 - 4.67 (m, 2 H) 7.24 - 7.40 (m, 1 H) 7.43 - 7.54 (m, 1 H) 7.62 - 7.73 (m, I H) 7.79 - 7.92 (m, 1 H) 7.95 - 8.07 (m, I H); MS(ESI) m/z 364$(M+H)^+$.

Example 68

3-(2-chlorophenyl)-N-[(2Z)-3-(2-methoxyethyl)-1,3-benzothiazol-2(3H)-ylidene]propanamide

The product of Example 64A (39 mg, 0.14 mmol) and 3-(2-chlorophenyl)propionic acid (31 mg, 0.17 mmol)

were processed using the method described in Example 3 to afford the title compound. $^1$H NMR (500 MHz, DMSO-$d_6$) δ ppm 2.82 (t, 2 H) 3.08 (t, 2 H) 3.18 - 3.25 (m, 3 H) 3.79 (t, 2 H) 4.54 (t, 2 H) 7.17 - 7.29 (m, 2 H) 7.30 - 7.44 (m, 3 H) 7.45 - 7.54 (m, I H) 7.59 - 7.75 (m, 1 H) 7.76 - 7.93 (m, I H).

Examole 69

N-[(2Z)-3-(2-methoxyethyl)-1,3-benzothiazol-2(3H)-ylidene]-3-methyl-2-phenylpentanamide

The product of Example 64A (39 mg, 0.14 mmol) and 3-methyl-2-phenylvaleric acid (33 mg, 0.17 mmol) were processed using the method described in Example 3 to afford the title compound. $^1$H NMR (500 MHz, DMSO-$d_6$) δ ppm 0.59 - 0.68 (m, 2 H) 0.72 - 0.82 (m, 2 H) 0.84 - 0.98 (m, 3 H) 1.08- 1 .27 (m, 1 H) 2.21 - 2.39 (m, I H) 3.11 - 3.24 (m, 3 H) 3.38-3.50 (m, 1 H) 3.65-3.82 (m, 2 H) 4.57 (t, 2 H) 7.16 - 7.23 (m, I H) 7.25 - 7.36 (m, 3 H) 7.37 - 7.54 (m, 3 H) 7.60 - 7.73 (m, I H) 7.75 - 7.88 (m, I H); MS(ESI) m/z 383(M+H)$^+$.

Example 70

4-ethyl-N-[(2Z)-3-(2-methoxyethyl)-1,3-benzothiazol-2(3H)-ylidene]benzamide

The product of Example 64A (39 mg, 0.14 mmol) and 4-ethylbenzoic acid (26 mg, 0.17 mmol) were processed using the method described in Example 3 to afford the title compound. $^1$H NMR (500 MHz, DMSO-$d_6$) δ ppm 1.22 (t, 3 H) 2.66 - 2.72 (m, 2 H) 3.25 - 3.26 (m, 3 H) 3.85 (t, 2 H) 4.75 (t, 2 H) 7.31 - 7.42 (m, 3 H) 7.47 - 7.60 (m, I H) 7.68 - 7.79 (m, I H) 7.85 - 7.94 (m, I H) 8.12 - 8.23 (m, 2 H); MS (ESI) m/z 341 (M+H)$^+$.

Example 71

3-fluoro-N-[(2Z)-3-(2-methoxyethyl)-1,3-benzothiazol-2(3H)-ylidene1-2-i-methylbenzainide

The product of Example 64A (39 mg, 0.14 mmol) and 3-fluoro-2-methylbenzoic acid (26 mg, 0.17 mmol) were processed using the method described in Example 3 to afford the title compound. $^1$H NMR (500 MHz, DMSO-$d_6$) δ ppm 2.51 - 2.53 (m, 3 H) 3.22 - 3.25 (m, 3 H) 3.80 (t, 2 H) 4.69 (t, 2 H) 7.26 - 7.43 (m, 3 H) 7.50 - 7.61 (m, 1 H) 7.70 - 7.79 (m, 1 H) 7.86 - 7.99 (m, 2 H); MS (ESI) m/z 345 (M+H)$^+$.

Example 72

5-fluoro-N-r(2Z)-3-(2-methoxyethyl)::1,3-benzothiazol-2(3H)-ylidenel-2-methylbenzainide

The product of Example 64A (39 mg, 0.14 mmol) and 5-fluoro-2-methylbenzoic acid (26 mg, 0.17 mmol) were processed using the method described in Example 3 to afford the title compound. $^1$H NMR (500 MHz, DMSO-$d_6$) δ ppm 2.51 - 2.53 (m, 3 H) 3.22 - 3.25 (m, 3 H) 3.80 (t, 2 H) 4.69 (t, 2 H) 7.26 - 7.43 (m, 3 H) 7.50 - 7.61 (m, 1 H) 7.70 - 7.79 (m, I H) 7.86 - 7.99 (m, 2 H); MS (ESI) m/z 345 (M+H)$^+$.

Example 73

3-fluoro-N-[(2Z)-3-(2-methoxyethyl)-1,3-benzothiazol-2(3H)-ylidene]-4-methylbenzamide

The product of Example 64A (39 mg, 0.14 mmol) and 5-fluoro-4-methylbenzoic acid (26 mg, 0.17 mmol) were processed using the method described in Example 3 to afford the title compound. $^1$H NMR (500 MHz, DMSO-$d_6$) δ ppm 2.31 - 2.34 (m, 3 H) 3.23 - 3.26 (m, 3 H) 3.83 (t, 2 H) 4.77 (t, 2 H) 7.33 - 7.40 (m, I H) 7.41 - 7.49 (m, 1 H) 7.49 - 7.59 (m, 1 H) 7.69 - 7.78 (m, ) 1 H) 7.86 - 7.95 (m, 2 H) 7.97 - 8.02 (m, I H); MS (ESI) m/z 345 (M+H)$^+$.

Example 74

2,3-difluoro-N-[(2Z)-3-(2-methoxyethyl)-1,3-benzothiazol-2(3H)-ylidene]benzamide

The product of Example 64A (39 mg, 0.14 mmol) and 2,3-difluorobenzoic acid (27 mg, 0.17 mmol) were processed using the method described in Example 3 to afford the title compound. $^1$H NMR (500 MHz, DMSO-$d_6$) δ ppm 3.20 - 3.26 (m, 3 H) 3.82 (t, 2 H) 4.72 (t, 2 H) 7.28 - 7.36 (m, I H) 7.37 - 7.43 (m, I H) 7.51 - 7.59 (m, I H) 7.59 - 7.67 (m, I H) 7.73 - 7.81 (m, 1 H)7.90 - 8.00 (m, 2 H); MS (ESI) m/z 349 (M+H)$^+$.

Example 75

2,5-difluoro-N-[(22)-3-(2-methoxyethyl)-1,3-benzothiazol-2(3H)-ylidene]benzamide

The product of Example 64A (39 mg, 0.14 mmol) and 2,5-difluorobenzoic acid (27 mg, 0.17 mmol) were processed using the method described in Example 3 to afford the title compound. $^1$H NMR (500 MHz, DMSO-$d_6$) $\delta$ ppm 3.23 (s, 3 H) 3.82 (t, 2 H) 4.72 (t, 2 H) 7.38 (s, 2 H) 7.46 (s, I H) 7.54 (s, 1H) 7.77 (s, I H) 7.89 (s, 1 H) 7.92 - 7.98 (m, 1 H); MS (ESI) m/z 349 $(M+H)^+$.

Example 76

2-acetyl-N-[(2Z)-3-(2-methoxyethyl)-1,3-benzothiazol-2(3H)-ylidene]benzamide

The product of Example 64A (39 mg, 0.14 mmol) and 2-acetylbenzoic acid (28 mg, 0.17 mmol) were processed using the method described in Example 3 to afford the title compound. $^1$H NMR (500 MHz, DMSO-$d_6$) $\delta$ ppm 2.44 - 2.48 (m, 3 H) 3.21 - 3.25 (m, 3 H) 3.77 (t, 2 H) 4.67 (t, 2 H) 7.30 - 7.44 (m, 2 H) 7.51 - 7.66 (m, 3 H) 7.70 - 7.82 (m, 1 H) 7.89 - 8.00 (m, 1 H) 8.11 - 8.25 (m, I H); MS (ESI) m/z 355 $(M+H)^+$.

Example 77

3-methoxy-N-[(2Z)-3-(2-methoxyethyl)-1.3-benzothiazol-2(3H)-ylidene]-4-methylbenzamide

The product of Example 64A (39 mg, 0.14 mmol) and 3-methoxy-4-methylbenzoic acid (28 mg, 0.17 mmol) were processed using the method described in Example 3 to afford the title compound. $^1$H NMR (500 MHz), DMSO-$d_6$) $\delta$ ppm 2.21 - 2.26 (m, 3 H) 3.25 - 3.28 (m, 3 H) 3.85 (t, 2 H) 3.88 - 3.90 (m, 3 H) 4.76 (t, 2 H) 7.26 - 7.31 (m, 1 H) 7.32 - 7.39 (m, 1H) 7.47 - 7.57 (m, I H) 7.71 - 7.78 (m, 2 H) 7.79 - 7.84 (m, 1 H) 7.88 - 7.93 (m, I H); MS (ESI) m/z 357 $(M+H)^+$.

Example 78

2-ethoxy-N-[(2Z)-3-(2-methoxyethyl)-1,3-benzothiazol-2(3H)-ylidene]benzamide

The product of Example 64A (39 mg, 0.14 mmol) and 2-ethoxybenzoic acid (28 mg, 0.17 mmol) were processed using the method described in Example 3 to afford the title compound. $^1$H NMR (500 MHz, DMSO-$d_6$) $\delta$ ppm 2.21 - 2.26 (m, 3 H) 3.25 - 3.28 (m, 3 H) 3.85 (t, 2 H) 3.88 - 3.90 (m, 3 H) 4.76 (t, 2 H) 7.26 - 7.31 (m, I H) 7.32 - 7.39 (m, 1H) 7.47 - 7.57 (m, 1 H) 7.71 - 7.78 (m, 2 H) 7.79 - 7.84 (m, 1H) 7.88 - 7.93 (m, I H); MS (ESI) m/z 357 $(M+H)^+$.

Example 79

N-[(2Z)-3-(2-methoxyethyl)-1,3-benzothiazol-2(3H)-ylidene]-(methylthio)benzamide

The product of Example 64A (39 mg, 0.14 mmol) and 4-methylsulfanylbenzoic acid (29 mg, 0.17 mmol) were processed using the method described in Example 3 to afford the title compound. $^1$H NMR (500 MHz, DMSO-$d_6$) $\delta$ ppm 2.53 - 2.58 (m, 3 H) 3.22 - 3.27 (m, 3 H) 3.84 (t, 2 H) 4.73 (t, 2H) 7.31 -7.44(m, 3H) 7.47-7.61 (m, 1 H) 7.67 - 7.79 (m, 1H) 7.82 - 8.02 (m, I H) 8.08 - 8.22 (m, 2 H); MS (ESI) m/z 359 $(M+H)^+$.

Example 80

N-[(22)-3-(2-methoxyethyl)-1,3-benzothiazol-2(3H)-2(3H)-ylidene]-1-naphthamide

The product of Example 64A (39 mg, 0.14 mmol) and 1-naphthoic acid (29 mg, 0.17 mmol) were processed using the method described in Example 3 to afford the title compound. $^1$H NMR (500 MHz, DMSO-$d_6$) $\delta$ ppm 3.23 - 3.27 (m, 3 H) 3.90 (t, 2 H) 4.71 (t, 2H) 7.32 - 7.42 (m, 1 H) 7.45 - 7.68 (m, 4 H) 7.71 - 7.79 (m, I H) 7.83 - 8.22 (m, 3 H) 8.40 - 8.56 (m, I H) 9.05 - 9.17 (m, I H); MS (ESI) m/z 363 $(M+H)^+$.

Example 81

N-[(2Z)-3-(2-methoxyethyl)-1,3-benzothiazol-2(3H)-ylidene]-2-nalphthamide

The product of Example 64A (39 mg, 0.14 mmol) and 2-naphthoic acid (29 mg, 0.17 mmol) were processed using the method described in Example 3 to afford the title compound. $^1$H NMR (500 MHz, DMSO-$d_6$) δ ppm 3.28 - 3.29 (m, 3 H) 3.91 (t, 2 H) 4.78 (t, 2 H) 7.25 - 7.44 (m, I H) 7.50-7.58(m, 1 H) 7.58 - 7.67 (m, 2 H) 7.71 - 7.82 (m, I H) 7.88 - 7.97 (m, I H) 7.99 - 8.07 (m, 2 H) 8.08 - 8.21 (m, I H) 8.26 - 8.40 (m, I H) 8.72 - 8.93 (m, I H); MS (ESI) m/z 363 $(M+H)^+$.

Example 82

5-chloro-2-hydroxy-N-[(2Z)-3-(2-methoxyethyl)-1.3-benzothiazol-2(3H)-ylidene]benzamide

The product of Example 64A (39 mg, 0.14 mmol) and 5-chloro-2-hydroxybenzoic acid (29 mg, 0.17 mmol) were processed using the method described in Example 3 to afford the title compound. $^1$H NMR (500 MHz, DMSO-$d_6$) δ ppm 3.22 - 3.25 (m, 3 H) 3.85 (t, 2 H) 4.73 (t, 2 H) 6.94 - 7.08 (m, 2 H) 7.36 - 7.53 (m, 2 H) 7.57 - 7.67 (m, 1 H) 7.79 - 7.92 (m, 1 H) 7.96 - 8.09 (m, 2 H); MS (ESI) m/z 363 $(M+H)^+$.

Example 83

5-chloro-2-methoxy-N-[(2Z)-3-(2-methoxyethyl)-1,3-benzothiazol-2(3H)-ylidene] benzamide

The product of Example 64A (39 mg, 0.14 mmol) and 5-chloro-2-methoxybenzoic acid (32 mg, 0.17 mmol) were processed using the method described in Example 3 to afford the title compound. $^1$H NMR (500 MHz, DMSO-$d_6$) δ ppm 3.22 - 3.26 (m, 3 H) 3.80 (t, 2 H) 3.83 - 3.88 (m, 3 H) 4.62 (t, 2 H) 7.13 - 7.23 (m, 1 H) 7.32 - 7.43 (m, 1 H) 7.46 - 7.59 (m, 2 H) 7.69 - 7.77 (m, 1 H) 7.79 - 7.88 (m, 1 H) 7.88 - 7.98 (m, 1 H); MS (ESI) m/z 377 $(M+H)^+$.

Example 84

1-hydroxy-N-[(2Z)-3-(2-methoxyethyl)-1,3-benzothiazol-2(3H)-ylidene]-2-naphthamide

The product of Example 64A (39 mg, 0.14 mmol) and 1-hydroxy-2-naphthoic acid (32 mg, 0.17 mmol) were processed using the method described in Example 3 to afford the title compound. $^1$H NMR (500 MHz, DMSO-$d_6$) δ ppm 3.25 - 3.28 (m, 3 H) 3.89 (t, 2 H) 4.75 (t, 2 H) 7.35 - 7.47 (m, 2 H) 7.53 - 7.63 (m, 2 H) 7.63 - 7.70 (m, 1 H) 7.79 - 7.93 (m, 2 H) 7.96 - 8.06 (m, 1 H) 8.04 - 8.19 (m, 1 H) 8.23 - 8.40 (m, 1 H); MS (ESI) m/z 379 $(M+H)^+$.

Example 85

4-fluoro-N-[(2Z)-3-(2-methoxyethyl)-13-benzothiazol-2(3H)-ylidene]-1-naphthamide

The product of Example 64A (39 mg, 0.14 mmol) and 4-fluoro-1-naphthoic acid (32 mg, 0.1 7 mmol) were processed using the method described in Example 3 to afford the title compound. $^1$H NMR (500 MHz, DMSO-$d_6$) δ ppm 3.22 - 3.26 (m, 3 H) 3.83 (t, 2 H) 4.67 (t, 2 H) 7.35 - 7.59 (m, 3 H) 7.65 - 7.82 (m, 3 H) 7.83 - 8.03 (m, 1 H) 8.09 - 8.28 (m, 1 H) 8.46 - 8.71 (m, 1 H) 9.08 - 9.38 (m, 1 H); MS (ESI) m/z 381 $(M+H)^+$.

Example 86

2-methoxy-N-[(2Z)-3-(2-methoxyethyl)-1,3-benzothiazol-2(3H)-ylidene]-4-(methylthio)benzamide

The product of Example 64A (39 mg, 0.14 mmol) and 2-methoxy-4-methylsulfanylbenzoic acid (34 mg, 0.17 mmol) were processed using the method described in Example 3 to afford the title compound. $^1$H NMR (500 MHz, DMSO-$d_6$) ppm 2.53 - 2.61 (m, 3 H) 3.21 - 3.25 (m, 3 H) 3.79 (t, 2 H) 3.82 - 3.89 (m, 3 H) 4.58 (t, 2 H) 6.73 - 7.05 (m, 2 H) 7.25 - 7.38 (m, 1 H) 7.47 - 7.61 (m, 1 H) 7.60 - 7.78 (m, 1 H) 7.80 - 7.91 (m, 1 H) 7.89 - 8.09 (m, 1 H); MS (ESI) m/z 389 $(M+H)^+$.

Example 87

2-chloro-N-[(2Z)-3-(2-methoxyethyl)-1,3-benzothiazol-2(3H)-ylidene]-5-(methylthio)benzamide

The product of Example 64A (39 mg, 0.14 mmol) and 2-chloro-5-methylsulfanylbenzoic acid (34 mg, 0.17 mmol) were processed using the method described in Example 3 to afford the title compound. $^1H$ NMR (500 MHz, DMSO-$d_6$) δ ppm 2.52 - 2.56 (m, 3 H) 3.20 - 3.26 (m, 3 H) 3.82 (t, 2 H) 4.68 (t, 2 H) 7.33 - 7.43 (m, 2 H) 7.44 - 7.51 (m, 1 H) 7.51 - 7.58 (m, 1 H) 7.71 - 7.86 (m, 2 H) 7.91 - 8.00 (m, 1 H); MS (ESI) m/z 393 $(M+H)^+$.

Example 88

2-fluoro-N-[(2Z)-3-(2-methoxyethyl)-1,3-benzothiazol-2(3H)-ylidene]-5-trifluoromethyl)benzamide

The product of Example 64A (39 mg, 0.14 mmol) and 2-fluoro-5-trifluoromethylbenzoic acid (34 mg, 0.17 mmol) were processed using the method described in Example 3 to afford the title compound. $^1H$ NMR (500 MHz, DMSO-$d_6$) δ ppm 3.23 - 3.26 (m, 3 H) 3.84 (t, 2 H) 4.67 (t, 2 H) 7.30 - 7.47 (m, 1 H) 7.52 - 7.64 (m, 2 H) 7.72 - 7.89 (m, 1 H) 7.91 - 8.07 (m, 2 H) 8.34 - 8.58 (m, 1 H); MS (ESI) m/z 399 $(M+H)^+$.

Example 89

2-benzyl-N-[(2Z)-3-(2-methoxyethyl)-1,3-benzothiazol-2(3H)-ylidene]benzamide

The product of Example 64A (39 mg, 0.14 mmol) and α-phenyl-o-toluic acid (36 mg, 0.17 mmol) were processed using the method described in Example 3 to afford the title compound. $^1H$ NMR (500 MHz, DMSO-$d_6$) δ ppm 3.18 - 3.20 (m, 3 H) 3.72 (t, 2 H) 4.48 - 4.52 (m, 2 H) 4.63 (t, 2 H) 7.07 - 7.30 (m, 6 H) 7.30 - 7.41 (m, 2 H) 7.40 - 7.47 (m, 1 H) 7.49 - 7.57 (m, 1 H) 7.67 - 7.79 (m, 1 H) 7.85 - 7.96 (m, 1 H) 8.02 - 8.12 (m, 1 H); MS (ESI) m/z 403 $(M+H)^+$.

Example 90

2-chloro-N-[(2Z)-3-(2-methoxyethyl)-1,3-benzothiazol-2(3H)-ylidene]-5-(trifluoromethyl)benzamide

The product of Example 64A (39 mg, 0.14 mmol) and 2-chloro-5-trifluoromethylbenzoic acid (38 mg, 0.17 mmol) were processed using the method described in Example 3 to afford the title compound. $^1H$ NMR (500 MHz, DMSO-$d_6$) δ ppm 3.20 - 3.25 (s, 3 H) 3.80 (t, 2 H) 4.70 (t, 2 H) 7.35 - 7.45 (m, 1 H) 7.50 - 7.65 (m, 1 H) 7.76 - 7.83 (m, 2 H) 7.84 - 7.91 (m, 1 H) 7.94 - 8.03 (m, 1 H) 8.22 - 8.32 (m, 1 H); MS (ESI) m/z 415 $(M+H)^+$.

Example 91

N-[(2Z)-3-(2-methoxyethyl)-1,3-benzothiazol-2(3H)-ylidene]-2-(2-phenylethyl)benzamide

The product of Example 64A (39 mg, 0.14 mmol) and 2-phenethylbenzoic acid (38 mg, 0.17 mmol) were processed using the method described in Example 3 to afford the title compound. $^1H$ NMR (500 MHz, DMSO-$d_6$) δ ppm 2.78 - 2.95 (m, 2 H) 3.16 - 3.22 (m, 3 H) 3.32 - 3.37 (m, 2 H) 3.76 (t, 2 H) 4.65 (t, 2 H) 6.99 - 7.46 (m, 9 H) 7.49 - 7.57 (m, 1 H) 7.67 - 7.80 (m, 1 H) 7.82 - 7.97 (m, 1 H) 7.97 - 8.15 (m, 1 H); MS (ESI) m/z 417 $(M+H)^+$.

Example 92

2-bromo-5-methoxy-N-[(2Z)-3-(2-methoxyethyl)-1,3-benzothiazol-2(3H)-ylidene]benzamide

The product of Example 64A (39 mg, 0.14 mmol) and 2-bromo-5-methoxybenzoic acid (39 mg, 0.17 mmol) were processed using the method described in Example 3 to afford the title compound. $^1H$ NMR (500 MHz, DMSO-$d_6$) δ ppm 3.20 - 3.26 (m, 3 H) 3.72 - 3.87 (m, 5 H) 4.67 (t, 2 H) 6.91 - 7.08 (m, 1 H) 7.30 - 7.43 (m, 1 H) 7.45 - 7.51 (m, 1 H) 7.51 - 7.65 (m, 2 H) 7.70 - 7.83 (m, 1 H) 7.86 - 7.98 (m, 1 H); MS (ESI) m/z 423 $(M+H)^+$.

Example 93

2-iodo-N-[(2Z)-3-(2-methoxyethyl)-1,3-benzothiazol-2(3H)-ylidene]benzamide

The product of Example 64A (39 mg, 0.14 mmol) and 2-iodobenzoic acid (42 mg, 0.17 mmol) were processed using the method described in Example 3 to afford the title compound. $^1$H NMR (500 MHz, DMSO-$d_6$) $\delta$ ppm 3.20 - 3.24 (m, 3 H) 3.81 (t, 2 H) 4.69 (t, 2 H) 7.15 - 7.25 (m, 1 H) 7.34 - 7.41 (m, 1 H) 7.47 - 7.62 (m, 2 H) 7.70 - 7.81 (m, 1 H) 7.87 - 7.97 (m, 2 H) 7.97 - 8.03 (m, 1 H); MS (ESI) m/z 439 $(M+H)^+$.

Example 94

3-iodo-N-[(2Z)-3-(2-methoxyethyl)-1,3-benzothiazol-2(3H)-ylidene]benzamide

The product of Example 64A (39 mg, 0.14 mmol) and 3-iodobenzoic acid (42 mg, 0.17 mmol) were processed using the method described in Example 3 to afford the title compound. $^1$H NMR (500 MHz, DMSO-$d_6$) $\delta$ ppm 3.26 - 3.27 (m, 3 H) 3.79 (t, 2 H) 4.76 (t, 2 H) 7.26 - 7.43 (m, 2 H) 7.48 - 7.61 (m, 1 H) 7.69 - 7.81 (m, 1 H) 7.89 - 8.00 (m, 2 H) 8.18 - 8.31 (m, 1 H) 8.49 - 8.59 (m, 1 H); MS (ESI) m/z 439 $(M+H)^+$.

Example 95

4-iodo-N-[(2Z)-3-(2-methoxyethyl)-1,3-benzothiazol-2(3H)-ylidene]benzamide

The product of Example 64A (39 mg, 0.14 mmol) and 4-iodobenzoic acid (42 mg, 0.17 mmol) were processed using the method described in Example 3 to afford the title compound. $^1$H NMR (500 MHz, DMSO-$d_6$) $\delta$ ppm 3.23 - 3.25 (m, 3 H) 3.80 (t, 2 H) 4.75 (t, 2 H) 7.29 - 7.45 (m, 1 H) 7.47 - 7.62 (m, 1 H) 7.70 - 7.80 (m, 1 H) 7.85 - 7.96 (m, 3 H) 7.98 - 8.05 (m, 2 H); MS (ESI) m/z 439 $(M+H)^+$.

Example 96

N-[(2Z)-3-(2-methoxyethyl)-1,3-benzothiazol-2(3H)-ylidene]-3-methylbutanamide

The product of Example 64A (39 mg, 0.14 mmol) and isovaleric acid (17 mg, 0.17 mmol) were processed using the method described in Example 3 to afford the title compound. $^1$H NMR (500 MHz, DMSO-$d_6$) $\delta$ ppm 0.93 (d, 6 H) 2.10 - 2.23 (m, 1 H) 2.37 (d, 2 H) 3.21 - 3.25 (m, 3 H) 3.74 (t, 2 H) 4.57 (t, 2 H) 7.31 (m, 1 H) 7.48 (m, 1 H) 7.66 (m, 1 H) 7.83 (m, 1 H); MS (ESI) m/z 393 $(M+H)^+$.

Example 97

N-[(2Z)-3-(2-methoxyethyl)-1,3-benzothiazol-2(3H)-ylidene]-2-methylpentanamide

The product of Example 64A (39 mg, 0.14 mmol) and 2-methylvaleric acid (20 mg, 0.17 mmol) were processed using the method described in Example 3 to afford the title compound. $^1$H NMR (500 MHz, DMSO-$d_6$) $\delta$ ppm 0.88 (t, 3 H) 1.14 (d, 3 H) 1.21 - 1.34 (m, 2 H) 1.35 - 1.47 (m, 1 H) 1.63-1.76(m, 1 H) 2.53-2.62 (m, 1 H) 3.22 - 3.26 (m, 3 H) 3.75 (t, 2 H) 4.58 (1, 2 H) 7.31 (m, 1 H) 7.49 (m, 1 H) 7.66 (m, 1 H) 7.83 (m, 1 H); MS (ESI) m/z 307 $(M+H)^+$.

Example 98

N-[(2Z)-3-(2-methoxyethy)-1,3-benzothiazol-2(3H)-ylidene]-3-methylpentanamide

The product of Example 64A (39 mg, 0.14 mmol) and 3-methylvaleric acid (20 mg, 0.17 mmol) were processed using the method described in Example 3 to afford the title compound. $^1$H NMR (500 MHz, DMSO-$d_6$) $\delta$ ppm 0.82 - 0.96 (m, 6 H) 1.14 - 1.27 (m, 1 H) 1.31 - 1.44 (m, 1 H) 1.87 - 2.05 (m, 1 H) 2.25 - 2.34 (m, 1 H) 2.43 - 2.49 (m, 1 H) 3.21 - 3.26 (m, 3 H) 3.74 (t, 2 H) 4.56 (t, 2 H) 7.31 (m, 1 H) 7.48 (m, 1 H) 7.66 (m, 1 H) 7.84 (m, 1 H); MS (ESI) m/z 307 $(M+H)^+$.

Example 99

N-[(2Z)-3-(2-methoxyethyl)-1,3-benzothiazol-2(3H)-ylidene]-4-methylpentanamide

The product of Example 64A (39 mg, 0.14 mmol) and 4-methylvaleric acid (20 mg, 0.17 mmol) were processed using the method described in Example 3 to afford the title compound. $^1$H NMR (500 MHz, DMSO-$d_6$) $\delta$ ppm 0.90 (d, 6 H) 1.49 - 1.61 (m, 3 H) 2.45 - 2.50 (m, 2 H) 3.21 - 3.26 (m, 3 H) 3.74 (t, 2 H) 4.58 (t, 2 H) 7.32 (m, 1 H) 7.48 (m, 1 H) 7.66 (m, 1 H) 7.84 (m, 1 H); MS (ESI) m/z 307 $(M+H)^+$.

Example 100

N-[(2Z)-3-(2-methoxyethyl)-1,3-benzothiazol-2(3H)-ylidene]-2,2-*d*imethylbutanamide

The product of Example 64A (39 mg, 0.14 mmol) and 2,2-dimethylbutyric acid (20 mg, 0.17 mmol) were processed using the method described in Example 3 to afford the title compound. $^1$H NMR (500 MHz, DMSO-$d_6$) $\delta$ ppm 0.76 (t, 3 H) 1.11 - 1.23 (m, 6 H) 1.62 (t, 2 H) 3.18 - 3.27 (m, 3 H) 3.75 (t, 2 H) 4.58 (t, 2 H) 7.26 (m, 1 H) 7.46 (m, 1 H) 7.66 (m, 1 H) 7.84 (m, 1 H); MS (ESI) m/z 307 $(M+H)^+$.

Example 101

N-[(2Z)-3-(2-methoxyethy)-1,3-benzothiazol-2(3H)-ylidene]-3,3-dimethylbutanamide

The product of Example 64A (0.64 g, 2.2 mmol) and 3,3-dimethyl-butyric acid (0.26 mL, 2.0 mmol) were processed using the method described in Example 3. Purification by column chromatography ($SiO_2$, 20-30% ethyl acetate/hexanes gradient) afforded 0.44 g (71 %) of the title compound. $^1$H NMR (DMSO-$d_6$, 300 MHz) $\delta$ ppm 1.03 (s, 9 H), 2.36-2.43 (s, 2 H), 3.22 (s, 3 H), 3.73 (t, *J*=5.4 Hz, 2 H), 4.56 (t, *J*=5.4 Hz, 2 H), 7.32 (m, 1 H), 7.49 (td, *J*=7.8, 1.0 Hz, 1 H), 7.67 (d, *J*=8.5 Hz, 1 H), 7.84 (dd, *J*=8.0, 1.2 Hz, 1 H); MS (DCI/$NH_3$) m/z 331 $(M+H)^+$. Anal. Calculated for $C_{16}H_{22}N_2O_2S$: C, 62.71; H, 7.24; N, 9.14. Found: C, 62.79; H, 7.41; N, 9.06.

Example 102

2-ethyl-N-[(2Z)-3 -(2-methoxyethyl)-1,3-benzothiazol-2(3H)-ylidene]butanamide

The product of Example 64A (39 mg, 0.14 mmol) and 2-ethylbutyric acid (20 mg, 0.17 mmol) were processed using the method described in Example 3 to afford the title compound. $^1$H NMR (500 MHz, DMSO-$d_6$) $\delta$ ppm 0.84 (t, 6 H) 1.47 - 1.59 (m, 2 H) 1.60 - 1.72 (m, 2 H) 2.27 - 2.39 (m, 1 H) 3.21 - 3.26 (m, 3 H) 3.75 (t, 2 H) 4.58 (t, 2 H) 7.32 (m, 1 H) 7.48 (m, 1 H) 7.65 (m, 1 H) 7.82 (m, 1 H); MS (ESI) m/z 307 $(M+H)^+$.

Example 103

N-[(2Z)-3-(2-methoxyethyl)-1,3-benzothiazol-2(3H)-ylidene]cyclopentanecarboxamide

The product of Example 64A (39 mg, 0.14 mmol) and cyclopentanecarboxylic acid (19 mg, 0.17 mmol) were processed using the method described in Example 3 to afford the title compound. $^1$H NMR (500 MHz, DMSO-$d_6$) $\delta$ ppm 1.50 - 1.62 (m, 2 H) 1.62 - 1.72 (m, 2 H) 1.76 - 1.95 (m, 4 H) 2.85 - 2.98 (m, 1 H) 3.22 - 3.26 (m, 3 H) 3.74 (t, 2 H) 4.56 (m, 2 H) 7.31 (m, 1 H) 7.48 (m, 1 H) 7.66 (m, 1 H) 7.83 (d, 1 H); MS (ESI) m/z 305 $(M+H)^+$.

Example 104

2-cyclopentyl-N-[(2Z)-3-(2-methoxyethyl)-1,3-benzothiazol-2(3H)-ylidene]acetamide

The product of Example 64A (39 mg, 0.14 mmol) and cyclopentylacetic acid (22 mg, 0.17 mmol) were processed using the method described in Example 3 to afford the title compound. $^1$H NMR (500 MHz, DMSO-$d_6$) $\delta$ ppm 1.09 - 1.25 (m, 2 H) 1.44 - 1.56 (m, 2 H) 1.55 - 1.65 (m, 2 H) 1.70 - 1.84 (m, 2 H) 2.20 - 2.38 (m, 1 H) 2.46 - 2.49 (m, 1 H) 3.21 - 3.24 (m, 3 H) 3.24 - 3.29 (m, 1 H) 3.74 (t, 2 H) 4.56 (t, 2 H) 7.32 (m, 1 H) 7.49 (m, 1 H) 7.65 (m, 1 H) 7.83 (m, 1 H); MS (ESI) m/z 319 $(M+H)^+$.

Example 105

N-[(2Z)-3-(2-methoxyethyl)-1,3-benzothiazol-2(3H)-ylidene]cyclohexanecarboxamide

The product of Example 64A (39 mg, 0.14 mmol) and cyclohexanecarboxylic acid (22 mg, 0.17 mmol) were processed using the method described in Example 3 to afford the title compound. $^1$H NMR (500 MHz, DMSO-$d_6$) δ ppm 1.13 - 1.36 (m, 3 H) 1.37 - 1.52 (m, 2 H) 1.58 - 1.67 (m, 1 H) 1.66 - 1.80 (m, 2 H) 1.84 - 2.00 (m, 2 H) 2.32 - 2.46 (m, 1 H) 3.22 - 3.25 (m, 3 H) 3.75 (t, 2 H) 4.56 (t, 2 H) 7.30 (m, 1 H) 7.48 (m, 1 H) 7.65 (m, 1 H) 7.83 (m, 1 H); MS (ESI) m/z 319 $(M+H)^+$.

Example 106

N-[(2Z)-3-(2-methoxyethyl)-1,3-benzothiazol-2(3H)-ylidene]-1-methylcyclohexanecarboxamide

The product of Example 64A (39 mg, 0.14 mmol) and 1-methylcyclohexane-carboxylic acid (24 mg, 0.17 mmol) were processed using the method described in Example 3 to afford the title compound. $^1$H NMR (500 MHz, DMSO-$d_6$) δ ppm 1.10 - 1.16 (m, 3 H) 1.21 - 1.39 (m, 5 H) 1.40 - 1.48 (m, 1 H) 1.48 - 1.58 (m, 2 H) 2.06 - 2.21 (m, 2 H) 3.21 - 3.25 (m, 3 H) 3.76 (t, 2 H) 4.58 (t, 2 H) 7.30 (m, 1 H) 7.47 (m, 1 H) 7.65 (m, 1 H) 7.82 (m, 1 H); MS (ESI) m/z 333 $(M+H)^+$.

Example 107

*cis-N*-[(2Z)-3-(2-methoxyethyl)-1,3-benzothiazol-2(3*H*)-ylidene]-2-methylcyclohexanecarboxamide

The product of Example 64A (39 mg, 0.14 mmol) and (cis)-2-methylcyclohexanecarboxylic acid (24 mg, 0.17 mmol) were processed using the method described in Example 3 to afford the title compound. $^1$H NMR (500 MHz, DMSO-$d_6$) δ ppm 0.79 (d, 3 H) 1.14 - 1.87 (m, 8 H) 2.24 - 2.39 (m, 1 H) 2.56 - 2.65 (m, 1 H) 3.19 - 3.23 (m, 3 H) 3.72 (t, 2 H) 4.54 (t, 2 H) 7.22 - 7.37 (m, 1 H) 7.42 - 7.50 (m, 1 H) 7.58 - 7.67 (m, 1 H) 7.75 - 7.88 (m, 1 H); MS (ESI) m/z 333 $(M+H)^+$.

Example 108

N-[(2Z)-3-(2-methoxyethyl)-1,3-benzothiazol-2(3H)-ylidene]-4-methylcyclobexanecarboxamide

The product of Example 64A (39 mg, 0.14 mmol) and 4-methylcyclohexanecarboxylic acid (24 mg, 0.17 mmol) were processed using the method described in Example 3 to afford the title compound. $^1$H NMR (500 MHz, DMSO-$d_6$) δ ppm 0.78 - 0.91 (m, 3 H) 0.94 - 1.26 (m, 2 H) 1.38 - 1.63 (m, 4 H) 1.69-2.01 (m, 1 H) 2.04-2.40 (m, 2 H) 2.53-2.65 (m, 1 H) 3.19 - 3.26 (m, 3 H) 3.76 (t, 2 H) 4.56 (t, 2 H) 7.23 - 7.37 (m, 1 H) 7.40 - 7.56 (m, 1 H) 7.59 - 7.70 (m, 1 H) 7.76 - 7.86 (m, 1 H); MS (ESI) m/z 333 $(M+H)^+$.

Example 109

3-cyclohexyl-N-[(2Z)-3-(2-methoxyethyl)-1,3-benzothiazol-2(3H)-ylidene]acetamide

The product of Example 64A (39 mg, 0.14 mmol) and cyclohexylacetic acid (24 mg, 0.17 mmol) were processed using the method described in Example 3 to afford the title compound. $^1$H NMR (500 MHz, DMSO-$d_6$) δ ppm 0.86 - 1.05 (m, 2 H) 1.06 - 1.35 (m, 3 H) 1.54 - 1.75 (m, 5 H) 1.77 - 1.92 (m, 1 H) 2.36 (d, 2 H) 3.19 - 3.25 (m, 3 H) 3.75 (t, 2 H) 4.54 (t, 2 H) 7.25 - 7.36 (m, 1 H) 7.43 - 7.56 (m, 1 H) 7.60 - 7.69 (m, 1 H) 7.77 - 7.88 (m, 1 H); MS (ESI) m/z 333 $(M+H)^+$.

Example 110

N-[(2Z)-3-(2-methoxyethyl)-1,3-benzothiazol-2(3H)-ylidene]cycloheptanecarboxamide

The product of Example 64A (39 mg, 0.14 mmol) and cycloheptylacetic acid (24 mg, 0.17 mmol) were processed using the method described in Example 3 to afford the title compound $^1$H NMR (500 MHz, DMSO-$d_6$) δ ppm 1.42 - 1.62 (m, 6 H) 1.63 - 1.82 (m, 4 H) 1.85 - 2.05 (m, 2 H) 2.54 - 2.72 (m, 1 H) 3.18 - 3.26 (m, 3 H) 3.72 (t, 2 H) 4.55 (t, 2 H) 7.23 - 7.40 (m, 1 H) 7.42 - 7.55 (m, 1 H) 7.60 - 7.72 (m, 1 H) 7.75 - 7.88 (m, 1 H); MS (ESI) m/z 333 $(M+H)^+$.

Example 111

N-[(2Z)-3-(2-methoxyethyl)-1,3-benzothiazol-2(3H)-ylidene]-5-methylthiophene-2-carboxamide

The product of Example 64A (39 mg, 0.14 mmol) and 5-methylthiophene-2-carboxylic acid (28 mg, 0.20 mmol) were processed using the method described in Example 3 to afford the title compound. $^1$H NMR (500 MHz, DMSO-*$d_6$*) δ ppm 2.51 (s, 3 H) 3.26 (s, 3 H) 3.82 (t, 2 H) 4.66 (t, 2 H) 6.91 - 6.93 (m, 1 H) 7.35 (t, 1 H) 7.51 (t, 1 H) 7.68 - 7.72 (m, 2 H) 7.89 (d, 1 H); MS (ESI) m/z 332 $(M+H)^+$.

Example 112

N-[(2E)-6-fluoro-3-(2-methoxyethyl)-1,3-benzothiazol-2(3H)-ylidene]-2,2,3,3-tetramethylcyclopropanecarboxamide

Example 112A

*N*-(6-fluoro-1,3-benzothiazol-2-yl)-2,2,3,3-tetramethylcyclopropanecarboxamide

A mixture of 6-fluoro-benzothiazol-2-ylamine (1 equiv), N-(3-dimethylaminopropyl)-N-ethylcarbodiimide hydrochloride (1 equiv), 1-hydroxybenzotriazole, triethylamine (1.1 equiv), and 2,2,3,3-tetramethylcyclopropanecarboxylic acid (0.8 equiv) in 3:1 THF/$Et_3N$ (1 M) and were stirred overnight at room temperature. The mixture was diluted with EtOAc, washed with 1 M aqueous $NaHCO_3$, dried ($Na_2SO_4$), filtered, and concentrated. Puried by silica gel chromatography afforded the title compound. MS ($ESI^+$) m/z 293 $(M+H)^+$.

Example 112B

2,2,3,3-Tetramethylcyclopropanecarboxylic acid [6-fluoro-3-(2-methoxyethyl)-3*H*-benzothiazol-2-ylidene]-amide

To a solution of the product of Example 112A (1 equiv) in 1:1 DMF/THF (0.1 M) was added sodium hydride (60% dispersion in mineral oil, 1.2 equiv) and 2-bromoethyl methyl ether (1.2 equiv). The mixture was stirred at 65 °C overnight then cooled to ambient temperature and diluted with EtOAc. The mixture was washed with I M saturated aqueous $NaHCO_3$, dried ($Na_2SO_4$), filtered and concentrated. Purifed by silica gel chromatography afforded the title compound. $^1$H NMR (300 MHz, DMSO-$d_6$) δ ppm 1.20 (s, 6 H), 1.27 (s, 6 H), 1.60 (s, 1 H), 3.23 (s, 3 H), 3.72 (t, *J*=5.4 Hz, 2 H), 4.54 (t, *J*=5.4 Hz, 2 H), 7.33 (td, *J*=9.0, 2.7 Hz, 1 H), 7.65 (dd, *J*=8.8, 4.4 Hz, 1 H), 7.75 (dd, *J*=8.1, 2.7 Hz, 1 H); MS ($ESI^+$) m/z 351 $(M+H)^+$.

Example 113

2-cyclopentyl-N-[(2Z)-6-fluoro-3-(2-methoxyethyl)-1,3 -benzothiazol-2(3H)-ylidene]acetamide

Example 113A

6-Fluoro-3-(2-methoxyethyl)-3*H*-benzothiazol-2-ylideneamine hydrobromide

Commercially available 6-fluoro-benzothiazol-2-ylamine and 2-bromoethyl) methyl ether were processed as described for example 12A to afford the title compound. MS ($ESI^+$) m/z 227 $(M+H)^+$.

Example 113B

2-cyclopentyl-N-[(2Z)-6-fluoro-(2-methoxyethyl)-1,3-benzothiazol-2(3H)-ylidene]acetamide

The product from Example 113A and cyclopentylacetyl chloride were processed as described for example 11 to afford the title compound. $^1$H NMR (400 MHz, DMSO-$d_6$) δ ppm 4.54 (d, *J*=5.76 Hz, 2 H) 7.65 (t, *J*=8.14 Hz, 1 H) 7.75 (dd, *J*=8.81, 2.71 Hz, 1 H) 7.83 (dd, *J*=7.97, 1.53 Hz, 2 H) 7.97 (dd, *J*=8.14, 1.36 Hz, 1 H) 8.41 (d, *J*=3.05 Hz, 1 H); MS ($ESI^+$) m/z 337 $(M+H)^+$; Anal. Calculated for $C_{17}H_{21}FN_2O_2S$: C, 60.69; H, 6.29; N, 8.33. Found: C, 60.67; H, 6.41; N, 8.25.

Example 114

N-[(2 Z)-6-fl uoro-3-(2-methoxyethyl)-1,3-benzothiazol-2(3H)-ylidene]-2-tetrahydro-2H-pyran-4-ylacetamide

Example 114A

(Tetrahydro-pyran-4-yl)-acetyl chloride

Commercially available (tetrahydro-pyran-4-yl)-acetic acid and oxalyl chloride were processed as described for example 9A to afford the title compound. MS ($DCI/NH_3$) m/z 159 $(M+H)^+$.

Example 114B

N-[6-Fluoro-3-(2-methoxyethyl)-3*H*-benzothiazol-2-ylidene]-2-(tetrahydro-pyran-4-yl)-acetamide

The product from Example 113A and the product from Example 114A were processed as described for Example 11 to afford the title compound. $^1H$ NMR (300 MHz, DMSO-$d_6$) δ ppm 1.13 - 1.35 (m, 2 H), 1.52 - 1.66 (m, 2 H), 2.05 (d, 1 H), 2.43 (d, *J*=7.1 Hz, 2 H), 3.22 (s, 3 H), 3.72 (t, *J*=5.3 Hz, 2 H), 3.78 - 3.88 (m, 4 H), 4.56 (t, *J*=5.3 Hz, 2 H), 7.36 (td, *J*=9.0, 2.7 Hz, 1 H), 7.70 (dd, *J*=9.0, 4.2 Hz, 1 H), 7.81 (dd, *J*=8.5,2.7 Hz, 1 H); MS ($ESI^+$) m/z 353 $(M+H)^+$; Anal. Calculated for $C_{17}H_{21}FN_2O_3S$: C, 57.94; H, 6.01; N, 7.95. Found: C, 58.03; H, 5.97; N, 7.87.

Example 115

5-fluoro-N-[(2Z)-6-fluoro-3-(2-methoxyethyl)-1,3-benzothiazol-2(3H)-ylidene]-2-methoxybenzamide

The product from Example 113A and 5-fluoro-2-methoxy-benzoic acid were processed using the methods described in Example 13 to afford the title compound. $^1H$ NMR (300 MHz, DMSO-$d_6$) δ ppm 3.23 (s, 3 H), 3.72 - 3.87 (m, 5 H), 4.65 (t, *J*=5.4 Hz, 2 H), 7.15 (dd, *J*=9.3, 4.2 Hz, 1 H), 7.37 (dd, 2 H), 7.64 (dd, *J*=9.2, 3.4 Hz, 1 H), 7.77 (dd, *J*=9.2, 4.4 Hz, 1 H), 7.88 (dd, *J*=8.1, 2.7 Hz, 1 H); MS ($ESI^+$) m/z 379 $(M+H)^+$; Anal. Calculated for $C_{18}H_{16}F_2N_2O_3S$: C, 57.13; H, 4.26; N, 7.40. Found: C, 57.05; H, 4.08; N, 7.35.

Example 116

5-chloro-N-[(2Z)-6-fluoro-3-(2-methoxyethyl)-1,3-benzothiazol-2(3H)-ylidene]-2-methoxybenzamide

The product from Example 113A and 5-chloro-2-methoxybenzoic acid were processed using the methods described in Example 13 to afford the title compound. $^1H$ NMR (300 MHz, DMSO-$d_6$) δ ppm 3.24 (s, 3 H), 3.79 (t, *J*=5.3 Hz, 2 H), 3.83 (s, 3 H), 4.65 (t, *J*=5.3 Hz, 2 H), 7.17 (d, *J*=9.2 Hz, 1 H), 7.41 (td, *J*=9.1, 2.5 Hz, 1 H), 7.52 (dd, *J*=8.8, 2.7 Hz, 1 H), 7.78 (dd, *J*=9.0, 4.2 Hz, 1 H), 7.83 (d, *J*=2.7 Hz, 1 H), 7.89 (dd, *J*=8.5, 2.7 Hz, 1 H); MS ($ESI^+$) m/z 395 $(M+H)^+$; Anal. Calculated for $C_{18}H_{16}ClFN_2O_3S$: C, 54.75; H, 4.08; N, 7.09. Found: C, 54.29; H, 3.94; N, 6.99.

Example 117

N-[(2Z)-3-(2-methoxyethyl)-4-methyl-5-moroholin-4-yl-1,3-thiazol-2(3H)-ylidene]-2,2,3,3-tetramethylcyclopropanecarboxamide

Example 117 A

2,2,3,3-Tetramethylcyclopropanecarboxylic acid (4-methyl-5-morpholin-4-yl-thiazol-2-yl)-amide

A mixture of 4-methyl-5-morpholin-4-yl-thiazol-2-ylamine (prepared using the method described in Christopher et al., Bioorganic and Medicinal Chemistry Letters 2004, 14(22), 5521-5525), N-(3-dimethylaminopropyl)-N-ethylcarbodiimide hydrochloride, 1-hydroxybenzotriazole, triethylamine, and 2,2,3,3-tetramethylcyclopropanecarboxylic acid were processed using the method described in Example 58 to afford the title compound MS ($ESI^+$) m/z 324 $(M+H)^+$.

Example 117B

2,2,3,3-Tetramethylcyclopropanecarboxylic acid [3-(2-methoxyethy\)-4-methyl-5-morpholin-4-yl-3H-thiazol-2-ylidene]-amide

The product from Example 117A was processed using the method described in Example 112B to afford the title compound. $^1$H NMR (300 MHz, DMSO-$d_6$) δ ppm 1.16 (s, 6 H), 1.23 (s, 6 H), 1.44(s, 1 H), 2.22 (s, 3 H), 2.70 - 2.79 (m, 4 H), 3.24 (s, 3 H), 3.62 (t, J=5.3 Hz, 2 H), 3.66 - 3.72 (m, 4 H), 4.21 (t, J=5.4 Hz, 2 H); MS (ESI$^+$) m/z 382 $(M+H)^+$.

Example 118

N-[f(2Z)-5-chloro-3-(2-methoxvethyl)-4-methyl-1,3-thiazol-2(3H)-ylidene]-2,2,3,3-tetramethylcyclopropanecarboxamide

Example 118A

2,2,3,3-Tetramethylcyclopropanecarboxylic acid (5-chloro-4-methyl-thiazol-2-yl)-amide

A mixture of 2-amino-5-chloro-2-methyl-thiazole (Matsuo, Masaaki; Ogino, Takashi; Igari, Norihiro; Seno, Hachiro; Shimomura, Kyoichi., EP 412404) (150 mg, 0.81 mmol), 2,2,3,3-tetramethylcyclopropanecarbonyl chloride (143 mg, 0.89 mmol), 4-dimethylaminopyridine (50.0 mg, 0.41 mmol) and triethylamine (226 μL, 1.62 mmol) in 15 mL of THF heated at reflux for 48 hours. The mixture was cooled to ambient temperature, diluted with EtOAc and washed with brine. The layers were separated and the aqueous phase was extracted with EtOAc (2X). The combined organic extracts were dried ($Na_2SO_4$), filtered, and concentrated. Purification by silica gel chromatography afforded the title compound: MS (LC/MS) m/z 273 $(M+H)^+$.

Example 118B

2,2,3,3-Tetramethylcyclopropanecarboxylic acid (5-chloro-3- (2-methoxy-ethy)- 4-methyl-3*H*-thiazol-2-ylidene]-amide

A mixture of the product of Example 118A (150 mg, 0.55 mmol), NaH (60% dispersion in mineral oil, 29.0 mg, 0.71 mmol) and 2-bromoethyl methyl ether (57 μL, 0.61 mmol) in 20 mL of 2:1 THF/DMF was processed according to the method desribed in Example 112B to provide the title compound: $^1$H NMR (300 MHz, DMSO-$d_6$) δ 1.18 (s, 6 H) 1.23 (s, 6 H) 1.50 (s, I H) 2.29 (s, 3 H) 3.25 (s, 3 H) 3.63 (t, *J*=5.3 Hz, 2 H) 4.27 (t, *J*=5.3 Hz, 2 H); MS (DCI/$NH_3$) m/z 331 $(M+H)^+$.

Example 119

N-[(2Z)-3-(2-methoxyethyl)-5-methyl-4-phenyl-1,3-thiazol-2(3H)-ylidene]-2,2,3,3-tetramethylcyclopropanecarboxamide

Example119A

3-(2-methoxyethyl)-5-methyl-4-phenyl-3*H*-thiazol-2-ylideneamine hydrobromide

A mixture of 5-methyl-4-phenyl-thiazol-2-ylamine (300 mg, 1.58 mmol) and 2-bromoethyl methyl ether (300 μL, 3.20 mmol) was processed using the method described in Example 12A to provide the title compound.

Example 119B

2,2,3,3-Tetramethylcyclopropanecarboxylic acid [3-(2-methoxy-ethy)-5-methyl-4-phenyl-3*H*-thiazol-2-ylidene]-amide

A mixture of the product of Example 119A (290 mg, 1.16 mmol), 2,2,3,3-tetramethylcyclopropanecarboxylic acid (182 mg, 1.28 mmol), HATU (661 mg, 1.74 mmol) and triethylamine (0.97 mL, 6.96 mmol) in 15 mL of DMF was processed according to the method of Example 2B to provide the title compound: $^1$H NMR (400 MHz, DMSO-$d_6$.) δ 1.17 (s, 6 H) 1.26 (s, 6 H) 1.49 (s, 1 H) 2.01 (s, 3 H) 3.02 (s, 3 H) 3.43 (t, *J*=6.0 Hz, 2 H) 4.06 (t, *J*=5.8 Hz, 2 H) 7.39 - 7.43 (m, 2 H) 7.50 - 7.57 (m, 3 H); MS (DCI/$NH_3$) m/z 373 $(M+H)^+$.

Example 120

N-[(2Z)-4-(4-chlorophenyl)-3-(2-methoxyethyl)-5-methyl-1,3-thiazol-2(3H)-ylidene]-2,2,3,3-tetramethylcyclopropanecarboxamide

Example 120A

4-(4-chlorophenyl)-3-(2-methoxyethyl)-5-methyl-3*H*-thiazol-2-ylideneamine hydrobromide

A mixture of 4-(4-chlorophenyl)-5-methyl-thiazol-2-ylamine (420 mg, 1.87 mmol) and 2-bromoethyl methyl ether (600 μL, 6.40 mmol) was processed according to the method of Example 12A to provide the title compound: MS (DCI/$NH_3$) m/z 283 $(M+H)^+$.

Example 120B

2,2,3,3-Tetramethylcyclopropanecarboxylic acid [4-(4-chlorophenyl)-3-(2-methoxy-ethy)-5-methyl-3*H*-thiazol-2-ylidene]-amid

A mixture of the product of Example 120A (156 mg, 0.55 mmol), 2,2,3,3-tetramethylcyclopropanecarboxylic acid (94 mg, 0.66 mmol), HATU (479 mg, 0.83 mmol) and triethylamine (0.46 mL, 3.30 mmol) in 10 mL of DMF was processed according to the method of Example 2B to provide the title compound: $^1H$ NMR (500 MHz, DMSO-$d_6$) δ 1.17 (s, 6 H) 1.25 (s, 6 H) 1.49 (s, 1H) 2.00 (s, 3 H) 3.04 (s, 3 H) 3.44 (t, *J*=5.8 Hz, 2 H) 4.04 (t, *J*=5.8 Hz, 2 H) 7.45 (d, *J*=8.2 Hz, 2 H) 7.60 (d, *J*=8.5 Hz, 2 H); MS (DCI/$NH_3$) m/z 407 $(M+H)^+$.

Example 121

N-[(2Z)-3-(2-methoxyethyl)-4,5,6,7-tetrahydro-1,3-benzothiazol-2(3H)-ylidene]-2,2,3,3-tetramethylcyclopropanecarboxamide

Example 121A

3-(2-methoxy-ethy)-4,5,6,7-tetrahydro-3*H*-benzothiazol-2-ylideneamine hydrobromide

A mixture of 4,5,6,7-tetrahydro-benzothiazol-2-ylamine (300 mg, 1.94 mmol) and 2-bromoethyl methyl ether (600 μL, 6.40 mmol) was processed according to the method of Example 12A to provide the title compound as crude product: MS (LC/MS) m/z 213 $(M+H)^+$.

Example 121B

2,2,3,3-Tetramethylcyclopropanecarboxylic acid [3-(2-methoxy-ethy)-4,5,6,7-tetrahydro-3*H*-benzothiazol -2-ylidene]-amide

A mixture of the product of Example 121A (156 mg, 0.55 mmol), 2,2,3,3-tetramethylcyclopropanecarboxylic acid (94 mg, 0.66 mmol), HATU (479 mg, 0.83 mmol) and triethylamine (0.46 mL, 3.30 mmol) in 10 mL of DMF was processed according to the method of Example 2B to provide the title compound: $^1H$ NMR (500 MHz, DMSO-$d_6$) δ 1.16 (s, 6 H) 1.23 (s, 6 H) 1.45(s, 1 H) 1.69 - 1.82 (m, 4 H) 2.43 - 2.48 (m, 2 H) 2.54 - 2.59 (m, 2 H) 3.24 (s, 3 H) 3.60 (t, *J*=5.3 Hz, 2 H) 4.16 (t, *J*=5.3 Hz, 2 H); MS (DCI/$NH_3$) m/z 337 $(M+H)^+$.

Example 122

N-[(2Z)-3-(2-methoxyethyl)-3,4,5,6-tetrahydro-2H-cyclopenta[d][1,3]thiazol-2-ylidenel-2,2,3,3-tetramethylcyclopropanecarboxamide

Example 122A

2,2,3,3-Tetramethylcyclopropanecarboxylic acid (5,6-dihydro-4*H*-cyclopentathiazol-2-yl)-amide

A mixture of 5,6-dihydro-4*H*-cyclopentathiazole-2-ylamine (177 mg, 1.26 mmol), 2,2,3,3-tetramethylcyclopropanecarboxylic acid (244 mg, 1.52 mmol), 4-dimethylaminopyridine (50.0 mg, 0.41 mmol) and triethylamine (351 μL, 2.52 mmol) in 20 mL of THF was processed according to the method of Example 118A to provide the title compound:

MS ($DCI/NH_3$) m/z 265 $(M+H)^+$.

Example 122B

2,2,3,3-Tetramethylcyclopropanecarboxylic acid[3-(2-methoxy-ethyl) (3,4,5,6-tetrahydrocyclopentathiazol-2 -ylidene)-amide

A mixture of the product of Example 122A (254 mg, 0.95 mmol), NaH (60% dispersion in mineral oil, 50.0 mg, 1.22 mmol) and 2-bromoethyl methyl ether (100 μL, 1.07 mmol) in 30 mL of THF/DMF (2/1) was processed according to the method of Example 112B to provide the title compound: $^1$H NMR (500 MHz, DMSO-$d_6$) δ 1.16 (s, 6 H) 1.23 (s, 6 H) 1.45 (s, 1H) 2.31 - 2.38 (m, 2 H) 2.72 (t, *J*=7.0 Hz, 2 H) 2.78 (t, *J*=7.2 Hz, 2 H) 3.24 (s, 3 H) 3.61 (t,*J*=5.3 Hz, 2 H) 4.15 (t, *J*=5.2 Hz, 2 H); MS ($DCI/NH_3$) m/z 323 $(M+H)^+$.

Example 123

N-[(7Z)-8-(2-methoxyethyl)-5,8-dihydro[1,3]thiazolo[4,5-e][2,1,3]benzoxadiazol-7(4H)-ylidene]2,2,3,3-tetramethylcyclopropanecarboxamide

Example 123A

4,5-dihydro[1,3]thiazolo[4,5-*e*][2,1,3]benzoxadiazol-7-amine hydrobromide

To a solution of commercially available 5-bromo-6,7-dihydro-5*H*-benzo[1,2,5]oxadiazol-4-one (1.1 g, 5.1 mmol) in absolute ethanol (60 mL) was added thiourea. The reaction mixture was stirred at 60 °C for overnight and then concentrated. The residue was triturated in hexanes to afford 1.3 g (90%) of the title compound. MS ($ESI^+$) m/z 195 $(M+H)^+$.

Example 123B

*N*-4,5-dihydro[1,3]thiazolo[4,5-*e*][2,1,3]benzoxadiazol-7-yl-2,2,3,3-tetramethylcyclopropanecarboxamide

Example 123A and 2,2,3,3-tetramethylcyclopropanecarbonyl chloride were processed as described for example 118A to afford the title compound. MS ($ESI^+$.) m/z 319 $(M+H)^+$.

Example 123C

*N*-[(7Z)-8-(2-methoxyethyl)-5,8-dihydro[1,3]thiazolo[4,5-*e*][2,1,3]benzoxadiazol-7(4*H*)-ylidene]-2,2,3,3-tetramethylcyclopronanecarboxamide

The product of Example 123B (1equiv), potassium tert-butoxide (1.1 equiv) and 2-bromoethyl methyl ether (1 equiv) were combined in DMF (0.1 M) and heated in a SmithSynthesizer™ microwave at 250 °C for 15 minutes. The mixture was diluted with EtOAc and washed with 1 M aqueous $NaHCO_3$. The phases were separated and the aqueous phase was extracted with EtOAc (3X). The combined organic extracts were dried over $Na_2SO_4$, filtered, and concentrated. Purification by silica gel chromatography afforded the title compound and the product of Example 124. $^1$HNMR(400MHz, DMSO-$d_6$) δ ppm 1.20 (s,6H), 1.26 (s, 6 H), 1.57 (s, 1 H),3.10(t, *J*=7.5 Hz, 2 H), 3.23 (s, 3 H), 3.26 (t, *J*=7.4 Hz, 2 H), 3.71 (t, *J*=5.8 Hz, 2 H), 4.67 (t, *J*=5.8 Hz, 2 H); MS ($ESI^+$) m/z 377 $(M+H)^+$.

Example 124

N-[(7Z)-8-(2-methoxyethyl)[1,3]thiazolo[4,5-*e*][2,1,3]benzoxadiazol-7(8*H*)-ylidene]-2,2,3,3-tetramethylcyclopropanecarboxamide

The title compound was obtained as byproduct during the synthesis of example 123C. $^1$H NMR (400 MHz, DMSO-$d_6$) δ ppm 1.23 (s, 6 H), 1.29 (s, 6 H), 1.67 (s, 1 H), 3.24 (s, 3 H), 3.85 (t, *J*=5.7 Hz, 2 H), 4.96 (t, *J*=5.5 Hz, 2 H), 7.94 (d, *J*=9.2 Hz, 1 H), 8.06 (d, *J*=9.5 Hz, 1 H); MS ($ESI^+$) m/z 374 $(M+H)^+$.

Example 125

2-Ethoxy-N-[(2Z)-3-(2-methoxyethyl)-4,6-dihydrofuro[3,4-d]thiazol-2(3H)-ylidene]-benzamide

Example 125A

4-Bromo-dihydrofuran-3-one

The title compound was prepared according to the procedure using the method described in Baker, Tracy J, Wiemer, David F, J. Org. Chem., 1998, 63(8), 2613-2618 and was used immediately using the method described in Example 125B.

Example 125B

3*a*-Ethoxy-3*a*,4,6,6*a*-tetrahydrofuro[3,4-d]thiazol-2-ylamine

The product of Example 125A and thiourea were processed using the method described in Example 123A to afford the title compound. MS (ESI$^+$) m/z 189 $(M+H)^+$.

Example 125C

3*a*-Ethoxy-3-(2-methoxy-ethyl)-tetrahydro-furo[3,4-d]thiazol-2-ylideneamine

The product from Example 125B and 1-bromo-2-methoxyethane were processed using the method described in Example 12A to afford the title compound. MS (ESI$^+$) m/z 247 $(M+H)^+$.

Example 125D

2-Ethoxy-N-[3a-ethoxy-3-(2-methoxy-ethyl)-tetrahydro-furo[3,4-d]thiazol-2-ylidene]-benzamide

The product from Example 125B and 2-ethoxybenzoyl chloride were processed as described for example 118A to afford the title compound. MS (ESI$^+$) m/z 395 $(M+H)^+$.

Example 125E

2-Ethoxy- N-[3-(2-methoxyethyl)-4,6-dihydro-3*H*-furor[3,4-d]thiazol-2-ylidene]-benzamide

To a solution of the product from Example 125D (15 mg, 0.04 mmol) in toluene (10 mL) was added *p*-toluenesulfonic acid monohydrate (2 mg). The mixture was refluxed for 3 hours and then cooled to room temperature, diluted with ethyl acetate, washed with 1 M $NaHCO_3$, dried ($Na_2SO_4$), filtered and concentrated. Purification by preparative HPLG on a waters Symmetry C8 column (40 mm X 100 mm, 7 μm particle size) using a gradient of 10 % to 100 % acetonitrile: ammonium acetate (10 mM) over 15 min at a flow rate of 70 mL/min afforded the title compound. $^1H$ NMR (300 MHz, DMSO-$d_6$) δ ppm 1.33 (t, *J*=7.0 Hz, 3 H), 3.24 (s, 3 H), 3.65 (t, *J*=4.9 Hz, 2 H), 4.07 (q, *J*=6.8 Hz, 2 H), 4.25 (t, *J*=5.1 Hz, 2 H), 4.96 (s, 4 H), 6.92 - 7.01 (m, 1H), 7.07 (d, *J*=8.1 Hz, 1H), 7.36 - 7.44 (m, 1H), 7.74 (dd, *J*=7.5, 1.7 Hz, I H); MS (ESI$^+$) m/z 349 $(M+H)^+$.

Example 126

3-chloro-2-fluoro-N-[(2Z)-3-(2-methoxyethyl)-4,5-dimethyl-1,3-thiazol-2(3H)-ylidene]-6-(trifluoromethyl)benzamide

Example 126A

3-(2-Methoxy-ethyl)-4,5-dimethyl-3H-thiazol-2-ylideneamine

The product of Example 12A was purified via flash column chromatography ($SiO_2$, 9:1:0.1 $CH_2Cl_2$: $CH_3OH$: $NH_4OH$) to provide the title compuond. $^1H$ NMR (300 MHz, $CDCl_3$) δ ppm 2.18 (s, 6 H) 3.32 (s, 3 H) 3.78 (t, *J*=5.10 Hz, 2 H) 4.39 (t, *J*=4.70 Hz, 2 H) 9.45 (s, 2 H); MS ($DCI/NH_3$) m/z 187 $(M+H)^+$.

Example 126B

3-Ghloro-2-fluoro-N-[3-(2-methoxy-ethyl)-4,5-dimethyl-3H-thiazol-2-ylidene]-6-trifluoromethylbenzamide

To a suspension of the product of Example 126A (0.20 g, 1.1 mmol) in 35 mL THF was added $Et_3N$ (0.37 mL, 2.7 mmol). This mixture was cooled to 0 °C and 3-chloro-2-fluoro-6-trifluoromethyl benzoyl chloride (Alfa Aesar, 0.35 g, 1.3 mnol) in 5 mL THF was added dropwise via syringe. The mixture was allowed to stir at ambient temperature for 1 hour, then was wanned to reflux and was allowed to stir for 8 hours. The mixture was then cooled to ambient temperature and filtered. The filtrate was concentrated under reduced pressure and purified via flash colwnn chromatography ($SiO_2$, 7:3 hexanes:EtOAc) to provide the title compound (0.20 g, 0.50 mmol, 46% yield). $^1H$ NMR (300 MHz, $CDCl_3$) $\delta$ ppm 2.27 (s, 3 H), 2.28 (s, 3 H), 3.27 (s, 3 H), 3.68 (t, *J*=4.7 Hz, 2 H), 4.28-4.37 (m, 2 H), 7.37-7.43 (m, 1 H), 7.44-7.52 (m, 1 H); MS (DCI/$NH_3$) m/z 411 $(M+H)^+$; Anal. calculated for $C_{16}H_{15}ClF_4N_2O_2S$: C, 46.78; H, 3.68; N, 6.82. Found: C, 46.83; H, 3.30; N, 6.65.

Example 127

5-chloro-N-[(2Z)-3-(2-methoxyethyl)-4,5-dimethyl-l,3-thiazol-2(3H)-ylidene]-2-(trifluoromethyl)benzamide

To the product of Example 126A (0.20 g, 1. 1 mnol) in 35 mL THF at 0 °C was added $Et_3N$ (0.37 mL, 2.7 mmol) followed by 5-chloro-2-trifluoromethyl-benzoyl chloride (Matrix, 0.26 g, 1.3 mmol) in 5 mL THF dropwise via syringe. This mixture was stirred at ambient temperature for I hour then was warmed to reflux and allowed to stir for 8 hours. The mixture was then cooled to ambient temperature and filtered. The filtrate was concentrated under reduced pressure and the residue was purified via flash column chromatography ($SiO_2$, 4:1 hexanes:EtOAc) to provide the title compound (0.23 g, 0.57 mmol, 53% yield). $^1H$ NMR (300 MHz, $CDCl_3$) $\delta$ ppm 2.26 (s, 3 H), 2.28 (s, 3 H), 3.30 (s, 3 H), 3.71 (t, *J*=4.9 Hz, 2 H), 4.36 (t, *J*=4.9 Hz, 2 H), 7.45 (ddd, *J*=8.5, 2.0,0.7 Hz, 1 H), 7.64 (d, *J*=8.5 Hz, 1 H), 7.84 (d, *J*=2.0 Hz, 1H); MS (DCI/$NH_3$) m/z 393 $(M+H)^+$; Anal. calculated for $C_{16}H_{16}ClF_3N_2O_2S$: C, 48.92; H, 4.11; N, 7.13. Found: C, 48.66; H, 3.81; N, 7.01.

Example 128

2,3-dichloro-N-[(2Z)-3-(2-methoxyethyly-4,5-dimethyl-1,3-thiazol-2(3H)-ylidene]benzamide

To the product of Example 126A (0.20 g, 1.1 mmol) in 35 mL THF was added $Et_3N$ (0.37 mL, 2.7 mmol). This mixture was cooled to 0 °C and 2,3-dichlorobenzoyl chloride (Lancaster, 0.27 g, 1.3 mmol) in 5 mL THF was added dropwise via syringe. This mixture stirred at ambient temperature for 1 hour then was wanned to reflux and was allowed to stir for 3 hours. The mixture was cooled to ambient temperature and was quenched with 5 mL saturated, aqueous $NH_4Cl$ and diluted with 10 mL EtOAc. The layers were separated and the aqueous layer was extracted 2 X 5 mL EtOAc and 2 X 5 mL $CH_2Cl_2$. The combined organics were washed 1 X 5 mL saturated, aqueous NaCl then were dried over anhydrous $Na_2SO_4$, filtered, concentrated under reduced pressure and purified via flash column chromatography ($SiO_2$, 10% $CH_3OH$:EtOAc). The material was still impure so it was purified again via flash column chromatography ($SiO_2$, 1: 1 hexanes:EtOAc) to provide the title compound (0.105 g, 0.29 mmol, 27% yield). $^1H$ NMR (300 MHz, $CDCl_3$) $\delta$ ppm 2.26 (s, 3 H), 2.28 (s, 3 H), 3.30 (s, 3 H), 3.76 (t, *J* = 4.6 Hz, 2 H), 4.40 (t, *J*=4.2 Hz, 2 H), 7.23 (t, *J*=7.7 Hz, I H), 7.49 (dd, *J*=8.0, 1.5 Hz, I H), 7.72 (dd, *J*=7.6, 1.5 Hz, 1 H); MS (DCI/$NH_3$) m/z 359 $(M+H)^+$; Anal. calculated for $C_{15}H_{16}Cl_2N_2O_2S$: C, 50.15; H, 4.49; N, 7.80. Found: C, 50.17; H, 4.26; N, 7.69.

Example 129

N-[(2Z)-3-(2-methoxyethyl)-4,5-dimethyl-1,3-thiazol-2(3H)-ylidene]-2,2-dimethyl-2,3-dihydro-1-benzofuran-7-carboxamide

To a solution of the product of Example 126A (0.20 g, 1.1 mmol) and $Et_3N$ (0.45 mL, 3.2 mmol) in 30 mL THF was added 2,2-dimethyl-2,3-dihydro-1-benzofuran-7-carbonyl chloride (Acros, 0.34 g, 1.6 mmol). This mixture was warmed to reflux and allowed to stir for 2 hours. The mixture was then cooled to ambient temperature and concentrated under reduced pressure. The residue was diluted with 10 mL EtOAc and washed with 5 mL saturated, aqueous $NH_4Cl$. The layers were separated and the aqueous layer was extracted 2 x 5 mL EtOAc. The combined organics were dried over anhydrous $Na_2SO_4$, filtered, concentrated under reduced pressure and purified via flash column chromatography ($SiO_2$, 4:1 hexanes:EtOAc) to provide the title compound (0.21 mmol, 0.57 mmol, 53% yield). $^1H$ NMR (300 MHz, $CDCl_3$) $\delta$ ppm 1.55 (s, 6 H), 2.22 (s, 3 H), 2.27 (s, 3 H), 3.02 (s, 2 H), 3.31 (s, 3 H), 3.77-3.89 (m, 2 H), 4.33-4.50 (m, 2 H), 6.85

(t, *J*=7.5 Hz, 1H), 7.22 (d, *J*=7.5 Hz, 1 H), 8.00 (d, *J*=8.1 Hz, 1 H); MS (DCI/$NH_3$) m/z 361 $(M+H)^+$; Anal. calculated for $C_{19}H_{24}N_2O_3S$ : C, 63.31; H, 6.71; N, 7.77. Found: C, 63.19; H, 6.50; N, 7.66.

Example 130

2,2-difluoro-N-[(2Z)-3-(2-methoxyethyl)-4,5-dimethyl-1,3-thiazol-2(3H)-ylidene]-1,3-benzodioxole-4-carboxamide

To the product of Example 126A (0.20 g, 1.1 mmol) in 30 mL THF was added $Et_3N$ (0.37 mL, 2.7 mmol) followed by 2,2-difluoro-1,3-benzodioxole-4-carbonyl chloride (Lancaster, 0.29 g, 1.3 mmol). This mixture was stirred at ambient temperature for 17 hours then was wanned to reflux and allowed to stir for an additional 4 hours. The mixture was then cooled to ambient temperature and additional 2,2-difluoro-1,3-benzodioxole-4-carbonyl chloride (73 mg, 0.33 mmol) and $Et_3N$ (0.37 mL, 2.7 mmol) were added. This mixture was wanned to reflux at which temperature it stirred for 2 hours. The mixture was then cooled to ambient temperature, diluted with 10 mL EtOAc and washed with 5 mL saturated, aqueous $NH_4Cl$. The layers were separated and the aqueous layer was extracted 2 x 5 mL EtOAc. The combined organics were dried over anhydrous $Na_2SO_4$, filtered, concentrated under reduced pressure and purified via flash column chromatography ($SiO_2$, 50% hexanes:EtOAc) to afford the title compound (0.22 g, 0.59 mmol, 55% yield). $^1H$ NMR (300 MHz, $CDCl_3$) $\delta$ ppm 2.26 (s, 3 H), 2.28 (s, 3 H), 3.32 (s, 3 H), 3.83 (t, *J*=5.1 Hz, 2 H), 4.44 (t, *J*=5.1 Hz, 2 H), 7.12 (d, *J*=4.4 Hz, 1 H), 7.14 (s, 1 H), 7.89 (dd, *J*=6.6, 2.9 Hz, 1 H); MS (DCI/$NH_3$) m/z 371 $(M+H)^+$; Anal. calculated for $C_{16}H_{16}F_2N_2O_4S$: C, 51.89; H, 4.35; N, 7.56. Found: C, 52.27; H, 4.24; N, 7.53.

Example 131

5-bromo-N-[(2Z)-3-(2-methoxyelhyl)-4,5-dimethyl-1,3-thiazol-2(3H)-ylidene-2,3-dihydro-1-benzofuran-7-carboxamide

The product of Example 126A (0.20 g, 1.1 mmol), $Et_3N$ (0.45 mL, 3.2 mmol) and 5-bromo-2,3-dihydrobenzo [b]-furan-7-carbonyl chloride (Maybridge, 0.42 g, 1.6 mmol) in 35 mL THF were processed as in Example 126B. The resulting crude material was purified via flash column chromatography ($SiO_2$, 7:3 hexanes:EtOAc) to afford the title compound (0.16 g, 0.39 mmol, 36% yield). $^1H$ NMR (300 MHz, $CDCl_3$) $\delta$ ppm 2.24 (s, 3 H), 2.28 (s, 3 H), 3.23 (t, *J*=8.5 Hz, 2 H), 3.33 (s, 3 H), 3.78 - 3.87 (m, 2 H), 4.45 - 4.56 (m, 2 H), 4.70 - 4.81 (m, 2 H), 7.35 - 7.39 (m, 1 H), 8.09 (d, *J*=2.0 Hz, 1 H); MS (DCI/$NH_3$) m/z 411, 413 $(M+H)^+$; Anal. calculated for $C_{17}H_{19}BrN_2O_3S \cdot 3H_2O$: C, 49.00; H, 4.74; N, 6.72. Found: C, 48.91; H, 4.36; N, 6.57.

Example 132

2-bromo-N-[(2Z)-3-(2-methoxyethyl)-4,5-dimethyl-1,3-thiazol-2(3H)-ylidene]benzamide

To a solution of the product of Example 126A (0.20 g, 1.1 mmol) and $Et_3N$ (0.3 mL, 2.2 mmol) in 25 mL THF was added 2-bromobenzoyl chloride (Aldrich, 0.18 mL, 1.4 mmol). This mixture stirred at ambient temperature for 20 hours then was concentrated under reduced pressure and the residue was diluted with 10 mL EtOAc and washed with 5 mL $NH_4Cl$. The layers were separated and the aqueous layer was extracted (2 X 5 mL EtOAc). The combined organic layers were dried over anhydrous $Na_2SO_4$, filtered, concentrated under reduced pressure and purified via recrystallization with 50% hexanes/EtOAc to afford 0.18 g of the title compound (0.49 mmol, 46% yield). $^1H$ NMR (300 MHz, $CDCl_3$) $\delta$ ppm 2.26 (s, 3 H), 2.28 (s, 3 H), 3.30 (s, 3 H), 3.78 (t,*J*=5.1 Hz, 2 H), 4.42 (t, *J*=5. Hz, 2 H), 7.22 (dt, *J*=7.8, 1.7 Hz, 1 H), 7.34 (dt, *J*=7.5, 1.4 Hz, 1 H), 7.63 (dd, *J*=8.0, 1.2 Hz, 1 H), 7.90 (dd, *J*=7.6, 1.9 Hz, 1 H); MS (DCI/$NH_3$) m/z 369, 371 $(M+H)^+$; Anal. calculated for $C_{15}H_{17}BrN_2O_2S$: C, 48.79; H, 4.64; N, 7.59. Found: C, 48.84; H, 4.49; N, 7.40.

Example 133

2,6-dichloro-N-[(2Z)-3-(2-methoxlethyl)-4,5-dimethy)-1,3-thiazol-2(3H)-ylidene]benzamide

The product of Example 126A (0.20 g, 1.1 mmol), $Et_3N$ (0.3 mL, 2.2 mmol) and 2,6-dichlorobenzoyl chloride (Aldrich, 0.2 mL, 1.4 mmol) in 25 mL THF were processed as in Example 132 to afford the title compound (0.12 g, 0.32 mmol, 30% yield). $^1H$ NMR (300 MHz, $CDCl_3$) $\delta$ ppm 2.28 (s, 6 H), 3.27 (s, 3 H), 3.74 (t, *J*=4.6 Hz, 2 H), 4.34-4.44 (m, 2 H), 7.19 (dd, *J*=8.8, 6.8 Hz, I H), 7.28-7.34 (m, 2 H); MS (DCI/$NH_3$) m/z 359 $(M+H)^+$.

Example 134

N-[(2Z)-3-(2-methoxyethyl)-4,5-dimethyl-1,3-thiazo(-2(3H)-ylidene]quinoline-4-carboxamide

Example 134A

Quinoline-4-carbonyl chloride

A solution of 4-quinolinecarboxylic acid (Aldrich, 0.25 g, 1.4 mmol) in 5 mL of thionyl chloride was warmed to reflux and allowed to stir for l hour. The mixture was then cooled to ambient temperature and concentrated under reduced pressure. This material was dissolved in 10 mL toluene and concentrated under reduced pressure (3X) to afford the title compound.

Example 134B

N-[(2Z)-3-(2-methoxyethyl)-4,5-dimethyl-1,3-thiazol-2(3H)-ylidene]quinoline-4-carboxamide

To a suspension of the product of Example 126A (0.20 g, 1. mmol) in 25 mL THF was added $Et_3N$ (0.3 mL, 2.2 mmol) followed by the freshly prepared quinoline-4-carbonyl chloride. This mixture was warmed to reflux and allowed to stir for 18 hours. The material was then concentrated under reduced pressure and 10 mL EtOAc, 5 mL $H_2O$ and 5 mL $NH_4OH$ were added. The layers were separated and the aqueous layer was extracted 2 X 5 mL EtOAc. The combined organic layers were dried over anhydrous $Na_2SO_4$, filtered, concentrated under reduced pressure and purified via flash column chromatography ($SiO_2$, 5% $CH_3OH$ in EtOAc) to afford the title compound (0.12 g, 0.35 mmol, 33% yield). $^1H$ NMR (300 MHz, $CD_2OD$) $\delta$ ppm 2.32 (d, *J*=0.7 Hz, 3 H), 2.35 (d, *J*=0.7 Hz, 3 H), 3.31 (s, 3 H), 3.78 (t, *J=5.3* Hz, 2 H), 4.48 (t, *J*=5.1 Hz, 2 H), 7.65 (ddd, *J*=8.5, 7.0, 1.2 Hz, 1 H), 7.80 (ddd, *J*=8.4, 6.9, 1.4 Hz, 1 H), 8.00 (d, *J*=4.4 Hz, 1 H), 8.06-8.11 (m, 1 H), 8.84 (ddd, *J*=8.5, 1.4, 0.7 Hz, 1 H), 8.93 (d, *J*=4.4 Hz, 1 H); MS (DCI/$NH_3$) m/z 342 $(M+H)^+$; Anal. calculated for $C_{18}H_{19}N_3O_2S$: C, 63.32; H, 5.61; N, 12.31. Found: C, 63.23; H, 5.46; N, 12.10.

Example 135

N-[(2Z)-3-(2-methoxyethyl)-4,5-dimethyl-1,3-thiazol-2(3H)-ylidene]quinoline-5-carboxamide

The quinoline-5-carboxylic acid (Lancaster, 0.25 g, 1.4 mmol) was converted to quinoline-5-carbonyl chloride using 5 mL $SOCl_2$ using the method described in Example 134A. The product of Example 126A (0.20 g, 1.1 mmol), $Et_2N$ (0.3 mL, 2.2 mmol) and quinoline-5-carbonyl chloride in 30 mL THF were processed as in Example 134B to afford the title compound (0.18 g, 0.53 mmol, 49% yield). $^1H$ NMR (300 MHz, $CD_3OD$) $\delta$ ppm 2.30 (d, *J*=0.7 Hz, 3 H), 2.34 (s, 3 H), 3.31 (s, 3 H), 3.80 (t, *J=5.1* Hz, 2 H), 4.49 (t, *J*=5.3 Hz, 2 H), 7.60 (dd, *J*=8.6,4.2 Hz, 1 H), 7.83 (dd, *J*=8.5, 7.1 Hz, 1 H), 8.15 (dt, *J* = 8.5, 1.0 Hz, 1 H), 8.40 (dd,*J*=7.3, 1.2 Hz, 1 H), 8.87 (dd, *J*=4.1, 1.7 Hz, 1 H), 9.55 (ddd, *J*=8.8, 1.7, 0.7 Hz, 1 H); MS (DCI/$NH_3$) m/z 342 $(M+H)^+$; Anal. calculated for $C_{18}H_{19}N_3O_2S$: C, 63.32; H, 5.61; N, 12.31. Found: C, 63.44; H, 5.05; N, 12.10.

Example 136

N-[(2Z)-3-(2-methoxyethyl)-4,5-dimethyll-1,3-thiazol-2 (3H)-ylidene]isoquinoline-5-carboxamide

The isoquinoline-5-carboxylic acid (Lancaster, 0.25 g, 1.4 mmol) was converted to the corresponding acid chloride with 5 mL of thionyl chloride using the method described in Example 134A. The product of Example 126A (0.20 g, 1.1 mmol), $Et_3N$ (0.3 mL, 2.2 mmol) and the acid chloride in 30 mL THF were processed as in Example 134B to afford the title compound (98 mg, 0.29 mmol, 27% yield). $^1H$ NMR (300 MHz, $CD_3OD$) $\delta$ ppm 2.31 (s, 3 H), 2.35 (s, 3 H), 3.31 (s, 3 H), 3.81 (t, *J*=5.3 Hz, 2 H), 4.50 (t, *J*=5.3 Hz, 2 H), 7.76 (dd, *J*=7.5, 7.5 Hz, 1 H), 8.23 (d, *J*=8. Hz, 1 H), 8.48 (d, *J*=6.4 Hz, 1 H), 8.61 (dd, *J*=7.1, 1.4 Hz, 1 H), 9.03 (d, *J*=6.1 Hz, 1 H), 9.27 (d, *J*=1.0 Hz, 1 H); MS (DCI/$NH_3$) m/z 342 $(M+H)^+$; Anal. calculated for $C_{18}H_{19}N_3O_2S$: C, 63.32; H, 5.61; N, 12.31. Found: C, 62.97; H, 5.54; N, 12.07.

Example 137

N-[(2Z)-3-(2-methoxyethyl)-4,5-dimethyl-1,3-thiazol-2(3H)-ylidene]-2,3-dihydro-1-benzofuran-7-carboxamide

The 2,3-dihydrobenzofuran-7-carboxylic acid (TCI-US, 0.25 g, 1.4 mmol) was converted to 2,3-dihydrobenzofuran-7-carbonyl chloride with 5 mL of thionyl chloride using the method described in Example 134A. The product of Example 126A (0.20 g, 1.1 mmol), $Et_3N$ (0.3 mL, 2.2 mmol) and the freshly prepared 2,3-dihydrobenzofuran-7-carbonyl chloride in 30 mL THF were processed as in Example 134B. Purification of the crude material via recrystallization with 50% hexanes:EtOAc gave the title compound (0.12 g, 0.36 mmol, 34% yield). $^1H$ NMR (300 MHz, $CD_3OD$) δ ppm 2.25 (s, 3 H), 2.30 (s, 3 H), 3.22 (t, *J*=8.8 Hz, 2 H), 3.31 (s, 3 H), 3.81 (t, *J*=5.3 Hz, 2 H), 4.44 (t, *J*=5.3 Hz, 2 H), 4.64 (t, *J*=8.8 Hz, 2 H), 6.86 (t, *J*=7.5 Hz, 1 H), 7.32 (dd, *J*=7.3, 1.2 Hz, 1 H), 7.93 (dd, *J*=8.0, 0.8 Hz, I H); MS ($DCI/NH_3$) m/z 333 $(M+H)^+$; Anal. calculated for $C_{17}H_{20}N_2O_3S$•$0.1H_2O$: C, 61.09; H, 6.09; N, 8.38. Found: C, 60.99; H, 5.91; N, 8.25.

Example 138

2-chloro-N-[(2Z)-3-(2-methoxyethyl)-4,5-dimethyl-1,3-thiazol-2(3H)-ylidene]quinoline-4-carboxamide

The 2-chloroquinoline-4-carboxylic acid (TCI-JP, 0.29 g, 1.4 mmol) was converted to 2-chloroquinoline-4-carbonyl chloride with 5 mL of thionyl chloride using the method described in Example 134A. The product of Example 126A (0.20 g, 1.1 mmol), $Et_3N$ (0.3 mL, 2.2 mmol) and 2-chloroquinoline-4-carbonyl chloride in 30 mL THF were processed as in Example 134B to afford the title compound (0.14 g, 0.37 mmol, 35% yield). $^1H$ NMR (300 MHz, $CD_3OD$) δ ppm 2.33 (s, 3 H), 2.35 (s, 3 H), 3.31 (s, 3 H), 3.78 (t, *J*=5.3 Hz, 2 H), 4.49 (t, *J*=5.3 Hz, 2 H), 7.66 (ddd, *J*=8.5, 7.1, 1.4 Hz, I H), 7.81 (ddd, *J*=8.5, 7.1, 1.4 Hz, I H), 7.94 (s, 1 H), 7.99 (dt, *J*=8.5, 0.7 Hz, 1 H), 8.83 (dd, *J*=8.6, 1.2 Hz, 1 H); MS ($DCI/NH_3$) m/z 376 $(M+H)^+$; Anal. calculated for $C_{18}H_{18}ClN_3O_2S$: C, 57.52; H, 4.83; N, 11.18. Found: C, 57.44; H, 4.59; N, 10.97.

Example 139

N-[(2Z)-3-(2-methoxyethyl)-4,5-dimethyl-1,3-thiazol-2(3H)-ylidene]-1,2-dihydroacenaphthylene-5-carboxamide

The acenaphthene-5-carboxylic acid (Aldrich, 0.29 g, 1.4 mmol) in 5 mL of thionyl chloride was converted to acenaphthene-5-carbonyl chloride using the method described in Example 134A. The product of Example 126A (0.20 g, 1.1 mmol), $Et_3N$ (0.3 mL, 2.2 mmol) and acenaphthene-5-carbonyl chloride in 30 mL THF were processed as in Example 134B to afford the title compound (87 mg, 0.24 mmol, 22% yield). $^1H$ NMR (300 MHz, $CD_3OD$) δ ppm 2.28 (d, *J*=0.7 Hz, 3 H), 2.33 (d, *J*=0.7 Hz, 3 H), 3.33 (s, 3 H), 3.42 (s, 4 H), 3.84 (t, *J*=5.3 Hz, 2 H), 4.5 (t, *J*=5.3 Hz, 2 H), 7.30-7.36 (m, 2 H), 7.52 (dd, *J*=8.6, 7.0 Hz, I H), 8.43 (d, *J*=7.5 Hz, I H), 8.81 (d, *J*=8.8 Hz, 1 H); MS ($DCI/NH_3$) m/z 367 $(M+H)^+$; Anal. calculated for $C_{21}H_{22}N_2O_2S$: C, 68.82; H, 6.05; N, 7.64. Found: C, 68.63; H, 5.72; N, 7.40.

Example 140

2,3-dichloro-N-[(2Z)-3-(2-methoxyethyl)-5-methyl-1,3-thiazol-2(3H)-ylidene]benzamide

Example 140 A

3-(2-Methoxy-ethyl)-5-methyl-3H-thiazol-2-ylideneamine

The product of Example 10A (17.6 g, 70 mmol) was treated with ~50 mL 20% aqueous $K_2CO_3$ then the mixture was extracted with EtOAc (3 X 25 mL). The combined organics were dried over anhydrous $Na_2SO_4$, filtered and concentrated under reduced pressure to afford the title compound (5.9 g, 34 mmol, 49% yield). $^1H$ NMR (300 MHz, $CDCl_3$) δ ppm 2.03 (d, *J*=1.70 Hz, 3 H) 3.36 (s, 3 H) 3.62 (t, *J*=5.10 Hz, 2 H) 3.83 (t, *J*=4.80 Hz, 2 H) 6.15 - 6.21 (m, 1 H); MS ($DCI/NH_3$) m/z 173 $(M+H)^+$.

Example 140 B

2,3-dichloro-N-[(2Z)-3-(2-methoxyethyl)-5-methyl-1,3-thiazol-2(3H)-ylidene]benzamide

To the product of Example 140A (0.20 g, 1.2 mmol) in 15 mL THF was added $Et_3N$ (0.48 mL, 3.5 mmol) followed by a solution of 2,3-dichlorobenzoyl chloride (Lancaster, 0.31 g, 1.5 mmol) in 5 mL THF. This mixture stirred at ambient temperature for 1 hour then was concentrated under reduced pressure, quenched with 5 mL saturated, aqueous $NH_4Cl$ and extracted with EtOAc (3 X 5 mL). The combined organic extracts were dried over anhydrous $Na_2SO_4$, filtered and concentrated under reduced pressure. The resulting solids were recrystallized from 50% hexanes/EtOAc to afford the title compound (0.27 g, 0.78 mmol, 68% yield). $^1H$ NMR (300 MHz, $CDCl_3$) δ ppm 2.32 (d, *J*=1.4 Hz, 3 H), 3.34 (s, 3 H), 3.71 (t, *J*=5.1 Hz, 2 H), 4.34 (t, *J*=4.8 Hz, 2 H), 6.82-6.86 (m, 1 H), 7.22 (t, *J*=7.8 Hz, I H), 7.49 (dd, *J*=8.1, 1.7 Hz, 1 H),

7.73 (dd, J=7.5, 1.7 Hz, 1 H); MS (DCI/$NH_3$) m/z 345 $(M+H)^+$; Anal. calculated for $C_{14}H_{14}Cl_2N_2O_2S$: C, 48.70; H, 4.09; N, 8.11. Found: C, 48.39; H, 3.70; N, 7.94.

Example 141

5-chloro-N-[(2Z)-3-(2-methoxyethyl)-5-methyl-1,3-thiazol-2(3H)-ylidene]-2-(trifluoromethyl)benzamide

The product of Example 140A (0.20 g, 1.2 mmol), $Et_3N$ (0.48 mL, 3.5 mmol) and 5-chloro-2-(trifluoromethyl) benzoyl chloride (Matrix, 0.31 g, 1.5 mmol) in 20 mL THF were processed using the method described in Example 140B to afford the title compound (0.16 g, 0.42 mmol, 36% yield). $^1H$ NMR (300 MHz, $CDCl_3$) δ ppm 2.33 (d, J=0.7 Hz, 3 H), 3.34 (s, 3 H), 3.69 (t, J=5.1 Hz, 2 H), 4.34 (1, J=4.6 Hz, 2 H), 6.85 (s, 1 H), 7.42-7.48 (m, 1 H), 7.64 (d, J=8.5 Hz, 1 H), 7.85 (d, J=2.0 Hz, I H); MS (DCI/$NH_3$) m/z 379 $(M+H)^+$; Anal. calculated for $C_{15}H_{14}ClF_3N_2O_2S$: C, 47.56; H, 3.73; N, 7.40. Found: C, 47.31; H, 3.30; N, 7.33.

Example 142

2-chloro-N-[(2Z)-3-(2-methoxyethyl)-4,5-dimethyl-1,3-thiazol-2(3H)-ylidene]benzamide

The product of Example 126A (0.20 g, 1.1 mmol), $Et_3N$ (0.45 mL, 3.2 mmol) and 2-chlorobenzoyl chloride (Aldrich, 0.26 g, 1.4 mmol) in 15 mL THF were processed as in Example 132 to afford the title compound (0.13 g, 0.40 mmol, 37% yield). $^1H$ NMR (300 MHz, $CD_3OD$) δ ppm 2.28 (s, 3 H), 2.32 (s, 3 H), 3.31 (s, 3 H), 3.76 (t, J=5.3 Hz, 2 H), 4.42 (t, J=5.1 Hz, 2 H), 7.30-7.46 (m, 3 H), 7.77-7.83 (m, 1 H); MS (DCI/$NH_3$) m/z 325 $(M+H)^+$; Anal. calculated for $C_{15}H_{17}ClN_2O_2S$: C, 55.46; H, 5.28; N, 8.62. Found: C, 55.59; H, 4.81; N, 8.47.

Example 143

N-[(2Z)-5-tert-butyl-3-(2-methoxyethyl)-4-methyl-1,3-thiazol-2(3H)-ylidene]-2-ethoxybenzamide

Example 143A

5-tert-Butyl-3-(2-methoxyethyl)-4-methyl-1,3-thiazol-2(3H)-ylideneamine

A mixture of 5-tert-butyl-4-methylthiazole-2-ylamine (1.5 g, 8.8 mmol) and 2-bromoethyl methyl ether (0.91 mL, 9.7 mmol) was warmed to 85 °C and allowed to stir for 24 hours. The crude material was dissolved in ~5 mL of a 1:1 mixture of $CH_2Cl_2$ and $CH_3OH$ and a small amount of silica gel was added. This mixture was concentrated to dryness and the residue was purified via flash column chromatography ($SiO_2$, 9:1:0.1 $CH_2Cl_2$:$CH_3OH$:$NH_4OH$) to afford the title compound (1.0 g, 4.4 mmol, 50% yield). $^1H$ NMR (300 MHz, $CD_3OD$) δ ppm 1.41 (s, 9 H) 2.38 (s, 3 H) 3.35 (s, 3 H) 3.66 (t, J=4.70 Hz, 2 H) 4.16 (t, J=4.70 Hz, 2 H); MS (DCI/$NH_3$) m/z 229 $(M+H)^+$.

Example 143B

N-[(2Z)-5-tert-butyl-3-(2-methoxyethyl)-4-methyl-1,3-thiazol-2(3H)-ylidene]-2-ethoxybenzamide

A mixture of the product of Example 143A (0.15 g, 0.66 mmol), $Et_3N$ (0.28 mL, 2.0 mmol) and 2-ethoxybenzoyl chloride (Aldrich, 0.16 g, 0.86 mmol) in 15 mL THF were processed using the method described in Example 129 to afford the title compound (0.12 g, 0.33 mmol, 50% yield). $^1H$ NMR (300 MHz, $CD_3OD$) δ ppm 1.41 (t, J=7.1 Hz, 3 H), 1.45 (s, 9 H), 2.47 (s, 3 H), 3.31 (s, 3 H), 3.77 (t, J=5.3 Hz, 2 H), 4.11 (q, J=6.8 Hz, 2 H), 4.43 (t, J=5.3 Hz, 2 H), 6.95 (dt, J=7.5, 1.0 Hz, 1 H), 7.04 (d, J=8.1 Hz, I H), 7.37 (ddd, J=8.7, 6.9, 1.7 Hz, 1 H), 7.78 (dd, J=7.6, 1.9 Hz, 1 H); MS (DCI/$NH_3$) m/z 377 $(M+H)^+$; Anal. calculated for $C_{20}H_{28}N_2O_3S$: C, 63.80; H, 7.50; N, 7.44. Found: C, 64.19; H, 7.44; N, 7.19.

Example 144

N-[(2Z)-5-tert-butyl-3-(2-methoxyethyl)-4-methyl-1,3-thiazol-2(3H)-ylidene]-2,3-dichlorobenzamide

A mixture of the product of Example 143A (0.15 g, 0.66 mmol), $Et_3N$ (0.28 mL, 2.0 mmol) and 2,3-dichlorobenzoyl chloride (Lancaster, 0.18 g, 0.86 mmol) in 15 mL THF were processed using the method described in Example 129 to afford the title compound (0.11 g, 0.27 mmol, 42% yield). $^1H$ NMR (300 MHz, $CDCl_3$) δ ppm 1.43 (s, 9 H), 2.43 (s, 3 H), 3.30 (s, 3 H), 3.74 (t, J=5.3 Hz, 2 H), 4.36 (t, J=5.3 Hz, 2 H), 7.22 (dd, J=8.0, 8.0 Hz, 1 H), 7.48 (dd, J=8.1, 1.7 Hz, 1 H),

7.72 (dd, *J*=7.6, 1.5 Hz, 1 H); MS (DCI/$NH_3$) m/z 401 $(M+H)^+$; Anal. calculated for $C_{18}H_{22}Cl_2N_2O_2S$: C, 53.87; H, 5.53; N, 6.98. Found: C, 53.86; H, 5.37; N, 6.76.

Example 145

N-[(2Z)-5-tert-butyl-3-(2-methoxyethyl)-4-methyl-1,3-thiazol-2(3H)-ylidene]-5-chloro-2-(trifluoromethyl)benzamide

A mixture of the product of Example 143A (0.15 g, 0.66 mmol), $Et_3N$ (0.28 mL, 2.0 mmol) and 5-chloro-2-trifluoromethylbenzoyl chloride (Matrix, 0.17 g, 0.86 mmol) in 15 mL THF were processed using the method described in Example 129 to afford the title compound (0.16 g, 0.37 mmol, 56% yield). $^1H$ NMR (300 MHz, $CD_3OD$) $\delta$ ppm 1.46 (s, 9 H), 2.49 (s, 3 H), 3.31 (s, 3 H), 3.70 (t, *J*=5.3 Hz, 2 H), 4.41 (t, *J*=5.3 Hz, 2 H), 7.56-7.62 (m, I H), 7.71-7.76 (m, 2 H); MS (DCI/$NH_3$) m/z 435 $(M+H)^+$; Anal. calculated for $C_{19}H_{22}ClF_3N_2O_2S$: C, 52.47; H, 5.10; N, 6.44. Found: C, 52.52; H, 4.94; N, 6.05.

Example 146

N-[(2Z)-3-(2-methoxyethyl)-5-methyl-1,3-thiazol-2(3H)-ylidene]-1-naphthamide

The product of Example 140A (0.20 g, 1.2 mmol), $Et_3N$ (0.48 mL, 3.5 mmol) and 1-naphthoyl chloride (Aldrich, 0.22 g, 1.5 mmol) in 15 mL THF were processed using the method described in Example 140B to afford the title compound (0.23 g, 0.69 mmol, 60% yield). $^1H$ NMR (300 MHz, $CD_3OD$) $\delta$ ppm 2.36 (d, *J*=1.0 Hz, 3 H), 3.35 (s, 3 H), 3.78 (t, *J*=5.4 Hz, 2 H), 4.45 (t, *J*=5.1 Hz, 2 H), 7.10-7.15 (m, 1 H), 7.46-7.58 (m, 3 H), 7.88-7.94 (m, 1 H), 7.97 (d, *J*=8.1 Hz, 1 H), 8.17 (dd, *J*=7.3, 1.2 Hz, 1 H), 8.83-8.90 (m, I H); MS (DCI/$NH_3$) m/z 327 $(M+H)^+$; Anal. calculated for $C_{18}H_{18}N_2O_2S$: C, 66.23; H, 5.56; N, 8.58. Found: C, 66.10; H, 5.64; N, 8.51.

Example 147

N-[[(2Z)-5-tert-butyl-3-(2-methoxyethyl)-4-methyl-1,3-thiazol-2(3H)-ylidene]-5-chloro-2-methoxybenzamide

The 5-chloro-2-methoxybenzoic acid (Aldrich, 0.21 g, 1.1 mmol) was converted to 5-chloro-2-methoxybenzoyl chloride with 5 mL of thionyl chloride using the method described in Example 134A. A mixture of the product of Example 143A (0.20 g, 0.88 mmol), $Et_3N$ (0.48 mL, 3.4 mmol) and 5-chloro-2-methoxybenzoyl chloride in 15 mL THF were processed using the method described in Example 129 to afford the title compound (0.13 g, 0.32 mmol, 37% yield). $^1H$ NMR (300 MHz, $CDCl_3$) $\delta$ ppm 1.41 (s, 9 H), 2.42 (s, 3 H), 3.32 (s, 3 H), 3.77 (t, *J*=5.3 Hz, 2 H), 3.90 (s, 3 H), 4.36 (t, *J=5.1* Hz, 2 H), 6.90 (d, *J*=8.8 Hz, 1 H), 7.31 (dd, *J*=8.8, 3.1 Hz, 1 H), 7.94 (d, *J*=2.7 Hz, I H); MS (DCI/$NH_3$) m/z 397 $(M+H)^+$; Anal. calculated for $C_{19}H_{25}ClN_2O_3S$: C, 57.49; H, 6.35; N, 7.06. Found: C, 57.51; H, 6.30; N, 6.85.

Example 148

N-[(2Z)-3-(2-methoxyethyl)-5-methyl-1,3-thiazol-2(3H)-ylidene]-2-methylbenzamide

The product of Example 140A (0.20 g, 1.2 mmol), $Et_3N$ (0.48 mL, 3.5 mmol) and o-toloyl chloride (Aldrich, 0.23 g, 1.2 mmol) in 15 mL THF were processed using the method described in Example 140B to afford the title compound (0.26 g, 0.90 mmol, 78% yield). $^1H$ NMR (300 MHz, $CDCl_3$) $\delta$ ppm 2.30 (s, 3 H), 2.70 (s, 3 H), 3.35 (s, 3 H), 3.74 (t, *J*=5.1 Hz, 2 H), 4.36 (t, *J*=4.8 Hz, 2 H), 6.77-6.81 (m, 1 H), 7.23 (t, *J*=7.3 Hz, 2 H), 7.28-7.35 (m, 1 H), 8.08-8.13 (m, 1 H); MS (DCI/$NH_3$) m/z 291 $(M+H)^+$; Anal. calculated for $C_{15}H_{18}N_2O_2S$: C, 62.04; H, 6.25; N, 9.65. Found: C, 62.40; H, 6.11; N, 9.70.

Example 149

2,3-dichloro-N-[(2Z)-5-methyl-3-(tetrahydrofuran-2-ylmethyl)-1,3-thiazol-2(3H)-ylidene]benzamide

Example 149A

5-Methyl-3-(tetrahydro-furan-2-ylmethyl)-3H-thiazol-2-ylideneamine

A mixture of 2-amino-5-methylthiazole (1.0 g, 8.7 mmol) and 2-(bromomethyl)tetrahydrofuran (Maybridge, 1.1 mL, 10 mmol) was wanned to 85 °C and allowed to stir for 24 hours. The mixture was then cooled to ambient temperature

and purified via column chromatography ($SiO_2$, 9:1:0.1 $CH_2Cl_2$:$CH_3OH$:$NH_4OH$) to afford the title compound (1.5 g, 7.5 mmol, 86% yield). $^1H$ NMR (300 MHz, $CD_3OD$) δ ppm 1.55 - 1.70 (m, I H) 1.89 - 2.01 (m, 2 H) 2.07 - 2.20 (m, I H) 2.28 (d, *J*=1.36 Hz, 3 H) 3.72 - 3.82 (m, I H) 3.86 - 4.00 (m, 2 H) 4.08 - 4.24 (m, 2 H) 6.98 - 7.04 (m, 1 H); MS (DCI/$NH_3$) m/z 199 $(M+H)^+$.

Example 149B

2,3-dichloro-N-[(2Z)-5-methyl-3-(tetrahydrofuran-2-ylmethyl)-1,3-thiazol-2(3H)-ylidene]benzamide

To a solution of the product of Example 149A (0.17 g, 0.86 mmol) in 10 mL THF and I mL DMF was added $Et_3N$ (0.36 mL, 2.6 mmol) followed by 2,3-dichlorobenzoyl chloride (Lancaster, 0.27 g, 1.3 mmol). This mixture was wanned to 50 °C and allowed to stir for 2 hours. The mixture was cooled to ambient temperature, diluted with 10 mL EtOAc, and quenched with 10 mL $NH_4Cl$. The layers were separated and the aqueous layer was extracted 2 X 5 mL EtOAc. The combined organics were dried over anhydrous $Na_2SO_4$, filtered, concentrated under reduced pressure and purified via column chromatography ($SiO_2$, 20% hexanes/EtOAc) to afford the title compound (0.24 g, 0.64 mmol, 75% yield). $^1H$ NMR (300 MHz, $CDCl_3$) δ ppm 1.58-1.68 (m, 1 H), 1.78-1.94 (m, 2 H), 2.00-2.13 (m, 1 H), 2.32 (s, 3 H), 3.72-3.91 (m, 2 H), 4.06-4.15 (m, I H), 4.24 (ddd, *J*= 14.0, 7.0, 2.7 Hz, I H), 4.47 (dd, *J*=13.6, 2.7 Hz, I H), 6.91-6.95 (m, I H), 7.22 (t, *J*=8.0 Hz, 1 H), 7.49 (dd, J=8.1, 1.7 Hz, I H), 7.72 (dd, *J*=7.5, 1.7 Hz, 1 H); MS (DCI/$NH_3$) m/z 371 $(M+H)^+$; Anal. calculated for $C_{16}H_{16}Cl_2N_2O_2S$: C, 51.76; H, 4.34; N, 7.55. Found: C, 51.66; H, 4.17; N, 7.46.

Example 150

N-[(2Z)-3-(2-methoxyethyl)-4,5-dimethyl-1,3-thiazol-2(3H)-ylidene]-2-methylbenzamide

The product of Example 126A (0.20 g, 1.1 mmol), $Et_3N$ (0.45 mL, 3.2 mmol) and o-toloyl chloride (Aldrich, 0.22 g, 1.4 mmol) in 15 mL THF were processed using the method described in Example 129 to afford the title compound (0.15 g, 0.49 mmol, 46% yield). $^1H$ NMR (300 MHz, $CDCl_3$) δ ppm 2.23 (d, *J*=0.7 Hz, 3 H), 2.26 (s, 3 H), 2.70 (s, 3 H), 3.31 (s, 3 H), 3.77 (t, *J*=5.3 Hz, 2 H), 4.35 (t, *J*=5.3 Hz, 2 H), 7.23 (t, J = 7.5 Hz, 2 H), 7.27-7.35 (m, 1 H), 8.08-8.14 (m, 1 H); MS (DCI/$NH_3$) m/z 305 $(M+H)^+$; Anal. calculated for $C_{16}H_{20}N_2O_2S$: C, 63.13; H, 6.62; N, 9.20. Found: C, 63.43; H, 6.53; N, 9.14.

Example 151

N-[(2Z)-3-(2-methoxyethyl)-4,5-dimethyl-1,3-thiazol-2(3H)-ylidene]benzamide

The product of Example 126A (0.20 g, 1.1 mmol), $Et_3N$ (0.45 mL, 3.2 mmol) and benzoyl chloride (Aldrich, 0.16 mL, 1.4 mmol) in 15 mL THF were processed using the method described in Example 129 to afford the title compound (0.11 g, 0.38 mmol, 35% yield). $^1H$ NMR (300 MHz, $CDCl_3$) δ ppm 2.24 (s, 3 H), 2.27 (d, *J*=0.7 Hz, 3 H), 3.32 (s, 3 H), 3.82 (t, *J*=5.3 Hz, 2 H), 4.41 (t, *J*=5.3 Hz, 2 H), 7.38-7.51 (m, 3 H), 8.27-8.34 (m, 2 H); MS (DCI/$NH_3$) m/z 291 $(M+H)^+$; Anal: calculated for $C_{15}H_{18}N_2O_2S$: C, 62.04; H, 6.25; N, 9.65. Found: C, 62.02; H, 6.05; N, 9.56.

Example 152

2-chloro-4-fluoro-N-[(2Z)-3-(2-methoxyethyl)-4,5-dimethyl-1,3-thiazol-2(3H)-ylidene]benzamide

The product of Example 126A (0.20 g, 1.1 mmol), $Et_3N$ (0.37 mL, 2.7 mmol) and 2-chloro-4-fluorobenzene-1-carbonyl chloride (Acros, 0.25 g, 1.3 mmol) in 15 mL THF were processed using the method described in Example 132 to afford the title compound (0.19 g, 0.56 mmol, 52% yield). $^1H$ NMR (300 MHz, $CDCl_3$) δ ppm 2.24 (s, 3 H), 2.27 (s, 3 H), 3.30 (s, 3 H), 3.76 (t, *J*=5.1 Hz, 2 H), 4.35 (t, *J*=5.1 Hz, 2 H), 7.00 (ddd, *J*=8.5, 7.8, 2.4 Hz, I H), 7.15 (dd, *J*=8.8, 2.4 Hz, I H), 8.04 (dd, *J*=8.8, 6.4 Hz, 1 H); MS (DCI/$NH_3$) m/z 343 $(M+H)^+$; Anal. calculated for $C_{15}H_{16}ClFN_2O_2S$: C, 52.55; H, 4.70; N, 8.17. Found: C, 52.60; H, 4.38; N, 8.06.

Example 153

2-chloro-4-fluoro-N-[(2Z)-3-(2-methoxyethyl)-5-methyl-1,3-thiazol-2(3H)-ylidene]benzamide

The product of Example 140A (0.17 g, 1.0 mmol), $Et_3N$ (0.35 mL, 2.5 mmol) and 2-chloro-4-fluorobenzene-1-carbonyl chloride (Acros, 0.23 g, 1.2 mmol) in 15 mL THF were processed using the method described in Example 140B

to afford the title compound (0.15 g, 0.46 mmol, 46% yield). $^1H$ NMR (300 MHz, $CDCl_3$) δ ppm 2.32 (d, *J*=1.4 Hz, 3 H), 3.35 (s, 3 H), 3.73 (t, *J*=5.4 Hz, 2 H), 4.36 (t, *J*=4.7 Hz, 2 H), 6.80-6.84 (m, 1 H), 7.00 (ddd, *J*=8.6, 7.8, 2.5 Hz, 1 H), 7.16 (dd, *J*=8.6, 2.5 Hz, 1 H), 8.04 (dd, *J*=8.8, 6.4 Hz, I H); MS ($DCI/NH_3$) m/z 329 $(M+H)^+$; Anal. calculated for $C_{14}H_{14}ClFN_2O_2S$: C, 51.15; H, 4.29; N, 8.52. Found: C, 51.11; H, 3.90; N, 8.43.

Example 154

2,5-dichloro-N-[(2Z)-3-(2-methoxyethyl)-4,5-dimethyl-1,3-thiazol-2(3H)-ylidene]benzamide

The 2,5-dichlorobenzoic acid (Aldrich, 0.28 g, 1.4 mmol) was converted to 2,5-dichlorobenzoyl chloride with 5 mL of thionyl chloride using the method described in Example 134A. The product of Example 126A (0.20 g, 1.1 mmol), $Et_3N$ (0.45 mL, 3.2 mmol) and 2,5-dichlorobenzoyl chloride in 15 mL THF were processed using the method described in Example 132 to afford the title compound (0.10 g, 0.28 mmol, 26% yield). $^1H$ NMR (300 MHz, $CDCl_3$) δ ppm 2.25 (d, *J*=1.0 Hz, 3 H), 2.28 (d, *J*=1.0 Hz, 3 H), 3.31 (s, 3 H), 3.76 (t, *J*=5.3 Hz, 2 H), 4.36 (t, *J*=5.3 Hz, 2 H), 7.28 (d, *J*=2.4 Hz, I H), 7.32-7.36 (m, 1 H), 7.94 (d, *J*=2.4 Hz, 1 H); MS ($DCI/NH_3$) m/z 359 $(M+H)^+$; Anal. calculated for $C_{15}H_{16}Cl_2N_2O_2S$: C, 50.15; H, 4.49; N, 7.80. Found: C, 50.22; H, 4.15; N, 7.63.

Example 155

2,5-dichloro-N-[(2Z)-3-(2-methoxyethyl)-5-methyl-1,3-thiazol-2(3H)-ylidene]benzamide

The 2,5-dichlorobenzoic acid (Aldrich, 0.28 g, 1.4 mmol) was converted to 2,5-dichlorobenzoyl chloride with 5 mL of thionyl chloride using the method described in Example 134A. The product of Example 140A (0.20 g, 1.1 mmol), $Et_3N$ (0.45 mL, 3.2 mmol) and 2,5-dichlorobenzoyl chloride in 15 mL THF were processed using the method described in Example 129 to afford the title compound (0.24 g, 0.70 mmol, 65% yield). $^1H$ NMR (300 MHz, $CDCl_3$) δ ppm 2.32 (d, *J*=1.4 Hz, 3 H), 3.35 (s, 3 H), 3.73 (t, *J=5.1* Hz, 2 H), 4.37 (t, *J*=4.7 Hz, 2 H), 6.83-6.86 (m, 1 H), 7.28 (dd, *J*=8.5, 2.4 Hz, 1 H), 7.35 (d, *J*=8.5 Hz, 1 H), 7.94 (d, *J*=2.7 Hz, I H); MS ($DCI/NH_3$) m/z 345 $(M+H)^+$; Anal. calculated for $C_{14}H_{14}Cl_2N_2O_2S$: C, 48.70; H, 4.09; N, 8.11. Found: C, 48.60; H, 3.78; N, 8.02.

Example 156

5-chloro-2-methoxy-N-[(2Z)-3-(2-methoxyethyl)-5-methyl-1,3-thiazol-2(3H)-ylidene]benzamide

The 5-chloro-2-methoxybenzoic acid (Aldrich, 0.28 g, 1.5 mmol) was converted to 5-chloro-2-methoxybenzoyl chloride with 5 mL of thionyl chloride using the method described in Example 134A. The product of Example 140A (0.19 g, 1.1 mmol), $Et_3N$ (0.45 mL, 3.2 mmol) and 5-chloro-2-methoxybenzoyl chloride in 15 mL THF were processed using the method described in Example 129 to afford the title compound (0.25 g, 0.72 mmol, 67% yield). $^1H$ NMR (300 MHz, $CDCl_3$) δ ppm 2.30 (d, *J*=1.0 Hz, 3 H), 3.36 (s, 3 H), 3.74 (t, *J*=5.1 Hz, 2 H), 3.90 (s, 3 H), 4.36 (t, *J*=4.7 Hz, 2 H), 6.82 (s, 1 H), 6.90 (d, *J*=8.8 Hz, 1 H), 7.33 (dd, *J*=8.8, 2.7 Hz, 1 H), 7.96 (d, *J*=2.7 Hz, I H); MS ($DCI/NH_3$) m/z 341 $(M+H)^+$; Anal. calculated for $C_{15}H_{17}ClN_2O_3S$: C, 52.86; H, 5.03; N, 8.22. Found: C, 52.84; H, 4.72; N, 8.13.

Example 157

2,3-dichloro-N-[(2Z)-5-methyl-3-(tetrahydro-2H-pyran-2-ylmethyl)-1,3-thiazol-2(3H)-ylidene]benzamide

Example 157A

5-Methyl-3-(tetrahydro-pyran-2-ylmethyl)-3H-thiazol-2-ylideneamine

A mixture of 2-amino-5-methylthiazole (1.2 g, 10.5 mmol) and 2-(bromomethyl)tetrahydro-2H-pyran (Aldrich, 1.5 mL, 11.6 mmol) was warmed to 85 °C and allowed to stir for 18 hours. The mixture was cooled to ambient temperature and purified by flash column chromatography ($SiO_2$, first 10% $CH_3OH$:EtOAc then 9:1:0.1 $CH_2Cl_2$:$CH_3OH$:$NH_4OH$) to afford the title compound (1.1 g, 5.2 mmol, 49% yield). $^1H$ NMR (300 MHz, $CDCl_3$) δ ppm 1.12 - 1.32 (m, 1 H) 1.44 - 1.63 (m, 2 H) 1.79 - 1.95 (m, 2 H) 2.06 (d, *J*=12.89 Hz, 1 H) 2.25 (d, *J*=1.36 Hz, 3 H) 3.33 - 3.45 (m, I H) 3.65 - 3.78 (m, I H) 3.86 - 4.01 (m, 2 H) 4.44 (dd, *J*=14.92, 2.03 Hz, I H) 6.56 - 6.65 (m, 1 H) 9.48 (s, I H); MS ($DCI/NH_3$) m/z 213 $(M+H)^+$.

Example 157B

2,3-dichloro-N-[(2Z)-5-methyl-3-(tetrahydro-2H-pyran-2-ylmethyl)-1,3-thiazol-2(3H)-ylidene]benzamide

The product of Example 157A (0.15 g, 0.71 mmol), $Et_3N$ (0.30 mL, 2.1 mmol) and 2,3-dichlorobenzoyl chloride (Lancaster, 0.19 g, 0.92 mmol) in 15 mL THF were processed as in Example 129 to afford the title compound (0.14 g, 0.36 mmol, 51% yield). $^1$H NMR (300 MHz, $CDCl_3$) $\delta$ ppm 1.16-1.33 (m, 1H), 1.46-1.56 (m, 3 H), 1.64-1.73 (m, I H), 1.80-1.92 (m, I H), 2.32 (d, *J*=1.4 Hz, 3 H), 3.30-3.43 (m, 1 H), 3.69 (qt, J = 11.5, 8.8, 2.0 Hz, 1 H), 3.91-4.01 (m, 2 H), 4.41 (dd, *J* = 13.9, 2.7 Hz, 1H), 6.86 (d, *J*=1.4 Hz, I H), 7.23 (t, *J*=7.8 Hz, 1 H), 7.49 (dd, *J*=8.0, 1.5 Hz, 1 H), 7.74 (dd, *J*=7.6, 1.5 Hz, 1 H); MS ($DCI/NH_3$) m/z 385 $(M+H)^+$; Anal. calculated for $C_{17}H_{18}Cl_2N_2O_2S$: C, 52.99; H, 4.71; N, 7.27. Found: C, 53.15; H, 4.72; N, 7.14.

Example 158

2-ethoxy-N-[(2Z)-5-methyl-3-(tetrahydro-2H-pyran-2-ylmethyl)-1,3-thiazol-2(3H)-ylidene]benzamide

The product of Example 157A (0.15 g, 0.71 mmol), $Et_3N$ (0.30 mL, 2.1 mmol) and 2-ethoxybenzoyl chloride (Aldrich, 0.18 g, 0.92 mmol) in 15 mL THF were processed as in Example 129 to afford the title compound (0.14 g, 0.39 mmol, 55% yield). $^1$H NMR (300 MHz, $CDCl_3$ $\delta$ ppm 1.20-1.32 (m, I H), 1.46 (t, *J*=7.0 Hz, 3 H), 1.50-1.54 (m, 3 H), 1.63-1.79 (m, I H), 1.80-1.90 (m, 1 H), 2.29 (s, 3 H), 3.32-3.43 (m, I H), 3.64-3.76 (m, 1 H), 3.92-4.00 (m, 2 H), 4.16 (q, *J*=6.8 Hz, 2 H), 4.37-4.47 (m, I H), 6.76-6.84 (m, 1 H), 6.93-7.00 (m, 2 H), 7.35 (t, *J*=8.1 Hz, 1 H), 7.96 (dd, *J*=8.0, 1.9 Hz, 1 H); MS ($DCI/NH_3$) m/z 361 $(M+H)^+$; Anal. calculated for $C_{19}H_{24}N_2O_3S$: C, 63.61; H, 6.71; N, 7.77. Found: C, 63.56; H, 6.73; N, 7.26.

Example 159

5-chloro-2-methoxy-N-[(2Z)-5-methyl-3-(tetrahydrofuran-2-ylmethyl)-1,3-thiazol-2(3H)-ylidene]benzamide

The 5-chloro-2-methoxybenzoic acid (Aldrich, 0.66 g, 3.5 mmol) was converted to 5-chloro-2-methoxybenzoyl chloride with 5 mL of thionyl chloride using the method described in Example 134A. The product of Example 149A (0.35 g, 0.18 mmol), $Et_3N$ (0.74 mL, 5.3 mmol) and 5-chloro-2-methoxybenzoyl chloride in 20 mL THF were processed using the method described in Example 149B to afford the title compound (0.25 g, 0.68 mmol, 20% yield). $^1$H NMR (300 MHz, $CD_3OD$) $\delta$ ppm 1.67 - 1.78 (m, I H), 1.84 - 1.96 (m, 2 H), 2.00-2.13 (m, 1 H), 2.34 (d, *J*=1.4 Hz, 3 H), 3.71 - 3.81 (m, 1 H), 3.85 (s, 3 H), 3.86 - 3.93 (m, 1 H), 4.20 - 4.42 (m, 3 H), 7.07 (d, *J*=9.2 Hz, 1 H), 7.12 - 7.16 (m, 1 H), 7.39 (dd, *J*=8.8, 2.7 Hz, I H), 7.81 (d, *J*=2.7 Hz, I H); MS ($DCI/NH_3$) m/z 367 $(M^+H)^+$; Anal. calculated for $C_{17}H_{19}ClN_2O_3S{\cdot}0.2H_2O$: C, 55.12; H, 5.28; N, 7.56. Found: C, 54.90; H, 4.95; N, 7.55.

Example 160

5-chloro-2-methoxy-N-[(2Z)-5-methyl-3-(tetrahydro-2H-pyran-2-ylmethyl)-1,3-thiazol-2(3H)-ylidene]benzamide

The 5-chloro-2-methoxybenzoic acid (Aldrich, 0.17 g, 0.92 mmol) was converted to 5-chloro-2-methoxybenzoyl chloride with 5 mL of thionyl chloride using the method described in Example 134A. A mixture of the product of Example 157A (0.15 g, 0.71 mmol), $Et_3N$ (0.30 mL, 2.1 mmol) and 5-chloro-2-methoxybenzoyl chloride in 15 mL THF were processed using the method described in Example 129 to afford the title compound (0.11 g, 0.29 mmol, 41% yield). $^1$H NMR (300 MHz, $CDCl_3$) $\delta$ ppm 1.20-1.36 (m, I H), 1.48-1.61 (m, 3 H), 1.66-1.76 (m, I H), 1.83-1.92 (m, 1 H), 2.30 (d *J*= 1.0 Hz, 3 H), 3.33-3.44 (m, I H), 3.67-3.77 (m, I H), 3.90 (s, 3 H), 3.93-4.05 (m, 2 H), 4.40 (dd, *J* = 13.9, 2.4 Hz, I H), 6.83 (s, I H), 6.90 (d, *J*=8.8 Hz, 1 H), 7.32 (dd, *J*=8.8, 2.7 Hz, 1 H), 7.97 (d, *J*=2.7 Hz, 1H); MS ($DCI/NH_3$) m/z 381 $(M+H)^+$; Anal. calculated for $C_{18}H_{21}ClN_2O_3S{\cdot}0.15H_2O$: C, 56.36; H, 5.60; N, 7.30. Found: C, 56.70; H, 5.41; N, 6.91.

Example 161

2-ethoxy-N-[(2Z)-5-methyl-3-(tetrahydrofuran-2-ylmethyl)-1,3-thiazol-2(3H)-ylidene]benzamide

The product of Example 149A (0.20 g, 1.0 mmol), $Et_3N$ (0.42 mL, 3.0 mmol) and the 2-ethoxybenzoyl chloride (Aldrich, 0.23 mL, 1.5 mmol) in 15 mL THF were processed using the method described in Example 149B to afford the title compound (0.18 g, 0.52 mmol, 52% yield). $^1$H NMR (300 MHz, $CD_3OD$) $\delta$ ppm 1.40 (t, *J*=7.0 Hz, 3 H), 1.64-1.75 (m, 1 H), 1.83-1.94 (m, 2 H), 2.00-2.12 (m, 1 H), 2.33 (d, *J*=1.4 Hz, 3 H), 3.70-3.80 (m, 1 H), 3.83-3.93 (m, I H), 4.11 (q,

*J*=7.1 Hz, 2 H), 4.18-4.27 (m, 1 H), 4.28-4.42 (m, 2 H), 6.96 (dt, *J*=7.5, 1.0 Hz, 1 H), 7.04 (d, *J*=8.1 Hz, 1 H), 7.09-7.13 (m, I H), 7.38 (ddd, *J*=8.4, 7.4, 1.9 Hz, I H), 7.77 (dd, *J*=7.6, 1.9 Hz, 1 H); MS (DCI/$NH_3$) m/z 347 $(M+H)^+$; Anal. calculated for $C_{18}H_{22}N_2O_3S{\cdot}0.1\ H_2O$: C, 62.40; H, 6.40; N, 8.09. Found: C, 63.49; H, 5.90; N, 7.84.

Example 162

5-chloro-2-methoxy-N-[(2Z)-5-methyl-3-(tetrahydrofuran-3-ylmethyl)-1,3-thiazol-2(3H)-ylidene]benzamide

Example 162A

Toluene-4-sulfonic acid tetrahydro-furan-3-ylmethyl ester

To a solution of tetrahydro-3-furanmethanol (Aldrich, 1.0 mL, 10.4 mmol) in 5 mL $CH_2Cl_2$ and 5 mL pyridine was added para-toluenesulfonyl chloride (3.0 g, 15.6 mmol) portion-wise over 15 minutes. This mixture stirred at ambient temperature for 3 hours then 5 mL $H_2O$ was added. The layers were separated and the aqueous layer was extracted 2 X 5 mL $CH_1Cl_2$. The combined organics were dried over $Na_2SO_4$, filtered, concentrated under reduced pressure and dried under vacuum (~1 mm Hg) to afford the title compound (2.62 g, 10.2 mmol, 98% yield). $^1H$ NMR (300 MHz, $CDCl_3$) $\delta$ ppm 1.49 - 1.63 (m, 1 H) 1.94 - 2.08 (m, 1H) 2.46 (s, 3 H) 2.52 - 2.68 (m, 1 H) 3.49 (dd, *J*=9.16, 5.09 Hz, 1 H) 3.64 - 3.84 (m, 3 H) 3.88 - 4.03 (m, 2 H) 7.36 (d, *J*=8.14 Hz, 2 H) 7.76 - 7.82 (m, 2 H); MS (DCI/$NH_3$) m/z 257 $(M+H)^+$.

Example 162B

5-Methyl-3-(tetrahydro-furan-3-ylmethyl)-3H-thiazol-2-ylideneamine

A mixture of the product of Example 162A (1.62 g, 6.3 mmol), 2-amino-5-methylthiazole (0.72 g, 6.3 mmol) and LiBr (55 mg, 0.63 mmol) in 2 mL DMF was warmed to 85 °C and allowed to stir for 16 hours. The mixture was then allowed to cool to ambient temperature, diluted with 10 mL $CH_2Cl_2$ and washed with 1 X 5 mL 10% aqueous $Na_2CO_3$ solution. The layers were separated and the aqueous layer was extracted 2 X 5 mL $CH_2Cl_2$. The combined organics were dried over anhydrous $Na_2SO_4$, filtered, concentrated under reduced pressure and purified by column chromatography ($SiO_2$, 9:1:0.1 $CH_2Cl_2$:$CH_3OH$:$NH_4OH$) to afford the title compound (0.31 g, 1.6 mmol, 25% yield). $^1H$ NMR (300 MHz, $CD_3OD$) $\delta$ ppm 1.61 - 1.74 (m, I H) 1.96 - 2.04 (m, 1 H) 2.05 (d, *J* = 1.36 Hz, 3 H) 2.69 - 2.84 (m, 1 H) 3.53 (dd, *J*=8.82, 5.76 Hz, I H) 3.63 (dd, *J*=7.63, 2.20 Hz, 2 H) 3.69 - 3.81 (m, 2 H) 3.89 (ddd, *J*=8.31, 5.42 Hz, I H) 6.36 - 6.42 (m, I H); MS (DCI/$NH_3$) m/z 199 $(M+H)^+$.

Example 162C

5-chloro-2-methoxy-N-[(2Z)-5-methyl-3-(tetrahydrofuran-3-ylmethyl)-1,3-thiazol-2(3H)-ylidene]benzamide

The 5-chloro-2-methoxybenzoic acid (Aldrich, 0.40 g, 2.1 mmol) was converted to 5-chloro-2-methoxybenzoyl chloride with 5 mL of thionyl chloride using the method described in Example 134A. The product of Example 162B (0.21 g, 1.1 mmol), $Et_3N$ (0.44 mL, 3.2 mmol) and 5-chloro-2-methoxybenzoyl chloride in 15 mL THF were processed using the method described in Example 149B to afford the title compound (0.28 g, 0.76 mmol, 72% yield). $^1H$ NMR (300 MHz, $CD_3OD$) $\delta$ ppm 1.69 - 1.83 (m, I H), 1.96 - 2.09 (m, I H), 2.34 (d, *J*=1.4 Hz, 3 H), 2.84 - 3.00 (m, 1 H), 3.64 (dd, *J*=8.8, 5.1 Hz, 1 H), 3.72-3.81 (m, 2H), 3.85(s, 3H), 3.89-3.99 (m, 1 H), 4.17-4.33 (m, 2H), 7.06 (d, *J*=8.8 Hz, 1 H), 7.14 - 7.17 (m, 1 H), 7.39 (dd, *J*=8.8, 2.7 Hz, 1 H), 7.84 (d, *J*=2.7 Hz, 1 H); MS (DCI/$NH_3$) m/z 367 $(M+H)^+$; Anal. calculated for $C_{17}H_{19}ClN_2O_3S$: C, 55.66; H, 5.22; N, 7.64. Found: C, 55.77; H, 4.85; N, 7.26.

Example 163

2-ethoxy-N-[(2Z)-5-methyl-3-(tetrahydrofuran-3-ylmethy)-1,3-thiazol-2(3H)-ylidene]benzamide

The product of Example 162B (0.17 g, 0.86 mmol), $Et_3N$ (0.36 mL, 2.6 mmol) and the 2-ethoxybcnzoyl chloride (Aldrich, 0.15 mL, 0.94 mmol) in 10 mL THF were processed using the method described in Example 149B to afford the title compound (0.26 g, 0.74 mmol, 86% yield). $^1H$ NMR (300 MHz, $CD_3OD$) $\delta$ ppm 1.39 (t, *J*=7.0 Hz, 3 H), 1.68-1.82 (m, 1 H), 1.92-2.05 (m, 1 H), 2.34 (d, *J*=1.4 Hz, 3 H), 2.85-3.02 (m, 1 H), 3.63 (dd, *J*=8.8, 5.4 Hz, I H), 3.70-3.80 (m, 2 H), 3.88-3.97 (m, 1 H), 4.12 (q, *J*=6.9 Hz, 2 H), 4.17-4.33 (m, 2 H), 6.96 (dt, *J*=7.5, 1.0 Hz, I H), 7.05 (d, *J*=7.8 Hz, 1 H), 7.11-7.16 (m, I H), 7.38 (ddd, *J*=8.6, 7.0, 1.7 Hz, I H), 7.79 (dd, *J*=7.8, 1.7 Hz, 1 H); MS (DCI/$NH_3$) m/z 347 $(M+H)^+$; Anal. calculated for $C_{18}H_{22}N_2O_3S$: C, 62.40; H, 6.40; N, 8.09. Found: C, 62.43; H, 6.29; N, 7.96.

Example 164

2-ethoxy-N-[(2Z)-3-[2-(2-methoxyethoxy)ethyl]-5-methyl-1,3-thiazol-2(3H)-ylidene]benzamide

Example 164A

3-[2-(2-Methoxyethoxy)ethyl]-5-methyl-1,3-thiazol-2(3H)-ylideneamine

A mixture of 2-amino-5-methylthiazole (1.5 g, 13.0 mmol) and 1-promo-2-(2-methoxyethoxy)ethane (Aldrich, 2.0 mL, 14.5 mmol) was processed as in Example 157A to afford the title compound (2.2 g, 10.9 mmol, 78% yield). $^1H$ NMR (300 MHz, $CD_3OD$) δ ppm 2.29 (d, *J*=1.36 Hz, 3 H) 3.34 (s, 3 H) 3.49 - 3.54 (m, 2 H) 3.60 - 3.66 (m, 2 H) 3.80 (t, *J*=5.10 Hz, 2 H) 4.13 (t, *J*=4.80 Hz, 2 H) 6.99 - 7.04 (m, I H); MS (DCI/$NH_3$) m/z 217 $(M+H)^+$.

Example 164B

2-ethoxy-N-[(2Z)-3-[2-(2-methoxyethoxy)ethyl]-5-methyl-1,3-thiazol-2(3H)-ylidene]benzamide

The product of Example 164A (0.22 g, 1.0 mmol), $Et_3N$ (0.23 mL, 2.0 mmol) and 2-ethoxybenzoyl chloride (Aldrich, 0.25 g, 1.3 mmol) in 15 mL THF were processed as in Example 129 to afford the title compound (0.15 g, 0.40 mmol, 40% yield). $^1H$ NMR (300 MHz, $CD_3OD$) δ ppm 1.40 (t, *J*=7.0 Hz, 3 H), 2.33 (d, *J*=1.4 Hz, 3 H), 3.31 (s, 3 H), 3.46-3.51 (m, 2 H), 3.58-3.63 (m, 2 H), 3.85 (t, *J*=5.3 Hz, 2 H), 4.11 (q, *J*=6.8 Hz, 2 H), 4.40 (t, *J*=5.3 Hz, 2 H), 6.96 (dt, *J*=7.5, 1.0 Hz, I H), 7.04 (d, *J*=8.5 Hz, I H), 7.11-7.15 (m, 1 H), 7.38 (ddd, *J*=8.4, 7.4, 1.9 Hz, I H), 7.79 (dd, *J*=7.6, 1.9 Hz, I H); MS (DCI/$NH_3$) m/z 365 $(M+H)^+$; Anal. calculated for $C_{18}H_{24}N_2O_4S$: C, 59.88; H, 6.86; N, 7.51. Found: C, 60.05; H, 6.81; N, 7.60.

Example 166

2,3-dichloro-N-[(2Z)-3-[2-(2-methoxyethoxy)ethyl]-5-methyl-1,3-thiazol-2(3H)-ylidene]benzamide

The product of Example 164A (0.22 g, 1.0 mmol), $Et_3N$ (0.23 mL, 2.0 mmol) and 2,3-dichlorobenzoyl chloride (Lancaster, 0.27 g, 1.3 mmol) in 15 mL THF were processed as in Example 129 to afford the title compound (95 mg, 0.24 mmol, 24% yield). $^1H$ NMR (300 MHz, $CD_3OD$) δ ppm 2.36 (d, *J*=1.4 Hz, 3 H), 3.31 (s, 3 H), 3.46-3.50 (m, 2 H), 3.57-3.62 (m, 2 H), 3.83 (t, *J*=5.4 Hz, 2 H), 4.40 (t, *J*=5.1 Hz, 2 H), 7.18-7.21 (m, 1 H), 7.33 (t, *J*=7.8 Hz, 1 H), 7.58 (dd, *J*=8.0, 1.5 Hz, 1 H), 7.64 (dd, *J*=7.6, 1.5 Hz, I H); MS (DCI/$NH_3$) m/z 389 $(M+H)^+$; Anal. calculated for $C_{16}H_{18}Cl_2N_2O_3S$: C, 49.36; H, 4.66; N, 7.20. Found: C, 48.98; H, 4.60; N, 6.99.

Example 167

5-chloro-2-methoxy-N-[(2Z)-3-[2-(2-methoxyethoxy)ethyl]-5-methyl-1,3-thiazol-2(3H)-ylidene]benzamide

The 5-chloro-2-methoxybenzoic acid (Aldrich, 0.24 g, 1.3 mmol) was converted to the 5-chloro-2-methoxybenzoyl chloride with 5 mL of thionyl chloride using the method described in Example 134A. The product of Example 164A (0.22 g, 1.0 mmol), $Et_3N$ (0.42 mL, 3.0 mmol) and 5-chloro-2-methoxybenzoyl chloride in 15 mL THF were processed using the method described in Example 129 to afford the title compound (0.21 g, 0.55 mmol, 55% yield). $^1H$ NMR (300 MHz, $CD_3OD$) δ ppm 2.34 (d, *J*=1.4 Hz, 3 H), 3.31 (s, 3H), 3.47-3.52 (m, 2 H), 3.59-3.64 (m, 2 H), 3.86 (s, 3 H), 3.88 (t, *J*=5.4 Hz, 2 H), 4.41 (t, *J*=4.8 Hz, 2 H), 7.07 (d, *J*=8.8 Hz, 1 H), 7.14-7.17 (m, 1 H), 7.39 (dd, *J*=9.0, 2.9 Hz, 1 H), 7.83 (d, *J*=2.7 Hz, 1H); MS (DCI/$NH_3$) m/z 385 $(M+H)^+$; Anal. calculated for $C_{17}H_{21}ClN_2O_4S$: C, 53.05; H, 5.50; N, 7.28. Found: C, 52.93; H, 5.61; N, 7.26.

Example 168

2-methoxy-N-[(2Z)-5-methyl-3-(tetrahydrofuran-2-ylmethyl)-1,3-thiazol-2(3H)-ylidene]benzamide

The product of Example 149A (0.15 g, 0.76 mmol), $Et_3N$ (0.32 mL, 2.3 mmol) and the o-anisoyl chloride (Aldrich, 0.15 mL, 1.1 mmol) in 10 mL THF were processed using the method described in Example 149B to afford the title compound (88 mg, 0.26 mmol, 35% yield). $^1H$ NMR (300 MHz, $CD_3OD$) δ ppm 1.66-1.76 (m, 1 H), 1.84-1.95 (m, 2 H), 2.02-2.12 (m, 1 H), 2.33 (d, *J*=1.4 Hz, 3 H), 3.71-3.79 (m, 1 H), 3.85 (s, 3 H), 3.84-3.92 (m, 1 H), 4.22-4.28 (m, 1 H), 4.29-4.41 (m, 2 H), 6.98 (dt, *J*=7.6, 1.0 Hz, 1 H), 7.07 (dd, *J*=8.5, 1.0 Hz, 1 H), 7.10-7.12 (m, 1 H), 7.42 (ddd, *J*=9.2, 7.5,

2.0 Hz, 1 H), 7.85 (dd, *J*=7.8, 1.7 Hz, 1 H); MS (DCI/$NH_3$) m/z 333 $(M+H)^+$; Anal. calculated for $C_{17}H_{20}N_2O_3S$: C, 61.42; H, 6.06; N, 8.43. Found: C, 61.35; H, 6.10; N, 8.28.

Example 169

1-(1,1-Dimethylpropyl)-3-[(2Z)-3-(2-methoxyethyl)-4,5-dimethyl-1,3-thiazol-2(3H)-ylidene]urea hydrochloride

Example 169A

1-(1,1-Dimethylpropyl)-3-[(2Z)-3-(2-methoxyethyl)-4,5-dimethyl-1,3-thiazol-2(3H)-ylidene]urea

To a solution of 1,1-dimethylpropylamine (174mg, 2.0 mmole) in 19 mL of THF and 1 mL of *N,N*-diisopropylethyl amine was added 4-nitrophenyl chloroformate (403 mg, 2.0 mmole). The solution was irradiated in a sealed tube placed in a single node microwave at 70°C for 300 sec (maximum power 300W) with stirring. The resulting solution was cooled to room temperature and 3-(2-methoxyethyl)-4,5-dimethyl-3H-thiazol-2-ylideneamine hydrobromide (587 mg, 2.2 mmole) from Example 12A was added. The sealed tube was irradiated at 120 °C for 1800 sec with stirring. The mixture was cooled and the volatile components were removed under reduced pressure. The residue was partitioned between water and ethyl acetate. The phases were separated and organic extract was dried over anhydrous $Na_2SO_4$, filtered, and concentrated. Purification by column chromatography ($SiO_2$, 0-70% ethyl acetate/hexanes gradient) afforded the title compound. $^1H$ NMR (300 MHz, DMSO-$d_6$) δ ppm 0.76 (t, *J*=7 Hz, 3 H) 1.20 (s, 6 H) 1.66 (d, *J*=7 Hz, 2 H) 2.06 (s, 3 H) 2.11 (s, 3 H) 3.30 (s, 3 H) 3.55 (t, *J*=5 Hz, 2 H) 4.06 (t, *J*=5 Hz, 2 H) 6.13 (s, 1 H)), MS (DCI/$NH_3$) m/z 300 $(M+H)^+$.

Example 169B

1-(1,1-Dimethylpropyl)-3-[(2Z)-3-(2-methoxyethyl)-4,5-dimethyl-1,3-thiazol-2(3H)-ylidene]urea hydrochloride

To a solution of the product from Example 169A in MeOH was added a solution of HCl in $Et_2O$. The title compound was isolated by filtration. $^1H$ NMR (300 MHz, DMSO-$d_6$) δ ppm 0.84 (t, *J*=7 Hz, 3 H) 1.26 (s, 6 H) 1.65 (q, *J*=7 Hz, 2 H) 2.24 (s, 6 H) 3.24 (s, 3 H) 3.64 (t, *J*=5 Hz, 2 H) 4.45 (s, 2 H), MS (DCI/$NH_3$) m/z 300 $(M+H)^+$. Anal. Calculated for $C_{14}H_{25}ClN_3O_2S$: C, 50.06 H, 7.80; N, 12.51. Found: C, 50.11; H, 7.87; N, 12.35.

Example 170

1-(1,1-Dimethyl-propyl)-3-[(2Z)-3-(2-methoxyethyl)-1,3-benzothiazol-2(3H)-ylidene]urea

To a solution of 1,1-dimethyl-propylamine (0.60 mL, 5.2 mmol) and triethylamine (0.40 mL, 2.9 mmol) in 12 mL of a 1:1 mixture of THF:DMF at 0 °C was added *p*-nitrophenylchloroformate (0.58 g, 2.9 mmol). After 30 minutes, the product of Example 64A (0.75 g, 2.6 mmol) and another aliquot of triethylamine (0.40 mL, 2.9 mmol) were added and the solution stirred at ambient temperature for 9 hours. The mixture was diluted with ethyl acetate then washed twice with water and brine. The organic extract was dried over magnesium sulfate, filtered, and concentrated under reduced pressure. Purification by column chromatography ($SiO_2$, 20-40% ethyl acetate/hexanes gradient) afforded 0.06 g (8%) of the title compound. $^1H$ NMR ($CDCl_3$, 300 MHz) δ ppm 0.91 (t, *J*=7.46 Hz, 3 H), 1.39 (s, 6 H), 1.78 (q, *J*=7.46 Hz, 2 H), 3.28 (s, 3 H), 3.86 (t, *J*=4.92 Hz, 2 H), 4.82 (t, *J*=4.58 Hz, 2 H), 7.46 (t, *J*=7.63 Hz, 1 H), 7.58 (t, *J*=7.63 Hz, 1 H), 7.64 - 7.70 (m, 1 H), 7.75 (d, *J*=7.80 Hz, 1 H), 9.06 (s, 1 H). MS (DCI/$NH_3$) m/z 322 $(M+H)^+$.

Example 171

1-[(2Z)-3-(2-Methoxyethyl)-4,5-dimethyl-1,3-thiazol-2(3H)-ylidene]-3-(3,3,5,5-tetramethylcyclohexyl)lurea

The product of Example 12A and 3,3,5,5-tetramethylcyclohexylamine were processed using the method described in Example 169A to afford the title compound. $^1H$ NMR (300 MHz, DMSO-$d_6$) δ ppm 0.90 (s, 6 H) 1.02 (s, 6 H) 1.17 - 1.27 (m, I H) 1.61 (d, *J*=12 Hz, I H) 2.18 - 2.27 (m, 6 H) 3.24 (s, 3H) 3.62 (t, *J*=5 Hz, 2 H) 3.76 - 3.87 (m, *J*=5 Hz, 2 H) 3.98 (s, 2 H) 4.40 (s, 2 H), MS (DCI/$NH_3$) m/z 366 $(M+H)^+$. Anal. Calculated for $C_{19}H_{14}ClN_3O_2S$•$0.7CH_4O$: C, 55.49, H, 8.7 N, 9.85. Found C, 55.81 H, 8.37 N, 9.52.

Example 172

1-[(2Z)-3-(2-Methoxyethyl)-4,5-dimethyl-1,3-thiazol-2(3H)-ylidene]-3-(1-methyl-3-phenylpropyl)urea

The product of Example 12A and 1-methyl-3-phenylpropylamine were processed using the method described in Example 169A to afford the title compound. $^1H$ NMR (300 MHz, DMSO-$d_6$) δ ppm 1.13 (d, *J*=6 Hz, 3 H) 1.60 - 1.82 (m, 2H) 2.18 (s, 3H) 2.20 (s, 3H) 2.55 - 2.68 (m, 2H) 3.25 (s, 3 H) 3.53 - 3.80 (m, 3 H) 4.31 (s, 2 H) 7.08 - 7.35 (m, 5 H), MS (DCI/$NH_3$) m/z 362 $(M+H)^+$.

Example 173

ethyl *N*-({[(2Z)-3-(2-methoxyethyl)-4,5-dimethyl-1,3-thiazol-2(3*H*)-ylidene]amino}carbonyl)-L-leucinate

The product of Example 12A and (2S)-ethyl-2-amino-4-methylpentanoate were processed using the method described in Example 169A to afford the title compound. $^1H$ NMR (300 MHz, DMSO-$d_6$) δ ppm 0.87 (dd, *J*=9, 6 Hz, 6 H) 1.37 - 1.50 (m, 1 H) 1.51 - 1.65 (m, 2 H) 1.84 (s, 3 H) 2.17 (s, 3 H) 2.21 (s, 3 H) 3.22 - 3.23 (m, 3 H) 3.62 (t, *J*=5 Hz, 2 H) 4.18 - 4.33 (m, 2 H) 4.37 - 4.48 (m, 1 H) 7.88 (d, *J*=9 Hz, 1 H)), MS (DCI/$NH_3$) m/z 342 $(M+H)^+$. Anal. Calculated for $C_{16}H_{27}N_3O_3S$: C, 56.26 H, 7.97 N, 12.31. Found C, 56.20 H, 8.04 N, 12.31.

Example 174

1-(1,1-Dimethylpropyl)-3-[(2Z)-5-methyl-3-(tetrahydropyran-2-ylmethyl)-1,3-thiazol-2(3H)-ylidene]urea

The product of Example 157A and 2,2-dimethylpropylamine were processed using the method described in Example 169A to afford the title compound. $^1H$ NMR (300 MHz, DMSO-$d_6$) δ ppm 0.76 (t, 3 H) 1.12 - 1.17 (m, 1 H) 1.20 (s, 6 H) 1.38 - 1.49 (m, *J*=3 Hz, 3 H) 1.50 - 1.59 (m, 1 H) 1.66 (q, *J*=7 Hz, 2 H) 1.75 - 1.82 (m, 1 H) 2.12 (s, 3 H) 3.22 - 3.30 (m, 1 H) 3.54 - 3.65 (m, 1 H) 3.80 - 3.93 (m, 3 H) 6.19 (s, 1 H) 6.79 (s, 1 H), MS (DCI/$NH_3$) m/z 326 $(M+H)^+$. Anal. Calculated for $C_{16}H_27N_3O_2S$: C, 59.04 H, 8.36 N, 12.91. Found C, 59.06 H, 8.36 N, 12.91.

Example 175

1-(1,2-Dimethyl-propyl)-3-[(2Z)-5-methyl-3-(tetrahydropyran-2-ylmethyl)-1,3-thiazol-2(3H)-ylidene]urea hydrochloride

The product of Example 157A and 1,2-dimethylpropylamine were processed according to the method described in Example 169A to afford the title compound. $^1H$ NMR (300 MHz, DMSO-$d_6$) δ ppm 0.81 (dd, *J*=7,3Hz, 1 H) 0.84 - 0.96 (m, 6 H) 1.06 (d, *J*=7 Hz, 3 H) 1.20 (t, I H) 1.34 - 1.54 (m, 3 H) 1.60 - 1.76 (m, 1 H) 1.69 - 1.95 (m, 2 H) 2.28 (s, 3 H) 3.22 - 3.37 (m, I H) 3.84 (d, *J*=11 Hz, 2 H) 4.10-4.30 (m, 1 H) 4.31 - 4.47 (m, 1 H) 7.23 - 7.41 (m, 1 H) 7.83-8.06 (m, 1 H), ), MS (DGI/$NH_3$) m/z 326 $(M+H)^+$. Anal. Calculated for $C_{16}H_{28}ClN_3O_2S$: C,53.10 H, 7.80 N, 11.16. Found C, 52.73 H, 7.96 N, 10.82.

Example 176

1-Cyclohexyl-3-[(2Z)-4,5-dimethyl-3-(tetrahydropyran-2-ylmethyl)-1,3-thiazol-2(3H)-ylidene]urea

Example 176A

4,5-Dimethyl-3-(tetrahydropyran-2-ylmethyl)-1,3-thiazol-2(3H)-ylideneamine hydrobromide

A mixture of 2-amino-4,5-dimethylthiazole and 2-(bromomethyl)tetrahydro-2H-pyran were processed using the method described in Example 12A to afford the title compound $^1H$ NMR (300 MHz, DMSO-$d_6$) δ ppm 1.13 - 1.31 (m, I H) 1.36 - 1.52 (m, 3 H) 1.64 - 1.85 (m, 2 H) 2.18 (d, *J*=4 Hz, 6 H) 3.19 - 3.33 (m, 1 H) 3.49 - 3.63 (m, 1 H) 3.77 - 3.89 (m, 1 H) 3.94 - 4.02 (m, 2 H) 9.34 (s, 2 H), ), MS (DCI/$NH_3$) m/z 227 $(M+H)^+$.

Example 176B

1-Cyclohexyl-3-[(2Z)-4,5-dimethyl-3-(tetrahydropyran-2-ylmethyl)-1,3-thiazol-2(3H)-ylidene]urea

The product of Example 176A and cyclohexylamine were processed using the method described in Example

169A to afford the title compound. $^1$H NMR (300 MHz, DMSO-$d_6$) δ ppm 1.05 - 1.29 (m, 6H) 1.37- 1.49 (m, 3 H) 1.51 - 1.63 (m, 2 H) 1.76 (m, 5H) 2.06 (s, 3 H) 2.11 (s, 3H) 3.19 - 3.30 (m, 1 H) 3.56 - 3.68 (m, I H) 3.70 - 3.88 (m, 3 H) 3.99 (dd, *J*=14, 3 Hz, 1 H) 6.59 (d, *J*=8 Hz, 1 H), ), MS (DCI/$NH_3$) m/z 352 $(M+H)^+$. Anal. Calculated for $C_{18}H_29N_3O_2S$•$0.1H_2O$: C, 61.19 H, 8.33 N, 11.89. Found C, 61.03 H, 8.45 N, 11.69.

Example 177

1-(4-Methylcyclohexyl)-3-[(2Z)-5-methyl-3-(tetrahydropyran-2-ylmethyl)-1,3-thiazol-2(3H)-ylidene]urea

The product of Example 157A and 4-methylcyclohexylamine were processed using the method described in Example 169A to afford the title compound. $^1$H NMR (300 MHz, DMSO-$d_6$) δ ppm 0.88 (dd, *J*=12, 7 Hz, 3 H) 0.96 (d, *J*=3 Hz, I H) 1.09 - 1.31 (m, 3 H) 1.31 - 1.70 (m, 9 H) 1.69 - 1.85 (m, 2 H) 2.12 (s, 3 H) 3.21 - 3.29 (m, 1 H) 3.55 - 3.64 (m, *J*=3 Hz, 2 H) 3.79 - 3.95 (m, 3 H) 6.62 (t, *J*=8 Hz, I H) 6.77 - 6.81 (m, *J*=1 Hz, 1 H), MS (DCI/$NH_3$) m/z 352 $(M+H)^+$.

Example 178

1-(1,1-Dimethylpropyl)-3-[(2Z)-3-(tetrahydropyran-2-ylmethyl)-1,3-thiazol-2(3H)-ylidene]urea

Example 178A

3-(tetrahydropyran-2-ylmethyl)-1,3-thiazol-2(3H)-ylideneamine hydrobromide

A mixture of 2-aminothiazole and 2-(bromomethyl)tetrahydro-2H-pyran were processed using the method described in Example 12A to afford the title compound. MS (DCI/$NH_3$) m/z 199 $(M+H)^+$

Example 178B

1-(1,1-Dimethylpropyl)-3-[(2Z)-3-(tetrahydropyran-2-ylmethyl)-1,3-thiazol-2(3H)-ylidene]urea

The product of Example 178A and 1,1-dimethylpropylamine were processed using the method described in Example 169A to afford the title compound. $^1$H NMR (300 MHz, DMSO-$d_6$) δ ppm 0.77 (t, *J*=7 Hz, 3 H) 1.14 - 1.19 (m, 1 H) 1.21 (s, 6 H) 1.39 - 1.49 (m, 3 H) 1.50 - 1.59 (m, 1 H) 1.61 - 1.72 (m, 2 H) 1.79 (s, 1 H) 3.19 - 3.28 (m, 1 H) 3.56 - 3.68 (m, 1 H) 3.78 - 3.89 (m, 1 H) 3.92 - 4.02 (m, 2 H) 6.23 (s, 1 H) 6.55 (d, *J*=5 Hz, 1 H) 7.08 (d, *J*=5 Hz, 1 H), MS (DCI/$NH_3$) m/z 312 $(M+H)^+$. Anal. Calculated for $C_{15}H_{25}N_3O_2S$: C, 57.85 H, 8.09 N, 13.49 Found C, 58.01 H, 8.23 N, 13.30.

Example 179

*N*-[(2*Z*)-4,5-dimethyl-3-(tetrahydro-2*H*-pyran-2-ylmethyl)-1,3-thiazol-2(3*H*)-ylidene]-*N*'-[(1*S*)-1,2,2-trimethylpropyl]urea

The product of Example 176A and (1S)-1,2,2-trimethylpropylamine were processed using the method described in Example 169A to afford the title compound. $^1$H NMR (300 MHz, DMSO-$d_6$) δ ppm 0.84 (s, 9 H) 0.98 (d, *J*=7 Hz, 3 H) 1.12 - 1.28 (m, 1 H) 1.40 - 1.49 (m, 3 H) 1.47 - 1.64 (m, *J*=13 Hz, 1 H) 1.73 - 1.84 (m, 1 H) 2.12 (s, 3 H) 3.23 - 3.30 (m, 1 H) 3.50 - 3.68 (m, 2 H) 3.80 - 4.03 (m, 3 H) 6.46 (dd, *J*=9, 3 Hz, I H) 6.80 (dd, *J*=5, 2 Hz, 1 H), MS (DCI/$NH_3$) m/z 354 $(M+H)^+$. Anal. Calculated for $C_{18}H_{31}N_3O_2S$ C, 61.15 H, 8.84 N, 11.69. Found C, 60.80 H, 8.88 N, 11.69.

Example 180

1-(2,2-Dimethylpropyl)-3-[(2Z)-4,5-dimethyl-3-(tetrahydropyran-2-ylmethyl)-1,3-thiazol-2(3H)-ylidene]urea

The product of Example 176A and 2,2-dimethylpropylamine were processed using the method described in Example 169A to afford the title compound. $^1$H NMR (300 MHz, DMSO-$d_6$) δ ppm 0.84 (s, 9 H) 1.16 - 1.28 (m, 1 H) 1.45 (s, 3 H) 1.56 - 1.68 (m, 1 H) 1.76 - 1.87 (m, 1 H) 2.06 (s, 3 H) 2.11 (s, 3 H) 2.77 - 2.87 (m, 1 H) 2.90 - 3.02 (m, 1 H) 3.15 - 3.28 (m, I H) 3.60 - 3.70 (m, I H) 3.72 - 3.86 (m, 2 H) 3.94 - 4.11 (m, 1 H) 6.58 - 6.78 (m, 1 H), ), MS (DCI/$NH_3$) m/z 339 $(M+H)^+$. Anal. Calculated for $C_{17}H_{19}N_3O_2S$: C, 60.14 H, 8.61 N, 12.38. Found C, 60.22 H, 8.71 N, 12.35.

Example 181

*N*-[(2*Z*)-3-(tetrahydro-2*H*-pyran-2-ylmethyl)-1,3-thiazol-2(3*H*)-ylidene]-*N*'-[(1*S*)-1,2,2-trimethylpropyl]urea

The product of Example 178A and (1*S*)-1,2,2-trimethylpropylamine were processed using the method described in Example 169A to afford the title compound. $^1$H NMR (300 MHz, DMSO-$d_6$) δ ppm 0.84 (s, 9 H) 0.99 (d, *J*=7 Hz, 3 H) 1.14 - 1.26 (m, *J*=11 Hz, 1 H) 1.36 - 1.49 (m, 3 H) 1.52 - 1.59 (m, I H) 1.74 - 1.82 (m, 1 H) 3.20 - 3.28 (m, 1 H) 3.52 - 3.70 (m, 2 H) 3.78 - 4.08 (m, 3 H) 6.46 - 6.60 (m, 2 H) 7.10 (t, *J*=5 Hz, 1 H), MS (DCI/$NH_3$) m/z 326 (M+H)$^+$. Anal. Calculated for $C_{16}H_{27}N_3O_2S$: C, 59.04 H, 8.36 N, 12.91. Found C, 59.08 H, 8.28 N, 12.80.

Example 182

*N*-[(2*Z*)-5-methyl-3-(tetrahydro-2*H*-pyran-2-ylmethyl)-1,3-thiazol-2(3*H*)-ylidene]-*N*'-[(1*S*)-1,2,2-trimethylpropyl]urea

The product of Example 157A and (1S)-1,2,2-trimethylpropylamine were processed using the method described in Example 169A to afford the title compound. $^1$H NMR (300 MHz, DMSO-$d_6$) δ ppm 0.84 (s, 9 H) 0.98 (d, *J*=7 Hz, 3 H) 1.11 - 1.30 (m, 1 H) 1.45 (s, 3 H) 1.51 (d, 2 H) 2.12 (s, 4 H) 3.21 - -3.30 (m, 1 H) 3.48 - 3.70 (m, 2 H) 3.78-4.05 (m, 3 H) 6.46 (dd, *J*=9, 3 Hz, 1 H) 6.80 (dd, *J*=5, 2 Hz, 1 H), MS (DCI/$NH_3$) m/z 339 (M+H)$^+$. Anal. Calculated for $C_{17}H_{29}N_3O_{2s}$ C, 60.14 H, 8.61 N, 12.38. Found C, 60.10 H, 8.81 N, 12.02.

Example 183

1-(4-Methylcyclohexyl)-3-[(2*Z*)-4,5-dimethyl-3-(tetrahydropyran-2-ylmethyl)-1,3-thiazol-2(3*H*)-ylidene]urea

The product of Example 176A and 4-methylcyclohexylamine were processed using the method described in Example 169A to afford the title compound. $^1$H NMR (300 MHz, DMSO-$d_6$) δ ppm 0.82 - 1.02 (m, 4 H) 1.15 - 1.29 (m, 3 H) 1.38 - 1.51 (m, 4 H) 1.52 - 1.70 (m, 4 H) 1.78 (s, 3 H) 2.06 (s, 3 H) 2.11 (s, 3 H) 3.19 - 3.28 (m, 1 H) 3.61 (s, 1 H) 3.69 - 3.86 (m, 3 H) 3.98 (dd, *J*=14, 3 Hz, 1 H) 6.57 (d, *J*=8 Hz, 1 H), MS (DCI/$NH_3$) m/z 366 (M+H)$^+$. Anal. Calculated for $C_{18}H_{23}N_3O_2S$: C, 62.45 H, 8.55 N, 11.50. Found C, 62.77 H, 8.86 N, 1150.

Example 184

1-(2,2-Dimethylpropyl)-3-[(2Z)-5-methyl-3-(tetrahydropyran-2-ylmethyl)-1,3-thiazol-2(3H)-ylidene]urea

The product of Example 157A and 2,2-dimethylpropylamine were processed using the method described in Example 169A to afford the title compound. $^1$H NMR (300 MHz, DMSO-$d_6$) δ ppm 0.81 (s, 9 H) 1.09 - 1.26 (m, 1 H) 1.34 - 1.59 (m, 4 H) 1.69 - 1.87 (m, 1 H) 2.12 (d, *J*=1 Hz, 3 H) 2.80 - 2.98 (in, 2 H) 3.08 - 3.27 (m, 1 H) 3.52 - 3.67 (m, I H) 3.80 - 4.06 (m, 3 H) 6.68 - 6.76 (m, 1 H) 6.78 - 6.90 (m, I H), MS (DCI/$NH_3$) m/z 326 (M+H)$^+$. Anal. Calculated for $C_{16}H_{27}N_3O_2S$: C, 59.04 H, 8.36 N, 12.91. found C, 58.89 H, 8.56 N, 12.71.

Example 185

1-tert-Butyl-3-[(2Z)-4,5-dimethyl-3-(tetrahydropyran-2-ylmethyl)-1,3-thiazol-2(3H)-ylidene]urea

The product of Example 176A and *tert*-butylamine were processed using the method described in Example 169A to afford the title compound. $^1$H NMR (300 MHz, DMSO-$d_6$) δ ppm 1.13 - 1.22 (m, 1 H) 1.27 (s, 9 H) 1.44 (d, *J*=4 Hz, 3 H) 1.60 (d, *J*=12 Hz, 1 H) 1.77 (s, 1 H) 2.06 (s, 3 H) 2.11 (s, 3 H) 3.16 - 3.28 (m, 1 H) 3.55 - 3.68 (m, 1 H) 3.68 - 3.87 (m, 2 H) 4.00 (dd, *J*=14, 3 Hz, 1 H) 6.90 (d, *J*=9 Hz, 1 H), MS (DCI/$NH_3$) m/z 326 (M+H)$^+$. Anal. Calculated for $C_{16}H_{27}N_3O_2S\cdot0.3H_2O$: C, 58.02 H, 8.41 N, 12.70. Found C, 58.44 H, 8.12 N, 12.41.

Example 186

1-(1,1-Dimethylpropyl)-3-[(2Z)-4,5-dimethyl-3-(tetrahydrofuran-2-ylmethyl)-1,3-thiazol-2(3H)-ylidene]urea

Example 186A

4,5-Dimethyl-3-(tetrahydrofuran-2-ylmethyl)-1,3-thiazol-2(3H)-ylideneamine hydrobromide

A mixture of 2-amino-4,5-dimethylthiazole and 2-(bromomethyl)tetrahydrofuran were processed using the method described in Example 12A to afford the title compound. $^1$H NMR (300 MHz, DMSO-$d_6$) $\delta$ ppm 1.43 - 1.64 (m, I H) 1.73 - 2.00 (m, 3 H) 2.17 (s, 3 H) 2.19 (s, 3 H) 3.53 - 3.68 (m, 1 H) 3.71 - 3.85 (m, 1 H) 3.91 - 4.17 (m, 3 H) 9.34 (s, 1 H), ), MS (DCI/$NH_3$) m/z 212 $(M+H)^+$.

Example 186B

1-(1,1-Dimethylpropyl)-3-[(2Z)-4,5-dimethyl-3-(tetrahydrofuran-2-ylmethyl)-1,3-thiazol-2(3H)-ylidene]urea

The product of Example 186A and 1,1-dimethylpropylamine were processed using the method described in Example 169A to afford the title compound. $^1$H NMR (300 MHz, DMSO-$d_6$) $\delta$ ppm 0.84 (t, *J*=7 Hz, 3 H) 1.22 - 1.31 (m, 6 H) 1.53 - 1.72 (m, 3 H) 1.75 - 1.86 (m, 1 H) 1.91 - 2.05 (m, I H) 2.08 - 2.20 (m, 1 H) 2.24 (s, 6 H) 3.55 - 3.71 (m, 1 H) 3.76 - 3.88 (m, 1 H) 4.09 - 4.20 (m, I H) 4.30 (s, 1 H) 4.40 - 4.54 (m, 1 H) 6.94 (d, *J*=9 Hz, 1 H), MS (DCI/$NH_3$) m/z 326 $(M+H)^+$.

Example 187

1-(2,2-Dimethylpropyl)-3-[(2Z)-4,5-dimethyl-3-(tetrahydrofuran-2-ylmethyl)-1,3-thiazol-2(3H)-ylidene]urea

The product of Example 186A and 2,2-dimethylpropylamine were processed using the method described in Example 169A to afford the title compound. $^1$H NMR (300 MHz, DMSO-$d_6$) $\delta$ ppm 0.86 (s, 9 H) 1.54 - 1.65 (m, I H) 1.77 - 1.97 (m, 3 H) 2.07 (s, 3 H) 2.10 - 2.14 (m, 3 H) 2.78 - 2.97 (m, 2 H) 3.54 - 3.65 (m, I H) 3.71 - 3.85 (m, 2 H) 3.98 - 4.11 (m, 1 H) 4.14 - 4.31 (m, I H) 6.66 (t, *J*=7 Hz, 1 H), MS (DCI/$NH_3$) m/z 326 $(M+H)^+$. Anal. Calculated for $C_{16}H_{17}N_3O_2S$: C, 59.04 H, 8.36 N, 12.91. Found C, 58.91 H, 8.64 N, 12.77.

Example 188

1-[(2Z)-4,5-Dimethyl-3-(tetrahydropyran-2-ylmethyl)-1,3-thiazol-2(3H)-ylidene]-3-(3,3,5,5-tetramethylcyclohexyl)urea

The product of Example 176A and 3,3,5,5-tetramethylcyclohexylamine were processed using the method described in Example 169A to afford the title compound. $^1$H NMR (300 MHz, DMSO-$d_6$) $\delta$ ppm 0.88 (s, 6 H) 0.91 - 1.01 (m, 2 H) 1.01 - 1.05 (m, 6 H) 1.20 (d, *J*=14 Hz, 2 H) 1.37 - 1.62 (m, 7 H) 1.77 (d, *J*=4 Hz, 1H) 2.04 - 2.09 (m, 3 H) 2.09 - 2.15 (m, 3 H) 3.09 - 3.28 (m, 1 H) 3.55 - 3.87 (m, 4 H) 3.98 (dd, *J*=14, 3 Hz, 1 H) 6.56 (d, *J*=8 Hz, 1 H), MS (DCI/$NH_3$) m/z 408 $(M+H)^+$. Anal. Calculated for $C_{22}H_{37}N_3O_2S\bullet0.4H_2O$: C, 63.7 H, 89.18N, 10.13. Found C, 63.49 H, 8.93 N, 10.12.

Example 189

*N*-[(2*Z*)-4,5-dimethyl-3-(tetrahydrofuran-2-ylmethyl)-1,3-thiazol-2(3*H*)-ylidene]-*N*'-[(1*S*)-1,2,2-trimethylpropyl]urea

The product of Example 186A and (1*S*)-1,2,2-trimethylpropylamine were processed using the method described in Example 169A to afford the title compound. $^1$H NMR (300 MHz, DMSO-$d_6$) $\delta$ ppm 0.83 (s, 9 H) 0.98 (dd, *J*=7, 2 Hz, 3 H) 1.53 - 1.64 (m, 1 H) 1.76 - 1.99 (m, 3 H) 2.06 (s, 3 H) 2.11 (s, 3 H) 3.53 - 3.65 (m, 2 H) 3.73 - 3.84 (m, 2 H) 4.05 - 4.14 (m, 1 H) 4.17 - 4.22 (m, 1 H) 6.34 - 6.42 (m, 1 H), MS (DCI/$NH_3$) m/z 339 $(M+H)^+$. Anal. Calculated for $C_{17}H_{19}N_3O_2S$: C, 60.14 H, 8.61 N, 12.38. Found C, 60.06 H, 8.95 N, 12.29.

Example 190

*N*-[(2*Z*)-4,5-dimethyl-3-(tetrahydro-2*H*-pyran-2-ylmethyl)-1,3-thiazol-2(3*H*)-ylidene]-*N*'-[(1*R*)-1,2,2-trimethylpropyl]urea

The product of Example 176A and (1R)-1,2,2-trimethylpropylamine were processed using the method described in Example 169A to afford the title compound. $^1$H NMR (300 MHz, DMSO-$d_6$) $\delta$ ppm 0.84 (s, 9 H) 0.99 (dd, *J*=7, 5 Hz,

3 H) 1.13 - 1.30 (m, 1H) 1.38 - 1.50 (m, 3 H) 1.54 - 1.68 (m, *J*=11 Hz, 1 H) 1.74 - 1.85 (m, 1 H) 2.06 (s, 3 H) 2.11 (s, 3 H) 3.19 - 3.27 (m, 1 H) 3.53 - 3.66 (m, 1 H) 3.71 - 3.88 (m, 3 H) 3.95 - 4.12 (m, 1 H) 6.37 (d, *J*=9 Hz, 1 H), MS (DCI/$NH_3$) m/z 354 $(M+H)^+$. Anal. Calculated for $C_{18}H_{31}N_3O_2S$: C, 61.15 H, 9.04 N, 11.89. Found C, 61.36 H, 9.08 N, 11.80.

Example 191

1-tert-Butyl-3-[(2Z)-5-methyl-3-(tetrahydropyran-2-ylmethyl)-1,3-thiazol-2(3H)-ylidene]urea

The product of Example 157A and *tert*-butyl amine were processed using the method described in Example 169A to afford the title compound. $^1H$ NMR (300 MHz, DMSO-$d_6$) $\delta$ ppm 1.11 - 1.22 (m, 1 H) 1.27 (s, 9 H) 1.42 - 1.47 (m, *J*=1 Hz, 3 H) 1.47 - 1.60 (m, 1 H) 1.78 (d, *J*=5 Hz, 1 H) 2.12 (s, 3H) 3.17 - 3.26 (m, 1 H) 3.55 - 3.66 (m, *J*=6 Hz, 1 H) 3.80-3.95 (m, 3 H) 6.34 (s, 1 H) 6.79 (s, I H), MS (DGI/$NH_3$) m/z 326 $(M+H)^+$. Anal. Calculated for $C_{16}H_{27}N_3O_2S$•$0.3H_2O$: C, 58.08 H, 8.41 N, 12.70. Found C, 58.44 H, 8.12 N, 12.41.

Example 192

1-(2,3-Dichlorophenyl)-3-[(2Z)-4,5-dimethyl-3-(tetrahydrofuran-2-ylmethyl)-1,3-thiazol-2(3H)-ylidene]urea

The product of Example 186A and 2,3-dichlorophenylamine were processed using the method described in Example 169A to afford the title compound. $^1H$ NMR (300 MHz, DMSO-$d_6$) $\delta$ ppm 1.50 - 1.67 (m, I H) 1.75 - 2.06 (m, 3 H) 2.15 (s, 3 H) 2.18 (s, 3 H) 3.57 - 3.69 (m, 1 H) 3.72 - 3.83 (m, 1 H) 3.83 - 3.96 (m, 1 H) 4.10 - 4.30 (m, 2 H) 7.30 (s, 1 H) 7.32 (d, *J*=1 Hz, 1 H) 7.97 - 8.03 (m, 1 H) 8.22 (s, I H), MS (DCI/$NH_3$) m/z 400, 401 $(M+H)^+$. Anal. Calculated for $C_{13}H_{13}Cl_3N_3O_2S$•0.3MeOH: C, 54.15 H, 8.23 N, 14.20. Found C, 54.47 H, 7.91N,13.99.

Example 193

1-(2,2-Dimethylpropyl)-3-[(2Z)-3-(2-methoxyethyl)-4-methyl-1,3-thiazol-2(3H)-ylidene]urea

The product of Example 5A and 2,2-dimethylpropylamine were processed using the method described in Example 169A to afford the title compound. $^1H$ NMR (300 MHz, DMSO-$d_6$) $\delta$ ppm 0.77 (t, *J*=7 Hz, 3 H) 1.21 (s, 6 H) 1.66 (q, *J*=7 Hz, 2 H) 2.21 (s, 3 H) 3.23 (s, 3 H) 3.57 (t, *J*=5 Hz, 2 H) 4.07 (t, *J*=5 Hz, 2 H) 6.21 (s, 1 H) 8.10 (d, *J*=9 Hz, 1 H),), MS (DCI/$NH_3$) m/z 286 $(M+H)^+$. Anal. Calculated for $C_{13}H_{23}N_3O_2S$•0.1MeOH: C, 54.70 H, 8.15 N, 14.26. Found C, 54.47 H, 7.91 N, 13.99.

Example 194

*N*-(2*Z*)-4,5-dimethyl-3-(tetrahydrofuran-2-ylmethyl)-1,3-thiazol-2(3*H*)-ylidene]-*N*'-[(1*R*)-1,2,2-trimethylpropyl]urea

The product of Example 186A and (1R)-1,2,2-trimethylpropylamine were processed using the method described in Example 169A to afford the title compound. $^1H$ NMR (300 MHz, DMSO-$d_6$) $\delta$ ppm 0.84 (s, 9 H) 0.98 (dd, *J*=7, 2 Hz, 3 H) 1.49 - 1.66 (m, 1 H) 1.74 - 1.99 (m, 3 H) 2.06 (s, 3 H) 2.11 (s, 3 H) 3.52 - 3.67 (m, 2 H) 3.72 - 3.88 (m, 2 H) 4.10 (dt, *J*=14, 4 Hz, 1 H) 4.16 - 4.27 (m, 1 H) 6.37 (dd, *J*=10,4 Hz, 1 H), m/z 339 $(M+H)^+$. Anal. Calculated for $C_{17}H_{29}N_3O_2S$: C, 60.14 H, 8.61 N, 12.38. Found C, 60.18 H, 8.88 N, 12.33.

Example 195

*N*-[(1*S*)-1,2-dimethylpropyl]-*N*'-[(2*Z*)-4,5-dimethyl-3-(tetrahydrofuran-2-ylmethyl)-1,3-thiazol-2(3*H*)-ylidene]urea

The product of Example 186A and (1S)-1,2,2-trimethylpropylamine were processed using the method described in Example 169A to afford the title compound. $^1H$ NMR (300 MHz, DMSO-$d_6$) $\delta$ ppm 0.83 (s, 9 H) 0.98 (dd, *J*=7, 2 Hz, 3 H) 1.52 - 1.66 (m, 1 H) 1.75 - 1.97 (m, 3 H) 2.06 (s, 3 H) 2.11 (s, 3 H) 3.51 - 3.66 (m, 2 H) 3.72 - 3.86 (m, 2 H) 4.10 (dt, *J*=14, 4 Hz, 1 H) 4.19 (s, 1 H) 6.37 (dd, *J*=10, 4 Hz, 1 H), m/z 339 $(M+H)^+$. Anal. Calculated for $C_{17}H_{29}N_3O_2S$: C, 60.14 H, 8.61 N, 12.38. Found C, 60.06 H, 8.95 N, 12.29.

Example 196

1-(1-Cyclopropylethyl)-3-[(2Z)-4,5-dimethyl-3-(tetrahydrofuran-2-ylmethyl)-1,3-thiazol-2(3H)-ylidene]urea

The product of Example 12A and 1-cyclopropylethylamine were processed using the method described in Example 169A to afford the title compound. $^1$H NMR (300 MHz, DMSO-$d_6$) $\delta$ ppm 0.05 - 0.15 (m, I H) 0.18 - 0.55 (m, 3 H) 0.78 - 0.92 (m, 1 H) 1.11 (dd, *J*=7, 2 Hz, 3 H) 1.51 - 1.64 (m, I H) 1.75 - 1.99 (m, 3 H) 2.07 (s, 3 H) 2.12 (s, 3 H) 3.06 - 3.22 (m, 1 H) 3.61 (dd, I H) 3.73 - 3.85 (m, 2 H) 4.02 - 4.14 (m, 1 H) 4.15 - 4.25 (m, 1 H) 6.57 - 6.70 (m, 1 H); m/z 324 $(M+H)^+$. Anal. Calculated for $C_{16}H_{25}N_3O_2S$: C, 59.41 H, 7.79 N, 12.99. Found C, 59.13 H, 7.78 N, 12.88.

Example 197

1-[(2Z)-5-(2,4-Difluorophenyl)-3-(2-methoxyethyl)-1,3-thiazol-2(3*H*)-ylidene]-3-(1,1-dimethylpropyl)urea

Example 197A

N-[5-chloro-3-(2-methoxyethyl)-1,3-thiazol-2(3H)-ylidene]acetamide

A flask was charged with 2-acetamido-5-chlorothiazole (Lancaster, 19.3 g, 110 mmol) in 200 mL of 2:1 THF/DMF. To the solution was added sodium hydride (60% dispersion in mineral oil, 5.44 g, 142 mmol). The mixture was stirred at room temperature for 15 min and then 2-bromoethyl methyl ether (18.3 g, 131 mmol) was added. The reaction mixture was warmed to 85 °C and stirred overnight. After cooling to room temperature, the mixture was diluted with ethyl acetate and washed with water. The organic extract was dried ($MgSO_4$), filtered, and concentrated. The residue was purified by flash chromatography on $SiO_2$ using a gradient of 0% to 100 % ethyl acetate: hexane to provide 10.3 g (42%) of the title compound as the more polar regioisomer: $^1$H NMR (300 MHz, $CDCl_3$) $\delta$ 2.28 (s, 3 H) 3.35 (s, 3 H) 3.65 - 3.71 (m, 2 H) 4.28 - 4.36 (m, 2 H) 7.00 (s, I H); MS (ESI$^+$) m/z 235 $(M+H)^+$.

Example 197B

N-[5-(2,4-difluorophenyl)-3-(2-methoxyethyl)-1,3-thiazol-2(3*H*)-ylidene]acetamide

A flask was charged with the product from Example 197A (10.2 g, 42.6 mmol), 2,6-difluorophenylboronic acid (8.08 g, 51.1 mmol), $Na_2CO_3$ (64.0 mL of a 2 M aqueous solution, 128 mmol) and $PdC)_2(PPh_3)_2$ (1.5 g, 2.13 mmol) in 100 mL of DME/$H_2O$/ethanol (7:3:2). The mixture was wanned to 85 °C and stirred overnight. After cooling to room temperature, the mixture was diluted with ethyl acetate and washed with water. The organic extract was dried ($MgSO_4$), filtered, and concentrated. The residue was purified by flash chromatography on $SiO_2$ using a gradient of 0% to 100 % ethyl acetate: hexane to provide 11.5 g (86 %) of the title compound: $^1$H NMR (300 MHz, $CDCl_3$) $\delta$ 2.17 (s, 3 H) 3.27 (s, 3 H) 3.71 (t, *J*=5.3 Hz, 2 H) 4.37 (t, *J*=5.4 Hz, 2 H) 7.17 - 7.24 (m, I H) 7.38 - 7.48 (m, 1 H) 7.64 - 7.74 (m, I H) 7.88 (s, 1 H); MS (ES I$^+$) m/z 313 $(M+H)^+$.

Example 197C

N-5-(2,4-difluoro-phenyl)-3-(2-methoxyethyl)-1,3-thiazol-2(3H)-ylideneamine

To a solution of the product from Example 197B (11.5 g, 36.8 mmol) in 100 mL of THF was added 25 mL of 5 N aqueous HCl. The mixture was wanned to 40 °C and stirred overnight. After cooling to room temperature, the solvent was removed under reduced pressure and the residue diluted with ethyl acetate. The mixture was neutralized to pH 7 with saturated aqueous $NaHCO_3$ and then washed with water. The organic extract was dried ($MgSO_4$), filtered and concentrated. The residue was purified by flash chromatography on $SiO_2$ using a gradient of 0% to 100 % ethyl acetate: hexane to provide 8.5 g (85 %) of the title compound: $^1$H NMR (300 MHz, DMSO-$d_6$) $\delta$ 3.27 (s, 3 H) 3.57 (t, *J*=5.3 Hz, 2 H) 3.86 (t, *J*=5.4 Hz, 2 H) 7.06 - 7.14 (m, Hz, 1 H) 7.25 (s, 1H) 7.29 (dd, *J*=9.2, 2.7 Hz, 2 H) 7.34 (dd, *J*=5.9, 3.2 Hz, 1 H) 7.94 (s, 1 H); MS (ES I$^+$) m/z 271 $(M+H)^+$.

Example 197D

1-[(2Z)-5-(2,4-Difluorophenyl)-3-(2-methoxyethyl)-1,3-thiazol-2-ylidene]-3-(1,1-dimethylpropyl)urea

A mixture of the product of Example 197C and 1,1-dimethylpropylamine were processed using the method

described in Example 169A to afford the title compound $^1H$ NMR (400 MHz, DMSO-$d_6$) δ ppm 0.78 (t, *J*=7.06 Hz, 3 H) 1.24 (s, 6 H) 1.68 (q, *J*=7.67 Hz, 2 H) 3.27 (s, 3 H) 3.66 (t, *J*=5.52 Hz, 2 H) 4.20 (t, *J*=5.22 Hz, 2 H) 6.49 (m, 1 H) 7.16 (td, *J*=7.98, 1.84 Hz, 1 H) 7.38 (m, 1 H) 7.58 (m, 1 H) 7.61 (s, 1 H); MS ($DCI/NH_3$) m/z 384 $(M+H)^+$.

Example 198

1-[(2Z)-5-(2,4-Difluorophenyl)-3-(2-methoxyethyl)-1,3-thiazol-2-ylidene]-3-(1-methylpropyl)urea

A mixture of the product of Example 197C and *sec*-butylamine were processed using the method described in Example 169A to afford the title compound. $^1H$ NMR (500 MHz), DMSO-$d_6$) δ ppm 0.84 (t, *J*=7.32 Hz, 3 H) 1.05 (d, *J*=6.41 Hz, 3 H) 1.42 (m, 2 H) 3.27 (s, 3 H) 3.59 (m, 1 H) 3.66 (t, *J*=5.49 Hz, 2 H) 4.21 (t, *J*=5.80 Hz, 2 H) 6.93 (d, *J*=8.54 Hz, 1 H) 7.18 (td, *J*=8.24, 2.44 Hz, 1 H) 7.39 (m, 1 H) 7.57 (td, *J*=8.85, 6.41 Hz, 1 H) 7.62 (brs, I H); MS ($DCI/NH_3$) m/z 370 $(M+H)^+$.

Example 199

1-Cyclopentyl-3-[(2Z)-5-(2,4-difluorophenyl)-3-(2-methoxyethyl)-1,3-thiazol-2(3H)-ylidene]urea

A mixture of the product of Example 197C and cyclopentylamine were processed using the method described in Example 169A to afford the title compound. $^1H$ NMR (500 MHz, DMSO-$d_6$) δ ppm 1.47 (m, 4 H) 1.65 (m, 2 H) 1.80 (m, 2 H) 3.27 (s, 3 H) 3.66 (t, *J*=5.49 Hz, 2 H) 3.95 (m, 1 H) 4.20 (t, *J*=5.19 Hz, 2 H) 7. 10 (d, *J*=7.63 Hz, 1 H) 7.18 (td, *J*=8.24, 2.44 Hz, 1 H) 7.39 (m, 1 H) 7.57 (m, 1 H) 7.63 (s, 1 H); MS ($DCI/NH_3$) m/z 382 $(M+H)^+$.

Example 200

1-[(2Z)-5-(2,4-Difluorophenyl)-3-(2-methoxyethyl-1,3-thiazol-2(3H)-ylidene]-3-(4-methylcyclohexyl)urea

A mixture of the product of Example 197C and 4-methylcyclohexylamine were processed using the method described in Example 169A to afford the title compound. $^1H$ NMR (400 MHz, DMSO-$d_6$) δ ppm 0.90 (m, 3 H) 0.98 (m, 2 H) 1.49 (m, 8 H) 3.27 (s, 3 H) 3.66 (m, 2 H) 4.21 (m, 2 H) 6.93 (1, *J*=8.90 Hz, 1 H) 7.17 (td, *J*=7.06, 2.45 Hz, 1 H) 7.38 (m, 1 H) 7.56 (m, 1 H) 7.62 (m, 1 H); MS ($DCI/NH_3$) m/z 410 $(M+H)^+$.

Example 202

N-[(2Z)-5-tert-butyl-3-(2-methoxyethyl)-1,3-thiazol-2(3H)-ylidene]-2-ethoxybenzamide

Example 202A

5-tert-butylthiazol-2-amine

To a flask equipped with a Dean-Stark trap was added 3,3-dimethylbutanal (Aldrich, 5.0 g, 50 mmol), pyrrolidine (Aldrich, 4.4 mL), 52 mmol) and p-toluenesulfonic acid monohydrate (10 mg) in cyclohexane (70 mL). The mixture was heated to reflux for 3 hours, the water was removed and the organic phase was concentrated under reduced pressure. The residue was dissolved in methanol (20 mL) and cooled to 0 °C. Sulfur (Aldrich, 1.6 g, 50 mmol) and a solution of cyanamide (Aldrich, 2.1 g, 50 mmol) in methanol (5 mL) were added. The reaction mixture was allowed to warm to ambient temperature, stirred for 12 hours, and was concentrated under reduced pressure. The residue was purified by column chromatography ($SiO_2$, 2% methanol in $CH_2Cl_2$) to afford the title compound. MS ($ESI^+$) m/z 157 $(M+H)^+$.

Example 202B

5-tert-butyl-3-(2-methoxyethyl)thiazol-2(3H)-imine hydrobromide

A mixture of Example 202A and commercially available 2-bromoethyl methyl ether (Aldrich) were processed according to the method described in Example 12A to afford the title compound. MS ($ESI^+$) m/z 215 $(M+H)^+$.

Example 202C

N-[(2Z)-5-tert-butyl-3-(2-methoxyethyl)-1,3-thiazol-2(3H)-ylidene]-2-ethoxybenzamide

Commercially available 2-ethoxybenzoic acid (Aldrich) and Example 202B were processed using the method described in Example 58 to afford the title compound. $^1H$ NMR (300 MHz, dimethylsulfoxide-$d_6$) δ ppm 1.28 - 1.36 (m, 12 H), 3.26 (s, 3 H), 3.71 (t, *J*=5.4 Hz, 2 H), 4.06 (q, *J*=6.9 Hz, 2 H), 4.31 (t, *J*=5.4 Hz, 2 H), 6.95 (td, *J*=7.4, 0.8 Hz, 1 H), 7.05 (dd, *J*=8.5, 0.7 Hz, 1 H), 7.21 (s, 1 H), 7.32 - 7.42 (m, 1 H), 7.67 (dd, *J*=7.6, 1.9 Hz, 1 H)); MS (ESI$^+$) m/z 363 $(M+H)^+$.

Example 203

2-ethoxy-N-[(2Z)-5-methyl-3-(tetrahydro-2H-pyran-4-ylmethyl)-1,3-thiazol-2(3H)-ylidene]benzamide

Example 203A

(tetrahydro-2H-pyran-4-yl)methyl 4-methylbenzenesulfonate

To a solution of tetrahydro-2H-pyran-4-ylmethanol (Combi-Blocks, 2.0 g, 17.2 mmol) in 10 mL of of $CH_2Cl_2$ and 10 mL of of pyridine was added *p*- toluenesulfonyl chloride (3.5 g, 18.1 mmol) in portions over 15 minutes. The mixture stirred at ambient temperature for 16 hours and was quenched with 10 mL of saturated, aqueous $NaHCO_3$. The layers were separated and the aqueous layer was extracted with three 10 mL portions of $CH_2Cl_2$. The combined organic extracts were dried over anhydrous $Na_2SO_4$, filtered and concentrated under reduced pressure to afford the title compound. $^1H$ NMR (300 MHz, dimethylsulfoxide-$d_6$) δ ppm 1.05 - 1.25 (m, 2 H), 1.40 - 1.53 (m, 2 H), 1.73 - 1.94 (m, 1 H), 2.43 (s, 3 H), 3.14 - 3.28 (m, 2 H), 3.71-3.84 (m, 2 H), 3.88 (d, *J*=6.4 Hz, 2 H), 7.48 (d, *J*=8.5 Hz, 2 H), 7.79 (d, *J*=8.5 Hz, 2 H); MS (DCI/$NH_3$) *mlz* 288 $(M+NH_4)^+$.

Example 203B

5-methyl-3-((tetrahydro-2H-pyran-4-yl)methyl)thiazol-2(3H)-imine

A mixture of Example 203A (1.9 g, 7.0 mmol), 2-amino-5-methylthiazole (0.80 g, 7.0 mmol) and tetrabutylammonium iodide (1.3 g, 3.5 mmol) in 3 mL of N,N- dimethylformamide was warmed to 85 °C and was allowed to stir for 24 hours. The mixture was diluted with 10 mL of $CH_2Cl_2$, washed with 10% aqueous $NaHCO_3$, dried over anhydrous $Na_2SO_4$, filtered and concentrated under reduced pressure. Purification via column chromatography ($SiO_2$, 10% methanol in ethyl acetate then 9:1:0.1 $CH_2Cl_2$ : methanol : $NH_4OH$) afforded the title compound. MS (DCI/$NH_3$) m/z 213 $(M+H)^+$.

Example 203C

2-ethoxy-N-[(2Z)-5-methyl-3-(tetrahydro-2H-pyran-4-ylmethyl)-1,3-thiazol-2(3H)-ylidene]benzamide

To a solution of Example 203B (0.11 g, 0.52 mmol) in 10 mL) of tetrahydrofuran and l mL of N,N- dimethylformamide at ambient temperature was added triethylamine (0.22 mL, 1.6 mmol) followed by 2-ethoxybenzoyl chloride (0.11 g, 0.57 mmol). This mixture was warmed to 50 °C, stirred for 3 hours, was quenched with 10 mL of saturated aqueous $NH_4Cl$, and diluted with 10 mL of $CH_2Cl_2$. The layers were separated and the aqueous layer was extracted with three 5 mL portions of $CH_2Cl_2$. The combined organic extracts were dried over anhydrous $Na_2SO_4$, filtered and concentrated under reduced pressure. Purification via flash column chromatography ($SiO_2$, 50% hexanes in ethyl acetate) afforded the title compound. $^1H$ NMR (300 MHz, $CDCl_3$) δ ppm 1.46 (t, *J*=7.0 Hz, 3 H), 1.52 - 1.61 (m, 1 H), 1.57 (s, 3 H), 2.14 - 2.26 (m, 1 H), 2.29 (d, *J*=1.4 Hz, 3 H), 3.36 (dt, *J*=11.7, 2.4 Hz, 2 H), 3.98 (ddd, *J*=11.4, 4.1, 1.5 Hz, 2 H), 4.06 (d, *J*=7.5 Hz, 2 H), 4.17 (q, *J*=6.8 Hz, 2 H), 6.59 - 6.62 (m, 1 H), 6.93 - 7.01 (m, 2 H), 7.36 (ddd, *J*=8.3, 7.5, 1.9 Hz, 1 H), 7.97 (dd, *J*=8.0, 1.9 Hz, 1 H); MS (DCI/$NH_3$) m/z 361 $(M+H)^+$. Anal. Calculated for $C_{19}H_{24}N_2O_3S$: C, 63.31; H, 6.71; N, 7.77. Found: C, 63.27; H, 6.57; N, 7.48.

Example 204

2,4-dimethoxy-N-[(2Z)-5-methyl-3-(tetrahydrofuran-2-ylmethyl)-1,3-thiazol-2(3H)-ylidene]benzamide

Example 204A

5-methyl-3-((tetrahydrofuran-2-yl)methyl)thiazol-2(3H)-imine

A mixture of 2-amino-5-methylthiazole (1 g, 8.7 mmol) and 2-(bromomethyl)tetrahydrofuran (1.1 mL, 10 mmol) was wanned to 85 °C and was allowed to stir for 24 hours. The mixture was cooled to ambient temperature and purified via flash column chromatography ($SiO_2$, 10% methanol in ethyl acetate then 9:1:0.1 $CH_2Cl_2$ : methanol : $NH_4OH$) to afford the title compound. MS ($DCI/NH_3$) m/z 199 $(M+H)^+$.

Example 204B

2,4-dimethoxybenzoyl chloride

A solution of 2,4-dimethoxybenzoic acid (0.25 g, 1.4 mmol) in 5 mL of $SOCl_2$ was warmed to reflux and was allowed to stir for 2 hours. The mixture was cooled to ambient temperature and concentrated under reduced pressure. The crude material was diluted with 5 mL of toluene and concentrated under reduced pressure. This dilution with toluene and concentration was repeated two additional times to give the crude title compound which was used without additional purification or characterization.

Example 204C

2,4-dimethoxy-N-[(2Z)-5-methyl-3-(tetrahydrofuran-2-ylmethyl)-1,3-thiazol-2(3H)-ylidene]benzamide

To a solution of Example 204A (0.18 g, 0.91 mmol) in 10 mL of tetrahydrofuran at ambient temperature was added triethylamine (0.38 mL, 2.7 mmol) followed by Example 204B (1.4 mmol) in 3 mL of tetrahydrofuran via cannula. This mixture was wanned to 50°C, stirred for 3 hours, then quenched with 10 mL of saturated aqueous $NH_4Cl$, and diluted with 10 mL of $CH_2Cl_2$. The layers were separated and the aqueous phase was extracted three 5 mL portions of $CH_2Cl_2$. The combined organic extracts were dried over anhydrous $Na_2SO_4$, filtered and concentrated under reduced pressure. Purification via column chromatography ($SiO_2$, 50% hexanes in ethyl acetate) afforded the title compound. $^1H$ NMR (300 MHz, $CD_3OD$) $\delta$ ppm 1.65 - 1.79 (m, 1 H), 1.84 - 1.96 (m, 2 H), 1.99 - 2.15 (m, 1 H), 2.31 (d, *J*=1.4 Hz, 3 H), 3.71 - 3.81 (m, I H), 3.84 - 3.93 (m, 1 H), 3.85 (s, 3 H), 3.86 (s, 3 H), 4.19 - 4.42 (m, 3 H), 6.51 - 6.62 (m, 2 H), 7.05 - 7.10 (m, 1 H), 8.03 (d, *J*=8.8 Hz, 1 H); MS ($DCI/NH_3$) m/z 363 $(M+H)^+$. Anal. Calculated for $C_{18}H_{22}N_2O_4S$: C, 59.65; H, 6.12; N, 7.73. Found: C, 59.47; H, 6.01; N, 7.62.

Example 205

5-chloro-2-methoxy-N-[(2Z)-4-methyl-3-(tetrahydro-2H-pyran-2-ylmethyl)-1,3-thiazol-2(3H)-ylidene]benzamide

Example 205A

4-methyl-3-((tetrahydro-2H-pyran-2-yl)methyl)thiazol-2(3H)-imine

A mixture of 2-amino-4-methylthiazole (1.0 g, 8.8 mmol) and 2-(bromomethyl)tetrahydropyran (1.1 mL, 8.8 mmol) was wanned to 85 °C and was allowed to stir for 24 hours. The mixture was cooled to ambient temperature and the crude material was purified via column chromatography ($SiO_2$, 10% methanol in ethyl acetate then 9:1:0.1 $CH_2Cl_2$ : methanol : $NH_4OH$) to afford the title compound. MS ($DCI/NH_3$) m/z 213 $(M+H)^+$.

Example 205B

5-chloro-2-methoxybenzoyl chloride

A solution of 2-methoxy-5-chlorobenzoic acid (0.37 g, 2.0 mmol) in 10 mL of $SOCl_2$ was wanned to reflux and was allowed to stir for 2 hours. The mixture was cooled to ambient temperature and concentrated under reduced pressure. The crude material was diluted with 5 mL of toluene and concentrated under reduced pressure. This dilution with toluene

and concentration was repeated two additional times to afford the title compound. $^1$H NMR (300 MHz), dimethylsulfoxide-$d_6$) δ ppm 3.82 (s, 3 H), 7.16 (d, *J*=8.8 Hz, 1 H), 7.49 - 7.59 (m, I H), 7.61 (d, *J*=2.7 Hz, 1 H).

Example 205C

5-chloro-2-methoxy-N-[(2Z)-4-methyl-3-(tetrahdro-2H-pyran-2-ylmethyl)-1,3-thiazol-2(3H)-ylidene]benzamide

To a solution of Example 205A (0.21 g, 1.0 mmol) in 10 mL of tetrahydrofuran at ambient temperature was added triethylamine (0.41 mL, 3.0 mmol) followed by Example 205B (2.0 mmol) in 5 mL of tetrahydrofuran via cannula. This mixture was warmed to 50°C, stirred for 2 hours, then quenched with 10 mL of saturated aqueous $NH_4Cl$, and diluted with 10 mL of $CH_2Cl_2$. The layers were separated and the aqueous phase was extracted with three 5 mL portions of $CH_2Cl_2$. The combined organic extracts were dried over anhydrous $Na_2SO_4$, filtered and concentrated under reduced pressure. Purification via column chromatography ($SiO_2$, 50% hexanes in ethyl acetate) afforded the title compound. $^1$H NMR (300 MHz, $CD_3OD$) δ ppm 1.31 - 1.48 (m, 1 H), 1.47 - 1.67 (m, 3 H), 1.70 - 1.82 (m, 1 H), 1.84 - 1.98 (m, 1 H), 2.40 (d, *J*=1.0 Hz, 3 H), 3.26 - 3.40 (m, 1 H), 3.82 - 3.94 (m, 2 H), 3.87 (s, 3 H), 4.00 - 4.11 (m, 1 H), 4.42 (dd, *J*= 13.9, 2.7 Hz, 1 H), 6.55 (d, *J*=1.0 Hz, 1 H), 7.07 (d, *J*=8.8 Hz, 1 H), 7.40 (dd, *J*=8.8, 2.7 Hz, 1 H), 7.87 (d, *J*=2.7 Hz, 1 H); MS (DCI/$NH_3$) m/z 381 $(M+H)^+$. Anal. Calculated for $C_{18}H_{21}ClN_2O_3S$: C, 56.76; H, 5.56; N, 7.35. Found: C, 56.58; H, 5.43; N, 7.19.

Example 206

5-chloro-2-methoxy-N-[(2Z)-5-methyl-3-(tetrahydro-2H-pyran-4-ylmethyl)-1,3-thiazol-2(3H)-ylidene]benzamide

To Example 203B (0.11 g, 0.52 mmol) and triethylamine (0.22 mL, 1.6 mmol) in 10 mL of tetrahydrofuran and I mL ofN,N- dimethylformamide was added Example 205B (0.68 mmol) in 2 mL of tetrahydrofuran. This mixture was warmed to 50 °C, stirred for 2 hours, then quenched with 10 mL of saturated aqueous $NH_4Cl$, and diluted with 10 mL of $CH_2Cl_2$. The layers were separated and the aqueous phase was extracted with three 5 mL portions of $CH_2Cl_2$. The combined organic extracts were dried over anhydrous $Na_2SO_4$, filtered and concentrated under reduced pressure. Purification via flash column chromatography ($SiO_2$, 50% hexanes in ethyl acetate) resulted in the title compound. $^1$H NMR (300 MHz, $CD_3OD$) δ ppm 1.34 - 1.60 (m, 4 H), 2.18 - 2.32 (m, 1 H), 2.34 (d, *J*=1.4 Hz, 3 H), 3.38 (dt, *J*=11.6, 2.5 Hz, 2 H), 3.86 (s, 3 H), 3.94 (ddd, *J*=11.6, 4.2, 1.9 Hz, 2 H), 4.14 (d, *J*=7.5 Hz, 2 H), 7.07 (d, *J*=8.8 Hz, 1 H), 7.12 (q, *J*=1.4 Hz, 1 H), 7.40 (dd, *J*=8.8, 2.7 Hz, 1 H), 7.83 (d, *J*=2.7 Hz, 1 H); MS (DCI/$NH_3$) m/z 381 $(M+H)^+$. Anal. Calculated for $C_{18}H_{21}ClN_2O_3S$: C, 56.76; H, 5.56; N, 7.35. Found: C, 56.48; H, 5.46; N, 7.23.

Example 207

5-chloro-2-methoxy-N-[(2Z)-3-(tetrahydro-2H-pyran-2-ylmethyl)-1,3-thiazol-2(3H)-ylidene]benzamide

Example 207A

3-((tetrahydro-2H-pyran-2-yl)methyl)thiazol-2(3H)-imine

A mixture of2-aminothiazole (1.0 g, 10 mmol) and 2-(bromomethyl)tetrahydro-2H-pyran (1.3 mL, 10 mmol) was warmed to 85 °C and was allowed to stir for 24 hours. The mixture was cooled to ambient temperature and the crude material was purified via column chromatography ($SiO_2$, 10% methanol in ethyl acetate then 9:1:0.1 $CH_2Cl_2$ : methanol : $NH_4OH$) to afford the title compound. MS (DCI/$NH_3$) m/z 199 $(M+H)^+$.

Example 207B

5-chloro-2-methoxy-N-[(2Z)-3-(tetrahydro-2H-pyran-2-ylmethyl)-1,3-thiazol-2(3H)-ylidene]benzamide

To a solution of Example 207A (0.19 g, 0.96 mmol) in 10 mL of tetrahydrofuran at ambient temperature was added triethylamine (0.40 mL, 2.9 mmol) followed by Example 205B (2.0 mmol) in 5 mL of tetrahydrofuran via cannula. This mixture was warmed to 50°C, stirred for 2 hours, then quenched with 10 mL of saturated aqueous $NH_4Cl$, and diluted with 10 mL of $CH_2Cl_2$. The layers were separated and the aqueous phase was extracted with three 5 mL portions of $CH_2Cl_2$. The combined organic extracts were dried over anhydrous $Na_2SO_4$, filtered and concentrated under reduced pressure. Purification via column chromatography ($SiO_2$, 50% hexanes in ethyl acetate) afforded the title compound. $^1$H NMR (300 MHz, $CD_3OD$) δ ppm 1.20 - 1.41 (m, 1 H), 1.46 - 1.63 (m, 3 H), 1.67 - 1.77 (m, 1 H), 1.82 - 1.96 (m, I H), 3.35

- 3.45 (m, 1 H), 3.75 - 3.85 (m, 1 H), 3.86 (s, 3 H), 3.89 - 4.00 (m, 1 H), 4.19 - 4.27 (m, I H), 4.38 - 4.46 (m, 1 H), 6.91 (d, *J*=4.7 Hz, I H), 7.08 (d, *J*=8.8 Hz, 1 H), 7.38 (d, *J*=4.7 Hz, I H), 7.41 (dd, *J*=8.8, 3.1 Hz, 1 H), 7.85 (d, .*J*=3.1 Hz, 1 H); MS (DCI/$NH_3$) m/z 367 $(M+H)^+$. Anal. Calculated for $C_{17}H_{19}ClN_2O_3S$: C, 55.66; H, 5.22; N, 7.64. Found: C, 55.72; H, 5.08; N, 7.55.

Example 208

5-chloro-2-methoxy-N-[(2Z)-5-methyl-3-[(2R)-tetrahydrofuran-2-ylmethyl]-1,3-thiazol-2(3H)-ylidene]benzamide

Example 208A

(R)-(tetrahydrofuran-2-yl)methyl -4-methylbenzenesulfonate

To a solution of (R)-tetrahydrofurfuryl alcohol (Lancaster, 1.0 g, 9.8 mmol) in 5 mL of $CH_2Cl_2$ and 5 mL of pyridine was added *p*- toluenesulfonyl chloride (2.8 g, 14.7 mmol) in portions over 15 minutes. The mixture was stirred at ambient temperature for 3 hours and was quenched with 10 mL of saturated, aqueous $NaHCO_3$. The layers were separated and the aqueous layer was extracted with three 5 mL portions of $CH_2Cl_2$. The combined organic extracts were dried over anhydrous $Na_2SO_4$, filtered and concentrated under reduced pressure to afford the title compound. MS (DCI/$NH_3$) m/z 257 $(M+H)^+$, 274 $(M+NH_4)^+$.

Example 208B

(R)-5-methyl-3-((tetrahydrofuran-2-yl)methyl)thiazol-2(3H)-imine

A mixture of Example 208A (1.5 g, 5.9 mmol), 2-amino-5-methylthiazole (0.68 g, 5.9 mmol) and tetrabutylammonium iodide (1.1 g, 3.0 mmol) in 3 mL of N,N- dimethylformamide was warmed to 85°C and was allowed to stir for 48 hours. The mixture was diluted with 10 mL of $CH_2Cl_2$ and the solution was quenched with 10 mL of saturated, aqueous $NaHCO_3$. The layers were separated and the aqueous layer was extracted twice with 10 mL portions of $CH_2Cl_2$. The combined organic extracts were dried over anhydrous $Na_2SO_4$, filtered. and concentrated under reduced pressure. Purification via column chromatography ($SiO_2$, 10% methanol in ethyl acetate then 9:1:0.1 $CH_2Cl_2$ : methanol : $NH_4OH$) afforded the title compound. MS (DCI/$NH_3$) m/z 199 $(M+H)^+$.

Example 208C

5-chloro-2-methoxybenzoyl chloride

A solution of 2-methoxy-5-chlorobenzoic acid (0.22 g, 1.2 mmol) in 10 mL of $SOCl_2$ was warmed to reflux and was allowed to stir for 2 hours. The mixture was cooled to ambient temperature and concentrated under reduced pressure. The crude material was diluted with 5 mL of toluene and concentrated under reduced pressure. This dilution with toluene and concentration was repeated two additional times to afford the title compound. $^1H$ NMR (300 MHz, dimethylsulfoxide-$d_6$) $\delta$ ppm 3.82 (s, 3 H), 7.16 (d, *J*=8.8 Hz, 1 H), 7.49 - 7.59 (m, 1 H), 7.61 (d, *J*=2.7 Hz, 1 H).

Example 208D

5-chloro-2-methoxy-N-[(2Z)-5-methyl-3-[(2R)-tetrahydrofuran-2-ylmethyl]l-1,3-thiazol-2(3H)-ylidene]benzamide

To a solution of Example 208B (0.23 g, 1.2 mmol) in 10 mL of tetrahydrofuran at ambient temperature was added triethylamine (0.49 mL, 3.5 mmol) followed by Example 208C (1.2 mmol) in 5 mL of tetrahydrofuran via cannula. This mixture was warmed to 50 °C and was allowed to stir for 3 hours and was quenched with 10 mL of $NH_4Cl$ and diluted with 10 mL of $CH_2Cl_2$. The layers were separated and the aqueous layer was extracted with three 5 mL portions of $CH_2Cl_2$. The combined organic extracts were dried over anhydrous $Na_2SO_4$, filtered and concentrated under reduced pressure. Purification via flash column chromatography ($SiO_2$, 1 : 1 : I hexanes : ethyl acetate : $CH_2Cl_2$) afforded the title compound. $^1H$ NMR (300 MHz, $CD_3OD$) $\delta$ ppm 1.64 - 1.79 (m, 1 H), 1.84 - 1.96 (m, 2 H), 2.00 - 2.14 (m, 1 H), 2.34 (d, *J*=1.4 Hz, 3 H), 3.69 - 3.81 (m, 1 H), 3.84 - 3.93 (m, 1 H), 3.85 (s, 3 H), 4.20 - 4.43 (m, 3 H), 7.07 (d, *J*=8.8 Hz, 1 H), 7.14 (q, *J*=1.1 Hz, 1 H), 7.39 (dd,*J*=9.0, 2.9 Hz, 1 H), 7.81 (d, *J*=2.7 Hz, 1 H); MS (DCI/$NH_3$) m/z 367 $(M+H)^+$. Anal. Calculated for $C_{17}H_{19}ClN_2O_3S$: C, 55.66; H, 5.22; N, 7.64. Found: C, 55.42; H, 5.08; N, 7.58.

Example 209

5-chloro-2-methoxy-N-[(2Z)-5-methyl-3-[(2S)-tetrahydrofuran-2-ylmethyl]-1,3-thiazol-2(3H)-ylidene]benzamide

Example 209A

(S)-(tetrahydrofuran-2-yl)methyl 4-methylbenzenesulfonate

To a solution of (S)-tetrahydrofurfuryl alcohol (Codexis, 1.6 g, 15.2 mmol) in 5 mL of $CH_2Cl_2$ and 5 mL of pyridine was added *p*- toluenesulfonyl chloride (4.3 g, 22.8 mmol) in portions over 15 minutes. The mixture stirred at ambient temperature for 3 hours and was quenched with 10 mL of saturated, aqueous $NaHCO_3$. The layers were separated and the aqueous layer was extracted with three 5 mL portions of $CH_2Cl_2$. The combined organic extracts were dried over anhydrous $Na_2SO_4$, filtered and concentrated under reduced pressure to afford the title compound. MS (DCI/$NH_3$) m/z 257 $(M+H)^+$, 274 $(M+NH_4)^+$.

Example 209B

(S)-5-methyl-3-((tetrahydrofuran-2-yl)methyl)thiazol-2(3H)-imine

A mixture of Example 209A (1.6 g, 6.1 mmol), 2-amino-5-methylthiazole (0.7 g, 6.1 mmol) and tetrabutylammonium iodide (2.3 g, 6.1 mmol) in 5 mL of N,N- dimethylformamide was wanned to 85°C and was allowed to stir for 18 hours. The mixture was diluted with 10 mL of $CH_2Cl_2$, washed with 10% aqueous $NaHCO_3$, dried over anhydrous $Na_2SO_4$, filtered and concentrated under reduced pressure. Purification via column chromatography ($SiO_2$, 10% methanol in ethyl acetate then 9:1:0.1 $CH_2Cl_2$ : methanol : $NH_4OH$) afforded the title compound. MS (DCI/$NH_3$) m/z 199 $(M+H)^+$.

Example 209C

5-chloro-2-methoxy-N-[(2Z)-5-methyl-3-[(2S)-tetrahydrofuran-2-ylmethyl]-1,3-thiazol-2(3H)-ylidene]benzamide

Example 209B (0.32 g, 1.6 mnol), triethylamine (0.67 mL, 4.8 mmol) and Example 205B (1.9 mmol) in 20 mL of tetrahydrofuran were processed as in Example 208D to afford the title compound. $^1H$ NMR (300 MHz, $CD_3OD$) $\delta$ ppm 1.64 - 1.79 (m, 1 H), 1.84 - 1.96 (m, 2 H), 2.00 - 2.13 (m, 1 H), 2.34 (d, *J*=1.4 Hz, 3 H), 3.71 - 3.81 (m, 1 H), 3.84 - 3.93 (m, 1 H), 3.85 (s, 3 H), 4.20 - 4.43 (m, 3 H), 7.07 (d, *J*=8.8 Hz, 1 H), 7.14 (q, *J*=1.4 Hz, 1 H), 7.39 (dd, *J*=9.0, 2.9 Hz, I H), 7.81 (d, *J*=2.7 Hz, 1 H); MS (DCI/$NH_3$) m/z 367 $(M+H)^+$. Anal. Calculated for $C_{17}H_{19}ClN_2O_3S$: C, 55.66; H, 5.22; N, 7.64. Found: C, 55.48; H, 4.96; N, 7.52.

Example 210

2,2,3,3-tetrafluoro-1-methyl-N-[(2Z)-5-methyl-3-(tetrahydrofuran-2-ylmethyl)-1,3-thiazol-2(3H)-ylidene]cyclobutanecarboxamide

Example 204A (0.20 g, 1.0 mmol), triethylamine (0.42 mL, 3.0 mmol) and 2,2,3,3-tetrafluoro-1-(methyl)cyclobutanecarbonyl chloride (ABCR, 0.27 g, 1.3 mmol) in 15 mL of tetrahydrofuran were processed as in Example 208D to afford the title compound. $^1H$ NMR (300 MHz, $CD_3OD$) $\delta$ ppm 1.53 (s, 3 H), 1.62 - 1.73 (m, I H), 1.81 - 1.93 (m, 2 H), 1.95 - 2.10 (m, 1 H), 2.27 - 2.44 (m, 1 H), 2.32 (d, *J*=1.4 Hz, 3 H), 3.33 - 3.43 (m, 1 H), 3.69 - 3.79 (m, 1 H), 3.80 - 3.90 (m, 1 H), 4.20 - 4.36 (m, 3 H), 7.11 (dd, *J*= 1.4, 0.7 Hz, 1 H); MS (DCI/$NH_3$) m/z 367 $(M+H)^+$. Anal. Calculated for $C_{15}H_{18}F_4N_2O_2S$: C, 49.17; H, 4.95; N, 7.65. Found: C, 49.27; H, 4.88; N, 7.58.

Example 211

5-chloro-2-methoxy-N-[(2Z)-5-methyl-3-(oxetan-2-ylmethyl)-1,3-thiazol-2(3H)-ylidene]benzamide

Example 211A

oxetan-2-ylmethyl 4-methylbenzenesulfonate

To a solution of 2-hydroxymethyloxetane (TCI-US, 2.0 g, 23 mmol) in 10 mL of $CH_2Cl_2$ and 10 mL of pyridine was added *p*- toluenesulfonyl chloride (6.5 g, 34 mmol) in portions over 15 minutes. The mixture was stirred at ambient

temperature for 3 hours and was quenched with 10 mL of saturated, aqueous $NaHCO_3$. The layers were separated and the aqueous layer was extracted with three 5 mL portions of $CH_2Cl_2$. The combined organic extracts were dried over anhydrous $Na_2SO_4$, filtered and concentrated under reduced pressure. Purification via column chromatography ($SiO_2$, 70% hexanes in ethyl acetate) afforded the title compound. MS (DCI/$NH_3$) m/z 243 $(M+H)^+$, 260 $(M+NH_4)^+$.

Example 211B

5-methyl-3-(oxetan-2-ylmethyl)thiazol-2(3H)-imine

A mixture of Example 211A (1.1 g, 4.6 mmol), 2-amino-5-methylthiazole (0.53 g, 4.6 mmol) and tetrabutylammonium iodide (0.85 g, 2.3 mmol) in 5 mL ofN,N- dimethylformamide was wanned to 85 °C and was allowed to stir for 18 hours. The mixture was diluted with 10 mL of $CH_2Cl_2$, washed with 10% aqueous $NaHCO_3$, dried over anhydrous $Na_2SO_4$, filtered and concentrated under reduced pressure. Purification via column chromatography ($SiO_2$, 10% methanol in ethyl acetate then 9:1:0.1 $CH_2Cl_2$ : methanol : $NH_4OH$) afforded the title compound. MS (DCI/$NH_3$) m/z 185 $(M+H)^+$.

Example 211C

5-chloro-2-methoxy-N-[(2Z)-5-methyl-3-oxetan-2-ylmethyl)-1,3-thiazol-2(3H)-ylidene]benzamide

Example 211B (0.26 g, 1.4 mmol), triethylamine (0.59 mL, 4.2 mmol) and Example 205B (1.7 mmol) in 15 mL of tetrahydrofuran were processed as in Example 208D to afford the title compound. $^1H$ NMR (300 MHz, $CD_3OD$) δ ppm 2.35 (d, *J=1.4* Hz, 3 H), 2.43 - 2.59 (m, 1 H), 2.70 - 2.85 (m, I H), 3.85 (s, 3 H), 4.37 - 4.51 (m, 2 H), 4.57 - 4.71 (m, 2 H), 5.15 - 5.25 (m, 1 H), 7.06 (d, *J*=9.2 Hz, 1 H), 7.18 (q, *J*=1.1 Hz, 1 H), 7.39 (dd, *J*=8.8, 2.7 Hz, 1 H), 7.79 (d, *J*=2.7 Hz, 1 H); MS (DCI/$NH_3$) m/z 353 $(M+H)^+$. Anal. Calculated for $C_{16}H_{17}ClN_2O_3S$: C, 54.46; H, 4.86; N, 7.94. Found: C, 54.41; H, 4.88; N, 7.80.

Example 212

5-chloro-N-[(2Z)-3-(1,3-dioxolan-2-ylmethyl)-5-methyl-1,3-thiazol-2(3H)-ylidene]-2-methoxybenzamide

Example 212A

3-((1,3-dioxolan-2-yl)methyl)-5-methylthiazol-2(3H)-imine

A mixture of 2-amino-5-methylthiazole (1 g, 8.7 mmol) and 2-bromomethyl-1,3-dioxolane (0.98 mL, 9.6 mmol) was warmed to 85 °C and was allowed to stir for 18 hours. The mixture was cooled to ambient temperature and purified via column chromatography ($SiO_2$, 10% methanol in ethyl acetate then 9:1:0.1 $CH_2Cl_2$: methanol : $NH_4OH$) to afford the title compound. MS (DCI/$NH_3$) m/z 201 $(M+H)^+$.

Example 212B

5-chloro-N-[(2Z)-3-(1,3-dioxolan-2-ylmethyl)-5-methyl-1,3-thiazol-2(3H)-ylidene]-2-methoxybenzamide

Example 212A (0.25 g, 1.3 mmol), triethylamine (0.52 mL, 3.8 mmol) and Example 205B (1.5 mmol) in 15 mL of tetrahydrofuran were processed as in Example 208D to afford the title compound. $^1H$ NMR (300 MHz, $CD_3OD$) δ ppm 2.33 (d, *J*=1.4 Hz, 3 H), 3.86 (s, 3 H), 3.87 - 3.99 (m, 4 H), 4.41 (d, *J*=4.1 Hz, 2 H), 5.27 (t, *J*=4.1 Hz, 1 H), 7.07 (d, *J*=8.8 Hz, 1 H), 7.12 (q,*J*=1.4 Hz, 1 H), 7.40 (dd, *J*=8.8, 2.7 Hz, 1 H), 7.87 (d, *J*=2.7 Hz, I H); MS (DCI/$NH_3$) m/z 369 $(M+H)^+$. Anal. Calculated for $C_{16}H1_7C)N_20_4S$: C, 52.1 U; H, 4.65; N, 7.60. Found: C, 52.15; H, 4.42; N, 7.44.

Example 213

5-chloro-N-[(2Z)-3-[2-(1,3-dioxolan-2-yl)ethyl]-5-methyl-1,3-thiazol-2(3H)-ylidene]-2-methoxybenzamide

Example 213A

3-(2-(1,3-dioxolan-2-yl)ethyl)-5-methylthiazol-2(3H)-imine

A mixture of 2-amino-5-methylthiazol (1.0 g, 8.7 mmol) and 2-(2-bromoethyl)-1,3-dioxolane (1.1 mL), 8.7 mmol)

was warmed to 85 °C and was allowed to stir for 18 hours. The mixture was cooled to ambient temperature and purified via column chromatography ($SiO_2$, 10% methanol in ethyl acetate then 9: 1:0.1 $CH_2Cl_2$ : methanol : $NH_4OH$) to afford the title compound. MS (DCI/$NH_3$) m/z 215 $(M+H)^+$.

Example 213B

5-chloro-N-[(2Z)-3-[2-(1,3-dioxolan-2-yl)ethyl)-5-methyl-1,3-thiazol-2(3H)-ylidene]-2-methoxvbenzamide

Example 213A (0.25 g, 1.2 mmol), triethylamine (0.49 mL, 3.5 mmol) and Example 205B (1.3 mmol) in 10 mL of tetrahydrofuran were processed as in Example 208D to afford the title compound. $^1H$ NMR (300 MHz, $CD_3OD$) $\delta$ ppm 2.18 - 2.25 (m, 2 H), 2.33 (d, *J*=1.0 Hz, 3 H), 3.82 - 3.87 (m, 2 H), 3.86 (s, 3 H), 3.93 - 4.01 (m, 2 H), 4.36 (dd, *J*=7.1 Hz, 2 H), 4.93 (t, *J*=4.4 Hz, 1 H), 7.07 (d, *J*=8.8 Hz, 1 H), 7.10 (q, *J*=1.4 Hz, I H), 7.40 (dd, *J*=8.8, 2.7 Hz, 1 H), 7.96 (d, *J*=3.1 Hz, 1 H); MS (DCI/$NH_3$) m/z 383 $(M+H)^+$. Anal. Calculated for $C_{17}H_{19}ClN_2O_4S$: C, 53.33; H, 5.00; N, 7.32. Found: C, 53.02; H, 4.52; N, 7.22.

Example 214

N-[(2Z)-3-(1,3-dioxolan-2-ylmethyl)-5-methyl-1,3-thiazol-2(3H)-ylidene]-2-ethoxybenzamide

Example 212A (0.20 g, 1.0 mmol), triethylamine (0.42 mL, 3.0 mmol) and 2-ethoxybenzoyl chloride (0.17 g, 1.1 mmol) in 10 mL of tetrahydrofuran were processed as in Example 208D to afford the title compound. $^1H$ NMR (300 MHz, $CD_3OD$) $\delta$ ppm 1.41 (t, *J*=7.0 Hz, 3 H), 2.33 (d, *J*=1.4 Hz, 3 H), 3.82 - 4.01 (m, 4 H), 4.12 (q, *J*=6.9 Hz, 2 H), 4.40 (d, *J*=4.4 Hz, 2 H), 5.27 (t, *J*=4.2 Hz, 1 H), 6.96 (dt, *J*=7.5, 0.8 Hz, 1 H), 7.05 (d, *J*=8.5 Hz, 1 H), 7.09 (q, *J*=1.4 Hz, 1 H), 7.39 (ddd, *J*=8.7, 6.9, 1.7 Hz, 1H), 7.83 (dd, *J*=7.6, 1.9 Hz, 1 H); MS (DCI/$NH_3$) m/z 349 $(M+H)^+$. Anal. Calculated for $C_{17}H_{20}N_2O_4S$: C, 58.60; H, 5.79; N, 8.04. Found: C, 58.22; H, 5.32; N, 7.93.

Example 215

5-bromo-2-ethoxy-N-[(2Z)-5-methyl-3-[(2R)-tetrahydrofuran-2-ylmethyl]-1,3-thiazol-2(3H)-ylidene]benzamide

Example 215A

5-bromo-2-ethoxybenzoic acid

To a solution of 2-ethoxybenzoic acid (3.3 g, 20.0 mmol) in 75 mL of acetonitrile at 0 °C was added *N*-bromosuccinimide (3.7 g, 21 mmol) in 15 mL of acetonitrile. The reaction mixture was warmed to ambient temperature and the mixture was allowed to stir for 48 hours. The mixture was quenched with 20 mL of $H_2O$ and the layers were separated. The aqueous layer was extracted with three 15 mL portions of $CH_2Cl_2$ and the combined organic extracts were dried over anhydrous $Na_2SO_4$, filtered and concentrated under reduced pressure. Purification via column chromatography ($SiO_2$, 10% hexanes in ethyl acetate) afforded the title compound. MS (DCI/$NH_3$) m/z 262, 264 $(M+NH_4)^+$.

Example 215B

5-bromo-2-ethoxybenzoyl chloride

A solution of Example 215A (0.21 g, 0.86 mmol) in 5 mL of $SOCl_2$ was warmed to reflux and was allowed to stir for 2 hours. The mixture was cooled to ambient temperature and concentrated under reduced pressure. The crude material was diluted with 5 mL of toluene and concentrated under reduced pressure. This dilution with toluene and concentration was repeated two additional times to give the crude title compound which was used without additional purification or characterization.

Example 215C

5-bromo-2-ethoxy-N-[(2Z)-5-methyl-3-[(2R)-tetrahydrofuran-2-ylmethyl]-1,3-thiazol-2(3H)-ylidene]benzamide

Example 208B (0.17 g, 0.86 mmol), triethylamine (0.36 mL, 2.6 mnol) and Example 215B (0.86 mmol) in 10 mL of tetrahydrofuran were processed as in Example 208D to afford the title compound. $^1H$ NMR (300 MHz, $CD_3OD$) $\delta$ ppm 1.39 (t, *J*=7.0 Hz, 3 H), 1.63 - 1.78 (m, 1 H), 1.84 - 1.96 (m, 2 H), 2.00 - 2.15 (m, 1 H), 2.34 (d, *J*=1.4 Hz, 3 H), 3.69

- 3.81 (m, 1 H), 3.84 - 3.95 (m, 1 H), 4.10 (q, *J*=7.0 Hz, 2 H), 4.19 - 4.43 (m, 3 H), 6.99 (d, *J*=8.8 Hz, 1 H), 7.14 (q, *J*=1.2 Hz, 1 H), 7.49 (dd, *J*=8.8, 2.7 Hz, 1 H), 7.87 (d, *J*=2.7 Hz, 1 H); MS (DCI/$NH_3$) m/z 425, 427 $(M+H)^+$. Anal. Calculated for $C_{18}H_{21}BrN_2O_3S$: C, 50.83; H, 4.98; N, 6.59. Found: C, 50.89; H, 4.87; N, 6.51.

Example 216

5-chloro-2-ethoxy-N-[(2Z)-5-methyl-3-[(2R)-tetrahydrofuran-2-ylmethyl]-1,3-thiazol-2(3H)-ylidene]benzamide

Example 216A

5-chloro-2-ethoxybenzoic acid

To a solution of 2-ethoxybenzoic acid (4.4 g, 26.6 mmol) in 80 mL of acetonitrile at 0 °C was added *N*-chlorosuccinimide (3.7 g, 28 mmol) in 20 mL of acetonitrile dropwise over 30 minutes. The reaction mixture was wanned to ambient temperature and the mixture was allowed to stir for 70 hours. The mixture was quenched with 20 mL of $H_2O$ and the layers were separated. The aqueous layer was extracted with three 15 mL portions of $CH_2Cl_2$ and the combined organic extracts were dried over anhydrous $Na_2SO_4$, filtered and concentrated under reduced pressure. Purification via recrystallization with ether and hexanes afforded the title compound. MS (DCI/$NH_3$) m/z 201 1 $(M+H)^1$, 218 $(M+NH_4)^+$.

Example 216B

5-chloro-2-ethoxybenzoyl chloride

A solution of Example 216A (0.25 g, 1.0 mmol) in 5 mL of $SOCl_2$ was wanned to reflux and was allowed to stir for 2 hours. The mixture was cooled to ambient temperature and concentrated under reduced pressure. The crude material was diluted with 5 mL of toluene and concentrated under reduced pressure. This dilution with toluene and concentration was repeated two additional times to give the crude title compound which was used without additional purification or characterization.

Example 216C

5-chloro-2-ethoxy-N-[(2Z)-5-methyl-3-[(2R)-tetrahydrofuran-2-ylmethyl]-1,3-thiazol-2(3H)-ylidene]benzamide

Example 208B (0.20 g, 1.0 mmol), triethylamine (0.42 mL, 3.0 mmol) and Example 216B (1.0 mmol) in 10 mL of tetrahydrofuran were processed as in Example 208D to afford the title compound. $^1H$ NMR (300 MHz, $CD_3OD$) $\delta$ ppm 1.39 (t, *J*=7.0 Hz, 3 H), 1.62 - 1.77 (m, 1 H), 1.83 - 1.97 (m, 2 H), 1.99 - 2.14 (m, 1 H), 2.34 (d, *J*=1.4 Hz, 3 H), 3.70 - 3.80 (m, 1 H), 3.84 - 3.93 (m, 1 H), 4.10 (q, *J*=6.9 Hz, 2 H), 4.20 - 4.44 (m, 3 H), 7.04 (d, *J=8.8* Hz, 1 H), 7.14 (q, *J*=1.4 Hz, 1 H), 7.35 (dd, *J*=8.8, 2.7 Hz, 1 H), 7.73 (d, *J*=2.7 Hz, 1 H); MS (DCI/$NH_3$) m/z 381 $(M+H)^+$. Anal. Calculated for $C_{18}H_{21}ClN_2O_3S$: C, 56.76; H, 5.56; N, 7.35. Found: C, 56.36; H, 5.28; N, 7.25.

Example 217

4-chloro-2-methoxy-N-[(2Z)-5-methyl-3-(tetrahydrofuran-2-ylmethyl)-1,3-thiazol-2(3H)-ylidene]benzamide

Example 217A

4-chloro-2-methoxybenzoyl chloride

A solution of the 4-chloro-2-methoxybenzoic acid (0.24 g, 1.3 mmol) in 7 mL of $SOCl_2$, was warmed to reflux and was allowed to stir for 2 hours. The mixture was cooled to ambient temperature and concentrated under reduced pressure. The crude material was diluted with 5 mL of toluene and concentrated under reduced pressure. This dilution with toluene and concentration was repeated two additional times to give the crude title compound which was used without additional purification or characterization.

Example 217B

4-chloro-2-methoxy-N-[(2Z)-5-methyl-3-(tetrahydrofuran-2-ylmethyl)-1,3-thiazol-2(3H)-ylidene]benzamide

Example 204A (0.20 g, 1.0 mmol), triethylamine (0.42 mL, 3.0 mmol) and Example 217A (1.3 mmol) in 15 mL of tetrahydrofuran and 1 mL of N,N- dimethylfonnamide were processed as in Example 208D to afford the title compound. $^1$H NMR (300 MHz, $CDCl_3$) δ ppm 1.61 - 1.74 (m, 1 H), 1.77 - 1.98 (m, 2 H), 1.99 - 2.14 (m, I H), 2.30 (d, *J*=1.4 Hz, 3 H), 3.72 - 3.82 (m, 1 H), 3.82 - 3.90 (m, 1 H), 3.91 (s, 3 H), 4.11 - 4.20 (m, 1 H), 4.27 (ddd, *J*=13.7, 6.8, 2.9 Hz, 1 H), 4.41 - 4.51 (m, 1 H), 6.87 - 6.91 (m, 1 H), 6.93 - 6.99 (m, 2 H), 7.97 (d, *J*=8.8 Hz, 1 H); MS (DCI/$NH_3$) m/z 367 $(M+H)^+$. Anal. Calculated for $C_{17}H_{19}ClN_2O_3S$: C, 55.66; H, 5.22; N, 7.64. Found: C, 55.40; H, 5.31; N, 7.48.

Example 218

5-chloro-2-methoxy-N-[(2Z)-1-(2-methoxyethyl)-1,4,6,7-tetrahydro-2H-pyrano[4,3-d][1,3]thiazol-2-ylidene]benzamide

Example 218A

6.7-dihydro-4*H*-pyrano[4,3-*d*]thiazol-2-amine

To a solution of tetrahydro-4H-pyran-4-one (Aldrich) (7.22 g, 72.11 mmol) in cyclohexane (70 mL) were added pyrrolidine (6.26 mL, 7.57 mmol) and *p*-toluenesulfonic acid monohydrate (13.72 mg, 0.07 mmol). The reaction mixture was refluxed for 3 hours with a Dean-Stark trap, cooled and concentrated. The residue was dissolved in methanol (20 mL) and then sulfur (2.31 g, 72.11 mmol) was added followed by a solution of cyanamide (3.03 g, 72.11 mmol) in methanol (5 mL) at 0 °C. The reaction mixture was stirred at room temperature overnight, filtered, concentrated and purified by column chromatography using an Analogic® Intelliflash280™ ($SiO_2$, 0-5% methanol in dichloromethane) to afford the title compound. MS ($ESI^+$) m/z 157 $(M+H)^+$.

Example 218B

1-(2-methoxyethyl)-6,7-dihydro-1*H*-pyrano[4,3-d]thiazol-2(4*H*)-imine hydrobromide

A mixture of product of Example 218A (1.0 g, 6.4 mmol) and 2-bromoethyl methyl ether (3.0 mL, 32.0 mmol) was processed according to the method of Example 2A to afford the title compound: MS (LC/MS) m/z 213 $(M+H)^+$.

Example 218C

5-chloro-2-methoxy-N-[(2Z)-1-(2-methoxyethyl)-1,4,6,7-tetrahydro-2H-pyrano[4,3-d][1,3]thiazol-2-ylidene]benzamide

To a solution of Example 218B (150.0 mg, 0.51 mmol) in tetrahydrofuran (10 mL) was added N-(3-dimethylaminopropyl)-N-ethylcarbodiimide hydrochloride (97.0 mg, 0.51 mmol), 1-hydroxybenzotriazole (69.0 mg, 0.51 mmol), triethylamine (178.0 μL, 1.28 mmol), and 5-chloro-2-methoxybenzoic acid (Aldrich) (95.0 mg, 0.51 mmol). The mixture was stirred overnight at 80 °C, and then diluted with ethyl acetate, washed with I M aqueous $NaHCO_3$, dried ($Na_2SO_4$), filtered and concentrated. Purification by preparative HPLC on a Waters Symmetry C8 column (40 mm X 100 mm, 7 μm particle size) using a gradient of 10 % to 100 % acetonitrile: ammonium acetate (10 mM) over 15 minutes at a flow rate of 70 mL/minutes afforded the title compound. $^1$H NMR (300 MHz, dimethylsulfoxide-$d_6$) δ 2.75 (t, *J*=5.4 Hz, 2 H), 3.25 (s, 3 H), 3.69 (t, *J*=5.3 Hz, 2 H), 3.80 (s, 3 H), 3.95 (t, *J*=5.4 Hz, 1 H), 4.27 (t, J=5.3 Hz, 2 H), 4.58 (s, 2 H), 7.12 (d, *J*=8.8 Hz, 1 H), 7.46 (dd, *J*=9.0, 2.9 Hz, 1 H), 7.69 (d, *J*=2.7 Hz, 1H); MS ($ESI^+$) m/z 383 $(M+H)^+$. Anal. Calculated for $C_{17}H_{19}ClN_2O_4S$: C, 53.33; H, 5.00; N, 7.32. Found: C, 53.21; H, 4.80; N, 7.27.

Example 219

5-bromo-2-methoxy-N-[(2Z)-5-methyl-3-[(2R)-tetrahydrofuran-2-ylmethyl]-1,3-thiazol-2(3H)-ylidene]benzamide

Example 219A

5-bromo-2-methoxybenzoic acid

To a solution of 2-methoxybenzoic acid (6 g, 39.4 mmol) in 80 mL of acetonitrile was added *N*-bromosuccinimide

(7.4 g, 41.4 mmol) in 20 mL of acetonitrile. The reaction mixture was wanned to ambient temperature and the mixture was allowed to stir for 16 hours. Additional *N*-bromosuccinimide (14.8 g, 82.8 mmol) was added and the reaction mixture stirred for an additional 48 hours. The mixture was quenched with 25 mL of $H_2O$ and the layers were separated. The aqueous layer was extracted with three 15 mL of portions of $CH_2Cl_2$ and the combined organic extracts were dried over anhydrous $Na_2SO_4$, filtered and concentrated under reduced pressure. Purification via column chromatography ($SiO_2$, 50% hexanes in ethyl acetate) afforded the title compound. MS ($DCI/NH_3$) m/z 248, 250 $(M+NH_4)^+$.

Example 219B

5-bromo-2-methoxybenzoyl chloride

A solution of Example 219A (0.28 g, 1.4 mmol) in 5 mL of $SOCl_2$ was warmed to reflux and was allowed to stir for 2 hours. The mixture was cooled to ambient temperature and concentrated under reduced pressure. The crude material was diluted with 5 mL of toluene and concentrated under reduced pressure. This dilution with toluene and concentration was repeated two additional times to give the crude title compound which was used without additional purification or characterization.

Example 219C

5-bromo-2-methoxy-N-[(2Z)-5-methyl-3-[(2R)-tetrahydrofuran-2-ylmethyl]-1,3-thiazol-2(3H)-ylidene]benzamide

Example 208B (0.25 g, 1.3 mmol), triethylamine (0.53 mL, 3.8 mmol) and Example 219B (1.4 mmol) in 15 mL of tetrahydrofuran were processed as in Example 208D to afford the title compound. $^1$H NMR (300 MHz, $CD_3OD$) $\delta$ ppm 1.65 - 1.80 (m, 1 H), 1.85 - 1.96 (m, 2 H), 2.01 - 2.14 (m, I H), 2.34 (d, *J*=1.4 Hz, 3 H), 3.71 - 3.80 (m, 1 H), 3.85 (s, 3 H), 3.85 - 3.93 (m, I H), 4.21 - 4.41 (m, 3 H), 7.02 (d, *J*=9.2 Hz, 1 H), 7.14 (q, *J*=1.1 Hz, 1 H), 7.53 (dd, *J*=8.8, 2.7 Hz, I H), 7.95 (d, *J*=2.4 Hz, I H); MS ($DCI/NH_3$) m/z 411, 413 $(M+H)^+$. Anal. Calculated for $C_{17}H_{19}BrN_2O_3S$: C, 49.64; H, 4.66; N, 6.81. Found: C, 49.48; H, 4.53; N, 6.72.

Example 220

5-chloro-2-methoxy-N[(2Z)-5-methyl-3-(2-tetrahydro-2H-pyran-4-ylethyl)-1,3-thiazol-2(3H)-ylidene]benzamide

Example 220A

2-(tetrahydro-2H-pyran-4-yl)ethyl 4-methylbenzenezulfonate

To a solution of 2-(tetrahydropyran-4-yl)-ethanol (1.5 g, 11.5 mmol) in 10 mL of $CH_2Cl_2$ and 7 mL of pyridine was added *p*-toluenesulfonyl chloride (2.4 g, 12.7 mmol) portion wise over 15 minutes. The mixture stirred at ambient temperature for 3 hours and was quenched with 10 mL of saturated, aqueous $NaHCO_3$. The layers were separated and the aqueous layer was extracted with three 5 mL of portions of $CH_2Cl_2$. The combined organic extracts were dried over anhydrous $Na_2SO_4$, filtered and concentrated under reduced pressure. Purification via column chromatography ($SiO_2$, 70% hexanes in ethyl acetate) afforded the title compound. MS ($DCI/NH_3$) m/z 302 $(M+NH_4)^+$.

Example 220B

5-methyl-3-(2-(tetrahydro-2H-pyran-4-yl)ethyl)thiazol-2(3H)-imine

A mixture of Example 220A (1.9 g, 6.7 mmol), 2-amino-5-methylthiazole (0.77 g, 6.7 mmol) and tetrabutylammonium iodide (1.1 g, 3.3 mmol) in 2 mL of N,N- dimethylfonnamide was wanned to 85 °C and was allowed to stir for 18 hours. The mixture was diluted with 10 mL of $CH_2Cl_2$, washed with 10 mL of 10% aqueous $NaHCO_3$, dried over anhydrous $Na_2SO_4$, filtered and concentrated under reduced pressure. Purification via column chromatography ($SiO_2$, 10% methanol in ethyl acetate then 9:1:0.1 $CH_2Cl_2$ : methanol : $NH_4OH$) afforded the title compound. MS ($DCI/NH_3$) m/z 227 $(M+H)^+$.

Example 220C

5-chloro-2-methoxy-N-[(2Z)-5-methyl-3-(2-tetrahydro-2H-pyran-4-ylethyl)-1,3-thiazol-2(3H)-ylidene]benzamide

Example 220B (0.20 g, 0.9 mmol), triethylamine (0.37 mL, 0.26 mmol) and Example 205B (0.9 mmol) in 10 mL of tetrahydrofuran were processed as in Example 208D to afford the title compound. $^1$H NMR (300 MHz, $CD_3OD$) δ ppm 1.26 - 1.44 (m, 2 H), 1.47 - 1.65 (m, 1 H), 1.71 - 1.89 (m, 4 H), 2.34 (d, *J*=1.4 Hz, 3 H), 3.32 - 3.41 (m, 2 H), 3.86 (s, 3 H), 3.86 - 3.94 (m, 2 H), 4.26 - 4.35 (m, 2 H), 7.07 (d, *J*=8.8 Hz, I H), 7.14 (q, *J*=1.4 Hz, I H), 7.40 (dd, *J*=9.0, 2.9 Hz, 1 H), 7.89 (d, *J*=3.1 Hz, 1 H); MS (DCI/$NH_3$) m/z 395 $(M+H)^+$. Anal. Calculated for $C_{19}H_{13}ClN_2O_3S$: C, 57.79; H, 5.87; N, 7.09. Found: C, 57.54; H, 5.67; N, 7.07.

Example 221

5-chloro-N-[(2Z)-5-ethyl-3-(tetrahydrofuran-2-ylmethyl)-1,3-thiazol-2(3H)-ylidene]-2-methoxybenzamide

Example 221 A

5-ethyl-3-((tetrahydrofuran-2-yl)methyl)thiazol-2(3H)-imine

A mixture of 5-ethylthiazol-2-amine and 2-(bromomethyl)tetrahydrofuran were processed using the method described in Example 2A to afford the title compound. MS (ESI) m/z 213 $(M+H)^+$.

Example 221 B

5-chloro-N-[(2Z)-5-ethyl-3-(tetrahydrofuran-2-ylmethyl)-1,3-thiazol-2(3H)-ylidene]-2-methoxybenzamide

Example 221A and 5-chlom-2-methoxybenzoic acid were processed using the method described in Example 2B to afford the title compound. $^1$H NMR (500 MHz, dimethylsulfoxide-$d_6$) δ ppm 1.21 (t, *J*=7.63 Hz, 3 H) 1.59 - 1.68 (m, I H) 1.77 - 1.85 (m, 2 H) 1.89 - 1.97 (m, I H) 2.67 (dd, *J*=1 5.56, 7.63 Hz, 2 H) 3.65 (dd, *J*=14.95, 7.02 Hz, 1 H) 3.75 - 3.82 (m, 1 H) 3.78 (s, 3 H) 4.13 - 4.24 (m, 2 H) 4.24 - 4.30 (m, I H) 7.1 (d, *J*=8.85 Hz, I H) 7.26 (t, *J*=1.22 Hz, 1 H) 7.44 (dd, *J*=8.85, 2.75 Hz, I H) 7.64 (d, *J*=2.75 Hz, I H); MS (ESI) m/z 381$(M+H)^+$.

Example 222

5-chloro-2-methoxy-N-[(2Z)-5-propyl-3-(tetrahydrofuran-2- ylmethyl)-1,3-thiazol-2(3H)-ylidene]benzamide

Example 222A

5-propyl-3-((tetrahydrofuran-2-yl)methyl)thiazol-2(3H)-imine

A mixture of 5-propylthiazol-2-amine and 2-(bromomethyl)tetrahydrofuran were processed using the method described in Example 2A to afford the title compound. MS (ESI) m/z 227 $(M+H)^+$.

Example 222B

5-chloro-2-methoxy-N-[(2Z)-5-propyl-3-(tetrahydrofuran-2-ylmethyl)-1,3-thiazol-2(3H)-ylidene]benzamide

Example 222A and 5-chloro-2-methoxybenzoic acid were processed using the method described in Example 2B to afford the title compound. $^1$H NMR (500 MHz, dimethylsulfoxide-$d_6$) δ ppm 0.93 (t, *J*=7.32 Hz, 3 H) 1.56 - 1.67 (m, 3 H) 1.77 - 1.85 (m, 2 H) 1.93 (dt, *J*=19.22, 7.02 Hz, I H) 2.62 (t, *J*=7.02 Hz, 2 H) 3.65 (dd, *J*= 14.95, 6.71 Hz, 1 H) 3.74 - 3.80 (m, 1 H) 3.77 - 3.79 (m, 3 H) 4.15 - 4.24 (m, 2 H) 4.24 - 4.30 (m, 1 H) 7.10 (d, *J*=8.85 Hz, I H) 7.25 - 7.28 (m, I H) 7.44 (dd, *J*=8.85, 2.75 Hz, I H) 7.64 (d, *J*=2.75 Hz, I H); MS (ESI) m/z 395$(M+H)^+$.

Example 223

5-chloro-N-[(2Z)-5-chloro-3-(tetrahydrofuran-2-ylmethyl)-1,3-thiazol-2(3H)-ylidene]-2-methoxybenzamide

Example 223A

5-chloro-N-(5-chlorothiazol-2-yl)-2-methoxybenzamide

A mixture of 5-chlorothiazol-2-amine hydrochloride (513 mg, 3 mmol), 5-chloro-2-methoxybenzoic acid (670 mg, 3.6mmol), 1-ethyl-3-[3-(dimethylamino)propyl]-carbodiimide hydrochloride (1.15 g, 6 mmol), 1-hydroxybenzotriazole hydrate (810 mg, 6 mmol) and 4-(dimethylamino)pyridine (73 mg, 0.6 mmol) in pyridine was stirred at room temperature for 2 hours. The volatiles were removed under vacuum, and the resulting mixture was diluted with water and extracted with ethyl acetate. The organic extract was dried ($Na_2SO_4$), filtered and concentrated. The residue was washed with a small amount of ethyl acetate, and filtered to afford the title compound. MS (ESI) m/z 303 $(M+H)^+$.

Example 223B

5-chloro-N-[(2Z)-5-chloro-3-(tetrahdrofuran-2-ylmethyl)-1,3-thiazol-2(3H)-ylidene]-2-methoxybenzamide

Example 223A (250 mg, 0.83 mmol) in tetrahydrofuran/N,N- dimethylformamide (1:2) (9 mL) was treated with NaH (60%) (40 mg, 1.0 mmol) for 10 minutes then 2-(bromomethyl)tetrahydrofuran (164 mg, 1.0 mmol) was added. The mixture was heated at 150 °C for 2 hours. After cooling to ambient temperature, the mixture was diluted with water and extracted with ethyl acetate. The organic extract was dried ($Na_2SO_4$), filtered and concentrated. Purification by reverse phase HPLC afforded the title compound. $^1H$ NMR (400 MHz, dimethylsulfoxide-$d_6$) $\delta$ ppm 1.60 - 1.7 (m, 1 H) 1.79 - 1.89 (m, 2 H) 1.92 - 2.01 (m, 1 H) 3.66 (dd, *J*=15.04, 7.06 Hz, I H) 3.76 - 3.83 (m, 1 H) 3.79 - 3.81 (m, 3 H) 4.15 - 4.23 (m, I H) 4.24 - 4.33 (m, 2 H) 7.14 (d, *J*=8.90 Hz, I H) 7.49 (dd, *J*=8.90, 2.76 Hz, 1 H) 7.74 (d, *J*=3.07 Hz, 1 H) 7.77 (s, I H). MS (ESI) m/z 387 $(M+H)^+$.

Example 224

4,5-dichloro-2-methoxy-N-[(2Z)-5-methyl-3-[(2R)-tetrahydrofuran-2-ylmethyl]-1,3-thiazol-2(3H)-ylidene]benzamide

Example 224A

4,5-dichloro-2-methoxybenzoic acid

To a solution of 4-chloro-2-methoxybenzoic acid (5 g, 26.8 mmol) in 200 mL of acetonitrile was added N-chlorosuccininide (17.9 g, 134 mmol). The mixture was allowed to stir for 72 hours at ambient temperature and was quenched with 50 mL of $H_2O$. The layers were separated and the aqueous layer was extracted with three 25 mL portions of $CH_2Cl_2$. The combined organic extracts were dried over anhydrous $Na_2SO_4$, filtered and concentrated under reduced pressure. Purification via column chromatography ($SiO_2$, 25% hexanes in ethyl acetate) afforded the title compound. MS ($DCI/NH_3$) m/z 238 $(M+NH_4)^+$.

Example 224B

4,5-dichloro-2-methoxybenzoyl chloride

A solution of Example 224A (0.18 g, 0.81 mmol) in 5 mL of $SOCl_2$ was warmed to reflux and was allowed to stir for 2 hours. The mixture was cooled to ambient temperature and concentrated under reduced pressure. The crude material was diluted with 5 mL of toluene and concentrated under reduced pressure. This dilution with toluene and concentration was repeated two additional times to afford the title compound which was used without additional purification or characterization.

Example 224C

4,5-dichloro-2-methoxy-N-[(2Z)-5-methyl-3-[(2R)-tetrahydrofuran-2-ylmethyl]-1,3-thiazol-2(3H) ylidene]benzamide

Example 208B (0.16 g, 0.81 mmol), triethylamine (0.34 mL, 2.4 mmol) and Example 224B (0.81 mmol) in 10

mL of tetrahydrofuran were processed as in Example 208D to afford the title compound. $^1H$ NMR (300 MHz, $CD_3OD$) δ ppm 1.64 - 1.79 (m, 1 H), 1.83 - 1.96 (m, 2 H), 2.03 - 2.15(m, 1 H), 2.34(d, *J*=1.4Hz,3H),3.70-3.80(m, 1 H),3.83-3.93 (m, 1 H), 3.87 (s, 3 H), 4.20 - 4.44 (m, 3 H), 7.15 (q, *J*=1.1 Hz, 1 H), 7.26 (s, I H), 7.99 (s, I H); MS (DCI/$NH_3$) m/z 401 $(M+H)^+$. Anal. Calculated for $C_{17}H_{18}Cl_2N_2O_3S$: C, 50.88; H, 4.52; N, 6.98. Found: C, 50.63; H, 4.41; N, 6.83.

Example 225

5-chloro-2-methoxy-N-[(2Z)-5-methyl-3-(tetrahydro-2H-pyran-3-ylmethyl)-1,3-thiazol-2(3H)-ylidene]benzamide

Example 225A

(tetrahydro-2H-pyran-3-yl)methyl 4-methylbenzenesulfonate

To a solution of (tetrahydropyran-3-yl)-methanol (Matrix, 1.67 g, 14.4 mmol) in 15 mL of $CH_2Cl_2$ and 15 mL of pyridine was added *p*- toluenesulfonyl chloride (2.9 g, 15.1 mmol) in portions over 10 minutes. The mixture stirred at ambient temperature for 18 hours and was quenched with 10 mL of saturated, aqueous $NaHCO_3$. The layers were separated and the aqueous phase was extracted three 5 mL of portions of $CH_2Cl_2$. The combined organic extracts were dried over anhydrous $Na_2SO_4$, filtered and concentrated under reduced pressure. Purification via column chromatography ($SiO_2$, 70% hexanes in ethyl acetate) afforded the title compound. MS (DCI/$NH_3$ m/z 288 $(M+NH_4)^+$.

Example 225B

5-methyl-3-((tetrahydro-2H-pyran-3-yl)methyl)thiazol-2(3H)-imine

A mixture of Example 225A (1.0 g, 3.7 mmol), 2-amino-5-methylthiazole (0.42 g, 3.7 mmol) and tetrabutylammonium iodide (0.68 g, 1.85 mmol) in 1 mL of N,N-dimethylformamide was warmed to 85 °C and was allowed to stir for 16 hours. The mixture was diluted with 10 mL of $CH_2Cl_2$, washed with 10 mL of 10% aqueous $NaHCO_3$, dried over anhydrous $Na_2SO_4$, filtered and concentrated under reduced pressure. Purification via column chromatography ($SiO_2$, 10% methanol in ethyl acetate then 9:1:0.1 $CH_2Cl_2$ : methanol : $NH_4OH$) afforded the title compound. MS (DCI/$NH_3$) m/z 213 $(M+H)^+$.

Example 225C

5-chloro-2-methoxy-N-[(2Z)-5-methyl-3-(tetrahydro-2H-pyran-3-ylmethyl)-1,3-thiazol-2(3H)-ylidene]benzamide

Example 225B (0.19 g, 0.89 mmol), triethylamine (0.29 mL, 2.1 mmol) and Example 205B (0.63 mmol) in 10 mL of tetrahydrofuran were processed as in Example 208D to afford the title compound. $^1H$ NMR (300 MHz, $CD_3OD$) δ ppm 1.33 - 1.49 (m, 1 H), 1.50 - 1.67 (m, 1 H), 1.69 - 1.86 (m, 2 H), 2.20 - 2.32 (m, 1 H), 2.34 (d, *J*=1.4 Hz, 3 H), 3.32 - 3.38 (m, 1 H), 3.50 (ddd, *J*=11.5, 9.5,3.1 Hz, 1 H), 3.72-3.82(m, 2 H), 3.86(s, 3 H), 4.17(d, *J*=7.5Hz, 2 H), 7.07 (d, *J* =8.8Hz,1 H), 7.1 (q, *J*=1.4 Hz, 1 H), 7.40 (dd, *J*=8.8, 2.7 Hz, 1 H), 7.85 (d, *J*=2.7 Hz, 1 H); MS (DCI/$NH_3$) m/z 381 $(M+H)^+$. Anal. Calculated for $C_{18}H_{21}ClN_2O_3S$: C, 56.76; H, 5.56; N, 7.35. Found: C, 56.84; H, 5.32; N, 7.29.

Example 226

2-chloro-N-[(2Z)-4,5-dimethyl-3-(tetrahydrofuran-2-ylmethyl)-1,3-thiazol-2(3H)-ylidene]nicotinamide

A mixture of Example 186A (0.15 g), 2-chloronicotinic acid (99 mg), 1-hydroxybenzotriazole hydrate (80 mg), 1-ethyl-3-[3-(dimethylamino)propyl]-carbodiimide hydrochloride (120 mg) and triethylamine (0.15 mL) in N,N- dimethylformamide was stirred overnight at room temperature, poured into water and extracted with ether (3x). The combined organic extracts were dried ($Na_2SO_4$), filtered and the solvent evaporated. The crude material was purified by gradient flash chromatography over silica gel eluting with ethyl acetate:hexane (1:4 to 1:1) to afford the title compound. $^1H$ NMR (300 MHz, dimethylsulfoxide-$d_6$) δ ppm 1.65 (m, 1H), 1.85 (m, 2H), 2.0 (m, 1H), 2.25 (s, 3H), 2.28 (s, 3H), 3.62 (dd, 1H), 3.77 (dd, 1H), 4.08 (dd, 1H), 4.28 (m, 1H), 4.38 (dd, 1H), 7.50 (dd, 1H), 8.24 (dd, 1H), 8.45 (dd, 1H); MS (ESI+) m/z 352 $(M+H)^+$,

Example 227

5-chloro-N-[(2Z)-4,5-dimethyl-3-[(3-methyl-4,5-dihydroisoxazol-5-yl)methyl]-1,3-thiazol-2(3H)-ylidene]-2-methoxybenzamide

Example 227A

3-allyl-4,5-dimethylthiazol-2(3H)-imine hydrobromide

A mixture of 4,5-dimethyl-1,3-thiazol-2-amine (1g) and allylbromide (0.95 g) in toluene (5 mL) was heated to 85 °C for 12 hours, cooled, diluted with ether, filtered and the solvent was evaporated to afford crude product that was taken on to the next step without further characterization.

Example 227B

(Z)-N-(3-allyl-4,5-dimethylthiazol-2(3H)-ylidene)-5-chloro-2-methoxybenzamide

A mixture of Example 227A (1.3 g), Example 205B (1.36 g) and triethylamine (1.0 g) in tetrahydrofuran (40 mL) was heated to 60 °C for 4 hours, cooled and solvent was evaporated. The crude material was triturated with ether, filtered and solvent evaporated. The crude was flash chromatographed over silica gel gradient eluting with ethyl acetate:hexane (2:3 to 3:2) to afford the title compound. $^1$H NMR (300 MHz, dimethylsulfoxide-$d_6$) δ ppm 2.22 (s, 3H), 2.23 (s, 3H), 3.78 (s, 3H), 4.85 (m, 2H), 4.96, (dq, J = 17.3, 1.3 Hz, 1H), 5.19 (dq, J = 10.5, 1.3 Hz, 1H), 5.92-6.05 (m, 1H), 7.10 (d, 1H), 7.44 (dd, 1H), 7.66 (d, 1H); MS (ESI+) m/z 337 $(M+H)^+$.

Example 227C

5-chloro-N-[(2Z)-4,5-dimethyl-3-[(3-methyl-4,5-dihydroisoxazol-5-yl)methyl]-1,3-thiazol-2(3H)-ylidene]-2-methoxybenzamide

To a solution of acetaldoxime (56 mg, 1.48 mmol) in $CHCl_3$ (10 mL) under $N_2$ was added N-chlorosuccinimide (200 mg) and pyridine (10 μL). After 4.5 hours at room temperature, Example 227B (100 mg) was added, followed by triethylamine (0.15 g) and the reaction continued to stir at room temperature for 21 hours. The reaction mixture was washed with water and partitioned. The aqueous layer was extracted again with $CH_2Cl_2$ and the combined organic extracts were dried ($MgSO_4$), filtered, and solvent evaporated. The crude was purified by flash chromatography over silica gel eluting with ethyl acetate:hexane (1:1) to afford the title compound. $^1$H NMR (300 MHz, dimethylsulfoxide-$d_6$) δ ppm 1.93 (s, 3H), 2.23 (s, 6H), 2.90 (dd, 1H), 3.14 (dd, 1H), 3.79 (s, 3H), 4.11 (dd, 1H), 4.27, (dd, 1 H), 4.98 (m, 1 H), 7.11 (d, 1H), 7.44 (dd, 1H), 7.67 (d, 1H). MS (ESI+) m/z 394 $(M+H)^+$.

Example 228

N-[(2Z)-4,5-dimethyl-3-(tetrahydrofuran-2-ylmethyl)-1,3-thiazol-2(3H)-ylidene]-4-(trifluoromethyl)nicotinamide

Example 186A (0.15 g) and 4-(trifluoromethyl)nicotinic acid (0.12 g) were processed according to the method of Example 226. The crude was purified by reverse phase preparative HPLC on a Waters Symmetry C8 column (25 mm x 100 mm, 7 μm particle size) using a gradient of 10% to 100% acetonitrile:0,1% aqueous trifluoroacetic acid over 8 minutes (10 minutes run time) at a flow rate of 40 mL/minutes) to afford the title compound. $^1$H NMR (300 MHz, dimethylsulfoxide-$d_6$) δ ppm 1.63 (m, 1H), 1.85 (m, 2H), 1.96 (m, 1H), 2.25 (s, 3H), 2.29 (s, 3H), 3.62 (dd, 1H), 3.77 (dd, 1H), 4.07 (dd, 1H), 4.25 (m, 1H), 4.38 (dd, 1H), 7.80 (d, 1H), 8.89 (d, 1H), 9.12 (s, 1H), MS (ESI+) m/z 386 $(M+H)^+$.

Example 229

N-[(2Z)-4,5-dimethyl-3-(tetrahydrofuran-2-ylmethyl)-1,3-thiazol-2(3H)-ylidene]-2-ethoxynicotinamide

Example 186A (0.15 g) and 2-ethoxynicotinic acid (0.1 g) were processed according to the method of Example 226. The crude was purified by reverse phase preparative HPLC on a Waters Symmetry C8 column (25 mm x 100 mm, 7 μm particle size) using a gradient of 10% to 100% acetonitrile:0.1% aqueous trifluoroacetic acid over 8 minutes (10 minutes run time) at a flow rate of 40 mL/minutes) to afford the title compound. $^1$H NMR (300 MHz, dimethylsulfoxide-$d_6$) δ ppm 1.32 (t, 3H), 1.66 (m, 1H), 1.85 (m, 2H), 1.97 (m, 1H), 2.22 (s, 3H), 2.26 (s, 3H), 3.62 (dd, 1H), 3.78 (dd, 1H), 4.06 (dd, 1H), 4.3-4.42 (m, 3H), 7.02 (dd, 1H), 8.13 (dd, 1H), 8.22 (dd, 1H). MS (ESI+) m/z 362 $(M+H)^+$.

Example 230

N-[(2Z)-4,5-dimethyl-3-(tetrahydrofuran-2-ylmethyl)-1,3-thiazol-2(3H)-ylidene]-2,3,6-trifluoroisonicotinamide

Example 186A (0.15 g) and 2,3,6-trifluoroisonicotinic acid (0.11 g) were processed according to the method of Example 226. The crude was purified by reverse phase preparative HPLC on a Waters Symmetry C8 column (25 mm x 100 mm, 7 μm particle size) using a gradient of 10% to 100% acetonitrile:0.1% aqueous trifluoroacetic acid over 8 minutes (10 minutes run time) at a flow rate of 40 mL/minutes) to afford the title compound. $^1$H NMR (300 MHz, dimethylsulfoxide-$d_6$) δ ppm 1.67 (m, 1H), 1.86 (m, 2H), 2.00 (m, 1H), 2.27 (s, 3H), 2.30 (s, 3H), 3.62 (dd, 1H), 3.78 (dd, 1H), 4.15 (dd, 1H), 4.28 (m, 1H), 4.43 (dd, 1H), 7.57 (t, 1H). MS (ESI+) m/z 372 $(M+H)^+$,

Example 231

6-chloro-N-[(2Z)-4,5-dimethyl-3-(tetrahydrofuran-2-ylmethyl)-1,3-thiazol-2(3H)-ylidene]-4-(trifluoromethyl)nicotinamide

Example 186A (0.15 g) and 6-chloro-4-(trifluoromethyl)nicotinic acid (0.17 g) were processed according to the method of Example 226. The crude was purified by reverse phase preparative HPLC on a Waters Symmetry C8 column (25 mm x 100 mm, 7 μm particle size) using a gradient of 10% to 100% acetonitrile:0.1% aqueous trifluoroacetic acid over 8 minutes (10 minutes run time) at a flow rate of 40 mL/minutes) to afford the title compound. $^1$H NMR (300 MHz, dimethylsulfoxide-$d_6$) δ ppm 1.62 (m, I H), 1.85 (m, 2H), 1.96 (m, 1H), 2.25 (s, 3H), 2.29 (s, 3H), 3.62 (dd, 1H), 3.77 (dd, 1H), 4.08 (dd, 1H), 4.24 (m, 1H), 4.38 (dd, 1H), 7.99 (s, 1H), 9.00 (s, 1H); MS (ESI+) m/z 420 $(M+H)^+$.

Example 232

5-chloro-2-methoxy-N-[(2Z)-5-methyl-3-[(3-methyl-4,5-dihydroisoxazol-5-yl)methyl]-1,3-thiazol-2(3H)-ylidenelbenzamide

Example 232A

3-allyl-5-methylthiazol-2(3H)-imine hydrobromide

A mixture of 2-amino-5-methylthiazole (2.5 g) and allylbromide (3.31 g) was processed according to the method of Example 227A to afford the title compound that was taken directly to the next step. MS (ESI+) m/z 155 $(M+H)^+$.

Example 232B

(Z)-N-(3-allyl-5-methylthiazol-2(3H)-ylidene)-5-chloro-2-methoxybenzamide

A mixture of Example 232A (0.5 g) and 5-chloro-2-methoxybenzoylchloride (0.57 g) were processed according to the method of Example 227B to afford the title compound $^1$H NMR (300 MHz, dimethylsulfoxide-$d_6$) δ ppm 2.28 (d, J = 1.4 Hz, 3H), 3.78 (s, 3H), 4.77 (d, 2H), 5.13 (dq, J = 16.9, 1.3 Hz, 1 H), 5.24 (dq, J = 10.5, 1.4 Hz, 2H), 5.94-6.07 (m, 1H), 7.11 (d, 1 H), 7.23 (q, J = 1.4 Hz, 1 H), 7.43 (dd, 1 H), 7.68 (d, 1 H); MS (ESI+) m/z 323 $(M+H)^+$.

Example 232C

5-chloro-2-methoxy-N-[(2Z)-5-methyl-3-[(3-methyl-4,5-dihydroisoxazol-5-yl)methyl]-1,3-thiazol-2(3H)-ylidene]benzamide

Example 232B was processed according to the method of Example 227C to afford the title compound. $^1$H NMR (300 MHz, dimethylsulfoxide-$d_6$) δ ppm 1.90 (s, 3H), 2.28 (s, 3H), 2.87 (dd, 1 H), 3.11 (dd, 1 H), 4.23 (d, 2H), 4.94 (m, 1 H), 7.11 (d, 1 H), 7.25 (s, 1 H), 7.45 (dd, 1H), 7.67 (d, 1 H); MS (ESI+) m/z 380 $(M+H)^+$.

Example 233

N-[(2Z)-4,5-dimethyl-3-(tetrahydrofuran-2-ylmethyl)-1,3-thiazol-2(3H)-ylidene]-2-(ethylamino)benzamide

Example 186A (0.15 g) and 2-ethylaminobenzoic acid (Pellon *Sym. Lett.* **2005**, *10*, 1606) (0.1 g) were processed according to the method of Example 226. The crude was purified by flash chromatography over silica gel gradient eluting

with ethyl acetate:hexane (1:9 to 1:3) to afford the title compound. $^1$H NMR (300 MHz, dimethylsulfoxide-$d_6$) $\delta$ ppm 1.25 (t, 3H), 1.70 (m, 1H), 1.87 (m, 2H), 2.01 (m, 1H), 2.20 (s, 3H), 2.26 (s, 3H), 3.20 (m, 2H), 3.62 (dd, 1H), 3.79 (dd, 1H), 4.12 (dd, 1 H), 4.28 (m, 1 H), 4.38 (dd, 1 H), 6.55 (t, 1 H), 6.67 (d, 1 H), 7.27 (t, 1 H), 8.22 (dd, 1 H), 8.52 (t, 1 H); MS (ESI+) m/z 360 $(M+H)^+$.

Example 234

N-[(2Z)-4,5-(dimethyl-3-(tetrahydrofuran-2-ylmethyl)-1,3-thiazol-2(3H)-ylidene]-5-iodo-2-(methylamino)benzamide

Example 186A (0.15 g) and 5-iodo-2-methylaminobenzoic acid (0.17 g) were processed according to the method of Example 226. The crude was purified by flash chromatography over silica gel gradient eluting with ethyl acetate: hexane (1:19 to 1:10) to afford the title compound.. $^1$H NMR (300 MHz, dimethylsulfoxide-$d_6$) $\delta$ ppm 1.7-1.82 (m, 1H), 1.84-1.95 (m, 2H), 1.95-2.08 (m, 1H), 2.22 9s, 3H), 2.27 (s, 3H), 2.84 (d, 3H), 3.65 (dd, 1H), 3.82 (dd, 1H), 4.12 (dd, 1H), 4.28 (m, 1H), 4.36 (dd, 1H), 6.52 (d, 1H), 7.53 (dd, 1H), 8.46 (d, 1H), 8.52 (q, 1H); MS (ESI+) m/z 472 $(M+H)^+$.

Example 235

5-bromo-N-[(2Z)-5-tert-butyl-3-[(2R)-tetrahydrofuran-2-ylmethyl]-1,3-thiazol-2(3H)-ylidene]-2-methoxybenzamide

To a mixture of 3,3-dimethylbutyraldehyde (Aldrich) (5 mL, 39.8 mmol) and Example 278A (641.0 mg, 4.0 mmol) was added a mixture of dimethylsulfoxide (560 µL, 8 mmol) and 12 N aqueous HCl (667 µL, 8 mmol). The reaction mixture was heated at 40°C overnight. The mixture was concentrated and the residue was dried under vacuum for 2 hours. The residue (252 mg. 0.9 mmol) was dissolved in tetrahydrofuran (10 mL). To this solution was added 5-bromo-2-methoxy-benzoic acid (209.0 mg, 0.9 mmol), N-(3-dimethylaminopropyl)-N-ethylcarbodimide hydrochloride (73 mg, 0.9 mmol), 1-hydroxybenzotriazole (122.0 mg, 0.9 mmol) and triethylamine (315.0 µL, 2.3 mmol). The mixture was stirred overnight at 80°C, and cooled to room temperature. The mixture was diluted with ethyl acetate, washed with 1 M aqueous $NaHCO_3$, dried ($Na_2SO_4$), filtered and concentrated. The residue was purified by flash chromatography using an Analogix® Intelliflash280™ ($SiO_2$, 0-75 % ethyl acetate in hexanes) to afford the title compound. $^1$H NMR (300 MHz, dimethylsulfoxide-$d_6$) $\delta$ 1.32 (s, 9 H), 1.58 - 1.71 (m, 1 H), 1.75 - 1.86 (m, 2 H), 1.87-2.00 (m, 1 H), 3.64 (dd, *J*=15, 6.8 Hz, 1 H), 3.78 (s, 3 H), 3.79 - 3.83 (m, 1 H), 4.19 (d, *J*=5.8 Hz, 2 H), 4.23 - 4.35 (m, 1 H), 7.05 (d, *J*=8.8 Hz, 1 H), 7.25 (s, 1 H), 7.56 (dd, *J*=8.8, 2.7 Hz, 1 H), 7.76 (d, *J*=2.7 Hz, 1 H.

Example 236

5-chloro-2-(cyclopropyloxy)-N-[(2Z)-5-methyl-3-[(2R)-tetrahydrofuran-2-ylmethyl]-1,3-thiazol-2(3H)-ylidene]benzamide

Example 236A

methyl 5-chloro-2-(2-chloroethoxy)benzoate

A mixture of methyl-5-chlorosalicylate (19.5 g, 105 mmol), 2-chloroethyl *p*-toluenesulfonate (19.3 mL, 107 mmol) and $K_2CO_3$ (28.9 g, 210 mmol) in 105 mL of N,N- dimethylformamide was warmed to 50 °C and allowed to stir for 18 hours. The mixture was cooled to ambient temperature, diluted with 25 mL of ethyl acetate and 25 mL of $H_2O$. The layers were separated and the organic layer was dried over anhydrous $Na_2SO_4$, filtered and concentrated under reduced pressure. Purification via column chromatography ($SiO_2$, 75% hexanes in ethyl acetate) afforded the title compound. MS ($DCI/NH_3$) m/z 249 $(M+H)^+$, 266 $(M+NH_4)^+$.

Example 236B

5-chloro-2-(vinyloxy)benzoic acid

To Example 236A (15 g, 60 mmol) in 100 mL of tetrahydrofuran at 0 °C was added potassium t-butoxide (8.9 g, 75.6 mmol) portion wise with the internal reaction temperature being maintained below 5 °C. After the addition was complete, the mixture was allowed to warm to ambient temperature and was allowed to stir for 18 hours. The mixture was diluted with 25 mL of $H_2O$ and 25 mL of ethyl acetate and the layers were separated. The aqueous layer was acidified with 1 N aqueous HCl to pH 7 and was extracted with three 15 mL portions of ethyl acetate. These organic extracts (excluding the original organic layer before acidification) were combined and dried over anhydrous $Na_2SO_4$, filtered and concentrated under reduced pressure to afford the title compound. MS ($DCI/NH_3$) m/z 216 $(M+NH_4)^+$.

Example 236C

methyl 5-chloro-2-(vinyloxy)benzoate

To Example 236B (5.1 g, 26 mmol) in 30 mL ofN,N- dimethylformamide was added $K_2CO_3$ (10.7 g, 78 mmol) followed by $CH_3I$ (1.8 mL, 29 mmol). The mixture stirred at ambient temperature for 3 hours and was diluted with 20 mL of $H_2O$ and 20 mL of ether. The layers were separated and the aqueous layer was extracted twice with 10 mL of ether. The combined organic extracts were dried over anhydrous $Na_2SO_4$, filtered and concentrated under reduced pressure. Purification via column chromatography ($SiO_2$, 75% hexanes in ethyl acetate) afforded the title compound. MS (DCI/$NH_3$) m/z 213 $(M+H)^+$, 230 $(M+NH_4)^+$.

Example 236D

methyl 5-chloro-2-cyclopropoxybenzoate

To a solution of Example 236C (1.29 g, 6.1 mmol) in 15 mL of dichloroethane at -5 °C was added chloroiodomethane (1.4 mL, 19.4 mmol). A solution of diethylzinc (1M solution in hexanes, 9.7 mL, 9.7 mmol) was added dropwise over 1 hour using a syringe pump. After the addition was complete, the mixture was allowed to warm to ambient temperature and was stirred for 45 minutes. The mixture was cooled to 0 °C and quenched with 5 mL of saturated, aqueous $NH_4Cl$ and 1 mL of concentrated. $NH_4OH$. This mixture was diluted with 10 mL of ethyl acetate and the layers were separated. The aqueous layer was extracted twice with 10 mL of ethyl acetate and the combined organic extracts were dried over anhydrous $Na_2SO_4$, filtered and concentrated under reduced pressure. Purification via column chromatography ($SiO_2$, 75% hexanes in ethyl acetate) afforded the title compound. MS (DCI/$NH_3$) m/z 227 $(M+H)^+$, 244 $(M+NH_4)^+$.

Example 236E

5-chloro-2-cyclopropoxybenzoic acid

To a solution of Example 236D (1.24 g, 5.5 mmol) in 10 mL of ethanol at ambient temperature was added 5 mL of 40% aqueous KOH. The mixture was stirred at ambient temperature for 2 hours and was partially concentrated to remove the ethanol. The aqueous residue was extracted with three 10 mL of portions of $CH_2Cl_2$. The aqueous layer was acidified with 10 % aqueous HCl to ~pH I and then extracted with three 10 mL of portions of $CH_2Cl_2$. The combined organic extracts (from both extractions) were dried over anhydrous $Na_2SO_4$, filtered and concentrated under reduced pressure to afford the title compound. MS (DCI/$NH_3$) m/z 213 $(M+H)^+$, 230 $(M+NH_4)^+$.

Example 236F

5chloro-2-(cyclopropyloxy)-N-[(2Z)-5-methyl-3-[(2R)-tetrahydrofuran-2-ylmethyl]-1,3-thiazol-2(3H)-ylidene]benzamide

A mixture of Example 236E (0.30 g, 1.4 mmol) and 1,1'-carbonyldiimidazole (0.27 g, 1.7 mmol) in 10 mL of ethyl acetate was stirred at ambient temperature for 4 hours. Example 208B (0.28 g, 1.4 mmol) in 2 mL of ethyl acetate and 2 mL of tetrahydrofuran was added and the mixture was wanned to reflux for 16 hours. The mixture was cooled to ambient temperature and was quenched with 10 mL of $H_2O$ and 5 mL 5% aqueous HCl and was diluted with 10 mL of ethyl acetate. The layers were separated and the aqueous layer was extracted twice with 5 mL of ethyl acetate. The combined organic extracts were dried over anhydrous $Na_2SO_4$, filtered and concentrated under reduced pressure. Purification via column chromatography ($SiO_2$, 40% hexanes in ethyl acetate) afforded the title compound. $^1H$ NMR (300 MHz, $CD_3OD$) $\delta$ ppm 0.76 - 0.82 (m, 2 H), 1.63 - 1.77 (m, 1H), 1.84- 1.96 (m, 2H), 2.01 -2.14 (m, 1H), 2.33 (d, *J*=1.4Hz, 3H), 3.69-3.93 (m, 4H), 4.18 - 4.43 (m, 4 H), 7.13 (q, *J*=1.4 Hz, 1 H), 7.38 - 7.41 (m, 2 H), 7.74 (dd, *J*=2.0, 1.0 Hz, 1 H); MS (DCI/$NH_3$) m/z 393 $(M+H)^+$. Anal. Calculated for $C_{19}H_{21}ClN_2O_3S$: C, 58.08; H, 5.39; N, 7.13. Found: C, 57.77; H, 5.45; N, 7.09.

Example 237

5-chloro-N-[(2Z)-3-(1,4-dioxan-2-ylmethyl)-5-methyl-1,3-thiazol-2(3H)-ylidene]-2-methoxybenzamide

Example 237A

3-((1,4-dioxan-2-yl)methyl)-5-methylthiazol-2(3H)-imine

A mixture of 2-amino-5-methylthiazole (0.77 g, 6.7 mmol) and 2-iodomethyl-1,4-dioxane (Synchem-OHG, 1.5 g, 6.7 mmol) was wanned to 85°C and was allowed to stir for 18 hours. The mixture was cooled to ambient temperature and the crude material was purified via column chromatography ($SiO_2$, 10% methanol in ethyl acetate then 9:1:0.1 $CH_2Cl_2$ : methanol : $NH_4OH$) to afford the title compound. MS ($DCI/NH_3$) m/z 215 $(M+H)^+$.

Example 237B

5-chloro-N-[(2Z)-3-(1,4-dioxan-2-ylmethyl)-5-methyl-1,3-thiazol-2(3H)ylidene]-2-methoxybenzamide

Example 237A (0.20 g, 0.93 mmol), triethylamine (0.39 mL, 2.8 mmol) and Example 205B (0.93 mmol) in 10 mL of tetrahydrofuran were processed as described in Example 208D to afford the title compound. $^1H$ NMR (300 MHz, $CD_3OD$) $\delta$ ppm 2.33 (d, $J$=1.4 Hz, 3 H), 3.34 (dd, $J$=11.5, 9.5 Hz, 1 H), 3.50 - 3.71 (m, 3 H), 3.82 (dt, $J$=10.5, 3.1 Hz, 2 H), 3.86 (s, 3 H), 3.99 - 4.09 (m, 1 H), 4.19 - 4.36 (m, 2 H), 7.08 (d, $J$=8.8 Hz, 1 H), 7.09 (q, $J$=1.0 Hz, 1 H), 7.40 (dd, $J$=9.0, 2.9 Hz, 1 H), 7.81 (d, $J$=2.7 Hz, 1 H); MS ($DCI/NH_3$) m/z 383 $(M+H)^+$. Anal. Calculated for $C_{17}H_{19}ClN_2O_4S$: C, 53.33; H, 5.00; N, 7.32. Found: C, 53.51; H, 4.93; N, 7.29.

Example 238

N-[(2Z)-5-acetyl-4-methyl-3-[(2R)-tetrahydrofuran-2-ylmethyl]-1,3-thiazol-2(3H)-ylidene]-5-chloro-2-methoxybenzamide

Example 238A

N-(5-acetyl-4-methylthiazol-2-yl)-5-chloro-2-methoxybenzamide

To a solution of 5-acetyl-2-amino-4-methylthiazole (5.0 g, 32 mmol) in 50 mL of tetrahydrofuran was added triethylamine (13.4 mL, 96 mmol) followed by Example 205B (32 mmol) in 10 mL) of tetrahydrofuran via cannula. The mixture was wanned to 50 °C and was allowed to stir for 18 hours. The mixture was cooled to ambient temperature quenched with 15 mL of $NH_4Cl$ and diluted with 15 mL of ethyl acetate. The layers were separated and the aqueous layer was extracted with three 10 mL of portions of ethyl acetate. The combined organic extracts were dried over anhydrous $Na_2SO_4$, filtered and concentrated under reduced pressure. The residue was washed with ethyl acetate and the remaining solids were pure title compound. MS ($DCI/NH_3$) m/z 325 $(M+H)^+$.

Example 238B

N-[(2Z)-5-acetyl-4-methyl-3-[(2R)-tetrahydrofuran-2-ylmethyl]-1,3-thiazol-2(3H)-ylidene]-5-chloro-2-methoxybenzamide

A mixture of Example 238A (1.1 g, 3.2 mmol), Example 208A (1.0 g, 3.9 mmol), tetrabutylammonium iodide (0.36 g, 0.98 mmol) and potassium t-butoxide (0.58 g, 4.9 mmol) in 12 mL of N,N- dimethylformamide was warmed to 65 °C and was allowed to stir for 16 hours. The mixture was cooled to ambient temperature quenched with 10 mL of $NH_4Cl$ and diluted with 10 mL of ethyl acetate. The layers were separated and the aqueous layer was extracted twice with 10 mL of ethyl acetate. The combined organic extracts were dried over anhydrous $Na_2SO_4$, filtered and concentrated under reduced pressure. Purification via column chromatography ($SiO_2$, 30% hexanes in ethyl acetate) afforded the title compound. $^1H$ NMR (300 MHz, $CD_3OD$) $\delta$ ppm 1.73 - 1.87 (m, I H), 1.89 - 2.05 (m, 2 H), 2.09 - 2.22 (m, 1 H), 2.52 (s, 3 H), 2.79 (s, 3 H), 3.69 - 3.78 (m, 1 H), 3.85 - 3.94 (m, 1 H), 3.88 (s, 3 H), 4.21 - 4.31 (m, 1 H), 4.38 - 4.49 (m, 1 H), 4.59 (dd, $J$=13.9, 3.1 Hz, 1 H), 7.10 (d, $J$=9.2 Hz, 1 H), 7.44 (dd, $J$=8.8, 2.7 Hz, 1 H), 7.94 (d, $J$=2.7 Hz, 1 H); MS ($DCI/NH_3$) m/z 409 $(M+H)^+$. Anal. Calculated for $C_{19}H_{21}ClN_2O_4S\cdot 0.12H_2O$: C, 55.52; H, 5.21; N, 6.81. Found: C, 55.83; H, 5.21; N, 6.41.

Example 239

5-chloro-N-[(2Z)-5-(1-hydroxy-1-methylethyl)-4-methyl-3-[(2R)-tetrahydrofuran-2-ylmethyl]-1,3-thiazol-2(3H)-ylidene]-2-methoxybenzamide

To Example 238B (0.11 g, 0.27 mmol) in 5 mL of tetrahydrofuran at -78 °C was added a solution of methyl lithium (1.6 M in ether, 0.50 mL, 0.81 mmol) dropwise over 5 minutes. The mixture stirred at -78 °C for 1 hour and was slowly wanned to ambient temperature and was allowed to stir for 18 hours. The mixture was quenched with 5 mL of $NH_4Cl$ and diluted with 5 mL of ethyl acetate. The layers were separated and the aqueous layer was extracted twice with 5 mL of ethyl acetate. The combined organic extracts were dried over anhydrous $Na_2SO_4$, filtered and concentrated under reduced pressure. Purification via column chromatography ($SiO_2$, 20% hexanes in ethyl acetate) afforded the title compound. $^1H$ NMR (300 MHz, $CD_3OD$) $\delta$ ppm 1.26 (none, 1 H), 1.61 (s, 6 H), 1.73 - 1.85 (m, I H), 1.88 - 2.01 (m, 2 H), 2.05 - 2.19 (m, 1 H), 2.52 (s, 3 H), 3.69 - 3.78 (m, 1 H), 3.85 (s, 3 H), 3.87 - 3.93 (m, 1 H), 4.02 - 4.19 (m, 2 H), 4.36 - 4.45 (m, 1 H), 4.50 (dd, *J*=13.9, 3.1 Hz, 1 H), 7.06 (d, *J*=8.8 Hz, 1 H), 7.39 (dd, *J*=8.8, 2.7 Hz, 1 H), 7.83 (d, *J*=2.7 Hz, 1 H); MS (DCI/$NH_3$) m/z 425 $(M+H)^+$. Anal. Calculated for $C_{10}H_{15}ClN_2O_4S{\cdot}0.1H_2O$: C, 56.29; H, 5.95; N, 6.56. Found: C, 55.95; H, 5.87; N, 6.47.

Example 240

N-[(2Z)-5-tert-butyl-3-[(2R)-tetrahydrofuran-2-ylmethyl]-1,3-thiazol-2(3H)-ylidene]-5-chloro-2-methoxybenzamide

Example 240A

5-tert-butylthiazol-2-amine

To a solution of 3,3-dimethylbutyraldehyde (10 g, 99.8 mmol) in 200 mL of cyclohexane was added pyrrolidine (8.7 mL, 0.11 mol) followed by *p*-toluenesulfonic acid monohydrate (0.95 g, 5.0 mmol). This reaction flask was equipped with a Dean-Stark trap and the mixture was wanned to reflux and was allowed to stir for 3 hours. The mixture was cooled to ambient temperature, filtered and concentrated under reduced pressure. The residue was dissolved in 75 mL of $CH_3OH$, sulfur was added (3.2 g, 99.8 mmol), and the mixture was cooled to 0 °C. Cyanamide (4.2 g, 99.8 mmol) was added portion wise over 10 minutes and the mixture was allowed to wann to ambient temperature and stir for 18 hours. The reaction mixture was concentrated under reduced pressure and purified by column chromatography ($SiO_2$, ethyl acetate then 10% methanol in ethyl acetate) to afford the title compound. MS (DCI/$NH_3$) m/z 157 $(M+H)^+$.

Example 240B

(R)-(tetrahydrofuran-2-yl)methyl 4-methylbenzenesulfonate

To a solution of (R)-tetrahydrofurfuryl alcohol (Lancaster, 1.0 g, 9.8 mmol) in 5 mL of $CH_2Cl_2$ and 5 mL of pyridine was added *p*- toluenesulfonyl chloride (2.8 g, 14.7 mmol) in portions over 15 minutes. The mixture was stirred at ambient temperature for 3 hours and was quenched with 5 mL of saturated, aqueous $NaHCO_3$. The layers were separated and the aqueous layer was extracted with three 5 mL of portions of $CH_2Cl_2$. The combined organic extracts were dried over anhydrous $Na_2SO_4$, filtered and concentrated under reduced pressure to afford the title compound. MS (DCI/$NH_3$) m/z 257 $(M+H)^+$, 274 $(M+NH_4)^+$.

Example 240C

(R)-5-tert-butyl-3-((tetrahydrofuran-2-yl)methyl)thiazol-2(3H)-imine 4-methylbenzenesulfonate

A mixture of Example 240A (9.8 g, 62.7 mmol), Example 240B (23.5 g, 91.7 mmol) and tetrabutylammonium iodide (11.6 g, 31.4 mmol) in 35 mL ofN,N- dimethylformamide was wanned to 85 °C and was allowed to stir for 72 hours. The mixture was diluted with 50 mL of $CH_2Cl_2$ and the layers were separated. The organic layer was washed with 15 mL of saturated, aqueous $NHCO_3$ and the combined aqueous layers were extracted with three 10 mL of portions of $CH_2Cl_2$. The combined organic extracts were dried over anhydrous $Na_2SO_4$, filtered and concentrated under reduced pressure. Purification via column chromatography ($SiO_2$, 10% methanol in ethyl acetate then 9:1:0.1 $CH_2Cl_2$ : methanol : $NH_4OH$) afforded the title compound. MS (DCI/$NH_3$) m/z 241 $(M+H)^+$.

Example 240D

5-chloro-2-methoxybenzoyl chloride

A solution of 2-methoxy-5-chlorobenzoic acid (6.9 g, 37 mmol) in 15 mL of $SOCl_2$ was warmed to reflux and was allowed to stir for 2 hours. The mixture was cooled to ambient temperature and concentrated under reduced pressure. The crude material was diluted with 5 mL of toluene and concentrated under reduced pressure. This dilution with toluene and concentration was repeated two additional times to afford the title compound. $^1H$ NMR (300 MHz, dimethylsulfoxide-$d_6$) $\delta$ ppm 3.82 (s, 3 H), 7.16 (d, *J*=8.8 Hz, 1 H), 7.49 - 7.59 (m, 1 H), 7.61 (d, *J*=2.7 Hz, 1 H).

Example 240E

N-[(2Z)-5-tert-butyl-3-[(2R)-tetrahydrofuran-2-ylmethyl]-1,3-thiazol-2(3H)-ylidene]-5-chloro-2-methoxybenzamide

To a solution of Example 240C (13.9 g, 34 mmol) in 120 mL of tetrahydrofuran at ambient temperature was added triethylamine (19 mL, 135 mmol) followed by Example 240D (7.6 g, 37 mmol) in 30 mL of tetrahydrofuran via cannula. This mixture was warmed to 60 °C and was allowed to stir for 3 hours and was quenched with 30 mL of $NH_4Cl$ and diluted with 50 mL of ethyl acetate. The layers were separated and the aqueous layer was extracted with three 5 mL of portions of ethyl acetate. The combined organic extracts were dried over anhydrous $Na_2SO_4$, filtered and concentrated under reduced pressure. Purification via flash column chromatography ($SiO_2$, 50% hexanes : ethyl acetate) afforded the title compound. $^1H$ NMR (300 MHz, $CDCl_3$) $\delta$ ppm 1.35 (s, 9 H), 1.64 - 1.92 (m, 3 H), 2.00 - 2.14 (m, 1 H), 3.72 - 3.88 (m, 2 H), 3.90 (s, 3 H), 4.19 - 4.34 (m, 2 H), 4.40 (dd, *J*= 12.9, 2.4 Hz, 1 H), 6.86 (s, 1 H), 6.90 (d, *J*=9.2 Hz, 1 H), 7.32 (dd, *J*=9.0, 2.9 Hz, 1 H), 7.95 (d, *J*=3.1 Hz, 1 H); MS (DCI/$NH_3$) m/z 409 $(M+H)^+$. Anal. Calculated for $C_{10}H_{15}ClN_2O_3S$: C, 58.74; H, 6.16; N, 6.85. Found: C, 58.74; H, 6.27; N, 6.81.

Example 241

N-[(2Z)-5-tert-butyl-3-(1,3-dioxolan-2-ylmethyl)-1,3-thiazol-2(3H)-ylidene]-5-chloro-2-methoxybenzamide

Example 241A

3-((1,3-dioxolan-2-yl)methyl)-5-tert-butylthiazol-2(3H)-imine

A mixture of Example 240A (0.17 g, 1.1 mmol) and 2-bromomethyl-1,3-dioxolane (0.21 g, 1.2 mnol) was warmed to 85 °C and was allowed to stir for 18 hours. The mixture was cooled to ambient temperature and the crude material was purified via flash column chromatography ($SiO_2$, 10% methanol in ethyl acetate then 9:1:0.1 $CH_2Cl_2$ : methanol : $NH_4OH$) to afford the title compound. MS (DCI/$NH_3$) m/z 243 $(M+H)^+$.

Example 241B

N-[2Z)-5-tert-butyl-3-(1,3-dioxolan-2-ylmethyl)-1,3-thiazol-2(3H)-ylidene]-5-chloro-2-methoxybenzamide

Example 241A (64 mg, 0.26 mmol), triethylamine (0.11 mL, 0.79 mmol) and Example 205B (0.26 mmol) in 2 mL of tetrahydrofuran and 0.5 mL of N,N- dimethylformamide were processed as in Example 208D to afford the title compound. $^1H$ NMR (300 MHz, $CD_3OD$) $\delta$ ppm 1.38 (s, 9 H), 3.86 (s, 3 H), 3.88 - 3.98 (m, 4H), 4.42 (d, *J*=4.1 Hz, 2 H), 5.29 (dd, *J*=4.1 Hz, 1 H), 7.07 (d, *J*=8.8 Hz, 1 H), 7.12 (s, 1 H), 7.40 (dd, *J*=9.0, 2.9 Hz, 1 H), 7.87 (d, *J*=2.7 Hz, I H); MS (DCI/$NH_3$) m/z 411 $(M+H)^+$. Anal. Calculated for $C_{19}H_{23}ClN_2O_4S$: C, 55.54; H, 5.64; N, 6.82. Found: C, 55.43; H, 5.60; N, 6.62.

Example 242

5-chloro-N-[(2Z)-5-chloro-3-[(2R)-tetrahydrofuran-2-ylmethyl]-1,3-thiazol-2(3H)-ylidene]-2-methoxybenzamide

Example 242A

5-chloro-N-(5-chlorothiazol-2-yl)-2-methoxybenzamide

A mixture of 2-amino-5-chlorothiazole hydrochloric acid (1.0 g, 5.9 mmol), 5-chloro-2-methoxybenzoic acid (1.3

g, 7.0 mmol), 1-ethyl-(3-dimethylaminopropyl)carbodiimide hydrochloride (Chem-Impex International, 2.2 g, 12 mmol), 1-hydroxbenzotriazole (0.95 g, 7.0 mmol) and 4-dimethylaminopyridine (0.14 g, 1.2 mmol) in 6 mL of pyridine was allowed to stir at ambient temperature for 72 hours. The reaction mixture was concentrated under reduced pressure and 10 mL of $H_2O$ was added. The resulting solids were isolated by filtration, washed with 5 mL of $H_2O$ and twice with 5 mL of ethyl acetate, and dried to afford the title compound. MS ($DCI/NH_3$) m/z 303 $(M+H)^+$.

Example 242B

5-chloro-N-[(2Z)-5-chloro-3-[(2R)-tetrahydrofuran-2-ylmethyl]-1,3-thiazol-2(3H)-ylidene]-2-methoxybenzamide

To a slurry of sodium hydride (40 mg of a 60% dispersion, 1.0 mmol) in 4 mL ofN,N-dimethylformamide at 0 °C was added Example 242A (0.20 g, 0.66 mmol). This mixture was allowed to warm to ambient temperature and stirred for I hour. The mixture was cooled to 0 °C and Example 208A (0.19 g, 0.73 mmol) was added. The mixture was wanned to 80 °C and allowed to stir for 24 hours and cooled to ambient temperature. The reaction mixture was quenched with ice and 5 mL of saturated, aqueous $NH_4Cl$ and diluted with 5 mL of ethyl acetate. The layers were separated and the aqueous layer was extracted with three 5 mL of portions of ethyl acetate. The combined organic extracts were dried over anhydrous $Na_2SO_4$, filtered and concentrated under reduced pressure. Purification via flash column chromatography ($SiO_2$, 50% hexanes : ethyl acetate) afforded the title compound. $^1H$ NMR (300 MHz, $CD_3OD$) $\delta$ ppm 1.65 - 1.80 (m, I H), 1.85 - 1.97 (m, 2 H), 2.02 - 2.18(m, 1 H), 3.72 - 3.82 (m, 1 H), 3.84 - 3.94 (m, 1 H), 3.87 (s, 3 H), 4.21 - 4.38 (m, 2 H), 4.40 - 4.48 (m, 1 H), 7.09 (d, *J*=8.8 Hz, I H), 7.43 (dd, *J*=9.0, 2.9 Hz, I H), 7.50 (s, 1 H), 7.90 (d, *J*=2.7 Hz, 1 H); MS ($DCI/NH_3$) m/z 387 $(M+H)^+$. Anal. Calculated for $C_{16}H_{16}Cl_2N_2O_3S$: C, 49.62; H, 4.16; N, 7.23. Found: C, 50.49; H, 4.03; N, 6.70.

Example 243

5-chloro-N-[(2Z)-5-chloro-3-(tetrahydro-2H-pyran-4-ylmethyl)-1,3-thiazol-2(3H)-ylidene]-2-methoxybenzamide

Example 242A (0.20 g, 0.66 mmol), Example 203A (0.20, 0.73 mmol) and NaH (40 mg, I mmol) in 4 mL of tetrahydrofuran and I mL of N,N- dimethylformamide were processed as in Example 242B to afford the title compound. $^1H$ NMR (300 MHz, $CD_3OD$) $\delta$ ppm 1.36 - 1.52 (m, 2 H), 1.53-1.61 (m, 2 H), 2.19-2.38 (m, 1 H), 3.40 (dt, *J*=11.7, 2.4 Hz, 2 H), 3.88 ( s, 3 H), 3.92-4.00 (m, 2 H), 4.19 (d, *J*=7.1 Hz, 2 H), 7.10 (d, *J*=8.8 Hz, 1 H), 7.44 (dd *J*=9.0, 2.9 Hz, 1 H), 7.55 (s, 1 H), 7.91 (d, *J*=2.7 Hz, 1 H); MS ($DCI/NH_3$) m/z 401 $(M+H)^+$.

Example 244

N-[(2Z)-5-tert-butyl)-3-[(3-methyl-4,5-dihydroisoxazol-5-yl)methyl]-1,3-thiazol-2(3H)-ylidene]-5-chloro-2-methoxybenzamide

Example 244A

N-(5-tert-butylthiazol-2-yl)-5-chloro-2-methoxybenzamide

To a solution of Example 202A (0.94 g, 6.0 mmol) in tetrahydrofuran (40 mL) was added Example 205B (1.23 g, 6.0 mmol), triethylamine (2.4 mL, 18 mmol), and 4-dimethylaminopyridine (7.5 mg, 0.06 mmol). The reaction mixture was stirred at 60°C for 14 hours and then cooled to ambient temperature, diluted with saturated aqueous $NaHCO_3$ (20 mL) and extracted with ethyl acetate (3 x 30 mL). The combined organic extracts were dried over anhydrous $Na_2SO_4$, filtered and concentrated under reduced pressure. The residue was purified by column chromatography using an Analogix® Intelliflash280 ™ ($SiO_2$, 0-100 % ethyl acetate in hexanes) to afford the title compound. MS ($ESI^+$) m/z 325 $(M+H)^+$

Example 244B

N-[(2Z)-3-allyl-5-tert-butyl-1,3-thiazol-2(3H)-ylidene]-5-chloro-2-methoxybenzamide

To a solution of Example 244A (410 mg, 1.3 mmol) in 5 mL of tetrahydrofuran:N,N-dimethylformamide (4/1) at 0 °C was added potassium *tert*-butoxide (230 mg, 1.9 mmol). The reaction mixture was stirred for 1 hour then allyl bromide (0.16 mL, 1.9 mmol) was added. The mixture was wanned to 65 °C and stirred overnight. The mixture was cooled to ambient temperature, concentrated, diluted with $CH_2Cl_2$ and washed with water and brine. The organic layer

was dried over magnesium sulfate, filtered, and concentrated under reduced pressure. Purification by chromatography (20-50% ethyl ecetate/hexane gradient) afforded the title compound. $^1$H NMR ($CDCl_3$, 300 MHz) $\delta$ ppm 1.34 (s, 9 H), 3.90 (s, 3 H), 4.78 - 4.85 (m, 3 H), 5.22 - 5.36 (m, 2 H), 5.90 - 6.09 (m, *J*=17.0, 10.17 Hz, 1 H), 6.62 (s, 1 H), 6.90 (d, *J*=9.2 Hz, I H), 7.32 (dd, *J*=8.8, 3.1 Hz, I H), 8.00 (d, *J*=2.7 Hz, I H); MS ($DCI/NH_3$) m/z 365 $(M+H)^+$.

Example 244C

N-[(2Z)-5-tert-butyl-3-[(3-methyl-4,5_dihydroisoxazol-5-yl)methyl]-1,3-thiazol-2(3H)-ylidene]-5-chloro-2-methoxybenzamide

Example 244B was processed according to the method of Example 227C to afford the title compound. MS ($DCI/NH_3$) m/z 422 $(M+H)^+$.

Example 245

N-[(2Z)-5-tert-butyl-3-[(2R)-tetrahydrofuran-2-ylmethyl]-1,3-thiazol-2(3H)-ylidene]-2,2-dimethyl-4-oxo-3,4-dihydro-2H-pyran-6-carboxamide

To a solution of the product from Example 240C (300 mg, 0.94 mmol) and 2,2-dimethyl-4-oxo-3,4-dihydro-2*H*-pyran-6-carboxylic acid (180 mg, 1.0 mmol) in 5 mL of N,N-dimethylformamide were added 1- hydroxybenzotriazole hydrate (190 mg, 1.4 mmol), triethylamine (0.30 mL, 2.1 mmol), and 1-ethyl-3-[3-(dimethylamino)propyl]-carbodiimide hydrochloride (260 mg, 1.4 mmol). The mixture was warmed to 65 °C and stirred overnight. The mixture was cooled to ambient temperature, diluted with $CH_2Cl_2$ and washed with water and brine. The organic extract was dried ($MgSO_4$), filtered, and concentrated under reduced pressure. Purification by column chromatography ($SiO_2$, 20-40% ethyl acetate/hexanes gradient) afforded the title compound. $^1$H NMR (500 MHz, $CDCl_3$) $\delta$ ppm 1.34 (s, 9H), 1.57 (s, 6H), 1.72 - 1.84, (m, I H), 1.83 - 1.94 (m, I H), 2.00 - 2.12 (m, 2H), 2.55 (s, 2H), 3.73 - 3.88 (m, 2H), 4.10 - 4.27 (m, 2H), 4.39 (dd, *J*=13.7, 2.8 Hz, 1H), 6.48 (s, 1H), 6.91 (s, 1H); MS ($DCI/NH_3$) m/z 393 $(M+H)^+$.

Example 246

N-[(2Z)-5-tert-butyl-3-(tetrahydro-2H-pyran-4-ylmethyl)-1,3-thiazol-2(3H)-ylidene]-5-chloro-2-methoxybenzamide

To a solution of Example 244A (1.0 g, 3.1mmol) in 4:1 N,N-dimethylfornamide/tetrahydrofuran (20 mL) were added potassium tert-butoxide (Aldrich, 0.42 g, 3.7 mmol) and 4-(iodomethyl)tetrahydro-2H-pyran (Maybridge, 0.97 g, 4.3 mmol). The reaction mixture was stirred at 80 °C for 16 hours, cooled to room temperature, quenched with saturated aqueous $NaHCO_3$ (20 mL) and extracted with ethyl acetate (3 x 20 mL). The combined organic extracts were dried over anhydrous $Na_2SO_4$, filtered and concentrated under reduced pressure. The residue was purified by column chromatography using an Analogix® Intelliflash280 ™ ($SiO_2$, 0-100 % ethyl acetate in hexanes) to afford the title compound. $^1$H NMR (300 MHz, dimethylsulfoxide-$d_6$) $\delta$ ppm 1.21 - 1.51 (m, 4 H), 1.32 (s, 9 H), 2.06 - 2.35 (m, I H), 3.20 - 3.30 (m, 2 H), 3.79 (s, 3 H), 3.80 - 3.91 (m, *J*=9.3, 2.2,2.0 Hz, 2 H), 4.06 (d, *J*=7.1 Hz, 2 H), 7.11 (d, *J*=8.8 Hz, 1 H), 7.30 (s, 1 H), 7.45 (dd, *J*=8.8, 3.1 Hz, 1 H), 7.64 (d, *J*=2.7 Hz, I H); MS ($ESI^+$) m/z 423 $(M+H)^+$; Anal. Calculated for $C_{21}H_{17}ClN_2O_3S$: C, 59.63; H, 6.43; N, 6.62. Found: C, 59.66; H, 6.36; N, 6.56.

Example 247

5-chloro-2-methoxy-*N*-[(2Z)-3-(tetrahydro-2*H*-pyran-4-ylmethyl)-4,5,6,7-tetrahydro-1,3-benzothiazol-2(3*H*)-ylidene] benzamide

Example 247A

5-chloro-2-methoxy-N-(4,5,6,7-tetrahydrobenzo[d]thiazol-2-yl)benzamide

Commercially available 4,5,6,7-tetrahydrobenzo[d]thiazol-2-amine (Aldrich) and 5-chloro-2-methoxybenzoic acid (Aldrich) were processed using the method described in Example 58 to afford the title compound. MS ($ESI^+$) m/z 323 $(M+H)^+$

Example 247B

5-chloro-2-methoxy-*N*-[(2Z)-3-(tetrahydro-2*H*-pyran-4-ylmethyl)-4,5,6,7-tetrahydro-1,3-benzothiazol-2(3*H*)-ylidene] benzamide

Example 203A and Example 247A were processed using the method described in Example 246 to afford the title compound. $^1$H NMR (300 MHz, dimethylsulfoxide-$d_6$) $\delta$ ppm 1.25 - 1.55 (m, 4 H), 1.69 - 1.94 (m, 4 H), 2.07-2.30 (m, 1 H), 2.52 - 2.59 (m, 2 H), 2.58-2.66 (m 2 H), 3.18-3.30 (m, 2 H), 3.80 (s, 3 H), 3.81 - 3.89 (m, 2 H), 4.03 (d,*J*=7.1 Hz, 2 H), 7.11 (d, *J*=8.8 Hz, I H), 7.45 (dd, *J*=8.8, 2.7 Hz, 1 H), 7.69 (d, *J*=3.1 Hz, 1 H); MS (ESI$^+$) m/z 421 (M+H)$^+$.

Example 248

5-chloro-2-methoxy-N-[(2Z)-3-(2-methoxyethyl)-4,4,6,6-tetramethyl-4,6-dihydrofuro[3,4-d][1,3]thiazol-2(3H)-ylidene] benzamide

Example 248A

4-bromo-2,2,5,5-tetramethyldihydrofuran-3(2H)-on

To a solution of commercially available 2,2,5,5-tetramethyldihydrofuran-3(2H)-one (Aldrich, 10.0 g, 0.07 mol) in $CH_2Cl_2$ (100 mL) was added bromine (Aldrich, 3.6 mL, 0.07 mol,) dropwise at room temperature. The reaction mixture was stirred for 2 hours over which time the reaction mixture became clear. Then, the reaction mixture was cooled to 0 °C, quenched with $NaHCO_3$ powder in small portions, filtered and concentrated under reduced pressure to afford the title compound. $^1$H NMR (300 MHz, dimethylsulfoxide-$d_6$) $\delta$ ppm 1.26 (s, 3 H), 1.27 (s, 3 H), 1.30 (s, 3 H), 1.39 (s, 3 H), 5.22 (s, 1 H).

Example 248B

4.4,6,6-tetramethyl-4,6-dihydrofuro[3,4-d]thiazol-2-amine

To a solution of Example 248A (10.0 g, 0.045 mol) in ethanol (100 mL) were added thiourea (3.8 g, 0.05 mol) and triethylamine (6.3 mL, 0.045 mol). The reaction mixture was heated at reflux overnight, then cooled, and concentrated under reduced pressure. The residue was diluted with water (25 mL) and extracted with ethyl acetate (3x50 mL). The combined organic extracts were dried ($Na_2SO_4$), filtered and concentrated. The residue was purified by column chromatography ($SiO_2$, 0-5 % methanol in $CH_2Cl_2$) to afford the title compound. MS (ESI$^+$) m/z 199 (M+H)$^+$.

Example 248C

5-chloro-methoxy-N-(4,4,6,6-tetramethyl-4,6-dihydrofuro[3,4-d]thiazol-2-yl)benzamide

Example 248B and Example 205B were processed using the method described in Example 244A to afford the title compound. MS (ESI$^+$) m/z 367 (M+H)$^+$.

Example 248D

5-chloro-2-methoxy-N-[(2Z)-3-(2-methoxyethyl)-4,4,6,6-tetramethyl-4,6-dihydrofuro[3,4-d][1,3]thiazol-2(3H)-ylidene] benzamide

Example 248C and commercially available 2-bromoethyl methyl ether (Aldrich) were processed using the method described in Example 246 to afford the title compound. $^1$H NMR (300 MHz, dimethylsulfoxide-$d_6$) $\delta$ ppm 1.48 (s, 6 H), 1.55 (s, 6 H), 3.25 (s, 3 H), 3.71 - 3.90 (m, 2 H), 3.81 (s, 3 H), 4.20 (t, *J*=5.6 Hz, 2 H), 7.13 (d, *J*=9.2 Hz, I H), 7.48 (dd, *J*=9.0, 2.9 Hz, 1 H), 7.68 (d, *J*=2.7 Hz, 1 H); MS (ESI$^+$) m/z 425 (M+H)$^+$

Example 249

5-chloro-2-methox-N-[(2Z)-3-(2-methoxyethyl)-6,6-dimethyl-4-oxo-4,6-dihydrofuro[3,4-d][1,3]thiazol-2(3H)-ylidene] benzamide

Example 249A

tert-butyl 6,6-dimethyl-4-oxo-4,6-dihydrofuro[3,4-d]thiazol-2-ylcarbamate

To a solution of diisopropylamine (23.5 mL, 165 mmol) in tetrahydrofuran (200 mL) was added butyllithium (103 mL, 1.6 M in hexanes, 165 mmol) dropwise at -78 °C. The solution was stirred at -78 °C for 30 minutes then transferred via cannula into a solution of methyl 2-(tert-butoxycarbonylamino)thiazole-4-carboxylate (Combi-Blocks, 14.2 g, 55 mmol) in tetrahydrofuran (300 mL) at -78 °C. After stirring at -78 °C for 30 minutes, dry acetone (Acros, 16.2 mL, 220 mmol) was added dropwise and the reaction mixture was allowed to warm to room temperature and stirred overnight. The reaction mixture was quenched with saturated aqueous $NH_4Cl$ solution (200 mL) and the aqueous layer was extracted with ethyl acetate (4 x 200 mL). The combined organic extracts were dried ($Na_2SO_4$), filtered and concentrated. The residue was purified by column chromatography ($SiO_2$, 0-100 % ethyl acetate in hexanes) to afford the title compound. MS ($ESI^+$) m/z 285 $(M+H)^+$.

Example 249B

2-amino-6,6-dimethylfuro[3,4-d]thiazol-4(6H)-one

To a solution of Example 249A (7.4 g, 26.0 mmol) in $CH_2Cl_2$ (20 mL) was added trifluoroacetic acid (20.0 mL, 260 mmol) slowly at 0 °C. The reaction mixture was stirred at room temperature for 3 hours, and then concentrated under vacuum. The residue was diluted with ethyl acetate (100 mL) and neutralized with saturated aqueous $NaHCO_3$ solution. The layers were separated and the aqueous phase was extracted with ethyl acetate (5 x 100 mL). The combined organic extracts were dried ($Na_2SO_4$), filtered and concentrated to afford the title compound. MS ($ESI^+$) m/z 185 $(M+H)^+$

Example 249C

2-imino-3-(2-methoxyethyl)-6,6-dimethyl-2,3-dihydrofuro[3,4-d]thiazol-4(6H)-one hydrobromide

A mixture of Example 249B and commercially available 2-bromoethyl methyl ether (Aldrich) was processed at 120 °C using the method described in Example 12A to afford the title compound. MS ($ESI^+$) m/z 243 $(M+H)^+$

Example 249D

5-chloro-2-methoxy-N-[(2Z)-3-(2-methoxyethyl)-6,6-dimethyl-4-oxo-4,6-dihydrofuro[3,4-d][1-3]thiazol-2(3H)-ylidene] benzamide

Example 249C and Example 205B were processed using the method described in Example 244A to afford the title compound. $^1H$ NMR (300 MHz, dimethylsulfoxide-$d_6$) $\delta$ ppm 1.71 (s, 6 H), 3.26 (s, 3 H), 3.78 (t, $J$=5.6 Hz, 2 H), 3.82 (s, 3 H), 4.46 (t, $J$=5.6 Hz, 2 H), 7.17 (d, $J$=8.8 Hz, I H), 7.53 (dd, $J$=9.0, 2.9 Hz, I H), 7.77 (d, $J$=2.7 Hz, 1 H)); MS ($ESI^+$) m/z 411 $(M+H)^+$; Anal. Calculated for $C_{18}H_{19}ClN_2O_5S$: C, 52.62; H, 4.66; N, 6.82. Found: C, 52.72; H, 4.49; N, 6.90.

Example 250

N-[(2Z)-5-acetyl-4-methyl-3-(oxetan-2-ylmethyl)-1,3-thiazol-2(3H)-ylidene]-5-chloro-2-methoxybenzamide

Example 238A and Example 211A were processed using the method described in Example 246 to afford the title compound. $^1H$ NMR (300 MHz, $CDCl_3$) $\delta$ ppm 2.49 (s, 3 H), 2.54 - 2.67 (m, I H), 2.74 - 2.82 (m, I H), 2.84 (s, 3 H), 3.93 (s, 3 H), 4.45 - 4.74 (m, 4 H), 5.30 (d, I H), 6.93 (d, $J$=9.2 Hz, 1 H), 7.38 (dd, $J$=8.8, 2.7 Hz, 1 H), 7.97 (d, $J$=2.7 Hz, 1 H); MS ($ESI^+$) m/z 493 $(M+H)^+$; Anal. Calculated for $C_{18}H_{19}ClN_2O4S$: C, 54.75; H, 4.85; N, 7.09. Found: C, 54.68; H, 4.70; N, 7.07.

Example 251

5-chloro-N-[(2Z)-4,4-dimethyl-1-(oxetan-2-ylmethyl)-1,4,6,7-tetrahydro-2H-pyrano[4,3-d][1,3]thiazol-2-ylidene]-2-methoxybenzamide

Example 251A

ethyl 2-(2-(tert-butoxycarbonylamino)thiazol-4-yl)acetate

The title compound was obtained from commercially available ethyl 2-(2-aminothiazol-4-yl)acetate (Aldrich) as per the procedure described in JP 06345736. MS ($ESI^+$) m/z 287 $(M+H)^+$.

Example 251 B

tert-butyl 4-(2-hydroxyethylthiazol-2-ylcarbamate

To a cooled solution of Example 251 A in tetrahydrofuran (100 mL) was added lithium borohydride (Aldrich, 100 mL, 2 M solution in tetrahydrofuran) at 0 °C. The reaction mixture was heated at reflux overnight, then cooled to 0 °C, quenched with water and extracted with ethyl acetate (3x100 mL). The combined organic extracts were dried ($Na_2SO_4$), filtered and concentrated. The residue was purified by column chromatography ($SiO_2$, 0-5 % methanol in $CH_2Cl_2$) to afford the title compound. MS ($ESI^+$) m/z 245 $(M+H)^+$

Example 251C

tert-butyl 4-(2-(tetrahydro-2H-pyran-2-yloxy)ethyl)thiazol-2-ylcarbamate

To a solution of Example 251B (6.3 g, 27.4 mmol) in $CH_2Cl_2$ (100 mL) was added commercially available 3,4-dihydro-2H-pyran (Aldrich, 21 g, 250 mmol) and pyridinium-p-toluenesulfonic acid (Aldrich, 3.5 g, 14.0 mmol). The reaction mixture was stirred overnight at room temperature and was diluted with $CH_2Cl_2$, washed with water, dried ($Na_2SO_4$), filtered and concentrated. The residue was purified by column chromatography ($SiO_2$, 0-100 % ethyl acetate in hexanes) to afford the title compound. MS ($ESI^+$) m/z 329 $(M+H)^+$

Example 251D

tert-butyl 5-(2-hydroxypropan-2-yl)-4-(2-(tetrahydro-2H-pyran-2-yloxy)ethyl)thiazol-2-ylcarbamate

Example 251C, diisopropylamine, butyllithium, and dry acetone (Acros) were processed as described for Example 249A to obtain the title compound. MS ($ESI^+$) m/z 387 $(M+H)^+$.

Example 251E

4,4-dimethyl-6,7-dihydro-4H-pyrano[4,3d]thiazol-2-amine

To a solution of Example 251D (4.6 g, 11 mmol) in tetrahydrofuran was added concentrated HCl (6.9 mL). The reaction mixture was heated at reflux for overnight and then cooled. The mixture was basified with 5N NaOH (17 mL) and extracted with ethyl acetate (3 x 50 mL). The combined organic extracts were dried ($Na_2SO_4$), tittered and concentrated. The residue was purified by column chromatography ($SiO_2$, 0-10% methanol in $CH_2Cl_2$) to afford the title compound. MS ($ESI^+$) m/z 185 $(M+H)^+$.

Example 251F

5-chloro-N-(4,4-dimethyl-6,7-dihydro-4H-pyrano[4,3-d]thiazol-2-yl)-2-methoxybenzamide

Example 251E and Example 205B were processed using the method described in Example 244A to afford the title compound. MS ($ESI^+$) m/z 353 $(M+H)^+$

Examole 251G

5-chloro-N-[(2Z)-4,4-dimethyl-1-(oxetan-2-ylmethyl)-1,4,6,7-tetrahydro-2H-pyrano[4,3-d][1,3]thiazol-2-ylidene]-2-methoxybenzamide

Example 25 F and Example 211A were processed using the method described in Example 246 to afford the title compound. $^1$H NMR (300 MHz, dimethylsulfoxide-$d_6$) δ ppm 1.45 (s, 3 H), 1.46 (s, 3 H), 2.67 - 2.75 (m, 2 H), 2.74 - 2.83 (m, 2 H), 3.79 (s, 3 H), 3.90 - 4.01 (m, 2 H), 4.32 - 4.44 (m, 2 H), 4.45 - 4.58 (m, 2 H), 5.06 (d, 1 H), 7.11 (d, *J*=8.8 Hz, 1 H), 7.45 (dd, *J*=8.8, 2.7 Hz, I H), 7.62 (d, *J*=2.7 Hz, 1 H); MS (ESI$^+$) m/z 423 (M+H)$^+$; Anal. Calculated for $C_{10}H_{23}ClN_2O_4S{\cdot}0.2C_4H_8O_2$: C, 56.71; H, 5.63; N, 6.36. Found: C, 56.33; H, 5.39; N, 6.41.

Example 252

5-chloro-N-{(2Z)-4,4-dimethyl-1-[(2R)-tetrahydrofuran-2-ylmethyl]-1,4,6,7-tetrahydro-2H-pyran[4,3-d][1,3]thiazol-2-ylidene}-2-methoxybenzamide

Example 251F and Example 208A were processed using the method described in Example 246 to afford the title compound. $^1$H NMR (300 MHz, dimethylsulfoxide-$d_6$) δ ppm 1.45 (s, 6 H), 1.61 - 2.04 (m, 4 H), 2.7 (t, *J*=5.8 Hz, 2 H), 3.56 - 3.70 (m, 1 H), 3.73 - 3.84 (m, 1 H), 3.79 (s, 3 H), 3.96 (t, *J*=5.3 Hz, 2 H), 4.00 - 4.05 (m, 1 H), 4.20 - 4.38 (m, 2 H), 7.11 (d, *J*=8.8 Hz, 1 H), 7.46 (dd, *J*=9.0, 2.9 Hz, 1 H), 7.66 (d, *J*=3.1 Hz, I H); MS (ESI$^+$) m/z 437 (M+H)$^+$; Anal. Calculated for $C_{21}H_{25}ClN_2O_4S$: C, 57.72; H, 5.77; N, 6.41. Found: C, 57.58; H, 5.86; N, 6.33.

Example 253

N-[(2Z)-5-acetyl-4-methyl-3-(tetrahydro-2H-pyran-2-ylmethyl)-1,3-thiazol-2(3H)-ylidene]-5-chloro-2-methoxybenzamide

Commercially available 2-(bromomethyl)tetrahydro-2H-pyran (Aldrich) and Example 238A were processed using the method described in Example 246 to afford the title compound. $^1$H NMR (300 MHz, dimethylsulfoxide-$d_6$) δ ppm 1.28 - 1.38 (m, 1 H), 1.41 - 1.52 (m, 3 H), 1.62 - 1.71 (m, 1 H), 1.78 - 1.88 (m, I H), 2.50 (s, 3 H), 2.61 - 2.84 (m, 3 H), 3.64-3.93 (m, 3 H), 3.83 (s, 3 H), 4.14 (dd, *J*=14.1, 8.6 Hz, 1 H), 4.37 (dd, *J*=14.2, 3.1 Hz, 1 H), 7.16 (d, *J*=8.8 Hz, 1 H), 7.51 (dd, *J*=9.0, 2.9 Hz, 1 H), 7.79 (d, *J*=2.7 Hz, 1 H); MS (ESI$^+$) m/z 423 (M+H)$^+$; Anal. Calculated for $C_{20}H_{23}ClN_2O_4S$ : C, 56.80; H, 5.48; N, 6.62. Found: C, 56.53; H, 5.27; N, 6.55.

Example 254

N-[(2Z)-5-acetyl-4-methyl-3-(tetrahydro-2H-pyran-3-ylmethyl)-1,3-thiazol-2(3H)-ylidene]-5-chloro-2-methoxybenzamide

Example 225A and Example 238A were processed using the method described in Example 246 to afford the title compound. $^1$H NMR (300 MHz, dimethylsulfoxide-$d_6$) δ ppm 1.47 (s, 6 H) 1.52 (s, 6 H) 3.72 (s, 3 H) 5.35 (s, 2 H) 7.07 (d, 3 H) 7.32 (d, 1 H) 7.37 (d, *J*=2.71 Hz, I H) 7.40 - 7.50 (m, *J*=8.81 Hz, 1 H); MS (ESI$^+$) m/z 423 (M+H)$^+$; Anal. Calculated for $C_{20}H_{23}ClN_2O_4S{\cdot}0.2H_2O$: C, 56.32; H, 5.53; N, 6.57. Found: C, 56.19; H, 5.50; N, 6.62.

Example 255

5-chloro-N-[(2Z)-5-(1-hydroxy-1-methylethyl)-4-methyl-3-(tetrahydro-2H-pyran-2-ylmethyl)-1,3-thiazol-2(3H)-ylidene]-2-methoxybenzamide

A solution of methyllithium (Aldrich, 1.6 M in diethyl ether, 0.41 mL, 0.66 mmol) was added slowly a solution of Example 253 (0.14 g, 0.33 mmol) in tetrahydrofuran (3 mL) at -78 °C. The reaction mixture was stirred at -78 °C for 30 minutes and was allowed to reach room temperature. The reaction mixture was quenched with water (6 mL) and extracted with ethyl acetate (2x10 mL). The combined organic extracts were dried over anhydrous $Na_2SO_4$, filtered and concentrated under reduced pressure. The residue was purified by column chromatography using an Analogic® Intelliflash280™ ($SiO_2$, 0-5 % methanol in $CH_2Cl_2$) to afford the title compound. $^1$H NMR (300 MHz, dimethylsulfoxide-$d_6$) δ ppm 1.21-1.36 (m, 1 H), 1.41 - 1.48 (m, 3 H), 1.49 (s, 3 H), 1.50 (s, 3 H), 1.57-1.67 (m, 1 H), 1.74-1.85 (m, 1H), 2.42 (s, 3 H), 3.68-3.89 (m, 3 H), 3.79 (s, 3 H), 3.92 - 4.09 (m, 1 H), 4.17-4.38 (m, 1 H), s.60 (s, 1H), 7.10 (d, *J*=8.8 Hz, 1 H), 7.44 (dd, *J*=8.8,2.7 Hz, 1 H), 7.68 (d, *J*=2.7 Hz, 1 H); MS (ESI$^+$) m/z 439 (M+H)$^+$; Anal. Calculated for $C_{21}H_{27}ClN_2O_4S$: C, 57.46; H, 6.20; N, 6.38. Found: C, 57.44; H, 5.88; N, 6.06.

Example 256

5-chloro-2-methoxy-N-[(2Z)-4-methyl-3-[(2R)-tetrahydrofuran-2-ylmethyl]-5-[2,2,2-trifluoro-1-hydroxy-1-(trifluoromethyl)ethyl]-1,3-thiazol-2(3H)-ylidene]benzamide

Example 256A

2-(2-amino-4-methylthiazol-5-yl)-1,1,1,3,3,3-hexafluoropronan-2-ol

The title compound was prepared from commercially available of 4-methylthiazol-2-amine (Aldrich) and hexafluoroacetone trihydrate (Aldrich) using the procedure described in European Journal of Organic Chemistry, (21), 4286-4291; 2003. MS (ESI$^+$) m/z 281 (M+H)$^+$.

Example 256B

5-chloro-N-(5-(1,1,1,3,3,3-hexafluoro-2-hydroxypropan-2-yl)-4-methylthiazol-2-yl)-2-methoxybenzamide

Commercially available 5-chloro-2-methoxybenzoic acid (Aldrich) and Example 256A were processed using the method described in Example 58 to afford the title compound. MS (ESI$^+$) m/z 449 (M+H)$^+$.

Example 256C

5-chloro-2-methox-N-[(2Z)-4-methyl-3-[(2R)-tetrahydrofuran-2-ylmethyl]-5-[2,2,2-trifluoro-1-hydroxy-1-(trifluoromethyl) ethyl]-1,3-thiazol-2(3H)-ylidene]benzamide

Example 256B and Example 208A were processed using the method described in Example 246 to afford the title compound. $^1$H NMR (300 MHz, dimethylsulfoxide-$d_6$) $\delta$ ppm 1.62 - 1.76 (m, 1 H), 1.78 - 1.93 (m, 2 H), 1.93 - 2.09 (m, 1 H), 2.57 (s, 3 H), 3.57 - 3.70 (m, 1 H), 3.74 - 3.88 (m, 1 H), 3.80 (s, 3 H), 4.12-4.26 (m, 1 H), 4.39 (d, 2 H), 7.14 (d, *J*=8.8 Hz, 1 H), 7.49 (dd, *J*=8.8, 2.7 Hz, 1 H), 7.74 (d, *J*=2.7 Hz, 1 H), 9.34 (s, I H); MS (ESI$^+$) m/z 533 (M+H)$^+$.

Example 257

5-chloro-N-[(2Z)-5-(1-hydroxy-1-methylethyl)-4-methyl-3-(tetrahydro-2H-pyran-3-ylmethyl)-1,3-thiazol-2(3H)-ylidene]-2-methoxybenzamide

Example 254 and commercially available methyllithium (Aldrich, 1.6 M in diethyl ether) were processed using the method described in Example 255 to afford the title compound. $^1$H NMR (300 MHz, dimethylsulfoxide-$d_6$) $\delta$ ppm 1.32 - 1.48 (m, 2 H), 1.51 (s, 6 H), 1.57 - 1.80 (m, 2 H), 2.02 - 2.23 (m, 1 H), 2.41 (s, 3 H), 3.19-3.28 (m, 1 H), 3.35 - 3.43 (m, 1 H), 3.55-3.73 (m, 2 H), 3.79 (s, 3 H), 3.95 - 4.29 (m, 2 H), 5.60 (s, 1 H), 7.1 (d, *J*=8.8 Hz, 1 H), 7.44 (dd, *J*=8.8,3.1 Hz, 1 H), 7.67 (d, *J*=2.7 Hz, I H); MS (ESI$^+$) m/z 439 (M+H)$^+$; Anal. Calculated for $C_{21}H_{27}ClN_2O_4S$: C, 57.46; H, 6.20; N, 6.38. Found: C, 57.14; H, 6.23; N, 6.53.

Example 258

5-chloro-N-[(2Z)-3-{[(4R)-2,2-dimethyl-1,3-dioxolan-4-yl]methyl}-5-(1-hydroxy-1-methylethyl)-4-methyl-1,3-thiazol-2 (3H)-ylidene]-2-methoxybenzamide

Example 258A

5-chloro-N-(5-(2-hydroxypropan-2-yl)-4-methylthiazol-2-yl)-2-methoxybenzamide

Example 238A and methyllithium (Aldrich, 1.6M in diethyl ether) were processed using the method described in Example 255 to afford the title compound. MS (ESI$^+$) m/z 341 (M+H)$^+$.

Example 258B

5-chloro-N-[(2Z)-3-{(4R)-2,2-dimethyl-1,3-dioxolan-4-yl]methyl}-5-(1-hydroxy-1-methylethyl)-4-methyl-1,3-thiazol-2 (3H)-ylidene]-2-methoxybenzamide

Example 258A and commercially available (S)-(2,2-dimethyl-1,3-dioxolan-4-yl)methyl 4-methylbenzenesulfonate (Aldrich) were processed using the method described in Example 246 to afford the title compound. $^1H$ NMR (300 MHz, dimethylsulfoxide-$d_6$) $\delta$ ppm 1.25 (s, 3 H), 1.31 (s, 3 H), 1.50 (s, 6 H), 2.45 (s, 3 H), 3.78 (s, 3 H), 3.84 (dd, *J*=8.8, 6. Hz, 1 H), 4.04 (dd, *J*=8.5, 6.4 Hz, 1 H), 4.18 - 4.29 (m, 1 H), 4.32 - 4.42 (m, 1 H), 4.46 - 4.58 (m, 1 H), 5.63 (s, 1 H), 7.10 (d, *J*=8.8 Hz, 1 H), 7.43 (dd, *J*=8.8, 2.7 Hz, 1 H), 7.64 (d, *J*=2.7 Hz, 1 H); MS (ESI$^+$) m/z 455 (M+H)$^+$; Anal. Calculated for $C_{21}H_{27}ClN_2O_5S$: C, 55.44; H, 5.98; N, 6.16. Found: C, 55.34; H, 5.79; N, 6.21.

Example 259

5-chloro-N-[(2Z)-6,6-dimethyl-4-oxo-3-[(2R)-tetrahydrofuran-2-ymethyl]-4,6-dihydrofuro[3,4-d][1-3]thiazol-2(3H)-ylidene]-2-methoxybenzamide

Example 259A

5-chloro-N-(6,6-dimethyl-4-oxo-4,6-dihydrofuro[3,4-d]thiazol-2-yl)-2-methoxybenzamide

Example 249B, triethylamine, 4-dimethylaminopyridine, and Example 205B were processed as described for Example 244A to obtain the title compound. MS (ESI$^+$) m/z 353 (M+H)$^+$.

Example 259B

5-chloro-N-[(2Z)-6,6-dimethyl-4-oxo-3-[(2R)-tetrahydrofuran-2-ylmethyl]-4,6-dihydrofuro[3,4-d][1,3]thiazol-2(3H)-ylidene]-2-methoxybenzamide

Example 259A and Example 208A were processed using the method described in Example 246 to afford the title compound. $^1H$ NMR (300 MHz, dimethylsulfoxide-$d_6$) $\delta$ ppm 1.71 (s, 6 H), 1.73 - 2.05 (m, 4 H), 3.60-3.69 (m, 1 H), 3.72-3.81 (m, 1 H), 3.82 (s, 3 H), 4.18 - 4.35 (m, 2 H), 4.35 - 4.47 (m, 1 H), 7.17 (d, *J*=8.8 Hz, 1 H), 7.53 (dd, *J*=8.8, 2.7 Hz, 1 H), 7.76 (d, *J*=2.7 Hz, 1 H); MS (ESI$^+$) m/z 437 (M+H)$^+$; Anal. Calculated for $C_{20}H_{21}ClN_2O_5S \cdot 0.4H_2O$: C, 54.09; H, 4.95; N, 6.31. Found: C, 53.81; H, 4.55; N, 5.99.

Example 260

5-chloro-N-[(2Z)-3-{(4S)2,2-dimethyl-1,3-dioxolan-4-yl]methyl}-5-(1-hydroxy-1-methylethyl)-4-methyl-1,3-thiazol-2 (3H)-ylidene]-2-methoxybenzamide

Example 258A and commercially available (R)-(2,2-dimethyl-1,3-dioxolan-4-yl)methyl 4-methylbenzensulfonate (Aldrich) were processed using the method described in Example 246 to afford the title compound. $^1H$ NMR (300 MHz, dimethylsulfoxide-$d_6$) $\delta$ ppm 1.25 (s, 3 H), 1.31 (s, 3 H), 1.50 (s, 6 H), 2.45 (s, 3 H), 3.78 (s, 3 H), 3.84 (dd, *J*=8.5, 6.1 Hz, 1 H), 4.04 (dd, *J*=8.8, 6.4 Hz, 1 H), 4.14 - 4.30 (m, 1 H), 4.32 - 4.43 (m, 1 H), 4.43 - 4.63 (m, 1 H), 5.63 (s, 1 H), 7.10 (d, *J*=9.2 Hz, 1 H), 7.43 (dd, *J*=9.0, 2.9 Hz, 1 H), 7.64 (d, *J*=2.7 Hz, 1 H); MS (ESI$^+$) m/z 455 (M+H)$^+$; Anal. Calculated for $C_{21}H_{27}ClN_2O_5S$: C, 55.44; H, 5.98; N, 6.16. Found: C, 55.73; H, 6.07; N, 6.07.

Example 261

N-[(2Z)-5-acetyl-3-(1,4-dioxan-2-ylmethyl)-4-methyl-1,3-thiazol-2(3H)-ylidene]-5-chloro-2-methoxybenzamide

Example 238A and commercially available 2-(iodomethyl)-1,4-dioxane (Synchem) were processed using the method described in Example 246 to afford the title compound. $^1H$ NMR (300 MHz, dimethylsulfoxide-$d_6$) $\delta$ ppm 2.50 (s, 3 H), 2.72 (s, 3 H), 3.33 - 3.42 (m, 1 H), 3.45 - 3.56 (m, 2 H), 3.59 - 3.77 (m, 2 H), 3.78-3.87 (m, 1 H), 3.83 (s, 3 H), 4.00 - 4.11 (m, 1 H), 4.12-4.25 (m, 1 H), 4.31 - 4.43 (m, 1 H), 7.16 (d, J=8.8 Hz, 1 H), 7.52 (dd, J=8.8, 3.1 Hz, 1 H), 7.79 (d, *J*=2.7 Hz, 1 H); MS (ESI$^+$) m/z 425 (M+H)$^+$; Anal. Calculated for $C_{19}H_{21}ClN_2O_5S$: C, 53.71; H, 4.98; N, 6.59. Found: C, 53.32; H, 4.73; N, 6.59.

Example 262

5-chloro-N-[(2Z)-5-(1hydroxy-1-methylethyl)-4-methyl-3-(oxetan-2-ylmethyl)-1,3-thiazol-2(3H)-ylidene]-2-methoxybenzamide

Example 258A and Example 211A were processed using the method described in Example 246 to afford the title compound. $^1$H NMR (300 MHz, dimethylsulfoxide-$d_6$) δ ppm 1.50 (s, 6 H), 2.47 (s, 3 H), 2.59 - 2.84 (m, 1 H), 3.78 (s, 3 H), 4.25 - 4.64 (m, 5 H), 5.00 - 5.19 (m, 1 H), 5.63 (s, 1 H), 7.09 (d, *J*=9_2 Hz, 1 H), 7.43 (dd, *J*=8.8, 3.1 Hz, 1 H), 7.60 (d, *J*=2.7 Hz, 1 H); MS (ESI$^+$) m/z 411 (M+H)$^+$; Anal. Calculated for $C_{19}H_{23}ClN_2O_4S$: C, 55.54; H, 5.64; N, 6.82. Found: C, 55.41; H, 5.51;

N, 6.78.

Example 263

5-chloro-N-[(2Z)-5-(1-hydroxy-1-methylethyl)-4-methyl-3-(tetrahydrofuran-3-ylmethyl)-1,3-thiazol-2(3H)-ylidene]-2-methoxybenzamide

Example 258A and Example 162A were processed using the method described in Example 246 to afford the title compound. $^1$H NMR (300 MHz, dimethylsulfoxide-$d_6$) δ ppm 1.51 (s, 6 H), 1.65 - 1.82 (m, 1 H), 1.84 - 2.03 (m, I H), 2.43 (s, 3 H), 2.68 - 2.89 (m, 1 H), 3.48 - 3.71 (m, 3 H), 3.78 (s, 3 H), 3.80 - 3.93 (m, I H), 4.08 - 4.34 (m, 2 H), 5.62 (s, 1 H), 7.09 (d, *J*=8.8 Hz, 1 H), 7.43 (dd, *J*=8.8, 3. 1 Hz, 1 H), 7.65 (d, *J*=2.7 Hz, 1 H); MS (ESI$^+$) m/z 425 (M+H)$^+$; Anal. Calculated for $C_{20}H_{25}ClN_2O4S$: C, 56.53; H, 5.93; N, 6.59. Found: C, 56.35; H, 5.55; N, 6.56.

Example 264

5-chloro-N-[(2Z)-3-(1,4-dioxan-2-ylmethyl)-5-(1-hydroxy-1-methylethyl)-4-methyl-1,3-thiazol-2(3H)-ylidene]-2-methoxybenzamide

Example 258A and commercially available 2-(iodomethyl)-1,4-dioxane (Synchein) were processed using the method described in Example 246 to afford the title compound. $^1$H NMR (300 MHz, dimethylsulfoxide-$d_6$) δ ppm 0.93 (t, *J*=7.46 Hz, 3 H) 1.28 - 1.38 (m, 2 H) 1.45 (s, 6 H) 1.63 - 1.78 (m, 2 H) 2.69 (t, *J*=5.42 Hz, 2 H) 3.79 (s, 3 H) 3.97 (t, *J*=5.42 Hz, 2 H) 4.07 - 4.17 (m, 2 H) 7.11 (d, *J*=8.81 Hz, 1 H) 7.46 (dd, *J*=8.81, 2.71 Hz, 1 H) 7.69 (d, *J*=2.71 Hz, 1 H); LCMS (ESI$^+$) m/z 441 (M+H)$^+$; Anal. Calculated for $C_{20}H_{25}ClN_2O_5S$: C, 54.48; H, 5.71; N, 6.35. Found: C, 54.54; H, 5.38; N, 6.43.

Example 265

N-[(2Z)-5-tert-butyl-3-((2R)-tetrahydrofuran-2-ylmethyl]-1,3-thiazol-2(3H)-ylidene]-3-methoxy-2-naphthamide

Commercially available 3-methoxy-2-naphthoic acid (Aldrich) and Example 240C were processed using the method described in Example 240E to afford the title compound. $^1$H NMR (300 MHz, dimethylsulfoxide-$d_6$) δ ppm 1.32 (s, 9 H), 1.58 - 1.74 (m, 1 H), 1.75 - 1.88 (m, 2 H), 1.88 - 1.97 (m, 1 H), 3.60 - 3.71 (m, 1 H), 3.74 - 3.85 (m, I H), 3.88 (s, 3 H), 4.15 - 4.26 (m, 2 H), 4.27 - 4.41 (m, 1 H), 7.24 (s, 1 H), 7.31 - 7.43 (m, 2 H), 7.45 - 7.55 (m, I H), 7.86 (dd, *J*=13.6, 8.5 Hz, 2 H), 8.12 (s, I H); MS (ESI$^+$) m/z 425 (M+H)$^+$; Anal. Calculated for $C_{20}H_{23}ClN_2O_4S{\cdot}0.2C_4H_8O_2{\cdot}0.2H_2O$: C, 66.82; H, 6.78; N, 6.28. Found: C, 66.70; H, 6.65; N, 6.33.

Example 266

N-[(2Z)-S-tert-butyl-3-[(3-methyloxetan-3-yl)methyl]-1,3-thiazol-2(3H)-ylidene]-5-chloro-2-methoxybenzamide

To a solution of Example 244A (0.75g, 2.31 mmol) in N,N-dimethylformamide/tetrahydrofuran (1:4, 20 mL) were added potassium tert-butoxide (0.39 g, 3.46 mmol), tetrabutylammoniwn iodide (0.09 mg, 0.23 mmol) and commercially available 3-(chloromethyl)-3-methyloxetane (TCI, 0.28 g, 2.31 mmol). The reaction mixture was stirred at 80 °C for 16 hours, cooled, diluted with ethyl acetate (20 mL) and quenched with saturated aqueous $NaHCO_3$ (20 mL). The aqueous layer was extracted with ethyl acetate (2 x 20 mL). The combined organic layers were washed with water (1 x 25 mL). The combined organic extracts were dried over anhydrous $Na_2SO_4$, filtered and concentrated under reduced pressure. The residue was purified by column chromatography using an Analogix® Intelliflash280™ the ($SiO_2$, 0-100 % ethyl acetate in hexanes) to afford the title compound. $^1$H NMR (300 MHz, dimethylsulfoxide-$d_6$) δ ppm 1.24 (s, 3 H), 1.33 (s, 9 H), 3.77 (s, 3 H), 4.19 (d, *J*=6.1 Hz, 2 H), 4.38 (s, 2 H), 4.69 (d, *J*=6.1 Hz, 2 H), 7.10 (d, *J*=9.2 Hz, 1 H), 7.29 (s, 1 H), 7.44 (dd, *J*=9.0, 2.9 Hz, I H), 7.58 (d, *J*=2.7 Hz, 1 H); MS (ESI$^+$) m/z 409 (M+H)$^+$; Anal. Calculated for

$C_{20}H_{25}ClN_2O_3S$: C, 58.74; H, 6.16; N, 6.85. Found: C, 58.92; H, 6.04; N, 6.84.

Example 267

5-chloro-2-methoxy-N-[(2Z)-3-(tetrahydrofuran-2-ylmethyl)-3,4,5,6-tetrahydro-2H-cyclopenta[d][1,3]thiazol-2-ylidene] benzamide

Example 267A

5,6-hhydro-4H-cyclopenta[d]thiazol-2-amine

A mixture of 2-chlorocyclopentanone (5.0 g, 40 mmol) and thiourea (3.0 g, 40 mmol) was heated at 70 °C for 3 hours. After cooling, the solid was triturated with ethanol and collected by filtration to afford the title compound. MS (ESI) m/z 141 $(M+H)^+$.

Example 267B

Example 267A and 5-chloro-2-methoxybenzoic acid were processed using the method described in Example 223A to afford the title compound. MS (ESI) m/z 309 $(M+H)^+$.

Example 267C

5-chloro-2-methoxy-N-[(2Z)-3-(tetrahydrofuran-2-ylmethyl)-3,4,5,6-tetrahydro-2H-cyclopenta[d][1,3]thiazol-2-ylidene] benzamide

Example 267B (150mg, 0.49 mmol) in tetrahydrofuran/N,N- dimethylformamide (2:1) (10 mL) was treated with NaH (60%) (24 mg, 0.58 mmol). After 10 minutes, 2-(bromomethyl)tetrahydrofuran (96 mg, 0.58 mmol) was added and the mixture was heated at 95 °C for 12 hours. After cooling to ambient temperature, the mixture was diluted with water, and extracted with ethyl acetate. The organic extract was dried ($Na_2SO_4$), filtered and concentrated. Purification by chromatography afforded the title compound. $^1H$ NMR (400 MHz, dimethylsulfoxide-$d_6$) $\delta$ ppm 1.62 - 1.73 (m, 1 H) 1.79-1.88 (m, 2 H) 1.91 - -2.02(m, I H) 2.35 - 2.44 (m, 2 H) 2.77 - 2.91 (m,4H)3.64 (dd, *J*=14.7, 7.1 Hz, 1 H) 3.74 - 3.81 (m, 1 H) 3.79 (s, 3 H) 4.00 (dd, *J*= 13.5, 8.0 Hz, 1 H) 4.23 (dd, *J*= 13.5, 3.7 Hz, 1 H) 4.26 - 4.33 (m, 1 H) 7.11 (d, *J*=8.9 Hz, 1 H) 7.44 (dd, *J*=8.9, 2.8 Hz, 1 H) 7.68 (d, *J*=2.8 Hz, 1 H); MS (ESI) m/z 393 $(M+H)^+$.

Example 268

5-chloro-2-methoxy-N-[(2Z)-3-(tetrahydro-2H-pyran-4-ylmethyl)-3,4,5,6-tetrahydro-2H-cyclonenta[d][1,3]thiazol-2-ylidene]benzamide

Example 267B and 4-(bromomethyl)tetrahydro-2H-pyran were processed using the method described in Example 267C to afford the title compound. $^1H$ NMR (500 MHz, dimethylsulfoxide-$d_6$) $\delta$ ppm 1.33 (ddd, *J*=24.7, 11.90,4.3 Hz, 2 H) 1.40 - 1.54 (m, 2 H) 1.83 - 1.86 (m, 1 H) 2.15 - 2.26 (m, I H) 2.36 - 2.45 (m, 2 H) 2.82 (dt, *J*=20.8, 6.7 Hz, 4 H) 3.26 (td, *J*=1 1.9, 1.8 Hz, 1 H) 3.80 (s, 3 H) 3.84 (dd, *J*=11.6, 2.4 Hz, 2 H) 3.99 (d, *J*=7.3 Hz, 2 H) 7.11 (d, *J*=8.9 Hz, 1 H) 7.45 (dd, *J*=8.9, 2.8 Hz, 1 H) 7.70 (d, *J*=2.8 Hz, 1 H); MS (ESI) m/z 407 $(M+H)^+$.

Example 269

N-[(2Z)-4,5-dimethyl-3-(tetrahydro-2H-pyran-4-ylmethyl)-1,3-thiazol-2(3H)-ylidene]-2,2-dimethyltetrahydro-2H-pyran-4-carboxamide

Example 269A

A mixture of 4,5-dimethylthiazol-2-amine (Aldrich) and 2,2-dimethyltetrahydro-2H-pyran-4-carboxylic acid (Chembridge Building Block Library) were processed using the method described in Example 223A to afford the title compound. MS (ESI) m/z 269 $(M+H)^+$.

Example 269B

N-[(2Z)-4,5-dimethyl-3-(tetrahydro-2H-pyran-4-ylmethyl)-1,3-thiazol-2(3H)-ylidene]-2,2-dimethyltetrahydro-2H-pyran-4-carboxamide

Example 269A and 4-(bromomethyl)tetrahydro-2H-pyran were processed using the method described in Example 267C to afford the title compound. $^1$H NMR (300 MHz, dimethylsulfoxide *$d_6$*) δ ppm 1.11 - 1.14 (m, 3 H) 1.15 - 1.18 (m, 3 H) 1.30 - 1.43 (m, 4 H) 1.43 - 1.56 (m, 2 H) 1.68 - 1.80 (m, 2 H) 2. 14 - 2. 18 (m, 3 H) 2.19 - 2.23 (m, 3 H) 2.55 - 2.71 (m, 1 H) 3.16 - 3.26 (m, 2 H) 3.35 - 3.41 (m, 1 H) 3.60 (dd, *J*=11.9,2.4 Hz, 1 H) 3.63 - 3.68 (m, 1 H) 3.79 - 3.83 (m, 1 H) 3.83 - 3.87 (m, 1 H) 4.01 - 4.03 (m, 1 H) 4.03 - 4.06 (m, 1 H); MS (ESI) m/z 376 $(M+H)^+$.

Example 270

N-[(2Z)-5-tert-butyl-3-(oxetan-2-ylmethyl)-1,3-thiazol-2(3H)-ylidene]-5-chloro-2-methoxybenzamide

Example 244A and the product from Example 211A were processed using the method described in Example 244B to afford the title compound. $^1$H NMR (400 MHz, $CDCl_3$) δ ppm 1.35 (s, 9 H) 2.39 - 2.56 (m, 1 H) 2.68 - 2.83 (m, 1 H) 3.86 - 3.90 (s, 3 H) 4.34 - 4.38 (m, 1 H) 4.37 - 4.43 (m, 1 H) 4.55 (dd, *J*=14.1, 5.8 Hz, 1 H) 4.62 - 4.68 (m, 1 H) 5.15 - 5.22 (m, 1 H) 6.89 (d, *J*=8.9 Hz, 1 H) 6.93 - 6.94 (m, 1 H) 7.31 (dd, *J*=8.9, 2.8 Hz, 1 H) 7.89 (d, *J*=2.8 Hz, 1 H); MS (ESI) m/z 395 $(M+H)^+$.

Example 271

N-[(2Z)-5-tert-butyl-3-(tetrahydro-2H-pyran-2-ylmethyl)-1,3-thiazol-2(3H)-ylidene]-5-chtoro-2-methoxybenzamide

Example 244A and 2-(bromomethyl)tetrahydro-2H-pyran were processed using the method described in Example 246 to afford the title compound. $^1$H NMR (500 MHz, $CDCl_3$) δ ppm 1.20 - 1.34 (m, 2 H) 1.34 (s, 9 H) 1.51 - 1.55 (m, 2 H) 1.71 (dt, *J*=12.8, 1.8 Hz, 1 H) 1.85 - 1.92 (m, 1 H) 3.39 (td, *J*=14.3, 11.3, 3.4 Hz, 1 H) 3.70 - 3.77 (m, 1 H) 3.90 (s, 3 H) 3.94 - 4.00 (m, 2 H) 4.40 (dd, *J*=14.0, 3.0 Hz, 1 H) 6.78 (s, 1 H) 6.90 (d, *J*=8.9 Hz, 1 H) 7.32 (dd, *J*=8.9, 2.8 Hz, 1 H) 7.95 (d, *J*=2.8 Hz, 1 H); MS (ESI) m/z 423 $(M+H)^+$.

Example 272

N-[(2Z)-5-tert-butyl-3-(1,4-dioxan-2-ylmethyl)-1,3-thiazol-2(3H)-ylidene]-5-chloro-2-methoxybenzamide

Example 244A and 2-(iodomethyl)-1,4-dioxane were processed using the method described in Example 246 to afford the title compound. $^1$H NMR (500 MHz, $CDCl_3$) δ ppm 1.34 (s, 9 H) 3.32 (dd, *J*=11.60, 10.1 Hz, 1 H) 3.52 - 3.62 (m, 1 H) 3.68 - 3.76 (m, 2 H) 3.80 (dd, *J*=11.0, 2.8 Hz, 1 H) 3.85 - 3.91 (m, 1 H) 3.90 (s, 3 H) 4.00 - 4.06 (m, 1 H) 4.05 (dd, *J*=19.5, 6.71 Hz, 1 H) 4.33 (dd, *J*=10.7, 3.1 Hz, 1 H) 6.74 (s, 1 H) 6.90 (d, *J*=8.9 Hz, 1 H) 7.33 (dd, *J*=8.9, 2.8 Hz, 1 H) 7.92 (d, *J*=2.8 Hz, 1 H); MS (ESI) m/z 425 $(M+H)^+$.

Example 273

N-[(2Z)-5-tert-butyl-3-{[(4S)-2,2-dimethyl-1,3-dioxolan-4-yl]methyl}-1,3-thiazol-2(3H)-ylidene]-5-chloro-2-methoxybenzamide

Example 244A and (*R*)-(2,2-dimethyl-1,3-dioxolan-4-yl)methyl 4-methylbenzenesulfonate were processed using the method described in Example 246 to afford the title compound. $^1$H NMR (500 MHz, $CDCl_3$) δ ppm 1.34 (s, 9 H) 1.36 (s, 3 H) 1.37 (s, 3 H) 3.76 (dd, *J*=8.9, 6.7 Hz, 1 H) 3.90 (s, 3 H) 4.11 (dd, *J*=8.9, 6.4 Hz, 1 H) 4.31 - 4.42 (m, 2 H) 4.47 - 4.53 (m, 1 H) 6.80 (s, 1 H) 6.90 (d, J=8.9 Hz, 1 H) 7.32 (dd, *J*=8.9, 2.75 Hz, 1 H) 7.90 (d, *J*=3. Hz, 1 H); MS (ESI) m/z 439 $(M+H)^+$.

Example 274

N-[(2Z)-5-tert-butyl-3-{[(4R)-2,2-dimethyl-1,3-dioxolan-4-yl]methyl}-1,3-thiazol-2(3H)-ylidene]-5-chloro-2-methoxybenzamide

Example 244A and (S)-(2,2-dimethyl-1,3-dioxolan-4-yl)methyl 4-methylbenzenesulfonate were processed using the method described in Example 246 to afford the title compound. $^1$H NMR (500 MHz, $CDCl_3$) δ ppm 1.35 (s, 9 H) 1.36 (s, 3 H) 1.37 (s, 3 H) 3.76 (dd, *J*=8.9, 6.7 Hz, 1 H) 3.90 (s, 3 H) 4.1 (dd, *J*=8.9, 6.4 Hz, 1 H) 4.33 - 4.43 (m, 2 H)

4.48 - 4.54 (m, 1 H) 6.81 (s, 1 H) 6.90 (d, *J*=8.9 Hz, 1 H) 7.33 (dd, *J*=8.9, 2.8 Hz, 1 H) 7.89 (d, J=2.8 Hz, 1 H); MS (ESI) m/z 439 $(M+H)^+$.

Example 275

N-[(2Z)-5-tert-butyl-3-(tetrahydrofuran-3-ylmethyl)-1,3-thiazol-2(3H)-ylidene]-5-chloro-2-methoxybenzamide

Example 244A and the product from Example 162A were processed using the method described in Example 246 to afford the title compound. $^1$H NMR (500 MHz, $CDCl_3$) δ ppm 1.35 (s, 9 H) 1.71 - 1.79 (m, 1 H) 1.98 - 2.10 (m, 1 H) 2.90 - 3.02 (m, 1 H) 3.64 (dd, *J*=9.15, 5.19 Hz, 1 H) 3.76- 3.83 (m, 2 H) 3.90 (s, 3 H) 3.95 - 4.01 (m, 1 H) 4.08 (dd, *J*=13.43, 7.93 Hz, 1 H) 4.24 (dd, *J*=13.12, 7.32 Hz, 1 H) 6.63 (s, 1 H) 6.91 (d, *J*=8.85 Hz, 1 H) 7.33 (dd, *J*=8.85, 3.05 Hz, 1 H) 7.97 (d, *J*=3.05 Hz, 1 H); MS (ESI) m/z 409 $(M+H)^+$.

Example 276

N-[(2Z)-5-tert-butyl-3-(tetrahydro-2H-pyran-3-ylmethyl)-1,3-thiazol-2(3H)-ylidene]-5-chloro-2-methoxybenzamide

Example 244A and the product from Example 225A were processed using the method described in Example 246 to afford the title compound. $^1$H NMR (500 MHz, $CDCl_3$) δ ppm 1.34 (s, 9 H) 1.39 - 1.48 (m, 1 H) 1.56 - 1.67 (m, 1 H) 1.69 - 1.77 (m, 1 H) 1.78 - 1.89 (m, 1 H) 2.23 - 2.34 (m, 1 H) 3.34 (dd, *J*=11.0, 8.2 Hz, 1 H) 3.53 (ddd, *J*=9.2, 3.1 Hz, 1 H) 3.74 - 3.85 (m, 2 H) 3.90 (s, 3 H) 4.04 - 4.16 (m, 2 H) 6.60 (s, 1 H) 6.90 (d, *J*=8.9 Hz, 1 H) 7.33 (dd, *J*=8.9, 2.75 Hz, 1 H) 7.98 (d, *J*=2.8 Hz, 1 H); MS (ESI) m/z 423 $(M+H)^+$.

Example 277

N-[(2Z)-5-tert-butyl-3-{[(2S)-5-oxotetrahydrofuran-2-yl]methyl}-1,3-thiazol-2(3H)-ylidene]-5-chloro-2-methoxybenzamide

Example 277A

(S)-(5-oxotetrahydrofuran-2-yl)methyl 4-methylbenzenesulfonate

A mixture of (S)-5-(hydroxymethyl)dihydrofuran-2(3H)-one, para-toluenesulfonyl chloride and pyridine were processed using the method described in Example 162A to afford the title compound. MS (ESI) m/z 288 $(M+18)^+$.

Example 277B

N-[(2Z)-S-tert-butyl-3-{[(2S)-5-oxotetrahydrofuran-2-yl]methyl}-1,3-thiazol-2(3H)-ylidene]-5-chloro-2-methoxybenzamide

Example 244A and the product from Example 277A were processed using the method described in Example 246 to afford the title compound. $^1$H NMR (400 MHz, $CDCl_3$) δ ppm 1.34 (s, 9 H) 2.07-2.20 (m, 1 H) 2.34-2.48 (m, 2 H) 2.49-2.61 (m, 1 H) 3.90 (s, 3 H) 4.39 (dd, *J*=14.4, 6.1 Hz, 1 H) 4.55 (dd, *J*=14.4, 3.1 Hz, 1 H) 4.92 - 5.01 (m, 1 H) 6.75 (s, 1 H) 6.92 (d, *J*=8.9 Hz, 1 H) 7.35 (dd, *J*=8.9, 2.8 Hz, 1 H) 7.92 (d, *J*=2.8 Hz, 1 H); MS (ESI) m/z 423 $(M+H)^+$.

Example 278

N-[(2Z)-5-acetyl-4-methyl-3-[(2R)-tetrahydrofuran-2-ylmethyl]-1,3-thiazol-2(3H)-ylidene]-1-benzofuran-5-carboxamide

Example 278A

(R)-1-((tetrahydrofuran-2-yl)methyl)thiourea

To a 0 °C solution of (R)-(tetrahydrofuran-2-yl)methanamine (5.0 g, 49.5 mmol) and triethylamine (690 uL, 4.95 mmol) in tetrahydrofuran (100 mL) was added carbon disulfide (5.65 g, 74.3 mmol). Stirring was continued for 0.5 hour followed by the dropwise addition of 30% hydrogen peroxide (5.6 g, 49.5 mmol) so that the temperature was maintained below 40°C. The reaction mixture was poured into water, and extracted with ethyl acetate. The organic extract was dried ($Na_2SO_4$), filtered and concentrated to afford an oil. The residue was dissolved in tetrahydrofuran, and treated with 7 N

ammonia in methanol (14.3 mL, 100 mmol). The precipitate was collected by filtration and washed with water to afford the title compound.

Example 278B

(R)-1-(2-imino-4-methyl-3-((tetrahydrofuran-2-yl)methyl)-2,3-dihydrothiazol-5-yl)ethanone

To a solution of pentane-2,4-dione (451 mg, 4.5 mmol) and the product from Example 278A (786 mg, 4.5 mmol) in tetrahydrofuran (5 mL) was added a mixture of dimethylsulfoxide (0.64 mL, 9.0 mmol) and concentrated HCl (0.75 mL, 9.0 mmol). The reaction mixture was heated at 40 °C for 12 hours. After cooling to ambient temperature, the mixture was diluted with ethyl acetate and washed with saturated aqueous $NaHCO_3$. The organic extract was dried ($Na_2SO_4$), filtered and concentrated to afford the title compound. MS (ESI) m/z 241 $(M+H)^+$.

Example 278C

N-[(2Z)-5-acetyl-4-methyl-3-[(2R)-tetrahydrofuran-2-ylmethyl]-1,3-thiazol-2(3H)-ylidene]-1-benzofuran-5-carboxamide

Example 298B and benzofuran-5-carboxylic acid were processed using the method described in Example 223A to afford the title compound. $^1H$ NMR (400 MHz, $CDCl_3$) δ ppm 1.73 - 1.84 (m, 1 H) 1.91 - 2.03 (m, 2 H) 2.16 - 2.26 (m, 1 H) 2.50 (s, 3 H) 2.81 (s, 3 H) 3.75 (dd, *J*=14.12, 6.44 Hz, 1 H) 3.90 (dd, *J*=14.1, 7.4 Hz, 1 H) 4.16 (dd, *J*=13.8, 8.0 Hz, 1 H) 4.41 - 4.51 (m, 1 H) 4.71 (dd, *J*=13.8, 3.4 Hz, 1 H) 6.88 (s, 1 H) 7.55 (d, *J*=8.6 Hz, 1 H) 7.68 (t, *J*=2.2 Hz, 1 H) 8.30 (dt, *J*=8.6, 1.5 Hz, 1 H) 8.57 - 8.59 (m, 1 H); MS (ESI) m/z 385 $(M+H)^+$.

Example 279

N-[(2Z)-5-(1-hydroxy-1-methylethyl)-4-methyl-3-[(2R)-tetrahydrofuran-2-ylmethyl]-1,3-thiazol-2(3H)-ylidene]-1-benzofuran-5-carboxamide

Example 278C (300 mg, 0.78 mmol) in tetrahydrofuran (20 mL) was treated with a solution of methyllithium in diethyl ether (1.56 mL, 1.56 mmol) at -78 °C,. The reaction mixture was stirred at -78 °C for 4 hours and quenched with saturated aqueous $NH_4Cl$, wanned to ambient temperature and extracted with ethyl acetate. The organic extract was dried ($Na_2SO_4$), filtered and concentrated. Purification by reverse phase HPLC afforded the title compound. $^1H$ NMR (500 MHz, $CDCl_3$) δ ppm 1.65 (s, 3 H) 1.67 (s, 3 H) 1.74 - 1.84 (m, 1 H) 1.89 - 2.00 (m, 2 H) 1.99 - 2.02 (m, 1 H) 2.12 - 2.22 (m, 1 H) 2.55 (s, 3 H) 3.76 (dd, *J*=14.3, 7.6 Hz, 1 H) 3.90 (dd, *J*=15.0, 6.7 Hz, 1 H) 4.06 (dd, *J*=13.7, 7.3 Hz, 1 H) 4.40-4.49 (m, 1 H) 4.64 (dd, *J*=14.0, 3.7 Hz, 1 H) 6.85 (dd, *J*=2.1, 0.9 Hz, 1 H) 7.52 (d, *J*=8.5 Hz, 1 H) 7.66 (d, *J*=2.1 Hz, 1 H) 8.30 (dd, *J*=8.5, 1.5 Hz, 1 H) 8.57 (d, *J*=1.5 Hz, 1 H); MS (ESI) m/z 401 $(M+H)^+$.

Example 280

N-[(2Z)-5-acetyl-4-methyl-3-[(2R)-tetrahydrofuran-2-ylmethyl]-1,3-thiazol-2(3H)-ylidene]-5-chloro-2-(2,2,2-trifluoroethoxy)benzamide

Example 280A

N-((2Z)-5-acetyl-4-methyl-3-(((2R)-tetrahydrofuran-2-yl)methyl)thiazol-2(3H)-ylidene)-5-chloro-2-fluorobenzamide

Example 278B and 5-chloro-2-fluorobenzoic acid were processed using the method described in Example 223A to afford the title compound. MS (ESI) m/z 397 $(M+H)^+$.

Example 280B

N-[(2Z)-5-acetyl-4-methyl-3-[(2R)-tetrahydrofuran-2-ylmethyl]-1,3-thiazol-2(3H)-ylidene]-5-chloro-2-(2,2,2-trifluoroethoxy)benzamide

To the solution of the product from Example 280A (360 mg, 0.91 mnol) in tetrahydrofuran (4 mL) was added 2,2,2-trifluoroethanol (227 mg, 2.27 mmol) and a 1.0 M solution of potassium tert-butoxide in tetrahydrofuran (2.27 mL, 2.27 mmol). The reaction mixture was stirred at room temperature for 24 hours. The mixture was diluted with water, and extracted with ethyl acetate. The organic extract was dried ($Na_2SO_4$), filtered and concentrated. Purification by reverse

phase HPLC afforded the title compound. $^1$H NMR (500 MHz, $CDCl_3$) δ ppm 1.64 - 1.75 (m, 1 H) 1.90 - 2.00 (m, 2 H) 2.10 - 2.20 (m, 1 H) 2.51 (s, 3 H) 2.80 (s, 3 H) 3.73 (dd, *J*=15.9, 7.3 Hz, 1 H) 3.88 (dd, *J*=15.3, 6.7 Hz, 1 H) 4.07 (dd, *J*=13.7, 8.2 Hz, 1 H) 4.33 - 4.41 (m, 1 H) 4.47 (dd, *J*=16.8, 8.2 Hz, 2 H) 4.62 (dd, *J*=13.7, 2.8 Hz, 1 H) 7.03 (d, J=8.5 Hz, 1 H) 7.39 (dd, *J*=8.5, 2.8 Hz, 1 H) 8.02 (d, *J*=2.8 Hz, 1 H); MS (ESI) m/z 477 $(M+H)^+$.

Example 281

N-[(2Z)-5-tert-butyl-3-[(5-methyltetrahydrofuran-2-yl)methyl]-13-thiazol-2(3H)-ylidene]-5-chloro-2-methoxybenzamide

Example 281A

hex-5-en-2-ol

A solution of hex-5-en-2-one (10 g, 102 mmol) in ether (60 mL) was treated with lithium aluminum hydride (4.0 g, 110 mmol) and the mixture was stirred at room temperature for I hour. The reaction mixture was quenched with saturated aqueous $NH_4Cl$, and extracted with ether. The organic extract was dried ($Na_2SO_4$), filtered and concentrated to afford the title compound.

Example 281B

2-(bromomethyl)-5-methyltetrahydrofuran

A solution of the product from Example 281A (6.9 g, 69 mmol) in $CH_2Cl_2$ (100 mL) was treated with N-bromosuccinimide (14.7, 83 mmol). The reaction mixture was stirred at room temperature for 48 hours, poured into water, and extracted with $CH_2Cl_2$. The organic extract was dried ($Na_2SO_4$), filtered and concentrated to afford the title compound. MS (ESI) m/z 179 $(M+H)^+$.

Example 281C

N-[(2Z)-5-tert-butyl-3-[(5-methyltetrahydrofuran-2-yl)methyl]-1,3-thiazol-2(3H)-ylidene]-5-chloro-2-methoxybenzamide

Example 244A and the product from Example 281B were processed using the method described in Example 246 to afford the title compounds. $^1$H NMR (500 MHz, $GDCl_3$) δ ppm 1.22 (d, *J*=6.1 Hz, 3 H) 1.33 - 1.37 (m, 9 H) 1.46 - 1.54 (m, 2 H) 1.67 - 1.76 (m, 1 H) 1.91 - 2.00 (m, 1 H) 3.90 (s, 3 H) 4.01 - 4.08 (m, 1 H) 4.29 - 4.34 (m, 2 H) 4.39 - 4.46 (m, 1 H) 6.89 - 6.90 (m, I H) 6.90 - 6.91 (m, 1 H) 7.32 (dd, *J*=8.5, 3.1 Hz, 1 H) 7.94 (d, *J*=2.8 Hz, 1 H); MS (ESI) m/z 423 $(M+H)^+$.

Example 282

N-[(2Z)-5-tert-butyl-3-[(5,5-dimethyltetrahydrofuran-2-yl)methyl]-1,3-thiazol-2(3H)-ylidene]-5-chloro-2-methoxybenzamide

Example 282A

2-methylhex-5-en-2-ol

A 0 °C solution of hex-5-en-2-one (10 g, 102 mmol) in ether (200 mL) was treated dropwise with a 3.0 M solution of methylmagnesium iodide in ether (102 mL, 306 mmol) over 20 minutes. The reaction mixture was gradually warmed to room temperature, and stirred for 1 hour, quenched with water, and filtered through Celite (ether wash). The filtrate was concentrated and the resulting residue was distilled (27-30 °C at 5 mm Hg) to afford the title compound. $^1$H NMR (500 MHz, $CDCl_3$) δ ppm 1.23 (s, 6 H) 1.54 - 1.62 (m, 2 H) 2.11 - 2.19 (m, 2 H) 4.96 (dq, *J*=11.29, 1.83, 1.22 Hz, 1 H) 5.05 (dq, J=117.39, 1.53 Hz, 1 H) 5.80 - 5.91 (m, I H).

Example 282B

5-(bromomethyl)-2,2-dimethyltetrahydrofuran

The product from 282A was processed using the method described in Example 281B to afford the title

compound. $^1H$ NMR (400 MHz, $CDCl_3$) δ ppm 1.23 (s, 3 H) 1.29 (s, 3 H) 1.73 - 1.82 (m, 2 H) 1.81 - 1.91 (m, 1 H) 2.10 - 2.22 (m, 1 H) 3.32 (dd, *J*=0.13, 7.06 Hz, 1 H) 3.43 (dd, *J*=9.82, 4.60 Hz, 1 H) 4.16 - 4.27 (m, 1 H).

Example 282C

N-[(2Z)-5-tert-butyl-3-[(5,5-dimethyltetrahydrofuran-2-yl)methyl]-1,3-thiazol-2(3H)-ylidene]-5-chloro-2-methoxybenzamide

Example 244A and the product from Example 282B were processed using the method described in Example 246 to afford the title compound. $^1H$ NMR (400 MHz, dimethylsulfoxide- $d_6$) δ ppm 1.13 (s, 3 H) 1.17 (s, 3 H) 1.31 (s, 9 H) 1.54 - 1.62 (m, 1 H) 1.64 - 1.72 (m, 1 H) 1.72 - 1.80 (m, 1 H) 1.93 - 2.04 (m, 1 H) 3.78 (s, 3 H) 4.12 (dd, *J*=15.34, 6.75 Hz, 1 H) 4.29 - 4.39 (m, 2 H) 7.10 (d, *J*=8.59 Hz, 1 H) 7.20 (s, 1 H) 7.44 (dd, *J*=8.59, 2.76 Hz, 1 H) 7.64 (d, *J*=2.76 Hz, 1 H); MS (ESI) m/z 437 $(M+H)^+$.

Example 283

N-[(2Z)-5-tert-butyl-3-[(2R)-tetrahydrofuran-2-ylmethyl]-1,3-thiazol-2(3H)-ylidene]-5-chloro-2-(2-methoxyethoxy)benzamide

Example 283A

Example 240C and 5-chloro-2-fluorobenzoic acid were processed using the method described in Example 223A to afford the title compound. MS (ESI) m/z 397 $(M+H)^+$.

Example 283B

N-[(2Z)-5-tert-butyl-3-[(2R)-tetrahydrofuran-2-ylmethyl]-1,3-thiazol-2(3H)-ylidene]-5-chloro-2-(2-methoxvethoxy)benzamide

Example 283A and 2-methoxyethanol were processed using the method described in Example 280B to afford the title compound. $^1H$ NMR (500 MHz, $CDCl_3$) δ ppm 1.36 (s, 9 H) 1.62 - 1.71 (m, 1 H) 1.74 - 1.83 (m, 1 H) 1.84 - 1.93 (m, 1 H) 2.02 - 2.11 (m, 1 H) 3.42 (s, 3 H) 3.77 (m, 3 H) 3.84 (dd, *J*=15.0, 6.71 Hz, 1 H) 4.21 (t, *J*=5.2 Hz, 2 H) 4.22 - 4.31 (m, 2 H) 4.43 (dd, *J*=10.4 Hz, 1 H) 6.90 (s, 1 H) 6.97 (d, *J*=8.9 Hz, 1 H) 7.30 (dd, *J*=8.5, 2.8 Hz, 1 H) 7.89 (d, *J*=2.8 Hz, 1 H)MS (ESI) m/z 477 $(M+H)^+$.

Example 284

N-[(2Z)-5-tert-butyl-3-[(2R)-tetrahydrofuran-2-ylmethyl]-1,3-thiazol-2(3H)-ylidene]-6-chloroquinoline-8-carboxamide

Example 240C and 6-chloroquinoline-8-carboxylic acid were processed using the method described in Example 223A to afford the title compound. $^1H$ NMR (400 MHz, $CDCl_3$) δ ppm 1.38 (s, 9 H) 1.63 - 1.74 (m, 1 H) 1.74 - 1.83 (m, 1 H) 1.82 - 1.93 (m, 1 H) 1.98 - 2.08 (m, 1 H) 3.72 - 3.88 (m, 2 H) 4.18 - 4.30 (m, 2 H) 4.40 (dd, *J*=13.2, 2.5 Hz, 1 H) 6.90 (s, 1 H) 7.40 (dd, *J*=8.3, 4.0 Hz, 1 H) 7.82 (d, *J*=2.5 Hz, 1 H) 7.99 (d, *J*=2.2 Hz, 1 H) 8.06 (dd, J=8.3, 1.84 Hz, 1 H) 9.02 (dd, *J*=4.3, 1.84 Hz, 1 H); MS (ESI) m/z 430 $(M+H)^+$.

Example 285

5-chloro-2-methoxy-N-[(2Z)-5-(1-methylcyclopropyl)-3-[(2R)-tetrahydrofuran-2-ylmethyl]1,3-thiazol-2(3H)-ylidene]benzamide

Example 285A

2-(2-aminothiazol-5-yl)propan-2-ol

To a -78 °C solution of thiazol-2-amine (7.0 g, 69.9 mmol) in tetrahydrofuran (200 mL) was added a 10.0 M solution of butyllithium in hexane (14 mL, 140 mmol). The mixture was stirred at -78 °C for 1 hour and chlorotrimethylsilane (15.2 g, 140 mnol) was added dropwise. The mixture was allowed to warm up to -40 °C, cooled to -78 °C and a 10.0 M solution of butyllithium in hexane (7.0, 70 mmol) was added. After 10 minutes, propan-2-one (8.12 g, 140 mmol) was

added and the mixture was stirred for 12 hours. The reaction mixture was quenched with saturated aqueous $NH_4Cl$ and extracted with ether. The organic extract was dried ($Na_2SO_4$), filtered and concentrated. The residue was purified by column chromatography using an Analogix® Intelliflash280 ™ ($SiO_2$, 0-100 % ethyl acetate in hexanes) to afford the title compound. $^1H$ NMR (500 MHz, $CDCl_3$) δ ppm 1.61 (s, 6 H) 5.04 - 5.19 (brs, 2 H) 6.89 (s, 1 H); MS (ESI) m/z 159 $(M+H)^+$.

Example 285B

(R)-5-(prop-1-en-2-yl)-3-((tetrahvdrofuran-2-yl)methyl)thiazol-2(3H)-imine

Example 285A and the product from Example 208A were processed using the method described in Example 240C to afford the title compound. MS (ESI) m/z 225 $(M+H)^+$.

Example 285C

(R,Z)-5-chloro-2-methoxy-N-(5-(prop-1-en-2-yl)-3-((tetrahydrofuran-2-yl)methyl)thiazol-2(3H)-ylidene)benzamide

Example 285B and the product from Example 205B were processed using the method described in Example 223A to afford the title compound. MS (ESI) m/z 393 $(M+H)^+$.

Example 285D

5-chloro-2-methoxy-N-[(2Z)-5-(1-methylcyclopropyl)-3-[(2R)-tetrahydrofuran-2-ylmethyl]-1,3-thiazol-2(3H)-ylidene] benzamide

A 20 mL vial was charged with 1 mL of $CH_2Cl_2$, and 1,2-dimethoxyethane (110 mg, 1.22 mmol). The solution was cooled to -10 °C and diethylzinc (151 mg, 1.22 mmol) was added. To this mixture was added dropwise diiodomethane (654 mg, 2.44 mmol). After the addition was complete, the resulting clear solution was stirred for 10 minutes at -10 °C. A solution of Example 285C (80mg, 0.204 mmol) was added. The mixture was allowed to warm to room temperature, stirred for 16 hours, then concentrated. Purification by reverse phase preparative HPLC on a Waters Symmetry C8 column (25 mm x 100 mm, 7 μm particle size) using a gradient of 10% to 100% acetonitrile:0.1% aqueous trifluoroacetic acid over 8 minutes (10 minutes run time) at a flow rate of 40 mL/minutes) afforded Example 285D and Example 286. Characterization for Example 285D: $^1H$ NMR (400 MHz, $CDCl_3$) δ ppm 0.97 - 1.02 (m, 2 H) 1.02 - 1.08 (m, 2 H) 1.50 (s, 3 H) 1.62 - 1.72 (m, I H) 1.82 - 1.91 (m, I H) 1.93 - 2.04 (m, 1 H) 2.21 - 2.31 (m, 1 H) 3.72 - 3.86 (m, 2 H) 4.04 (s, 3 H) 4.29 - 4.39 (m, 1 H) 4.48 - 4.61 (m, 1 H) 4.80-4.93 (m, 1 H) 7.02 (d, *J*=9.2 Hz, I H) 7.54 (dd, *J*=8.9, 2.8 Hz, 1 H) 7.53 - 7.55 (m, 1 H) 8.04 (d, *J*=2.8 Hz, I H); MS (ESI) m/z 407 $(M+H)^+$.

Example 286

5-chloro-N-[(2Z)-5-(1-hydroxy-3-iodo-1-methylpropyl)-3-[(2R)-tetrahydrofuran-2-ylmethyl]-1,3-thiazol-2(3H)-ylidene]-2-methoxybenzamide

See Example 285D for experimental details $^1H$ NMR (500 MHz, $CDCl_3$) δ ppm 1.61 (d, *J*=1.53 Hz, 3 H) 1.63 - 1.72 (m, 1 H) 1.79 - 1.96 (m, 2 H) 2.03 - 2.14 (m, 2 H) 2.40 - 2.47 (m, 2 H) 3.02 - 3.11 (m, 1 H) 3.19 (dd, *J*=17.4, 9.2 Hz, 1 H) 3.78 (dd, *J*=15.0, 7.0 Hz, 1 H) 3.83 - 3.89 (m, I H) 3.91 (s, 3 H) 4.15 - 4.22 (m, 1 H) 4.25 - 4.31 (m, I H) 6.91 (d, *J*=8.9 Hz, I H) 7.11 (d, *J*=5.5 Hz, I H) 7.34 (dd, *J*=8.5, 3.1 Hz, 1 H) 7.96 (d, *J*=2.8 Hz, I H); MS (ESI) m/z 550 $(M+H)^+$.

Example 287

N-[(2Z)-5-tert-butyl-3-[(2R)-tetrahydrofuran-2-ylmethyl]-1,3-thiazol-2(3H)-ylidene]-5-chloro-2-[(1-methycyclopropyl) methoxy]benzamide

The product from 283A and (1-methylcyclopropyl)methanol were processed using the method described in Example 280B to afford the title compound. $^1H$ NMR (500 MHz, $CDCl_3$) δ ppm 0.36 (t, *J*=4.8 Hz, 2 H) 0.55 (t, *J*=4.6 Hz, 2 H) 1.22 (s, 3 H) 1.36 (s, 9H) 1.61 - 1.70 (m, 1 H) 1.74 - 1.83 (m, 1 H) 1.83 - 1.92 (m, 1 H) 2.01 - 2.10 (m, 1 H) 3.77 (dd, *J*=14.3, 7.6 Hz, 1 H) 3.81 (s, 2H) 3.84 (dd, *J*=15.9, 8.2 Hz, 1 H) 4.21 (dd, *J*=13.4, 6.41 Hz, 1 H) 4.23 -4.29 (m, 1 H) 4.42 (dd, *J*=13.4, 2.4 Hz, 1 H) 6.86 (d, *J*=9.5 Hz, 1 H) 6.87-6.88 (m, 1 H) 7.26 (dd, *J*=7.9, 3.7 Hz, 1 H) 7.81 (d, *J*=2.8 Hz, 1 H); MS (ESI) m/z 463 $(M+H)^+$.

Example 288

N-[(2Z)-5-tert-butyl-3-[(2R)-tetrahydrofuran-2-ylmethyl]-1,3-thiazol-2(3H)-ylidene]-5-chloro-2-isopropoxybenzamide

Example 283A and propan-2-ol were processed using the method described in Example 280B to afford the title compound. $^1$H NMR (500 MHz, $CDCl_3$) δ ppm 1.35 (s, 9 H) 1.35 (d, *J*=5.8 Hz, 6 H) 1.63 - 1.71 (m, 1 H) 1.75 - 1.82 (m, 1 H) 1.83 - 1.92 (m, 1 H) 2.01 - 2.10 (m, 1 H) 3.77 (dd, *J*=14.65, 7.32 Hz, 1H) 3.85 (dd, *J*=14.7, 6.7 Hz, 1 H) 4.20 (dd, *J*=13.7, 6.4 Hz, 1 H) 4.24-4.30 (m, 1 H) 4.42 (dd, *J*=13.4, 2.8 Hz, 1 H) 4.49 - 4.57 (m, 1 H) 6.86 (s, 1 H) 6.89 (d, *J*=8.9 Hz, 1 H) 7.26 (dd, *J*=8.9, 2.8 Hz, 1 H) 7.84 (d, *J*=2.8 Hz, 1 H); MS (ESI) m/z 437 $(M+H)^+$.

Example 289

N-[(2Z)-5-tert-butyl-3-[(2R)-tetrahydrofuran-2-ylmethyl]-1,3-thiazol-2(3H)-ylidene]-5-chloro-2-ethoxybenzamide

Example 283A and ethanol were processed using the method described in Example 280B to afford the title compound. $^1$H NMR (500 MHz, $CDCl_3$) δ ppm 1.35 (s, 9 H) 1.44 (t, *J*=7.02 Hz, 3 H) 1.62-1.73(m, 1 H) 1.75 - 1.83 (m, 1 H) 1.84 - 1.92 (m, 1 H) 2.01 - 2.11 (m, 1 H) 3.77 (dd, *J*=13.7, 7.6 Hz, 1 H) 3.84 (dd, *J*=14.7, 6.7 Hz, 1 H) 4.13 (dd, *J*=14.0, 7.2 Hz, 2 H) 4.22 (dd, *J*=13.4, 6.0 Hz, 1 H) 4.25 - 4.31 (m, 1 H) 4.41 (dd, *J*=13.4, 2.8 Hz, 1 H) 6.86 (s, 1 H) 6.89 (d, *J*=8.9 Hz, 1 H) 7.29 (dd, *J*=8.9, 2.8 Hz, 1 H) 7.91 (d, *J*=2.8 Hz, 1 H); MS (ESI) m/z 423 $(M+H)^+$.

Example 290

N-[(2Z)-5-tert-butyl-3-[(2R)-tetrahydrofuran-2-ylmethyl]-1,3-thiazol-2(3H)-yldene]-5-chloro-2-(tetrahydrofuran-3-yloxy) benzamide

Example 283A and tetrahydrofuran-3-ol were processed using the method described in Example 280B to afford the title compound. $^1$H NMR (500 MHz, $CDCl_3$) δ ppm $^1$H NMR (500 MHz, $CDCl_3$) δ ppm 1.36 (s, 9 H) 1.61 - 1.70 (m, 1 H) 1.76 - 1.83 (m, 1 H) 1.84 - 1.93 (m, 1 H) 2.03 - 2.10 (m, 1 H) 2.11 - 2.25 (m, 2H) 3.77 (dd, *J*=14.0, 7.3 Hz, 1 H) 3.82-3.91 (m, 2 H) 3.98 (dd, *J*=15.3, 8.5 Hz, 1 H) 4.02 (d, *J*=3.7 Hz, 2 H) 4.20 (ddd, *J*=6.4, 1.5 Hz, 1 H) 4.23 - 4.30 (m, 1 H) 4.41 (dd, *J*=13.7, 3.1 Hz, 1 H) 4.88 - 5.00 (m, 1 H) 6.83 (dd, *J*=8.9, 0.6 Hz, 1 H) 6.88 (d, *J*=0.9 Hz, 1 H) 7.28 (dd, *J*=8.9, 2.75 Hz, 1 H) 7.87 (t, *J*=3.1 Hz, 1 H); MS (ESI) m/z 423 $(M+H)^+$.

Example 291

N-[(2Z)-5-tert-butyl-3-[(2R)-tetrahydrofuran-2-ylmethyl]-1,3-thiazol-2(3H)-ylidene]-5-chloro-2-[(2-methoxyethyl)(methyl)amino]benzamide

A mixture of the product from Example 283A (120 mg, 0.3 mmol), 2-methoxy-N-methylethanamine (54 mg, 0.6 mmol) and triethylamine (127 ul, 0.91 mmol) in tetrahydrofuran (1 mL) was heated at 120°C in a microwave (CEM) for 2 hours. The reaction mixture was diluted with water and extracted with ethyl acetate. The organic extract was dried ($Na_2SO_4$), filtered, and concentrated. Purification by reverse phase preparative HPLC on a Waters Symmetry C8 column (25 mm x 100 mm, 7 μm particle size) using a gradient of 10% to 100% acetonitrile:0.1 % aqueous trifluoroacetic acid over 8 minutes (10 minutes run time) at a flow rate of 40 mL/minutes) to afford the title compound afforded the title compound. $^1$H NMR (500 MHz, $CDCl_3$) δ ppm 1.36 (s, 9 H) 1.62 - 1.71 (m, 1 H) 1.78 - 1.86 (m, 1 H) 1.86 - 1.93 (m, 1 H) 2.01 - 2.11 (m, 1 H) 2.89 (s, 3 H) 3.26 (s, 3 H) 3.29 (t, *J*=6.1 Hz, 2 H) 3.54 (t, *J*=6.4 Hz, 2 H) 3.78 (dd, *J*=14.7, 7.3 Hz, 1 H) 3.85 (dd, *J*=15.0, 8.2 Hz, 1 H) 4.18 (dd, *J*=13.7, 6.4 Hz, 1 H) 4.23 - 4.30 (m, 1 H) 4.40 (dd, *J*=13.7, 3.1 Hz, 1 H) 6.85 (s, 1 H) 6.92 (d, *J*=8.9 Hz, 1 H) 7.20 (dd, *J*=8.9, 2.8 Hz, 1 H) 7.67 (d, *J*=2.4 Hz, 1 H); MS (ESI) m/z 466 $(M+H)^+$.

Example 292

N-[(2Z)-5-tert-butyl-3-[(2R)-tetrahydrofuran-2-ylmethyl-1,3-thiazol-2(3H)-ylidene]-5-chloro-2-(difluoromethoxy)benzamide

Example 240C and 5-chloro-2-(difluoromethoxy)benzoic acid were processed using the method described in Example 223A to afford the title compound. $^1$H NMR (500 MHz, $CDCl_3$ δ ppm 1.36 (s, 9 H) 1.60 - 1.69 (m, 1 H) 1.76 - 1.84 (m, 1 H) 1.85 - 1.93 (m, 1 H) 2.02 - 2.12 (m, 1 H) 3.78 (dd, *J*=13.7, 7.3 Hz, 1 H) 3.85 (dd, *J*=15.0, 6.7 Hz, 1 H) 4.20 (dd, *J*=13.4, 6.4 Hz, 1 H) 4.24 - 4.30 (m, 1 H) 4.44 (dd, *J*=13.7, 2.8 Hz, 1 H) 6.73 (t, *J*=6.3 Hz, 1 H) 6.91 (s, 1 H) 7.17 (d, *J*=8.5 Hz, 1 H) 7.38 (dd, *J*=8.5, 2.8 Hz, 1 H) 8.05 (d, *J*=2.8 Hz, 1 H); MS (ESI) m/z 445 $(M+H)^+$.

Example 293

N-[(2Z)-5-tert-butyl-3-[(2R)-tetrahydrofuran-2-ylmethyl]-1,3-thiazol-2(3H)-ylidene]-5-chloro-2-(trifluoromethoxy)benzamide

Example 240C and 5-chloro-2-(trifluoromethoxy)benzoic acid were processed using the method described in Example 223A to afford the title compound. $^1$H NMR (500 MHz, $CDCl_3$) $\delta$ ppm 1.36 (s, 9H) 1.58 - 1.67 (m, 1 H) 1.75 - 1.83 (m, 1 H) 1.84 - 1.93 (m, 1 H) 2.01 - 2.11 (m, 1 H) 3.77 (dd, *J*=13.7, 6.4 Hz, 1 H) 3.84 (dd, *J*=15.0, 6.7 Hz, 1 H) 4.18 (dd, *J*=13.7, 6.7 Hz, 1 H) 4.22 - 4.29 (m, 1 H) 4.46 (dd, *J*=13.7, 2.8 Hz, 1 H) 6.91 (s, 1 H) 7.23 (dd, *J*=8.5, 1.2 Hz, 1 H) 7.40 (dd, *J*=8.9, 2.8 Hz, 1 H) 8.06 (d, *J*=2.8 Hz, 1 H); MS (ESI) m/z 463 $(M+H)^+$.

Example 294

N-[(2Z)-5-tert-butyl-3-[(2R)-tetrahydrofuran-2-ylmethyl]-1,3-thiazol-2(3H)-ylidene]-5-chloro-2-(2,2,2-trifluoroethoxy) benzamide

The product from 283A and 2,2,2-trifluoroethanol were processed using the method described in Example 280B to afford the title compound. $^1$H NMR (500 MHz, $CDCl_3$) $\delta$ ppm 1.37 (s, 9 H) 1.60 - 1.69 (m, 1 H) 1.76 - 1.84 (m, 1 H) 1.85 - 1.93 (m, 1 H) 2.02 - 2.11 (m, 1 H) 3.78 (dd, *J*=13.7, 7.3 Hz, 1 H) 3.85 (dd, *J*=15.0, 7.0 Hz, 1 H) 4.21 (dd, *J*=13.4, 6.4 Hz, 1 H) 4.24-4.29 (m, 1 H) 4.43 (dd, *J*=11.0, 2.8 Hz, 1 H) 4.47 (dd, *J*=17.1, 8.5 Hz, 2 H) 6.91 (s, 1 H) 7.01 (d, *J*=8.9Hz, 1 H) 7.33 (dd, *J*=8.5, 2.8 Hz, 1 H) 7.99 (d, *J*=2.8 Hz, 1 H); MS (ESI) m/z 477 $(M+H)^+$.

Example 295

N-[(2Z)-5-tert-butyl-3-[(2R)-tetrahydrofuran-2-ylmethyl]-1,3-thiazol-2(3H)-ylidene]-5-chloro-2-[3-(dimethylamino)propoxy]benzamide

The product from 283A and 3-(dimethylamino)propan-1-ol were processed using the method described in Example 280B to afford the title compound. $^1$H NMR (500 MHz, $CDCl_3$) $\delta$ ppm 1.38 (s, 9 H) 1.62 - 1.71 (m, 1 H) 1.84 - 1.91 (m, 1 H) 1.91 - 1.98 (m, 1 H) 2.09 - 2.19 (m, 1 H) 2.29 - 2.36 (m, 2 H) 3.04 - 3.09 (m, *J*=4.9 Hz, 2 H) 3.81 (dd, *J*=13.7, 6.4 Hz, 1 H) 3.87 (dd, *J*=15.0, 7.0 Hz, 1 H) 4.25 - 4.34 (m, 4 H) 4.51 (d, *J*=12.8 Hz, 1 H) 6.93 (d, *J*=9.2 Hz, 1 H) 7.00 - 7.02 (m, 1 H) 7.44 (dd, *J*=8.9, 2.9 Hz, 1 H) 8.20 (d, *J*=2.8 Hz, 1 H) 10.35 - 10.51 (m, 1 H); MS (ESI) m/z 480 $(M+H)^+$.

Example 296

5,6-dichloro-N-[(2Z)-4,5-dimethyl-3-(tetrahydrofuran-2-ylmethyl)-1,3-thiazol-2(3H)-ylidene]quinoline-8-carboxamide

A mixture of Example 186A (145 mg), 5,6-dichloroquinoline-8-carboxylic acid (99.65 mg) (Bailey J. Heterocycl. Chem. 1974, 11, 229), 1-hydroxybenzotriazole hydrate (55 mg), 1-ethyl-3-[3-(dimethylamino)propyl]-carbodiimide hydrochloride (79 mg) and triethylamine (0.14 mL) in tetrahydrofuran (3 mL) was heated at 70 °C on a shaker overnight, cooled, quenched in saturated $NaHCO_3$ and extracted with ethyl acetate (2x). The organic extracts were dried ($Na_2SO_4$), filtered and the solvent was evaporated The crude material was purified by reverse phase preparative HPLC on a Waters Symmetry C8 column (25 mm x 100 mm, 7 $\mu$m particle size) using a gradient of 10% to 100% acetonitrile,: 10 mM ammonium acetate over 8 minutes (10 minutes run time) at a flow rate of 40 mL/minutes) to afford the title compound. $^1$H NMR (300 MHz, $CDCl_3$) $\delta$ ppm 1.62-1.75 (m, 1H), 1.85-1.95 (m, 2H), 2.0-2.1 (m, 1H), 2.26 (s, 3H), 2.30 (s, 3H), 3.70-3.77 (m, 1H), 3.83-3.90 (m, 1H), 3.97 (dd, 1H), 4.35-4.42 (m, 1H), 4.55 (dd, 1H), 7.53 (dd, 1H), 8.60 (dd, 1H), 9.07 (m, 1H); MS ($DCI/NH_3$) m/z 436, 438 $(M+H)^+$.

Example 297

6-chloro-N-[(2Z)-4,5-dimethyl-3-(tetrahydrofuran-2-ylmethyl)-1,3-thiazol-2(3H)-ylidene]quinoline-8-carboxamide

A mixture of Example 186A (106 mg), 6-chloro-quinoline-8-carboxylic acid (73 mg) (Weyer et al, Arzneim. Forsch 1974, 24, 269), 1-hydroxybenzotriazole hydrate (47 mg), 1-ethyl-3-[3-(dimethylamino)propyl]-carbodiimide hydrochloride (69 mg), and triethylamine (0.15 mL) in tetrahydrofuran (2 mL) was heated to 70 °C on a shaker overnight, cooled, poured into saturated $NaHCO_3$ and extracted with ethyl acetate (2x). The organic extracts were dried ($Na_2SO_4$), filtered and the solvent was evaporated. The crude material was dissolved in wann methanol and allowed to cool overnight. The solid precipitate was discarded, the filtrate was concentrated to dryness, triturated with cold methanol,

and collected to afford the title compound. $^1$H NMR (300 MHz, dimethylsulfoxide-$d_6$) δ ppm 1.56-1.64 (m, 1H), 1.70-1.95 (m, 3H), 2.26 (s, 3H), 2.27 (s, 3H), 3.57-3.65 (m, 1H), 3.73-3.80 (m, 1H), 3.97-4.05 (m, 1H), 4.24-4.35 (m, 2H), 7.60 (dd, 1H), 7.78 (d, 1H), 8.15 (d, 1H), 8.38 (dd, 1H), 8.91 (dd, 1H); MS (DCI/$NH_3$) m/z 402 $(M+H)^+$.

Example 298

6-chloro-N-[(2Z)-5-methyl-3-(tetrahydrofuran-2-ylmethyl)-1,3-thiazol-2(3H)-ylidenel]quinoline-8-carboxamide

A mixture of Example 149A (142 mg), 6-chloro-quinoline-8-carboxylic acid (105 mg) (Weyer et al, Arzneim. Forsch 1974, 24, 269), 1-hydroxybenzotriazole hydrate (67 mg), 1-ethyl-3-[3-(dimethylamino)propyl]-carboiiimide hydrochloride (94 mg), and triethylamine (0.15 mL) in tetrahydrofuran (3 mL) was heated to 70 °C for 24 hours, cooled, poured into saturated $NaHCO_3$ and extracted with ethyl acetate (2x). The organic extracts were dried ($Na_2SO_4$), filtered and the solvent was evaporated. The crude material was purified by reverse phase preparative HPLC on a Waters Symmetry C8 column (25 mm x 100 mm, 7 μm particle size) using a gradient of 10% to 100% acetonitrile: 10 mM ammonium acetate over 8 minutes (10 minutes run time) at a flow rate of 40 mL/minutes) to afford the title compound. $^1$H NMR (300 MHz, $CDCl_3$) δ ppm 1.65-1.72 (m, 1H), 1.80-1.95 (m, 2H), 2.0-2.1 (m, 1H), 2.34 (d, J = 1.4 Hz, 3H), 3.74-3.90 (m, 2H), 4.14 (dd, 1H), 4.2-4.3 (m, 1H), 4.47 (dd, 1H), 6.95 (q, J = 1.4 Hz, 1H), 7.42 (dd, 1H), 7.84 (d, 1H), 8.03 (d, 1H), 8.08 (dd, 1H), 9.04 (dd, 1H); MS (DCI/$NH_3$) m/z 388 $(M+H)^+$.

Example 299

5,6-dichloro-N-[(2Z)-5-methyl-3-(tetrahydrofuran-2-ylmethyl)-1,3-thiazol-2(3H)-ylidene]quinoline-8-carboxamide

A mixture of Example 149A (117 mg), 5,6-dichloroquinoline-8-carboxylic acid (71 mg), 1-hydroxybenzotriazole hydrate (51 mg), 1-ethyl-3-[3-(dimethylamino)propyl]-carbodiimide hydrochloride (79 mg) and triethylamine (0.15 mL) in tetrahydrofuran was processed and purified according to the method of Example 298 to afford the title compound. $^1$H NMR (300 MHz, $CDCl_3$) δ ppm 1.6-1.71 (m, 1H), 1.78-1.95 (m, 2H), 2.0-2.1 (m, 1H), 2.34 (d, J = 1.4 Hz, 3H), 3.72-3.90 (m, 2H), 4.12 (dd, 1 H), 4.2-4.3 (m, 1H), 4.46 (dd, 1 H), 6.94 (q, J = 1.4 Hz, 1H), 7.53 (dd, 1H), 8.60 (dd, 1H), 9.06 (dd, 1H); MS (DCI/$NH_3$) m/z 422 $(M+H)^+$.

Example 300

3-methoxy-N-[(2Z)-5-methyl-3-(tetrahydrofuran-2-ylmethyl)-1,3-thiazol-2(3H)-ylidene]-2-naphthamide

A mixture of Example 149A (113 mg), 3-methoxy-2-naphthoic acid (Aldrich) (69 mg), 1-hydroxybenzotriazole hydrate (50 mg), 1-ethyl-3-[3-(dimethylamino)propyl]-carbodiimide hydrochloride (79 mg) and triethylamine (0.15 mL) in tetrahydrofuran (2 mL) was shaken over the weekend at room temperature, poured into saturated $NaHCO_3$ and extracted with ethyl acetate (2x). The organic extracts were dried ($Na_2SO_4$), filtered and the solvent was evaporated. The crude material was purified by flash chromatography over silica gel eluting with ethyl acetate:hexane (7:3) to afford the title compound. $^1$H NMR (300 MHz, $CDCl_3$) δ ppm 1.64-1.78 (m, 1H), 1.80-1.95 (m, 2H), 2.02-2.15 (m, 1H), 2.32 (d, J = 1.4 Hz, 3H), 3.74-3.92 (m, 2H), 4.01 (s, 3H), 4.2 (dd, 1H), 4.27-4.35 (m, 1H), 4.51 (d, 1H), 6.91 (br s, 1H), 7.20 (s, 1H), 7.33 (m, 1H), 7.46 (m, 1H), 7.73 (d, 1H), 7.81 (d, 1H), 8.42 (s, 1H); MS (DCI/$NH_3$) m/z 383 $(M+H)^+$.

Example 301

N-[(2Z)-5-tert-butyl-3-[(cis)-(3-methoxycyclobutyl)methyl]-1,3-thiazol-2(3H)-ylidene]-5-chloro-2-methoxybenzamide

Example 301A

cis-3-benzyloxymethylcyclobutanol methyl ether

To a solution of cis-3-benzyloxymethylcyclobutanol (Albany Molecular Research Institute, 1.0 g, 5.2 mmol) in 10 mL of tetrahydrofuran at 0 °C was added NaH (0.62 g, 15.6 mmol). The mixture stirred for 15 minutes and iodomethane (0.49 mL, 7.8 mmol) was added and the mixture was allowed to warm to ambient temperature and stirred for 16 hours. Some starting material remained by TLC so additional NaH (0.21, 5.2 mmol) and iodomethane (0.32 mL, 5.2 mmol) were added and the mixture stirred for an additional 2 hours. The mixture was quenched with 10 mL of $NH_4Cl$ and diluted with 10 mL of ethyl acetate. The layers were separated and the aqueous layer was extracted twice with 5 mL portions of ethyl acetate. The combined organic extracts were dried over anhydrous $Na_2SO_4$, filtered and concentrated underre-

duced pressure. Purification via column chromatography ($SiO_2$, 75% hexanes in ethyl acetate) afforded the title compound. MS ($DCI/NH_3$) m/z 207 $(M+H)^+$.

Example 301B

(cis-3-methoxycyclobutyl)methanol

A solution of Example 301 A (1.05 g, 5.2 mmol) in 10 mL of ethanol was degassed and the flask was filled with $N_2$. This was repeated two additional times. Pd/C (0.1 g, 10 wt%) was added and the mixture was degassed again and flushed with $N_2$. This was repeated two additional times and the flask was put under 1 atm. of $H_2$ and the mixture was allowed to stir at ambient temperature for 72 hours. The mixture was degassed and the flask was filled with $N_2$. The reaction mixture was filtered, concentrated under reduced pressure and purified by column chromatography ($SiO_2$, 25% hexanes in ethyl acetate) to afford the title compound. MS ($DCI/NH_3$) m/z 134 $(M+NH_4)^+$.

Example 301C

(cis-3-methoxycyclobutyl)methyl 4-methylbenzenesulfonate

Example 301B (0.49 g, 4.2 mmol) and p- toluenesulfonyl chloride (0.80 g, 4.2 mmol) in 5 mL of $CH_2Cl_2$ and 5 mL of pyridine were processed as in Example 203A to afford the title compound. MS ($DCI/NH_3$) m/z 288 $(M+NH_4)^+$.

Example 301D

5-tert-butyl-3-((cis-3-methoxycyclobutyl)methyl)thiazol-2(3H)-imine

Example 240A (0.25 g, 1.6 mmol), Example 301C (0.44 g, 1.6 mmol) and tetrabutylammonium iodide (0.30 g, 0.81 mmol) in 0.5 mL of N,N- dimethylformamide were processed as in Example 240C to afford the title compound. MS ($DCI/NH_3$) m/z 266 $(M+H)^+$.

Example 301E

N-[(2Z)-5-tert-butyl-3-[(cis)-(3-methoxyclobutyl)methyl]-1,3-thiazol-2(3H)-ylidene]-5-chloro-2-methoxybenzamide

Example 301D (0.19 g, 0.75 mmol), triethylamine (0.31 mL, 2.2 mmol) and Example 205B (0.75 mmol) in 10 mL of tetrahydrofuran were processed as in Example 208D to afford the title compound (0.105 g, 0.25 mmol, 33% yield). $^1H$ NMR (300 MHz, $CD_3OD$) $\delta$ ppm 1.38 (s, 9 H), 1.71 - 1.84 (m, 2 H), 2.31 - 2.47 (m, 3 H), 3.21 (s, 3H), 3.71 - 3.83 (m, 1H), 3.86 (s, 3 H), 4.29 (d, *J*=6.4 Hz, 2 H), 7.07 (d, *J*=8.8 Hz, 1 H), 7.14 (s, 1 H), 7.40 (dd, *J*=8.8, 3.1 Hz, 1 H), 7.83 (d, *J*=2.7 Hz, 1 H); MS ($DCI/NH_3$) m/z 423 $(M+H)^+$. Anal. Calculated for $C_{21}H_{27}ClN_2O_3S$: C, 59.63; H, 6.43; N, 6.62. Found: C, 59.66; H, 6.28; N, 6.44.

Example 302

N-[(2Z)-5-tert-butyl-3-[(cis)-(3-hydroxycyclobutyl)methyl]-1,3-thiazol-2(3H)-ylidene]-5-chloro-2 - methoxybenzamide

Example 302A

(cis-3-(benzyloxymethyl)cyclobutoxy)(tert-butyl)dimethylsilane

To a solution of cis-3-benzyloxymethylcyclobutanol (Albany Molecular Research Institute, 1.0 g, 5.2 mmol) in 50 mL of $CH_2Cl_2$ was added imidazole (2.7 g, 39 mmol) followed by *tert*butyldimethylsilyl chloride (3.9 g, 26 mmol). This mixture stirred at ambient temperature for 2 hours and was quenched with 10 mL of saturated aqueous $NH_4Cl$. The layers were separated and the aqueous layer was extracted with three 5 mL of portions of $CH_2Cl_2$. The combined organic extracts were dried over anhydrous $Na_2SO_4$, filtered and concentrated under reduced pressure. Purification via column chromatography ($SiO_2$, 75% hexanes in ethyl acetate) afforded the title compound. MS ($DCI/NH_3$) m/z 307 $(M+H)^+$.

Example 302B

(cis-3-(tert-butyldimethylsilyloxy)cyclobutyl)methanol

A solution of Example 302A (3.7 g, 12 mmol) in 20 mL of ethanol was degassed and the flask was flushed with $N_2$. This was repeated two additional times. Pd/C (0.37 g, 10 wt%) was added and the mixture was degassed again and flushed with $N_2$. This was repeated two additional times then the flask was put under I atm. of $H_2$ and the reaction mixture was allowed to stir at ambient temperature for 48 hours. The mixture was degassed and the flask was filled with $N_2$ then the reaction mixture was filtered, concentrated under reduced pressure and purified by column chromatography ($SiO_2$, 50% hexanes in ethyl acetate) to afford the title compound MS (DCI/$NH_3$) m/z 217 $(M+NH_4)^+$.

Example 302C

(cis-3-(tert-butyldimethylsilyloxy)cyclobutyl)methyl 4-methylbenzenesulfonate

Example 302B (1.2 g, 5.5 mmol) and p- toluenesulfonyl chloride (1.1 g, 5.5 mmol) in 7 mL of $CH_2Cl_2$ and 7 mL of pyridine were processed as in Example 203A to afford the title compound. MS (DCI/$NH_3$) m/z 371 $(M+H)^+$, 388 $(M+NH_4)^+$.

Example 302D

5-tert-butyl-3-((cis-3-(tert-butyldimethylsilyloxy)cyclobutyl)methyl)thiazol-2(3H)-imine

Example 240A (0.72 g, 4.6 mmol), Example 302C (1.7 g, 4.6 mmol) and tetrabutylammonium iodide (0.85 g, 2.3 mmol) in 1.5 mL ofN,N- dimethylformamide were processed as in Example 240C to afford the title compound. MS (DCI/$NH_3$) m/z 355 $(M+H)^+$.

Example 302E

(Z)-N-(5-tert-butyl-3-((cis-3-(tert-butyldimethylsilyloxy)cyclobutyl)methyl)thiazol-2(3H)-ylidene)-5-chloro-2-methoxybenzamide

Example 302D (0.57 g, 1.6 mmol), triethylamine (0.67 mL, 4.8 mmol) and Example 205B (1.6 mmol) in 10 mL of tetrahydrofuran were processed as in Example 208D to afford the title compound. MS (DCI/$NH_3$) m/z 523 $(M+H)^+$.

Example 302F

N-[(2Z)-5-tert-butyl-3-[(cis)-(3-hydroxycyclobutyl)methyl]-1,3-thiazol-2(3H)-ylidene]-5-chloro-2-methoxybenzamide

To Example 302E (0.78 g, 1.5 mmol) in 10 mL of tetrahydrofuran at ambient temperature was added tetrabutylammonium fluoride (1 M in tetrahydrofuran, 1.8 mL, 1.8 mmol) dropwise via syringe pump over 30 minutes. The reaction mixture was stirred at ambient temperature for 2 hours and was concentrated under reduced pressure and purified by column chromatography ($SiO_2$, 50% hexanes in ethyl acetate then 100% ethyl acetate) to afford the title compound. $^1H$ NMR (300 MHz, $CD_3OD$) $\delta$ ppm 1.38 (s, 9 H), 1.70 - 1.82 (m, 2 H), 2.25 - 2.42 (m, 3 H), 3.86 (s, 3 H), 4.00 - 4.10 (m, 1 H), 4.28 (d, *J*=6.4 Hz, 2 H), 7.07 (d, *J*=9.2 Hz, 1H), 7.12 (s, 1H), 7.39 (dd, *J*=8.8, 2.7 Hz, 1H), 7.80 (d, *J*=2.7 Hz, 1H); MS (DCI/$NH_3$) m/z 409 $(M+H)^+$.

Example 303

N-[(2Z)-5-tert-butyl-3-[((cis)-3-hydroxy-3-methylcyclobutyl)methyl]-1,3-thiazol-2(3H)-ylidene]-5-chloro-2-methoxybenzamide

Example 303A

(Z)-N-(5-tert-butyl-3-((3-oxocyclobutyl)methyl)thiazol-2(3H)-ylidene)-5-chloro-2-methoxybenzamide

To Example 302F (0.57 g, 1.4 mmol) in 15 mL of $CH_2Cl_2$ was added 4-methylmorpholine *N*-oxide (0.82 g, 7.0 mmol) followed by approximately 0.5 g powdered 4 A molecular sieves. This mixture was stirred at ambient temperature

for 15 minutes and was cooled to 0 °C and tetrapropylammonium perruthenate (49 mg, 0.14 mmol) was added in portions over 5 minutes. The mixture was stirred at 0 °C for 5 minutes and was allowed to wann to ambient temperature and stirred for an additional 90 minutes. The mixture was filtered through Celite, concentrated under reduced pressure and purified by column chromatography ($SiO_2$, 20% hexanes in ethyl acetate) to afford the title compound. MS (DCI/$NH_3$) m/z 407 $(M+H)^+$.

Example 303B

N-[(2Z)-5-tert-butyl-3-[((cis)-3-hydroxy-3-methylcyclobutyl)methyl)-1,3-thiazol-2(3H)-ylidene]-5-chloro-2-methoxybenzamide

To Example 302A (0.21 g, 0.52 mmol) in 10 mL of tetrahydrofuran at -78 °C was added a 1.6 M solution of methyllithium in diethyl ether (1.0 mL, 1.6 mmol) dropwise over 5 minutes. The mixture was stirred at -78 °C for 2 hours and was slowly warmed to ambient temperature and was allowed to stir for 18 hours. The mixture was quenched with 5 mL of saturated aqueous $NH_4Cl$ and diluted with 5 mL of ethyl acetate. The layers were separated and the aqueous layer was extracted twice with 5 mL of ethyl acetate. The combined organic extracts were dried over anhydrous $Na_2SO_4$, filtered and concentrated under reduced pressure. Purification via column chromatography ($SiO_2$, 30% hexanes in ethyl acetate) afforded the title compound. $^1H$ NMR (300 MHz, $CD_3OD$) $\delta$ ppm 1.32 (s,3H), 1.38(s,9H), 1.90 - 2.00 (m, 2 H), 2.06 - 2.16 (m, 2 H), 2.35 - 2.52 (m, 1 H), 3.86 (s, 3 H), 4.30 (d, *J*=7.1 Hz, 2 H), 7.07 (d, *J*=8.8 Hz, 1 H), 7.12 (s, 1 H), 7.39 (dd, *J*=8.8, 2.7 Hz, 1 H), 7.80 (d, *J*=3.1 Hz, 1 H); MS (DCI/$NH_3$) m/z 423 $(M+H)^+$. Anal. Calculated for $C_{21}H_{27}ClN_2O_3S{\cdot}0.1H_2O$: C, 59.38; H, 6.45; N, 6.59. Found: C, 59.17; H, 6.62; N, 6.28.

Example 304

5-chloro-N-[(2Z)-5-(1-hydroxy-1-methylethyl)-3-[((cis)-3-methoxycyclobutyl)methyl]-4-methyl-1,3-thiazol-2(3H)-ylidene]-2-methoxybenzamide

Example 304A

(Z)-N-(5-acety)-3-(((cis)-3-methoxycyclobutyl)methyl-4-methylthiazol-2(3H)-ylidene)-5-chloro-2-methoxybenzamide

Example 238A (0.57 g, 1.8 mmol), Example 301C (0.48 g, 1.8 mmol) and potassium tert-butoxide (0.42 g, 3.5 mmol) were processed as described in the procedure for Example 238B to afford the title compound. MS (DCI/$NH_3$) m/z 423 $(M+H)^+$.

Example 304B

5-chloro-N-[(2Z)-5-(1-hydroxy-1-methylethyl)-3-[((cis)-3-methoxycyclobutyl)methyl]-4-methyl-1,3-thiazol-2(3H)-ylidene]-2-methoxybenzamide

Example 304A (20 mg, 0.047 mmol) and methyllithium (1.6 M in ether, 88 $\mu$L, 0.14 mmol) in I mL of tetrahydrofuran were processed as described in Example 239 to afford the title compound. $^1H$ NMR (300 MHz, $CD_3OD$) $\delta$ ppm 1.61 (s, 6 H), 1.80 - 1.87 (m, 2 H), 2.32 - 2.43 (m, 2 H), 2.49 (s, 3 H), 3.20 - 3.24 (m, 1H), 3.20 (s, 3 H), 3.34 - 3.41 (m, I H), 3.68 - 3.78 (m, I H), 3.86 (s, 3 H), 4.38 (d, *J*=6.1 Hz, 2 H), 7.06 (d, *J*=8.8 Hz, 1 H), 7.39 (dd, *J*=8.8, 2.7 Hz, 1 H), 7.86 (d, *J*=2.7 Hz, I H) MS (DCI/$NH_3$) m/z 439 $(M+H)^+$.

Example 305

N-[(2Z)-5-tert-butyl-3-[2-(2-methoxyethoxy)ethyl]-1,3-thiazol-2(3H)-ylidene]-5-chloro-2-methoxybenzamide

Example 305A

5-tert-butyl-3-(2-(2-methoxyethoxy)ethyl)thiazol-2(3H)-imine hydrobromide

A mixture of Example 240A (0.20 g, 1.3 mmol) and 1-bromo-2-(2-methoxyethoxy)ethane (0.27 g, 1.4 mmol) was warmed to 85 °C and was allowed to stir for 24 hours. The mixture was cooled to ambient temperature and the crude solids were triturated with ethanol and ether to afford the title compound. MS (DCI/$NH_3$) m/z 259 $(M+H)^+$.

Example 305B

N-[(2Z)-5-tert-butyl-3-[2-(2-methoxyethoxy)ethyl]-1,3-thiazol-2(3H)-ylidene]-5-chloro-2-methoxybenzamide

Example 305A (0.3 g, 0.88 mmol), triethylamine (0.49 mL, 3.5 mmol) and Example 205B (0.88 mmol) in 10 mL of tetrahydrofuran and 1.5 mL ofN,N- dimethylformamide were processed as described in Example 208D to afford the title compound. $^1$H NMR (300 MHz, $CD_3OD$) δ ppm 1.38 (s,9H),3.31(s,3H),3.47-3.52(m, 2H),3.59-3.65(m,2H),3.87(dd, *J*=5.4Hz,2H),3.86(s,3 3 H), 4.42 (dd, *J*=5.1 Hz, 2 H), 7.07 (d, *J*=8.8 Hz, 1 H), 7.18 (s, 1 H), 7.39 (dd, *J*=8.8, 2.7 Hz, 1 H), 7.82 (d, *J*=2.7 Hz, 1 H); MS (DCI/$NH_3$) m/z 427 $(M+H)^+$. Anal. Calculated for $C_{10}H_{17}ClN_2O_4S$: C, 56.26; H, 6.37; N, 6.56. Found: C, 56.06; H, 5.50; N, 6.43.

Example 306

N-[(2Z)-5-tert-butyl-3-(-methoxypropyl)-1,3-thiazol-2(3H)-ylidene]-5-chloro-2-methoxybenzamide

Example 306A

5-tert-butyl-3-(3-methoxypropyl)thiazol-2(3H)-imine

A mixture of Example 240A (0.20 g, 1.3 mmol) and 1-bromo-3-methoxypropane (0.22 g, 1.4 mmol) was warmed to 85 °C and was allowed to stir for 24 hours. The mixture was cooled to ambient temperature, concentrated under reduced pressure and purified via column chromatography ($SiO_2$, 10% methanol in ethyl acetate then 9:1:0.1 $CH_2Cl_2$ : methanol : $Na_4OH$) to afford the title compound. MS (DCI/$NH_3$) m/z 229 $(M+H)^+$.

Example 306B

N-[(2Z)-5-tert-butyl-3-(3-methoxypropyl)-1,3-thiazol-2 (3H)-ylidene]-5-chloro-2-methoxybenzamide

Example 306A (0.25 g, 0.81 mmol), triethylamine (0.34 mL, 2.4 mmol) and Example 205B (0.81 mmol) in 10 mL of tetrahydrofuran and 1 mL of N,N- dimethylformamide were processed as in Example 208D to afford the title compound. $^1$H NMR (300 MHz), $CD_3OD$) δ ppm 1.38 (s, 9 H), 2.05 - 2.17 (m, 2 H), 3.32 (s, 3 H), 3.41 (t, *J*=5.9 Hz, 2 H), 3.86 (s, 3 H), 4.33 (t, *J*=7.0 Hz, 2 H), 7.07 (d, *J*=8.8 Hz, 1 H), 7.11 (s, 1 H), 7.40 (dd, *J*=9.0, 2.9 Hz, 1 H), 7.89 (d, *J*=2.7 Hz, 1 H); MS (DCI/$NH_3$) m/z 397 $(M+H)^+$. Anal. Calculated for $C_{19}H_{15}ClN_2O_3S$: C, 57.49; H, 6.35; N, 7.06. Found: C, 57.18; H, 6.21; N, 6.94.

Example 307

N-[(2Z)-5-tert-butyl-3-(2-ethoxyethyl)-1,3-thiazol-2(3H)-ylidene]-5-chloro-2-methoxybenzamide

Example 307A

5-tert-butyl-3-(2-ethoxyethyl)thiazol-2(3H)-imine

A mixture of Example 240A (0.17 g, 1.1 mnnol) and 2-(bromoethyl)ether (0.20 g, 1.2 mmol) was wanned to 85 °C and was allowed to stir for 24 hours. The mixture was cooled to ambient temperature, concentrated and purified via column chromatography ($SiO_2$, 10% methanol in ethyl acetate then 9:1:0.1 $CH_2Cl_2$ : methanol : $NH_4OH$) to afford the title compound. MS (DCI/$NH_3$) m/z 229 $(M+H)^+$.

Example 307B

N-[(2Z)-5-tert-butyl-3-(2-ethoxyethyl)-1,3-thiazol-2(3H)-ylidene]-5-chloro-2-methoxybenzamide

Example 307A (0.24 g, 1.05 mmol), triethylamine (0.44 mL, 3.2 mmol) and Example 205B (1.05 mmol) in 15 mL of tetrahydrofuran were processed as in Example 208D to afford the title compound. $^1$H NMR (300 MHz, $CD_3OD$) δ ppm 1.14 (t, *J*=7.0 Hz, 3 H), 1.38 (s, 9 H), 3.52 (q, *J*=6.9 Hz, 2 H), 3.81 (t, *J*=5.3 Hz, 2 H), 3.86 (s, 3 H), 4.41 (t, *J*=5.3 Hz, 2 H), 7.07 (d, *J*=8.8 Hz, 1 H), 7.14 (s, 1 H), 7.39 (dd, *J*=8.8, 2.7 Hz, 1 H), 7.83 (d, *J*=3. Hz, 1 H); MS (DCI/$NH_3$) m/z 397 $(M+H)^+$. Anal. Calculated for $C_{19}H_{25}ClN_2O_3S$: C, 57.49; H, 6.35; N, 7.06. Found: C, 57.34; H, 6.04; N, 6.94.

Example 308

N-[(2*Z*)-5-*tert*-butyl-3-(3-hydroxy-3-methylbutyl)-1,3-thiazol-2(3*H*)-ylidene]-5-chloro-2-methoxybenzamide

Example 308A

3-hydroxy-3-methylbutyl 4-methylbenzenesulfonate

To a solution of 3-methylbutane-1,3-diol (2.13 mL, 20 mmol) in pyridine (20 mL) at 0 °C was added para-toluenesulfonyl chloride (3.8 g, 20 mmol) in pyridine (10 mL) drop-wise over 15 minutes. This mixture stirred at ambient temperature for 3 hours and 35 mL $H_2O$ was added and the mixture was extracted with ethyl acetate (2 x 50 mL). The combined organics were washed with $H_2O$ (2 x 50 mL), dried over $MgSO_4$, filtered, concentrated under reduced pressure and dried under vacuum (~1 mm Hg) to afford the title compound. MS (ESI) m/z 276 $(M+18)^+$.

Example 308B

N-[(2Z)-5-tert-butyl-3-(3-hydroxy-3-methylbutyl)-1,3-thiazol-2(3H)-ylidene]-5-chloro-2-methoxybenzamide

A solution of Example 244A (75 mg, 0.2 3mmol) in N,N-dimethylformamide (2 mL) was treated with NaH 60% dispersion in oil (9.5 mg, 0.23 mmol) followed by Example 308A (60 mg, 0.23 mmol). The reaction mixture was stirred at room temperature for 18 hours, poured into water and extracted with ethyl acetate. The organic layer was washed with $H_2O$ (2 x 50 mL), dried over $MgSO_4$, filtered, concentrated under reduced pressure and dried under vacuum (~1 mm Hg) to afford the title compound. $^1H$ NMR (300 MHz, dimethylsulfoxide-$d_6$) $\delta$ ppm 1.17 (s, 6 H), 1.32 (s, 9 H), 1.76 - 1.91 (m, 2 H), 3.79 (s, 3 H), 4.14 - 4.31 (m, 2 H), 4.43 (s, I H), 7.10 (d, *J*=8.8 Hz, 1 H), 7.31 (s, 1 H), 7.44 (dd, *J*=9.0, 2.9 Hz, 1 H), 7.76 (d, *J*=3.1 Hz, 1H); MS (ESI) m/z 411 $(M+H)^+$ Anal. Calculated for $C_{20}H_{27}ClN_2O_3S$ C, 58.45 H, 6.62 N, 6.82. Found C, 58.30 H, 6.51 N, 6.71.

Example 309

*N*[(2*Z*)-5-*tert*-butyl-3-(3-hydroxy-3-methylbutyl)-4-methyl-1,3-thiazol-2(3*H*)-ylidene]-5-chloro-2-methoxybenzamide

Example 309A

(Z)-N-(5-tert-butyl-4-methylthiazol-2(3H)-ylidene)-5-chloro-2-methoxybenzamide

A mixture of 5-tert-butyl-4-methylthiazol-2(3H)imine (250 mg, 1.5 mmol) in tetrahydrofuran (10 mL) was treated with triethylamine (0.25mL, 1.8 mmol) and 5-chloro-2-methoxybenzoylchloride (307 mg, 1.5 mmol). The reaction mixture was stirred at 60 °C for 18 hours then concentrated under reduced pressure. The residue was diluted with ethyl acetate and $H_2O$. The organic extract was dried over $MgSO_4$, filtered concentrated under reduced pressure and dried under vacuum to afford the title compound (490 mg, 96% yield). MS (ESI) m/z 339 $(M+H)^+$.

Example 309B

*N*-[(2*Z*)-5-*tert*-butyl-3-(3-hydroxy-3-methylbutyl)-4-methyl-1,3-thiazol-2(3*H*)-ylidene]-5-chloro-2-methoxybenzamide

Example 308A and Example 309A were processed using the method described in Example 308B to afford the title compound. $^1H$ NMR (300 MHz, dimethylsulfoxide-$d_6$) $\delta$ ppm 1.19 (s, 6 H), 1.38(s,9H), 1.65-1.81 (m, 2 H), 2.4 (s, 3 H), 3.78 (s, 3 H), 4.17 - 4.33 (m, 2 H), 4.49 (s, I H), 7.09 (d, *J*=8.8 Hz, 1 H), 7.43 (dd, *J*=8.8, 3.1 Hz, 1 H), 7.78 (d, *J*=2.7 Hz, 1 H). MS (ESI) m/z 425 $(M+H)^+$. Anal. Calculated for $C_{21}H_{29}ClN_2O_3S$ C, 59.35 H, 6.88 N, 6.55. Found C, 58.83 H, 7.13 N, 6.41.

Example 310

N-[(2Z)-5-tert-butyl-3-(2-methoxyethyl)-1,3-thiazol-2(3H)-ylidene]-5-chloro-2-methoxybenzamide

Commercially available 2-bromoethyl methyl ether (Aldrich) and Example 244A were processed using the method described in Example 246 to afford the title compound. $^1H$ NMR (300 MHz, dimethylsulfoxide-$d_6$) $\delta$ ppm 1.35 (s, 9 H), 3.36 (s, 3 H), 3.68 - 3.84 (m, 2 H), 3.90 (s, 3 H), 4.36 (t, *J*=5.1 Hz, 2 H), 6.77 (s, 1 H), 6.90 (d, *J*=8.8 Hz, 1 H),

7.32 (dd, *J*=8.8, 2.7 Hz, 1 H), 7.95 (d, *J*=2.7 Hz, 1 H); MS ($ESI^+$) m/z 383 $(M+H)^+$; Anal. Calculated for $C_{18}H_{23}ClN_2O_3S$: C, 56.46; H, 6.05; N, 7.32. Found: C, 56.69; H, 6.02; N, 7.42.

Example 311

5-chloro-N-[(2Z)-5-(1-hydroxy-1-methylethyl)-3-(2-methoxyethyl)-4-methyl-1,3-thiazol-2(3H)-ylidene]-2-methoxybenzamide

Example 311A

(Z)-N-(5-acetyl-3-(2-methoxyethyl)-4-methylthiazol-2(3H)-ylidene)-5-chloro-2-methoxybenzamide

Commercially available 2-bromoethyl methyl ether (Aldrich) and Example 238A were processed using the method described in Example 246 to afford the title compound. MS ($ESI^+$) m/z 383 $(M+H)^+$.

Example 311B

5-chloro-N-[(2Z)-5-(1-hydroxy-1-methylethyl)-3-(2-methoxyethyl)-4-methyl-1,3-thiazol-2(3H)-ylidene]-2-methoxybenzamide

Example 311A and commercially available methyllithium (Aldrich, 1.6 M in diethyl ether) were processed using the method described in Example 255 to afford the title compound. $^1H$ NMR (300 MHz, dimethylsulfoxide-$d_6$) $\delta$ ppm 1.50 (s, 6 H), 2.42 (s, 3 H), 3.25 (s, 3 H), 3.68 (t, *J*=5.4 Hz, 2 H), 3.78 (s, 3 H), 4.30 (t, *J*=5.4 Hz, 2 H), 5.62 (s, 1 H), 7.09 (d, *J*=9.2 Hz, 1 H), 7.43 (dd, 1 H), 7.63 (d, *J*=2.7 Hz, 1 H); MS ($ESI^+$) m/z 399 $(M+H)^+$; Anal. Calculated for $C_{18}H_{23}ClN_2O_4S$: C, 54.20; H, 5.81; N, 7.02. Found: C, 54.30; H, 5.68; N, 6.91.

Example 312

N-[(2Z)-5-tert-butyl-3-(2-methoxy-2-methylpropyl)-1,3-thiazol-2(3H)-ylidene]-5-chloro-2-methoxybenzamide

Example 244A and 1-bromo-2-methoxy-2-methylpropane were processed using the method described in Example 246 to afford the title compound. $^1H$ NMR (500 MHz, $CDCl_3$) $\delta$ ppm 1.23 (s, 6 H) 1.36 (s, 9 H) 3.22 (s, 3 H) 3.89 (s, 3 H) 4.32 (s, 2 H) 6.89 (s, 1H) 6.91 (s, 1 H) 7.33 (dd, *J*=8.9, 2.8 Hz, 1 H) 7.92 (d, *J*=2.8 Hz, 1H); MS (ESI) m/z 411 $(M+H)^+$.

Example 313

*N*-[(2*Z*)-3-butyl-5-(1-hydroxy-1-methylethyl)-4-methyl-1,3-thiazol-2(3*H*)-ylidene]-5-chloro-2-methoxybenzamide

Example 313A

(Z)-N-(5-acetyl-3-butyl-4-methylthiazol-2(3H)-ylidene)-5-chloro-2-methoxybenzamide

A mixture of Example 238A (0.40 g, 1.2 mmol), 1-brornobutane (0.16 mL, 1.5 mmol), and potassium t-butoxide (0.22 g, 1.9 mmol) in 5 mL N,N-dimethylformamide was warmed to 65 °C and stirred for 20 hours. The mixture was cooled to ambient temperature quenched with 5 mL of saturated aqueous $NH_4Cl$ and diluted with 5 mL of ethyl acetate. The layers were separated and the aqueous phase was extracted twice with 5 mL of ethyl acetate. The combined organic extracts were dried over anhydrous $Na_2SO_4$, filtered and concentrated under reduced pressure. Purification via column chromatography ($SiO_2$, 40% hexanes in ethyl acetate) afforded the title compound. $^1H$ NMR (300 MHz, $CD_3OD$) $\delta$ ppm 1.04 (t, *J*=7.3 Hz, 3 H), 1.42 - 1.55 (m, 2 H), 1.77 - 1.90 (m, 2 H), 2.51 (s, 3 H), 2.76 (s, 3 H), 3.88 (s, 3 H), 4.32-4.41 (m, 2H), 7.10 (d, *J*=8.8 Hz, I H), 7.45 (dd, *J*=8.8, 2.7 Hz, 1 H), 7.99 (d, *J*=3.1 Hz, 1 H); MS (DCI/$NH_3$) m/z 381 $(M+H)^+$. Anal. Calculated for $C_{18}H_{21}ClN_2O_3S$: C, 56.76; H, 5.56; N, 7.35. Found: C, 56.68; H, 5.49; N, 7.26.

Example 313B

*N*-[(2Z)-3-butyl-5-(1-hydroxy-1-methylethyl)-4-methyl-1,3-thiazol-2(3H)-ylidene]-5-chloro-2-methoxybenzamide

To the product of Example 313A (90 mg, 0.24 mmol) in 5 mL of THF at -78 °C was added a solution of methyllithium (1.6 M in diethyl ether, 0.44 mL, 0.71 mmol) dropwise over 5 minutes. The mixture was stirred at -78 °C

for 4 hours and was slowly wanned to ambient temperature and allowed to stir for 12 hours. The mixture was quenched with 3 mL of saturated aqueous $NH_4Cl$ and diluted with 5 mL of ethyl acetate. The layers were separated and the aqueous phase was extracted twice with 5 mL ethyl acetate. The combined organic extracts were dried over anhydrous $Na_2SO_4$, filtered and concentrated under reduced pressure. Purification via column chromatography ($SiO_2$, 20% hexanes in ethyl acetate) afforded the title compound. $^1H$ NMR (300 MHz, $CD_3OD$) δ ppm 1.02 (t, *J*=7.3 Hz, 3 H), 1.39 - 1.54 (m, 2 H), 1.61 (s, 6 H), 1.71 - 1.84 (m, 2 H), 2.51 (s, 3 H), 3.85 (s, 3 H), 4.23 - 4.31 (m, 2 H), 7.06 (d, *J*=8.8 Hz, 1 H), 7.39 (dd, *J*=9.0, 2.9 Hz, 1 H), 7.87 (d, *J*=3. Hz, 1 H); MS (DCI/$NH_3$) m/z 397 $(M+H)^+$. Anal. Calculated for $C_{19}H_{25}ClN_2O_3S$: C, 57.49; H, 6.35; N, 7.06. Found: C, 57.36; H, 6.33; N, 6.85.

Example 314

(5-chloro-*N*-[(2Z)-3-(cyclobutylmethyl)-5-(1-hydroxy-1-methylethyl)-4-methyl-1,3-thiazol-2(3*H*)-ylidene]-2-methoxybenzamide

Example 314A

(Z)-N-(5-acetyl-3-(cyclobu£tylmethyl)-4-methylthiazol-2(3H)-ylidene)-5-chloro-2-methoxybenzamide

A mixture of the product of Example 238A (0.75 g, 2.3 mmol), (bromomethyl)cyclobutane (0.31 mL, 2.8 mmol), and potassium t-butoxide (0.41 g, 3.5 mmol) in 7 mL N,N-dimethylfonnamide was wanned to 65 °C and stirred for 16 hours. The mixture was cooled to ambient temperature, quenched with 5 mL of saturated aqueous $NH_4Cl$ and diluted with 10 mL of ethyl acetate. The layers were separated and the aqueous phase was extracted twice with 7 mL of ethyl acetate. The combined organic extracts were dried over anhydrous $Na_2SO_4$, filtered and concentrated under reduced pressure. Purification via column chromatography ($SiO_2$, 40% hexanes in ethyl acetate) afforded the title compound. $^1H$ NMR (300 MHz, $CD_3OD$) δ ppm 1.88 - 2.15 (m, 6 H), 2.51 (s, 3 H), 2.75 (s, 3 H), 2.83 - 2.96 (m, 1 H), 3.89 (s, 3 H), 4.46 (d, *J*=7.5 Hz, 2 H), 7.11 (d, *J*=9.2 Hz, 1 H), 7.45 (dd, *J*=8.8, 2.7 Hz, 1 H), 7.99 (d, *J*=3. Hz, 1 H); MS (DCI/$NH_3$) m/z 393 $(M+H)^+$. Anal. Calculated for $C_{19}H_{21}ClN_2O_3S$: C, 58.08; H, 5.39; N, 7.13. Found: C, 58.06; H, 5.20; N, 7.06.

Example 314B

(5-chloro-*N*-[(2Z)-3-(cyclobutylmethyl)-5-(1-hydroxy-1-methylethy)-4-methyl-1,3-thiazol-2(3*H*)-ylidene]-2-methoxybenzamide

To the product of Example 314A (0.13 g, 0.33 mmol) in 5 mL of tetrahydrofuran at-78 °C was added a solution of methyllithium (1.6 M in diethyl ether, 0.62 mL, 0.99 mmol) dropwise over 5 minutes. The mixture was stirred at -78 °C for 1 hour then slowly wanned to ambient temperature and allowed to stir for 16 hours. The mixture was quenched with 5 mL of saturated aqueous $NH_4Cl$ and diluted with 5 mL of ethyl acetate. The layers were separated and the aqueous phase was extracted twice with 5 mL ethyl acetate. The combined organic extacts were dried over anhydrous $Na_2SO_4$, filtered and concentrated under reduced pressure. Purification via column chromatography ($SiO_2$, 30% hexanes in ethyl acetate) afforded the title compound. $^1H$ NMR (300 MHz, $CD_3OD$) δ ppm 1.61 (s,6H),1.86-2.13(m,6H),2.49(s,3H), 2.79-2.93(m, 1 H),3.86(s,3H),4.37(d, *J*=7.1 Hz, 2 H), 7.06 (d, *J*=8.8 Hz, 1 H), 7.39 (dd, *J*=8.8, 2.7 Hz, 1H), 7.87 (d, *J*=2.7 Hz, 1 H); MS (DCI/$NH_3$) m/z 409 $(M+H)^+$. Anal. Calculated for $C_{20}H_{25}ClN_2O_3S$: C, 58.74; H, 6.16; N, 6.85. Found: C, 58.70; H, 6.12; N, 6.74.

Example 315

N-[(2Z)-5-tert-butyl-3-[(25)-tetrahydrofuran-2-ylmethyl]-1,3-thiazol-2(3H)-ylidene]-5-chloro-2-methoxybenzamide

Example 315A

5-*tert*-Butyl-3-[(S)-1-(tetrahydro-furan-2-yl)methyl]-3*H*-thiazol-2-ylideneamine

To a solution of 3,3-dimethylbutanal (Aldrich) (1.52 mL, 12.1 mmol) and (S)-(tetrahydrofuran-2-yl)methanamine (Aldrich) (1.00 g, 9.89 mmol) in 12 mL of acetonitrile was added 1.20 g of molecular sieves (4 Ä beads, 8-12 mesh). The mixture was stirred for 12 h at 22 °C. The mixture was filtered and to the filtrate was added potassium thiocyanate (1.42 g, 14.6 mmol). The temperature was adjusted at 50 °C and the mixture was stirred until all solids were dissolved, then iodine (5.58 g, 22.0 mmol) was added. The reaction was stirred at 50 °C for 12 h. The reaction was cooled to room temperature and diluted with EtOAc. The solution was washed with a solution of sodium bisulfate. The aqueous layers

was brought to pH = 9 by adding aq. NaOH (25%) and extracted with EtOAc. The organic extracts were combined, dried with sodium sulfate, filtered, and concentrated. The residue was crystallized from EtOAc/Hexane to give the title compound. MS ($ESI^+$) m/z 241 $(M+H)^+$.

Example 315B

N-[(2Z)-5-tert-butyl-3-[(2S)-tetrahydrofuran-2-ylmethyl]-1,3-thiazol-2(3H)-ylidene]-5-chloro-2-methoxybenzamide

The products from Example 315A and 5-chloro-2-methoxy-benzoic acid were processed using the method described in Example 300 to afford the title compound. $^1H$ NMR (300 MHz, $CDCl_3$) δ ppm 1.35 (s, 9 H), 1.62 - 1.95 (m, 3 H), 2.00 - 2.12 (m, 1 H), 3.72 - 3.87 (m, 2 H), 3.90 (s, 3 H), 4.18 - 4.34 (m, 2 H), 4.42 (dd, *J*=12.0, 2.4 Hz, I H), 6.86 (s, 1 H), 6.90 (d, *J*=8.8 Hz, I H), 7.32 (dd, *J*=8.8, 2.7 Hz, 1H), 7.95 (d, *J*=2.7 Hz, 1 H). MS ($ESI^+$) m/z 409 $(M+H)^+$.

Example 316

5-chloro-2-methoxy-N-[(2Z)-4,4,6,6-tetramethyl-3-[(2R)-tetrahydrofuran-2-ylmethyl]-4,6-dihydrofuro[3,4-d][1,3]thiazol-2(3H)-ylidene]benzamide

Example 316A

4,4,6,6-Tetramethyl-3-[(R)-1-(tetrahydro-furan-2-yl)methyl]-4,6-dihydro-3*H*-furo[3,4-*d*]thiazol-2-ylideneamine

2,2,5,5-tetramethyldihydrofuran-3(2H)-one (Aldrich) and (R)-(tetrahydrofuran-2-yl)methanamine (Aldrich) were processed using the method described in Example 315A to afford the title compound. MS ($ESI^+$) m/z 283 $(M+H)^+$.

Example 316B

5-chloro-2-methoxy-N-[(2Z)-4,4,6,6-tetramethyl-3-[(2R)-tetrahydrofuran-2-ylmethyl]-4,6-dihydrofuro[3,4-d][1,3]thiazol-2(3H)-ylidene]benzamide

The product from Example 316A and 5-chloro-2-methoxy-benzoic acid were processed using the method described in Example 300 to afford the title compound. $^1H$ NMR (300 MHz, $CDCl_3$) δ ppm 1.52 (s, 3 H), 1.55 (s, 3 H), 1.58 (s, 3 H), 1.66 (s, 3 H), 1.70 - 1.82 (m, I H), 1.90 - 2.02 (m, 2 H), 2.10 - 2.24 (m, 1 H), 3.70 - 3.82 (m, 2 H), 3.83 - 3.89 (m, 1 H), 3.90 (s, 3 H), 4.42 (dd *J*=1 3.6, 3.7 Hz, 1 H), 4.54 - 4.66 (m, 1 H), 6.91 (d, *J*=8.8 Hz, 1 H), 7.35 (dd, *J*=8. 8, 2.7 Hz, 1 H), 7.99 (d, *J*=2.7 Hz, 1 H). MS ($ESI^+$) m/z 451 $(M+H)^+$.

Example 317

N-[(2Z)-5-tert-butyl-3-[(2R)-tetrahydrofuran-2-ylmethyl]-1,3-thiazol-2(3H)-ylidene]-5-cyano-2-methoxybenzamide

Example 317A

5-*tert*-Butyl-3-[(R)-1-(tetrahydro-furan-2-yl)methyl]-3*H*-thiazol-2-ylideneamine

3,3-dimethylbutanal (Aldrich) and (R)-(tetrahydrofuran-2-yl)methanamine (Aldrich) were processed using the method described in Example 315A to afford the title compound. MS ($ESI^+$) m/z 241 $(M+H)^+$.

Example 317B

5-cyano-2-methoxy-benzoic acid methyl ester

A mixture of 3-bromo-4-methoxybenzonitrile (Aldrich) (10.0 g, 47.2 mmol), $PdCl_2$(dppf)-$CH_2Cl_2$ adduct (Aldrich) (1.00 g, 1.22 mmol) and triethylamine (12.5 mL, 89.7 mmol) in 100 mL of methanol in a high pressure vessel was heated to 100 °C at 60 psi of $CO_2$ for 4 h. The mixture was cooled to room temperature and filtered. The mixtwe was concentrated under reduced pressure to afford the title compound. $^1H$ NMR (500 MHz, $CDCl_3$ δ ppm 3.91 (s, 3 H), 3.98 (s, 3 H), 7.06 (d, *J*=8.5 Hz, 1 H), 7.76 (dd, *J*=8.7, 2.3 Hz, 1 H), 8.10 (d, *J*=2.1 Hz, 1 H).

Example 317C

5-cyano-2-methoxy-benzoic

A mixture of Example 317B (6.10 g, 31.9 mmol) and lithium hydroxide monohydrate (5.36 g, 128 mmol) in 150 mL of THF/water (2/1) was stirred at 22 °C for 3 h. The organic solvent was evaporated under reduced pressure and the aqueous solution was acidified to pH 2 with 6 N HCl. The mixture was extracted with dichloromethane. The organic extracts were dried over sodium sulfate, filtered, and concentrated under reduced pressure to afford the title compound. $^1$H NMR (500 MHz, $CDCl_3$) $\delta$ ppm 4.15 (s, 3 H), 7.17 (d, *J*=8.8 Hz, 1 H), 7.86 (dd, *J*=8.7, 2.3 Hz, 1 H), 8.47 (d, *J*=2. Hz, 1 H), 9.50 - 10.21 (brs, 1 H).

Example 317D

N-[(2Z)-5-tert-butyl-3-[(2R)-tetrahydrofuran-2-ylmethyl]-1,3-thiazol-2(3H)-ylidene]-5-cyano-2-methoxybenzamide

The products from Example 317A and Example 317C were processed using the method described in Example 300 to afford the title compound. $^1$H NMR (300 MHz, $CDCl_3$) $\delta$ ppm 1.36 (s, 9 H), 1.60- 1.97 (m, 3 H), 2.01 -2.16(m, 1 H), 3.73 - 3.89 (1m 2 H), 3.97 (s, 3 H), 4.18-4.34 (m 2 H), 4.39 (dd, *J*=12.0,2.4 Hz, 1 H), 6.90 (s, 1 H), 7.03 (d, *J*=8.8 Hz, 1 H), 7.67 (dd, *J*=8.8, 2.4 Hz, 1 H), 8.29 (d, *J*=2.4 Hz, 1 H). MS (ESI$^+$) m/z 400 $(M+H)^+$.

Example 318

N-[(2Z)-4-tert-butyl-3-[(2R)-tetrahydrofuran-2-ylmethyl]-1,3-thiazol-2(3H)-ylidene]-5-chloro-2-methoxybenzamide

Example 318A

4-*tert*-Butyl-3-[(R)-1-(tetrahydro-furan-2-yl)methyl]-3*H*-thiazol-2-ylideneamine

3,3-dimethylbutan-2-one (Aldrich) and (R)-(tetrahydrofuran-2-yl)methanamine (Aldrich) were processed using the method described in Example 315A to afford the title compound. MS (ESI$^+$) m/z 241 $(M+H)^+$.

Example 318B

N-[(2Z)-4-tert-butyl-3-[(2R)-tetrahydrofuran-2-ylmethyl]-1,3-thiazol-2(3H)-ylidene]-5-chloro-2-methoxybenzamide

The product from Example 318A and 5-chloro-2-methoxy-benzoic acid were processed using the method described in Example 300 to afford the title compound. $^1$H NMR (300 MHz, $CDCl_3$) - 2.23 (m, I H), 3.67-3.76 (m, 1H), 3.83-3.89 (m, 1 H), 3.90 (s, 3 H), 4.40 (dd, *J*=15.0, 7.5 Hz, 1 H), 4.57 - 4.68 (m, 2 H), 6.35 (s, 1 H), 6.91 (d, *J*=8.7 Hz, I H), 7.33 (dd, *J*=8.7, 2.8 Hz, 1 H), 8.04 (d, *J*=2.8 Hz, 1H). MS (ESI$^+$) m/z 409 $(M+H)^+$.

Example 319

N-[(2Z)-5-tert-butyl-4-methyl-3-[(2R)-tetrahydrofuran-2-ylmethyl]-1,3-thiazol-2(3H)ylidene]-5-chloro-2-methoxybenzamide

Example 319 A

N-(5-tert-butyl-4-methylthiazol-2-yl)-5-chloro-2-methoxybenzamide

To 5-tert-butyl-4-methylthiazol-2-amine (Matrix, 204mg, 1.2mmol) in $CH_2Cl_2$ (10ml) at 0 °C was added Example 205B (246mg, 1.2mmol), followed by triethylamine (0.2ml, 1,44mmol). The mixture was stirred at room temperature for 4 hours and checked with LC/MS. When the starting material disappeared, water (20ml) and $CH_2Cl_2$ (20ml) were added. The organic layer was washed with brine and concentrated under reduced pressure. The residue was purified by chromatography on a silica gel cartridge, eluting with ethyl acetate/hexane in 5-30% gradient to yield the title compound (305mg, 75%). $^1$H NMR (300 MHz, DMSO-$d_6$) $\delta$ ppm 11.64 (s, 1 H), 7.47 - 7.77 (m, 2 H), 7.22 (d, *J*=8.82 Hz, 1 H), 3.89 (s, 3 H), 2.34 (s, 3 H), 1.39 (s, 9 H). MS (ESI) *m*/*z* 339 $[M+H]^+$, 337{M-H]$^-$.

Example 319B

N[(2Z)-5-tert-butyl-4-methyl-3-[(2R)-tetrahydrofuran-2-ylmethyl]-1,3-thiazol-2(3H)-ylidene]-5-chloro-2-methoxybenzamide

To the mixture of Example 319A (160mg, 0.472mnnol), Example 208A (182mg, 0.71 mmol) and $K_2CO_3$ (131mg, 0.944mmol) in toluene (6ml) were added phase transfer agents of n-$Bu_4$NI (5%) and n-$Bu_4NHSO_4$ (5%). The reaction mixture was heated at 100 °C for 24 hrs, and then cooled to ambient temperature and filtered. The solid was washed with ethyl acetate (10 mL x 2) and the filtrate was concentrated under reduced pressure. The residue was purified by preparative reverse phase HPLC using a gradient of 10 % to 100 % acetonitrile: ammonium acetate (10 mM) to afford the title compound (101 mg, 50.6%). $^1$H NMR (300 MHz, DMSO-d6) $\delta$ppm 7.64 (d, *J*=2.71 Hz, I H), 7.40-7.46 (m, 1 H), 7.09 (d, *J*=8.81 Hz, 1 H), 4.23 - 4.38 (m, 2 H), 4.03 - 4.13 (m, 1 H), 3.74 - 3.83 (m, 4 H), 3.59 - 3.68 (m, 1 H), 2.41 (s, 3 H), 1.63 - 2.02 (m, 4 H), 1.38 (s, 9 H); MS (ESI) *m*/*z* 423 $[M+H]^+$,

Example 320

5-chloro-2-methoxy-N-{(2Z)-3-[(2R)-tetrahydrofuran-2-ylmethyl]-3,4,5,6,7,8-hexahydro-2H-4,7-epoxycyclohepta[d][1,3]thiazol-2-ylidene}benzamide

Example 320A

3-[(2*R*)-tetrahydrofuran-2-ylmethyl]-3,4,5,6,7,8-hexahydro-2*H*-4,7-epoxycyclohepta[d][1,3]thiazol-2-imine

A mixture of 8-oxabicyclo[3.2.1]octan-2-one (obtained as described in Vogel et al. Tetrahedron 1993, 49 (8), 1649-1664) (0.53 g, 4.2 mmol), (R)-(tetrahydrofuran-2-yl)methanamine (0.43 mL, 4.2 mmol) and 1 g of 4 A molecular sieves (8-12 mesh beads) in acetonitrile (4 mL) was stirred at ambient temperature for 16 h. The material was filtered through Celite® with acetonitrile (additional 10 mL) then the filtrate treated with potassium thiocyanate (0.54 g, 5.6 mmol) was added and the mixture was warmed to 50 °C. Iodine (2.1 g, 8.4 mmol) was added and the mixture stirred at 50 °C for 72 h. The mixture was cooled to ambient temperature and was concentrated under reduced pressure. The crude material was diluted with 20 mL $CH_2Cl_2$ and was stirred with sodium metabisulfite (20 mL of 20% aqueous solution) for 15 min. The layers were separated and the aqueous layer was extracted 3 X 10 mL $CH_2Cl_2$. The combined organics were dried over $Na_2SO_4$, filtered and concentrated under reduced pressure to give the crude material (1.0 g, 3.8 mmol, 89% yield) which was carried on without further purification. MS (DCI/$NH_3$) m/z 267 $(M+H)^+$.

Example 320B

5-chloro-2-methoxy-*N*-{(2*Z*)-3[(2*R*)-tetrahydrofuran-2-ylmethyl]-3,4,5,6,7,8-hexahydro-2*H*-4,7-epoxycyclohepta[*d*][1,3]thiazol-2-ylidene}benzamide

To a solution of the product of Example 320A (1.0 g, 3.8 mmol) in THF (25 mL) was added triethylamine (1.6 mL, 11.3 mmol). To this mixture was added Example 205B (0.77 g, 3.8 mmol). The mixture was wanned to 50 °C and allowed to stir for 16 hours. The mixture was cooled to ambient temperature then was quenched with saturated, aqueous $NaHCO_3$, extracted with EtOAc, dried over $Na_2SO_4$, filtered and concentrated under reduced pressure. The mixture was purified by column chromatography ($SiO_2$, 40% hexanes in EtOAc) and then was further purified by HPLC (Waters XTerra RP185$\mu$ column, 30x100 mm, 40 mL/min flow rate, 5-95% gradient of acetonitrile in 0.1 % aqueous trifluoroacetic acid over 22 min, UV detection at 254 nm) to give the title compound (0.40 g, 0.92 mmol, 25% yield). $^1$H NMR (300 MHz, $CDCl_3$) $\delta$ ppm 1.64 - 2.02 (m, 4 H), 2.08 - 2.33 (m, 5 H), 3.19(dt, *J* = 10.9,5.4 Hz, 1 H), 3.69-3.88 (m, 2 H), 3.88-3.95 (m, 1 H), 3.90 (s, 3 H), 4.17 - 4.78 (m, 2 H), 4.80-4.89 (m, 1 H), 5.29 (dd, *J* = 12.2, 5.8 Hz, 1 H), 6.91 (d, *J* = 8.8 Hz, 1 H), 7.36 (ddd, *J* = 8.8, 2.7, 1.4 Hz, 1 H), 7.96 (d, *J* = 2.7 Hz, 1 H); MS (DCI/$NH_3$) m/z 435 $(M+H)^+$; Anal. calculated for $C_{21}H_{13}ClN_2O_4S\cdot0.5\ CF_3CO_2H$: C, 53.71; H, 4.81; N, 5.69. Found: C, 53.95; H, 4.95; N, 5.80.

Example 321

5-chloro-2-methoxy-N-[(2Z)-3-[(2R)-tetrahydrofuran-2-ylmethyl]-6,7-dihydro-4H-pyrano[3,4-d][1,3]thiazol-2(3H)-ylidene]benzamide

Example 321A

(R)-3-((tetrahydrofuran-2-ylmethyl)-3,4,6,7-tetrahydro-2H-pyrano[3,4-d]thiazol-2-imine

Commercially available dihydro-2H-pyran-3(4H)-one (Small Molecules Inc), (R)-(tetrahydrofuran-2-yl)methanamine (Aldrich), potassium thiocyanate (Aldrich) and iodine (Aldrich) were processed using the method described in Example 315A to afford the title compound. LCMS ($ESI^+$) m/z 241 $(M+H)^+$.

Example 321 B

5-chloro-2-methoxy-N-[(2Z)-3-[(2R)-tetrahydrofuran-2-ylmethyl]-6,7-dihydro-4H-pyrano[3,4-d][1,3]thiazol-2(3H)-ylidene]benzamide

Example 32 1 A and 5-chloro-2-methoxybenzoic acid (Aldrich) were processed using the method described in Example 58 to afford the title compound. $^1H$ NMR (300 MHz, DMSO-$d_6$) $\delta$ ppm 1.49 - 1.74 (m, 1 H), 1.75 - 1.89 (m, 2 H), 1.91 - 2.06 (m, 1 H), 2.67 (t, *J*=5.4 Hz, 2 H), 3.57 - 3.68 (m, 1 H), 3.72 - 3.83 (m, 1 H), 3.79 (s, 3 H), 3.87 - 3.95 (m, 3 H), 4.16 - 4.35 (m, 2 H), 4.59 - 4.78 (m, 2 H), 7.12 (d, *J*=8.8 Hz, 1 H), 7.46 (dd, *J*=8.8, 2.7 Hz, 1 H), 7.69 (d, *J*=2.7 Hz, 1 H); MS ($ESI^+$) m/z 409 $(M+H)^+$; Anal. Calculated for $C_{19}H_{21}ClN_2O_4S$: C, 55.81; H, 5.18; N, 6.85. Found: C, 55.88; H, 5.00; N, 6.84.

Example 322

5-chloro-2-methoxy-N-{(2Z)-1-[(2R)-tetrahydrofuran-2-ylmethyl-1,4,6,7-tetrahydro-2H-pyrano[4,3-d][1,3]thiazol-2-ylidene}benzamide

Example 322A

6,7-dihydro-4H-pyrano14,3-d]thiazol-2-amine

Commercially available dihydro-2H-pyran-4(3H)-one (Aldrich), pyrrolidine (Aldrich), *p*-toluenesulfonic acid monohydrate (Aldrich) sulfur (Aldrich) and cyanamide (Aldrich) were processed using the method described in Example 240A to afford the title compound MS ($ESI^+$) m/z 157 $(M+H)^+$.

Example 322B

(Z)-5-chloro-N-(6,7-dihydro-1H-pyrano[4,3-d]thiazol-2(4H)-ylidene)-2-methoxybenzamide

Example 322A and 5-chloro-2-methoxybenzoic acid (Aldrich) were processed using the method described in Example 58 to afford the title compound MS ($ESI^+$) m/z 325 $(M+H)^+$.

Example 322C

5-chloro-2-methoxy-N-{(2Z)-1-[(2R)-tetrahydrofuran-2-ylmethyl]-1,4,6,7-tetrahydro-2H-pyrano[4,3-d][1,3]thiazol-2-ylidene}benzamide

Example 322B and Example 208A were processed using the method described in Example 246 to afford the title compound. $^1H$ NMR (300 MHz, DMSO-$d_6$) $\delta$ ppm 1.60 - 2.05 (m, 4 H), 2.64 - 2.88 (m, 2 H), 3.56 - 3.69 (m, 1 H), 3.73 - 3.84 (m, 1 H), 3.79 (s, 3 H), 3.95 (t, *J*=5.6 Hz, 2 H), 3.97 - 4.12 (m, 1 H), 4.19 - 4.34 (m, 2 H), 4.58 (s, 2 H), 7.12 (d, *J*=9.2 Hz, 1 H), 7.46 (dd, *J*=8.8, 2.7 Hz, I H), 7.69 (d, *J*=2.7 Hz, 1 H); MS ($ESI^+$) m/z 409 $(M+H)^+$; Anal. Calculated for $C_{19}H_{21}ClN_2O_4S$: C, 55.81; H, 5.18; N, 6.85. Found: C, 55.77; H, 4.93; N, 6.72.

Example 323

N-[(2Z)-5-tert-buty)-3-(2-morpholin-4-ylethyl)-1,3-thiazol-2(3H)-ylidene]-5-chloro-2-methoxybenzamide

Example 323A

5-tert-butyl-3-(2-morpholinoethyl)thiazol-2-(3H)-imine

Commercially available 3,3-dimethylbutanal (Aldrich), 2-morpholinoethanamine (Aldrich), potassium thiocyanate (Aldrich) and iodine (EMD chemicals) were processed using the method described in Example 315A to afford the title compound. MS ($ESI^+$) m/z 270 $(M+H)^+$.

Example 323B

N-[(2Z)-5-tert-butyl-3-(2-morholin-4-ylethyl)-1,3-thiazol-2(3H)-ylidene]-5-chloro-2-methoxybenzamide

Example 323A and Example 205B were processed using the method described in Example 244A to afford the title compound $^1H$ NMR (300 MHz), DMSO-$d_6$) $\delta$ ppm 1.32 (s, 9 H), 2.39 - 2.49 (m, 4 H), 2.69 (t, *J*=6.3 Hz, 2 H), 3.46 - 3.58 (m, 4 H), 3.78 (s, 3 H), 4.25 (t, *J*=6.5 Hz, 2 H), 7.10 (d, *J*=8.7 Hz, 1 H), 7.30 (s, 1 H), 7.44 (dd, *J*=8.7,2.8 Hz, 1 H), 7.66 (d, *J*=2.8 Hz, 1 H); MS ($ESI^+$) m/z 438 $(M+H)^+$.

Example 324

N-[(2Z)-5-tert-butyl-3-[(2R)-tetrahydrofuran-2-ylmethyl]-1,3-thiazol-2(3H)-ylidene]-5-chloro-2-hydroxybenzamide

Example 317A and 5-chloro-2-hydroxybenzoic acid (Aldrich) were processed using the method described in Example 58 to afford the title compound. $^1H$ NMR (300 MHz, DMSO-$d_6$) $\delta$ ppm 1.34 (s, 9 H), 1.59 - 1.75 (m, 1 H), 1.77 - 1.94 (m, 2 H), 1.94 - 2.08 (m, 1 H), 3.58 - 3.72 (m, 1 H), 3.75 - 3.88 (m, 1 H), 4.23 - 4.34 (m, 3 H), 6.94 (d, *J*=8.8 Hz, 1 H), 7.37 - 7.48 (m, 1 H), 7.45 (s, 1 H), 7.95 (d, *J*=2.7 Hz, 1 H), 13.02 (s, 1 H); MS ($ESI^+$) m/z 395 $(M+H)^+$.

Example 325

N-[(2Z)-5-tert-butyl-3-[(2R)-tetrahydrofuran-2-ylmethyl]-1,3-thiazol-2(3H)-ylidene]-2-methoxy-5-(trifluoromethyl)benzamide

Example 317A and 2-methoxy-5-(trifluoromethyl)benzoyl chloride (JRD Fluorochemicals Ltd) were processed using the method described in Example 244A to afford the title compound. $^1H$ NMR (300 MHz, DMSO-$d_6$) $\delta$ ppm 1.32 (s, 9 H), 1.58 - 1.73 (m, 1 H), 1.74 - 1.86 (m, 2 H), 1.85 - 1.96 (m, 1 H), 3.57 - 3.71 (m, 1 H), 3.73 - 3.83 (m, 1 H), 3.87 (s, 3 H), 4.19 (dd, *J*=5.9, 1.6 Hz, 2 H), 4.31 (dd, 1 H), 7.29 (s, 1 H), 7.27 (s, 1 H), 7.77 (dd, *J*=9.1, 2.4 Hz, 1 H), 7.97 (d, *J*=2.4 Hz, 1 H); MS ($ESI^+$) m/z 443 $(M+H)^+$.

Example 326

N-[(2Z)-5-tert-butyl-3-tetrahydro-2H-pyran-4-yl-1,3-thiazol-2(3H)-ylidene]-5-chloro-2-methoxybenzamide

Example 326A

5-tert-butyl-3-(tetrahydro-2H-pyran-4-yl)thiazol-2(3H)-imine

Commercially available 3,3-dimethylbutanal (Aldrich), tetrahydro-2H-pyran-4-amine (Matrix), potassium thiocyanate (Aldrich) and iodine (EMD chemicals) were processed using the method described in Example 315A to afford the title compound. MS ($ESI^+$) m/z 241 $(M+H)^+$.

Example 326B

N-[(2Z)-5-tert-butyl-3-tetrahydro-2H-pyran-4-yl-1,3-thiazol-2(3H)-ylidene]-5-chloro-2-methoxybenzamide

Example 326A and Example 205B were processed using the method described in Example 244A to afford the

title compound. $1^H$ NMR (300 MHz, DMSO-$d_6$) δ ppm 1.32 (s, 9 H), 1.86 (dd, *J*=12.0, 2.5 Hz, 2 H), 2.03 - 2.22 (m, 2 H), 3.46 (t, *J*=11.7 Hz, 2 H), 3.79 (s, 3 H), 3.93 - 4.03 (m, 2 H), 4.81 - 5.16 (m, 1 H), 7.11 (d, *J*=8.8 Hz, 1 H), 7.39 (s, 1 H), 7.44 (dd, *J*=8.8, 2.7 Hz, 1 H), 7.62 (d, *J*=3.1 Hz, 1 H); MS (ESI$^+$) m/z 409 $(M+H)^+$; Anal. Calculated for $C_{10}H_{25}ClN_1O_3S$: C, 58.74; H, 6.16; N, 6.85. Found: C, 58.59; H, 6.24; N, 6.76.

Example 327

N-[(2Z)-5-tert-butyl-3-{2-[(cis)-2,6-dimethylmorpholin-4-yl]ethyl}-1,3-thiazol-2(3H)-ylidene]-5-chloro-2-methoxybenzamide

Example 327A

5-tert-butyl-3-(2-((cis)-2,6-dimethylmorpholino)ethyl)thiazol-2(3H)-imine Commercially available 3,3-dimethylbutanal (Aldrich), 4-(2-aminoethyl)-cis-2,6-dimethylmorpholine (Oakwood), potassium thiocyanate (Aldrich) and iodine (EMD chemicals) were processed using the method described in Example 315A to afford the title compound. MS (ESI$^+$) m/z 298 $(M+H)^+$.

Example 327B

N-[(2Z)-5-tert-butyl-3-{2-[(cis)-2,6-dimethylorphoholin-yl]ethyl}-1,3-thiazol-2(3H)ylidene]-5-chloro-2-methoxybenzamide

Example 327A and 5-chloro-2-methoxybenzoic acid (Aldrich) were processed using the method described in Example 58 to afford the title compound. $^1$H NMR (300 MHz, DMSO-$d_6$) δ ppm 0.97 (s, 3 H), 0.99 (s, 3 H), 1.31 (s, 9 H), 1.68 (t, *J*=10.5 Hz, 2 H), 2.66 (t, *J*=6.4 Hz, 2 H), 2.80 (d, *J*=10.2 Hz, 2 H), 3.35 - 3.50 (m, 2 H), 3.78 (s, 3 H), 4.25 (t, *J*=6.3 Hz, 2 H), 7.10 (d, *J*=8.8 Hz, 1 H), 7.30 (s, 1 H), 7.44 (dd, *J*=8.8, 2.7 Hz, 1 H), 7.67 (d, *J*=2.7 Hz, 1 H); MS (ESI$^+$) m/z 466 $(M+H)^+$; Anal. Calculated for $C_{23}H_{32}ClN_3O_3S{\cdot}0.5H_2O{\cdot}0.1C_4H_8O_2$: C, 58.09; H, 7.04; N, 8.68. Found: C, 57.84; H, 6.96; N, 8.58.

Example 328

N-[(2Z)-5-tert-butyl-3-{[(2R)-5-oxotetrahydrofuran-2-yl]-methyl-1,3-thiazol-2(3H)-ylidene]-5-chloro-2-methoxybenzamide

Example 328A

(R)-5-(tosylmethyl)dihydrofuran-2(3H)-one

(R)-5-(hydroxymethyl)dihydrofuran-2(3H)-one (1.0 g, 8.6 mmol) was processed using the method described in Example 162A to afford the title compound. MS (ESI) m/z 288 $(M+18)^+$.

Example 328B

N-[(2Z)-5-tert-butyl-3-{[(2R)-5-oxotetrahydrofuran-2-yl]methyl}-1,3-thiazol-2(3H)-ylidene]-5-chloro-2-methoxybenzamide

Example 244A and Example 328A were processed using the method described in Example 238B to afford the title compound. 1 H NMR (500 MHz, $CDCl_3$) δ ppm 1.35 (s, 9 H) 2.08 - 2.19 (m, 1 H) 2.34 - 2.48 (m, 2 H) 2.49 - 2.61 (m, 1 H) 3.90 (s, 3 H) 4.39 (dd, *J*=14.34, 6.10 Hz, 1 H) 4.55 (dd, *J*=14.65, 3.05 Hz, 1 H) 4.92 - 4.99 (m, 1 H) 6.75 (s, 1 H) 6.91 (d, *J*=8.85 Hz, 1 H) 7.34 (dd, *J*=8.85, 2.75 Hz, 1 H) 7.92 (d, *J*=2.75 Hz, 1 H); MS (ESI) m/z 423 $(M+H)^+$.

Example 329

5-chloro-N-[(2Z)-5-(1-hydroxy-methylethyl)-4-methyl-3-(2R)-tetrahydrofuran-2-ylmethyl]-1,3-thiazol-2(3H)-ylidene]-2-(2,2,2-trifluoroethoxy)benzamide

Example 280 B was processed using the method described in Example 255 to afford the title compound. 1H NMR (500 MHz, $CDCl_3$) δ ppm 1.63 - 1.74 (m, 1 H) 1.66 (d, *J*=10.68 Hz, 6 H) 1.88 - 1.98 (m, 3 H) 2.08 - 2.16 (m, 1 H)

2.56 (s, 3 H) 3.74 (dd, *J*=14.04, 7.32 Hz, 1 H) 3.88 (dd, *J*=15.26, 7.02 Hz, 1 H) 3.97 (dd, *J*=14.04, 7.93 Hz, 1 H) 4.33 - 4.40 (m, 1 H) 4.46 (dd, *J*=17.09, 8.54 Hz, 2 H) 4.55 (dd, *J*=13.73, 3.05 Hz, 1 H) 7.02 (d, *J*=8.85 Hz, 1 H) 7.33 (dd, *J*=8.85, 2.75 Hz, 1 H) 7.99 (d, *J*=2.75 Hz, 1 H); MS (ESI) m/z 493 $(M+H)^+$.

Example 330

N-[(2Z)-5-tert-butyl-3-[(2R)-tetrahydrofuran-2-ylmethyl]-1,3-thiazol-2(3H)-ylidene1-5-chloro-2-[(E)-(hydroxyimino)methyl]benzamide

A mixture of Example 331B (0.020g, 0.054 mmmol) and hydroxylamine hydrochloride (0.004 g, 0.054 mmol) in pyridine (5 mL) was stirred at room temperature for 8 h. The mixture was concentrated under reduced pressure and the residue was partitioned between EtOAc and water. The organic layer was washed with water, brine, dried with $MgSO_4$, filtered, and concentrated under reduced pressure to afford 17 mg of product.. $^1H$ NMR (300 MHz-DMSO-$d_6$) $\delta$ 1.33 (s, 9H), 1.62 (m, 1H), 1.84 (m, 2H), 1.99 (m, 1H), 3.65 (m, 1H), 3.80 (m, 1H), 4.26 (m, 3H), 7.36 (s, 1H), 7.55 (dd, J = 9 Hz, 3 Hz, 1H), 7.78 (d, J = 9 Hz, 1H), 8.04 (d, J = 3 Hz, 1H), 8.87 (s, 1H), 11.27 (s, 1H); MS (DCI/$NH_3$) m/z 422 $(M+H)^+$.

Example 331

N-[(2Z)-5-tert-butyl-3-[(2R)-tetrahydrofuran-2-ylmethyl]-1,3-thiazol-2(3H)-ylidene]-5-chloro-2-formylbenzamide

Example 331 A

methyl N-[(2Z)-5-*tert*-butyl-3-[(2R)-tetrahydrofuran-2-ylmethyl]-1,3-thiazol-2(3*H*)-ylidene]-*N*'-cyanoimidothiocarbamate

A mixture of Example 240C (0.361 g, 1.5 mmol) and dimethyl cyanocarbonimidodithioate (0.219 g, 1.5 mmol) in THF (35 mL) was treated with triethylamine (0.21 mL, 1.5 mmol) and the resulting mixture was stirred at 45 °C for 12 h. The mixture was concentrated under reduced pressure and the residue was purified by chromatography (hexane-EtOAc 1:1) to afford 430 mg of the title compound. $^1H$ NMR (300 MHz-DMSO-$d_6$) $\delta$ 1.31 (s, 9H), 1.60 (m, 1H), 1.82 (quintet, J = 7 Hz, 2H), 1.95 (m, 1H), 2.53 (s, 3H), 3.65 (m, 1H), 3.75 (m, 1H), 4.26 (m, 3H), 7.43 (s, 1H); MS (DCI/$NH_3$) m/z 339 $(M+H)^+$.

Example 331B

N-[(2Z)-5-tert-butyl-3-[(2R)-tetrahydrofuran-2-ylmethyl]-1,3-thiazol-2(3H)-ylidene]-5-chloro-2-formylbenzamide

To a mixture of Example 331A (0.102 g, 0.3 mmol), 5-chloro-2-formylboronic acid (0.184 g, 1 mmol), copper (1)acetate (0.123 g, 1 mmol) in DME (25 mL) were added tris(dibenzylideneacetone)dipalladium(0) 0.045 g, 0.05 mmol) and triethyl phosphate (0.024 mg, 0.14 mmol) and the mixture was refluxed for 16 h. The mixture was then concentrated under reduced pressure and the residue was chromatographed ($SiO_2$; hexane-EtOAc 1:1) to afford 30 mg of the title compound. $^1H$ NMR (300 MHz-DMSO-$d_6$) $\delta$ 1.33 (s, 9H), 1.62 (m, 1H), 1.84 (m, 2H), 1.99 (m, 1H), 3.65 (m, 1H), 3.80 (m, 1H), 4.26 (m, 3H), 7.36 (s, 1H), 7.70 (m, 2H), 8.06 (d, J = Hz, 1H), 10.53 (s, 1H); MS (DCI/$NH_3$) m/z 407 $(M+H)^+$.

Example 332

N-[(2Z)-5-tert-butyl-3-[(2R)-tetrahydrofuran-2-ylmethyl]-1,3-thiazol-2(3H)-ylidene]-N'-(1,1-dimethylpropyl)urea

Example 332A

(R,Z)4-nitrophenyl 5-tert-butyl-3-((tetrahydrofuran-2-yl)methyl)thiazol-2(3H)-ylidenecarbamate

In a 20 mL vial, Example 317A was dissolved in dichloromethane (5 ml). Triethylamine (0.417 g, 4.12 mmol) was added followed by addition of 4-nitrophenylcarbonyl chloridate (0.755 g, 3.74 mmol) and the reaction was stirred for 2hrs. The reaction was washed with water, dried with sodium sulfate, filtered, and concentrated in vacuo. The residue was purified by silica gel flash chromatography using 0-30% ethyl acetate in hexanes provided the title compound as a white solid. $^1H$ NMR (300 MHz, DMSO-$d_6$) $\delta$ ppm 1.29 (s, 9 H) 1.73 - 1.87 (m, 3 H) 1.87 - 1.96 (m, 1 H) 3.65 (m, 1 H) 3.75 (m, 1 H) 4.03 (m, 2 H) 4.19 (m, 1 H) 7.22 (s, 1 H) 7.41 - 7.49 (m, 2 H) 8.24 - 8.30 (m, 2 H). MS (DCI) m/z 406 $(M + H)^+$.

Example 332B

N-[(2Z)-5-tert-butyl-3-[(2R)-tetrahydrofuran-2-ylmethyl]-1,3-thiaiol-2(3H)-ylidene]-N'-(1,1-dimethylpropyl)urea

To a 10 mL microwave vial, Example 332A (100 mg, 0.247 mmol) was dissolved in acetonitrile (1.5 ml) followed by addition of 2-methylbutan-2-amine (32.2 mg, 0.370 mmol) and heated in a microwave reactor at 140 °C for 10 min. The reaction was concentrated and the residue was purified by reverse phase HPLC using a Waters Sunfire C8 column (30x75mm) eluting with a gradient of acetonitrile and 0.1% trifluoroacetic acid in water at a flow rate of 50 mL/min. Fractions selected by mass spectrometry to provide the title compound as a TFA salt. $^1H$ NMR (300 MHz, DMSO-D6) δ ppm 0.79 (t, *J*=7.34 Hz, 3 H) 1.23 (s, 6 H) 1.27 (s, 9 H) 1.54 (m, I H) 1.67 (m, 2 H) 1.77 - 1.88 (m, 2 H) 1.95 (m, I H) 3.61 - 3.69 (m, I H) 3.75 - 3.83 (m, I H) 3.98 - 4.11 (m, 2 H) 4.19 (m, 1 H) 6.65 (s, 1 H) 7.11 (s, I H). MS (DCI) m/z 354 $(M + H)^+$.

Example 333

N-[(2Z)-5-tert-butyl-3-[(2R)-tetrahydrofuran-2-ylmethyl]-1,3-thiazol-2(3H)-ylidene]-N'-(1-methyl-1-phenylethyl)urea

The title compound was prepared and purified as described in Example 332B, substituting cumylamine for 2-methylbutan-2-amine. $^1H$ NMR (300 MHz, $CDCl_3$) δ ppm 1.20 - 1.30 (m, 9 H) 1.55 (s, 6 H) 1.65 (s, 2 H) 1.79 - 1.90 (m, 2 H) 3.72 - 3.86 (m, 2 H) 3.93 (dd, *J*=14.48, 6.54 Hz, 1 H) 4.11 (s, 1 H) 4.19 (s, 1 H) 5.53 (s, 1 H) 6.56 (s, 1 H) 7.12 - 7.22 (m, 1 H) 7.27 - 7.35 (m, 2 H) 7.46 (d, *J*=7.54 Hz, 2 H). MS (DCI) m/z 402 $(M + H)^+$. Anal. Calculated for $C_{16}H_{16}BrClN_2O_2S$: C, 65.41; H, 7.74; N, 10.23. Found: C, 65.8; H, 7.78; N, 10.46.

Example 334

N-[(2Z)-5-tert-butyl-3-[(2R)-tetrahydrofuran-2-ylmethyl]-1,3-thiazol-2(3H)-ylidene]-N'-(2-hydroxy-_ 1,1-dimethylethyl) urea

The title compound was prepared and purified as described in Example 332B, substituting 2-amino-2-methyl-1-propanol for 2-methylbutan-2-amine. $^1H$ NMR (300 MHz, $CD_3OD$) δ ppm 1.34 (s, 6 H) 1.38 (s, 9H) 1.64 (m, 1 H) 1.88 - 1.98 (m, 2 H) 2.12 (m, 1 H) 3.58 (s, 2 H) 3.76 (m, 1 H) 3.90 (m, 1 H) 4.13 (m, 1 H) 4.26 (m, 2 H) 7.26 (s, 1 H). MS (DCI) m/z 356 $(M + H)^+$.

Example 335

methyl N-({[2Z)-5-tert-butyl-3-[(2R)-tetrahydrofuran-2-ylmethyl]-1,3-thiazol-2(3H)-ylidene]amino}carbonyl)-2-methylalaninate

In a 20 mL microwave vial, Example 332A (60 mg, 0.148 mmol) was dissolved in acetonitrile (1.5 ml), followed by addition of triethylamine (0.045 ml, 0.326 mmol) and 2-amino isobutyric acid methyl ester hydrochloride (45.5 mg, 0.296 mmol). The reaction mixture was microwaved at 120 °C for 20 min. The reaction was concentrated and the residue was purified according to Example 332B. $^1H$ NMR (300 MHz, $CD_3OD$) δ ppm 1.34 (s, 9 H) 1.52 (s, 6 H) 1.63 (m, I H) 1.91 (m, 2 H) 2.09 (m, 1 H) 3.63 - 3.74 (s, 3 H) 3.77 (m, 1 H) 3.90 (m, I H) 4.07 (m, 1 H) 4.23 (m, 2 H), 7.08 (s, 1 H). MS (DCI) m/z 384 $(M + H)^+$. Anal. Calculated for $C_{18}H_{29}N_3O_4S$ 0.75 TFA: C, 49.94; H, 6.39; N, 8.96. Found: C, 50.28; H, 6.02; N, 9.05.

Example 336

N-[(2Z)-5-tert-butyl-3-(1,3-oxazol-2-ylmethyl)-1,3-thiazol-2(3H)-ylidene]-5-chloro-2-methoxybenzamide

Example 244A (0.125 g, 0.386 mmol) in DMF (2 mL) was treated with NaH (11 mg, 0.462 mmol), then 2-(chloromethyl)-oxazole (54 mg, 0.46 mmol) followed by stirring at room temperature overnight. After workup, the product was purified by silica gel chromatography. $^1H$ NMR ($CDCl_3$, 300 MHz) δ ppm 1.33 (s, 9 H), 3.75 (s, 3 H), 5.51 (s, 2 H), 7.08 (d, 1 H), 7.21 (d, I H), 7.42 (s, 1 H), 7.44 (dd, 1H), 7.57 (d, 1H), 8.11 (d, 1 H); MS $(ESI^+)$ m/z 406 $(M+H)^+$.

Example 337

N-[(2Z)-5-tert-butyl-3-(1,2,4-oxadiazol-3 ylmethyl)-1,3-thiazol-2(3H)-ylidene]-5-chloro-2-methoxybenzamide

Example 244A and commercially available 3-(chloromethyl)-1,2,4-oxadiazole (May bridge) were processed using the method described in Example 266 to afford the title compound. $^1H$ NMR (300 MHz, DMSO-$d_6$) δ ppm 1.33 (s, 9 H), 3.74 (s, 3 H), 5.59 (s, 2 H), 7.08 (d, *J*=9.2 Hz, 1 H), 7.39 - 7.49 (m, 1 H), 7.43 (s, I H), 7.59 (d, *J*=2.7 Hz, 1 H), 9.63 (s, 1 H); MS (ESI$^+$) m/z 407 $(M+H)^+$; Anal. Calculated for $C_{18}H_{19}ClN_4O_3S$: C, 53.13; H, 4.71; N, 13.77. Found: C, 53.14; H, 4.57; N, 13.56.

Example 338

N-[(2Z)-5-tert-butyl-3-(2-furylmethyl)-1,3-thiazol-2(3H)-ylidene]-5-chloro-2-methoxybenzamide

Example 338A

5-tert-butyl-3-(furan-2-ylmethyl)thiazol-2(3H)-imine

Commercially available 3,3-dimethylbutanal (Aldrich), 2- furan-2-ylmethanamine (Aldrich), potassium thiocyanate (Aldrich) and iodine (EMD chemicals) were processed using the method described in Example 315A to afford the title compound. MS (ESI$^+$) m/z 237 $(M+H)^+$.

Example 338B

(Z)-N-(5-tert-butyl-3-(furan-2-ylmethyl)thiazol-2(3H)-ylidene-5-chloro-2-methoxybenzamide

Example 338A and Example 205B were processed using the method described in Example 244A to afford the title compound. $^1H$ NMR (300 MHz, DMSO-$d_6$) δ ppm 1.31 (s, 9 H), 3.79 (s, 3 H), 5.37 (s, 2 H), 6.22 - 6.59 (m, 2 H), 7.12 (d, *J*=8.7 Hz, 1 H), 7.30 (s, 1 H), 7.46 (dd, *J*=8.9, 3.0 Hz, 1 H), 7.65 (dd, *J*=1.8, 1.0 Hz, 1 H), 7.73 (d, *J*=2.8 Hz, 1 H); MS (ESI$^+$) m/z 405 $(M+H)^+$.

Example 339

5-chloro-N-[(2Z)-4,5-dimethyl-3-[(3-methylisoxazol-5-yl-)methyl]-1,3-thiazol-2(3H)-ylidene]-2-methoxybenzamide

Example 339A

4,5-dimethyl-3-(prop-2-ynyl)thiazol-2(3H)-imine hydrobromide

To a solution of 4,5-dimethylthiazol-2-amine (2.5 g, 19.5 mmol) in 10 mL toluene was added propargyl bromide (2.78 g, 23.4 mmol). The reaction was heated at 85 °C for 12 h then cooled. The crude material was diluted with diethyl ether and the solid was collected via filtration to give 3.5 g of the title compound which was used without further purification. m/z 167.1 $(M+H)^+$.

Example 339B

5-chloro-*N*-[(2Z)-4,5-dimethyl-3-prop-2-yn-1-yl-1,3-thiazol-2(3*H*)-ylidene]-2-methoxybenzamide

To a solution of Example 339A (0.5 g, 2.03 mmol) in 15 mL acetonitrile was added Example 205B (0.54 g, 2.69 mmol) and triethylamine (0.81 g, 8.12 mmol). The reaction was heated at 65 °C for 6 h, then at room temperature overnight. The crude reaction was triturated with methylene chloride/hexane and the resulting solid collected. The filtrate was chromatographed over silica gel (gradient elution, 20-40% ethyl acetate/hexane) and combined with the material from the aforementioned trituration to give 0.3 g of the title compound. m/z 334.9 $(M+H)^+$.

Example 339C

5-chloro-N-[(2Z)-4,5-dimethyl-3-[(3-methylisoxazol-5-yl)methyl]-1,3-thiazol-2(3H)-ylidene]-2-methoxybenzamide

To a solution of acetaldehyde oxime (0.085 g, 2.24 mmol) in 10 mL chloroform was added N-chlorosuccinimide (0.27 g, 2 mmol) and a drop of pyridine. The solution was stirred at ambient temperature for 4 h when Example 339B (0.15 g, 0.45 mmol) and triethylamine (0.224 g, 2.24 mmol) were added. The reaction was held at ambient temperature overnight, then diluted with ethyl acetate, washed with water, brine, dried over $MgSO_4$, filtered, and concentrated. The material was purified by flash chromatography over silica gel (50% ethyl acetate/hexane) to give 0.075 g of the title compound. $^1H$ NMR (300 MHz, DMSO-$d_6$) $\delta$ ppm 2.20 (s, 3 H) 2.23 (s, 3 H) 2.26 (s, 3 H) 3.77 (s, 3 H) 5.57 (s, 2 H) 6.28 (s, 1 H) 7.10 (d, *J*=9.15 Hz, 1 H) 7.34 - 7.59 (m, 1 H) 7.69 (d, *J*=2.71 Hz, 1 H). m/z 392.0 $(M+H)^+$.

Example 340

5-chloro-2-methoxy-N-[(2Z)-5-methyl-3-{[5-(trifluoromethyl)-2-furyl]methyl}-1,3-thiazol-2(3H)-ylidene]benzamide

Example 340A

5-chloro-2-methoxy-N-(5-methylthiazol-2-yl)benzamide

To a solution of 5-methylthiazol-2-amine (0.25 g, 2.25 mmol) in 15 mL acetonitrile was added Example 205B (0.5 g, 2.7 mmol) and triethylamine (0.45 g, 4.5 mmol). The reaction was heated at reflux for 4 h and then 65 °C overnight. The reaction was cooled, diluted with ethyl acetate and washed with $NaHCO_3$, brine, dried over $MgSO_4$, filtered, and concentrated to give 0.4 g of the crude title compound, which was used without further purification. m/z 282.9 $(M+H)^+$.

Example 340B

5-chloro-2-methoxy-N-[(2Z)-5-methyl-3-{[5-(trifluoromethyl)-2-furyl]methyl}-1,3-thiazol-2(3H)-ylidene]benzamide

To a solution of Example 340A (0.1 g, 0.36 mmol) in 5 mL DMF was added sodium hydride (0.01 g, 0.39 mmol, 95%). The solution was allowed to stir at ambient temperature for 30 min when 2-(bromomethyl)-5-(trifluoromethyl)furan (0.09 g, 0.39 mmol) was added. The reaction was held at ambient temperature overnight and then quenched with water. The crude was extracted with ethyl acetate and the organics washed with water, then dried over $MgSO_4$, filtered, and concentrated. Flash chromatography over silica gel (50% ethyl acetate/hexane) gave 0.05 g of the title compound. $^1H$ NMR (300 MHz, DMSO-$d_6$) $\delta$ ppm 2.28 (d, *J*=1.36 Hz, 3 H) 3.78 (s, 3 H) 5.47 (s, 2 H) 6.66 (d, *J*=2.71 Hz, 1 H) 7.11 (d, *J*=8.81 Hz, 1 H) 7.22 (dd, *J*=3.39, 1.36 Hz, 1 H) 7.31 - 7.39 (m, *J*=1.36 Hz, 1 H) 7.41 - 7.52 (m, 1 H) 7.72 (d, *J*=2.71 Hz, 1 H). m/z 431.0 $(M+H)^+$.

Example 341

5-chloro-N-[(2Z)-3-(2-furylmethyl)-5-methyl-1,3-thiazol-2(3H)-ylidene]-2-methoxybenzamide

To a solution of Example 340A (0.15 g, 0.53 mmol) in 5 mL DMF was added sodium hydride (0.016 g, 0.67 mmol, 95%). After the solution was allowed to stir at ambient temperature for 30 min, 2-(chloromethyl)furan (0.08 g, 0.67 mmol) was added. The reaction was held at ambient temperature overnight and then quenched with water. The crude was extracted with ethyl acetate and the organics washed with water, then dried over $MgSO_4$, filtered, and concentrated. Flash chromatography over silica gel (50% ethyl acetate/hexane) gave 0.075 g of the title compound. $^1H$ NMR (300 MHz, DMSO-$d_6$) $\delta$ ppm 2.26 (d, *J*=1.36 Hz, 3 H) 3.80 (s, 3 H) 5.18 (s, 2 H) 6.54 (s, I H) 7.12 (d, *J*=8.81 Hz, 1 H) 7.29 (d, *J*=1.36 Hz, 1 H) 7.46 (dd, *J*=8.81, 2.71 Hz, 1 H) 7.64 (t, *J*=1.70 Hz, 1 H) 7.67 - 7.76 (m, 2 H). m/z 363.0 $(M+H)^+$.

Example 342

5-chloro-N-[(2Z)-3-(3-furylmethyl)-5-methyl-1,3-thiazol-2(3H)-ylidene]-2-methoxybenzamide

To a solution of Example 340A (0.15 g, 0.53 mmol) in 5 mL DMF was added sodium hydride (0.016 g, 0.67 mmol, 95%). After the solution was allowed to stir at ambient temperature for 30 min, 3-(chloromethyl)furan (0.08 g, 0.67 mmol) was added. The reaction was held at ambient temperature overnight and then quenched with water. The crude was extracted with ethyl acetate and the organics washed with water, then dried over $MgSO_4$, filtered, and concentrated.

Flash chromatography over silica gel (50% ethyl acetate/hexane) gave 0.035 g of the title compound. $^1H$ NMR (300 MHz, DMSO-$d_6$) δ ppm 2.27 (d, *J*=1.36 Hz, 3 H) 3.80 (s, 3 H) 5.38 (s, 2 H) 6.39 - 6.55 (m, 2 H) 7.12 (d, *J*=8.82 Hz, 1 H) 7.26 (d, *J*=1.36 Hz, 1 H) 7.46 (dd, *J*=8.82, 2.71 Hz, 1 H) 7.64 (s, 1 H) 7.75 (d, *J*=2.71 Hz, 1 H). m/z 363.0 $(M+H)^+$.

Example 343

N-[(2Z)-5-tert-butyl-3-[(2R)-tetrahydrofuran-2-ylmethyl]-1,3-thiazol-2(3H)-ylidene]-2-ethoxy-5-(trifluoromethyl)benzamide

Example 343A

*N*-[(2Z)-5-*tert*-butyl-3-[(2*R*)-tetrahydrofuran-2-ylmethyl]-1,3-thiazol-2(3*H*)-ylidene]-2-fluoro-5-(fluoromethyl)benzamide

To the product of Example 240C (0.62 g, 1.5 mmol) in THF (15 mL) was added triethylamine (1.1 mL, 7.7 mmol) followed by 2-fluoro-5-(trifluoromethyl)benzoyl chloride (0.39 mL, 2.6 mmol). This mixture was wanned to 50 °C and was allowed to stir for 4 h. The mixture was cooled to ambient temperature then was quenched with saturated, aqueous $NH_4Cl$ (5 mL) and was diluted with EtOAc (5 mL). The layers were separated and the aqueous layer was extracted with EtOAc (3 X 5 mL). The combined organics were dried over anhydrous $Na_2SO_4$, filtered and concentrated under reduced pressure. The crude material was purified by column chromatography ($SiO_2$, 60% hexanes in EtOAc) to give the title compound (0.44 g, 1.0 mmol, 66% yield). MS (DCI/$NH_3$) m/z 431 $(M+H)^+$.

Example 343B

N-[(2Z)-5-tert-butyl-3-[(2R)-tetrahydrofuran-2-ylmethyl]-1,3-thiazol-2(3H)-ylidene]-2-ethoxy-5-(trifluoromethyl)benzamide

To ethanol (0.13 mL, 2.15 mmol) in 5 mL THF was added potassium tert-butoxide (0.23 g, 2.0 mmol). The mixture was stirred at ambient temperature for 20 min then the product of Example 343A (0.44 g, 1.0 mmol) in THF (10 mL) was added via cannula. The mixture was stirred for 1 h at ambient temperature then was quenched with saturated, aqueous $NH_4Cl$ (5 mL) and was diluted with EtOAc (5 mL). The layers were separated, the aqueous layer was extracted with EtOAc (3 X 5 mL) and the combined organics were dried over anhydrous $Na_2SO_4$, filtered and concentrated under reduced pressure. The crude material was purified by column chromatography ($SiO_2$, 60% hexanes in EtOAc) to give the title compound (0.45 g, 0.99 mmol, 96% yield). $^1H$ NMR (300 MHz, $CDCl_3$) δ ppm 1.36 (s, 9 H), 1.48 (t, *J*=7.0 Hz, 3 H), 1.62 - 1.77 (m, 1 H), 1.76 - 1.95 (m, 2 H), 1.99 - 2.12 (m, I H), 3.73 - 3.90 (m, 2 H), 4.16 - 4.24 (m, 3 H), 4.29 (dt, *J*=13.1, 6.7, 2.5 Hz, I H), 4.42 (dd, *J*=13.6, 3.1 Hz, I H), 6.86 (s, I H), 7.01 (d, *J*=8.5 Hz, I H), 7.59 (dd, *J*=9.0, 2.2 Hz, I H), 8.24 (d, *J*=2.4 Hz, 1 H); MS (DCI/NH3) m/z 457 (M+H)+. Anal. Calculated for $C_{21}H_{27}F_3N_2O_3S$; Calc: C, 57.88; H, 5.96; N, 6.14; Found: C, 57.91; H, 5.91; N, 6.10.

Example 344

5-chloro-2-methoxy-[(2Z)-5-methyl-3-(2-morpholin-4-ylethyl)-1,3-thiazol-2(3H)-ylidene]benzamide

Example 344A

5-methyl-3-(2-morpholinoethyl)thiazol-2(3H)-imine

A mixture of 2-amino-5-methylthiazole (1.0 g, 8.8 mmol), 4-(2-chloroethyl)-morpholine hydrochloride (1.7 g, 9.2 mmol) and $Et_2N$ (3.7 mL, 26 mmol) in DMF (5 mL) was wanned to 80 °C and was allowed to stir for 24 h. The mixture was then cooled to ambient temperature, quenched with saturated aqueous $NaHCO_3$ (5 mL), and diluted with $CH_2Cl_2$ (5 mL). The layers were separated and the aqueous layer was extracted with $CH_2Cl_2$ (3 X 5 mL). The combined organics were dried over anhydrous $Na_2SO_4$, filtered concentrated under reduced pressure and purified via column chromatography ($SiO_2$, 10% $CH_3OH$ in EtOAc then 9:1:0.1 $CH_2Cl_2$:$CH_3OH$:$NH_4OH$) to give the title compound (0.88 g, 3.9 mmol, 44% yield). MS (DCI/$NH_3$) m/z 228 $(M+H)^+$.

Example 344B

5-chloro-2-methoxy-N-[(2Z)-5-methyl-3-(2-morpholin-4-ylethyl)-1,3-thiazol-2(3H)-ylidene]benzamide

To a solution of the product of Example 344A (0.15 g, 0.66 mmol) in THF (10 mL) was added $Et_3N$ (0.28 mL, 2.0 mmol) followed by Example 205B (0.66 mmol) in 5 mL THF via cannula. This mixture was wanned to 50 °C and was stirred for 4 h. The mixture was cooled to ambient temperature, was quenched with saturated, aqueous $NaHCO_3$ (5 mL) and was diluted with EtOAc (5 mL). The layers were separated and the aqueous layer was extracted with EtOAc (3 X 5 mL). The combined organics were dried over anhydrous $Na_2SO_4$, filtered, concentrated under reduced pressure and purified via column chromatography ($SiO_2$, 9:1:0.1 $CH_2Cl_2$:$CH_3OH$:$NH_4OH$) to give the title compound (0.16 g, 0.39 mmol, 59% yield). $^1H$ NMR (300 MHz, $CD_3OD$) δ ppm 2.34 (d, *J*=1.4 Hz, 3 H), 2.59 - 2.65 (m, 4 H), 2.86 (t, *J*=6.4 Hz, 2 H), 3.59 - 3.67 (m, 4 H), 3.86 (s, 3 H), 4.40 (t, *J*=6.3 Hz, 2 H), 7.08 (d, *J*=8.8 Hz, I H), 7.16 (d, *J*=1.4 Hz, 1 H), 7.40 (dd, *J*=8.8, 2.7 Hz, 1 H), 7.85 (d, *J*=3.1 Hz, I H); MS (DCI/NH3) m/z 396 $(M+H)^+$. Anal. Calculated for $C_{18}H_{21}ClN_3O_3S{\bullet}0.5H_2O$; Calc: C, 53.39; H, 5.73; N, 10.38; Found: C, 53.62; H, 5.33; N, 10.00.

Example 345

N-[(2Z)-5-tert-butyl-3-{[(4S)-2-oxo-1,3-oxazolidin-4-y]methyl}-13-thiazol-2(3H)-ylidene]1-5-chloro-2-methoxybenzamide

A mixture of Example 244A (180 mg, 0.55 mmol), (S)-(2-oxo-oxazolidin-4-yl)methyl 4-methylbenzenesulfonate (180 mg, 0.67 mmol), potassium carbonate (153 mg, 1.1 mmol), tetrabutylammonium iodide (10 mg, 0.03 mmol), tetrabutylammonium hydrogen sulfate (10 mg, 0.03 mmol) and tetraethylammonium iodide (10 mg, 0.04 mmol) in toluene (35 mL) was refluxed for 14 h. The mixture was washed with water, brine, dried with $MgSO_4$, filtered, and concentrated under reduced pressure. The residue was purified by chromatography over silica gel (EtOAc as eluent) to afford 100 mg of the title compound. $^1H$ NMR (300 MHz, $CDCl_3$) δ ppm 1.36 (s, 9 H), 3.92 (s, 3 H), 4.11 - 4.26 (m, 2 H), 4.30 - 4.41 (m, 2 H), 4.57 (t, *J*=8.6 Hz, 1H), 6.33 (s, 1 H), 6.62 (s, 1 H), 6.93 (d, *J*=8.8 Hz, 1 H), 7.35 (dd, *J*=9.0, 2.9 Hz, 1 H), 7.79 (d, *J*=2.7 Hz, I H). MS (DCI/$NH_3$) m/z 424 $(M+H)^+$.

Example 346

2-[(1-aminocyclopentyl)methxy]-N-[(2Z)-5-tert-bytyl-3-[(2R)-tetraphydrofuran-2-ylmethyl]-1,3-thiazol-2(3H)-ylidene]-5-(trifluoromethyl)-benzamide thiazol-2(3H)-ylidene]-5-(trifluoromethyl)benzamide

A mixture of Example 343A (215 mg, 0.5 mmol), (1-aminocyclopentyl)methanol (115 mg, 1.0 mmol) and potassimn tert-butoxide (IN solution in THF) (0.75 mL, 0.75 mmol) in THF (15 mL) was stirred at room temperature for 2 h. The mixture was then acidified to pH 5 with acetic acid and concentrated under reduced pressure. The residue was treated with a saturated solution of $NaHCO_3$ and extracted with ethyl acetate. The organic layer was washed with brine, dried with $MgSO_4$, filtered, and concentrated under reduced pressure. The residue was purified by chromatography over silica gel (EtOAc-MeOH 4:1 as eluent) to afford 175 mg of the title compound. $^1H$ NMR (300 MHz, DMSO-$d_6$) δ ppm 1.32 (s, 9 H), 1.37 - 2.08 (m, 14 H), 3.59 - 3.66 (m, 1 H), 3.77 (t, *J*=7.3 Hz, 1 H), 3.92 (s, 2 H), 4.15 - 4.35 (m, 3 H), 7.20 - 7.32 (m, 2 H), 7.74 (dd, *J*=8.7, 2.4 Hz, 1 H), 8.02 (d, *J*=2.4 Hz, 1 H). MS (DCI/$NH_3$) m/z 526 $(M+H)^+$. Anal. calculated for $C_{26}H_{34}F_3N_3O_3S$: C, 59.41 H, 6.52 N, 7.99. Found: C, 59.37 H, 6.74 N, 7.60.

Example 347

N-[(2Z)-5-tert-butyl-3-[(2R)-tetrahydrofuran-2-ylmethyl]-1,3-thiazol-2(3H)-ylidene]-2-{[(3S)-1-(2-oxopropyl)pyrrolidin-3-yl]oxy}-5-(trifluoromethyl)benzamide

Example 347A

*N*-[(2*Z*)-S-*tert*-butyl-3-[(2R)-tetrahydrofuran-2-ylmethyl]-1,3-thiazol-2(3*H*)-ylidene]-2-[(3*S*)-pyrrolidin-3-yloxy]-5-(trifluoromethyl)benzamide

A mixture of Example 343A (860 mg, 2 mmol), (S)-pyrrolidin-3-ol (348 mg, 4.0 mmol) and potassium tert-butoxide (IN solution in THF) (3 mL, 3 mmol) in THF (15 mL) was stirred at room temperature for 1 h. The mixture was then acidified to pH 5 with acetic acid and concentrated under reduced pressure. The residue was treated with a saturated solution of $NaHCO_3$ and extracted with ethyl acetate. The organic layer was washed with brine, dried with $MgSO_4$ filtered,

and concentrated under reduced pressure to afford 950 mg of the crude title compound. MS ($DCI/NH_3$) m/z 498 $(M+H)^+$.

Example 347B

N-[(2Z)-5-tert-butyl-3-[(2R)-tetrahydrofuran-2-ylmethyl]-1,3-thiazol-2(3H)-ylidene]-2-{(3S)-1-(2-oxopropyl)pyrrolidin-3-yl]oxy}-5-(trifluoromethyl)benzamide

To a mixture of Example 347A (400 mg, 0.8 mmol) and $K_2CO_3$ (222 mg, 1.6 mmol) in acetonitrile (35 mL) was added at ambient temperature 1-chloropropan-2-one (91 mg, 1 mmol) and the resulting mixture was stirred at room temperature for 15 h. The mixture was concentrated under reduced pressure, the residue was dissolved in EtOAc, washed with water, brine, dried with $MgSO_4$, filtered, and concentrated under reduced pressure. The residue was purified by chromatography (EtOAc-MeOH 9:1 as eluent) to afford 370 mg of the title compound. $^1H$ NMR (300 MHz, DMSO-$d_6$) $\delta$ ppm 1.29 - 1.36 (m, 9 H), 1.58 - 1.67 (m, 1 H), 1.71 - 1.98 (m, 6 H), 2.04 (s, 3 H), 2.21 - 2.35 (m, *J*=13.6, 6.4 Hz, 1 H), 2.53 - 2.59 (m, *J*=6.4 Hz, 1 H), 2.65 - 2.77 (m, 2 H), 3.01 (dd, *J*=10.3, 6.3 Hz, 1 H), 3.60 - 3.71 (m, 1 H), 3.73 - 3.83 (m, 1 H), 4.14 - 4.35 (m, 3 H), 4.94 - 5.07 (m, 1 H), 7.16 - 7.30 (m, 2 H), 7.72 (dd, *J*=8.8, 2.0 Hz, 1 H), 7.97 (d, *J*=2.0 Hz, 1 H). MS ($DCI/NH_3$) m/z 554 $(M+H)^+$.

Example 348

N-[(2Z)-5-tert-butyl-3-[(2R)-tetrahydrofuran-2-ylmethyl]-1,3-thiazol-2(3H)-ylidene]-2-({-1-[2-(hydroxyimino)propyl]azetidin-3-yl}oxy)-5-(trifluoromethyl)benzamide

Example 348A

*N*-[(2Z)-5-*tert*-butyl-3-[(2*R*)-tetrahydrofuran-2-ylmethyl]-1,3-thiazol-2(3*H*)-ylidene]-2-{[1-(2-oxopropyl)azetidin-3-yl]oxy}-5-(trifluoromethyl)benzamide

To a mixture of Example 382B (485 mg, 1 mmol)and $K_2CO_3$ (348 mg, 2.5 mmol) in acetonitrile (35 mL) was added at ambient temperature 1-chloropropan-2-one (139 mg, 1.5 mmol) and the resulting mixture was stirred at room temperature for 16 h. The mixture was concentrated under reduced pressure, the residue was dissolved in EtOAc, washed with water, brine, dried with $MgSO_4$, filtered, and concentrated under reduced pressure. The residue was purified by chromatography ($CH_2Cl_2$-MeOH 9:1 as eluent) to afford 200 mg of the title compound. MS ($DCI/NH_3$) m/z 540 $(M+H)^+$.

Example 348B

N-[(2Z)-5-tert-butyl-3-[(2R)-tetrahydrofuran-2-ylmethyl]-1,3-thiazol-2(3H)-ylidene]-2-({1-[2-(hydroxyimino)propyl]azetidin-3-yl}oxy)-5-(trifluoromethyl)benzamide

A mixture of Example 348A (162 mg, 0.3 mmol) and hydroxylamine hydrochloride (31 mg, 0.45 mmol) in pyridine (10 mL) was stirred at room temperature for 14 h and then was concentrated under reduced pressure. A saturated solution of sodium bicarbonate was added to the residue and the mixture was extracted with ethyl acetate. The ethyl acetate extract was washed with water, brine, dried with $MgSO_4$, filtered, and concentrated under reduced pressure. Purification by silica gel chromatography (Ethyl acetate-MeOH 9:1) afforded 60 mg of the title compound. $^1H$ NMR (300 MHz, DMSO-$d_6$) $\delta$ ppm 1.33 (s, 9 H), 1.58 - 2.00 (m, 7 H), 3.00 - 3.04 (m, 2 H), 3.10 (s, 2 H), 3.62 - 3.85 (m, 4 H), 4.19 - 4.39 (m, 3 H), 4.94 (t, *J*=5.8 Hz, 1 H), 7.01 (d, *J*=8.8 Hz, I H), 7.22 - 7.33 (m, 1 H), 7.70 (dd, *J*=8.5, 2.0 Hz, I H), 8.01 (d, *J*=2.0 Hz, I H), 10.52 (s, I H). MS ($DCI/NH_3$) m/z 555 $(M+H)^+$.

Example 349

5-chloro-2-(4-chlorophenoxy)-N-[(2Z)-3-(2-methoxyethyl)-5-methyl-1,3-thiazol-2(3H)-ylidene]benzamide

Example 349A

5-chloro-2-fluoro-*N*-[(2Z)-3-(2-methoxyethyl)-5-methyl-1,3-thiazol-2(3*H*)-ylidene]benzamide

Example 140A and 5-chloro-2-fluorobenzoic acid were processed using the method described in Example 223A to afford the title compound. MS (ESI) m/z 329 $(M+H)^+$.

Example 349B

5-chloro-2-(4-chlorophenoxy)-N-[(2Z)-3-(2-methoxyethyl)-5-methyl-1,3-thiazol-2(3H)-ylidene]benzamide

Example 349A and 4-chlorophenol were processed using the method described in Example 280B to afford the title compound. $^1$H NMR (400 MHz, DMSO-$d_6$) δ ppm 2.25 (s, 3 H) 3.18 (s, 3 H) 3.44 (t, *J*=5.22 Hz, 2 H) 4.06 (t, *J*=5.22 Hz, 2 H) 6.87 - 6.94 (m, 2 H) 7.1 (d, *J*=8.90 Hz, I H) 7.18 (d, *J*=1.53 Hz, 1 H) 7.33 - 7.40 (m, 2 H) 7.56 (dd, *J*=8.59, 2.76 Hz, I H) 7.91 (d, *J*=2.76 Hz, 1 H); MS (ESI) m/z 437 $(M+H)^+$.

Example 350

5-chloro-N-[(2Z)-3-(2-methoxyethyl)-5-methyl-1,3-thiazol-2(3H)-ylidene]-2-(tetrahydrofuran-3-yloxy)benzamide

Example 349A and tetrahydrofuran-3-ol were processed using the method described in Example 280B to afford the title compound. $^1$H NMR (500 MHz, DMSO-$d_6$) δ ppm 1.93 - 2.00 (m, 1 H) 2.12 - 2.21 (m, 1 H) 2.28 (s, 3 H) 3.26 (s, 3 H) 3.68 (t, *J*=5.19 Hz, 2 H) 3.70-3.75 (m, 1 H) 3.75 - 3.84 (m, 2 H) 3.88 (dd, *J*=10.07, 4,88 Hz, I H) 4.32 (t, *J*=5.19 Hz, 2 H) 5.02 - 5.07 (m, 1 H) 7.09 (d, *J*=8.85 Hz, 1 H) 7.24 (d, *J*=1.22 Hz, 1 H) 7.42 (dd, *J*=8.85, 2.75 Hz, 1 H) 7.68 (d, *J*=2.75 Hz, 1 H); MS (ESI) m/z 397 $(M+H)^+$.

Example 351

N-[(2Z)-5-tert-butyl-4-methyl-3-[(2R)-tetrahydrofuran-2-ylmethyl]-1,3-thiazol-2(3H)-ylidene]-2-fluoro-3-(trifluoromethyl) benzamide

The title compound was prepared according to the procedure described in Examples 319A and 319B, replacing Example 205B with 2-fluoro-3-(trifluoromethyl)benzoyl chloride in 67.7% yield. $^1$H NMR (300 MHz, $DMSO_4$) δ ppm 1.39 (s, 9 H), 1.58 - 2.05 (m, 4 H), 2.44 (s, 3 H), 3.63 (q, *J*=7.54 Hz, 1 H), 3.72 - 3.84 (m, 1 H), 4.07 - 4.21 (m, 1 H), 4.22 - 4.34 (m, 1 H), 4.42 (dd, *J*= 13.88, 2.78 Hz, 1 H), 7.45 - 7.52 (m, 1 H), 7.84 - 7.94 (m, 1 H), 8.19 - 8.34 (m, I H); MS (ESI) *m*/*z* 445 $[M+H]^+$, 443 [M-H].

Example 352

N-[(2Z)-5-tert-butyl-3-[(2R)-tetrahydrofuran-2-ylmethyl]-1,3-thiazol-2(3H)-ylidene]-2-[2-(dimethylamino)ethoxy]-5-(trifluoromethyl)benzamide

To a solution of 2-(dimethylamino)ethanol (89 mg, 1.0 mmol) in THF (2 mL) was added potassium tert-butoxide 1.0M solution in THF (1 mL). The mixture was stirred at room temperature for 10 min and then Example 372B (215 mg, 0.5 mmol) was added and stirred at room temperature for another 2 hrs. The reaction mixture was quenched with saturated aqueous ammonium chloride and extracted by ethyl acetate (3 x 10 mL). The combined organic layers were washed with brine, dried over $MgSO_4$, filtered, and concentrated. Purification by column chromatography eluting with $NH_3$/MeOH (1:9) in 5-30% gradient in ethyl acetate afforded the title compound (190 mg, 76%). $^1$H NMR (300 MHz, DMSO-$d_6$) δ ppm 1.32 (s, 9 H), 1.57 - 2.01 (m, 4 H), 2.36 - 2.46 (m, 6 H), 2.90 (s, 2 H), 3.59 - 3.84 (m, 2 H), 4.17 - 4.35 (m, 5 H), 7.26 - 7.37 (m, 2 H), 7.77 (dd, *J*=8.72, 1.98 Hz, I H), 8.00 (d, *J*=2.38 Hz, 1 H); MS (ESI) *m*/*z* 500 $[M+H]^+$.

Example 353

N-[(2Z)-5-tert-butyl-3-[(2R)-tetrahydrofuran-2-ylmethyl]-1,3-thiazol-2(3H)-ylidene]-2-[2-(dimethylamino)-2-methylpropoxy]-5-(trifluoromethyl)benzamide

The title compound was prepared according to the procedure described in Example 352, replacing 2-(dimethylamino)ethanol with 2-(dimethylamino)-2-methylpropan-1-ol in 80% yield. $^1$H NMR (300 MHz, DMSO-$_{d6}$) δ ppm 1.10 (s, 6 H), 1.29 - 1.40 (m, 9 H), 1.55 - 1.69 (m, 1 H), 1.73 - 1.97 (m, 3 H), 2.06 - 2.34 (m, 6 H), 3.59 - 3.68 (m, I H), 3.73 - 3.83 (m, I H), 3.94 (s, 2 H), 4.15 - 4.31 (m, 3 H), 7.21 - 7.37 (m, 2 H), 7.73 (d, *J*=9.12 Hz, 1 H), 7.95 (s, 1 H); MS (ESI) *m*/*z* 528 $[M+H]^+$.

Example 354

N-[(2Z)-5-tert-butyl-3-[(2R)-tetrahydrofuran-2-ylmethyl]-1,3-thiazol-2(3H)-ylidene]-2-(2-morpholin-4-ylethoxy)-5-(trifluoromethyl)benzamide

The title compound was prepared according to the procedure described in Example 352, replacing 2-(dimethylamino)ethanol with 2-morpholinoethanol in 85% yield. $^1H$ NMR (300 MHz, DMSO-$d_6$) δ ppm 1.32 (s, 9 H), 1.57 - 1.69 (m, I H), 1.73 - 1.94 (m, 3 H), 2.36 - 2.48 (m, 4 H), 2.69 (t, *J*=5.93 Hz, 2 H), 3.45 - 3.57 (m, 4 H), 3.58 - 3.71 (m, 1 H), 3.72 - 3.85 (m, I H), 4.12 - 4.37 (m, 5 H), 7.22 - 7.34 (m, 2 H), 7.73 (dd, *J*=8.82, 2.03 Hz, 1 H), 7.92 (d, *J*=2.37 Hz, 1 H); MS (ESI) *m/z* 542 $[M+H]^+$.

Example 355

N-[(2Z)-5-tert-butyl-3-[(2R)-tetrahydrofuran-2-ylmethyl]-1,3-thiazol-2(3H)-ylidene]-2-[2-(diethylamino)ethoxy]-5-(trifluoromethyl)benzamide

The title compound was prepared according to the procedure described in Example 352, replacing 2-(dimethylamino)ethanol with 2-(diethylamino)ethanol in 82% yield. $^1H$ NMR (300 MHz, DMSO-$d_6$) δ ppm 0.73 - 1.19 (m, 6 H), 1.32 (s, 9 H), 1.55 - 2.02 (m, 4 H), 2.49 - 2.67 (m, 4 H), 2.69 - 2.98 (m, 2 H), 3.58 - 3.84 (m, 2 H), 3.92 - 4.55 (m, 5 H), 7.14 - 7.41 (m, 2 H), 7.75 (s, I H), 7.95 (s, I H); MS (ESI) *m/z* 528 $[M+H]^+$.

Example 356

N-[(2Z)-5-tert-butyl-3-[(2R)-tetrahydrofuran-2-ylmethyl]-1,3-thiazol-2(3H)-ylidene]-2-[2-(1,1-dioxidothiomorpholin-4-yl)ethoxy]-5-(trifluoromethyl)benzamide

The title compound was prepared according to the procedure described in Example 352, replacing 2-(dimethylamino)ethanol with 4-(2-hydroxyethyl)thiomorpholine-1,1-dioxide in 80% yield. $^1H$ NMR (300 MHz, DMSO-$d_6$) δ ppm 1.32 (s, 9 H), 1.55 - 2.01 (m, 4 H), 2.93 (t, *J*=5.55 Hz, 2 H), 2.96 - 3.10 (m, 8 H), 3.60 - 3.68 (m, I H), 3.72 - 3.82 (m, 1 H), 4.11 - 4.36 (m, 5 H), 7.24 - 7.33 (m, 2 H), 7.75 (dd, *J*=8.92, 2.58 Hz, 1 H), 7.95 (d, *J*=2.38 Hz, 1 H); MS (ESI) *m/z* 590 $[M+H]^+$.

Example 357

N-[(2Z)-5-tert-butyl-3-[(2R)-tetrahydrofuran-2-ylmethyl]-1,3-thiazol-2(3H)-ylidene]-2-(2-piperidin-1-ylethoxyl)-5-(trifluoromethyl)benzamide

The title compound was prepared according to the procedure described in Example 352, replacing 2-(dimethylamino)ethanol with 2-(piperidin-1-yl)ethanol in 69% yield. $^1H$ NMR (300 MHz, DMSO-$d_6$) δ ppm 1.32 (s, 9 H), 1.33 - 1.39 (m, 2 H), 1.39 - 1.49 (m, 4 H), 1.57 - 1.70 (m, I H), 1.74 - 1.86 (m, 2 H), 1.86 - 1.98 (m, 1 H), 2.36 - 2.46 (m, 4 H,) 2.65 (t, *J*=6.35 Hz, 2 H), 3.61 - 3.70 (m, 1 H), 3.74 - 3.83 (m, 1 H), 4.15 - 4.23 (m, 4 H), 4.24 - 4.33 (m, 1 H), 7.25 (s, 1 H), 7.30 (d, *J*=9.12 Hz, I H), 7.73 (dd, *J*=8.73, 2.38 Hz, 1 H), 7.92 (d, *J*=2.38 Hz, 1 H); MS (+DCI) *m/z* 540 $[M+H]^+$.

Example 358

N-[(2Z)-5-tert-butyl-3-[(2R)-tetrahydrofuran-2-ylmethyl]-1,3-thiazol-2(3H)-ylidene]-2-(3-methoxy-3-methylbutoxy)-5-(trifluoromethyl)benzamide

The title compound was prepared according to the procedure described in Example 352, replacing 2-(dimethylamino)ethanol with 3-methoxy-3-methylbutan-1-ol in 55% yield. $^1H$ NMR (300 MHz, DMSO-$d_6$) δ ppm 1.15 (s, 6 H), 1.32 (s, 9 H), 1.58 - 1.70 (m, I H), 1.75 - 1.98 (m, 5 H), 3.10 (s, 3 H), 3.60 - 3.70 (m, 1 H), 3.73 - 3.83 (m, 1 H), 4.10 - 4.35 (m, 5 H), 7.23 - 7.32 (m, 2 H), 7.73 (dd, *J*=8.72, 2.38 Hz, 1 H), 7.94 (d, *J*=2.38 Hz, 1 H); MS (+DCI) *m/z* 529 $[M+H]^+$.

Example 359

N-[(2Z)-5-tert-butyl-3-[(2R)-tetrahydrofuran-2-ylmethyl]-1,3-thiazol-2(3H)-ylidene]-2-[2-(2-oxopyrrolidin-1-yl)ethoxy]-5-(trifluoromethyl)benzamide

The title compound was prepared according to the procedure described in Example 352, replacing 2-(dimethylamino)ethanol with 1-(2-hydroxyethyl)pyrrolidin-2-one in 35% yield. $^1H$ NMR (300 MHz, DMSO-$d_6$.) δ ppm) δ ppm 1.32

(s, 9 H) 1.54 - 1.97 (m, 6 H) 2.11 - 2.22 (m, 2 H) 3.43 (t, *J*=6.95 Hz, 2 H) 3.52 (t *J*=5.43 Hz, 2 H) 3.59 - 3.70 (m, 1 H) 3.72 - 3.83 (m, 1 H) 4.13 - 4.34 (m, 5 H) 7.23 - 7.33 (m, 2 H) 7.74 (dd, *J*=8.82, 2.03 Hz, 1 H) 7.93 (d, *J*=2.03 Hz, 1 H); MS (+DCI) *m/z* 540 $[M+H]^+$.

Example 360

1-benzyl-3-tert-butyl-N-[(2Z)-5-tert-butyl-3-[(2R)-tetrahydrofuran-2-ylmethyl]-1,3-thiazol-2(3H)-ylidenel]-1H-pyrazole-5-carboxamide

To a solution of Example 372A (737mg, 2.0 mmol) and triethylamine (1.4 mL, 10.0 mmol) in dichloromethane (10 mL) was added 1-benzyl-3-(tert-butyl)-1H-pyrazole-5-carbonyl chloride (720 mg, 1.3 mmol) dropwise. The mixture was stirred at room temperature for 2 hr, and then water (10 mL) was added. The reaction mixture was extracted with dichloromethane (10 mL x 2). The combined organic layer was washed with brine, dried over magnesium sulfate and concentrated. The residue was purified by column chromatography over silica gel eluting with ethyl acetate in hexane in 5-30% gradient to provide the title compound (680 mg, 70.7% yield). $^1H$ NMR (300 MHz, DMSO-$d_6$) $\delta$ ppm 1.26 (s, 9 H), 1.29 (s, 9 H), 1.46 - 1.60 (m, 1 H), 1.70 - 1.92 (m, 3 H), 3.57 - 3.66 (m, 1 H), 3.70-3.80 (m, 1 H), 4.12 - 4.24 (m, 3 H), 5.79 - 5.92 (m, 2 H), 6.77 (s, 1 H), 7.06 (d, *J*=7.14 Hz, 2 H), 7.18 - 7.33 (m, 4 H); MS (+ESI) *m/z* 526 $[M+H]^+$.

Example 361

methyl 2-({(2Z)-5-tert-butyl-3-[(2R)-tetrahydrofuran-2-ylmethyl]-1,3-thiazol-2(3H)-ylidene]amino}carbonyl)-4-(trifluoromethyl)benzoate

To Example 386B (2.6 g, 5.29 mmol) and MeOH (50 mL) in a 250 mL stainless steel pressure bottle was added Pd-dppf (Heraeus) (0.194 g, 0.265 mmol) and triethylamine (1.475 mL, 10.58 mnol). The mixture was pressurized with carbon monoxide (60 psi) and stirred at 95 °C for 16 hrs. The reaction mixture was concentrated to dryness and purified by column chromatography over silica gel eluting with ethyl acetate in hexane in 5-30% gradient to provide the title compound (2.3 g, 92% yield). $^1H$ NMR (300 MHz, DMSO-$d_6$) $\delta$ ppm 1.33 (s, 9 H), 1.52 - 2.02 (m, 4 H), 3.60 - 3.69 (m, I H), 3.73 - 3.83 (m, 4 H), 4.14 - 4.34 (m, 3 H), 7.32 (s, I H), 7.89 (s, I H), 7.96 (d, *J*=8.33 Hz, I H), 8.16 (d, *J*=7.93 Hz, 1 H); MS (+ESI) *m/z* 526 $[M+H]^+$.

Example 362

3-tert-butyl-N-[(2Z)-5-tert-butyl-3-[(2R)-tetrahydrofuran-2-ylmethyl]-1,3-thiazol-2(3H)-ylidene]-1-methyl-1H-pyrazole-5-carboxamide

The title compound was prepared according to the procedure described in Example 360, replacing 1-benzyl-3-(tert-butyl)-1H-pyrazole-5-carbonyl chloride with 3-tert-butyl-1-methyl-1H-pyrazole-5-carbonyl chloride in 79% yield. $^1H$ NMR (300 MHz, DMSO-$d_6$) $\delta$ ppm 1.25 (s, 9 H) 1.31 (s, 9 H) 1.56 - 2.04 (m, 4 H) 3.59 - 3.70 (m, 1 H) 3.73 - 3.84 (m, 1 H) 4.10 (s, 3 H) 4.19 - 4.33 (m, 3 H) 6.67 (s, I H) 7.26 (s, 1 H); MS (+ESI) *m/z* 417 $[M+H]^+$.

Example 363

N-[(2Z)-5-tert-butyl-3-[(2R)-tetrahydrofuran-2-ylmethyl]-1,3-thiazol-2(3H)-ylidene]-1-methyl-3-(trifluoromethyl)-1H-pyrazole-5-carboxamide

The mixture of 1-methyl-3-(trifluoromethyl)-1 H-pyrazole-5-carboxylic acid (234 mg, 1.2 mmol) and O-benzotriazol-1-yl-N,N,N',N'-tetramethyluronium tetrafluoroborate (TBTU) (383 mg, 1.5 mmol) in DMF (4 mL) was stirred at room temperature for 10 min, and then Example 372A (241 mg, 1.0 mmol) was added, followed by triethylamine (0.14 mL, 0.1 mmol) dropwise. The mixture was stirred for another 2 hrs and monitored by LC/MS. Water (10 mL) and ethyl acetate (20 mL) were added and the organic layer was washed with saturated $NaHCO_3$ and brine and concentrated. Purification by column chromatography over silica gel eluting with ethyl acetate in hexane in 5-40% gradient yielded the title compound (340 mg, 82 % yield). $^1H$ NMR (300 MHz, DMSO-$d_6$) $\delta$ ppm 1.32 (s, 9 H), 1.55 - 2.05 (m, 4 H), 3.58 - 3.71 (m, 1 H), 3.72 - 3.83 (m, I H), 4.26 (s, 3 H), 4.27 - 4.33 (m, 3 H), 7.23 (s, I H), 7.33 (s, 1 H); MS (+ESI) *m/z* 417 $[M+H]^+$.

Example 364

2-[2-(tert-butylamino)ethoxyl-N-[2Z)-5-tert-butyl-3-[(2R)-tetrahydrofuran-2-ylmethyl]-1,3-thiazol-2(3H)-ylidene]-5-(trifluoromethyl)benzamide

The title compound was prepared according to the procedure described in Example 352, replacing 2-(dimethylamino)ethanol with 2-(tert-butylamino)ethanol in 89% yield. $^1$H NMR (300 MHz, DMSO-$d_6$) δ ppm 0.96 - 1.18 (m, 9 H), 1.32 (s, 9 H), 1.56 - 2.02 (m, 4 H), 2.89 (s, 2 H), 3.59 - 3.70 (m, 1 H), 3.72 - 3.84 (m, 1 H), 4.08 - 4.35 (m, 5 H), 7.25 - 7.39 (m, 2 H), 7.77 (dd, *J*=8.81, 2.03 Hz, I H), 8.01 (s, I H); MS (+ESI) *m/z* 528 $[M+H]^+$.

Example 365

tert-butyl 3-{[3-tert-butyl-5-({[(2Z)-5-tert-butyl-3-[(2R)-tetrahydrofuran-2-ylmethyl]-1,3-thiazol-2(3H)-ylidene]amino}carbonyl)-1H-pyrazol-1-yl]-methyl}azetidine-1-carboxylate

Example 365A

3-*tert*-butyl-*N*-[(2Z)-5-*tert*-butyl-3-[(2*R*)-tetrahydrofuran-2-ylmethyl]-1,3-thiazol-2(3*H*)-ylidene]-1*H*-pyrazole-5-carboxamide

To Example 360 (600 mg, 1.248 mmol) and ethanol (5 mL) in a 20 mL pressure bottle was added 20% Pd $(OH)_2$/C, wet (300 mg, 2.135 mmol) and hydrochloric acid (0.208 mL, 2.497 mmol). The mixture was stirred at 50 °C under hydrogen (60 psi) for 2.5 days. HPLC analysis showed conversion completed. The mixture was, filtered, and concentrated to dryness to afford the title compound (450 mg, 92 % yield). LC/MS (TFA-method) 391 $[M+H]^+$.

Example 365B

tert-butyl 3-{[3-tert-butyl-5-({[(2Z)-5-tert-butyl-3-[(2R)-tetrahydrofuran-2-ylmethyl]-1,3-thiazol-2(3H)-ylidene]amino}carbonyl)-1H-pyrazol-1-yl]methyl}azetidine-1-carboxylate

Example 365A (155 mg, 0.397 mmol), potassium tert-butoxide (89 mg, 0.794 mmol), and tert-butyl 3-(iodomethyl)azetidine-1-carboxylate (142 mg, 0.476 mmol) in dimethylacetamide (4 mL) were reacted at ambient temperature for 2 hrs. The reaction was quenched with saturated $NaHCO_3$ and diluted with ethyl acetate. The water layer was extracted with ethyl acetate (10 10 mL x 2). The combined organics were washed with brine and dried over $MgSO_4$, filtered, and concentrated. Purification by column chromatography (silica gel, ethyl acetate in hexane in 5-40% gradient) provided the title compound (145 mg, 65.3 % yield $^1$H NMR (300 MHz, DMSO-$d_6$) δ ppm 1.31 (s, 9 H), 1.35 (s, 9 H), 1.43 (s, 9 H), 1.61 - 2.14 (m, 4 H), 3.06 - 3.23 (m, I H), 3.70 - 3.98 (m, 6 H), 4.14 - 4.45 (m, 3 H), 4.88 (t, *J*=6.95 Hz, 2 H), 6.74 (s, 1 H), 6.86 (s, I H); MS (+ESI) *m/z* 560 $[M+H]^+$.

Example 366

1-(azetidin-3-ylmethyl)-3-tert-butyl-N-[(2Z)-5-tert-butyl-3-[(2R)-tetrahydrofuran-2-ylmethyl]-1,3-thiazol-2(3H)-ylidene]-1H-pyrazole-5-carboxamide

A mixture of Example 365B (140 mg, 0.25 mmol) and 2,2,2-trifluoroacetic acid (0.193 mL, 2.5 mmol) in $CH_2Cl_2$ (5 mL) was stirred at room temperature overnight. The mixture was concentrated under reduced pressure and the residue was purified by HPLC on a Waters Symmetry C8 column (25 mm x 100 mm, 7 μm particle size) using a gradient of 10% to 100% acetonitrile:0.1% aqueous trifluoroacetic acid over 8 minutes (10 minutes run time) at a flow rate of 40 mL/minutes) to yield the title compound (78mg, 67.8% yield) as a TFA salt. $^1$H NMR (300 MHz, DMSO-$d_6$) δ ppm 1.24 - 1.29 (m, 9 H), 1.32 (s, 9 H), 1.62 - 1.70 (m, I H), 1.79 - 1.88 (m, 2 H), 1.92 - 2.02 (m, I H), 3.20 - 3.34 (m, 1 H), 3.66 (q, *J*=7.08 Hz, 1 H), 3.76 - 3.83 (m, 1 H), 3.84 - 4.01 (m, 4 H), 4.18 - 4.43 (m, 3 H), 4.81 (d, *J*=6.92 Hz, 2 H), 6.74 (s, 1 H), 7.29 - 7.31 (m, 1 H), 8.63 (s, I H); MS (+ESI) *m/z* 460 $[M+H]^+$.

Example 367

N-[(2Z)-5-tert-butyl-3-[(5-methylisoxazol-3-yl)methyl]-1,3-thiazol-2(3H)-ylidene]-5-chloro-2-methoxybenzamide

A solution of Example 244A (275 mg, 0.84 mmol) in DMF at 0 °C was treated with 60% NaH in mineral oil (40 mg, 1.03 mmol). After the gas evolution subsided, the reaction mixture was allowed to stir for 30 min and then treated with commercially available 3-(chloromethyl)-5-methylisoxazole (111 mg, 0.847 mmol). The resulting mixture was stirred

at room temperature for 18 hr, poured into brine and extracted with EtOAc. The organics were combined and washed with water and brine, dried over $MgSO_4$, filtered, and concentrated. The residue was purified by flash chromatography over silica gel using 20% EtOAc in hexane to afford the title compound (165 mg, 37% yield). $^1H$ NMR (500 MHz, DMSO-$d_6$) δ ppm 1.32 (s, 9 H), 2.38 (s, 3 H), 3.77 (s, 3 H), 5.39 (s, 2 H), 6.23 (s, I H), 7.10 (d, *J*=8.8 Hz, I H), 7.39 (s, 1 H), 7.45 (dd, *J*=9.0, 2.9 Hz, I H), 7.65 (d, *J*=3.1 Hz, 1 H). MS (DCI) m/z 420 $(M+H)^+$. Elemental analysis calculated for $C_{20}H_{22}ClN_3O_3S$ : C, 57.20; H, 5.28; N, 10.01. Found: C, 57.16; H, 5.42; N, 9.75.

Example 368

N-[(2Z)-5-tert-butyl-3-[(2R)-tetrahydrofuran-2-ylmethyl]-1,3-thiazol-2(3H)-ylidene]-2-({(3S)-1-[2-(hydroxymino)propyl] pyrrolin-3-yl}oxy)-5-(trifluoromethyl)benzamide

A solution of Example 347B (340 mg, 0.614 mmol) in pyridine (5 mL) was treated with commercially available hydroxylamine hydrochloride (51 mg, 0.73 mmol) and stirred at room temperature for 14 h. The reaction mixture was concentrated under reduced pressure and the residue was partitioned between saturated sodium bicarbonate and ethyl acetate. The organic layer was washed with brine, dried with $MgSO_4$, filtered, and concentrated under reduced pressure. The residue was purified by flash chromatography over silica gel using EtOAc: MeOH (9:1) as eluent to afford the title compound (320 mg, 91% yield). $^1H$ NMR (300 MHz, DMSO-$d_6$) δ ppm 1.32 (s, 9 H), 1.54 - 2.04 (m, 9 H), 2.30 (dd, *J*=13.4,6.6 Hz, I H), 2.41 - 2.75 (m, 3 H), 2.91 (dd, *J*=10.2, 6.4 Hz, I H), 3.06 (s, 1 H), 3.52 - 3.90 (m, 2 H), 4.11 - 4.45 (m, 3 H), 5.00 (s, I H), 7.10 - 7.32 (m, 2 H), 7.71 (dd, *J*=8.6, 1.9 Hz, I H), 7.95 (d, *J*=2.0 Hz, I H), 10.45 (s, I H). MS (DCI) m/z 569 $(M+H)^+$. Anal. calculated for $C_{17}H_{35}F_3N_4O_4S{\bullet}0.4H_2O$: C, 56.31; H, 6.27; N, 9.73; N. Found: C, 56.33; H, 6.39; N, 9.46.

Example 369

N-[(2Z)-5-tert-butyl-3-(2-hydroxyethyl)-1,3-thiazol-2(3H)-ylidene]-5-chloro-2-methoxybenzamide

Example 369A

2-(5-tert-butyl-2-iminothiazol-3-(2H)-yl)ethanol

Commercially available 3,3-dimethylbutanal (Aldrich), 2-aminoethanol (Aldrich), potassium thiocyanate (Aldrich) and iodine (EMD chemicals) were processed using the method described in Example 315A to afford the title compound. MS $(ESI^+)$ m/z 201 $(M+H)^+$.

Example 369B

N-[(2Z)-5-tert-butyl-3-(2-hydroxyethyl)-1,3-thiazol-2(3H)-ylidene]-5-chloro-2-methoxybenzamide

Example 369A and 5-chloro-2-methoxybenzoic acid (Aldrich) were processed using the method described in Example 58 to afford the title compound. $^1H$ NMR (300 MHz), DMSO-$d_6$) δ ppm 1.32 (s, 9 H), 3.71 - 3.83 (m, 2 H), 3.78 (s, 3 H), 4.20 (t, *J*=5.4 Hz, 2 H), 4.95 (t, I H), 7.10 (d, *J*=8.8 Hz, I H), 7.23 (s, I H), 7.44 (dd, *J*=8.8, 2.7 Hz, 1 H), 7.62 (d, *J*=2.7 Hz, 1 H); MS $(ESI^+)$ m/z 369 $(M+H)^+$, Anal. Calculated for $C_{17}H_{21}ClN_2O_3S$: C, 55.35; H, 5.74; N, 7.59. Found: C, 55.02; H, 6.16; N, 7.28.

Example 370

5-chloro-N-[(2Z)-5-(4,4-difluorocyclohexyl)-3-(2-methoxyethyl)-1,3-thiazol-2(3H)-ylidene]-2-methoxybenzamide

Example 370A

(4,4-difluorocyclohexyl)methanol

To a suspension of lithium aluminum hydride (2.6 g, 69 mmol) in diethyl ether (160 mL) was added slowly a solution of commercially available ethyl 4,4-difluorocyclohexanecarboxylate (Matrix, 11.0 g, 57 mmol) in diethyl ether (20 mL). The reaction mixture was refluxed for 4 hours, then cooled in an ice bath, quenched cautiously with sequential addition of water (2.6 mL), 15% NaOH (2.6 mL) and water (7.8 mL) and extracted with ethyl acetate (3x100 mL). The mixture was filtered and concentrated to afford the title compound.

Example 370B

(4,4-difluorocyclohexyl)methyl 4-methylbenzenesulfonate

To a solution of Example 370A (8.5 g, 57 mmol) in dichloromethane (100 mL) were added triethylamine (Aldrich, 25 mL, 180 mmol) and tosyl chloride (Aldrich, 11.4 g, 60 mmol). The reaction mixture was stirred at room temperature for 16 hours and washed with water (50 mL) and brine (50 mL). The organic layer was dried over anhydrous $Na_2SO_4$, filtered and concentrated under reduced pressure. The residue was purified by column chromatography using an Analogix® Intelliflash280 ™ ($SiO_2$, 0-50 % ethyl acetate in hexanes) to afford the title compound. $^1$H NMR (300 MHz, $CDCl_3$) $\delta$ ppm 1.17 - 1.42 (m, 2 H), 1.57 - 1.69 (m, I H), 1.70 - 1.91 (m, 4 H), 1.93 - 2.18 (m, 2 H), 2.46 (s, 3 H), 3.86 (d, *J*=6.4 Hz, 2 H), 7.35 (d, *J*=8.5 Hz, 2 H), 7.78 (d, *J*=8.1 Hz, 2 H).

Example 370C

2-(4,4-difluorocyclohexyl)acetonitrile

To a solution of Example 370B (4.5 g, 15 mmol) in dimethylsulfoxide (100 mL) was added sodium cyanide (Aldrich, 2.2 g, 45 mmol). The reaction mixture was stirred at 80 °C for 14 hours, cooled to room temperature, quenched with saturated aqueous $NaHCO_3$ (50 mL) and extracted with diethyl ether (3 x 50 mL). The combined organic extracts were dried over anhydrous $MgSO_4$, filtered and concentrated under reduced pressure. The residue was purified by column chromatography using an Analogic® Intelliflash280™ ($SiO_2$, 50 % pentane in ether) to afford the title compound. MS ($ESI^+$) m/z 177 $(M+NH_4)^+$.

Example 370D

2-(4,4-difluorocyclohexyl)acetaldehyde

To a solution of Example 370C (3.8 g, 24 mmol) in dichloromethane (50 mL) was added diisobutylaluminum hydride (1.6M in cyclohexane, 22.5 mL), 36 mmol), dropwise. The reaction mixture was stirred at room temperature for 3 hours. The reaction mixture was quenched with 1M tartaric acid (40 mL), stirred for 1 hour and the layers were separated. The aqueous layer was extracted with dichloromethane (3 x 50 mL). The combined organic extracts were dried over anhydrous $MgSO_4$, filtered and concentrated under reduced pressure to afford the title compound. MS ($ESI^+$) m/z 162 $(M+NH_4-H_2O)^+$.

Example 370E

5-(4,4-difluorocyclohexyl)-2,3-dihydrothiazol-2-amine

Example 370D, pyrrolidine, *p*-toluenesulfonic acid monohydrate, sulfur and cyanamide were processed using the method described in Example 202A to obtain the title compound. MS ($ESI^+$) m/z 219 $(M+H)^+$.

Example 370F

5-(4,4-difluorocyclohexyl)-3-(2-methoxyethyl)thiazol-2(3H)-imine hydrobromide

A mixture of Example 370E and commercially available 2-bromoethyl methyl ether (Aldrich) was processed using the method described in Example 12A to afford the title compound. MS ($ESI^+$) m/z 277 $(M+H)^+$.

Example 370G

5-chloro-N-[(2Z)-5-(4,4-difluorocyclohexyl)-3-(2-methoxyethyl)-1,3-thiazol-2(3H)-ylidene]-2-methoxybenzamide

Example 370F and 5-chloro-2-methoxybenzoic acid (Aldrich) were processed using the method described in Example 58 to afford the title compound. $^1$H NMR (300 MHz, DMSO-$d_6$) $\delta$ ppm 1.44 - 1.76 (m, 2 H), 1.80 - 2.2 (m, 6 H), 2.80 - 2.99 (m, I H), 3.26 (s, 3 H), 3.72 (t, *J*=5.4 Hz, 2 H), 3.78 (s, 3 H), 4.32 (t, *J*=5.3 Hz, 2 H), 7.11 (d, *J*=9.2 Hz, 1 H), 7.34 (s, I H), 7.45 (dd, *J*=8.8, 3.1 Hz, 1 H), 7.65 (d, *J*=2.7 Hz, 1 H); MS ($ESI^+$) m/z 445 $(M+H)^+$, Anal. Calculated for $C_{20}H_{13}ClF_2N_2O_3S$: C, 53.99; H, 5.21; N, 6.30. Found: C, 54.03; H, 5.19; N, 6.27.

Example 371

N-[(2Z)-5-tert-butyl-3-[(2R)-tetrahydrofuran-2-ylmethyl]-1,3-thiazol-2(3H)-ylidene]-2-cyano-5-(trifluoromethyl)benzamide

A mixture of Example 343A (180 mg, 0.42 mmol) and sodium cyanide (41.0 mg, 0.84 mmol) in 0.4 mL of DMSO was heated at 120 °C for 2 h. The reaction was cooled to room temperature and diluted with ether. The mixture was washed with brine, and the layers were separated. The aqueous layer was extracted with ether (2 x 10 mL). The combined organic extracts were dried ($MgSO_4$), filtered, and concentrated. The residue was purified by column chromatography using an Analogic® Intelliflash280™ ($SiO_2$, 0-50 % ethyl acetate in hexanes) to afford the title compound. $^1H$ NMR (300 MHz, $CDCl_3$) $\delta$ ppm 1.38 (s, 9 H), 1.58 - 1.72 (m, I H), 1.73 - 1.95 (m, 2 H), 2.09 - 2.23 (m 1 H), 3.73 - 3.89 (m, 2 H), 4.25 - 4.39 (m, 2 H), 4.65 - 4.79 (m, 1 H), 6.98 (s, I H), 7.78 (dd, *J*=8.0, 1.9 Hz, 1 H), 7.90 (d, *J*=7.9 Hz, I H), 8.69 (d, *J*=1.6 Hz, I H); MS ($ESI^+$) m/z 438 $(M+H)^+$.

Example 372

2-tert-butoxy-N-[(2Z)-5-tert-butyl-3-[(2R)-tetrahydrofuran-2-ylmethyl]-1,3-thiazol-2(3H)-ylidene]-5-(trifluoromethyl)benzamide

Example 372A

5-*tert*-butyl-3-[(2*R*)-tetrahydrofuran-2-ylmethyl]-1,3-thiazol-2(3*H*)-imine hydroiodide

To a solution of 3,3-dimethylbutanal (9.90 g, 99 mmol) in acetonitrile (60 mL) were added molecular sieves (8 g) and (R)-(tetrahydrofuran-2-yl)methanamine (10 g, 99 mmol). The reaction mixture was stirred at room temperature for 24 hr and then filtered. To the filtrate was added potassium thiocyanate (12.78 g, 131 mmol). The temperature was adjusted to 50 °C and the mixture was stirred until the solids were dissolved. Then, iodine (25.09 g, 99 mmol) was added to the mixture and stirred at 50 °C for 24 hr. The reaction mixture was cooled, and to the mixture was added sodium metabisulfite (20%, 100 mL) and stirred for 30 min. The organic layer was separated. The aqueous layer was washed with dichloromethane (3 x 40 mL). The combined organic extracts were dried with sodium sulfate, filtered and concentrated to obtain the crude product as a yellow solid. The residue was taken into dichloromethane (20 mL) and ethyl acetate (80 mL) was added, the mixture was warmed to 40 °C, sonicated, and left in the refrigerator overnight. The solid was collected and washed with cold ethyl acetate to obtain the title compound as a white solid (18.2 g, 50%). $^1H$ NMR (300 MHz, DMSO-$d_6$) $\delta$ ppm 1.27 (s, 9H) 1.48 - 1.61 (m, 1 H) 1.78 - 1.93 (m, 2 H) 1.94 - 2.07 (m, 1 H) 3.62 - 3.71 (m, 1 H) 3.76 - 3.84 (m, I H) 3.92 - 4.08 (m, 2 H) 4.11 - 4.20 (m, 1 H) 7.19 (s, 1 H) 9.39 (s, 2 H); MS (DCI/$NH_3$); m/z 241 $(M+H)^+$.

Example 372B

*N*-[(2*Z*)-5-*tert*-butyl-3[(2*R*)-tetrahydrofuran-2-ylmethyl]-1,3-thiazol-2(3*H*)-ylidene]-2-fluoro-5-(trifluoromethyl)benzamide

In a 250 mL round-bottomed flask, Example 372A (6 g, 24.96 mmol) was dissolved in dichloromethane (50 mL), triethylamine (8.70 mL, 62.4 mmol) was added followed by addition of 2-fluoro-5-(trifluoromethyl)benzoyl chloride (3.78 mL, 24.96 mmol) dropwise and stirred for 2 hr. The reaction was washed with water and dried with sodium sulfate. The solution was filtere and concentrated, and the residue was purified by column chromatography using an Analogix® Intelliflash280 the ($SiO_2$, 0-30% ethyl acetate in hexane over 25 min). The product was concentrated to provide the title compound as a viscous liquid (7.14 g, 66.4 % yield). $^1H$ NMR (300 MHz, DMSO-$d_6$) $\delta$ ppm 1.33 (s, 9 H) 1.62 - 1.72 (m, 1 H) 1.78 - 1.88 (m, 2 H) 1.89 - 1.99 (m, 1 H) 3.62 - 3.70 (m, 1 H) 3.75 - 3.83 (m, 1 H) 4.22 - 4.29 (m, 2 H) 4.29 - 4.37 (m, 1 H) 7.34 (s, I H) 7.48 - 7.57 (m, I H) 7.86 - 7.97 (m, 1 H) 8.31 (dd, *J*=6.78, 2.37 Hz, 1 H); MS (DCI/$NH_3$) *mlz* 431 $(M+H)^+$.

Example 372C

2-tert-butoxy-N-[(2Z)-5-tert-butyl-3-[(2R)-tetrahydrofuran-2-ylmethyl]-1,3-thiazol-2(3H)-ylidene]-5-(trifluoromethyl)benzamide

To a solution of potassium tert-butoxide in tetrahydrofuran (1 M, 2 mL) was added Example 372B (163 mg, 0.38 mmol). The mixture was stirred at room temperature overnight. The reaction was quenched with saturated aqueous ammonium chloride and extracted by ethyl acetate (3 x 10 mL). The organic layers were combined, washed with water, dried, filtered, and concentrated, and the residue was purified by column chromatography using an Analogix®

Intelliflash280™ ($SiO_2$, 0-100% ethyl acetate in hexane) to afford the title compound (0.16 g, 80 %). $^1H$ NMR (3us MHz, DMSO-$d_6$) δ ppm 1.32 (s, 9 H) 1.33 (s, 9 H) 1.57 - 1.68 (m, 1 H) 1.77 - 1.92 (m, 3 H) 3.60 - 3.68 (m, 1 H) 3.78 (dt, *J*=8.39, 6.49 Hz, I H) 4.17 - 4.22 (m, 2 H) 4.24 - 4.33 (m, 1 H) 7.26 (s, 1 H) 7.32 (d, *J*=8.48 Hz, I H) 7.67 (dd, *J*=8.99, 2.20 Hz, I H) 7.88 (d, *J*=2.37 Hz, I H); MS (DCI/$NH_3$) *m/z* 485 $(M+H)^+$.

Example 373

N-[(2Z)-5-tert-butyl-3-[(2R)-tetrahydrofuran-2-ylmethyl]-1,3-thiazol-2(3H)-ylidene]-2-{(3R)-1-methylpyrrolidin-3-yl]oxy}-5-(trifluoromethyl)benzamide

To a solution of (R)-1-methylpyrrolidin-3-ol (162 mg, 1.6 mmol) in tetrahydrofuran (2 mL) was added sodium tert-butoxide (161 mg, 1.68 mmol). The mixture was stirred at room temperature for 20 min before a solution of Example 372B (344 ing, 0.8 mmol) in tetrahydrofuran (1 mL) was added dropwise. The mixture was stirred at room temperature for 2 hr before it was quenched with saturated aqueous ammonium chloride and extracted by ethyl acetate (3 x 10mL). The organic layers were combined, washed with brine, dried, filtered, concentrated and the residue was purified by column chromatography using an Analogix® Intelliflash280 ™ ($SiO_2$, gradient elution over 25 min with solvents A:B (100: 0 to 10:90); solvent A = $CH_2Cl_2$; solvent B = 7M $NH_3$/MeOH (1 ):$CH_2Cl_2$ (9)) to afford the title compound (150 mg, 37%). $^1H$ NMR (300 MHz, DMSO-$d_6$) δ ppm 1.32 (s, 9 H) 1.57 - 1.70 (m, I H) 1.76 - 1.83 (m, 3 H) 1.88 - 1.96 (m, I H) 2.25 (s, 3 H) 2.29 - 2.44 (m, 2 H) 2.60 - 2.73 (m, 2 H) 2.78 - 2.84 (m, 1 H) 3.61 - 3.69 (m, 1 H) 3.74 - 3.83 (m, 1 H) 4.18 - 4.24 (m, 2 H) 4.25 - 4.33 (m, 1 H) 4.96 - 5.04 (m, 1 H) 7.18 (d, *J*=8.73 Hz, 1 H) 7.26 (s, 1 H) 7.72 (dd, *J*=8.73, 2.78 Hz, 1 H) 7.96 (d, *J*=2.78 Hz, 1 H); MS (DCI/$NH_3$) *m/z* 512 $(M+H)^+$. Anal. calcd $C_{24}H_{31}F_3N_2O_3S$: C, 58.69; H, 6.3; N, 8.21. Found: C, 58.58; H, 6.29; N, 8.18.

Example 374

N-[(2Z)-5-tert-butyl-3-[(2R)-tetrahydrofuran-2-ylmethyl]-1,3-thiazol-2(3H)-ylidene]-2-{[(3S)-1-methylpyrrolidin-3-yl]oxy}-5-(trifluoromethyl)benzamide

The title compound was prepared and isolated as described in Example 373, substituting (S)-1-methylpyrrolidin-3-ol for (R)-1-methylpyrrolidin-3-ol in 42% yield. $^1H$ NMR (300 MHz, DMSO-$d_6$) δ ppin 1.31 (s, 9 H) 1.58 - 1.69 (m, 1 H) 1.74 - 1.85 (m, 3 H) 1.87 - 1.98 (m, 1 H) 2.25 (s, 3 H) 2.29 - 2.41 (m, 2 H) 2.59 - 2.69 (m, 2 H) 2.78 - 2.84 (m, 1 H) 3.60 - 3.69 (m, 1 H) 3.74 - 3.83 (m, 1 H) 4.18 - 4.32 (m, 3 H) 4.95-5.04 (m, 1 H) 7.18 (d, *J*=8.73 Hz, 1 H) 7.26 (s, 1 H) 7.71 (dd, *J*=9.12, 2.38 Hz, 1 H) 7.97 (d, *J*=2.38 Hz, 1 H); MS (DCI/$NH_3$) *m/z* 512 $(M+H)^+$. Anal. calcd $C_{24}H_{31}F_3N_2O_3S$•0.5 $H_2O$: C, 57.68; H, 6.39; N, 8.18. Found: C, 57.79; H, 6.39; N, 8.04.

Example 375

N-[(2Z)-5-tert-butyl-3-[(2R)-tetrahydrofuran-2-ylmethyl]-1,3-thiazol-2(3H)-ylidene]-2-formyl-5-(trifluoromethyl)benzamide

Example 375A

2-formyl-5-(trifluoromethyl)benzoic acid

To a 250 mL round-bottomed flask, was added 2.5 M n-butyllithium/hexane (8.92 mL, 22.30 mmol) in tetrahydrofuran (40 mL) and cooled to -78 °C, followed by dropwise addition of 2-bromo-5-(trifluoromethyl)benzoic acid (3 g, 11.15 mmol) in tetrahydrofuran, (20 mL). After 30 min N,N-dimethylformamide (0.978 g, 13.38 mmol) was added and the solution slowly allowed to wann to rt. To the reaction was added saturated ammonium chloride solution, followed by addition of saturated $NaHCO_3$ solution until basic. The aqueous layer was separated, and 2 N HCl was added until acidic. The mixture was extracted with ethyl acetate (3 x 20 mL). The organic layers were combined, dried, filtered, and concentrated. The resulting solid was used without further purification. MS (DCI/$NH_3$) *m/z* 512 $(M+NH_4)^+$.

Example 375B

2-formyl-5-(trifluoromethyl)benzoyl chloride

A solution of Example 375A (0.6 g) and thionyl chloride (3.27 g, 27.5 mmol) was refluxed for 2 hr. The reaction solution was cooled to room temperature, concentrated, azeotroped with toluene, and used without further purification.

Example 375C

N-[(2Z)-5-tert-butyl-3-[(2R)-tetrahydrofuran-2-ylmethyl]-1,3-thiazol-2(3H)-ylidene]-2-formyl-5-(trifluoromethyl)benzamide

The title compound was prepared and isolated as described in Example 372B, substituting Example 375B for 2-fluoro-(5-trifluoromethyl)benzoyl chloride in 25% yield. $^1$H NMR (300 MHz, DMSO-$d_6$) δ ppm 1.34 (s, 9 H) 1.58 - 1.70 (m, I H) 1.77 - 1.92 (m, 2 H) 1.93 - 2.04 (m, I H) 3.62 - 3.70 (m, I H) 3.76 - 3.84 (m, 1 H) 4.22 - 4.32 (m, 3 H) 7.39 (s, 1 H) 7.81 (d, *J*=8.14 Hz, 1 H) 8.00 - 8.03 (m, I H) 8.37 - 8.38 (m, 1 H) 10.62 (s, 1 H); MS (APCI) *m/z* 441 $(M+H)^+$.

Example 376

N-[(2Z)-5-tert-butyl-3-[(2R)-tetrahydrofuran-2-ylmethyl]-1,3-thiazol-2(3H)-ylidene]-3-(trifluoromethyl)benzamide

The title compound was obtained as a side product from Example 375C. $^1$H NMR (300 MHz, DMSO-$d_6$) δ ppm 1.33 (s, 9 H) 1.64 - 1.76 (m, 1 H) 1.78 - 1.90 (m, 2 H) 1.91 - 2.04 (m, I H) 3.62 - 3.72 (m, 1 H) 3.76 - 3.85 (m, 1 H) 4.26 - 4.39 (m, 3 H) 7.32 (s, 1 H) 7.71 - 7.77 (m, I H) 7.89 - 7.92 (m, I H) 8.41 - 8.47 (m, 2 H); MS (DCI/$NH_3$) *m/z* 413 $(M+H)^+$.

Example 377

2-(azetidin-1-ylmethyl)-N-[(2Z)-5-tert-butyl-3-[(2R)-tetrahydrofuran-2-ylmethyl]-1,3-thiazol-2(3H)-ylidene]-5-(trifluoromethyl)benzamide

In a 20 mL vial, to a solution of Example 375C (52 mg, 0.118 mmol) in dichloromethane (2 mL) was added azetidine (20.22 mg, 0.354 mmol), followed by addition of acetic acid (7.09 mg, 0.118 mmol) and sodium triacetoxyborohydride (37.5 mg, 0.177 mmol) and the mixture was stirred for 2 hr. The reaction was quenched with saturated $NaHCO_3$ and extracted with dichloromethane (3 x 5 mL). The organics were combined, dried, filtered, concentrated and the residue was purified by column chromatography using an Analogix® Intelliflash280™ ($SiO_2$, gradient elution over 25 min with solvents A:B (100:0 to 10:90); solvent A = $CH_2Cl_2$; solvent B = 7M $NH_3$/MeOH (1):$CH_2Cl_2$, (9)) to afford the title compound in 79% yield $^1$H NMR (300 MHz, DMSO-$d_6$) δ ppm 1.34 (s, 9 H) 1.61 - 1.76 (m, I H) 1.79 - 1.91 (m, 2 H) 1.91 - 2.06 (m, 3 H) 3.18 (t, *J*=6.94 Hz, 4 H) 3.60 - 3.71 (m, I H) 3.74 - 3.85 (m, I H) 4.06 (s, 2 H) 4.23 - 4.36 (m, 3 H) 7.32 (s, I H) 7.74 - 7.78 (m, 2 H) 8.21 - 8.25 (m, 1 H); MS (DCI/$NH_3$) *m/z* 482 $(M+H)^+$. Anal. calcd $C_{25}H_{32}F_3N_3O_2S$•0.3 $C_6H_6$: C, 62.02; H, 6.56; N, 8.10. Found: C, 61.94; H, 6.43; N, 8.38.

Example 378

N-[(2Z)-5-tert-butyl-3-[(2R)-tetrahydrofuran-2-ylmethyl]-1,3-thiazol-2(3H)-ylidene]-2-(pyrrolidin-1-yhmethyl)-5-(trifluoromethyl)benzamide

The title compound was prepared and isolated as described in Example 377, substituting pyrrolidine for azetidine in 72% yield. $^1$H NMR (300 MHz, DMSO-$d_6$) δ ppm 1.33 (s, 9 H) 1.62 - 1.72 (m, 5 H) 1.79 - 1.88 (m, 2 H) 1.90 - 1.98 (m, 1 H) 2.43 - 2.50 (m, 4 H) 3.62 - 3.70 (m, 1 H) 3.76 - 3.83 (m, 1 H) 4.10 (s, 2 H) 4.22 - 4.33 (m, 3 H) 7.30 (s, 1 H) 7.76 - 7.86 (m, 2 H) 8.16 (d, *J*=1.70 Hz, 1 H); MS (DCI/$NH_3$) *m/z* 496 $(M+H)^+$.

Example 379

N-[(2Z)-5-tert-butyl-3-[(2R)-tetrahydrofuran-2-ylmethyl]1-1,3-thiazol-2(3H)-ylidene]-2-{[(3R)-3-hydroxypyrrolidin-1-yl]methyl}-5-(trifluoromethyl)benzamide

The title compound was prepared as described in Example 377, substituting (R)-pyrrolidin-3-ol for azetidine, and isolated by preparative HPLC on a Phenomenex Luna Combi-HTS C8(2) column (5μm, 100Å, 2.1 mm × 30mm), using a gradient of 10-100% acetonitrile (A) and 10 mM ammonium acetate in water (B), at a flow rate of 2.0 mL/min (0-0.1 min 10% A, 0.1-2.6 min 10-100% A, 2.6-2.9 min 100% A, 2.9-3.0 min 100-10% A. 0.5min post-run delay) in 70% yield $^1$H NMR (300 MHz, DMSO-$d_6$) δ ppm 1.33 (s, 9 H) 1.53 - 1.69 (m, 2 H) 1.78 - 1.89 (m, 2 H) 1.92 - 2.04 (m, 2 H) 2.35 (dd, *J*=9.52, 3.57 Hz, I H) 2.42 - 2.48 (m, 1 H) 2.58 - 2.74 (m, 2 H) 3.61 - 3.71 (m, 1 H) 3.75 - 3.84 (m, I H) 4.09 - 4.11 (d, *J*=5.95 Hz, 2 H) 4.16 - 4.27 (m, 3 H) 4.29 - 4.33 (m, 1 H) 4.67 (d, *J*=4.36 Hz, 1 H) 7.30 (s, 1 H) 7.76 - 7.81 (m, 1 H) 7.83 - 7.88 (m, 1 H) 8.17 - 8.18 (m, I H); MS (DCI/$NH_3$) *m/z* 512 $(M+H)^+$.

Example 380

N-[(2Z)-5-tert-butyl-3-[(2R)-tetrahydrofuran-2-ylmethyl]-1,3-thiazol-2(3H)-ylidene]-2-{[(3S)-3-hydroxypyrrolidin-1-yl]methyl}-5-(trifluoromethyl)benzamide

The title compound was prepared as described in Example 377, substituting (S)-pyrrolidin-3-ol for azetidine, and isolated by preparative HPLC on a Phenomenex Luna Combi-HTS C8(2) column (5μm, 100Å, 2.1mm × 30mm), using a gradient of 10-100% acetonitrile (A) and 10 mM ammonium acetate in water (B), at a flow rate of 2.0 mL/min (0-0.1 min 10% A, 0.1-2.6 min 10-100% A, 2.6-2.9 min 100% A, 2.9-3.0 min 100-10% A. 0.5min post-run delay) in 60% yield. $^1$H NMR (300 MHz, DMSO-$d_6$) δ ppm 1.33 (s, 9 H) 1.53 - 1.60 (m, I H) 1.62 - 1.69 (m, I H) 1.77 - 1.89 (m, 2 H) 1.92 - 2.04 (m, 2 H) 2.32 - 2.47 (m, 2 H) 2.59 - 2.74 (m, 2 H) 3.62 - 3.70 (m, I H) 3.75 - 3.84 (m, I H) 4.02 - 4.13 (m, 2 H) 4.17 - 4.33 (m, 4 H) 4.67 (d, *J*=4.07 Hz, I H) 7.30(s, 1 H) 7.76 - 7.81 (m, 1 H) 7.83 - 7.88 (m, I H) 8.17 (s, I H); MS (DCI/$NH_3$) *m*/*z* 512 $(M+H)^+$.

Example 381

N-[(2Z)-5-tert-butyl-3-[(2R)-tetrahydrofuran-2-ylmethyl]-1,3-thiazol-2(3H)-ylidene]-2-methyl-5-(trifluoromethyl)benzamide

Example 381A

2-methyl-5-(trifluoromethyl)benzoyl chloride

The title compound was prepared and isolated using the method described in Example 375B, substituting 2-methyl-5-trifluoromethyl benzoic acid for Example 375A.

Example 381B

N-[(2Z)-5-tert-butyl-3-[(2R)-tetrahydrofuran-2-ylmethyl]-1,3-thiazol-2(3H)-ylidene]-2-methyl-5-(trifluoromethyl)benzamide

The title compound was prepared and isolated as described in Example 372B, substituting Example 381A for 2-fluoro-(5-trifluoro)benzoyl chloride in 63% yield. $^1$H NMR (300 MHz, DMSO-$d_6$) δ ppm 1.33 (s, 9 H) 1.60 - 1.72 (m, I H) 1.78 - 1.86 (m, 2 H) 1.87 - 1.99 (m, 1 H) 2.67 (s, 3 H) 3.62 - 3.70 (m, I H) 3.75 - 3.83 (m, I H) 4.23 - 4.34 (m, 3 H) 7.31 (s, 1 H) 7.50 (d, *J*=8.14 Hz, I H) 7.70 (dd, *J*=7.97, 1.86 Hz, 1 H) 8.28 (d, *J*=2.03 Hz, 1 H); MS (DCI/$NH_3$) *m*/*z* 427 $(M+H)^+$.

Example 382

2-(azetidin-3-yloxy)-N-[(2Z)-5-tert-butyl-3-[(2R)-tetrahydrofuran-2-ylmethyl]-1,3-thiazol-2(3H)-ylidene]-5-(trifluoromethyl)benzamide

Example 382A

*tert*-butyl 3-[2-{[(2*Z*)-5-*tert*-butyl-3-[(2*R*)-tetrahydrofuran-2-ylmethyl]-1,3-thiazol-2(3*H*)-ylidene]carbamoyl}-4-(trifluoromethyl)phenoxy]azetidine-1-carboxylate

To a solution of tert-butyl 3-hydroxyazetidine-1-carboxylate (4 g, 23.09 mmol) in tetrahydrofuran (30 mL) was added sodium tert-butoxide (2.330 g, 24.25 mmol). The mixture was stirred at room temperature for 20 minutes before Example 372B (4.97 g, 11.55 mmol) was added. The mixture was stirred at room temperature for 2 hr, quenched with saturated aqueous ammonium chloride and extracted with ethyl acetate (3 x 30 mL). The organic layers were combined, washed with brine, dried, filtered, concentrated and the residue was purified by column chromatography using an Analogic® Intelliflash280 ™ ($SiO_2$, 0-100% ethyl acetate in hexane over 25 min) to afford the title compound (6.32 g, 10.83 mmol, 94 % yield). $^1$H NMR (300 MHz, DMSO-$d_6$) δ ppm 1.33 (s, 9 H) 1.39 (s, 9 H) 1.58 - 1.69 (m, 1 H) 1.76 - 1.87 (m, 2 H) 1.88 - 1.99 (m, 1 H) 3.60 - 3.70 (m, 1 H) 3.75 - 3.86 (m, 3 H) 4.20 - 4.22 (m, 2 H) 4.29 - 4.35 (m, 3 H) 5.09 - 5.17 (m, 1 H) 7.00 (d, *J*=8.48 Hz, 1 H) 7.73 (dd, *J*=8.82, 2.37 Hz, 1 H) 8.04 (d, *J*=2.37 Hz, 1 H); MS (DCI/$NH_3$) *m*/*z* 584 $(M+H)^+$.

Example 382B

2-(azetidin-3-yloxy)-N-[(2Z)-5-tert-butyl-3-[(2R)-tetrahydrofuran-2-ylmethyl]-1,3-thiazol-2(3H)-ylidene]-5-(trifluoromethyl)benzamide

To a solution of Example 382A (500 mg, 0.857 mmol) in ethyl acetate (1 mL) was added sulfuric acid (84 mg, 0.857 mmol) in ethyl acetate (0.5 mL). The mixture was stirred at room temperature for 6 hr. More sulfuric acid (84 mg, 0.857 mmol) was added and the reaction was heated at 75 °C overnight. The reaction mixture was cooled to room temperature and filtered. The solid was washed with ethyl acetate. The title compound was obtained as a disulfate salt (175 mg, 30%). $^1$H NMR (300 MHz, $CD_3OD$) δ ppm 1.44 (s, 9 H) 1.64 - 1.76 (m, 1 H) 1.83 - 2.00 (m, 2 H) 2.08 - 2.19 (m, 1 H) 3.74 - 3.90 (m, 2 H) 4.30 - 4.38 (m, 3 H) 4.39 - 4.48 (m, 1 H) 4.54 - 4.66 (m, 3 H) 5.34 - 5.41 (m, 1 H) 7.06 (d, *J*=8.72 Hz, 1 H) 7.47 (s, 1 H) 7.84 (dd, *J*=9.12, 2.38 Hz, 1 H) 8.26 (d, *J*=2.38 Hz, 1 H); (DCI/$NH_3$) *m/z* 484 $(M+H)^+$. Anal. calcd $C_{13}H_{28}F_3N_3O_3S$•2 $H_2SO_4$: C, 40.64; H, 4.75; N, 6.18; Found: C, 40.66; H, 4.57; N, 6.04.

Example 383

N-[(2Z)-5-tert-butyl-3-[(2R)-tetrahydrofuran-2-ylmethyl]-1,3-thiazol-2(3H)-ylidene]-2-[(1-methylazetidin-3-yl)oxy]-5-(trifluoromethyl)benzamide

Example 383A

2-(azetidin-3-yloxy)-N-[(2Z)-5-tert-butyl-3-[(2R)-tetrahydrofuran-2-ylmethyl]-1,3-thiazol-2(3H)-ylidene]-5-(trifluoromethyl)benzamide

To a solution of Example 382A (0.619 g, 1.06 mmol) in dichloromethane (2 mL) was added 2,2,2-trifluoroacetic acid (1 mL, 10.60 mmol). The mixture was stirred at room temperature for 2 hr and then concentrated as the TFA salt.

Example 383B

N-[(2Z)-5-tert-butyl-3-[(2R)-tetrahydrofuran-2-ylmethyl]-1,3-thiazol-2(3H)-ylidene]-2-[(1-methylazetidin-3-yl)oxy]-5-(trifluoromethyl)benzamide

To Example 383A (317 mg, 0.53 mmol) in dichloromethane (5 mL) was added a solution of formaldehyde in water (318 μL, 30%, 95 mg), sodium acetate (130 mg, 1.590 mmol) and sodium triacetoxyborohydride (674 mg, 3.18 mmol). The mixture was stirred at room temperature overnight and quenched with saturated aqueous $NaHCO_3$ and extracted with dichloromethane (3x10 mL). The combined organic layers were dried, filtered, concentrated under reduced pressure, and the residue was purified by column chromatography using an Analogic® Intelliflash280 ™ ($SiO_2$, gradient elution over 25 min with solvents A:B (100:0 to 10:90); solvent A = $CH_2Cl_2$; solvent B = 7M $NH_3$/MeOH (1):$CH_2Cl_2$ (9)) to provide the title compound (105 mg, 0.211 mmol, 39.8 % yield). $^1$H NMR (300 MHz, DMSO-$d_6$) d ppm 1.33 (s, 9 H) 1.67- 1.70 (m, 1 H) 1.76 - 1.85 (m, 2 H) 1.88 - 1.93 (m, 1 H) 2.29 (s, 3 H) 2.96 - 3.02 (m, 2 H) 3.63 - 3.67 (m, 1 H) 3.73 - 3.83 (m, 3 H) 4.21 - 4.23 (m, 2 H) 4.31 - 4.33 (m, 1 H) 4.87 - 4.89 (m, 1 H) 6.99 - 7.02 (m , 1H), 7.27 (s, 1 H) 7.70 - 7.73 (m, I H) 8.00 - 8.02 (m, I H); MS (DCI/$NH_3$) *m/z* 498 $(M+H)^+$.

Example 384

N-[(2Z)-5-tert-butyl-3-[(2R)-tetrahydrofuran-2-ylmethyl]-1,3-thiazol-2(3H)-ylidene]-2-[(3R)-pyrrolidin-3-yloxy]-5-(trifluoromethyl)benzamide

Example 384A

*tert*-butyl (3*R*)-3-[2-{[(2*Z*)-5-*tert*-butyl-3-[(2*R*)-tetrahydrofuran-2-ylmethyl]-1,3-thiazol-2(3*H*)-ylidene]carbamoyl}-4-(trifluoromethyl)phenoxy]pyrrolidine-1-carboxylate

The title compound was prepared and isolated as described in Example 373, substituting (R)-tert-butyl 3-hydroxypyrrolidine-1-carboxylate for (R)-1-methylpyrrolidin-3-ol in 88% yield. $^1$H NMR (300 MHz, DMSO-$d_6$) δ ppm 1.32 (s, 9 H) 1.38 (s, 9 H) 1.57 - 1.68 (m, I H) 1.75 - 1.84 (m, 2 H) 1.86 -1.95 (m, 1 H) 2.10 - 2.16 (m, 2 H) 3.34 - 3.45 (m, 3 H) 3.53 - 3.68 (m, 2 H) 3.73 - 3.82 (m, 1 H) 4.15-4.22 (m, 2 H) 4.22 - 4.31 (m, 1 H) 5.13-5.20 (m, 1 H) 7.27 (s, I H) 7.30-7.34 (m, 1 H) 7.75 (dd, *J*=8.92, 2.18 Hz, 1 H) 7.97 - 7.99 (m, 1 H); MS (DCI/$NH_3$) *m/z* 598 $(M+H)^+$.

Example 384B

N-[(2Z)-5-tert-butyl-3-[(2R)-tetrahydrofuran-2-ylmethyl]-1,3-thiazol-2(3H)-ylidene]-2-[(3R)-pyrrolidin-3-yloxy]-5-(trifluoromethyl)benzamide

To a solution of Example 384A (1.223 g, 2.046 mmol) in dichloromethane (5 mL) was added 2,2,2-trifluoroacetic acid (2.333 g, 20.46 mmol). The mixture was stirred at room temperature for 2 hr and then concentrated. The mixture was taken up in dichloromethane and washed with sodium bicarbonate. The organic layers were combined, dried and concentrated to give the title compound (0.92 g, 90%). $^1$H NMR (300 MHz, $CDCl_3$) $\delta$ ppm 1.37 (s, 9 H) 1.58 - 1.72 (m, 1H) 1.79 - 1.94 (m, 2 H) 2.01 - 2.15 (m, 3 H) 2.96 - 3.06 (m, 2 H) 3.23 - 3.34 (m, 2 H) 3.74-3.89 (m, 2 H) 4.14-4.21 (m, 1 H) 4.24-4.32 (m, 1H) 4.39-4.46 (m, 1 H) 4.97-5.03 (m, 1H) 6.88 (s, 1 H) 7.05 (d, *J*=8.73 Hz, I H) 7.60 (dd, *J*=8.72, 2.38 Hz, 1 H) 8.17 (d, *J*=2.38 Hz, 1 H); MS (DCI/$NH_3$) *m/z* 498 $(M+H)^+$. Anal. calcd $C_{24}H_{30}F_3N_3O_3S$•1.2 $H_2O$: C, 55.52; H, 6.29; N, 8.09; Found: C, 55.39; H, 6.63; N, 8.01.

Example 385

N-[(2Z)-5-tert-butyl-3-[(2R)-tetrahydrofuran-2-ylmethyl]-1,3-thiazol-2(3H)-ylidene]-2-[(3S)-pyrrolidin-3-yloxy]-5-(trifluoromethyl)benzamide

Example 385A

tert-butyl (3*S*)-3-[2-{[(2*Z*)-5-*tert*-butyl-3-[(2*R*)-tetrahydrofuran-2-ylmethyl]-1,3-thiazol-2(3*H*)-ylidene]carbamoyl}-4-(trifluoromethyl)phenoxy]pyrrolidine-1-carboxylate

The title compound was prepared and isolated as described in Example 373, substituting (S)-tert-butyl 3-hydroxypyrrolidine-1-carboxylate for (R)-1-methylpyrrolidin-3-ol in 84% yield. $^1$H NMR (300 MHz, $CDCl_3$) $\delta$ ppm 1.36 (s, 9 H) 1.45 (s, 9 H) 1.67 - 1.72 (m, 1 H) 1.75 - 1.91 (m, 2 H) 1.91 - 2.15 (m, 2 H) 2.23-2.29 (m, 1 H) 3.46-3.61 (m, 3 H) 3.65-3.69 (m, 1 H) 3.73-3.88 (m, 2 H) 4.13 - 4.29 (m, 2 H) 4.36 - 4.48 (m, 1 H) 4.99 - 5.03 (m, 1 H) 6.88 (s, 1 H) 6.97 (d, *J*=8.72 Hz, 1 H) 7.57-7.61 (m, 1 H) 8.18 (d, *J*=2.38 Hz, 1 H); MS (DCI/$NH_3$) *m/z* 598 $(M+H)^+$.

Example 385B

N-[(2Z)-5-tert-butyl-3-[(2R)-tetrahydrofuran-2-ylmethyl]-1,3-thiazol-2(3H)-ylidene]-2-[(3S)-pyrrolidin-3-yloxy]-5-(trifluoromethyl)benzamide

The title compound was prepared and isolated as described in Example 384B, substituting Example 385A for Example 384A in 61% yield. $^1$H NMR (300 MHz, DMSO-$d_6$) $\delta$ ppm 1.32 (s, 9 H) 1.60 - 1.69 (m, 1 H) 1.77 - 1.86 (m, 2 H) 1.87 - 1.98 (m, 2 H) 2.03 - 2.15 (m, I H) 2.95 - 3.11 (m, 3 H) 3.20 - 3.26 (m, 2 H) 3.61 - 3.69 (m, 1 H) 3.75-3.83 (m, 1 H) 4.21 - 4.22 (m, 2 H) 4.24-4.33 (m, 1 H) 5.11 - 5.14 (m, 1 H) 7.28 (s, 1 H) 7.31 (d, *J*=8.82 Hz, 1 H) 7.76 (dd, *J*=8.82, 2.03 Hz, 1 H) 8.03 (d, *J*=2.03 Hz, I H); MS (DCI/$NH_3$) *m/z* 498 $(M+H)^+$. Anal. calcd $C_{14}H_{30}F_3N_3O_3S$•0.4 EtOAc•0.3 $CH_2Cl_2$: C, 53.18; H, 5.79; N, 7.27; Found: C, 53.33; H, 5.47; N, 6.94.

1115877 Example 386 Xueqing Wang

N-[(2Z)-5-tert-butyl-3-[(2R)-tetrahydrofuran-2-ylmethyl]-1,3-thiazol-2(3H)-ylidene]-5-(trifluoromethyl)-2-vinylbenzamide

Example 386A

2-bromo-5-(trifluoromethyl)benzoyl chloride

The title compound was prepared using the method described in Example 375B, substituting 2-bromo-5-(trifluoromethyl)benzoic acid for Example 375A.

Example 386B

2-bromo-*N*-[(2*Z*)-5-*tert*-butyl-3-[(2*R*)-tetrahydrofuran-2-ylmethyl]-1,3-thiazol-2(3*H*)-ylidene]-5-(trifluoromethyl)benzamide

The title compound was prepared as described in Example 372B, substituting Example 386A for 2-fluoro-5-(trifluoromethyl)benzoyl chloride in 88% yield. $^1$H NMR (300 MHz, DMSO-$d_6$) δ ppm 1.33 (s, 9 H) 1.54 - 1.69 (m, 1 H) 1.77- 1.86 (m, 2 H) 1.88 - 1.97 (m, 1 H) 3.61 - 3.69 (m, 1 H) 3.74 - 3.82 (m, 1 H) 4.21 - 4.34 (m, 3 H) 7.34 (s, 1 H) 7.71 (dd, *J*=8.33, 2.38 Hz, 1 H) 7.93 (d, *J*=8.33 Hz, 1 H) 8.08 (d, *J*=2.38 Hz, 1 H); MS (DCI/$NH_3$) *m/z* 491 $(M+H)^+$.

Example 386C

N-[(2Z)-5-tert-butyl-3-[(2R)-tetrahydrofuran-2-ylmethyl]-1,3-thiazol-2(3H)-ylidene]-5-(trifluoromethyl)-2-vinylbenzamide

To a solution Example 386B (200 mg, 0.407 mmol) and dibutylvinylboronate (150 mg, 0.814 mmol) in 1,2-dimethoxyethane (1 mL) and methyl alcohol (0.500 mL) was added palladium tetrakistriphenylphosphine (47.0 mg, 0.041 mmol) and cesium fluoride (185 mg, 1.22 mmol). This mixture was heated in a microwave at 110 °C for 15 min. The reaction mixture was cooled to room temperature and concentrated, and the residue was purified by column chromatography using an Analogix® Intelliflash280™ ($SiO_2$, 0-30% ethyl acetate in hexane over 25 min) to obtain the title compound (150 mg, 84%). $^1$H NMR (300 MHz, $CDCl_3$) δ ppm 1.36 (s, 9 H) 1.61 - 1.73 (m, 1 H) 1.79 - 1.95 (m, 2 H) 2.02 - 2.13 (m, 1 H) 3.75 - 3.89 (m, 2 H) 4.14 - 4.24 (m, 1 H) 4.27 - 4.33 (m, 1 H) 4.42 - 4.48 (m, 1 H) 5.34 - 5.41 (m, 1 H) 5.64 - 5.72 (m, 1 H) 6.88 (s, 1 H) 7.60 - 7.73 (m, 3 H) 8.39 (s, 1 H); MS (DCI/$NH_3$) *m/z* 439 $(M+H)^+$.

Example 387

tert-butyl 4-[2-({[(2Z)-5-tert-butyl-3-[(2R)-tetrahydrofuran-2-ylmethyl]-1,3-thiazol-2(3H)-ylidene]amino}carbonyl)-4-(trifluoromethyl)phenyl]-3,6-dihydropyridine-1(2H)-carboxylate

The title compound was prepared and isolated as described in Example 386C, substituting 1-(tert-butoxycarbonyl)-1,2,3,6-tetrahydropyridin-4-ylboronic acid for dibutylvinylboronate in 93% yield. $^1$H NMR (300 MHz, $CDCl_3$) δ ppm 1.36 (s, 9 H) 1.48 (s, 9 H) 1.64 - 1.70 (m, 1 H) 1.79 - 1.90 (m, 2H) 1.99 - 2.10 (m, 1 H) 2.42 - 2.46 (m, 1 H) 3.59 - 3.63 (m, 2 H) 3.70 - 3.88 (m, 3 H) 4.03 - 4.07 (m, 2 H) 4.10 - 4.17 (m, 1 H) 4.22 - 4.27 (m, 1 H) 4.39 - 4.45 (m, 1 H) 5.63 - 5.67 (m, 1 H) 6.88 (s, 1H) 7.31 (d, *J*=7.93 Hz, 1 H) 7.60 (dd, *J*=7.73, 1.78 Hz, 1 H) 8.21 -8.24 (m, 1 H); MS (DCI/$NH_3$) *m/z* 594 $(M+H)^+$.

Example 388

N-[(2Z)-5-tert-butyl-3-[(2R)-tetrahydrofuran-2-ylmethyl]-1,3-thiazol-2(3H)-ylidene]-2-(1,2,3,6-tetrahypyridin-4-yl)-5-(trifluoromethyl)benzamide

The title compound was prepared and isolated using the method in Example 384B, substituting Example 387 for example 384A in 61% yield. $^1$H NMR (300 MHz, $CDCl_3$) δ ppm 1.36 (s, 9 H) 1.64-1.68 (m, 1 H) 1.81 - 1.91 (m, 2H) 2.03-2.11 (m, 2 H) 2.31-2.33 (m, 1H) 3.10 (t, *J*=5.55 Hz, 2 H) 3.51 - 3.55 (m, 2 H) 3.75 - 3.90 (m, 2 H) 4.10 - 4.21 (m, 1 H) 4.25 - 4.32 (m, 1 H) 4.46 (dd, *J*=13.68, 2.97 Hz, 1 H) 5.68 - 5.72 (m, 1 H) 6.88 (s, 1 H) 7.32 (d, *J*=7.93 Hz, 1 H) 7.60 (dd, *J*=8.13, 1.78 Hz, 1 H) 8.20 (d, *J*=1.59 Hz, 1 H); MS (DCI/$NH_3$) *mlz* 494 $(M+H)^+$.

Example 389

N-[(2Z)-5-tert-butyl-3-[(2R)-tetrahydrofuran-2-ylmethyl]-1,3-thiazol-2(3H)-ylidene]-2-ethyl-5-(trifluoromethyl)benzamide

A mixture of Example 386C (120 mg, 0.274 mmol) and palladium hydroxide (19.22 mg, 0.137 mmol) in methyl alcohol (2.0 mL) was hydrogenated under 1 atm $H_2$ at 20 °C for 4 hr. The reaction was filtered and the filtrate concentrated The residue was isolated by preparative HPLC on a Phenomenex Luna Combi-HTS C8(2) column (5 μm, 100A, 2.1mm × 30mm), using a gradient of 10-100% acetonitrile (A) and 10 mM ammonium acetate in water (B), at a flow rate of 2.0 mL/min (0-0.1 min 10% A, 0.1-2.6 min 10-100% A, 2.6-2.9 min 100% A, 2.9-3.0 min 100-10% A. 0.5min post-run delay) to provide the title compound (92 mg, 76%). $^1$H NMR (300 MHz, DMSO-$d_6$) δ ppm 1.19 (t, *J*=7.29 Hz, 3 H) 1.33 (s, 9 H) 1.59- 1.70 (m, 1 H) 1.78 - 1.87 (m, 2 H) 1.88 - 1.99 (m, 1 H) 3.09 (q, *J*=7.46 Hz, 2 H) 3.62 - 3.70 (m, 1 H) 3.75 - 3.83 (m, 1 H) 4.22 - 4.32 (m, 3 H) 7.30 (s, 1 H) 7.52 (d, *J*=8.14 Hz, 1 H) 7.72 (d, *J*=7.12 Hz, 1 H) 8.19 (s, 1 H); MS (DCI/$NH_3$) *m/z* 441 $(M+H)^+$. Anal. calcd $C_{22}H_{27}F_3N_2O_2S{\bullet}0.3\ H_2O$: C, 59.26; H, 6.24; N, 6.28. Found: C, 59.14; H, 6.06; N, 6.20.

Example 390

N-[(2Z)-5-tert-butyl-3-[(2R)-tetrahydrofuran-2-ylmethyl]-1,3-thiazol-2(3H)-ylidene]-2-[(E)-2-(methylsulfonyl)vinyl]-5-(trifluoromethyl)benzamide

To a solution of Example 386B (172 mg, 0.350 mmol), triethylamine (53 mg, 0.525 mmol) and methylvinylsulfone (55.7 mg, 0.525 mmol) in acetonitrile (1 mL) was added palladium acetate(4.72 mg, 0.021 mmol) and tri(o-tolyl)phosphine (23.44 mg, 0.077 mmol). This mixture was heated in a microwave at 140 °C for 30 min. The reaction was heated another 30 min at 180 °C. The reaction mixture was cooled to room temperature and filtered. The residue was purified by preparative HPLC on a Phenomenex Luna Combi-HTS C8(2) column (5μm, 100Å, 2.1mm × 30mm), using a gradient of 10-100% acetonitrile (A) and 10 mM ammonium acetate in water (B), at a flow rate of 2.0 mL/min (0-0.1 min 10% A, 0.1-2.6 min 10-100% A, 2.6-2.9 min 100% A, 2.9-3.0 min 100-10% A. 0.5min post-run delay) to provide the title compound (85 mg, 47%). $^1$H NMR (300 MHz, $CDCl_3$) δ ppm 1.38 (s, 9 H) 1.62-1.71 (m, 1 H) 1.76 - 1.92 (m, 2 H) 2.02-2.14 (m, 1 H) 3.08 (s, 3 H) 3.73 - 3.87 (m, 2 H) 4.22-4.34 (m, 2 H) 4.50 (d, *J*=11.10 Hz, 1 H) 6.80 (d, *J*=15.47 Hz, 1 H) 6.97 (s, 1 H) 7.59-7.72 (m, 2H) 8.52 (s, 1 H) 8.83 (d, *J*=15.47 Hz, 1 H); MS (DCI/$NH_3$) *m/z* 517 $(M+H)^+$.

Example 391

N-[(2Z)-5-tert-butyl-3-[(2R)-tetrahydrofuran-2-ylmethyl]-1,3-thiazol-2(3H)-ylidene]-2-[2-(methylsulfonyl)ethyl]-5-(trifluoromethyl)benzamide

The title compound was prepared and isolated using the method described in Example 389, substituting Example 390 for Example 386C in 87% yield. $^1$H NMR (300 MHz, $CDCl_3$) δ ppm 1.38 (m, 9H) 1.64-1.74 (m, 1 H) 1.81 - 1.97 (m, 2 H) 2.04 - 2.15 (m, 1 H) 3.01 (s, 3 H) 3.40-3.47 (m, 2 H) 3.59-3.67 (m, 2 H) 3.75 - 3.90 (m, 2 H) 4.18 - 4.26 (m, 1 H) 4.27-4.34 (m, 1 H) 4.46 (dd, *J*=13.39, 2.88 Hz, 1 H) 6.91 (s, 1 H) 7.43 (d, *J*=8.14 Hz, 1 H) 7.60 - 7.66 (m, 1 H) 8.51 (d, *J*=1.70 Hz, 1 H); MS (DCI/$NH_3$) *m/z* 519 $(M+H)^+$.

Example 392

N-[(2Z)-5-tert-butyl-3-[(2R)-tetrahydrofuran-2-ylmethyl]-1,3-thiazol-2(3H)-ylidene]-2-{[(2S)-2-(hydroxymethyl)pyrrolidin-1-yl]methyl}-5-(trifluoromethyl)benzamide

In a vial (5 mL), a mixture of Example 375C (88 mg, 0.200 mmol), support-bound cyanoborohydride resin (0.256 g, 0.599 mmol), (S)-pyrrolidin-2-ylmethanol (60 mg, 0.593 mmol), and acetic acid (18 mg, 0.300 mmol) in dichloromethane (1 mL) and methyl alcohol (1 mL) was shaken on an orbit shaker for 2 hr. The reaction was filtered, concentrated and the residue was purified by preparative HPLC on a Phenomenex Luna Combi-HTS C8(2) column (5μm, 100Å, 2.1mm × 30mm), using a gradient of 10-100% acetonitrile (A) and 0.1% trifluoroacetic acid in water (B), at a flow rate of 2.0 mL/min (0-0.1 min 10% A, 0.1-2.6 min 10-100% A, 2.6-2.9 min 100% A, 2.9-3.0 min 100-10% A. 0.5min post-run delay) in 38% yield as TFA salt. $^1$H NMR (300 MHz, $CD_3OD$) δ ppm 1.41 (s, 9 H) 1.72 - 1.81 (m, 1 H) 1.88 - 1.99 (m, 2 H) 2.05 - 2.20 (m, 3 H) 2.27 - 2.41 (m, 1 H) 3.34 - 3.42 (m, 1 H) 3.52 - 3.66 (m, 1 H) 3.67 - 3.82 (m, 4 H) 3.85 - 3.96 (m, 1 H) 4.36 - 4.46 (m, 3 H) 4.57 (d, *J*=12.69 Hz, 1 H) 4.86 - 4.93 (m, 2 H) 7.28 (s, 1 H) 7.76 - 7.82 (m, 1 H) 7.87 - 7.94 (m, 1 H) 8.73 - 8.75 (m, 1 H); MS (DCI/$NH_3$) *m/z* 526 $(M+H)^+$. Anal. calcd calcd $C_{26}H_{34}F_3N_3O_3S$•1.9 $CF_3CO_2H$: C, 48.22; H, 4.87; N, 5.66; Found: C, 48.28; H, 4.63; N, 5.67.

Example 393

N-[(2Z)-5-tert-butyl-3-[(2R)-tetrahydrofuran-2-ylmethyl]-1,3-thiazol-2(3H)-ylidene]-2-(morpholin-4-ylmethyl)-5-(trifluoromethyl)benzamide

The title compound was prepared and purified as described in Example 392, substituting morpholine for (S)-pyrrolidin-2-ylmethanol in 42% yield as the TFA salt. $^1$H NMR (300 MHz, $CD_3OD$) δ ppm 1.41 (s, 9H) 1.71 - 1.82 (m, 1 H) 1.88 - 1.98 (m, 2 H) 2.06-2.13 (m, 1 H) 3.36 - 3.40 (m, 2 H) 3.46 - 3.57 (m, 2 H) 3.75 - 3.91 (m, 4 H) 4.05 - 4.16 (m, 2 H) 4.37 - 4.47 (m, 3 H) 4.68 (s, 2 H) 7.29 (s, 1 H) 7.76 (d, *J*=7.93 Hz, 1 H) 7.92 (dd *J*=7.93, 1.98 Hz, 1 H) 8.79 (s, 1 H); MS (DCI/$NH_3$) *m/z* 512 $(M+H)^+$. Anal. Calcd $C_{25}H_{32}F_3N_3O_3S$•1.9 $CF_3CO_2H$: C, 47.50; H, 4.69; N, 5.67; Found: C, 47.62; H, 4.67; N, 5.86.

Example 394

N-[(2Z)-5-tert-butyl-3-[(2R)-tetrahydrofuran-2-ylmethyl]-1,3-thiazol-2(3H)-ylidene]-2-(thiomorholin-4-ylmethyl)-5-(trifluoromethyl)benzamide

The TFA salt of the title compound was prepared and purified as described in Example 392, substituting thiomorpholine for (S)-pyrrolidin-2-ylmethanol in 45% yield. $^1H$ NMR (300 MHz, $CD_3OD$) δ ppm 1.40 (s, 9 H) 1.70 - 1.81 (m, 1 H) 1.88 - 1.98 (m, 2 H) 2.15 - 2.15 (m, 1 H) 2.93 - 3.02 (m, 2 H) 3.04 - 3.14 (m, 2 H) 3.40 - 3.48 (m, 2 H) 3.73 - 3.93 (m, 4 H) 4.37 - 4.47 (m, 3 H) 4.66 (s, 2 H) 7.29 (s, 1 H) 7.74 - 7.76 (m, 1 H) 7.90 - 7.92 (m, 1 H) 8.75- 8.77 (m, 1 H); MS (DCI/$NH_3$) *m/z* 528 $(M+H)^+$. Anal. calcd $C_{25}H_{32}F_3N_3O_2S_2$•1.1 $CF_3CO_2H$: C, 50.02; H, 5.11; N, 6.43; Found: C, 49.97; H, 5.05; N, 6.40.

Example 395

N-[(2Z)-5-tert-butyl-3-[(2R)-tetrahydrofuran-2-ylmethyl]-1,3-thiazol-2(3H)-ylidene]-2-[(4-methylpiperazin-1-yl)methyl]-5-(trifluoromethyl)benzamide

The TFA salt of the title compound was prepared and purified as described in Example 392, substituting *N*-methyl piperidine for (S)-pyrrolidin-2-ylmethanol in 45% yield. $^1H$ NMR (300 MHz, $CD_3OD$) δ ppm 1.40 (s, 9 H) 1.66 - 1.78 (m, I H) 1.87 - 1.97 (m, 2 H) 2.02 - 2.14 (m, I H) 2.81 (s, 3 H) 2.94 - 3.08 (m, 4 H) 3.18-3.28 (m, 4 H) 3.72-3.81 (m, I H) 3.85-3.93 (m, I H) 4.28-4.41 (m, 5 H) 7.23 (s, 1H) 7.71 - 7.80 (m, 2 H) 8.33 (s, I H); MS (DCI/$NH_3$) *m/z* 525 $(M+H)^+$. Anal. calcd $C_{26}H_{35}F_3N_4O_2S$•2.0 $CF_3CO_2H$: C, 47.87; H, 4.95; N, 7.44; Found: C, 47.69; H, 4.92; N, 7.38.

Example 396

N-[(2Z)-5-tert-butyl-3-[(2R)-tetrahydrofuran-2-ylmethyl]-1,3-thiazol-2(3H)-ylidene]-2-{[(cyanomethyl)(methyl)amino]methyl}-5-(trifluoromethyl)benzamide

The title compound was prepared and purified as described in Example 392, substituting 2-(methylamino) acetonitrile for (S)-pyrrolidin-2-ylmethanol in 45% yield. $^1H$ NMR (300 MHz, $CD_3OD$) δ ppm 1.40 (s, 9 H) 1.69- 1.75 (m, 1 H) 1.85 - 1.97 (m, 2 H) 2.04 - 2.17 (m, 1 H) 2.53 (s, 3 H) 3.73 - 3.80 (m, 1 H) 3.85 - 3.92 (m, 3 H) 4.29 (s, 2H), 4.30 - 4.42 (m, 3 H) 7.21 (s, 1 H) 7.71 - 7.80 (m, 2 H) 8.28 - 8.30 (m, 1 H); MS (DCI/$NH_3$) *m/z* 495 $(M+H)^+$.

Example 397

N-[(2Z)-tert-butyl-3-[(2R)-tetrahydrofuran-2-ylmethyl]-1,3-thiazol-2(3H)-ylidene]-2-{[(2R)-2-(hydroxymethyl)pyrrolidin-1-yl]methyl}-5-(trifluommethyl)benzamide

The TFA salt of the title compound was prepared and purified as described in Example 392, substituting (R)-pyrrolidin-2-ylmethanol for (S)-pyrrolidin-2-ylmetbanol in 60% yield. $^1H$ NMR (300 MHz, $CD_3OD$) δ ppm 1.41 (s, 9 H) 1.70 - 1.82 (m, 1 H) 1.88 -1.98 (m, 2 H) 2.05 - 2.22 (m, 4 H) 2.33 - 2.37 (m, 1 H) 3.33 - 3.40 (m, 1 H) 3.51 - 3.59 (m, 1 H) 3.68 - 3.82 (m, 4 H) 3.86 - 3.94 (m, 1 H) 4.36 - 4.44 (m, 3 H) 4.53 - 4.61 (m, 1 H) 4.88 - 4.92 (m, 1 H) 7.28 (s, 1 H) 7.75 - 7.81 (m, 1 H) 7.90 (dd, *J*=7.97, 1.53 Hz, 1 H) 8.74 (d, *J*=1.70 Hz, 1 H); MS (DCI/$NH_3$) *m/z* 526 $(M+H)^+$. Anal. calcd $C_{26}H_{34}F_3N_3O_3S$•2.0 $CF_3CO_2H$: C, 47.81; H, 4.81; N, 5.58; Found: C, 47.72; H, 4.91; N, 5.49.

Example 398

2-[(tert-butylamino)methyl]-N-[(2Z)-5-tert-butyl-3-[(2R)-tetrahydrofuran-2-ylmethyl]-1,3-thiazol-2(3H)-ylidene]-5-(trifluoromethyl)benzamide

The TFA salt of the title compound was prepared and purified as described in Example 392, substituting tert-butyl amine for (S)-pyrrolidin-2-ylmethanol in 80% yield. $^1H$ NMR (300 MHz, $CD_3OD$) δ ppm 1.40 (s, 9 H) 1.55 (s, 9 H) 1.71 - 1.82 (m, 1 H) 1.88 - 1.98 (m, 2 H) 2.04 - 2.15 (m, 1 H) 3.73 - 3.81 (m, 1 H) 3.86 - 3.94 (m, 1 H) 4.36 - 4.46 (m, 3 H), 4.48 (s, 2 H) 7.27 (s, 1 H) 7.76 (d, *J*=8.14 Hz, 1 H) 7.88 (dd, *J*=7.97, 1.86 Hz, 1 H) 8.69 (d, *J*=1.36 Hz, 1 H); MS (DCI/$NH_3$) *m/z* 498 $(M+H)^+$. Anal. calcd $C_{25}H_{34}F_3N_3O_2S$•1.0 $CF_3CO_2H$: C, 53.02; H, 5.77; N, 6.87; Found: C, 52.92; H, 5.71; N, 6.80.

It is understood that the foregoing detailed description and accompanying examples are merely illustrative and are not to be taken as limitations upon the scope of the invention, which is defined solely by the appended claims and

their equivalents.

## Claims

1. A compound according to formula (I),

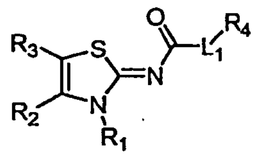

(I),

or a pharmaceutically acceptable salt, thereof, wherein
$R_1$ is A or A-alkylene-;
$R_2$ is hydrogen, alkoxy, alkoxyalkyl, alkoxycarbonyl, alkoxycarbonylalkyl, alkyl, akyl-$S(O)_2$-, aryl, arylalkyl, arylalkenyl, azidoalkyl, cyano, cycloalkyl, halo, haloalkyl, heteroaryl, heterocycle, $-(CR_{21}R_{22})_m$-OH, $R_aR_bN$-, $R_aR_bN$-alkyl-, $R_cR_dNC(O)$-, or $R_8$-$R_7$-;
$R_3$ is hydrogen, alkoxy, alkoxyalkyl, alkyl, alkylcarbonyl, alkyl-$S(O)_2$-, aryl, arylalkyl, arylalkenyl, cyano, cycloalkyl, halo, haloalkyl, heteroaryl, heterocycle, $-(CR_{31}R_{32})_m$-OH, $R_aR_bN$-, $R_aR_bN$-alkyl-, or $R_8$-$R_7$-; or
$R_2$ and $R_3$, together with the carbon atoms to which they are attached, form a 4-, 5-, 6-, or 7-membered monocyclic ring, optionally fused to benzo or oxadiazole, said monocyclic ring contains zero or one additional double bond, zero or one oxygen atom, and zero or one nitrogen atom as ring atoms; two non-adjacent atoms of said monocyclic ring are optionally linked by an alkenylene bridge of 2, 3, or 4 carbon atoms, or optionally linked by an alkylene bridge of 1, 2, 3, or 4 carbon atoms, said monocyclic ring is independently unsubstituted or substituted with 1, 2, 3, 4, or 5 substituents independently selected from the group consisting of oxo, alkyl, halo, -OH, -O(alkyl), and haloalkyl; two substituents on the same carbon atom of said monocyclic ring, together with the carbon atom to which they are attached, optionally form a 3-, 4-, 5-, or 6-membered monocyclic cycloalkyl ring, wherein the monocyclic cycloalkyl ring is optionally substituted with 1, 2, 3, 4, 5, or 6 substituents independently selected from the group consisting of alkyl and haloalkyl;
$R_4$ is alkyl, alkynyl, cycloalkyl; cycloalkenyl, aryl, heteroaryl, heterocycle, cycloalkylalkyl, cycloalkenylalkyl, arylalkyl, heteooarylalkyl, heterocyclealkyl, or $R_{10}$-$L_2$-$R_9$-; wherein the alkyl group is optionally substituted with one substituent selected from the group consisting of alkoxy, alkoxycarbonyl, carboxy, halo, -OH, and $R_eR_fN$-;
$R_7$, $R_8$, and $R_9$, are each independently aryl, cycloalkyl, cycloalkenyl, heteroaryl, or heterocycle;
$R_{10}$ is aryl, cycloalkyl, cycloalkenyl, heteroaryl, heterocycle, or cycloalkylalkyl;
$R_a$ and $R_b$; at each occurrence, are each independently hydrogen, alkoxycarbonyl, alkyl, alkylcarbonyl, alkyl-$S(O)_2$-, or arylalkyl;
$R_c$ and $R_d$, are each independently hydrogen or alkyl;
$R_e$ and $R_f$, are each independently hydrogen, alkyl, or alkylcarbonyl;
A is furanyl, oxazolyl, isoxazolyl, or oxadiazolyl; each ring A is optionally fused with a monocyclic ring selected from the group consisting ofbenzo, cycloalkyl, cycloalkenyl, heterocycle and heteroaryl; and each A is independently unsubstituted or substituted with 1, 2, 3, 4, 5, or 6 substituents independently selected from the group consisting of oxo, alkyl, halo, -OH, -O(alkyl), and haloalkyl;
$L_1$ is a single bond or $-NR_g$-;
$L_2$ is a single bond, alkylene, or -O-;
$R_g$ is hydrogen or alkyl;
the aryl, cycloalkyl, cycloalkenyl, heterocycle, or heteroaryl moieties, as a substituent, or as part of a substituent, as represented by $R_2$, $R_3$, $R_4$, $R_a$, $R_b$, $R_7$, $R_8$, $R_9$, and $R_{10}$, are each independently unsubstituted or substituted with 1, 2, 3, 4, 5, or 6 substituents independently selected from the group consisting of alkenyl, alkoxy, alkoxyalkoxy, alkoxyalkoxyalkyl, alkoxyalkyl, alkoxycarbonyl, alkoxycarbonylalkyl, alkoxysulfonyl, alkyl, alkylcarbonyl, alkylcarbonylalkyl, alkylcarbonyloxy, alkylsulfinyl, alkylsulfinylalkyl, alkyl-$S(O)_2$-, alkyl-$S(O)_2$-$(CR_{41}R_{42})_p$=$C(R_{41})$-, alkyl-S$(O)_2$-$(CR_{41}R_{42})_p$-, alkyl-S-, alkyl-S-$(CR_{41}R_{42})_p$-, alkynyl, carboxy, carboxyalkyl, cyano, cyanoalkyl, formyl, formylalkyl, halogen, haloalkyl, haloalkoxy, hydroxy, hydroxyalkyl, oxo, -SH, $N(O)_2$, $-C(R_{41})$=N-O$(R_{42})$, $-(CR_{41}R_{42})_p$-$C(R_{41})$=N-O$(R_{42})$, =N-O(alkyl), =N-OH, $NZ_1Z_2$-$(CR_{41}R_{42})_p$-O-, -O-$(CR_{41}R_{42})_p$-$G_1$, $G_1$, $-NZ_1Z_2$, $-(CR_{41}R_{42})_p$-$NZ_1Z_2$, and $(NL_3Z_4)$carbonyl;

$G_1$ is a 4-, 5-, 6-, or 7-membered monocyclic heterocycle containing one nitrogen atom and optionally 1 or 2 additional heteroatom in the ring, wherein said ring is attached to the parent moiety through the nitrogen atom, and said ring is optionally substituted with 1, 2, 3, 4, or 5 substituents selected from the group consisting of alkyl, alkenyl, alkynyl, halo, haloalkyl, =N-CN, =N-$OR_{51}$, -CN, oxo, -$OR_{51}$, -OC(O)$R_{51}$, -OC(O)N$(R_{51})_2$, -S$(O)_2R_{52}$, -S$(O)_2$N$(R_{51})_2$, -C(O)$R_{51}$, -C(O)$OR_{51}$,- C(O)N$(R_{51})_2$, -N$(R_{51})_2$, -N$(R_{51})$C(O)$R_{51}$, -N$(R_{51})$S$(O)_2R_{52}$, -N$(R_{51})$C(O)O$(R_{52})$, -N$(R_{51})$C(O)N$(R_{51})_2$, $-(CR_{1c}R_{1d})_q$-$OR_{51}$, $-(CR_{1c}R_{1d})_q$-OC(O)$R_{51}$, $-(CR_{1c}R_{1d})_q$-OC(O)N$(R_{51})_2$, $-(CR_{1c}R_{1d})_q$-S$(O)_2R_{52}$, $-(CR_{1c}R_{1d})_q$-S$(O)_2$N$(R_{51})_2$, $-(CR_{1c}R_{1d})_q$-C(O)$R_{51}$, $-(CR_{1c}R_{1d})_q$-C(O)$OR_{51}$, $-(CR_{1c}R_{1d})_q$-C(O)N$(R_{51})_2$, $-(CR_{1c}R_{1d})_q$-N$(R_{51})_2$, $-(CR_{1c}R_{1d})_q$-N$(R_{51})$C(O)$R_{51}$, $-(CR_{1c}R_{1d})_q$-N$(R_{51})$S$(O)_2R_{52}$, $-(CR_{1c}R_{1d})_q$-N$(R_{51})$C(O)O$(R_{52})$, $-(CR_{1c}R_{1d})_q$-N$(R_{51})$C(O)N$(R_{51})_2$, and $-(CR_{1c}R_{1d})_q$-CN;

$R_{51}$, at each occurrence, is independently hydrogen, $C_1$-$C_4$alkyl, $C_1$-$C_4$haloalkyl, $-(CR_{2c}R_{2d})_u$-$OR^{53}$, monocyclic cycloalkyl, or $-(CR_{1c}R_{1d})_u$-(monocyclic cycloalkyl); wherein $R_{53}$ is hydrogen, $C_1$-$C_4$ alkyl, $C_1$-$C_4$ haloalkyl, monocyclic cycloalkyl, or $-(CR_{2c}R_{2d})_u$-(monocyclic cycloalkyl);

$R_{52}$, at each occurrence, is independently $C_1$-$C_4$ alkyl, $C_1$-$C_4$ haloalkyl, monocyclic cycloalkyl, or $-(CR_{2c}R_{2d})_u$-(monocyclic cycloalkyl);

the monocyclic cycloalkyl moiety, as a substituent, or as part of a substituent, as represented by $R_{51}$, $R_{52}$, and $R_{53}$ are each independently unsubstituted or substituted with 1, 2, 3, or 4 substituents selected from the group consisting of $C_1$-$C_4$ alkyl, halo, hydroxy, $C_1$-$C_4$ alkoxy, $C_1$-$C_4$ haloalkoxy, and $C_1$-$C_4$haloalkyl;

$R_{21}$, $R_{22}$, $R_{31}$, $R_{32}$, $R_{41}$, $R_{42}$, $R_{1c}$, $R_{1d}$, $R_{2c}$, and $R_{2d}$, at each occurrence, are each independently hydrogen, alkyl, haloalkyl, or halo;

m, p, q, and u, at each occurrence, are each independently 1, 2, 3, or 4;

$Z_1$ and $Z_2$ are each independently hydrogen, alkyl, alkoxyalkyl, alkylcarbonyl, cyanoalkyl, haloalkyl, or formyl; and

$Z_3$ and $Z_4$ are each independently hydrogen, alkyl, haloalkyl, phenyl or benzyl wherein the phenyl moiety is optionally substituted with 1, 2, 3, or 4 substituents selected from the group consisting of alkyl, hydroxyl, and haloalkyl.

**2.** The compound of claim 1, or a pharmaceutically acceptable salt thereof wherein
$R_2$ is hydrogen, alkoxycarbonyl, alkoxycarbonylalkyl, alkyl aryl, halo, haloalkyl, or $-(CR_{21}R_{22})_m$-OH;
$R_3$ is hydrogen, alkyl, alkylcarbonyl, aryl, cycloalkyl, halo, haloalkyl, heterocycle, or $-(CR_{31}R_{32})_m$-OH;
$L_1$ is a bond; and
$R_4$ is aryl, heteroaryl, cycloalkyl, heterocycle, arylalkyl, heteroarylalkyl, heterocyclealkyl, cycloalkylalkyl, $R_{10}$-$L_2$-$R_9$-, alkynyl, or alkyl; wherein the alkyl is optionally substituted with one substituent selected from the group consisting of alkoxycarbonyl, -OH, or $R_eR_f$N-.

**3.** The compound of claim 2, or a pharmaceutically acceptable salt thereof, wherein
$R_4$ is optionally substituted phenyl or $R_{10}$-$L_2$-$R_9$-;
$L_2$ is alkyene or O;
$R_9$ is optionally substituted aryl.

**4.** The compound of claims 1, or a pharmaceutically acceptable salt thereof, wherein
$R_2$ is hydrogen or alkyl;
$R_3$ is alkyl or $-(CR_{31}R_{32})_m$-OH;
L, is a bond;
$R_4$ is optionally substituted phenyl or $R_{10}$-$L_2$-$R_9$-;
$L_2$ is O; and
$R_9$ is optionally substituted aryl.

**5.** The compound of claim 1, or a pharmaceutically acceptable salt thereof, wherein
$R_2$ is hydrogen, alkoxycarbonyl, alkoxycarbonylalkyl, alkyl, aryl, halo, haloalkyl, or $-(CR_{21}R_{22})_m$-OH;
$R_3$ is hydrogen, alkyl, alkylcarbonyl, aryl, cycloalkyl, halo, haloalkyl, heterocycle, or $-(CR_{31}R_{32})_m$-OH;
L, is a $NR_g$; and
$R_4$ is arylalkyl, cyclolalkyl, cycloalkylalkyl, or alkyl; wherein the alkyl is optionally substituted with one substituent selected from the group consisting of alkoxycarbonyl, -OH, or $R_eR_f$N-.

**6.** The compound of claim 1, or a pharmaceutically acceptable salt
thereof, wherein $R_2$ and $R_3$, together with the carbon atoms to which they are attached, form a 4-, 5-, 6-, or 7-membered monocyclic ring optionally fused to benzo or oxadiazole, said monocyclic ring contains zero or one additional double bond, zero oxygen atom and zero nitrogen atom as ring atoms; two non-adjacent atoms of said monocyclic ring are linked by an alkenylene bridge of 2, 3, or 4 carbon atoms, or linked by an alkylene bridge of 1, 2, 3, or 4 carbon atoms, said monocyclic ring is independently unsubstituted or substituted with 1, 2, 3, 4, or 5

substituents independently selected from the group consisting ofoxo, alkyl, halo, -OH, -O(alkyl), and haloalkyl; two substituents on the same carbon atom of said monocyclic ring, together with the carbon atom to which they are attached, optionally form a 3-, 4-, 5-, or 6-membered monocyclic cycloalkyl ring, wherein the monocyclic cycloalkyl ring is optionally substituted with 1, 2, 3, 4, 5, or 6 substituents independently selected from the group consisting of alkyl and haloalkyl.

**7.** The compound of claim 1, or a pharmaceutically acceptable salt
thereof, wherein $R_2$ and $R_3$, together with the carbon atoms to which they are attached, form an optionally substituted ring as represented by

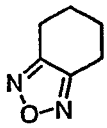

(x)

or

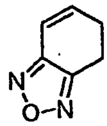

(xa)

**8.** The compound of claim 1, or a pharmaceutically acceptable salt
thereof, wherein $R_2$ and $R_3$, together with the carbon atoms to which they are attached, form a 4-, 5-, 6-, or 7-membered monocyclic ring optionally fused to benzo or oxadiazole, said monocyclic ring contains zero or one additional double bond, and one oxygen atom and zero or one nitrogen atom as ring atoms; two non-adjacent atoms of said monocyclic ring can be optionally linked by an alkenylene bridge of 2, 3, or 4 carbon atoms, or optionally linked by an alkylene bridge of 1, 2, 3, or 4 carbon atoms, said monocyclic ring is independently unsubstituted or substituted with 1, 2, 3, 4, or 5 substituents independently selected from the group consisting ofoxo, alkyl, halo, -OH, -O(alkyl), and haloalkyl; two substituents on the same carbon atom of said monocyclic ring, together with the carbon atom to which they are attached, optionally form a 3-, 4-, 5-, or 6-membered monocyclic cycloalkyl ring, wherein the monocyclic cycloalkyl ring is optionally substituted with 1, 2, 3, 4, 5, or 6 substituents independently selected from the group consisting of alkyl and haloalkyl.

**9.** The compound of claims 6 or 8 , or a pharmaceutically acceptable salt thereof, wherein
$R_4$ is aryl, heteroaryl, cycloalkyl, heterocycle, arylalkyl, heteroarylalkyl, heterocyclealkyl, cycloalkylalkyl, $R_{10}$-$L_2$-$R_9$, alkynyl, or alkyl; wherein the alkyl is optionally substituted with one substituent selected from the group consisting of alkoxycarbonyl, -OH, or $R_eR_fN$-.

**10.** The compound of claim 1, or a pharmaceutically acceptable salt thereof, wherein
$R_2$ and $R_3$, together with the carbon atoms to which they are attached, form a ring as represented by

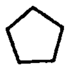

(viii)

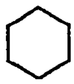

(ix)

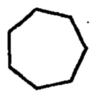

(xi)

**11.** The compound of claim 1 s elected from the group consisting of
N-[(2Z)-5-tert-butyl-3-(1,3-oxazol-2-ylmethyl)-1,3-thiazol-2(3H)-ylidene]-5-chloro-2-methoxybenzamide;
N-[(2Z)-5-tert-butyl-3-(1,2,4-oxadiazol-3-ylmethyl)-1,3-thiazol-2(3H)-ylidene]-5-chloro-2-methoxybenzamide;
N-[(2Z)-5-tert-butyl-3-(2-furylmethyl)-1,3-thiazol-2(3H)-ylidene]-5-chloro-2-methoxybenzamide;
5-chloro-N-[(2Z)-4,5-dimethyl-3-[(3-methylisoxazol-5-yl)methyl]-1,3-thiazol-2(3H)-ylidene]-2-methoxybenzamide;
5-chloro-2-methoxyN-[(2Z)-5-methyl-3-{[5-(triffuoromethyl)-2-furyl]methyl}-1,3-thiazol-2(3H)-ylidene]benzamide;
5-chloro-N-[(2Z)-3-(2-furylmethyl)-5-methyl-1,3-thiazol-2(3H)-ylidene]-2-methoxybenzamide;
5-chloro-N-[(2Z)-3-(3-furylmethyl)-5-methyl-1,3-thiazol-2(3H)-ylidene]-2-methoxybenzamide; and
N-[(2Z)-5-tert-butyl-3-[(5-methylisoxazol-3-yl)methyl]-1,3-thiazol-2(3H)-ylidene]-5-chloro-2-methoxybenzamide;
or a pharmaceutically acceptable salt thereof.

**12.** A pharmaceutical composition comprising therapeutically effective amount of a compound of claim 1 having formula

(I) or a pharmaceutically acceptable salt thereof, in combination with a pharmaceutically acceptable carrier.

13. A compounds of claim 1 having formula (I) or a pharmaceutically acceptable salt thereof for use in treating neuropathic pain, nociceptive pain, and inflammatory pain in a mammal in need of such treatment by administering to the mammal a therapeutically effective amount of said compound.

## Patentansprüche

1. Eine Verbindung gemäß Formel (I)

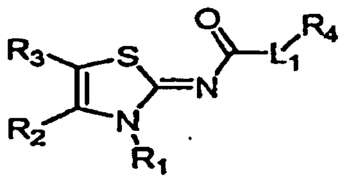

(I),

oder ein pharmazeutisch verträgliches Salz davon, worin
$R_1$ A oder A-Alkylen ist;
$R_2$ ist Wasserstoff, Alkoxy, Alkoxyalkyl, Alkoxycarbonyl, Alkoxycarbonylalkyl, Alkyl, Alkyl-$S(O)_2$-, Aryl, Arylalkyl, Arylalkenyl, Azidoalkyl, Cyano, Cycloalkyl, Halo, Haloalkyl, Heteroaryl, Heterozyklus, $-(CR_{21}R_{22})_m$-OH, $R_aR_bN$-, $R_aR_bN$-Alkyl, $R_cR_dNC$ (O) - oder $R_8$-$R_7$-;
$R_3$ ist Wasserstoff, Alkoxy, Aloxyalkyl, Alkyl, Alkylcarbonyl, Alkyl-$S(O)_2$-, Aryl, Arylalkyl, Arylalkenyl, Cyano, Cycloalkyl, Halo, Haloalkyl, Heteroaryl, Heterozyklus, $-(CR_{31}R_{32})_m$-OH, $R_aR_bN$-, $R_aR_bN$-Alkyl- oder $R_8$-$R_7$-; oder
$R_2$ und $R_3$ zusammen mit den Kohlenstoffatomen, an welche sie gebunden sind, bilden einen 4-, 5-, 6- oder 7-gliedrigen monozyklischen Ring, wahlweise ankondensiert an Benzo oder Oxadiazol, der monozyklische Ring enthält null oder eine zusätzliche Doppelbindung, null oder ein Sauerstoffatom und null oder ein Stickstoffatom als Ringatome; zwei nicht angrenzende Atome des monozyklischen Rings sind wahlweise verbunden durch eine Alkenylenbrücke von 2, 3 oder 4 Kohlenstoffatomen, oder sind wahlweise verbunden durch eine Alkylenbrücke von 1, 2, 3 oder 4 Kohlenstoffatomen, der monozyklische Ring ist unabhängig unsubstituiert oder substituiert mit 1, 2, 3, 4 oder 5 Substituenten unabhängig gewählt aus der Gruppe bestehend aus Oxo, Alkyl, Halo, -OH, -O(Alkyl) und Haloalkyl; zwei Substituenten auf demselben Kohlenstoffatom des monozyklischen Rings, zusammen mit dem Kohlenstoffatom, an welches sie gebunden sind, bilden wahlweise einen 3-, 4-, 5- oder 6-gliedriegen monozyklischen Cycloalkylring, worin der monozyklische Cycloalkylring wahlweise substituiert ist mit 1, 2, 3, 4, 5 oder 6 Substituenten unabhängig gewählt aus der Gruppe bestehend aus Alkyl und Haloalkyl;
$R_4$ ist Alkyl, Alkinyl, Cycloalkyl, Cycloalkenyl, Aryl, Heteroaryl, Heterozyklus, Cycloalkylalkyl, Cycloalkenylalkyl, Arylalkyl, Heteroarylalkyl, Heterozyklusalkyl oder $R_{10}$-$L_2$-$R_9$; worin die Alkylgruppe wahlweise substituiert ist mit einem Substituenten gewählt aus der Gruppe bestehend aus Alkoxy, Alkoxycarbonyl, Carboxy, Halo, -OH und $R_eR_fN$-;
$R_7$, $R_8$ und $R_9$ sind jeweils unabhängig Aryl, Cycloalkyl, Cycloalkenyl, Heteroaryl oder Heterozyklus;
$R_{10}$ ist Aryl, Cycloalkyl, Cycloalkenyl, Heteroaryl, Heterozyklus oder Cycloalkylalkyl;
$R_a$ und $R_b$ sind, jedesmal wenn sie vorkommen, jeweils unabhängig Wasserstoff, Alkoxycarbonyl, Alkyl, Alkylcarbonyl, Alkyl-$S(O)_2$- oder Arylalkyl;
$R_c$ und $R_d$ sind jeweils unabhängig Wasserstoff oder Alkyl; $R_e$ und $R_f$ sind jeweils unabhängig Wasserstoff, Alkyl oder Alkylcarbonyl;
A ist Furanyl, Oxazolyl, Isoxazolyl oder Oxadiazolyl; jeder Ring A ist wahlweise ankondensiert mit einem monozyklischen Ring gewählt aus der Gruppe bestehend aus Benzo, Cycloalkyl, Cycloalkenyl, Heterozyklus und Heteroaryl; und jedes A ist unabhängig unsubstituiert oder substituiert mit 1, 2, 3, 4, 5 oder 6 Substituenten unabhängig gewählt aus der Gruppe bestehend aus Oxo, Alkyl, Halo, -OH, -O(Alkyl) und Haloalkyl;
$L_1$ ist eine Einzelbindung oder $-NR_g$-;
$L_2$ ist eine Einzelbindung, Alkylen oder -O-;
$R_g$ ist Wasserstoff oder Alkyl;
die Aryl-, Cycloalkyl-, Cycloalkenyl-, Heterozyklus- oder Heteroarylanteile sind, als ein Substituent oder als ein Teil

von einem Substituenten, dargestellt durch $R_2$, $R_3$, $R_4$, $R_a$, $R_b$, $R_7$, $R_8$, $R_9$ und $R_{10}$, sind jeweils unabhängig unsubstituiert odder substituiert mit 1, 2, 3, 4, 5 oder 6 Substituenten unabhängig gewählt aus der Gruppe bestehend aus Alkenyl, Alkoxy, Alkoxyalkoxy, Alkoxyalkoxyalkyl, Alkoxyalkyl, Alkoxycarbonyl, Alkoxycarbonylalkyl, Alkoxysulfonyl, Alkyl, Alkylcarbonyl, Alkylcarbonylalkyl, Alkylcarbonyloxy, Alkylsulfinyl, Alkylsulfinylalkyl, Alkyl-$S(O)_2$-, Alkyl-$S(O)_2$-$(CR_{41}R_{42})_p$=$C(R_{41})$-, Alkyl-$S(O)_2$-$(CR_{41}R_{42})_p$-, Alkyl-S-, Alkyl-S-$(CR_{41}R_{42})_p$-, Alkinyl, Carboxy, Carboxyalkyl, Cyano, Cyanoalkyl, Formyl, Formylalkyl, Halogen, Haloalkyl, Haloalkoxy, Hydroxy, Hydroxyalkyl, Oxo, -SH, $N(O)_2$, $-C(R_{41})=N-O(R_{42})$, $-(CR_{41}R_{42})_p-C(R_{41})=N-O(R_{42})$, =N-O(Alkyl), =N-OH, $NZ_1Z_2-(CR_{41}R_{42})_p-O-$, $-O-(CR_{41}R_{42})_p-G_1$, $G_1$, $-NZ_1Z_2$, $-(CR_{41}R_{42})_p-NZ_1Z_2$ und $(NZ_3Z_4)$Carbonyl;

$G_1$ ist ein 4-, 5-, 6- oder 7-gliedriger monozyklischer Heterozyklus, enthaltend ein Stickstoffatom und wahlweise 1 oder 2 zusätzliche Heteroatome in dem Ring, worin der Ring gebunden ist an den Stammanteil durch das Stickstoffatom, und der Ring ist wahlweise substituiert mit 1, 2, 3, 4 oder 5 Substituenten gewählt aus der Gruppe bestehend aus Alkyl, Alkenyl, Alkinyl, Halo, Haloalkyl, =N-CN, $=N-OR_{51}$, -CN, Oxo, $-OR_{51}$, $-OC(O)R_{51}$, $-OC(O)N(R_{51})_2$, $-S(O)_2R_{52}$, $-S(O)_2N(R_{51})_2$, $-C(O)\ R_{51}$, $-C(O)OR_{51}$, $-C(O)N(R_{51})_2$, $-N(R_{51})_2$, $-N(R_{51})C(O)R_{51}$, $-N(R_{51})\ S(O)_2R_{52}$, $-N(R_{51})C(O)O(R_{52})$, $-N\ (R_{51})\ C\ (O)\ N\ (R_{51})_2$, $-\ (CR_{1c}R_{1d})_q-OR_{51}$, $-\ (CR_{1c}R_{1d})_q-OC(O)R_{51}$, $-\ (CR_{1c}R_{1d})_q-OC(O)N(R_{51})_2$, $-\ (CR_{1c}R_{1d})_q-S(O)_2R_{52}$, $-\ (CR_{1c}R_{1d})_q-S(O)_2N(R_{51})_2$, $-(CR_{1c}R_{1d})_q-C(O)R_{51}$, $-(CR_{1c}R_{1d})_q-C(O)OR_{51}$, $-(CR_{1c}R_{1d})_q-C(O)N(R_{51})_2$, $-(CR_{1c}R_{1d})_q-N(R_{51})_2$, $-(CR_{1c}R_{1d})_q-N(R_{51})C(O)R_{51}$, $-(CR_{1c}R_{1d})_q-N(R_{51})S(O)_2R_{52}$, $-(CR_{1c}R_{1d})_q-N(R_{51})C(O)O(R_{52})$, $-(CR_{1c}R_{1d})_q-N(R_{51})C(O)N(R_{51})_2$ und $-(CR_{1c}R_{1d})_q-CN$;

$R_{51}$ ist, jedesmal wenn es vorkommt, unabhängig Wasserstoff, $C_1$-$C_4$ Alkyl, $C_1$-$C_4$ Haloalkyl, $-\ (CR_{2c}R_{2d})_n-OR^{53}$, monozyklisches Cycloalkyl oder $-\ (CR_{2c}R_{2d})_a-$ (monozyklisches Cycloalkyl); worin $R_{53}$ Wasserstoff ist, $C_1$-$C_4$ Alkyl, $C_1$-$C_4$ Haloalkyl, monozyklisches Cycloalkyl oder $-\ (CR_{2c}R_{2d})_u-$(monzyklisches Cycloalkyl);

$R_{52}$ ist, jedesmal wenn es vorkommt, unabhängig $C_1$-$C_4$ Alkyl, $C_1$-$C_4$ Haloalkyl, monozyklisches Cycloalkyl oder $-(CR_{2c}R_{2d})_a-$(monozyklisches Cycloalkyl);

der monozyklische Cycloalkylanteil ist, als ein Substituent oder als Teil von einem Substituenten, wie dargestellt durch $R_{51}$, $R_{52}$ und $R_{53}$, jeweils unabhängig unsubstituiert oder substituiert mit 1, 2, 3 oder 4 Substituenten gewählt aus der Gruppe bestehend aus $C_1$-$C_4$ Alkyl, Halo, Hydroxy, $C_1$-$C_4$ Alkoxy, $C_1$-$C_4$ Haloalkoxy und $C_1$-$C_4$ Haloalkyl;

$R_{21}$, $R_{22}$, $R_{31}$, $R_{32}$, $R_{41}$, $R_{42}$, $R_{1c}$, $R_{1d}$, $R_{2c}$ und $R_{2d}$ sind, jedesmal wenn sie vorkommen, jeweils unabhängig Wasserstoff, Alkyl, Haloalkyl oder Halo;

m, p, q und u sind, jedesmal wenn sie vorkommen, jeweils unabhängig 1, 2, 3 oder 4;

$Z_1$ und $Z_2$ sind jeweils unabhängig Wasserstoff, Alkyl, Alkoxyalkyl, Alkylcarbonyl, Cyanoalkyl, Haloalkyl oder Formyl; und

$Z_3$ und $Z_4$ sind jeweils unabhängig Wasserstoff, Alkyl, Haloalkyl, Phenyl oder Benzyl, worin der Phenylanteil wahlweise substituiert ist mit 1, 2, 3 oder 4 Substituenten gewählt aus der Gruppe bestehend aus Alkyl, Hydroxyl und Haloalkyl.

**2.** Die Verbindung gemäß Anspruch 1 odder ein pharmazeutisch verträgliches Salz davon, worin
$R_2$ Wasserstoff, Alkoxycarbonyl, Alkoxycarbonylalkyl, Alkyl, Aryl, Halo, Haloalkyl oder $-(CR_{21}R_{22})_m-OH$ ist;
$R_3$ ist Wasserstoff, Alkyl, Alkylcarbonyl, Aryl, Cycloalkyl, Halo, Haloalkyl, Heterozyklus oder $-(CR_{31}R_{32})_m-OH$;
$L_1$ ist ein Bindung; und
$R_4$ ist Aryl, Heteroaryl, Cycloalkyl, Heterozyklus, Arylalkyl, Heteroarylalkyl, Heterozyklusalkyl, Cycloalkylalkyl, $R_{10}$-$L_2$-$R_9$-, Alkinyl oder Alkyl; worin das Alkyl wahlweise substituiert ist mit einem Substituenten gewählt aus der Gruppe bestehend aus Alkoxycarbonyl, -OH oder $R_eR_fN$-.

**3.** Die Verbindung gemäß Anspruch 2 oder ein pharmazeutisch verträgliches Salz davon, worin
$R_4$ wahlweise substituiertes Phenyl oder $R_{10}$-$L_2$-$R_9$- ist;
$L_2$ ist Alkylen oder O;
$R_9$ ist wahlweise substituiertes Aryl.

**4.** Die Verbindung gemäß Anspruch 1 oder ein pharmazeutisch verträgliches Salz davon, worin
$R_2$ Wasserstoff oder Alkyl ist;
$R_3$ ist Alkyl oder $-(CR_{31}R_{32})_m-OH$;
$L_1$ ist eine Bindung;
$R_4$ ist wahlweise substituiertes Phenyl oder $R_{10}$-$L_2$-$R_9$-;
$L_2$ ist O; und
$R_9$ ist wahlweise substituiertes Aryl.

**5.** Die Verbindung gemäß Anspruch 1 oder ein pharmazeutisch verträgliches Salz davon, worin
$R_2$ Wasserstoff, Alkoxycarbonyl, Alkoxycarbonylalkyl, Alkyl, Aryl, Halo, Haloalkyl oder $-\ (CR_{21}R_{22})_m-OH$ ist;
$R_3$ ist Wasserstoff, Alkyl, Alkylcarbonyl, Aryl, Cycloalkyl, Halo, Haloalkyl, Heterozyklus oder $-(CR_{31}R_{32})_m-OH$;

$L_1$ ist NRg; und
$R_4$ ist Arylalkyl, Cycloalkyl, Cycloalkylalkyl oder Alkyl; worin das Alkyl wahlweise substituiert ist mit einem Substituenten gewählt aus der Gruppe bestehend aus Alkoxycarbonyl, -OH oder $R_eR_fN$-.

**6.** Die Verbindung gemäß Anspruch 1 oder ein pharmazeutisch verträgliches Salz davon, worin $R_2$ und $R_3$ zusammengenommen mit den Kohlenstoffatomen, an welche sie gebunden sind, einen 4-, 5-, 6- oder 7-gliedrigen monozyklischen Ring bilden, wahlweise ankondensiert an Benzo oder Oxadiazol, der monozyklische Ring enthält null odder eine zusätzliche Doppelbindung, null Sauerstoffatome und null Stickstoffatome als Ringatome; zwei nicht angrenzende Atome von monozyklischem Ring sind verknüpft durch eine Alkenylenbrücke von 2, 3 oder 4 Kohlenstoffatomen oder sind verknüpft durch eine Alkylenbrücke von 1, 2, 3 oder 4 Kohlenstoffatomen, der monozyklische Ring ist unabhängig unsubstituiert oder substituiert mit 1, 2, 3, 4 oder 5 Substituenten unabhängig gewählt aus der Gruppe bestehend aus Oxo, Alkyl, Halo, -OH, -O(Alkyl) und Haloalkyl; zwei Substituenten auf demselben Kohlenstoffatom des monozyklischen Rings bilden zusammengenommen mit dem Kohlenstoffatom, an welches sie gebunden sind, wahlweise einen 3-, 4-, 5- oder 6-gliedrigen monozyklischen Cycloalkylring, worin der monozyklische Cycloalkylring wahlweise substituiert ist mit 1, 2, 3, 4, 5 oder 6 Substituenten unabhängig gewählt aus der Gruppe bestehend aus Alkyl und Haloalkyl.

**7.** Die Verbindung gemäß Anspruch 1 oder ein pharmazeutisch verträgliches Salz davon, worin $R_2$ und $R_3$ zusammengenommen mit den Kohlenstoffatomen, an welche sie gebunden sind, einen wahlweise substituierten Ring bilden, dargestellt durch

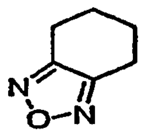

(x)

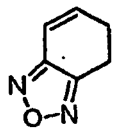

(xa)

oder

**8.** Die Verbindung gemäß Anspruch 1 oder ein pharmazeutisch verträgliches Salz davon, worin $R_2$ und $R_3$ zusammengenommen mit den Kohlenstoffatomen, an welche sie gebunden sind, einen 4-, 5-, 6- oder 7-gliedrigen Ring bilden, wahlweise ankondensiert an Benzo oder Oxadiazol, der monozyklische Ring enthält null oder eine zusätzliche Doppelbindung und ein Sauerstoffatom und null oder ein Stickstoffatom als Ringatome; zwei nicht angrenzende Atome von dem monozyklischen Ring können wahlweise verknüpft sein durch eine Alkenylenbrücke von 2, 3 oder 4 Kohlenstoffatomen, oder wahlweise verknüpft sein durch eine Alkylenbrücke von 1, 2, 3 oder 4 Kohlenstoffatomen, der monozyklische Ring ist unabhängig unsubstituiert oder substituiert mit 1, 2, 3, 4 oder 5 Substituenten unabhängig gewählt aus der Gruppe bestehend aus Oxo, Alkyl, Halo, -OH, -O(Alkyl) und Haloalkyl; zwei Substituenten auf demselben Kohlenstoffatom des monozyklischen Rings bilden, zusammengenommen mit dem Kohlenstoffatom, an welches sie gebunden sind, wahlweise einen 3-, 4-, 5- oder 6-gliedrigen monozyklischen Cycloalkylring, worin der monozyklische Cycloalkylring wahlweise substituiert ist mit 1, 2, 3, 4, 5 oder 6 Substituenten unabhängig gewählt aus der Gruppe bestehend aus Alkyl und Haloalkyl.

**9.** Die Verbindung gemäß Ansprüchen 6 oder 8, oder ein pharmazeutisch verträgliches Salz davon, worin
$R_4$ Aryl, Heteroaryl, Cycloalkyl, Heterozyklus, Arylalkyl, Heteroarylalkyl, Heterozyklusalkyl, Cycloalkylalkyl, $R_{10}$-$L_2$-$R_9$-, Alkinyl oder Alkyl ist; worin das Alkyl wahlweise substituiert ist mit einem Substituenten gewählt aus der Gruppe bestehend aus Alkoxycarbonyl, -OH oder $R_eR_fN$-.

**10.** Die Verbindung gemäß Anspruch 1 oder ein pharmazeutisch verträgliches Salz davon, worin
$R_2$ und $R_3$ zusammengenommen mit den Kohlenstoffatomen, an welche sie gebunden sind, einen Ring bilden, dargestellt durch

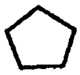

(viii)

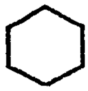

(ix)

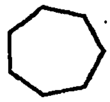

(xi)

**11.** Die Verbindung gemäß Anspruch 1 gewählt aus der Gruppe bestehend aus
N-[(2Z)-5-tert-Butyl-3-(1,3-oxazol-2-ylmethyl)-1,3-thiazol-2(3H)-yliden]-5-chlor-2-methoxybenzamid;
N-[(2Z)-5-tert-Butyl-3-(1,2,4-oxadiazol-3-ylmethyl)-1,3-thiazol-2(3H)-yliden]-5-chlor-2-methoxybenzamid;
N-[(2Z)-5-tert-Butyl-3-(2-furylmethyl)-1,3-thiazol-2(3H)-yliden]-5-chlor-2-methoxybenzamid;
5-Chlor-N-[(2Z)-4,5-dimethyl-3-[(3-methylisoxazol-5-yl)methyl]-1,3-thiazol-2(3H)-yliden]-2-methoxybenzamid;
5-Chlor-2-methoxy-N-[(2Z)-5-methyl-3-{[5-(trifluormethyl)-2-furyl]methyl}-1,3-thiazol-2(3H)-yliden]benzamid;
5-Chlor-N-[(2Z)-3-(2-furylmethyl)-5-methyl-1,3-thiazol-2(3H)-yliden]-2-methoxybenzamid;
5-Chlor-N-[(2Z)-3-(3-furylmethyl)-5-methyl-1,3-thiazol-2(3H)-yliden]-2-methoxybenzamid; und
N-[(2Z)-5-tert-Butyl-3-[(5-methylisoxazol-3-yl)methyl]-1,3-thiazol-2(3H)-yliden]-5-chlor-2-methoxybenzamid;
oder ein pharmazeutisch verträgliches Salz davon.

**12.** Eine pharmazeutische Zusammensetzung, umfassend eine therapeutisch wirksame Menge einer Verbindung gemäß Anspruch 1, welche die Formel (I) hat, oder ein pharmazeutisch verträgliches Salz davon, in Kombination mit einem pharmazeutisch verträglichen Träger.

**13.** Eine Verbindung gemäß Anspruch 1, welche die Formel (I) hat, oder ein pharmazeutisch verträgliches Salz davon, für die Verwendung in der Behandlung von neuropathischen Schmerzen, nozizeptiven Schmerzen und Entzündungsschmerzen in einem Säugetier, das eine solche Behandlung benötigt, durch Verabreichen einer therapeutisch wirksamen Menge der Verbindung an das Säugetier.

## Revendications

**1.** Composé selon la formule (I),

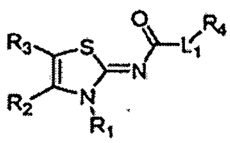

(I),

ou sel pharmaceutiquement acceptable de celui-ci, où
$R_1$ est A ou A-alkylène ;
$R_2$ est l'hydrogène, alcoxy, alcoxyalkyle, alcoxycarbonyle, alcoxycabonylalkyle, alkyle, alkyl-$S(O)_2$-, aryle, arylalkyle, arylalcényle, azidoalkyle, cyano, cycloalkyle, halo, haloalkyle, hétéroaryle, hétérocycle, $-(CR_{21}R_{22})_m$-OH, $R_aR_bN$-, $R_aR_bN$-alkyle, $R_cR_dNC(O)$-, ou $R_8$-$R_7$- ;
$R_3$ est l'hydrogène, alcoxy, alcoxyalkyle, alkyle, alkylcarbonyle, alkyl-$S(O)_2$-, aryle, arylalkyle, arylalcényle, cyano, cycloalkyle, halo, haloalkyle, hétéroaryle, hétérocycle, $-(CR_{31}R_{32})_m$-OH, $R_aR_bN$-, $R_aR_bN$-alkyl-, ou $R_8$-$R_7$- ; ou
$R_2$ et $R_3$, avec les atomes de carbone auxquels ils sont liés, forment un cycle monocyclique à 4, 5, 6 ou 7 chaînons, éventuellement condensé à benzo ou oxadiazole, ledit cycle monocyclique contient zéro ou une double liaison supplémentaire, zéro ou un atome d'oxygène, et zéro ou un atome d'azote comme atomes du cycle ; deux atomes non adjacents dudit cycle monocyclique sont éventuellement liés par un pont alcénylène de 2, 3 ou 4 atomes de carbone, ou éventuellement liés par un pont alkylène de 1, 2, 3 ou 4 atomes de carbone, ledit cycle monocyclique est indépendamment non substitué ou substitué avec 1, 2, 3, 4 ou 5 substituants choisis indépendamment dans le groupe consistant en oxo, alkyle, halo, -OH, -O(alkyle), et haloalkyle ; deux substituants sur le même atome de

carbone dudit cycle monocyclique, avec l'atome de carbone auxquels ils sont liés, forment éventuellement un cycle cycloalkyle monocyclique à 3, 4, 5 ou 6 chaînons, où le cycle cycloalkyle monocyclique est éventuellement substitué avec 1, 2, 3, 4, 5 ou 6 substituants choisis indépendamment dans le groupe consistant en alkyle et haloalkyle ;

$R_4$ est alkyle, alcynyle, cycloalkyle, cycloalcényle, aryle, hétéroaryle, hétérocycle, cycloalkylalkyle, cycloalcénylalkyle, arylalkyle, hétéroarylalkyle, hétérocyclealkyle, ou $R_{10}$-$L_2$-$R_9$- ; où le groupe alkyle est éventuellement substitué avec un substituant choisi dans le groupe consistant en alcoxy, alcoxycarbonyle, carboxy, halo, -OH, et $R_eR_fN$- ;

$R_7$, $R_8$ et $R_9$, sont chacun indépendamment aryle, cycloalkyle, cycloalcényle, hétéroaryle ou hétérocycle ;

$R_{10}$ est aryle, cycloalkyle, cycloalcényle, hétéroaryle, hétérocycle, ou cycloalkylalkyle ;

$R_a$ et $R_b$, à chaque occurrence, sont chacun indépendamment l'hydrogène, alcoxycarbonyle, alkyle, alkylcarbonyle, alkyl-S-$(O)_2$-, ou arylalkyle ;

$R_c$ et $R_d$, sont chacun indépendamment l'hydrogène ou alkyle ;

$R_e$ et $R_f$, sont chacun indépendamment l'hydrogène, alkyle ou alkylcarbonyle ;

A est furanyle, oxazolyle, isoxazolyle, ou oxadiazolyle ; chaque cycle A est éventuellement condensé avec un cycle monocyclique choisi dans le groupe consistant en benzo, cycloalkyle, cycloalcényle, hétérocycle et hétéroaryle ; et chaque A est indépendamment non substitué ou substitué avec 1, 2, 3, 4, 5 ou 6 substituants choisis indépendamment dans le groupe consistant en oxo, alkyle, halo, -OH, -O(alkyle), et haloalkyle ;

$L_1$ est une simple liaison ou -$NR_g$- ;

$L_2$ est une simple liaison, alkylène, ou -O-;

$R_g$ est l'hydrogène ou alkyle;

les groupements aryle, cycloalkyle, cycloalcényle, hétérocycle ou hétéroaryle, comme substituant, ou comme partie d'un substituant, tels que représentés par $R_2$, $R_3$, $R_4$, $R_a$, $R_b$, $R_7$, $R_8$, $R_9$ et $R_{10}$, sont chacun indépendamment non substitué ou substitué avec 1, 2, 3, 4, 5 ou 6 substituants choisis indépendamment dans le groupe consistant en alcényle, alcoxy, alcoxyalcoxy, alcoxyalcoxyalkyle, alcoxyalkyle, alcoxycarbonyle, alcoxycarbonylalkyle, alcoxysulfonyle, alkyle, alkylcarbonyle, alkylcarbonylalkyle, alkylcarbonyloxy, alkylsulfinyle, alkylsulfinylalkyle, alkyl-S$(O)_2$-, alkyl-S$(O)_2$-$(CR_{41}R_{42})_p$=C($R_{41}$)-, alkyl-S$(O)_2$-$(CR_{41}R_{42})_p$-, alkyl-S-, alkyl-S-$(CR_{41}R_{42})_p$-, alcynyle, carboxy, carboxyalkyle, cyano, cyanoalkyle, formyle, formylalkyle, halogène, haloalkyle, haloalcoxy, hydroxy, hydroxyalkyle, oxo, -SH, $N(O)_2$, -C($R_{41}$)=N-O($R_{42}$), -$(CR_{41}R_{42})_p$-C($R_{41}$)=N-O($R_{42}$), =N-O(alkyle), =N-OH, $NZ_1Z_2$-$(CR_{41}R_{42})_p$-O-, -O-$(CR_{41}R_{42})_p$-$G_1$, $G_1$, -$NZ_1Z_2$, -$(CR_{41}R_{42})_p$-$NZ_1Z_2$, et ($NZ_3Z_4$)carbonyle ;

$G_1$ est un hétérocycle monocyclique à 4, 5, 6 ou 7 chaînons contenant un atome d'azote et éventuellement 1 ou 2 hétéroatomes supplémentaires dans le cycle, où ledit cycle est lié au groupement mère par le biais de l'atome d'azote, et ledit cycle est éventuellement substitué avec 1, 2, 3, 4 ou 5 substituants choisis dans le groupe consistant en alkyle, alcényle, alcynyle, halo, haloalkyle, =N-CN, =N-$OR_{51}$, -CN, oxo, -$OR_{51}$, -OC(O)$R_{51}$, -OC(O)N$(R_{51})_2$, -S$(O)_2R_{52}$, -S$(O)_2$N$(R_{51})_2$, -C(O)$R_{51}$, -C(O)O$R_{51}$, -C(O)N$(R_{51})_2$, -$(R_{51})_2$, -N($R_{51}$)C(O)$R_{51}$, -N($R_{51}$)S$(O)_2R_{52}$, -N($R_{51}$)C(O)O($R_{52}$), -N($R_{51}$)C(O)N$(R_{51})_2$, -$(CR_{1c}R_{1d})_q$-$OR_{51}$, -$(CR_{1c}R_{1d})_q$-OC(O)$R_{51}$, -$(CR_{1c}R_{1d})_q$-OC(O)N$(R_{51})_2$, -$CR_{1c}R_{1d})_q$-S$(O)_2R_{52}$, -$(CR_{1c}R_{1d})_q$-S$(O)_2$N$(R_{51})_2$, -$(CR_{1c}R_{1d})_q$-C(O)$R_{51}$, -$(CR_{1c}R_{1d})_q$-C(O)$R_{51}$, -$(CR_{1c}R_{1d})_q$-C(O)N$(R_{51})_2$, -$(CR_{1c}R_{1d})_q$-N$(R_{51})_2$, -$(CR_{1c}R_{1d})_q$-N($R_{51}$)C(O)$R_{51}$, -$CR_{1c}R_{1d})_q$-N-$R_{51}$)S$(O)_2R_{52}$, -$(CR_{1c}R_{1d})_q$-N($R_{51}$)C(O)O($R_{52}$), -$CR_{1c}R_{1d})_q$-N($R_{51}$)C(O)N$(R_{51})_2$, et -$(CR_{1c}R_{1d})_q$-CN ;

$R_{51}$, à chaque occurrence, est indépendamment l'hydrogène, alkyle en $C_1$-$C_4$, haloalkyle en $C_1$-$C_4$, -$(CR_{2c}R_{2d})_u$-$OR_{53}$, cycloalkyle monocyclique, ou -$(CR_{2c}R_{2d})_u$-(cycloalkyle monocyclique) ; où $R_{53}$ est l'hydrogène, alkyle en $C_1$-$C_4$, haloalkyle en $C_1$-$C_4$, cycloalkyle monocyclique, -$(CR_{2c}R_{2d})_u$-(cycloalkyle monocyclique) ;

$R_{52}$, à chaque occurrence, est indépendamment alkyle en $C_1$-$C_4$, haloalkyle en $C_1$-$C_4$, cycloalkyle monocyclique, ou -$(CR_{2c}R_{2d})_u$-(cycloalkyle monocyclique) ;

le groupement cycloalkyle monocyclique, comme substituant, ou comme partie d'un substituant, tel qu'il est représenté par $R_{51}$, $R_{52}$ et $R_{53}$ sont chacun indépendamment non substitué ou substitué avec 1, 2, 3, ou 4 substituants choisis dans le groupe consistant en alkyle en $C_1$-$C_4$, halo, hydroxy, alcoxy en $C_1$-$C_4$, haloalcoxy en $C_1$-$C_4$, et haloalkyle en $C_1$-$C_4$;

$R_{21}$, $R_{22}$, $R_{31}$, $R_{32}$, $R_{41}$, $R_{42}$, $R_{1c}$, $R_{1d}$, $R_{2c}$ et $R_{2d}$, à chaque occurrence sont chacun indépendamment l'hydrogène, alkyle, haloalkyle ou halo;

m, p, q et u, à chaque occurrence, sont chacun indépendamment 1, 2, 3 ou 4 ;

$Z_1$ et $Z_2$ sont chacun indépendamment l'hydrogène, alkyle, alcoxyalkyle, alkylcarbonyle, cyanoalkyle, haloalkyle ou formyle; et

$Z_3$ et $Z_4$ sont chacun indépendamment l'hydrogène, alkyle, haloalkyle, phényle ou benzyle, où le groupement phényle est éventuellement substitué avec 1, 2, 3 ou 4 substituants choisis dans le groupe consistant en alkyle, hydroxyle et haloalkyle.

**2.** Composé selon la revendication 1, ou sel pharmaceutiquement acceptable de celui-ci, où

$R_2$ est l'hydrogène, alcoxycarbonyle, alcoxycabonylalkyle, alkyle, aryle, halo, haloalkyle, ou -$(CR_{21}R_{22})_m$-OH;

$R_3$ est l'hydrogène, alkyle, alkylcarbonyle, aryle, cycloalkyle, halo, haloalkyle, hétérocycle, ou -$(CR_{31}R_{32})_m$-OH ;

$L_1$ est une liaison ; et
$R_4$ est aryle, hétéroaryle, cycloalkyle, hétérocycle, aralkyle, hétéroarylalkyle, hétérocyclealkyle, cycloalkylalkyle, $R_{10}$-$L_2$-$R_9$-, alcynyle, ou alkyle; où l'alkyle est éventuellement substitué avec un substituant choisi dans le groupe consistant en alcoxycarbonyle, -OH, ou $R_eR_fN$-.

**3.** Composé selon la revendication 2, ou sel pharmaceutiquement acceptable de celui-ci, où
$R_4$ est phényle éventuellement substitué ou $R_{10}$-$L_2$-$R_9$-,
$L_2$ est alkylène ou O ;
$R_9$ est aryle éventuellement substitué.

**4.** Composé selon la revendication 1, ou sel pharmaceutiquement acceptable de celui-ci, où
$R_2$ est l'hydrogène ou alkyle;
$R_3$ est alkyle ou -$(CR_{31}R_{32})_m$-OH ;
$L_1$ est une liaison ;
$R_4$ est phényle éventuellement substitué ou $R_{10}$-$L_2$-$R_9$- ;
$L_2$ est O ; et
$R_9$ est aryle éventuellement substitué.

**5.** Composé selon la revendication 1, ou sel pharmaceutiquement acceptable de celui-ci, où
$R_2$ est l'hydrogène, alcoxycarbonyle, alcoxycarbonylalkyle, alkyle, aryle, halo, haloalkyle, ou -$(CR_{21}R_{22})_m$-OH ;
$R_3$ est l'hydrogène, alkyle, alkylcarbonyle, aryle, cycloalkyle, halo, haloalkyle, hétérocycle, ou -$(CR_{31}R_{32})_m$-OH ;
$L_1$ est un $NR_g$ ; et
$R_4$ est arylalkyle, cycloalkyle, cycloalkylalkyle, ou alkyle ; où l'alkyle est éventuellement substitué avec un substituant choisi dans le groupe consistant en alcoxycarbonyle, -OH ou $R_eR_fN$.

**6.** Composé selon la revendication 1, ou sel pharmaceutiquement acceptable de celui-ci, où $R_2$ et $R_3$, avec les atomes de carbone auxquels ils sont liés, forment un cycle monocyclique à 4, 5, 6 ou 7 chaînons, éventuellement condensé à benzo ou oxadiazole, ledit cycle monocyclique contient zéro ou une double liaison supplémentaire, zéro atome d'oxygène et zéro atome d'azote comme atomes du cycle ; deux atomes non adjacents dudit cycle monocyclique sont liés par un pont alcénylène de 2, 3 ou 4 atomes de carbone, ou liés par un pont alkylène de 1, 2, 3 ou 4 atomes de carbone, ledit cycle monocyclique est indépendamment non substitué ou substitué avec 1, 2, 3, 4 ou 5 substituants choisis indépendamment dans le groupe consistant en oxo, alkyle, halo, -OH, -O(alkyle) et haloalkyle ; deux substituants sur le même atome de carbone dudit cycle monocyclique, avec l'atome de carbone auxquels ils sont liés, forment éventuellement un cycle cycloalkyle monocyclique à 3, 4, 5 ou 6 chaînons, où le cycle cycloalkyle monocyclique est éventuellement substitué avec 1, 2, 3, 4, 5 ou 6 substituants choisis indépendamment dans le groupe consistant en alkyle et haloalkyle.

**7.** Composé selon la revendication 1, où sel pharmaceutiquement acceptable de celui-ci, où $R_2$ et $R_3$, avec les atomes de carbone auxquels ils sont liés, forment un cycle éventuellement substitué représenté par

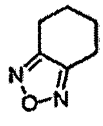
(x)
ou
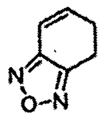
(xa)

**8.** Composé selon la revendication 1, ou sel pharmaceutiquement acceptable de celui-ci,
où $R_2$ et $R_3$, avec les atomes de carbone auxquels ils sont liés, forment un cycle monocyclique à 4, 5, 6 ou 7 chaînons éventuellement condensé à benzo ou oxadiazole, ledit cycle monocyclique contient zéro ou une double liaison supplémentaire, et un atome d'oxygène et zéro ou un atome d'azote comme atomes du cycle ; deux atomes non adjacents dudit cycle monocyclique peuvent être éventuellement liés par un pont alcénylène de 2, 3 ou 4 atomes de carbone, ou éventuellement liés par un pont alkylène de 1, 2, 3 ou 4 atomes de carbone, ledit cycle monocyclique est indépendamment non substitué ou substitué avec 1, 2, 3, 4 ou 5 substituants choisis indépendamment dans le groupe consistant en oxo, alkyle, halo, -OH, -O(alkyle), et haloalkyle ; deux substituants sur le même atome de carbone dudit cycle monocyclique, avec l'atome de carbone auxquels ils sont liés, forment éventuellement un cycle cycloalkyle monocyclique à 3, 4, 5 ou 6 chaînons, où le cycle cycloalkyle monocyclique est éventuellement substitué

avec 1, 2, 3, 4, 5 ou 6 substituants choisis indépendamment dans le groupe consistant en alkyle et haloalkyle.

**9.** Composé selon la revendication 6 ou 8, ou sel pharmaceutiquement acceptable de celui-ci, où $R_4$ est aryle, hétéroaryle, cycloalkyle, hétérocycle, arylalkyle, hétéroarylalkyle, hétérocyclealkyle, cycloalkylalkyle, $R_{10}$-$L_2$-$R_9$-, alcynyle, ou alkyle; où l'alkyle est éventuellement substitué avec un substituant choisi dans le groupe consistant en alcoxycarbonyle, -OH ou $R_eR_fN$-.

**10.** Composé selon la revendication 1, ou sel pharmaceutiquement acceptable de celui-ci, où $R_2$ et $R_3$, avec les atomes de carbone auxquels ils sont liés, forment un cycle tel que représenté par

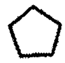
(viii)

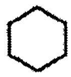
(ix)

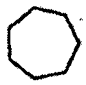
(xi)

**11.** Composé selon la revendication 1 choisi dans le groupe consistant en
N-[(2Z)-5-tert-butyl-3-(1,3-oxazol-2-ylméthyl)-1,3-thiazol-2(3H)-ylidène]-5-chloro-2-méthoxybenzamide ;
N-[(2Z)-5-tert-butyl-3-(1,2,4-oxadiazol-3-ylméthyl)-1,3-thiazol-2(3H)-ylidène]-5-chloro-2-méthoxybenzamide ;
N-[(2Z)-5-tert-butyl-3-(2-furylméthyl)-1,3-thiazol-2(3H)-ylidène]-5-chloro-2-méthoxybenzamide ;
5-chloro-N-[(2Z)-4,5-diméthyl-3-[(3-méthylisoxazol-5-yl)-méthyl]-1,3-thiazol-2(3H)-ylidène]-2-méthoxybenzamide ;
5-chloro-2-méthoxy-N-[(2Z)-5-méthyl-3-{[5-(trifluorométhyl)-2-furyl]méthyl}-1,3-thiazol-2(3H)-ylidène]benzamide ;
5-chloro-N-[(2Z)-3-(2-furylméthyl)-5-méthyl-1,3-thiazol-2(3H)-ylidène]-2-méthoxybenzamide ;
5-chloro-N-[(2Z)-3-(3-furylméthyl)-5-méthyl-1,3-thiazol-2(3H)-ylidène]-2-méthoxybenzamide ; et
N-[(2Z)-5-tert-butyl-3-[(5-méthylisoxazol-3-yl)méthyl]-1,3-thiazol-2(3H)-ylidène]-5-chloro-2-méthoxybenzamide ;
ou sel pharmaceutiquement acceptable de celui-ci.

**12.** Composition pharmaceutique comprenant une quantité thérapeutiquement efficace d'un composé selon la revendication 1 ayant la formule (I) ou d'un sel pharmaceutiquement acceptable de celui-ci, en combinaison avec un vecteur pharmaceutiquement acceptable.

**13.** Composé selon la revendication 1 ayant la formule (I) ou sel pharmaceutiquement acceptable de celui-ci destiné à être utilisé dans le traitement de la douleur neuropathique, de la douleur nociceptive et de la douleur inflammatoire chez un mammifère ayant besoin d'un tel traitement par administration au mammifère d'une quantité thérapeutiquement efficace dudit composé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2004077617 A, Bennani, Y.L.
- EP 412404 A, Matsuo, Masaaki; Ogino, Takashi; Igari, Norihiro; Seno, Hachiro; Shimomura, Kyoichi.
- JP 06345736 B


### Non-patent literature cited in the description


- N-Alkylidenearylcarboxamides as new potent and selective CB2 cannabinoid receptor agonists with good oral bioavailability. **OHTA et al.** Bioorganic and Medicinal Chemistry Letters. Pergamon, Elsevier Science, GB, 12 October 2007, vol. 17, 6299-6304
- **T.W. GREENE ; P.G.M. WUTS.** Protective Groups in Organic Synthesis. John Wiley & Sons, 1999
- IUPAC 1974 Recommendations for Section E, Fundamental Stereochemistry. *Pure Appl. Chem.,* 1976, vol. 45, 13-30
- **BRENNAN et al.** *Pain,* 1996, vol. 64, 493
- **CHAPLAN, S.R. ; F.W. BACH ; J.W. POGREL ; J.M. CHUNG ; T.L. YAKSH.** Quantitative Assessment of Tactile Allodynia in the Rat Paw. *J. Neurosci. Methods,* 1994, vol. 53, 55
- **DIXON, W.J.** Efficient Analysis of Experimental Observations. *Ann. Rev. Pharmacol. Toxicol.,* 1980, vol. 20, 441
- **HARGREAVES.** *Pain,* 1988, vol. 32, 77
- **KIM, S.H. ; J.M. CHUNG.** An experimental model for peripheral neuropathy produced by segmental spinal nerve ligation in the rat. *Pain,* 1992, vol. 50, 355
- **CHAPLAN, S.R. ; F.W. BACH ; J.W. PORGREL ; J.M. CHUNG ; T.L. YAKSH.** Quantitative assessment of tactile allodynia in the rat paw. *J. Neurosci. Methods,* 1994, vol. 53, 55
- **DIXON, W.J.** Efficient analysis of experimental observations. *Ann. Rev. Pharmacol. Toxicol.,* 1980, vol. 20, 441
- **JOSHI et al.** *Neuroscience,* 2006, vol. 143, 587-596
- **HANUS, L. et al.** *Proc. Nat. Acad. Sci.,* 1999, vol. 96, 14228-14233
- **MALAN, T. P. et al.** *Pain,* 2001, vol. 93, 239-245
- **IBRAHIM, M. M. et al.** *Proc. Nat. Acad. Sci.,* 2005, vol. 102 (8), 3093-3098
- **HOHMANN, A. G. et al.** *J. Pharmacol. Exp. Ther.,* 2004, vol. 308, 446-453
- **NACKLEY, A. G. et al.** *Neuroscience,* 2003, vol. 119, 747-757
- **QUARTILHO, A. et al.** *Anesthesiology,* 2003, vol. 99, 955-60
- **IBRAHIM, M. M. et al.** *Proc. Nat. Acad. Sci.,* 2003, vol. 100, 10529-10533
- **VALENZANO, K. J. et al.** *Neuropharmacology,* 2005, vol. 48, 658-672
- **CLAYTON, N. et al.** *Pain,* 2002, vol. 96, 253-260
- **CICHEWICZ, D. L.** *Life Sci.,* 2004, vol. 74, 1317-1324
- **GALIEGUE et al.** *Eur. J. Biochem.,* 1995, vol. 232, 54-61
- **BUCKLEY, N. E. et al.** *Eur. J. Pharmacol.,* 2000, vol. 396, 141-149
- **CARLISLE, S. J. et al.** *Int. Immunopharmacol.,* 2002, vol. 2, 69
- **CARRIER, E. J. et al.** *Current Drug Targets - CNS & Neurological Disorders,* 2005, vol. 4, 657-665
- **PERTWEE, R. G.** *Pharmacol. Ther.,* 2002, vol. 95, 165-174
- **MARESZ, K. et al.** *J. Neurochem,* 2005, vol. 95, 437-445
- **NI, X. et al.** *Multiple Sclerosis,* 2004, vol. 10, 158-164
- **AREVALO-MARTIN, A. et al.** *J. Neurosci.,* 2003, vol. 23 (7), 2511-2516
- **BENITO, C. et al.** *J. Neurosci.,* 2003, vol. 23 (35), 11136-11141
- **RAMIREZ, B. G. et al.** *J. Neurosci.,* 2005, vol. 25 (8), 1904-1913
- **WRIGHT, K. et al.** *Gastroenterology,* 2005, vol. 129, 437-453
- **MATHISON, R. et al.** *Br. J. Pharmacol.,* 2004, vol. 142, 1247-1254
- **IHENETU, K. et al.** *Eur. J. Pharmacol.,* 2003, vol. 458, 207-215

- **WARHURST, A. C. et al.** *Gut,* 1998, vol. 42, 208-213
- **LOTERSZTAJN, S. et al.** *Annu. Rev. Pharmacol. Toxicol.,* 2005, vol. 45, 605-628
- **JULIEN, B. et al.** *Gastroenterology,* 2005, vol. 128, 742-755
- **PATEL, H. J. et al.** *Brit. J. Pharmacol.,* 2003, vol. 140, 261-268
- **YOSHIHARA, S. et al.** *Am. J. Respir. Crit. Care Med.,* 2004, vol. 170, 941-946
- **YOSHIHARA, S. et al.** *J. Phannacol. Sci.,* 2005, vol. 98 (1), 77-82
- **YOSHIHARA, S. et al.** *Allergy and Immunology,* 2005, vol. 138, 80-87
- **KARSAK, M. et al.** *Human Molecular Genetics,* 2005, vol. 14 (22), 3389-3396
- **BOYLE, W. J et al.** *Nature,* 2003, vol. 423, 337-342
- **GROTENHENNEN, F. ; MULLER-VAHL, K.** *Expert Opin. Phannacother.,* 2003, vol. 4 (12), 2367-2371
- **RALSTON, S. H. et al.** *Nature Medicine,* 2005, vol. 11, 774-779
- **STEFFENS, S. et al.** *Nature,* 2005, vol. 434, 782-786
- **SANCHEZ, C. et al.** *Cancer Res.,* 2001, vol. 61, 5784-5789
- **CASANOVA, M. L. et al.** *J. Clin. Invest.,* 2003, vol. 111, 43-50
- **MCKALLIP, R. J. et al.** *Blood,* 2002, vol. 15 (2), 637-634
- **LEPICIER, P. et al.** *Brit. J. Phann.,* 2003, vol. 139, 805-815
- **BOUCHARD, J.-F. et al.** *Life Sci.,* 2003, vol. 72, 1859-1870
- **FILIPPO, C. D. et al.** *J. Leukoc. Biol.,* 2004, vol. 75, 453-459
- Methods in Cell Biology. Academic Press, 1976, vol. XIV, 33
- *J. Pharmaceutical Sciences,* 1977, vol. 66, 1
- **NEGISHI, E. A.** Handbook of Organopalladium Chemistry for Organic Synthesis. Wiley-Interscience, 2002
- **MIYAURA, N.** Cross-Coupling Reactions: A Practical Guide. Springer, 2002
- **T. GREENE ; P. WUTS.** Protecting Groups in Chemical Synthesis. John Wiley & Sons, 1999
- **HARTMANN, WILLY et al.** *Synthesis,* 1989, vol. 4, 272-4
- **CHRISTOPHER et al.** *Bioorganic and Medicinal Chemistry Letters,* 2004, vol. 14 (22), 5521-5525
- **BAKER, TRACY J ; WIEMER, DAVID F.** *J. Org. Chem.,* 1998, vol. 63 (8), 2613-2618
- *European Journal of Organic Chemistry,* 2003, 4286-4291
- **WEYER et al.** *Arzneim. Forsch,* 1974, vol. 24, 269
- **VOGEL et al.** *Tetrahedron,* 1993, vol. 49 (8), 1649-1664